# EUROPEAN PATENT SPECIFICATION

(11) **EP 1 795 597 B1**
(45) Date of publication and mention of the grant of the patent: **18.08.2010**
(21) Application number: 06017801.9
(22) Date of filing: 30.05.2002
(51) Int. Cl.: C12N 15/12, C12Q 1/68, G01N 33/50

(54) **Nucleic acids isolated from a neuroblastoma**
Aus einem Neuroblastom isolierte Nukleinsäuren
Acides nucléiques isolés d'un neuroblastome

(30) Priority: 31.05.2001 JP 2001163666; 24.08.2001 JP 2001255260
(43) Date of publication of application: 13.06.2007
(62) Divisional of application: 02730804.8
(73) Proprietor: Chiba-Prefecture, Chiba-shi, Chiba 260-8667 (JP); Hisamitsu Pharmaceutical Co., Inc., Tosu-shi, Saga 841-0017 (JP)
(72) Inventor: Nakagawara, Akira, Chuo-ku Chiba-shi, Chiba 260-0801 (JP)
(74) Representative: Grünecker, Kinkeldey, Stockmair & Schwanhäusser Anwaltssozietät

(56) References cited:
- DATABASE EMBL [Online] 20 March 2001 (2001-03-20), "Homo sapiens 24-dehydrocholesterol reductase, mRNA (cDNA clone MGC:10569 IMAGE:3638639), complete cds." XP002429722 retrieved from EBI accession no. EMBL:BC004375 Database accession no. BC004375
- DATABASE EMBL [Online] 8 February 2000 (2000-02-08), "Homo sapiens chromosome 8, clone RP11-531A24, complete sequence." XP002429723 retrieved from EBI accession no. EMBL:AC022893 Database accession no. AC022893
- DATABASE Geneseq [Online] 8 February 2001 (2001-02-08), "Human ORFX ORF1696 polynucleotide sequence SEQ ID NO:3391." XP002429724 retrieved from EBI accession no. GSN:AAC76141 Database accession no. AAC76141
- DATABASE EMBL [Online] 7 July 1998 (1998-07-07), "Rattus norvegicus m-tomosyn mRNA, complete cds." XP002429725 retrieved from EBI accession no. EMBL:U92072 Database accession no. U92072
- DATABASE EMBL [Online] 22 November 1999 (1999-11-22), "Homo sapiens clone RP11-25P1, WORKING DRAFT SEQUENCE, 22 unordered pieces." XP002429726 retrieved from EBI accession no. EMBL:AC016097 Database accession no. AC016097
- DATABASE EMBL [Online] 8 February 2000 (2000-02-08), "Homo sapiens chromosome 3 clone RP11-723O4 map 3, WORKING DRAFT SEQUENCE, 14 unordered pieces." XP002429727 retrieved from EBI accession no. EMBL:AC022993 Database accession no. AC022993
- DATABASE EMBL [Online] 2 May 2001 (2001-05-02), "Mus musculus testis expressed gene 10, mRNA (cDNA clone IMAGE:3595167), complete cds." XP002429728 retrieved from EBI accession no. EMBL:BC006867 Database accession no. BC006867
- DATABASE Geneseq [Online] 9 July 1999 (1999-07-09), "cDNA encoding a protein identified by the signal sequence trap method." XP002429729 retrieved from EBI accession no. GSN:AAX35737 Database accession no. AAX35737
- DATABASE EMBL [Online] 23 February 2001 (2001-02-23), "602381532F1 NIH_MGC_93 Homo sapiens cDNA clone IMAGE:4499190 5', mRNA sequence." XP002429730 retrieved from EBI accession no. EMBL:BG289571 Database accession no. BG289571
- DATABASE EMBL [Online] 29 November 1999 (1999-11-29), "Homo sapiens chromosome 10 clone RP11-541M12, complete sequence." XP002429731 retrieved from EBI accession no. EMBL:AC016397 Database accession no. AC016397
- DATABASE EMBL [Online] 25 March 2001 (2001-03-25), "Homo sapiens Yip1 domain family, member 4, mRNA (cDNA clone MGC:11061 IMAGE:3687693), complete cds." XP002429732 retrieved from EBI accession no. EMBL:BC004875 Database accession no. BC004875
- NAKAGAWARA AKIRA ET AL: "Association between high levels of expression of the TRK gene and favorable outcome in human neuroblastoma" NEW ENGLAND JOURNAL OF MEDICINE, vol. 328, no. 12, 1993, pages 847-854, XP009038449 ISSN: 0028-4793
- NAKAGAWARA A ET AL: "INVERSE RELATIONSHIP BETWEEN TRK EXPRESSION AND N-MYC AMPLIFICATION IN HUMAN NEUROBLASTOMAS" CANCER RESEARCH, vol. 52, no. 5, 1992, pages 1364-1368, XP001203506 ISSN: 0008-5472
- HOEHNER J C ET AL: "Spatial association of apoptosis-related gene expression and cellular death in clinical neuroblastoma." BRITISH JOURNAL OF CANCER 1997, vol. 75, no. 8, 1997, pages 1185-1194, XP009082234 ISSN: 0007-0920
- KOIZUMI HIROTAKA ET AL: "Apoptosis in favourable neuroblastomas is not dependent on Fas (CD95/APO-1) expression but on activated caspase 3 (CPP32)" JOURNAL OF PATHOLOGY, vol. 189, no. 3, November 1999 (1999-11), pages 410-415, XP009082208 ISSN: 0022-3417
- YAMASHIRO D J ET AL: "Expression and function of Trk-C in favourable human neuroblastomas." EUROPEAN JOURNAL OF CANCER (OXFORD, ENGLAND : 1990) OCT 1997, vol. 33, no. 12, October 1997 (1997-10), pages 2054-2057, XP004284644 ISSN: 0959-8049

### SEQUENCE LISTING

<110> Chiba-Prefecture
<120> Nucleic acids isolated in neuroblastoma
<130> EP29236IFZ
<140> not yet assigned
   <141> 2006-08-25
<150> EP 02 730 804.8
   <151> 2002-05-30
<150> JP2001-163666
   <151> 2001-05-31
<150> JP2001-255260
   <151> 2001-08-24
<160> 366
<170> PatentIn ver. 2.1
<210> 1
   <211> 824
   <212> DNA
   <213> Homo sapiens
<220>
   <221> misc_feature
   <222> (1)...(824)
   <223> n = a, t, c or g
<220>
   <223> nbla-00015-f
<400> 1
   ggatcctcga gcactgngtc gcctactggg atattgacag tgcctcagtg cccagcttag 60
   aagagctgaa ccagttcagg caacatgtga caactttgag ggaaacaaag gcttctaggc 120
   gtgaggagtt tgtcagtata aagagacaga tcatactgtg tatggaagaa ttagaccaca 180
   ccccagacac aagctttgaa agaggtgtgg tgtgtgaaga cgaagatgcc ttttgtttgt 240
   ctttggagaa tattgcaaca ctacaaaagt tgctacggca gctggaaatg cagaaatcac 300
   aaaatgaagc agtgtgtgag gggctgcgta ctcaaatccg agagctctgg gacaggttgc 360
   aaatacctga agaagaaaga gaagctgtgg ccaccattat gtctgggtca aaggccaagg 420
   tccggaaagc gctgcaatta gaagtggatc ggttggaaga actgaaaatg caaaacatga 480
   agaaagtgat tgaggcaatt cgagtggagc tggttcagta ctgggaccag tgcttttata 540
   gccaggaaca gagacaagct tttgcccctt tctgtgctga ggactacaca gaaagtctgc 600
   tccagctcca cgatgctgaa gattgtgcgg ttaaaaaact actatgaagt tcacaaggaa 660
   ctccttgaag gtgtccaaaa gtgggaagaa acctggaggc tttcctaaag tttgaganaa 720
   aagcntcaaa ttcaaatcca nttacaaacc ganggaggaa tcctccaaaa gaagaaaaca 780
   acgaagccaa nctccaaaaa atgctgccca annctggaan aana 824
<210> 2
   <211> 806
   <212> DNA
   <213> Homo sapiens
<220>
   <221> misc_feature
   <222> (1)...(806)
   <223> n = a, t, c or g
<220>
   <223> nbla-00015-r1
<400> 2
   ttttttttgt nttttttttt ttttgttttt tcattatggt tctcggtgta ctttattctc 60
   ctgctgtcta gctaagcagc ccacacagga cagaaatggg tancactgag gaatgatcag 120
   attttttttc tatccccaat atttctgccc cctccctaan ccctgaccca accctatctt 180
   aataatcacg aatggttagt acatcattct gtctctcaaa gaacaactga aatggaaaca 240
   aggcattaga ggaccangcc tcttcttaat agggcttang anctggtatg agctctttat 300
   tcacaatgga gcgtctctcc ttcaaatacc tggatttttt ttttttttgt acactggttc 360
   atagatcggc acttggcttt gaacctggca ccaaaaggca caatatctga taccctgtac 420
   aagagctatt agagatgctg ccatatggat gggcaaaact gagccaatcc cacttaggaa 480
   tggaaggctt ggacatggaa gggaggatat aaacgaggag ttggagaaaa acgcaagccc 540
   antttttgct anagtggaaa tgaaagtggg aatgagggtc ttgtttttan tcctctaagg 600
   nccaaggaag caattttaaa acttccctgg gttttctgaa agcagcatat tcaaaatgcc 660
   aacaaaaact cctaacaact gnaaaaccaa aagagggtta aagctcaaca aaatcccttc 720
   ctaatgctga aaggntctaa aattcangat gccctgttcc cttgtaaagg gaaaatantt 780
   aatctgattt natgggaatc caancn806
<210> 3
   <211> 3181
   <212> DNA
   <213> Homo sapiens
<220>
   <223> nbla-00018
<400> 3
   cctactggta tgatgatcta gttgtgtagg aaagaacttg gcatgtatgg cacagatcag 60
   tgactggtta acatgtccat tgggctgcct ttttcaaata ttgggtattt tatacaaggt 120
   tttgatcgat attggaccac attcagaacc ctactaaatc atacaaaggg ttactttatt 180
   taagatattt taatgtttgg ataaaatgag agcaatgttt ttagatactt gaactactaa 240
   gcctgttggt ggaaaaataa aaggattaga cttctcttct gcacttatat actgttggta 300
   ggaacgtaaa ttagttcagc cacaaattgg aaagcagttt ggagatttct caaagaactt 360
   aaactaccat tcaacccagc aatcccatta ctgaatatgt attcagaaga aaataaattg 420
   tcttgccaaa aaatacgtgt tcacatgttc attgaagcac tatttatgat agcaaagaca 480
   cagaatcaac ctaggtgccc atcaacagtg gactggatga agaaaatgtg gtacatatat 540
   actatggact accacacagc catgagaaag gaacaaagtc atgttttttg cagcaacatg 600
   gatgcagctg gaggccatta tcccaagcaa attaatgcag gaacaggaaa ccaagtacta 660
   cgtattctca tttactagtg ggagctaaat gtggggtact catggacata aagatgacaa 720
   caatagacac tggggactac tgttgtggca gagttgaaaa actacctatt gggtactatg 780
   ctcacaacct aggtgatgag atctttcgca caccaaactt cagtgtcatg caatataccc 840
   acgtaacaaa cctgcacata tacctcctga atataaaggt tgagattatt tttttaaaag 900
   aattatgttc tgtccaaacc tgaggccaac atggcttagc acatgcatga gcaaaacagt 960
   atccatggat gagcattatc tgggggattt cttttctaaa ttcaatccaa tttagcaata 1020
   tttatagagc atctactata agctggacct gaaggttcag acttgagtta cctactctac 1080
   cattagtact tgttactaat gtgtgaatac ttagtacttt attagtccct acactaccaa 1140
   tttgttcagg caccaaggtg ccaaaccata taatacaatt tctcccgtaa gtccccaaag 1200
   catagctgca tctaggctat aatgctgaat aggacttttc taaaaactca ggaattgatg 1260
   aaaggagttg aaagcagaaa ggcaaagctg aaggataggt cctctgtaac agtgtcccca 1320
   aagaccacag gctgtgcccc cttccttgga gttcgtctgg tgaccccttc tgagcccccg 1380
   gagaccactg tgttgcagag tctatctcat ttcccccaag tgggaacccc tttaccagat 1440
   ctctcctagc cagagtccag gcctcttaac cacctctggg ccccagtgag gaaggaaagt 1500
   ctgttttctc tacacctggg ctatgttact tcagtagcca ctaacctggg gaactagctt 1560
   tgatttaaat aaattttttt ttattctcaa gagcatttta agtgctcaat agctagtggc 1620
   tactgtctta ttaaatggca cagatatatc gcatgtccat catcatagaa agttattttg 1680
   aatgaagcca tgacaccatc catcttaggc cttccttccc ttcaatgtca taatgatctg 1740
   aaaatgcaac atttccctca aggaagatgg ttagtccctg accccatagg tgagcatcac 1800
   agcctgtact tccactcaca ttaggcctca aatataattt tatttatctg ctattgtcta 1860
   ttttgcacac tctatttgct atctaagccc ctggaaagtt gaggtgccct agtcatcctc 1920
   tgtctctagg ttttcctttg gtccccaggt gcttaccatg tgcctggctc tgtgcaagtg 1980
   actttaacat gaacttaatt ctctcagtct ttcagaggat gatttgtttt tgctgcctga 2040
   tagccttttt tctatttacc ttccttgaca tattaataga gtcaccaaaa tgttcagctg 2100
   catttcattc acctagaagg caatgaatgt aaaaacatca acaattctgc ctgctacgaa 2160
   ataagtgctc agtaaatgtc cattcccttc tttacttctg caggtgatat ttatagtaat 2220
   aatgattttt taagttataa tttattatcc agcttcttta ttaggcattt tacacttatt 2280
   gtcataacaa tcctatgagg gctaaagtaa aaatgtaata aaatatagct ctctcccaca 2340
   tcctgttttt catctcacat gcagattttg aattccccaa gaacagacct gtgtcatctt 2400
   acacattttg gaatccctgg agtatagccc agtacctggt gctgggaggg actcagtata 2460
   tatttattga attcaaaaat gaatgaataa atcacttctt ggccttcctg tccgccactc 2520
   ctggtcaccc agcacctata caaacctgct ttcctctccc tagcctatca aaattcctgt 2580
   tacttcaact tgaatctctc ctggtctagg agctgctttc caaactaaac tcagctgcgt 2640
   taagggagat ggaaaaattc acaggatact aatagaattt tagaccagtg caccaaaccc 2700
   aatgtcagag gcagaggggc aagtttattt gagcagcaag gagactttgc attcagctct 2760
   gtagatgacc ttggaagaca ggcctaggct aactgggcta agcctaggct aaaagtctat 2820
   cgccaagctt tgttcaaaat taagagtgtt ttaagcaggg cctcaggctc tgggctgtga 2880
   aggccccaag tgaccaccag aagatgctag aaaggacatc agctactgga gcatggagag 2940
   gaggtgagcc tggctccagt aagagtgtgg caaactcctg atggttttac aagggtgtag 3000
   ggcttttgga gactctactt tggtttgtgt agtgatgcat ttgcaaacat atgtttaaaa 3060
   tactaacggc tctccacatt cttagatttc acatccatgg ttttaaccaa ccatggatgg 3120
   aagatatttg gggggaaaaa aaaaaaaaaa aaaaaaaagg ccacatgtgc tcgagctgca 3180
   g                                                                 3181
<210> 4
   <211> 869
   <212> DNA
   <213> Homo sapiens
<220>
   <221> misc_feature
<222> (1)...(869)
   <223> n = a, t, c or g
<220>
   <223> nbla-00059-f
<400> 4
   cncgancact gttggcctac tggttttttt ttttgcggct ccacgtcggc accagctgcg 60
   gggcaagatg gaggcgctga ttttggaacc ttccctgtat actgtcaaag ccatcctgat 120
   tctggacaat gatggagatc gactttttgc caagtactat gacgacacct accccagtgt 180
   caaggagcaa aaggcctttg agaagaacat tttcaacaag acccatcgga ctgacagtga 240
   aattgccctc ttggaaggcc tgacagtggt atacaaaagc agtatagatc tctatttcta 300
   tgtgattggc agctcctatg aaaatgagct gatgcttatg gctgttctga actgtctctt 360
   cgactcattg agccagatgc tgaggaaaaa tgtagaaaag cgagcactgc tggagaacat 420
   ggaggggctg ttcttggctg tggatgaaat tgtagatgga ggggtgatcc tagagagtga 480
   tccccagcaa gtggtacacc gggtggcatt aaggggtgaa gatgtccccc ttacggngca 540
   aancgtgtct caagtgctgc agtcagccaa agaacagtna agtggtcaca ccttcggtga 600
   agactcactg ttcctggctc ctcatcctcc tcaaaaaatt gcatgtccgc tgtgaatttt 660
   catccaattc cccaatcgat gctccaaggg tcatctccgg ggatnacaag gattcttaaa 720
   tctcantctg tttgtggntg cccctcaacc tcccctanaa ccttccnatc ctttcaatcc 780
   tcttcaattc tgggagtaaa gctccaanca tattnagtaa taggcanggg aaacaacctc 840
   cttcttttan acnggnttaa gcncaaatg 869
<210> 5
   <211> 906
   <212> DNA
   <213> Homo sapiens
<220>
   <221> misc_feature
   <222> (1)...(906)
   <223> n = a, t, c or g
<220>
   <223> nbla-00059-r1
<400> 5
   gnngnnnnnn tgtggctttt tttttttttt tcagtttgga aaaaatgttt tattcctctt 60
   tgcacagagc agtttatgaa ggtgggtttt ctcctgactc catgcatctt ttacacaaag 120
   atgccccctt aaatatgccc agttatctgc cccacctcag tgctggagaa ctggcagtta 180
   gtaagtgggg cagaatgctt aagtctcagg aaggttttta aaggcatttt tgtggggagg 240
   aagttctggg tcaaggggaa agattagacc caagagtgag tattccattc tccatcttcc 300
   tggggaaatc caaaccccaa aggttttatg aagaaaagca cctctctcag cgacctagag 360
   acagggagag cacagaccta ctgcttgggt gtaaggctga ggcagagaga gggtaggttg 420
   cagcgactgc agacccacgg cagagagtaa atgcatgtcg ggagctgagg gggacagaga 480
   cagcctanag gccaagtcat aagttccact ccttccccag ttctgagtag aaactttctt 540
   cccaagacta naatggagtt ttaagtttan gaactggctt tgcccaagga cacagagaag 600
   acaaaccaag caacgattcc acangtagta angggtggac agttaaggta gctaactaag 660
   agatgggcac tcgccactgc agttttgaac tatatgncaa atnanggtac agatgcattt 720
   tatatgncct ggtcatacaa tttaaattgc gttttccatg ttgtcccttc cctatgaact 780
   attccaaaag cctcttccaa ggaaagggan agggnaattn gaaggaatgg ganaaaacac 840
   ggaggcacnn aantggggta nggctnaant ggtaaatcct accatcccgn ccaaancgtn 900
   tatnaa 906
<210> 6
   <211> 906
   <212> DNA
   <213> Homo sapiens
<220>
   <221> misc_feature
   <222> (1)...(906)
   <223> n = a, t, c or g
<220>
   <223> nbla-00072-f
<400> 6
   gngnggnnnn nnnnaatctc gancactgtt ggctactggg gtggctagcc tgtcctgaca 60
   ggggagagtt aagctcccgt tctccaccgt gccggctggg ccaggtgggc tgagggtgac 120
   cgagagacag gacccttgac atctccatct gcacagaggt cctggctgga ccgagcagcc 180
   tcctcctcct aggatgacct caccctccag ctctccagtt ttcaggttgg agacattaga 240
   tggaggccaa gaagatggct ctgaggcgga cagaggaaag ctggattttg ggagcgggct 300
   gcctcccatg gagtcacagt tccagggcga ggaccggaaa ttcgcccctc agataagagt 360
   caacctcaac taccgaaagg gaacaggtgc cagtcagccg gatncaaacc gatttgaccg 420
   agatcggctc ttcaatgcgg tctcccgggg tgtccccgan gatctggctg gacttccaga 480
   gtacctgagc aagaccaaca agtacctcac cgactcggaa tacacagagg gctccacaag 540
   taagacgtgc ctgatgaang ctgtgctgaa ccttaaggac ggagtcaatg cctgcattct 600
   gccactgctg caaatngaca aggactctgg aatcctcaan ccctggtaaa tgcccaantg 660
   cacanatgac tattaccgan ggcacaacgc tctgcacatc gccantgaga agangagtct 720
   gcaatgtgtg aagctcctgg tgganaatgg gggcaatgtt caagnccngg cctgcggcgc 780
   tcctcaaaaa gggcaangga cttgcnttaa ttcggtgagt ancctcncct ttggcgcttc 840
   acaagcatgg gatntggaac tactcctggg aancaaacaa ccgcaacctt gaagcactgn 900
   tcccag906
<210> 7
   <211> 891
   <212> DNA
   <213> Homo sapiens
<220>
   <221> misc_feature
   <222> (1)...(891)
   <223> n = a, t, c or g
<220>
   <223> nbla-00072-r1
<400> 7
   tgtggctttt ttttttntnt tttagttagt gaaaatatat atttgccacc agaattcact 60
   gggaccccag agccagcaga tgtggttgga aagatcctct gctctgtcct ctggcctcct 120
   gctgcatctg ggccatcagt tggactggag gagctggacg ggcacatagt tttcctcaga 180
   ggcaccatcc tcatcctcct tgggagggga agccaggaca gggttctcga gagttcgagg 240
   gacacctgcc cctgacgggt cctcacacag cgtaggcagc gtctgctccc atgaagccca 300
   gttcacctcc tccaccctga agcaccagcg ctcatcgggg ctgccatctg gcttagtgcc 360
   aacggtcagc atcacacctg cccgctgctt cttcctgcac caccaatagc cattctccat 420
   ctccaggaca gagatggctt tctgcagctt ccagatgctc cagctgtcag tggcgacact 480
   gttgacggtc tcgctcatga nggcgatgag catgttgagc aacangatgt aggtgagcag 540
   cacgtaggcc aacaagcaac ancaccatgc cgcggaagtg caactgctcc tggaanggca 600
   actcgcccat gccgatggtg aatttgaaga actccaagga gnttccagga taccctgtac 660
   tgggcccgtt tgccctcntc ctcctgtccc tccatgggct gcactgactc cgttgcantg 720
   gggcctgtan gancttcngg gcgccaaacc tcctggntca agctcaacaa gggctacanc 780
   gaanccnaaa agggannCta anttnnttan aaggaacnca anaaggtccc caggatnacc 840
   ttcggnttat gaaacgtnaa tncctgtntt ctgggaacca ctnntnnatn a     891
<210> 8
   <211> 3381
   <212> DNA
   <213> Homo sapiens
<220>
   <223> nbla-00086
<400> 8
   gcctactggt cgacgtgtgg cgtcggctct acccggaatg gagaatatcc aggagaaaag 60
   caaagaaggg atgatcgaca tcaagctggg caaaccccag gagcccccgg ccagcgaggg 120
   cggctgctcc tgctaatgca gagccgacct gtggcttccc atgacactcc ttgcttgttg 180
   tgttgcttcc tattggctag cttcctaagg ggggagggaa ccgagttatc aagatgggag 240
   gatttttctt ttctctctgt ctttaggagt agggtgggat ggggagggag gctgggcatc 300
   agggatcaca tcactcttaa cggctgttac ttaaacaact attttttggt ttggttgtaa 360
   tatattgtac tttattaaga ttgccaaaaa ctgttaaaat ttaaaaaaaa tttaaatcat 420
   gtgtatacaa ttttttgcca gataaaaatg tagtcatttt tatttgaaag atgtgctttt 480
   tgtttttgta tatttgtaaa cttatagaga accttttcca cacacctcct ccttcctgtt 540
   ctctttgaac cgttcatcac ctctgccttc ctcctatccc cagcccaata aattaaaaca 600
   attaactgag caaattaatt aggcttcagt ctggggccat ctggccccac tctctaggcc 660
   ctactccagt taaatcaaac attggttgaa cacatcagcc tctgaaaagg tagctctgac 720
   tcttgtcttt ccatggccag agtgggtcgt caacctcgtg tggagctgag accaaggtct 780
   gtgggctcta ggcagcccgg cagcagcccc cagaggaact gctcccacct ctccttctct 840
   ttccctgaaa agccttgaag gggttgaggg gtacaggcaa ccttgtgttc tctgtgccct 900
   cagggactgg cagagcaaga ggccatcaga aggacgaggt gctgttggca attgcagctg 960
   cgtggtgcgt ggtttataga ctggggtgcc atgtgtactt ctgaaaatat tttaaccagc 1020
   ccacccagca gtcagtacac atgcctgggc caagtctaag aaactcagtg tgtgatgttg 1080
   ggcagtgtgc catcctgaag ccttcctcct ggatgtcagg gaagctggac cacctggagt 1140
   tgatcctctg tgatggggag gcgggggggg gggggtgttg tctgcagaac caccaccttg 1200
   aaagatccat gttcttagag cctcagctgc taatattgtt gagtgctttc tggaaaagct 1260
   gatttgttgc tatgttttca ccccagtggc atggcgcagt tgaagtcttt gtggacatat 1320
   ctttgtggct actgtggctg cgctgtggtc acctgaggat gtggttcagg tggggtctcc 1380
   agaaggctgc ttgcccccag agagccatct cttccatttt tgattcattc ctgagaatgg 1440
   cttagtcctg agaatctgca aagtctctga agagaactct ctcttcccac taagtgatat 1500
   aacctacttg gccagcattc caaatagact cagactcact gccctgagcc tgcacagagc 1560
   caggtgcaat gggcagtgag tctggccaaa atgtaaaccc caccctccct gctgtcattg 1620
   gctctccctc caggctgctg ctgggcatgt catagatggg atgaatgacc atctggggta 1680
   tggtgggctg aactccacaa ggtcgttgga atgaattttg aaacttctca gaaattaatc 1740
   tgaaatggca ggcagtaagt gcagcagaat ctggcaggac tgatatttgt gcagtgggcc 1800
   tgccacacac cctctgccta ggaggctgta tgccagagct catctctagc aaggaattta 1860
   tcttcaggtg aagaagagag gaagcaggat cccactgagc acctcttgca gaaggcaagg 1920
   accaatgttt gaacgtgttt cagatgtcat gtgtttagtt tttatatgtg tgtacataat 1980
   tatatataga gatagacttg tgtatacacc ttccacaaac acatcctcac ggagatacat 2040
   gtgctcaatg tatattcttg tttctcctgc tctcaagttg gaacttgcat tgtgataagt 2100
   tttggaaatt caaaagtact gcaacgtgtt gaaataagaa atacttaata ttgtcaaata 2160
   acttgtgatt gtcttgccat caataaaata atgcatagtt gtgttaaact acaggcaaac 2220
   atagcccacg ggaatatctc caccatttct ctatgtactt tatatgcacg ggtgtgcatg 2280
   cctgtgtggg ccagtgtccc cccaccatgg gcacaccccc aagtggtgct tgttggaaga 2340
   gtgcttactt acctcgagag aatttacagg agtccacatg acagtgtcct gcctggttgg 2400
   cagcgggggc catcacctgt ggcctctgag accgccccat ttacaggaat ctgcactggg 2460
   tctcagtgaa tgaacgtatg gtagattttt attttcttgg cactaaagat tagctttttt 2520
   aaaaaaacat cccataaaac agtagaaact cagttccctt ctcaggggca cacacatcca 2580
   aggcagccag acctcttttt cagaagttgt tggttccctg atttttgtct tgagcaggca 2640
   ccatccctgt ccccttgctt gcaggtagaa ttggcacaga gtgaagtttc catttttcat 2700
   ccattgctgc cctgtgtgtg aacaaatgtg actccatatt tattacacag caaggactgc 2760
   tgtgtgaggg tgtgttcctc agaccatctc tactcttttt ggttaggaca ctaggttctt 2820
   cccagcatat gagtgagaat attggtggta ttttgggctc ctctactgca caagggccag 2880
   tccccaagtc tactctgatt gattgtcatg ggcacccaca cagatcagga caccctttga 2940
   atttttgttt gacatgaact atttaggtgt tccaaaacct tgggatctca atgtgcaaac 3000
   tccttggact ccagacccct gtctggttgg cactcccagc agtgtgtggt ttcatcagga 3060
   aatccttgca gcaggggttt agatggttcc ttcaggaggt gggaagcctc catagcattg 3120
   aaggccagcc tgcatctcgc ggtgtgtgtc cacttctccc ctcactttcc cctcacccat 3180
   ccctttaaag tgattggctg tgaggagcac agagggtggc aattccatct gttatgggct 3240
   cagaagacat agccgccatg ttgatttcac attaaactgt gtaaaatgtg gaaaagactc 3300
   agcatgttgg tcaaacaata aactgtccta attctcaaaa aaaaaaaaaa aaaaaaaaaa 3360
   aaaaaggcca catgtgctcg a                                      3381
<210> 9
   <211> 866
   <212> DNA
   <213> Homo sapiens
<220>
   <221> misc_feature
   <222> (1)...(866)
   <223> n = a, t, c or g
<220>
   <223> nbla-00110-f
<400> 9
   tctcgagcac tgtngggcct actgggctca ttttctcttg ctgccgccat gattctgagg 60
   cctccccagc catgtggaac tgcaatactt taaagacaca aagtctcaac aaccatcttc 120
   cgcttgacga gacagatcat tctaatttga gcagaagcta ctatgtcctg ccctttgaac 180
   gcggcggccc ggacagctga caaggacaca ctgtgtattt ccattccaat tctgggagtg 240
   ctctgaggcc tctgggggag aaggacccat gaaatattca aaacataagt gaataaaata 300
   tctaggtgct agatatgggc caggaagagc cctcggccct gcaaagtgtg tgtgatggtg 360
   agaagctacc ggaagagatg gtccctgtgc ttggttcatt ccttggacat ttatcaagct 420
   gacgaatgta gcagaggtgc ttcagtcggc tgtaattcca cgggtggagt gctggctgga 480
   gagttacctg gggctgtcac actgcatggg ctccgggaca ctgtggctgc ccttatgtgg 540
   tgtccccgga gggccctgca ngtgtcacac cgctgctcca cactgccacc tgctgtcaac 600
   atctgtgcaa cgtatccaag tctctggggg ctaaantgaa aaacatgcat ctcgtaacca 660
   atgaaatccg ggcttgtcct gaanactcct gcantcatcc antccacact gctataaaga 720
   catactaana ctgggcactt catggnaaaa aaaggtttgg ttggtcacgg ntctgaaggc 780
   tttanaggaa ccgtggaact ttcanttntg gggaagcctc aaggaacttt tancaanggn 840
   ggaaggtgaa nggggganaa ggntnt                                  866
<210> 10
   <211> 888
   <212> DNA
   <213> Homo sapiens
<220>
   <221> misc_feature
   <222> (1)...(888)
   <223> n = a, t, c or g
<220>
   <223> nbla-00110-r1
<400> 10
   tgaaggnngt tcactgccac acaacatctg catcagagac aaaagtaacg gggctcgagg 60
   cgggactggg ctggctctgc ccagtgcacc atataacagc acggacaaaa ctacacaaat 120
   cccagctggg tacagtggct cacgcctgtt ttcccagcat tgttcgaggc tgcggtggga 180
   ggatcacttg aggtcaggag ttcgagacct gcctggccaa catggtgaaa ccccatctcc 240
   actaaaaata caaaaattag cagggcatgg tggcacgtgc ctgtgatccc agctactcgg 300
   gaggttgggt gatttataaa gaaaagaggt ctatttggca cacagttctg caggctgtac 360
   aagaagcacg gtgccagcat ctgcttctgg tgaggcctca ngaagtgtcc actcatggca 420
   gaaggcgatg ggagcaagtg catgcagacc atatggtgag agagggaaca agagagagtg 480
   ggcanaggan gtgccaagcg tttttaacaa tcagttcttg tgagaaggaa taaagcatga 540
   atgcattcat gaccgcgagg acagcaccaa gctctcatgt cctaagcacc ttctgtcaag 600
   tcccacctcc aacattgggg ttcaaatttc aacatgaggn tggaagtggg acctggtggg 660
   aaaggattgg atcatggtgg tggtccctca caagtgggtt ancgccaact tcttggngct 720
   gttcncatga taatgagtta ccgttaaatn tgggtgttca aaatgtcggg accccctcct 780
   tttctccccn cnacnntccc atttaaaacn cctttccgcc ttcaacttcc aacatgacta 840
   taatttcctg aggcctccca aaaattgaaa ctgcannctc cttaaanc888
<210> 11
   <211> 850
   <212> DNA
   <213> Homo sapiens
<220>
   <221> misc_feature
   <222> (1)...(850)
   <223> n = a, t, c or g
<220>
   <223> nbla-00132-f
<400> 11
   gnngnnnnnn nnnnnnnnnn ggattctcgt ngcactgtgn cgcctactgg taagatacca 60
   aggaggagtg agagcgtccc tgccttcgag gagcagacag ggcaccgcga ggcgtttgcc 120
   tcagcatgga gggcggggcc gcggcagcca cccccacagc actgccttac tacgtggcct 180
   tctcccagct gctgggcctg accttggtgg ccatgaccgg cgcgtggctc gggctgtacc 240
   gaggcggcat tgcctgggag agcgacctgc agttcaacgc gcaccccctc tgcatggtca 300
   taggcctgat cttcctgcag ggaaatgccc tgctggttta ccgtgtcttc aggaacgaag 360
   ctaaacgcac caccaaggtc ctgcacgggc tgctgcacat ctttgcgctc gtcatcgccc 420
   tggttggtga gttcccgggc gcggccttcc ccgccacctg cctgcctctt caggcttggt 480
   ggcggtgttc gactaccaca ggaagaaggg ctacgctgac ctgtacagcc tacacagctg 540
   gtgcgggatt cttgtctttg tcctgtactt tgtgcaattg gctggtgggc ttcagcttcn 600
   tcctgttccc cggagcttca ntctccctgc ggagccgcta ccgcccacan cacatcttcn 660
   ttggtgctac atcntcctcc nttccgttgg caacgccctg ctgggcctga aggaggaatg 720
   ctgttcaacc tcnggggcaa tntancncan ttnancccca nggtgtcctg gccaaagtnc 780
   tgggcctgct gctggcctgc ttcngtgggg nggtgcncna aancttgacc cgggccnnct 840
   ggaancggcn                                                  850
<210> 12
   <211> 860
   <212> DNA
   <213> Homo sapiens
<220>
   <221> misc_feature
   <222> (1)...(860)
   <223> n = a, t, c or g
<220>
   <223> nbla-00132-r1
<400> 12
   ggtgnnnnnn tgtgggccgt ttttttgcct tttttttttt tcngctgtag acatttattg 60
   aatcatctgt tgcaccaaca gcacattgta tttcacagaa gattggccac aggcaaaata 120
   aaataaccca ccacaaatcc gcttctgggg gccttcttca gctccttagt catccagtgt 180
   tcactttggc aaacagacca gtttacagac ttttgttgca aatctaaggt tccatttctc 240
   tctgtagcat tccggctaga gagagacacg attgtacttt ggtagtttgt ctaaggcgga 300
   gttttcccac atgtaaacat ttcactatac aagtgtgtgc cagctgattg aagaactgac 360
   ggnaccccta cccctgaaga gtgtacagtc taaacaggag gcgaacactg tcatccggac 420
   aggctgagag gagcagcctg ggaggaggcc cgcctgcatc tgccaagaga aagtctgtct 480
   gaagtcatgg gcttctatcc cctctcccaa caaagccatc ctaaggctaa agggttaaag 540
   aagangaaag tcangactac agctgggcca cagcctcatc tcaaaagctt cccaaggggc 600
   aatccctccc ctctcctggg gaancttgct ttcaccanca acatacttta cacatcatat 660
   cacttgctcc tctggcaaaa nggcgggncc aatttgtgcc acgtgttant caagctggag 720
   ggacnccntg nttgantcaa caaccatcgt gggctttcaa gcctgtccct gagggccccc 780
   aatttccctg gncttgncca agcatctgnt gncaanaagg gtggggcctc cccncngctt 840
   cttaaanatt ggngnccgcg                                             860
<210> 13
   <211> 3730
   <212> DNA
   <213> Homo sapiens
<220>
   <223> nbla-00170
<400> 13
   cggaagtgtt acttctgctc taaaagctgc ggaattcctc gagcactgtt ggcctactgg 60
   gagacctcta gaaaaagagg ctgtaggcca acttaagcct acaggaaaag aggacacaca 120
   gacattgcaa tccctgcaaa aggagaatca agaactaatg aaatctcttg aaggtaatct 180
   agagacattt ttatttccag gaacggaaaa tcaagaatta gtaagttctc tgcaagagaa 240
   cttagagtca ttgacagctc tggaaaagga gaatcaagag ccactgagat ctccagaagt 300
   aggggatgag gaggcactga gacctctgac aaaggagaat caggaacccc tgaggtctct 360
   tgaagatgag aacaaagagg cctttagatc tctagaaaaa gagaaccagg agccactgaa 420
   gactctagaa gaagaggacc agagtattgt gagacctcta gaaacagaga atcacaaatc 480
   actgaggtct ttagaagaac aggaccaaga gacattgaga actcttgaaa aagagactca 540
   acagcgacgg aggtctctag gggaacagga tcagatgaca ttaagacccc cagaaaaagt 600
   ggatctagaa ccactgaagt ctcttgacca ggagatagct agacctcttg aaaatgagaa 660
   tcaagagttc ttaaagtcac tcaaagaaga gagcatagag gcagtaaaat ctttagaaac 720
   agagatccta gaatcactga agtctgcggg acaagagaac ctggaaacac tgaaatctcc 780
   agaaactcaa gcaccactgt ggactccaga agaaataaat cagggggcaa tgaatcctct 840
   agaaaaggaa attcaagaac cactggagtc tgtggaagtg aaccaagaga cattcagact 900
   cctggaagag gagaatcagg aatcattgag atctctggga gcatggaacc tggagaattt 960
   gagatctcca gaggaggtag acaaggaaag tcaaaggaat ctggaagagg aagagaacct 1020
   gggaaaggga gagtaccaag agtcactgag gtctctggag gaggagggac aggagctgcc 1080
   gcagtctgca gatgtgcaga ggtgggaaga tacggtggag aaggaccagg aactggctca 1140
   ggaaagcccc cctgggatgg ctggagtgga aaatgaggat gaggcagagc tgaatctgag 1200
   ggagcaggat ggcttcactg ggaaggagga ggtggtagag cagggagagc tgaatgccac 1260
   agaggaggtc tggatcccag gcgaggggca cccagagagc cctgagccca aagagcagag 1320
   aggcctggtt gagggagcca gtgtgaaggg aggggctgag ggcctccagg accctgaagg 1380
   gcaatcacaa caggtggggg ccccaggcct ccaggctccc caggggctgc cagaggcgat 1440
   agagcccctg gtggaagatg atgtggcccc agggggtgac caagcctccc cagaggtcat 1500
   gttggggtca gagcctgcca tgggtgagtc tgctgcggga gctgagccag gcccggggca 1560
   gggggtggga gggctggggg acccaggcca tctgaccagg gaagaggtga tggaaccacc 1620
   cctggaagag gagagtttgg aggcaaagag ggttcagggc ttggaagggc ctagaaagga 1680
   cctagaggag gcaggtggtc tggggacaga gttctccgag ctgcctggga agagcagaga 1740
   cccttgggag cctcccaggg agggtaggga ggagtcagag gctgaggccc ccaggggagc 1800
   agaggaggcg ttccctgctg agaccctggg ccacactgga agtgatgccc cttcaccttg 1860
   gcctctgggg tcagaggaag ctgaggagga tgtaccacca gtgctggtct cccccagccc 1920
   aacgtacacc ccgatcctgg aagatgcccc tgggcctcag cctcaggctg aagggagtca 1980
   ggaggctagc tggggggtgc aggggagggc tgaagccctg gggaaagtag agagcgagca 2040
   ggaggagttg ggttctgggg agatccccga gggcccccag gaggaagggg aggagagcag 2100
   agaagagagc gaggaggatg agctcgggga gacccttcca gactccactc ccctgggctt 2160
   ctacctcagg tcccccacct cccccaggtg ggaccccact ggagagcaga ggccaccccc 2220
   tcaaggggag actggaaagg agggctggga tcctgctgtc ctggcttccg agggccttga 2280
   ggccccaccc tcagaaaagg aggaggggga ggagggagaa gaggagtgtg gccgtgactc 2340
   tgacctgtca gaagaatttg aggacctggg gactgaggca ccttttcttc ctggggtccc 2400
   tggggaggtg gcagaacctc tgggccaggt gccccagctg ctactggatc ctgcagcctg 2460
   ggatcgagat ggggagtccg atgggtttgc agatgaggaa gaaagtgggg aggagggaga 2520
   ggaggatcag gaggagggga gggagccagg ggctgggcgg tgggggccag ggtcttctgt 2580
   tggcagcctc caggccctga gtagctccca gagaggggaa ttcctggagt ctgattctgt 2640
   gagtgtcagt gtcccctggg atgacaactt gaggggtgca gtggctggtg cccccaagac 2700
   tgccctggaa acggagtccc aggacagtgc tgagccttct ggctcagagg aagagtctga 2760
   ccctgtttcc ttggagaggg aggacaaagt ccctggccct ctagagatcc ccagtgggat 2820
   ggaggatgca ggcccagggg cagacatcat tggtgttaat ggccagggtc ccaacatgga 2880
   ggggaagtca cagcatgtga atgggggagt gatgaacggg ctggagcagt ctgaggaagt 2940
   ggggcaagga atgccgctag tctctgaggg agaccgaggg agcccctttc aggaggagga 3000
   ggggagtgct ctgaagacct cttgggcagg ggctcctgtt cacctgggcc agggtcagtt 3060
   cctgaagttc actcagaggg aaggagatag agagtcctgg tcctcagggg aggactagga 3120
   aaagaccatc tgcccggcac tggggactta ggggtgcggg gaggggaagg acgcctccaa 3180
   gcccgctccc tgctcaggag cagcactctt aacttacgat ctcttgacat atggtttctg 3240
   gctgagaggc ctggcccgct aaggtgaaaa ggggtgtggc aaaggagcct actccaagaa 3300
   tggaggctgt aggaatataa cctcccaccc tgcaaaggga atctcttgcc tgctccatct 3360
   cataggctaa gtcagctgaa tcccgatagt actaggtccc cttccctccg catcccgtca 3420
   gctggaaaag gcctgtggcc cagaggcttc tccaaaggga gggtgacatg ctggcttttg 3480
   tgcccaagct caccagccct gcgccacctc actgcagtag tgcaccatct cactgcagta 3540
   gcacgccctc ctgggccgtc tggcctgtgg ctaatggagg tgacggcact cccatgtgct 3600
   gactcccccc atccctgcca cgctgtggcc ctgcctggct agtccctgcc tgaataaagt 3660
   aatgcctccg cttcaaaaaa aaaaaaaaaa aaaaaaaaaa aaaaaaaggc cacatgtgct 3720
   cgagctgcag 3730
<210> 14
   <211> 899
   <212> DNA
   <213> Homo sapiens
<220>
   <221> misc_feature
   <222> (1)...(899)
   <223> n = a, t, c or g
<220>
   <223> nbla-00185-f
<400> 14
   ggnngnnnnn nnngnnnnnt ttnnnngggc ccnnaatcct cgngnacgtg gccctactgg 60
   gcacaaatgt acaatactgt ttgggaatat gggaagacct gggatttaga atatgccaag 120
   acagatataa attgtggcac agacttgatg ttttatatag aaatggaccc accagcactg 180
   cctcctaaac caccaaaacc tactactgta gccaacaacg gtatgaataa caatatgtcc 240
   ttacaagatg ctgaatggta ctggggagat atctcgaggg aagaagtgaa tgaaaaactt 300
   cgagatacag cagacgggac ctttttggta cgagatgcgt ctactaaaat gcatggtgat 360
   tatactctta cactaaggaa agggggaaat aacaaattaa tcaaaatatt tcatcgagat 420
   gggaaatatg gcttctctga cccattaacc ttcagttctg tggttgaatt aataaaccac 480
   taccggaatg aatctctagc tcagtataat cccaaattgg atgtgaaatt actttatcca 540
   gtatccaaat accaacagga tcaagttgtc aaagaagata atattgaagc tgtagggaaa 600
   aaattacatg aatataacac tcagtttcaa gaaaaaagtc gagaatatga tagattatat 660
   gaagaatata cccgcacatc ccaggaaatc caaatgaaaa ggncaactat tgaagcatta 720
   atgaaaccat aaaaatattt gaagaacaat gccagaccca agagcggtac aacaaagaat 780
   cattgaaaag tttaacgtga aggcaatgag aaaagaattc aaaggattat cctaattatg 840
   ataantttaa ntcccnaatc cattgaaatt attgccattt naanaanatt ggganaaga  899
<210> 15
   <211> 887
   <212> DNA
   <213> Homo sapiens
<220>
   <221> misc_feature
   <222> (1)...(887)
   <223> n = a, t, c or g
<220>
   <223> nbla-00185-r1
<400> 15
   nggggttttg tnggctnttc ctcnnttgtn ctcgttttgg ttttngggcc tnggggcccn 60
   tnttttgttt aaaaatgctc gntntntttt tttttgtntn ttttntngtt ttgtggtccc 120
   cncttntntt gttntnttan tttttttntt gtcntnttnt ttttttttnt tttttttttt 180
   ttnttgtttn gtgngtttgg ggggtttttg ggggtttttt tttttntttt tntntttnta 240
   ttttgtnttt ttnttttgtt ttttttnttt ttcttttgtt ttttntttgt nttttttttt 300
   ttngttttnt nttttttttt ctgttttttc tttntgnngn gtttgttntt ntttttntct 360
   tttttntttn gttgtgtgtc tttttttttt gtttatttnt tttttttttt tgtttttttt 420
   ttnttttntt tttttttttt ttgattttgt tttttnttat tttgttgttt tttttttttt 480
   gttntttttt ttttctgtnn nttntttttt ttttctgntt ttctcttttt ttgtnttgtt 540
   ttgttntttt ttttgttttt tntttttttt nttttttttt ntttnntntc tttttttttn 600
   tntnttcttt tntttttttc tnttntnttg ttttttgttt tntttttgtt cttgttttgt 660
   ttttttnttt ttgttgtttt tttntnnttt ntctnntntt ttttnttctg tgttggtttn 720
   ttttttgttn gtnatttctt ntttgtntnt nttttnttnt ttnntttgtn tttncttttt 780
   tttttttatg tttntcggtn ttntttcttt tttctttttg tntgtttttt tnntgtcgtt 840
   cttttttttt ttttgtnttt cgtttttttc tttcgattnt ctttttt       887
<210> 16
   <211> 864
   <212> DNA
   <213> Homo sapiens
<220>
   <221> misc_feature
   <222> (1)...(864)
   <223> n = a, t, c or g
<220>
   <223> nbla-00226-f
<400> 16
   ngagttnnnn ngggcccnng ggntctcttn cacggggccc ctactgggga aagnaacttt 60
   acagggtcgc tagctagtag gagggcttta tggagcagaa aaacgacaaa gcgagaaaaa 120
   ttattttcca ctccagaaat taatgatcat gagctcgttt ttgatggact ctaactacat 180
   cgatccgaaa tttcctccat gcgaagaata ttcgcaaaat agctacatcc ctgaacacag 240
   tccggaatat tacggccgga ccagggaatc gggattccag catcaccacc aggagctgta 300
   cccaccaccg cctccgcgcc ctagctaccc tgagcgccag tatagctgca ccagtctcca 360
   gggacccggc aattcgcgag gccacgggcc ggcccaggcg ggccaccacc accccgagaa 420
   atcacagtcg ctctgcgagc cggcgcctct ctcaggcgcc tccgcctccc cgtccccagc 480
   cccgccagcc tgcagccaag ccagcccccg accatccctc caagcgccgc caagcaaagc 540
   aacccatagt ctacccatgg gatgaaaaaa attcacgtta gcacggtgaa ccccaattat 600
   aacggngggg aaaccaancg ctcgaggaca agctatancc ggcaacaagt cctggaatta 660
   gngaaagagt tcaattnaan cgctacctga cccgaaggag aanggtngan attgccaatc 720
   gctgtgcctc tctgaaaagn aagntnaaaa tccgggtcca aaancgttnc atgaatggan 780
   naagggcaac ggattcccaa aacaaannca ggtcagaaac cccggcnggg gngggcccaa 840
   nnccttttan cantgagttt cttn864
<210> 17
   <211> 872
   <212> DNA
   <213> Homo sapiens
<220>
   <221> misc_feature
   <222> (1)...(872)
   <223> n = a, t, c or g
<220>
   <223> nbla-00226-r1
<400> 17
   gantgnnnnt gttngngttt ttttggcccn nggntttttt ttcctcaggc ncccctgaaa 60
   ttcanccaag ccagnccatc acaccttgca atatataatt tttgcagctt tttttcttaa 120
   aaaataaata accccccaaa atggcctttt aaaaaaaaaa acccagcagg taccatgcta 180
   agacaacatc acatgcttaa aagggtcctg ggcgcagcgc cggccggggg tgctgacctg 240
   actttggtgt tggggagtcg gtggtccttc ttccatttca tgcgacggtt ttggaaccag 300
   attttgatct gcctctcaga gaggcacagc gagtgggcga tctcgatcct tctccttcgg 360
   gtcaggtagc ggttgtaatg aaactctttc tctaattcca ggacttgctg ccgggtatag 420
   gctgtcctcg agcgcttggg ttcccctccg ttataattgg ggttcaccgt gctaacgtga 480
   atttttttca tccatgggta gactatgggt tgcttgctgg cggcgctgga gggatggtcg 540
   ggggctggct ggctgcaagc tggcggggct ggggacgggg aagcggangc gcctgaaaaa 600
   agcgccggct cccaaaacga ctgtgaattt cccgggtggt ggtggccgcc tgggccggnc 660
   cgttgnctcn ccnaantgcc cgggtccctg gaaaactggt gcaactatac nggngctcaa 720
   ggtaaccaan ggccccgaag gcngttgtgg gtanaactcc cngnnggnat cctggaatcc 780
   caattcctgg tncggccgta anattccgga cngtnttaaa ggaatntnnc caattttgcg 840
   aanatcctcc cannggngga aattncggtt tn                              872
<210> 18
   <211> 928
   <212> DNA
   <213> Homo sapiens
<220>
<221> misc_feature
   <222> (1)...(928)
   <223> n = a, t, c or g
<220>
   <223> nbla-00258-f
<400> 18
   ggnngnnnnt ggnggccnnt tttnnnnggg cccnnngatc ctcgntaacg gtngccctac 60
   tggactcgca cgttaggagg ctcgggtcgt tgtggtgcgc tgtcttcccg cttgcgtcag 120
   ggacctgccc gactcagtgg aagcggggag aggtggtgag agatgacccc tgttgaggat 180
   ttcattggtg tctgccacag gccgccatgg catcagatga aggcaaactt tttgttggag 240
   ggctgagttt tgacaccaat gagcagtcgc tggagcaggt cttctcaaag tacggacaga 300
   tctctgaagt ggtggttgtg aaagacaggg agacccagag atctcgggga tttgggtttg 360
   tcacctttga gaacattgac gacgctaagg atgccatgat ggccatgaat gggaagtctg 420
   tagatggacg gcagatccga gtagaccagg caagcaagtc gtcagacaac cgatcccgtg 480
   ggtaccgtgg tggctctgcc gggggccggg gcttcttccg tgggggccga ngacggggcc 540
   gtgggttctc taaaaganga ngggaccgan gctatggggg gaancggttc gagtccaaga 600
   atgggggcta cggaggctcc anagactact atancaaccg gagtcaaaat ggtggctaca 660
   attaccggaa ctccggcggg tcctacanag acaattacga caattaccta cacacaacga 720
   gttaaaancc ntcctgctca anattgtccn tccaatggnt gtgtgttaaa aattgttgga 780
   acttccctga acnttaatgt ttnataaatg cacctccctt gtantcccaa ntttcnaanc 840
   aattcggttc natcttgtaa aaccaacctg ancgctttta ncccggaatg gccccttaca 900
   aaactttcnt ttaaggaatg cgtttttt                                928
<210> 19
   <211> 931
   <212> DNA
   <213> Homo sapiens
<220>
   <221> misc_feature
   <222> (1)...(931)
   <223> n = a, t, c or g
<220>
   <223> nbla-00258-r1
<400> 19
   ggnngnnnnt ggnggcnttt tttttgggcc tcagttcacg nttactgtng gcccgagctt 60
   cgtaaaataa agaaaactgc gtacaagaat tcctcagcca ctgcaaatac aacatcgatg 120
   gagtatgcgt acgtcttcca gtaaaaaagg acaatataaa taaacatctt caagaacctc 180
   tcagggacgt ctgcacgtct actgtggggc gcacagcaca agatggacgc aggggctggg 240
   gctccagggc agttgctgcc acacccgccg gggtcctacc acactacact caagaaacgt 300
   cacacggagt tggaaaaaag ccacagcttg ctcagcttcc gacaaagagc ggattcacaa 360
   gtccgttcga tntcagagaa gtgagtgggg caaccacttc atggggccaa ccccactaaa 420
   aaaaaaaagg caacgcaacc aaaaactaaa acaaaaacca aaaaccacac aacccccgat 480
   ttttttgaaa ctcatggctc gtggtcaaaa aagtaaatgc ttttcaaaat gttttgaaaa 540
   ccctcaaaaa aaaacagcac ttccttaaaa agaaaatcta gtaacgaggc atcccggcgt 600
   caaaagcggt caagctgggt ttgcaagatn agaaccgaaa tgactacgaa aagtgggaat 660
   acaaggaggt gcattactac acattaacgt tcaacgaaac tccacaatcc ttaaacacac 720
   aaccanttgg aaggacattc tnaacaagga aggtttttac ncnttgtgtg tancgtaact 780
   gtctaactgt ctctgtngga nccgcccaac tcccggtcac tgtnncaaca atctaatccn 840
   ggtggcaaaa taatccctgg agcctccgtn acccccaatc ctggantcca accggnttcc 900
   ccaaaacctc ggtccctcct cctctaaaaa a                          931
<210> 20
   <211> 880
   <212> DNA
   <213> Homo sapiens
<220>
   <221> misc_feature
   <222> (1)...(880)
   <223> n = a, t, c or g
<220>
   <223> nbla-00312-f
<400> 20
   gnnnnttnnn nnggccgnat cccgagcacg ttggcctact ggattagaaa ccacaatctg 60
   ccaaagcaac ctccacttgc cctagcaatg ggacaggcca cacaatgtct aggactgatg 120
   gctcgatcag gaattggatc ctgcgcattt gccacagtgg aagactacct acattagcta 180
   tgcatttcaa gagctcacac ttatattgtg gcatatagtc aacatggaag tagaccagct 240
   ctgctgattt gaaatttaga ttttttaaat tatgtactgg ggacaggttt ttgtcgcttt 300
   acattgcttc ctaatttaca gcatgatgca aatgattttc taacttaagt gttaggagaa 360
   attattttcc atctttaacc tcctaagtgt ctaagagtta aatattactg aatttcagac 420
   gttcaaattg atcatcacaa atcctttaaa acaattacct aaaagaaacc aaaaatcctg 480
   ccttccttgt gggggaaggg ggagagaggg gaanggaatg gaacaagttg tgtttgtgtt 540
   aacatgtggg tgatgtaaac ttcaaattgg gagatgttcc gacccccact cccatataag 600
   cattcaatca gtgtaacttg caaaatgcat aaacatcccg acagtttgaa tttatagtgt 660
   tgatggaaga aatcantttt aatgtgtact gtaaacttga aatactcang gagctgagtg 720
   atangttggt gcaacttaca atcccganct ggtaatccaa tantttgggt ttaacaatgg 780
   ccttttcaac nttggttccc ggttaactac agatgacttc cggtggnnac ncncaagttt 840
   aacnggtggn ttanacaatt ttncattcaa cggggnggtn                    880
<210> 21
   <211> 887
   <212> DNA
   <213> Homo sapiens
<220>
   <221> misc_feature
   <222> (1)...(887)
   <223> n = a, t, c or g
<220>
   <223> nbla-00312-r1
<400> 21
   ggnggctttt tttttnggct tggttaatta tatcacnaca tttnattgca atatagtact 60
   catttaagca cttaaaaatg gaaggtgtac aaagattaaa ttaagacacg gtaaattgac 120
   taaatatttg gtttttatat aaataaaggt cataaccaca ccgttgacat gtaatactgt 180
   tataatacaa cagttaaact tgtgagtcta caacagaagt catctgtagt taaacaggaa 240
   acaaagttga aaaagaccat gttaaaacaa aactactggg actaacaggt cgggattgta 300
   agtagcaaca aacatattca ctcagctcct gagtatttca agttttacag tacacattaa 360
   aaatgatttc ttccatcaac actataaatt caaactgtcg gatgtttatg cattttgcaa 420
   gttacactga ttgaatgctt atatgggagt gggggtcggg acatctccca atttgaagtt 480
   tacatcaccc acatgctaac acaaacacaa cttgttccat ttccttcccc tctctccccc 540
   ctcccccaca aagaaggnag gattttgggt tcctttaagt aattggttta aagggnttgt 600
   gatgatcaan ttgaacgtct gaaattcagt aatatttaac tcctaagcaa ctaaagaggg 660
   ttaagatngg aaataaattt ccccaacact aagttngnaa atcaattgca catgctgtaa 720
   ctanggaaca atgttaagcg acaaaaacct gtcccaannc ataatttaaa aatccaaatt 780
   tcaaatcaac aaaactnggc cacttccatg ttgactaaat gccacatana agtgtggctc 840
   ctgaaatgca aaacctaang tnggtagnct ccaccngggg naaatgn 887
<210> 22
   <211> 1740
   <212> DNA
   <213> Homo sapiens
<220>
   <221> misc_feature
   <222> (1)...(1740)
   <223> n = a, t, c or g
<220>
   <223> nbla-00315
<400> 22
   gaattcctcg agcactgttg gcctactggt tacaagatcg agaatggtta acagaacttt 60
   ttcaacaaag caaggtcaat atgcagaaaa tgctcccaga aattgatcag aataaggacc 120
   gcatgttgga gattttggaa ggaaagggac tgagtttctt attcccactc ctcaaattgg 180
   agaaggaact gttgaagcaa ataaagttgg atccatcccc tcaaaccata tataaatgga 240
   ttaaagataa catctctccc aaacttcatg tagataaagg atttgtgaac atcttaatga 300
   ctagcttctt acagtacatt tctagtgaag taaacccccc cagcgatgaa acagactcat 360
   cctctgctcc ttccaaagaa cagttagagc aggaaaaaca actactacta tctttcaagc 420
   cagtaatgca gaaatttctt catgatcacg ttgatctaca agtcagtgcc ctgtatgctc 480
   tccaggtgca ctgctataac agcaacttcc caaaaggcat gttactcgct tttttgtgca 540
   cttctatgac atggaaatta ttgaagaaga agctttcttg gcttggaaag aagatataac 600
   ccaagagttt ccgggaaaag gcaaggcttt gttccaggtg aatcagtggc taacctggtt 660
   agaaactgct gaagaagaag aatcagagga agaagctgac taaagaacca gccaaagcct 720
   taaattgtgc aaaacatact gttgctatga tgtaactgca tttgacctaa ccactgcgaa 780
   aattcattcc gctgtaatgt tttcacaata tttaaagcag aagcactttt tttcactgca 840
   ttctagttgt ggtttgtcca aactcatcaa tgtatcttat catgtctgga tccccgggta 900
   ccgagctcga attaattcct cttccgcttc ctcgctcact gactcgctgc gctcgtcgtt 960
   cggctgcggc gagcggtatc agctcactca aaggcggtaa tacggttatc cacagaatca 1020
   gggggtcagt taggatttcc ttctgcataa ggtttttttg tagtgtaatg tcttaatcat 1080
   agtctaccat caaatatttt aggagtatct ttaatgttta gatagtatat tagcagcatg 1140
   caataattac atcataagtt ctcaagcaga ggcagtctat tgcaaggacc ttctttgctg 1200
   ccagttatca taggctgttt taagttagaa aactgaatag caacactgaa tactgtagaa 1260
   atgcactttg ctcagtaata cttgagttgt tgcaatattt gattatccat ttggttgtta 1320
   cagaaaaatt cttaactgta attgatggtt gttgccgtaa tagtatattg cctgtatttc 1380
   tacctctagt aatgggcttt atgtgctaga ttttaatatc cttgagcctg ggcaagtgca 1440
   caagtctttt taaaagaaac atggtttact tgcacaaaac tgatcagttt tgagagatcg 1500
   ttaatgccct tgaagtggtt tttgtgggtg tgaaacaaat ggtgagaatt tgaattgctc 1560
   cctcctatta tagtattgaa attaagtcta cttaatttat caagtcatgt tcatgccctg 1620
   attttatata cttgtatcta tcaataaaca ttgtgatact tgaaaaaaaa aaaaaaaaaa 1680
   aaaaaaaaaa aaaaaaaaag gccacatgtg ctcgagctgc aggtcgcggc cgctagacta 1740
<210> 23
   <211> 843
   <212> DNA
   <213> Homo sapiens
<220>
   <221> misc_feature
   <222> (1)...(843)
   <223> n = a, t, c or g
<220>
   <223> nbla-00417-f
<400> 23
   gngnntttnn nngggccngg atctcgtngc acggnggccc tactgggatt tcaagcacag 60
   cacatctctc actcaggatg aaagcactct taccgaagtg aaatcctacc attgtaatga 120
   ctgtggggaa gactttagtc acattacaga ctttactgac catcagagga tccatactgc 180
   agagaacccc tatgattgtg agcaggcttt tagtcagcaa gctatttctc atcctggaga 240
   gaaaccctat caatgtaatg tatgtgggaa agctttcaaa aggagtacaa gtttcataga 300
   gcatcacaga attcatactg gagagaaacc ctatgaatgt aatgagtgtg gagaagcctt 360
   tagtcgacgc tcatcgctta ctcaacatga gagaacccac actggagaga aaccctatga 420
   atgtattgac tgtgggaaag cctttagtca aagttcatct ctcattcagc atgagagaac 480
   tcatactgga gagaagccct atgaatgtaa tgaatgtggg agagccttcc gaaaaaaaac 540
   caacctgcat gatcatcaag agaattcata ctggngaaaa accctaatcc tgtaagggat 600
   gtgggaaaaa cttcaaccga agttcaactc ttactaaaca ccaagagaat tcatactcga 660
   aataaactcc taggaacgtt gaaattangg antttgcana tgcnttanct aaaatgttcc 720
   ngatcanggt nagagggntc ttaanacctt ggaatgtnan gaattactgg ggggttaaac 780
   nttgngtnng ggnaaaantg caattnacaa attttttttt ttttacanna ngnccatccc 840
   nat 843
<210> 24
   <211> 836
   <212> DNA
   <213> Homo sapiens
<220>
   <221> misc_feature
   <222> (1)...(836)
   <223> n = a, t, c or g
<220>
   <223> nbLa-00417-r1
<400> 24
   gggntgnnnn nnntgtggct ttttttttgg cctttaaaga gatggngttt naccatgttg 60
   tccaaggctg ggtcttgaac tcctgggttc aagcagtcta tctgccttag ccacccaaag 120
   tgctgggatt acaggtgtga gacaccatac ctagccaagt taattttttt aatggtgaaa 180
   tcttttcttt gcacataaaa tgagccagtg catgttgctt ctctgagtac aagacaaaat 240
   ttatggcaat gggcaattag acttatactt ttctgcaaga aaattaacgg gaaaattctc 300
   ctcttagttt tctgttgttt tcccattgat ctgatactgt aggcttaaga aagtgctttt 360
   tcatgggcat gccataaaaa gtacaataag gggacttaat agttctgtga aactggcata 420
   tgttagctga aagtataatt gtaactggga aaaggggaaa aaagtcacta agtagttcaa 480
   ccatctacag tttctgttaa attgtggttt gtaaagcctc caagaagttg ctttaaataa 540
   tttgtgataa atttgcatac attttgctcc cacttatacc tttaagaant cncaaaagtg 600
   tccaanccat aaggggccan taaatgtttg nggtanccga tacatctaaa attatttaaa 660
   gcctctgatc caaaaataac ctttcacngg aaaggcaagg ggcccaaatc aagnaaccgc 720
   attttaacca ncttanccta aaggcggnnc aacnggngna acanttaaac cttggnttna 780
   ancctcaacg gtnaannaat ggttggcaat tntcaatcct ttaaggggnc tcccng     836
<210> 25
   <211> 844
   <212> DNA
   <213> Homo sapiens
<220>
   <221> misc_feature
   <222> (1)...(844)
   <223> n = a, t, c or g
<220>
   <223> nbla-00428-f
<400> 25
   gnnnntttnn nnnggccngg atcctcntnc acggggcccc tactgggcat tcnatttctt 60
   ttggnttgaa gctaaagaga tgtgtagctc acaggggcta ccttaccagt ataaagagct 120
   gataacagta ctcagaagaa tctgtgaaca aatacttgaa aatgggttca atgtagactg 180
   ccattatgtg tggtcttccc attaaatgtg aacattttaa tatgtatgca ttcaccttgc 240
   ctcttgcaca aatgtcaaaa aaaagatggt aatatctcaa agaaatgaac ttgtagatta 300
   ccaagcagtt tgctaaaaat tcaatctttg acccaagctg tagcattttt ttttcatgtg 360
   tggcatcttt ttcatgccac caacaaactt gttgtgtgtg tgcgtgtgtg tgtgtgtgtg 420
   tgtgtgtgtg tgtgtgtgtt ctgtacccac taggatttgt ttaggtgccc attgcatctt 480
   tttgtgctat ggagttgttt acattaagca tgaccgaacg agagacaata ctatttccca 540
   caggagtcca ttgggttcag ctttgaaaga ggaatagaat cgaggctcct ttgaccatca 600
   aaatgatgaa ctttactatg tggtanccaa tgccagaatg tnagagttgc aagtgatttt 660
   gtgctgctat cattaaaact tgtattccaa tcctgccaac ntaaggngat naagatatta 720
   anangnancc ctgatttaat ttccagtatt cagtagtaaa atttcctgtc cactggtgan 780
   caaancctgn tcacncaatt aacctgggca cacnaanaag ggtttatttg atggggtccn 840
   ttnc844
<210> 26
   <211> 842
   <212> DNA
   <213> Homo sapiens
<220>
   <221> misc_feature
   <222> (1)...(842)
   <223> n = a, t, c or g
<220>
   <223> nbla-00428-r1
<400> 26
   ggntgnnnnn ntgtggcttt tttttgcctt gtttgtttgt ttgtttntgt ttcttacctt 60
   ggggggtagg aggagttaaa ggaggagaaa ttttactatt ggggggggga aacgaacaca 120
   atcaaaataa aaccttatta gtgtgtccaa ggttaaatag agtgactcag gctttgattc 180
   acagtggaca ggaaaatttt actactgaat actggaaaat aaatcaagga tacctcttaa 240
   tatcttgatc tccttaagct ggcaagactg gaatacaagt tttaatgaat agcagcacaa 300
   aatcacttgc aactcttaca ttctggcatt gggtaccaca taagtaaagt tcatcatttt 360
   gatggtcaaa ggagcctcga ttctattcct ctttcaaagc tgaacccaat ggactcctgt 420
   gggaaatagt attgtctctc gttcggtcat gcttaatgta aacaactcca tagcacaaaa 480
   agatgcaatg ggcacctaaa caaatcctan tgggtacaga acacacacac acacacacac 540
   acacacacac acacacgcac acacacaaca agtttgttng gggcatgaaa aaagatgcca 600
   cacatgaaaa aaaaatgcta caacttgggt caaagattga atttttagca aactgcttgg 660
   aaaccacaag ntcattcctt gagatattac aacctttttt gcanttgtca aaaaggaaag 720
   gtaatgnntc aatattaaat gtccaattta tgggaanncc cccttanggg atccaaattg 780
   acccantttc aataattgtc aaaaatcctc cgaatacggt tacaaaccct ttaacnggga 840
   ag                                                           842
<210> 27
   <211> 879
   <212> DNA
   <213> Homo sapiens
<220>
   <221> misc_feature
   <222> (1)...(879)
   <223> n = a, t, c or g
<220>
   <223> nbla-00434-f
<400> 27
   gngnnnnnnn nnnngaggnn nttnnnnngg cccnggatcc tcgnncacgg tggccctact 60
   ggtatatact gggccagtaa aatgggcctc ccaattgctg tttcagcagg ttttaaacct 120
   tcaggaacac cagttaggaa aatagctcca gaaaatatag atatatttta tttttattaa 180
   aatggcagtc tacatcataa ttggcatttc tcaagactgt ctttaccaga atctgtgtga 240
   aataaggcaa tctagtctcc ttgaaaaaaa aatctcttgg atgtttagga aggaagactt 300
   ggccgtgatg tggtgtcctg gctttgtggt gtagtgctgt gtgtatggag ttagtgtaaa 360
   aacatggatt acaccaagtg gaagaaacgt cttcttgcca agctcattct tagaacttac 420
   acatctagaa cagcttccac tttggcagtg aggtcgtagc cttttangtg gaagaagtga 480
   gggtgcaacg tgtcagacac aacattcatg ttactcttac attgggatct gaaggtagtt 540
   cagacttcgt gggttttgtt tttaagcaaa acaatgtgaa aacatttaag tttgaaatgt 600
   tgcatttgaa gttatgatca tttaatatat tcatattacc aagactatta tactggaagt 660
   ggnttttgtg ttataaaggg ttaattttac ataaggcagt tacntaangt gatttttaac 720
   ccntaaaaaa gtggggatgt ntacatttgt taacaatgcc atgaaagcaa tttccttccc 780
   ccaanggaaa agtgaattct aacagaatat ccggctggnc ctgtaattta attaaataaa 840
   attttgggga acaannnnnn nnnnnnnnnn nnnnnnnna879
<210> 28
   <211> 866
   <212> DNA
   <213> Homo sapiens
<220>
   <221> misc_feature
   <222> (1)...(866)
   <223> n = a, t, c or g
<220>
   <223> nbla-00434-r1
<400> 28
   ggntgnnnnt gggggctttt tttgggcctn ntttttttaa ttggcctngn nntttttttt 60
   gnttctccaa aattttattt taatttaaat tacagggcca gccagatatt ctgataagaa 120
   attcactttt ccttaggaga aagaaaatgc tttcatggca ttgttaacaa atgtatacat 180
   ctccactttt ttaagggtta aaaatcacat taagtaactg ccttatgtaa aattaaacct 240
   ttataacaca aaaaccactt ccagtataat agncttggna atatgaatat attaaatgat 300
   cataacttca aatgcaacat ttcaaactta aatgttttca cattgttttg cttaaaaaca 360
   aaacccacga agtctgaact accttcagat tccaatgtaa gagtaacatg aatgttgtgt 420
   ctgacacgct gnaccctcac ttcttccacc taaaagggta cgacctcacn gncaaagtgg 480
   aactgttcna aatgggtaag ttccaaaaat gagcttggna agaanacgtt tcctccactt 540
   ggggnaatcc angtttttac actaactcca tacacacagc actacaccac aaagccaagg 600
   acaccacatc acgggcaagt cttccttccc aaacatccaa ggagattttt tttcaanggn 660
   gaccaaaatg ccctaattca cacagattcn gggaaaagac agtcctgana aatgccaaat 720
   tttgngtnaa ccggcaattt aanaaaataa atanacccaa antttccggn gccaatttcc 780
   caacnggggt tcccgaaggt taaaccngcn gnaaaancaa ttgganggcc attttcnggc 840
   aagtnnncca attgggcaan aangnc 866
<210> 29
   <211> 847
   <212> DNA
   <213> Homo sapiens
<220>
   <221> misc_feature
   <222> (1)...(847)
   <223> n = a, t, c or g
<220>
   <223> nbla-00460-f
<400> 29
   gngnntttnn nngggccngg atctcntgca cggggcncct actggttctt ccagtcacct 60
   cggcccggga tcgggaagtg tcaagcgggc gctcccccat ctccgccgct attaccactg 120
   aacccggacc ccctacccag gtccagggcc agccgccatg acgaacgtgt actccttgga 180
   tgggattctg gtgtttggtt tgctctttgt ttgcacctgt gcctacttca agaaagtacc 240
   tcgtctcaaa acctggctgc tatcagagaa gaagggtgtt tggggtgtgt tttacaaagc 300
   cgctgtgatt ggaaccaggc tgcatgctgc tgtggcaatt gcttgtgttg taatggcctt 360
   ttacgtcctg tttataaaat gaattccaaa gcacccaagt catcaactgc caaccaaggg 420
   gacggggatg aagaacctgt tggagacctg aacccagtgt aggagagttc agctgaaatc 480
   atcggtcccc aggatgacac cacagcatct gcccctgcta tatgtgggga aaactcatgg 540
   tcacgaacat tatttatgct tcaggggact acagaaagcc agcttccttt gatctatgtg 600
   taaatcagtc cttggcagag tgcatataat gtccggataa attcatctag tataagaact 660
   tgtgatgtta gattgaagtt ttgtcatcct ataaaagaca acaaacttan tttctgttta 720
   agtccgaatg ttaatggcaa nttttanttg attacttaat tttctaacca aattttaant 780
   ttcctcanan tgcaattgcc cttaattgtc nctggnaatt ccaattactt taggncatga 840
   gaaattn 847
<210> 30
   <211> 862
   <212> DNA
   <213> Homo sapiens
<220>
   <221> misc_feature
   <222> (1)...(862)
   <223> n = a, t, c or g
<220>
   <223> nbla-00460-r1
<400> 30
   ggntgnnnnn ntggggcctt tttttggccc tttagggaan aaaagtncat tttcancgga 60
   tttgtgcaga caatggtgaa taaaaaattg tatttcaact ataaaaagga gctgacttca 120
   ttccaccctg gcctacaggg tttatggtga tgccagtttg aatctgaggc aatactgcta 180
   tccccattcc tccacctgta aaagattccc ccagaggaat aacattctat aacttttagt 240
   caaaaatatg taagacacat caaacaaagt aatgagttaa acaagtttct gcttatctgc 300
   ttagggaaag tcaagaaatg aaacaaatgt atatttgttt ctggctgtaa attaccttta 360
   ccttgtgcac tttttggtac attctggtat gaaaacctct caaaatgtaa caacacaaga 420
   gtttgggtca agacgaccca cccaggaggc tgtaaaaact ggtttgaact aaaactgtgg 480
   aatgggacta gtttaaaata tgaagcaact ctaaacacca agcttanaga catttgccct 540
   attaagaaaa caaagatcat taaagctaca aaataacaag tgcaaacatg ctgaacctgt 600
   ttccaaggag tgacattccc ntctgccaac aagtcccaaa ctcacaccca caangngtaa 660
   ctctccttcc tggtccacta aanttccttt ctctcanctc aaangncctc aaaaatgaca 720
   atgggaaacg tatgaattgt tgaaanttan cctgtggncc aantcccgga agagataaca 780
   accacaactc ctganatgat tanacatgca ntgtttactt gatgactttc cgnanttcta 840
   aaaaccccaa agcaataaaa tg                                     862
<210> 31
   <211> 859
   <212> DNA
   <213> Homo sapiens
<220>
   <221> misc_feature
   <222> (1)...(859)
   <223> n = a, t, c or g
<220>
   <223> nbla-00478-f
<400> 31
   gngnttttnn ngggcccngg atcctcntgc acggggcncc tactgggacg ggaatttttt 60
   gggagctggg aaaggaattc atcttagccc caaatgacca ttttaataat ttgcctgtga 120
   acatcagtct aagtgacgtc caagtaccaa cgaacatgta caacaaagac cctgcaattg 180
   tcaatggggt tttttggtct gaatctctaa acaaagtttt tgtagataac tttgaccgtg 240
   acccatctct catatggcag tactttggaa gtgcaaaggg cttttttagg cagtatccgg 300
   ggattaaatg ggaaccagat gagaatggag tcattgcctt cgactgcagg aaccgaaaat 360
   ggtacatcca ggcagcaact tctccgaaag acgtggtcat tttagttgat gtcagtggca 420
   gcatgaaagg actccgtctg actatcgcga agcaaacagt ctcatccatt ttggatacac 480
   ttggggatga tgacttcttc aacataattg cttataatga ggagcttcac tatgtggaac 540
   cttgcctgaa tggaactttg gtgcaagccg acaggacaaa caaagagcac ttcagggagc 600
   atctggacaa acttttcgcc aaaggaattg gatgttggat atanctctga atgaggcctt 660
   caacattctg agtgatttca accacacggg acaaggaagt atctgcagtc anggcatcat 720
   gctcataact gatggggcgg tggncaccta tgatacaatc tttgcaaaat acaaattggg 780
   cagatngaaa aggtcgcatc ttcacatacc tcantgggcg aaaagctgcc tttgcanaca 840
   atccaaagtg gntggccng859
<210> 32
   <211> 863
   <212> DNA
   <213> Homo sapiens
<220>
   <221> misc_feature
   <222> (1)...(863)
   <223> n = a, t, c or g
<220>
   <223> nbla-00478-r1
<400> 32
   ggtgnnttgt nggcgtcttt tctgggttct gctggtntta gtggcctggg cccttttttt 60
   tnaaggccnt ttttttttnt ttttttttgt tngtttttgt tttgggtcnc ntttntgttt 120
   gtntttnntn nttgctttgt tttttttttt ttttttttgn tttttntttt ttngngtttt 180
   ttgtngnttn tttngtgttt ttcttntttt ttcngttttt gttttttttn tttttttttt 240
   tntctntntt tntttttntt tttgtntttt gtttttttnt nnttgttngt nttgttgntn 300
   ntnttntnct nttnnttttt gtgttttgtt tttggttntn ttngtnttnt ttttttcttt 360
   ngtcgttttn ntttctnttt tnttnntttt tttntnttnn tctgttttgg gnngtggtgn 420
   tnttctnttt tttnnnnttt tttnntttnt tgttntggtt ntttcttttn ntttttctcn 480
   tgntttnttt tnttntttct tgttntgttt ttgtttnntt tgtttttttt gtnttttttn 540
   tttttttttt tttnnctttt tttnntnntc ctgttttttt gngtttgttg tgttttcttt 600
   tnttnttncg tttgtgtnct cttcngtctt tntntttntn ttttttttnt nttttttttn 660
   ttcctttnct nntntnnggt tttgtgtgtg tntntgtttt tnttttgttt tttttttttt 720
   nnnttgtntt tttttngttt nttttnnttt ttttnttntn tttnnttttg tttcntttgt 780
   tttgtnttct tnttttttnt tngtttnntn tttctttttg ttccnttgtt ttgtttgntt 840
   gtttttnttg ttntttcttt ttg                                863
<210> 33
   <211> 831
   <212> DNA
   <213> Homo sapiens
<220>
   <221> misc_feature
   <222> (1)...(831)
   <223> n = a, t, c or g
<220>
   <223> nbla-00483-f
<400> 33
   ggatctcgng cactgtggcg cctactgggt ttagtataag atgtgccatt ttgtcctctg 60
   tatgtctgaa tgaagctata acatttgcct ttttattgca ggttttcctt tggaatatgg 120
   ataaatacac catgatacgg aaactagaag gacatcacca tgatgtggta gcttgtgact 180
   tttctcctga tggagcatta ctggctactg catcttatga tactcgagta tatatctggg 240
   atccacataa tggagacatt ctgatggaat ttgggcacct gtttccccca cctactccaa 300
   tatttgctgg aggagcaaat gaccggtggg tacgatctgt atcttttagc catgatggac 360
   tgcatgttgc aagccttgct gatgataaaa tggtgaggtt ctggagaatt gatgaggatt 420
   atccagtgca agttgcacct ttgagcaatg gtctttgctg tgccttctct actgatggca 480
   gtgttttagc tgctgggaca catgacggaa gtgtgtattt ttgggccact ccacggcagg 540
   tccctaacct gcaacattta tgtcgcatgt caatccgaag agtgatgccc acccaagaag 600
   ttcangagct gccgattcct tccaagcttt tggagtttct cccgtatcgt anttanaaga 660
   ttctgccttc cctagtaata gggactgaca gaatacactt aacacaaacc tcaaagcttt 720
   actgacttca attaaccgtt tttaaagacn ttnaagatta attaatttga aaggtcctgg 780
   ncngcatttt gatcaattnn cctttaaaat atanttatng ncatanantt c          831
<210> 34
   <211> 874
   <212> DNA
   <213> Homo sapiens
<220>
   <221> misc_feature
   <222> (1)...(874)
   <223> n = a, t, c or g
<220>
   <223> nbla-00483-r1
<400> 34
   gggtnnnnnn nnntggtggc cttttttttt tnttttttgt atttccagta gagacagggt 60
   ttcatcatgt ttgccaggct agtctcaaac tgccgacctc aggtgatttg cccgcctcac 120
   ccaattcaac tttcgtaagt cagtatttac catctaactc agtgtcccag aatttaaaat 180
   ttccttgcac tttacagcaa agatacatat tgtggctcta ctgaagcaat atatacatgt 240
   cagaactaaa aatcagaaaa gcaaaagggt ccattcaaca tatagcagct tatatctaaa 300
   tatgtacagg tatgtatgtt ttcacagtta gatctttaaa aaaatttata tttgatatgt 360
   tcaagaaata cttctattgt ctataaataa tattttaaaa gctcaactga tcaaaatgca 420
   gtacaagaac atatcaaatt aaataaatct tctacgtctt taaaaacaga taattgaagt 480
   cagtaaagct tgaggtttgt gttaagtgta ttctgtcagt ccctactact anggaaggca 540
   gaatcttcta aatacgatac gggagaaact ccaaaagctt ggaaggatcg gcagctcctg 600
   aacttcctgg gtgggcatca ctcctcggat tgacatgcga cataaatgtt gcangctaag 660
   ggacctgccg tggagtgggc caaaaataca cacttccgtc atgtgtccca gcagctaaaa 720
   cactgccatc aantanagaa ngnacagcaa agaccantgc tcaaaggngc aaccttgcac 780
   tggntaatcc tcatcaattc tccaaaacct caccaatttn acaatcagca aggnttgcan 840
   aatnagtcca caanggntaa aagntacann tngt                            874
<210> 35
   <211> 879
   <212> DNA
   <213> Homo sapiens
<220>
   <221> misc_feature
   <222> (1)...(879)
   <223> n = a, t, c or g
<220>
   <223> nbla-00491-f
<400> 35
   gnnnnttnnn nnggccnngg atcncgagca cggttggcct actgggtatg aacgaacctc 60
   cttcagtcca actatccaga cgttcattct ctctgccatt tggcacgggg tatacccagg 120
   atattatcta acgtttctaa caggggtgtt aatgacatta gcagcaagag ctatgagaaa 180
   taactttaga cattatttca ttgaaccttc ccaactgaaa ttattttatg atgttataac 240
   atggatagta actcaagtag caataagtta cacagttgtg ccatttgtgc ttctttctat 300
   aaaaccatca ctcacgtttt acagctcctg gtattattgc ctgcacattc ttggtatctt 360
   agtattattg tgttgccagt gaaaaaaact caaagaagaa agaatacaca tgaaaacatt 420
   cagctctcac aatccaaaaa gtttgatgaa ggagaaaatt ctttgggaca gaacagtttt 480
   tctacaacaa acaatgtttg caatcagaat caagaaatag cctcgagaca ttcatcacta 540
   aagcagtgat cgggaaggcc tganggctgt tttttttttt tgatgttaac aagaaaccta 600
   tcttancanc ttttcaaggg gtttgagttt gttgggaaag caattaactg gggggaaatg 660
   gncagttatn gataaggaat tcctgtcacc agattgggaa tggggtgaaa caagccctcc 720
   aaggcatgtc cccgtggggc acgccntatg ttagataatt ccatatttca ntngggantc 780
   ccaacctcag nacttgtccg taggggcana acncgtgccc tgntgccgaa nggnnggttg 840
   cgttncccaa gggactgaan aggnggnaaa naaacccgg                  879
<210> 36
   <211> 892
   <212> DNA
   <213> Homo sapiens
<220>
   <221> misc_feature
   <222> (1)...(892)
   <223> n = a, t, c or g
<220>
   <223> nbla-00491-r1
<400> 36
   ggntgnnnnn ntggggcttt tttttnggct tttgtgagac ggagtcttcc tctgtcaccc 60
   gggctggagt gcagtggcac aattttagct cactgcaacc tccacctccc aggttcaagc 120
   aattctcctg cctcagcctc ccaagtagct gggattacag gtgtgcgcca ccatgcccgt 180
   ctaatttttg tgttttcagt agagacgggg attcaccacg ttggccaggc tggtctccaa 240
   ctcctgacct caagtgatcc gcccacctcg gcctcccaaa gtgctaggat tacaggtgtg 300
   agccaccatg ctcagccaac ataatttcaa tgcatgggaa aacagaggaa atttatttag 360
   ttatcaatcc tttcctgggt agggttagcc ttatcagccg gaaaacactt acttttagat 420
   aaataaaaat agattaggat atttctaaac ttcaaaaaag acacatgccc atttttgctg 480
   agacctcaaa agctgcaagt gcatttgctt ttcctttcct ccttaaaatg aagtatatat 540
   agcataagcc anatggttgc cttgtcttaa cttcatttgc aaatttatta agtgactcca 600
   acccccgaaa aaaagagaga gagaacccat cacaagaaaa ttgcattcat taaaaattta 660
   angnaaaaag taaaaagtaa gttccataca tangtcaaac aaaggngaan tcaaaangnt 720
   ttgcccctcc cctcctanaa gggaaggagg taaatccngg ggctgaggca ctccaatggc 780
   ctggatcaaa tcccatcctc ggcttcannt cctggtggca acaanagtcc tccgttggga 840
   ttccaaggaa atgcttataa tccaaggnaa gattagttgt gggaaaaaan gg       892

<210> 37
   <211> 885
   <212> DNA
   <213> Homo sapiens
<220>
   <221> misc_feature
   <222> (1)...(885)
   <223> n = a, t, c or g
<220>
   <223> nbla-00494-f
<400> 37
   gngnntnnnn ngggccngga tctcgagcac tgtggcccta ctggagagtg tggagcgtgg 60
   agcgccggga ctgtgcacgc ttgaccggaa gcccagacca gtgcggtcct agccagagag 120
   aaaggacatt tgccaacaat gagacacgaa gcgcccatgc agatggcctc tgcccaagat 180
   gccaggtacg gccagaaaga ctcctctgat cagaactttg actacatgtt caaattactc 240
   atcatcggca atagcagtgt ggggaaaaca tcttttctat tccgttatgc agatgactcc 300
   tttacatctg cattcgtcag cacagttggg atcgatttca aagtaaaaac tgtattcaaa 360
   aatgaaaaga gaatcaagct tcagatttgg gacacagcag gccaggaaag atacaggact 420
   atcaccacag cctattatcg tggagccatg ggctttattt taatgtatga cattacaaat 480
   gaagaatcct tcaatgcagt acaagattgg tcaactcaaa tcaaaacata ctcttgggac 540
   aatgcccaag ttattctggt tgggaacaag tgtgacatgg aagacgagcg ggtcatctca 600
   actgagcgan gtcaacattt aggagaacag cttgggtttg agttttttga aacaagtgcc 660
   aaggncaaca ttaatgtcaa gcaagacatt ttgagcgccn tgtgggtatc atctgcgaca 720
   aaatgtcaga naatttggng actgattctg ccatcactgc tgcaaagcaa naacacgaag 780
   actcaaggga actcctcctc aacgnaaccc aactgtgcct gcnaatgtcc ccgtgcanan 840
   aggnaactcc agggggtccn ggttccaana aacanaattt ttaat           885
<210> 38
   <211> 890
   <212> DNA
   <213> Homo sapiens
<220>
   <221> misc_feature
   <222> (1)...(890)
   <223> n = a, t, c or g
<220>
   <223> nbla-00494-r1
<400> 38
   ggnngnnnnn ntgtgggctt tttttggcct ttttttttat agtctcntag atatttattg 60
   taatcttcaa gaaataaaac aaactcaaag aacaaaaacc actttcttta tagaattcac 120
   atacaaactg aatacctaat agagcacata ccatttttac acaattaatt tcaataaaga 180
   taattaaaac ttcacaatgt gaatacactt catagcttta aacccacaca agagctcctg 240
   tggacacatg aagagtcaca atatttgcta tgcagcagta gttttcaact aaatgtcggt 300
   cactatatga aagcattaag ctgttttcaa tattttctta agaagagttc cacagtgcag 360
   tcactgagaa ggggccaaat gtgcctggtt ccaaaggaag atcttcatct gtgcatttcc 420
   aatgcccttt atgggaaaca tggagctaca agaaaaatct ctgggtacac atctgccaag 480
   tggcattagg tcggncaatc tctcatgggt tggtgcactg gcaaatgtct aaatcaactg 540
   ccgtaatatg gcattaaagc aatttgcata atggggctga gtcctgagct gctcaacaca 600
   agaggaatat gataatgcaa caagcttgtc ctcctcacgg ngactaaaag gtagggagct 660
   aaactggggt aanttgaatg ggttcaaagg ggcaatttgg ctgtcctaag aaaacaatga 720
   tctanaactg ataacnttgn cncncctcca ntcaaaggga acataactgt ggccttccca 780
   cttaatttcc tggaaaaact cctcactact aaatcnttcc gggantaagt ttggcccctc 840
   aaaggggncc tctctgaatc ctcaanttct aaagggcccc aatcccggtg            890
<210> 39
   <211> 862
   <212> DNA
   <213> Homo sapiens
<220>
   <221> misc_feature
   <222> (1)...(862)
   <223> n = a, t, c or g
<220>
   <223> nbla-00499-f
<400> 39
   gnngnttnnn nnggccnngg atcctcgagc actgtggncc tactgggttt aacttgcacc 60
   aagcaagaaa atataaatac agttaactgc attaagataa tcacgttaaa attgttacta 120
   tgcagcacag aacttcattc ttatagtatt cttgggttca acctttgaat caattttacc 180
   actgatttaa taaatgactc aaagacatct gtaagtcatg ctgctgtgtt ttgaaagtct 240
   ttaactaaat taagattgca gaatgatagt gattattcaa ttagatttta agtaaggatt 300
   gtgatattag aggctggaaa tccttatttt ttaaaaaatc agataggcat aaatagttaa 360
   atcactttca ttctccccaa acctgtagtt acagaaaaag ttttatgcta gaggtgggat 420
   gccaagtttt cactatccat gaagcagcgc tgcatgtcac taggtaacac agattcatcc 480
   agatggtgtt tacatttgat ttaattggga tcttattgac atcaggtata cttgggaaga 540
   catttccttt atcctcagtg tatgaattta aagctatttt tgtaaatatt ctaatcancg 600
   ataattctac ctangttctc aaccaactta accagtttgt tttcanagcc tgtantctaa 660
   tgggaaccaa tttatcagtg cgccttaatg atgtggattt tgcanacatc aaacattacc 720
   acannttcac aagtgcacct aanttcacca ttaacttaat cccaagtcaa tttttcccgg 780
   ncacncaanc anngntntnt tnnnnacttt tgaaaagccg gaanactttg ctcaaaatgg 840
   gaaggttaaa tnaggtnaaa nc                                862
<210> 40
   <211> 888
   <212> DNA
   <213> Homo sapiens
<220>
   <221> misc_feature
   <222> (1)...(888)
   <223> n = a, t, c or g
<220>
   <223> nbla-00499-r1
<400> 40
   ggnngnnnnn ntgtgggctt ttttttggcc tcctttttaa catgtatntc tcanaataag 60
   attcaccatg aacattattt tagatatttt tacatcttct ttagctaacc aagtgaaaaa 120
   tccaatatac acaaataggt actttcatca gtacataaaa cagcaatagg tagcaaggtt 180
   agaaatcaga atacaagttc aattgaataa atacaatact agtacagaga cacccatttt 240
   taacccacaa tgaaaaatct gagccatgaa taggttttcc attttgaatc taacttctag 300
   atgatctgta ttttgacctt aaaggggcca aaatatttaa tatgggtgaa gctcatgttc 360
   cattacccgt aaaggtctaa tgcaacagaa aaatggtcat tttacattgg ttagaatttt 420
   taactaggtc tttcatatta ttttgtttaa gcacatgaca gacttataca catattttaa 480
   aaaatcaaac atgggatttc tatatacatc aaacctttaa caatgtctgt ctctgaattg 540
   ctattanagt gttcaaaatt atacaaacaa acatccagta aacagcattt tattttatga 600
   cactctgtgt taatacaata aatatacaaa acttccaacc cactgaaaca tgcaacatta 660
   gagggacaca tgggagggca tttctgtnga accatgacag tcaccatgat ggcagcgcaa 720
   tataagttac tgacaaatat tcaaacatct ctagataaat aatccaagtc ccaaattnca 780
   aatcactgag taatgacttt ccgnggtcaa acnttagttt ggaataaaat tgcaataatt 840
   aatattccaa aatggtaatg gcacggtgac tgcngttgaa nccatttt          888
<210> 41
   <211> 923
   <212> DNA
   <213> Homo sapiens
<220>
   <221> misc_feature
   <222> (1)...(923)
   <223> n = a, t, c or g
<220>
   <223> nbla-00536-f
<400> 41
   gngntgntnn ntggaggttc annggcccag gatctcttaa acgggcccta ntggatttna 60
   acccaagcat tctgattcct gggatgagcc attagagctg ttcagaagtg gaatgggctg 120
   tcttttagga cagtgctcct ctcttctttc cgggtcgctg ccagcaagta cggagagagg 180
   agctatgact ttaaggtctt agantttgtg gaatgcagtt actccccatt tggccaaaat 240
   ctcagaattt gtgtcgtgtc tctaaaccag ataaatggag agtactaaaa gcattgttgc 300
   ttctgctctg aaattgttat aggattattt ttattgcatc caagtcttaa acactgnnat 360
   ggctggggat gggttctttc tacgaatatg tangtcctga gcatngncgn gtnncanaca 420
   gccctgtgat cagnggagcn ttnncngngt gagatatgtc catgtacant gnnngggcac 480
   cagacgtnca tngagnattn tcnagtngct cctgtgnacn ncaatnnant cactcaangn 540
   tactcctgan ngnagtctnt taccttgant ttacnngagt atanggntgg ttgggaaaat 600
   ngcttncaaa nnnnngggca tngtnttnna tggaatgcag atgatgtttg naacatgtgc 660
   tnatgnaggg aangnnntna cacnctcaat gttggnagna ttgntntcta ntnggtcnnt 720
   ntcannnttg ngngagtcct ntgacngcnn gtncnatncg tcanannatg nnnngnccat 780
   cantttnnng nagncancng tgnntnnnnt aanttngagg tangnnntgc tanncnatnn 840
   ctgtanacat gnnggcgatc gnnnngntga cttcntcaca nttacnncat nnttttcnnn 900
   gcgttannnn atnngcccca gtg                                  923
<210> 42
   <211> 877
   <212> DNA
   <213> Homo sapiens
<220>
   <221> misc_feature
   <222> (1)...(877)
   <223> n = a, t, c or g
<220>
   <223> nbla-00536-r1
<400> 42
   gngtggnttn gtgggncttt tcnnagcgcc ttgctttgac tanacatcgc acttggccng 60
   taataggaaa tcanatcatc cagaatttaa aaagtgactg tgggggcttg ttgtcagctg 120
   tcgggggcag gacttatgtg cagagctagg tccgtggctc tggtcaaggc actgccttgg 180
   gtcactctgt gctggcccag gcnngctcca tgcctggctg ctggataanc cagttctctt 240
   ctgaccccca aagcaatcca atggtggggc aaactcagga tctctctcan cccanagatc 300
   tgactgatga ggcatangtt tttanctatt tgtaanaaaa tgaaagtatc tcatactacc 360
   attgacacag ccaggtggga nggggtcact ggngaaacgc naactgnnnc tgcccatggg 420
   atggaacctc gncanggttt angccctttg cagtgggaan ganctggtcc ctcctcntcc 480
   tggtgtanaa cctgagattc antctttcaa gtgggaagaa gcactgganc atggnacant 540
   gtcccaangg agagttntgt ttcccctttt cntctttttc acccnntana accctgtctt 600
   ctcaccantc acaatgtncn gtgnaccctn aattttcant ngncatggnn cannnaaccc 660
   tgtcctatat ttgaanctnn aggnanattc ctgtnnacca gngnatnttg gtgattttgc 720
   ccctaggaan ttttcagang gncattttnn tgggtangnc ttngcnaanc aanccggntt 780
   ttntcctggg cttttcggca antaacnttt ngntccnnac ctttntgatn ttatncnatt 840
   cngtngtcac anactacctn nnggtcttna tcagctt                     877
<210> 43
   <211> 2105
   <212> DNA
   <213> Homo sapiens
<220>
   <221> misc_feature
   <222> (1)...(2105)
   <223> n = a, t, c or g
<220>
   <223> nbla-00538
<400> 43
   tggcctactg ctagcaaaac ttgtttagct tagcaaaaac aaacacacaa aaaaactgag 60
   aactctgctg tttcagatat gccataacat acatctgaaa cacatgtgta acaatcaaaa 120
   tggtgggctc tagaatggtt ttggagctcg agatcttcat gggttagact tgctggtcag 180
   acccaggagc acctgtggct cacaccttct gttcccctcc tggcctgtgc agaatgtaaa 240
   cagcagactc atactcaatg ggcactacag gccttatcag acgttttata caagcctgga 300
   ttgcttagta ggggaataag gcattctctg agggggcttt ccacttagat tgagaatttt 360
   atttgaaaag aatctggttt aaatggcatt gtggtccgag gtagctgctc tccccactga 420
   gagctgagcc gaaatataag aataatatat ttgtgcttcg agttggtgtt tctttcagtg 480
   taatgcatgc agtggtcaca acccagttac tcataatatt tggattgtat ttgttcgtag 540
   atatgcccag aagactagag aattagtgtt atataccata tagaacttac tgtcagtcaa 600
   ctataaacag gcccaattaa aaactgttcc attactacgc aaacacatat tagaggcctt 660
   tgctgatgac acattagctg gatcttagcc accccagaaa gggtttgatt tgaagctgat 720
   tgttgccaga tatgcatatt ggaatcccat ctacccatag ttcctctgaa ggtgattttg 780
   taatttgcaa aagggtatag gaaaatatac ctaaaagcga atttgtggct gagaggataa 840
   acagaagctg tttgctcatg ttctgtgccc cacacccacc aatacctaaa tctgttaagg 900
   aagacagaaa atgttttctt tgtgctcatt gagtagttcc agacagaaga agaatatact 960
   ctttaaaatg tatttacctg ttagctggaa gtacccagaa ttatcagaaa cgaatgcaaa 1020
   aaaaaaaaaa aaaaaaaaaa gcttacacag cttcttagca attttttttt ttttttgccg 1080
   aaacaataaa ttgcctttag cagcagttta aaatcctatc gtgaacaacc tatattttcg 1140
   ccattttaca atggagagtt gtgacaagta caggttatca agtttgcact taactatgcc 1200
   aaaaaaagtt tgaagcgctc tattctcaga catgctgtat tattacttct cattcaagat 1260
   tgaaaaatat aaaggtatcc aaactctgtc ttaatgtaaa tgtaactatt tttccttcaa 1320
   gtgttgacta gggagtcggt ttctctctta aagacactca ctgtacaact gaaagcagct 1380
   gtcatatttc tggcaaaatg tgtttacgta tctgacaagt tgtacatttg tgtatgaact 1440
   gacataaaat gtgaaagcct gtaagtgtac atgtagtggt gtggtgttct gtctagagga 1500
   tacaactgaa tgtttttaat ttgctgactt acagacacag gctgtttaca aaatgctagc 1560
   tggaaagtct gtaatgttca tgtcataact tttagttaat tgccattgag cacctgttct 1620
   gaggaggtga gatgtggact tgtgcttata aactggagag tttagtcata atccctcctg 1680
   gctttgtgtg aatagcttgc tcactttgct ggcctttgaa atgtgttctc cgtgataagc 1740
   tatccatgtg tttgtgataa gagtgcttgt caaccatgac catctttgag ccttcctagt 1800
   cctccacctg gcacagtatt tgaaatggca aaggatgtgc ttcatcctct aacaaacagt 1860
   gtacactccc agagctgata ttctggattg tgactgtgca catttcctct agttcatgtc 1920
   tgtagtccct atagaatgat ctgtaataaa atagtatact ggactgtgca tcaaagggat 1980
   gtaaaattac agtattccaa aggttgaagt tctgctgttt tgttataatg cctgatacac 2040
   atcttgaata aagtcttaac atttttcttt taaaaaaaaa aaaaaaaaaa ggccacatgt 2100
   gctcg 2105
<210> 44
   <211> 863
   <212> DNA
   <213> Homo sapiens
<220>
   <221> misc_feature
   <222> (1)...(863)
   <223> n = a, t, c or g
<220>
   <223> nbla-00543-f
<400> 44
   gnnnnnnnnn nggnnnngga tctcgagcac tgtggcgcct actgggacct ttcggcgacc 60
   tcagatagac ccagctgttg aaggatttat tcgagacaaa tatgagaaga agaaatacat 120
   ggaccgaagt ctggacatca atgcctttag gaaagaaaaa gatgacaagt ggaaaagagg 180
   gagcgaacca gttccagaaa aaaaattgga acctgttgtt tttgagaagg tgaaaatgcc 240
   acagaaaaaa gaagacccac agctacctcg gaaaagctcc ccgaaatcca cagcgcctgt 300
   catggatttg ttgggccttg atgctcctgt ggcctgctcc attgcaaata gtaagaccag 360
   caatacccta gagaaggatt tagatctgtt ggcctctgtt ccatcccctt tttcttcggg 420
   ttccagaaag gttgtaggtt ccatgccaac tgcagggagt gccggctctg ttcctgaaaa 480
   tctgaacctg tttccggagc cagggagcaa atcagaagaa ataggcaaga aacagctctc 540
   taaagactcc attctttcac tgtatggatt ccaaacgcct caaatgccta ctcaagcaat 600
   gttcatggct cccgctcaga tggatatccc acaagcctac cccagcttcc ccggggttac 660
   acctcctaac agcataatgg ggagcatgat gcctccacca ntaggcatgg ttgctcaacc 720
   aaggaacttc tggcatgcag gcatcaatga tgggtgtgcc gaatggaatg atgaccancc 780
   ancaagctgg tacatggcaa gcatggaact atgccccaaa ctgtgtatgg ggccaaccaa 840
   ctcaacaact gcaatggaac ttn                                        863
<210> 45
   <211> 874
   <212> DNA
   <213> Homo sapiens
<220>
   <221> misc_feature
   <222> (1)...(874)
   <223> n = a, t, c or g
<220>
   <223> nbla-00543-r1
<400> 45
   gngntgnnnn nntgtgggct ttttttttgg cnnntttttt ttttttccct gcaatattta 60
   ttaaggaatt tacacataca caggagaaag gcagccagga actgtggttc ccacacatga 120
   aatcttttaa gcaaagtttt cttgtctgaa ttttcaagtg gggtgaacaa tgactgagag 180
   gaaagctgtc ccggccctct gcctcgtaca cctgggaacg gtggggaaac agagcaccct 240
   ggatacacag gcatgaaaga gtgatcagca gaccgggaga agggaaggga gaaagggagt 300
   tatcaatgac atggcgtttt ttaaaccata agaaaaacac aacagtttta ggctgctgat 360
   aaattaattc ctctctgttg taaacctaaa actaaacaaa aacaaaaata cccagagcag 420
   atggggaaag ggtgggaggc ggaacacaca ctcatacaca cacacacact cacgcacaca 480
   cacaagcagt acacgttaag ctctgcattc ccactatagg ggttggtcct ccagccacgg 540
   agccagattc tactgaaggc aagggggaag cnggaaggga tgtgtgtcng tgtgtgggat 600
   nttcanaaag tccttggggc taataaaggn tgactgaanc aacaagtcct gaaaccccaa 660
   attcaaaaag caactcaagg cgaananagt antaattttg ctcatcctca tangangggg 720
   cttgcctcaa cccaaggcta aggtcaaact gacccaagac ccanggagta ttaatttgat 780
   ttcaatactt gcttcaatcc cataaatcat gngaacatga tgcntttcaa atttaaacac 840
   tttnaacaac tttttacaca tnttgaaggg ccct 874
<210> 46
   <211> 898
   <212> DNA
   <213> Homo sapiens
<220>
   <221> misc_feature
   <222> (1)...(898)
   <223> n = a, t, c or g
<220>
   <223> nbla-00547-f
<400> 46
   gnngnngnnn nntgtggctt tttnnnnngg ccnngnnctc gatcacgtgg gcctactgtg 60
   gtgagagacg gaccgagacc ggagatgttt tcaagcccgg gctccggcgg ctttacaggc 120
   ggctgcagcg gcgacgaaga caacgacagc gacggctacg ccgaagcact cgttccgggg 180
   gtgaagcctc ctgcgccggc cttgcctcgg atccaggatg agaagactga taaaagaaga 240
   agctagctga acagctgtaa aatgcccaaa tctgggttca cagaaccaat tcagagtgaa 300
   aattctgaca gtgacagcaa tatggtagag aaaccatatg gaagaaagag taaagacaag 360
   attgcatcct acagcaaaac tccaaaaatt gaacgaagtg atgtgagcaa ggagatgaaa 420
   gagaaatcat ccatgaaacg taaacttcct tttactatta gcccatcaag aaatgaagaa 480
   cgagattcag acacagattc agattcagga catacaagtg aaaattgggg ggagagactt 540
   atatcttctt acaggacata ctcagagaaa gaaggtccag aaaagaagaa gacaaaaaag 600
   gaagctggaa ataagaaatc cacaccaatt agcattcctt ttggttatcc actctctgag 660
   cgaaaacaga tggcacttcn tatgcagatg acagcaagag acaacaatcc agattccaca 720
   ccaaatcatc catcacaaac aacgcctgnc caaaagaaaa ctcccaattc ttcatctcga 780
   cagaangnta aagntaataa aagaaatgaa cgtggggaaa ctccttacac atggntgcaa 840
   tccagganat gtgaacaagt taangaatta taagtttagg gcaaatttaa tgttaaaa   898
<210> 47
   <211> 840
   <212> DNA
   <213> Homo sapiens
<220>
   <221> misc_feature
   <222> (1)...(840)
   <223> n = a, t, c or g
<220>
   <223> nbla-00547-r1
<400> 47
   gggntgnnnn ngtggctttt ttttgnntna atgtcnacct tcngtcccat ttttttcant 60
   gtgcaatctg aattttcaaa tgttggacta tgatcttcat cctttatttt ggcatttgat 120
   ttttcaccaa attcnaagtg gaaatctttc tgatgtttgc tttttcctta agcttacaag 180
   attttacact tgaactttct ggcaagaatt cagattctgt attatcatac cgaacaagat 240
   caggctgtaa tgacatttca gaacttcctg gtgataaaca tgttttaagt atgttcttgt 300
   tttagtggtg ttgactgttt ttcacttaat ccacaagaat gtttgggaga tttaccagtg 360
   ggaatatgaa ataaatgtaa tgaagtatca tcttttgggc taaaagattc ctaaagtccc 420
   ctcctctctg gcctttctga acaatctagc cagtatttac tgtcaagttt gtaatcctgg 480
   antttccttt ccttcctttt ctgcttaact cctgggtctc ttaatttaat ctgatttcaa 540
   tttcctttaa ctttgccttc cgtttgtgtc acctgaaaca catatccttt ttgggttgca 600
   aacaaaattg angttcaagg atgacggaca tgaagtanaa anctttatcc gaaanagctt 660
   ctcacaaaac ttcagtactt ganataagac ccaaatggnt ttggttttac ctttaaagga 720
   ttaagntaaa anggttcncc cgnttgcaaa aaangagttt ttccngctca aggncaattc 780
   cnttccgngg nccttcccaa aaaaagccna ncacaancaa cctaanaaat tctcaacaan 840
<210> 48
   <211> 871
   <212> DNA
   <213> Homo sapiens
<220>
   <221> misc_feature
   <222> (1)...(871)
   <223> n = a, t, c or g
<220>
   <223> nbla-00567-f
<400> 48
   ggnttnnnng gcccggtccc gntcacgtgg ccctactggt atgaangaac ctccttcagt 60
   ccaactatcc agacgttcat tctctctgcc atttggcacg gggtataccc aggatattat 120
   ctaacgtttc taacaggggt gttaatgaca ttagcagcaa gagctatgag aaataacttt 180
   agacattatt tcattgaacc ttcccaactg aaattatttt atgatgttat aacatggata 240
   gtaactcaag tagcaataag ttacacagtt gtgccatttg tgcttctttc tataaaacca 300
   tcactcacgt tttacagctc ctggtattat tgcctgcaca ttcttggtat cttagtatta 360
   ttgtgttgcc agtgaaaaaa actcaaagaa gaaagaatac acatgaaaac attcagctct 420
   cacaatccaa aaagtttgat gaaggagaaa attctttggg acagaacagt ttttctacaa 480
   caaacaatgt ttgcaatcag aatcaagaaa tagcctcgag acattcatca ctaaagcagt 540
   gatcgggaag gctctgaggg ctgttttttt tttttgatgt taacagaaac ctatcttagc 600
   accttttcaa ggggtttgag tttgttggaa aagcagttaa ctggggggaa atggncagtt 660
   atagataagg aattcctgtn caccagattg ggaatggngt gaaacaagcc tcccatggca 720
   tgtcccgtgg gccacgccnt atgtagaata ttccataatt caatgggnac tcccaaactc 780
   aacacttgtc cgtnngggca caccgtgncc tgttgcngaa ggtnggttgc gtatcccaag 840
   ggactggaan aggnggaaaa taanntgtcc a                                871
<210> 49
   <211> 901
   <212> DNA
   <213> Homo sapiens
<220>
   <221> misc_feature
   <222> (1)...(901)
   <223> n = a, t, c or g
<220>
   <223> nbla-00567-r1
<400> 49
   gngntgnnnn tgtggggctt ttttttngcc tttgtgagac ngaatctngc nctgtcaccc 60
   gggctgggag tgcagtgggc acaattttag ctcactgcaa cctccacctc ccaggttcaa 120
   gcaattctcc tgcctcagcc tcccaagtag ctgggattac aggtgtgcgc caccatgccc 180
   gtctaatttt tgtgttttca gtagagacgg ggattcacca cgttggccag gctggtctcc 240
   aactcctgac ctcaagtgat ccgcccacct cggcctccca aagtgctagg attacaggtg 300
   tgagccacca tgctcagcca acataatttc aatgcatggg aaaacagagg aaatttattt 360
   agttatcaat cctttcctgg gtagggttag ccttatcagc cggaaaacac ttacttttag 420
   ataaataaaa atagattagg atatttccaa acttcaaaaa agacacatgc ccatttttgc 480
   tgagacctca aaagctgcaa gtgcatttgc ttttcccttc ctccttaaat gaagtattat 540
   atagcataag ccaaatgggt gccttgncct taactcaatt tgcaaattta taatggctcc 600
   aaccccccga aaaaaagnga gagagaaccc atcacaagaa aanttgcatt caattaaaat 660
   taaggnaaaa agttaaaant agnttcaaac aataggtcaa acaaangngg aatcaaaaag 720
   gnttgcccct cccctcccaa aaggnaaggn ggnanaaacc tggggntnaa ggcantccaa 780
   aggcctggga ncaaaatcca atcctttggn ntttaanncc cgggggcaac aaaantccnc 840
   cggngggatt tcangggaat gctnnnaatc caagggaaan ttantttngg gaaaaanggc 900
   c 901
<210> 50
   <211> 881
   <212> DNA
   <213> Homo sapiens
<220>
   <221> misc_feature
   <222> (1)...(881)
   <223> n = a, t, c or g
<220>
   <223> nbla-00569-f
<400> 50
   gngnnnnnnn nggccgggat cctcgntcac gtggccctac tgggcaaccc tcccaaaact 60
   gtggggcatt gatgacctga ctggggagcc tctcattcag cgtgaggatg ataaaccaga 120
   gacggttatc aagagactaa aggcttatga agaccaaaca aagccagtcc tggaatatta 180
   ccagaaaaaa ggggtgctgg aaacattctc cggaacagaa accaacaaga tttggcccta 240
   tgtatatgct ttcctacaaa ctaaagttcc acaaagaagc cagaaagctt cagttactcc 300
   atgaggagaa atgtgtgtaa ctattaatag taagatgggc aaacctccta gtccttgcat 360
   ttagaagctg cttttcctaa gacttctagt atgtatgaat tctttgaaaa ttatattact 420
   tttatttcta ctgattttat tttggatact aaggatgtgc caaatgattc ggatactaag 480
   atgcatcgtt tgaaatcatc tantgtgttg tatgcagtta tcctcaaaaa catcagcgat 540
   gtctgaacct ttaaaacatc tgttagagca aaattaaaaa gagcatttgg tagtaatcca 600
   actttttggt caattaataa gtggntgata aaagtttcca naattttccg ggaaagttaa 660
   aaaaaagtta catgtcantt ggngaaaata cgttatccag aanttgtgca taaantgatg 720
   ccaaaaaagg canttcaaca ttgtggacaa ngggagacac tntttaaant cccgaaagaa 780
   agcaatgggt tttcannttt atgggngaaa caaattaang ggggccttna acnaanaaat 840
   gttatgttaa ccaanangcc gngnttnatt tggcnnnaan n 881
<210> 51
   <211> 884
   <212> DNA
   <213> Homo sapiens
<220>
   <221> misc_feature
   <222> (1)...(884)
   <223> n = a, t, c or g
<220>
   <223> nbla-00569-r1
<400> 51
   ggnngnnntg nngggctttt tttggnttgt ttaagatcat ttagnagaac agtcattcag 60
   aagccattga gacatcaggc agcagaaagg aaggtgggat ggagcaggcc ctgtgaagga 120
   ccaagaacaa agtaatagcc acagttatga aatttcattt tattctgata aagactaata 180
   tatgcttgat aacctagtga taatccataa gtttggtatt tcacaacatt ttttagaaag 240
   cacataagat taacattcaa ataaggcatt atagaaagtt ttataaagaa tgaagtgttt 300
   cctataattc ttttaaaaaa ccttggttca tcttgaaaga tcgatgaatt ttttaaatat 360
   cagaagaaaa gggaaataaa attttccccc caaaacacat aagaaccact tactggcact 420
   tgtattttaa gtacctggga aaaaaacggg acagattttt aaaggnaata acgacttgta 480
   agacggctgn ttcattgatt tggcacgaag taaagtaaga gtaaatatgc catgggagac 540
   ataatcaagt ttttcctcca nctcccaaaa ttccccactt ccaacaagac acacagtnca 600
   tcaacaanca ncccttaaat tccaantttt aaatngntgn tcaaacgaca ncaataaaag 660
   tccttccccc ttaaataagg gggnaatnna tgctacaagn atcaaattcc ttggtnaagg 720
   acaaagnant ccctcaagga aatcaanttc ccgggccctg gtaanccaac aagnnaatcc 780
   ntttatngga caaccggtta acaaattaaa aatgntggca attnttncca ggggacaant 840
   tccaagnaag cccaaccatt ttcnanaacn aagntggntt tctt884
<210> 52
   <211> 793
   <212> DNA
   <213> Homo sapiens
<220>
   <221> misc_feature
   <222> (1)...(793)
   <223> n = a, t, c or g
<220>
   <223> nbla-00639-f
<400> 52
   gtttaatgcc tagttatgca acttgagtgc tttttttttt ttttgcagga taattttaag 60
   tgtatatatt ttaactgcac tggtacattg aacatgtcag tgtcgatgcc actggaccaa 120
   cagagcattt tgtggctata gggtgcctga taataatgtc ttgatttttc tttgggattc 180
   aactatatcg aaggtttttc ccccagcagt atgggggcgt acaggtgatc gatcattaat 240
   gtcattgcaa ttgttatttt ttaaaataaa tttataaaaa taaccgaaaa gtaatgtgac 300
   ttttgangaa tctgcaactc ctgggttcta ntttgtgttt ccagtgttga aattttgatc 360
   tcccccgaca agacttgtct gcatggacgg tgtctctgta aacagtatca aacttngtna 420
   tgtctgagcc tcttagcttt atctccctta atttcntccc ttttggccct tccaccctgt 480
   tgaggagant tttctgtcac ctgcanggct gatnatacat ttgatgctac nactgaangt 540
   gttatcngaa tgtgnnnnan tnantncatg angaaaatta acccgtantc acaaaaacag 600
   gcttaantan tgnanaaatt caggtagtga nngattanan ntnctnttgg ntagaatttg 660
   ttttgngaga aagncanncn anangtnctt tctnaatngg nanaatccca annnnncnca 720
   accttgnaac gggttnctta agggcncnng gaatcnnanc acnannncca naagccncca 780
   tantnnntag tnn                                                793
<210> 53
   <211> 882
   <212> DNA
   <213> Homo sapiens
<220>
   <221> misc_feature
   <222> (1)...(882)
   <223> n = a, t, c or g
<220>
   <223> nbla-00666f
<400> 53
   gnnttnnnnn ggcccnnnnn ggattcctcn ttcactgtgg cccctactgg gacaccgcca 60
   aaggaagtca anaaggaagt taagaaggaa gagaagaagg aagtgaaaaa ggaagaaaag 120
   gaacccaaaa aagaaattaa gaagctccct aaagacgcaa agaaatcatc tactcctctg 180
   tctgaagcaa aaaaaccagc tgctttaaaa ccaaaagtac ccaagaagga agagtctgtc 240
   aagaaagatt ctgttgctgc cggaaagcca aaggagaagg ggaaaataaa agtcattaag 300
   aaggaaggca aggccgcaga ggctgtcgct gcagctgccg gcactggagc caccacagca 360
   gctgtcatgg cggcagctgg aatagcagcc attggccctg ccaaagaact cgaagctgag 420
   aggtccctta tgtcatctcc tgaggatcta accaaggact ttgaagagtt aaaggctgaa 480
   gaggtcgatg taacaaagga catcaagcct cagctggagc taatcgaaga cgaagagaaa 540
   ctgaaggaaa ctgagccagt cgaagcctac gtcatccaga aggagagaga agtcaccaaa 600
   ggtcctgccg agtcccctga tgagggaatc actaccactg aaggggaagg cgaatgtgaa 660
   caagacacct gangaactgg ancccgtcna gaaacaagga atanacgaca ttgaaaaatt 720
   ttgaagatga angganccgn tttgaagaat cctcanagac tggngactat gaanagaang 780
   nanaaactga ggangctgag gagccanaan angatgggga aggaacactt tgttttancg 840
   cctccaaagc anaagcccac tgagggtnna ggaaatttcc aa                882
<210> 54
   <211> 961
   <212> DNA
   <213> Homo sapiens
<220>
   <221> misc_feature
   <222> (1)...(961)
   <223> n = a, t, c or g
<220>
   <223> nbla-00666r1
<400> 54
   cnnnnannan nnnnanngna annanannnn nnnnnnnnnn ngnnnnnann ggnngnnctn 60
   agnagnanna nncnnngntg attantnagg ccnannngnn ttattttttg gccttttttt 120
   ttttnanttt tttttntnnt ttttttttaa aaantcaaat ncnggntttt tttttattta 180
   agaggaatgt aaatngtnaa nncncggtta ccaagggana nataaaanca aaancaaaaa 240
   acgggnctna nttggccngg aaacccttgt tttcnancct tttcttcaaa aggantttcg 300
   gaaccncggc naaaaccctt gnnaccaana cttaaaaaan tttcaaaagn aaacccggtt 360
   cnaangaggg gngggntgcg taaagggnaa aaaanttttt caaaaggaaa cnctgantca 420
   ggnacgggcn canccanggg gcttatggaa cccctttnan tccnaccttt ggaaacacgg 480
   anttcaaaac ccacgggaac ngcngggggt ttttcaanaa cttntgtngg aaaaanacng 540
   gatttcccgc cagtaggaaa ttgataanaa ngngngtgac caacaactgc cattcacggg 600
   cngaaaatgg gganacaact tccaaaancn cttgnccnca ggactancna aaagttcttc 660
   aaaaanttnt tgggggcccc ncangnaaaa accggggggn aattntggcc cccgaaaaaa 720
   aatttnaacc caatgggggg ncnaaaaaaa aanttcaaan ctaaggtttc accccaaggg 780
   ggggcttttt ccaaaggggg aatgggggaa annggcccca gggaaggggc tccttccaaa 840
   ggggcgnaac tttttnactt tnaaaaancn gaaaagccaa ccttgangtc ccaanagggg 900
   gngggggttt nccttnaana nnantanccc cccctnagca attnggcgaa tttnncnccc 960
   c                                                          961
<210> 55
   <211> 801
   <212> DNA
   <213> Homo sapiens
<220>
   <221> misc_feature
   <222> (1)...(801)
   <223> n = a, t, c or g
<220>
   <223> nbla-00724-f
<400> 55
   gnnntgtngc ttttnnnngg gcatcctcta gcactggcng nctactggga aanctacaat 60
   ttcancccca ctggctgaga tgaccagctg acctttaagg ctgttgaagg atctctcatc 120
   ccactctcct ggcactaggg atttctccac acccaccatc tccgaaggaa aagcagaatc 180
   cactccctcc cacactcttt ctgccatttc attcatgcat gcctgaatca gaaccctcac 240
   tctctctgcc attcattcat acatgcctga atcagaacct tcactctctc tgccattcat 300
   tcatacatgc ctgaatcaga accctcactc tctctgtcat ttcattcata catgcctgaa 360
   tcanaaccct cacactctct gtcatttcat tcatacatgc ctgaatcana accctgccca 420
   gcgacacaaa cggagtgctc agttctgccg tgaagatgca gccgacaaag aagtcnccaa 480
   ggtctccccg gagcctctgc ggatgacnaa acgaaaaggg gaagtaagtg gaggttaaga 540
   aaccaccaga aggaaaattc caacgaaggn tactgaaaag ggtaactgtg acnggcaaag 600
   gacgctcaat angnaagcgc agactactgt ancgctgctt ccgttgnaac angcactttg 660
   atngtccaag ccctaagatt caaangcaaa ttnanacang gtgacggggt tccacaacaa 720
   nctctgaatt ttngtgacaa cccgnaagga aggntgaaag cctacttttg tgtnccaact 780
   tatcatgcaa nanactnant c 801
<210> 56
   <211> 791
   <212> DNA
   <213> Homo sapiens
<220>
   <221> misc_feature
   <222> (1)...(791)
   <223> n = a, t, c or g
<220>
   <223> nbla-00724-r1
<400> 56
   gnntgtggct tttttttggc nttttctgag acagtctcac tctgcngccn cagctgggag 60
   tgcagtgggt gtgatctcag ctcactgcaa cctccgcctc ccgggttcaa gcgattctcc 120
   tgcctcagcc tcctgagtag ctgggattgc aggtgcccac caccacgccc ggctaatgtt 180
   tgtattttta gtagagatgg ggttttgcca tgttggccag gctgacttca aactcctgaa 240
   ctcaagtgat ccgcccgcct cggcctccca aagttctggg attacaggca tgagccactg 300
   cgcccggcct gagctagatg tcttgaccgt tacttaggag tcgggattgc ccctcttttc 360
   tttgccactc ataggcaccc attctccttg tgcctgctgc ttcagagcca cctcccccag 420
   gtgaatagtg gaggaacacc attacacatg aaacgttcat gatcctcctt accaacatca 480
   acatcaacat actttttctt ttttgagaca gggtttcact ctgtcacccc gggtggagtg 540
   cagttgttgg atnacagctc actgtacctc aaactactgg nctcaagtga tcctcctgcc 600
   tctgcttcca agaaagctgg gaacacaggc acacactaca acgtccaact aattcctgta 660
   attttgtana ntttctccat gttgcccaag ctggtctcaa actcntgggc tcaagtgatc 720
   tcctgcctaa ggctccaaag tgctgggatt acaagggcna ancaatgnaa ccgggcccaa 780
   aaattctttt t                                                     791
<210> 57
   <211> 796
   <212> DNA
   <213> Homo sapiens
<220>
   <221> misc_feature
   <222> (1)...(796)
   <223> n = a, t, c or g
<220>
   <223> nbla-00727-f
<400> 57
   gnnnnnnnnt ttttnnnggc ntcttttgca ctggctcgcc tactggactn aggagtggga 60
   gccccggggt cagagcgata attcccccac aggaggcatc cgctctacgg cgggagattg 120
   aggacaagct gaagcaggag gaggagactc tgtccttcat ccgagacagc ctggagaaga 180
   gcgaccagct cactaagaac atggtgtcta tcttatcatc ctttgagagc cgccttatga 240
   agctggagaa ctccatcatc cctgtgcaca agcagacgga gaatctgcag cggctgcagg 300
   agaatgttga gaagacgctg tcctgcctgg accatgtcat cagctactac catgtggcca 360
   gtgacactga gaagatnatc agagagggcc ccacaggtag gctggaagag tacctgggaa 420
   gcatggccaa gattcagaag gcagtggagt atttccagga caacagccca gacagcccgg 480
   aactcaacaa agtgaaactg ctctttganc gcgggaagga ngccctggag tccgaatttc 540
   gcancctgat gacgcggcac agtaaggtcg tctcgcccgt gctcatcttg gatntgatca 600
   gtggtgacga tgatntggag ggccaagagg acntnaccct ggagcacctg ccgaaaacgt 660
   gctccaagat gtcantcgca tctcccgctg gctggttgga tatggccgca accaagantt 720
   catgacgtct actaacaaga tanctccaac caactggacc gctccatcaa agggctgang 780
   gncatttcat tanaan 796
<210> 58
   <211> 799
   <212> DNA
   <213> Homo sapiens
<220>
   <221> misc_feature
   <222> (1)...(799)
   <223> n = a, t, c or g
<220>
   <223> nbla-00727-r1
<400> 58
   gntgtggctt ttttttggcc tttttttttt ttgccctttt tttttncaaa gctaacactg 60
   ggncttatct gtccaatgaa acgccagtct ggtggaacca ggcagggcta gcagcaggct 120
   caggcagagg tgtcgaaaag gcgatcgatc atgtcgccca cctgctccac cccgtacttg 180
   atgtacttct ccgggttctt ggtgaagggc acgctgccaa acttctgtag aaaggccccg 240
   taggtctcct tgacaatggt cttctgggcc tggcgaatcc tgtccctctg ctctgtgtct 300
   ggaatanccc angccttctg gattttgcac agttcttcga ggccatcatt gaagccctta 360
   aaacgctcct tgataatctg ccgctccttg tcccggagct tgactcccgg ctggaacaca 420
   ggtagattct tctctgcgat gtaatcagtc acctttaacc agctgcgctg gtaggtctgg 480
   atntgctgct caatgtgctc ccggtaggag cgctcaacan tcttctgtgt cactgccacc 540
   anctggatna gttcaaactt ctccaaggac ttgaggatgt aattgtantt gttgtgcaag 600
   aaaatggcgc tcaaanctgg gtcctcgtan ancttggnct tgctcaacaa gttcaantgc 660
   angttgccaa cactttncaa atatnngtgc ttaacaaccg ctttctgaac tcaaaactgt 720
   anctgggngg cccaaaaact ggtntctccg gggggtctaa aaggaatatt gtatgtgtcc 780
   ccaaaaaant tgggangnc                                              799
<210> 59
   <211> 484
   <212> DNA
   <213> Homo sapiens
<220>
   <221> misc_feature
   <222> (1)...(484)
   <223> n = a, t, corp
<220>
   <223> nbla-00751-f
<400> 59
   ggggnnnntt nncttgcngc ctgtttcctc gtgcactgtt ggtaactggt cggcctcgct 60
   tcaggaaaaa aagtacctca tnctcggcca ccgaaaccac gtgagtgaga tgagccaaca 120
   gcaccggatc cacagaatgt ttcttctctg ccttaaagag ctattcacta ataacataga 180
   aatccgcaag ctgggtgtgc tttgagtgtg cagcctcaca aacatggcct tttctctctc 240
   cccttccact tttaaggatt tattttttcc cccttttctt tattttgctg gggagaggct 300
   aaagggaaag gtagtagggg cgggggtggt gacctttaag tcttctgagg ttggtaattt 360
   tccacaattg gattgtcatt atagacagca gtgtgttttt tagaaagata agagaatcac 420
   ccctatgctg ctgagatgta catttgtaat ttatctgttg catacttagt ttttagtcct 480
   gtaa484
<210> 60
   <211> 485
   <212> DNA
   <213> Homo sapiens
<220>
   <221> misc_feature
   <222> (1)-...(485)
   <223> n = a, t, c or g
<220>
   <223> nbla-00751-r1
<400> 60
   gnnncntgtn tgcctttgct tttttttttt aaagccaaaa gtnttaaaga ctttattcca 60
   gatgtgtatc angcattata acagaacagc agaacttcaa cctttggaat actgtaattt 120
   tacatccctt tgatgcacag tccagtatac tattttatta cagatcattc tatagggact 180
   acagacatga actagaggaa atgtgcacag tcacaatcca gaatatcagc tctgggagtg 240
   tacactgttt gttagaggat gaagcacatc ctttgccatt tcaaatactg tgccaggtgg 300
   aggactagga aggctcaaag atggtcatgg ttgacaagca ctcttatcac aaacacatgg 360
   atagcttatc acggagaaca catttcaaag gccagcaaag tgagcaagct attcacacaa 420
   agccaggagg gattatgact aaactctcca gtttataagc acaagtccac atctcacctc 480
   ctcag                                                             485
<210> 61
   <211> 473
   <212> DNA
   <213> Homo sapiens
<220>
   <221> misc_feature
   <222> (1)...(473)
   <223> n = a, t, c or g
<220>
   <223> nbla-00757-f
<400> 61
   tggccngcnn gtttcctcgc ccactgttgg tanactggag ttgcgcaaga tggcgccngt 60
   gggggtggag aagaagctgc tgctaggtcc caacgggccc gcggtggcgg ccgccggcga 120
   cctgaccagt gaggaggagg aaggccagag cctatggtcc tccattctga gcgaagtgtc 180
   cacccgcgcc aggtccaagc tgccgtccgg caagaacatc ctggtcttcg gtgaagatgg 240
   ttctggtaaa acaaccctca tgactaaact acaaggagct gagcatggca aaaaaggaag 300
   aggcctagaa tatctctacc tcagtgtcca tgatgaggac cgagatgatc acacgcgctg 360
   caacgtgtgg attctggatg gagacttgta ccacaaaggc ctgctgaaat ttgcagtttc 420
   tgctgaatcc ttgccagaga ccctcgtcat ttttgttgca gacatgtcta gac   473
<210> 62
   <211> 487
   <212> DNA
   <213> Homo sapiens
<220>
   <221> misc_feature
   <222> (1)...(487)
   <223> n = a, t, c or g
<220>
   <223> nbla-00757-r1
<400> 62
   gnnngctgtt tgccttngnc tttttttttt ttaagccaca aggccgcata atgtaaccag 60
   ccataatgtg cataaatagt cataaaattg tctttaacag tctaccatac aagtgtactc 120
   tgcaaataaa atgaatttta ctttaaaaac taaaggctgt ttattatttg gcctttggtt 180
   ccaatgcttg aaaaccccaa aataacaaaa ctcatctccc ttcctcccac ctcccaaaaa 240
   gaaccctgca gctccccaaa gttggtagac tttggcaacc actgagcaca tgacactatg 300
   tatcagcttc ttctgatact aggagcttcc acttctcatg agtaaataaa agtggtctcc 360
   aagaaacctt ccttcacaag acatgtctta gcatttcatc agttttagta tattttatat 420
   tgacacaaaa agtgcttatc agttcaaatg ataaaaaata aagacaaatt aaaattttgc 480
   attaaaa                                                          487
<210> 63
   <211> 2815
   <212> DNA
   <213> Homo sapiens
<220>
   <221> misc_feature
   <222> (1)...(2815)
   <223> n = a, t, c or g
<220>
   <223> nbla-00761
<400> 63
   gaattcctcg agcactgttg gcctactgga acagcaccac ttagatccta gctctgctgt 60
   aagaaaaaca aaacgggaaa aaatggaaac aatgttaagg aatgattctc tcagttcaga 120
   ccagtcagag tcagtgagac ctccaccacc aaagcctcat aaatcaaaga aaggcggtaa 180
   aatgcgccag atttcgttga gcagttcaga ggaggaattg gcttccacgc ctgaatatac 240
   aagttgtgat gatgttgaga ttgaaagtga gagtgtaagt gaaaaaggag acatggatta 300
   caactggttg gatcatacgt cttggcatag cagtgaggca tccccaatgt ctttgcaccc 360
   tgtaacctgg caaccatcta aagatggaga tcgtttaatt ggtcgcattt tattaaataa 420
   gcgtctaaaa gatggaagtg tacctcgaga ttcaggagca atgcttggct tgaaggttgt 480
   aggaggaaag atgactgaat caggtcggct ttgtgcattt attactaaag taaaaaaagg 540
   aagtttagct gatactgtag gacatcttag accaggtgat gaagtattag aatggaatgg 600
   aagactactg caaggagcca catttgagga agtgtacaac atcattctag aatccaaacc 660
   tgaaccacaa gtagaacttg tagtttcaag gcctattgga gatataccgc gaatacctga 720
   tagcacacat gcacaactgg agtccagttc tagctccttt gaatctcaaa aaatggatcg 780
   tccttctatt tctgttacct ctcccatgag tcctggaatg ttgagggatg tcccacagtt 840
   cttatcagga caactttcaa gccaaagcct tagtagaaga acaacgcctt ttgttcctag 900
   ggttcagata aaactatggt ttgacaaggt tggtcaccaa ttaatagtta caattttggg 960
   agcaaaagat ctcccttcca gggaagatgg gaggccaagg aatccttatg ttaaaattta 1020
   ctttcttcca gacagaagtg ataaaaacaa gagaagaact aaaacagtaa agaaaacatt 1080
   ggaacccaaa tggaaccaaa cattcattta ttctccagtc caccgaagag aatttcggga 1140
   acgaatgcta gagattaccc tttgggatca agctcgtgtt cgagaggaag aaagtgaatt 1200
   cttaggcgag attttaattg aattagaaac agcattatta gatgatgagc cacattggta 1260
   caaacttcag acgcatgatg tctcttcatt gccacttccc cacccttctc catatatgcc 1320
   acgaagacag ctccatggag agagcccaac acggaggttg caaaggtcaa agagaataag 1380
   tgatagtgaa gtctctgact atgactgtga tgatggaatt ggtgtagtat cagattatcg 1440
   acatgatggt cgagatcttc aaagctcaac attatcagtg ccagaacaag taatgtcatc 1500
   aaaccactgt tcaccatcag ggtctcctca tcgagtagat gttataggaa ggactagatc 1560
   atggtcaccc agtgtccctc ctccacaaag tcggaatgtg gaacaggggc ttcgagggac 1620
   ccgcactatg accggacatt ataatacaat tagccgaatg gacagacatc gtgtcatgga 1680
   tgaccattat tctccagata gagacaggga ttgtgaagca gcagatagac agccatatca 1740
   cagatccaga tcaacagaac aacggcctct ccttgagcgg accaccaccc gctccagatc 1800
   cactgaacgt cctgatacaa acctcatgag gtcgatgcct tcattaatga ctggaagatc 1860
   tgcccctcct tcacctgcct tatcgaggtc tcatcctcgt actgggtctg tccagacaag 1920
   cccatcaagt actccagtcg caggacgaag gggccgacag cttccacagc ttccaccaaa 1980
   gggaacgttg gatagaaaag caggaggtaa aaaactaagg agcactgtcc aaagaagtac 2040
   agaaacaggc ctggccgtgg aaatgaggaa ctggatgact cgacaggcaa gccgagagtc 2100
   tacagatggt agcatgaaca gctacagctc agaaggaaat ctgattttcc ctggtgttcg 2160
   cttggcctct gatagccagt tcagtgattt cctggatggc cttggccctg ctcagctagt 2220
   gggacgccag actctggcaa cacctgcaat gggtgacatt caagtaggaa tgatggacaa 2280
   aaagggacag ctggaggtag aaatcatccg ggcccgtggc cttgttgtaa aaccaggttc 2340
   caagacactg ccagcaccgt atgtaaaagt gtatctatta gataacggag tctgcatagc 2400
   caaaaagaaa acaaaagtgg caagaaaaac gctggaaccc ctttaccagc agctattatc 2460
   tttcgaagag agtccacaag gaaaagtttt acagatcatc gtctggggag attatggccg 2520
   catggatcac aaatctttta tgggagtggc ccagatactt ttagatgaac tagagctatc 2580
   caatatggtg atcggatggt tcaaactttt cccaccttcc tccctagtag atccaacctt 2640
   ggctcctctg acaagaagag cttcccaatc atctctggaa agttcaactg gaccttctta 2700
   ctctcgttca tagcagctgt aaaaaaattg ttgtcacagc aaccagcgtt acaaaaaaaa 2760
   aaaaaaaaaa aggccacatg tgctcgagct gcaggtcgcg gccgctagac tagtc 2815
<210> 64
   <211> 815
   <212> DNA
   <213> Homo sapiens
<220>
   <221> misc_feature
   <222> (1)...(815)
   <223> n = a, t, c or g
<220>
   <223> nbla-00775-f
<400> 64
   gcaattctaa ttctttttgc agatctctct gcctccaggg gnttaccnct gagctgagaa 60
   agccnaaaac ggagttctgt ttatttctgc gcccctctca agtaggtatt aaaaaagatt 120
   gangacatga aagaaagaat aactgaatgt tactgatgcc agaagcaact ctcgctgggc 180
   gcaactctcg ctgggcgcaa ctctcgctgg gcctcagttt ctcatgagca gaactgcagg 240
   gggtaggtgg tcccaagtct ctagggtctc caccaagctt ctcctcagtg cccaagaagt 300
   ggtacagtgt gctctcaata gtccttcagc ttatgacagc ctcagctcat gggacttgat 360
   gacggggaag agcaagctgc atgccatgaa gtcagccaag tttgcctgtg gaggctgctc 420
   ccatctcctt tcaaaactgc cccctcccct atcaagnccc cacttcttcc ccanggcctt 480
   gctctcagcc aactgtctgg gatggntttc ctgtatagct ttgagctccc tcagcacaag 540
   gagcctgaca tggngttang aaaaaagaaa ggaaaatgat tanaggnncc aaaagnccat 600
   gatnccataa ncccaaaggt taagtacctt aaggggggat tangtgccct gtnanncaga 660
   atnagagaga tnaaagttgg gnnncaanca atagtaaggg gctttaggtn acctggnaac 720
   tccaggagct ccaanggcca anannnatgt tttattcacg gcaactcccc ngnaattggg 780
   aagtttccan catacacatg ggaanaaacc naagg815
<210> 65
   <211> 799
   <212> DNA
   <213> Homo sapiens
<220>
   <221> misc_feature
   <222> (1)...(799)
   <223> n = a, t, c or g
<220>
   <223> nbla-00775-r1
<400> 65
   aacctccacc tcttgggtcc aagtgattct cctgcctcag cctcccgagt agctaggact 60
   acaggcgtgc accacgccca gctaattttt gtattttttt ttttttagta gagacggggt 120
   tttgccaggt tggccagggt agtctcaaac tcctgacctc aggtgatctg cctgcctcgg 180
   cttcccaaag tgtttggatt acaggtgtga gccactgcac ccatccatgg cccttttctt 240
   gtcctcccca cggggctttg tctgatggat ttcaagcatc agcaaaggaa atactgggtg 300
   tcactgtcat ctgggaccaa cctgattctg gaggagccaa aagtctcatg gtgattgatc 360
   tcgtccgcnt ggggtgtatg gttgtaatcc ttcnctctgg cattanggca cacacgaatt 420
   cctaacatcc cactccaacg aaacacttgt gcacagatta caaacaccaa tatgatttaa 480
   tcaaacacaa gnaaagaaca atgaccntca aaaccgggta aaaataataa anttaaaacc 540
   atggtaaaaa tttanacaat cncgttaaaa gttngaagga atgattcccc tcctccccaa 600
   ttgataaatc aacaccanat aaaaataant tgttccaaaa aaaanccctg gtccccaaaa 660
   aatnactggg ngggggccca aggattccct tacaatccca atgncacaaa ctaaaattcc 720
   cccccccaaa ttttaagnga aaatggnccc caactntagg aaaaggnggg nccagccana 780
   ttnannnntt taagggann                                        799
<210> 66
   <211> 867
   <212> DNA
   <213> Homo sapiens
<220>
   <221> misc_feature
   <222> (1)...(867)
   <223> n = a, t, c or g
<220>
   <223> nbla-00784-f
<400> 66
   gnngnnnnnn nnnnnggnnn nttnnnnntg gccggatcct ctttcactga ggcccctact 60
   ggctaatatt atattttcng ccccagtgtt tttaattaaa aatatgtgat agggaccana 120
   taagtaaagt acatttttct ccactaaatt tgaagtgagg gaaagaggag ccaaagtaag 180
   agattttttt ttaaggaaac ttaatctgat tgtgaaaatc atacatctgg agaaacatca 240
   gatcaggcaa tagagtcaga gggtcatgag caatagacga tgatgcgagg catttgggga 300
   gcttcctgga ggaaaattaa gtttttttcc tagcaaacta ccatgtccta caagaacttg 360
   gttatataat ggtgcgtccc tgaatcactg attaaaacca gttgcttctg attttaagtc 420
   acaggtttta caagtattca gctctccctc atgtttcatt tcntttttta agataatcta 480
   tcaacctttt ttaaatttta aaatttttag atgtanagtt tataagtaaa atatattttt 540
   agccattgtt cngttanctg aactgatgtg tttggtctaa anggcctgac ttcaaatacc 600
   cttgggatcn tgtnagggct ctacacaaac ttcantaagt ntgggaaatt gtatcctaan 660
   ggattgtata nanaatgcnt tcnntanaag tacatccact tccggangtc cccttgnaan 720
   cccaattgcn tantcagggg ttcaaanccn ttcanaaaaa taattcacta actgtccaan 780
   gggattttaa aacgtttacc ggtnnanaac ncnntnnggg tccttcaaaa ntcccnaana 840
   nacggttana aaacctcccg gnaaagn 867
<210> 67
   <211> 879
   <212> DNA
   <213> Homo sapiens
<220>
   <221> misc_feature
   <222> (1)...(879)
   <223> n = a, t, c or g
<220>
   <223> nbla-00784-r1
<400> 67
   gggnngtnnt gtggcttttt tnggcctttt acttatnang ctttnggcca ctnacgaata 60
   taaaactgac agatattttc aaacattttt gcaaaaaaag taaaaccctc ccctgtcacc 120
   cctccccaca agatagagaa taaggacttg gacagagaaa caatacaaat ctaaacatga 180
   aactgtcgct catcggttgg tcccaagagg ctccaacata ttatattcta agtaaaggtc 240
   cattaaatta agaagttaac aagccacttg acacttttat agtttaactt gccttctact 300
   ctgattatga tgctcattca aaatgctgag agacaagaac aggtaacatt aacactgcac 360
   gtgacaagat attcaaagtg cataaatcta tgaaaccaca aacattcata agaatactan 420
   gcatgggatt aaacagtgac ttaaaaatta atccaaggga atctatgtac attttaacct 480
   ttggatctgg tcaacatgtt tcctccttat gtagacatta atttggtaaa gtctantaac 540
   caagattaaa tttatctgga cacatttctc atctgacaca aattgtccac aagccaantt 600
   cacatgaggn aatttgggct gtttaatggc antctantgg aaattctcaa tgntacatng 660
   gaattnttca tttaacatgc caatgggact gggtcaaaan ccttctaggg aacaacaaga 720
   agtnntttca anagcatcct tcccaacaan aatggctcca tattcacctn ttaacangcn 780
   ccatnnncaa ggggtccgtg tgggtaaaat ttnactttct nagggggttc aaccatccca 840
   agggggggtt cggtttntaa ggggnaaggg agggaatan 879
<210> 68
   <211> 2120
   <212> DNA
   <213> Homo sapiens
<220>
   <221> misc_feature
   <222> (1)...(2120)
   <223> n = a, t, c or g
<220>
   <223> nbla-00798
<400> 68
   agtgttactt ctgctctaaa agctgcggaa ttcctcgagc actgttggcc tactggttaa 60
   ctttatattt tcagaaaagt gtttttaatt aaaaatatgt gatagggacc aaataagtaa 120
   agtacatttt tctccactaa atttgaagtg agggaaagag gagccaaagt aagagatttt 180
   tttttaagga aacttaatct gattgtgaaa atcatacatc tggagaaaca tcagatcagg 240
   caatagagtc agagggtcat gagcaataga cgatgatgcg aggcatttgg ggagcttcct 300
   ggaggaaaat taagtttttt tcctagcaaa ctaccatgtc ctacaagaac ttggttatat 360
   aatggtgcgt ccctgaatca ctgattaaaa ccagttgctt ctgattttag tcacaggttt 420
   tacaagtatt cagctctccc tcatgtttca tttctttttt taagataatc tatcaacctt 480
   ttttaaattt taaaattttt agatgtagag tttataagta aaatatattt ttagccattg 540
   ttctgttagc tgagctgatg tgtttggtct tagagggcct gacttcagat actctttgtg 600
   atcttgtaag ggctctacac aaacttcatt atgtatggta aatttgtatt cttatggatt 660
   gtatatagaa tgctttcgtt agaagtacat tctacttctg tatgtccctt tgtaatccgc 720
   agttgcttac tcaggggttt catagtcatt tcataaaaaa taattcacta gctgtctaat 780
   ggtattttaa gactgtttat ctgtatcaca acgtcattag gagttctttc aacaattcca 840
   taaatatact gtttactaga ccttccctgt aaatgttccc aattcccatc ctgtctcaga 900
   cagtcaatag tcctgtgtac agtgactatt tgcatgattt ctcattgcac tgctgcattc 960
   aggcactcca gggcatgatt aaacagtcat taacagtgcc tctctggtac agttgatgca 1020
   tgcattgatc tttcttctct gctgttttta tatagccttt aattaaaagg aaaaaaatac 1080
   cactactctg caatgcaaaa gtcttcaaaa ttctttgttt cctgtattaa tcacttctgt 1140
   tccagagtga acaaatgttt tcagctaagc tatgtgagaa tgtaagaata atatcctgct 1200
   tgttctaaat agttcatata tttaaagtgt ggtcagtatt tcctccctgt accttacaaa 1260
   cagaaaccac cctgggatgg ttgatacccc ttacgaagtc gatcttaccc acacagactc 1320
   ctgtgtatgc gtgtctgttt ataggtgtat atggagtcag tgttgatagg aaggatgctc 1380
   tagaagtact tctgttgttt cctagaaggc tatgagccag ttccatggca tgtttaatgt 1440
   ataattccca tgtatcatga gaatttcact agaatgtcat taaacagccc aactacctca 1500
   tgtgaaattg gctgtggaca atctgtgtca gatgagaaat gtgttcagat aaatttaatc 1560
   tggttaatag acttaacaaa ttaatgtcta cataaagaag aaacatgata gaccagatgc 1620
   caaaggctaa aatgtacata gatttccttg gattaatttt taagtcactg tttaattcca 1680
   tgcctagtat tcttatgaat gtttgtggtt tcatagattt atgcactttg aatatctgtc 1740
   acgtgcagtg ttaatgttac ctgttcttgt ctctcagcat tttgaatgag catcataatc 1800
   agagtagaag gcaagttaaa ctataaaagt gtcaagtggc ttgttaactt cttaatttaa 1860
   tggaccttta cttagaatat aatatgttgg agcctcttgg gaccaaccga tgagcgacag 1920
   tttcatgttt agatttgtat tgtttctctg tccaagtcct tattctctat cttgtgggga 1980
   ggggtgacag gggagggttt tacttttttt gcaaaaatgt ttgaaaatat ctgtcagatt 2040
   ttatattcgt tagttataat aaacttattt ttaaagtaaa aaaaaaaaaa aaaaaaaggc 2100
   cacatgtgct cgagctgcag                                        2120
<210> 69
   <211> 2134
   <212> DNA
   <213> Homo sapiens
<220>
   <221> misc_feature
   <222> (1)...(2134)
   <223> n = a, t, c or g
<220>
   <223> nbla-00830
<400> 69
   ggcctactgg aaaggtattt aacatcctaa aacacttttg aaaaagcccc aatttatata 60
   gaatctgaaa aacatgaaaa tgccagaatt aaatagtagt tgtttgtgat cccttgaact 120
   ctgatcattt taattagcaa aaagcaataa tattttatat tccatacttc ccagaaagtt 180
   aattgattca aattcttgct atgttatgcc gtcatgcaga tcaaagagaa cttgtacaaa 240
   caaaaagata ttaactgttt tttctagaaa aacctttgtt agttatttag ctatagcata 300
   atacacagta gaactcattt ttccgaagat gattgccata aagctttacc attttacaaa 360
   gctctagcat tttaaaatgt tttcctaggc attctctcat tcagtcgtta cctcaaagct 420
   ttgaagggag tataattatc ttcatttcac aaatcatgaa gctaatgaca tttcatggaa 480
   gctattttta taggaattat ggaattggag ccctcctttg agtgtctttg gatgttgtca 540
   ccaccatcaa caggcagtgt tatctccaac atgtttgatg agaaaaaaac ataaaggaaa 600
   gggacttggg tttcatggca agagattcag cttagatggg aatgagctgg tctctgaggc 660
   agagtttcct gtctcatctt tgtataaact ccttcttaga aaccagcaga ggctcttaat 720
   gtacgtccat cttctccttc ccaccacaga cacccactcc tatgtgtgtt tctgaaaatt 780
   acagggtcat ttgtggatgt gctttaattt agtatagata taatctgata tcattatctt 840
   tctttccccc cgctccccgc caccagcatt ccccctgcac tcaatttgtg cttatattcc 900
   ttttgctgta tatatacccc tggctcattg cttctggaag ctgcattact tattctagag 960
   tgtgggtctc ctacaattta tttatatagc cccctaaatc agtggttctc aactgggggt 1020
   gattttatgc tccccaggtg tgatttggca gtgtctggag acatttttgg ttttcacaac 1080
   tggaggtggg cactgctggt acctaggtgt atagatgcca aggaggctgc taaatatcct 1140
   accatgccca ggacagcccc cattacacag aatagccagt cccagagaaa ctcagcctta 1200
   gattagtgtt tctccttcct ggctgcacat taaaattatc aggggagctt ttaaaataca 1260
   ctgatgctgg gatccacccc tgaccaatta aatcagaccc tctgggatgg ggcctgggct 1320
   ttagagtgtg tttttaacac tccccaggtg attctaatgt gcagccaggt ttgagaatct 1380
   ttactctaag gactcaatct ttgaaagtta cagcattgta gaggggaagg atgatccaga 1440
   attaattaaa gaaggaagaa gagtgaacag tactgaactg gatgcaagat tatgactgtg 1500
   caaatcctcc tatttcagat ttaggttctt tactggtaaa ttggataaaa agttggcagt 1560
   tcttttactc actggagcat gatgaagata aatgagttac ggttgcccta gacttttacg 1620
   attagtaaaa ccaatggatg gacagaataa ataagtataa gcagacgcct atagtctaat 1680
   atcgcaactc cagaaaatct ttgaggaaat ctcacacatg atgggagtgg gacagggagt 1740
   tgttttgttt ttgcttccct tgagtctcag caaaagaaca gtcatagaaa atatttccaa 1800
   aaaatttaga tataattttg cttggcaatt agaatatacc ctctgtcagc tttgttcagt 1860
   tgttggacct attaaccaat ccataccaga ctaatttatt tctaaacaaa gtggcgaatg 1920
   gtgtccaatg ccttttctga gaaagagaaa aatggcctct tcaaaagacg caatttggta 1980
   tagacttgtc tggccgtttt gtagtgttct ggagtgggga gcatatacta ccctcacagg 2040
   taaacgaaac cttacctgta tgtattgtct cacccatatc ctgctacagg agaaaaaaaa 2100
   aaaaaaaaaa aggccacatg tgctcgagct gcag                              2134
<210> 70
   <211> 2146
   <212> DNA
   <213> Homo sapiens
<220>
   <221> misc_feature
   <222> (1)...(2146)
   <223> n = a, t, c or g
<220>
   <223> nbla-00831
<400> 70
   ggcctactgg ttctgcaaga tgtcaagatt gttgaacacc ccagatggaa gcccttacac 60
   ttggtgggtt ggcaaagcca acgagaagca ctactgctgg ggaggctctg ggcctggaat 120
   ccagaaatgt gcctgcggca tcgaacgcaa ctgcacagat cccaagtact actgtaactg 180
   cgacgcggac tacaagcaat ggaggaagga tgctggtttc ttatcataca aagatcacct 240
   gccagtgagc caagtggtgg ttggagatac tgaccgtcaa ggctcagaag ccaaattgag 300
   cgtaggtcct ctgcgctgcc aaggagacag gaattattgg aatgccgcct ctttcccaaa 360
   cccatcctcc tacctgcact tctctacttt ccaaggggaa actagcgctg acatttcttt 420
   ctacttcaaa acattaaccc cctggggagt gtttcttgaa aatatgggaa aggaagattt 480
   catcaagctg gagctgaagt ctgccacaga agtgtccttt tcatttgatg tgggaaatgg 540
   gccagtagag attgtagtga ggtcaccaac ccctctcaac gatgaccagt ggcaccgggt 600
   cactgcagag aggaatgtca agcaggccag cctacaggtg gaccggctac cgcagcagat 660
   ccgcaaggcc ccaacagaag gccacacccg cctggagctc tacagccagt tatttgtggg 720
   tggtgctggg ggccagcagg gcttcctggg ctgcatccgc tccttgagga tgaatggggt 780
   gacacttgac ctggaggaaa gagcaaaggt cacacctggg ttcatatccg gatgctcggg 840
   ccattgcacc agctatggaa caaactgtga aaatggaggc aaatgcctag agagatacca 900
   cggttactcc tgcgattgct ctaatactgc atatgatgga acattttgca acaaagatgt 960
   tggtgcattt tttgaagaag ggatgtggct acgatataac tttcaggcac cagcaacaaa 1020
   tgccagagac tccagcagca gagtagacaa cgctcccgac cagcagaact cccacccgga 1080
   cctggcacag gaggagatcc gcttcagctt cagcaccacc aaggcgccct gtattctcct 1140
   ctacatcagc tccttcacca cagacttctt ggcagtcctc gtcaaaccca ctggaagctt 1200
   acagattcga tacaacctgg gtggcacccg agagccatac aatattgacg tagaccacag 1260
   gaacatggcc aatggacagc cccacagtgt caacatcacc cgccacgaga agaccatctt 1320
   tctcaagctc gatcattatc cttctgtgag ttaccatctg ccaagttcat ccgacaccct 1380
   cttcaattct cccaagtcgc tctttctggg aaaagttata gaaacaggga aaattgacca 1440
   agagattcac aaatacaaca ccccaggatt cactggttgc ctctccagag tccagttcaa 1500
   ccagatcgcc cctctcaagg ccgccttgag gcagacaaac gcctcggctc acgtccacat 1560
   ccagggcgag ctggtggagt ccaactgcgg ggcctcgccg ctgaccctct cccccatgtc 1620
   gtccgccacc gacccctggc acctggatca cctggattca gccagtgcgg attttccata 1680
   taatccagga caaggccaag ctataagaaa tggagtcaac agaaactcgg ctatcattgg 1740
   aggcgtcatt gctgtggtga ttttcaccat cctgtgcacc ctggtcttcc tgatccggta 1800
   catgttccgc cacaagggca cctaccatac caacgaagca aagggggcgg agtcggcaga 1860
   gagcgcggac gccgccatca tgaacaacga ccccaacttc acagagacca ttgatgaaag 1920
   caaaaaggaa tggctcattt gaggggtggc tacttggcta tgggataggg aggagggaat 1980
   tactagggag gagagaaagg gacaaaagca ccctgcttca tactcttgag cacatcctta 2040
   aaatatcagc acaagttggg ggaggcaggc gatggaatat aatggaatat tcttgagact 2100
   gatcacaaaa aaaaaaaaaa aaaggccaca tgtgctcgag ctgcag2146
<210> 71
   <211> 1878
   <212> DNA
   <213> Homo sapiens
<220>
   <221> misc_feature
   <222> (1)...(1878)
   <223> n = a, t, c or g
<220>
   <223> nbla-00832
<400> 71
   ggcctactgg aaagaaaaaa tgtgctgcgt tctgaaaaat aactccttag cttggtctga 60
   ttgttttcag accttaaaat ataaacttgt ttcacaagct ttaatccatg tggatttttt 120
   tttcttagag aaccacaaaa cataaaagga gcaagtcgga ctgaatacct gtttccatag 180
   tgcccacagg gtattcctca cattttctcc atagaagatg ctttttccca aggctagaac 240
   gacttccacc atgatgaatt tgctttttag gtcttaatta tttcacttct ttttagaaac 300
   ttaggaagaa gtggataatc ctgaggtcac acaatctgtc ctcccagaaa tgaacaaaag 360
   tcatcacctt ttctgcttgc tacacaggca acgattcccc catcagctgc ccggaccctt 420
   tggcctggct tggtgtgcag gcctgtctgt ttgcttaaag tcagtgggtt ctggtgcagg 480
   gagtgagaag tgggggaagt gaaagggaaa gcatccgtga gaaagcggcc acggttttcc 540
   ctccttgtgt gcccatgggg caccagctca tggtcttttt cagtcatccc agtttgtaca 600
   gacttagctt ctgaactcta agaatgccaa agggaccgac gagactcccc atcacagcga 660
   gctctgtcct tacatgtatt tgatgtgcat cagcggagga gaacactggc ttggccctgc 720
   tccgctgagt gtctgtgaaa tacctctact ttccctccca tatccagaac aaaatgatac 780
   ttgacatcct tccacaaaag tcagcctaaa gaagttatgg tatcatatgt taaactaagc 840
   tttcaaaaac cttagtgaaa tagcaagtga ctgctttcaa gcagcagtcg acatgtaaat 900
   gaaggtgttc ttagaattcg cattttgcca gctcagcgca cctccacaac gaatgaaatg 960
   ctccgtatga tttgcacaaa tgacatagac ctccccaaaa gttaactggc tctccttcct 1020
   cacacagttc atcataaccc aaccccccac ccccgggtca tgaaaatcac agaacttata 1080
   aacacattga accctagatc tcaggcttcc tgacctaccg ccagtggccc cttgctggcc 1140
   accctatagg gtcctccttc cctggcagcc ccccatgtgg gagaaatacc tgattctccc 1200
   aatctgcagt gggagagctt tgctgaattc catcccaaag tcaaacatgg gcaagaggtg 1260
   aggatttcac ttttaccctc aagtccgatt tgtctgtgat tttaaactaa ctgtgtatgt 1320
   attgatgttt ggaagattgt ttgaatttta aagcgataat agtacttaat gttatccagt 1380
   attgttcatt aaatggtgtt atcctaaagc tgcacttggg atttttacct aacgctttac 1440
   tgattctctc aagcacatgg caaagtttga tttgcactcc gttcatttct gacacgtttt 1500
   gctgcctcct acctttctaa gcgtcatgca aattcgagaa tggagaagga cgctgccggt 1560
   ccctgagcgg tgtggagagg gcggaaggtg gactccagcg cagcttgagg ggctgaggac 1620
   ggaggctgca gcatctgtgc cgttctactg agcacgcttc tctgcctcgc tcctgactca 1680
   gcactttgtt cactggctca gcagttatgt ttacacatca tttttatgtt cctgctttgt 1740
   aattcatgtt tgagatgggt ggccactgta cagatattta ttacgctttc cagactttct 1800
   gaatagattt ttttgaataa acatggtttt atgaaaaaaa aaaaaaaaaa aaaaaggcca 1860
   catgtgctcg agctgcag 1878
<210> 72
   <211> 2291
   <212> DNA
   <213> Homo sapiens
<220>
   <221> misc_feature
   <222> (1)...(2291)
   <223> n = a, t, c or g
<220>
   <223> nbla-00908
<400> 72
   cactgttggc ctactgggaa atacattttg tgtgcatatt gtttcatagc aagaattcgt 60
   tgcaaaaatg ctttattttt gaacaatgct tggaaatatt atgtgacttt tttgtttgtt 120
   tgttttagga ggatggtgta tggtgggggc aataaatgag gttttttgca ttccaaggaa 180
   atggcatatg gattaactgt aagaaatgaa ataagtaatt tattgtaaga caacatcaag 240
   ccatggaaac ttggcagaag attcaaagca gcttaaacag cacttttaaa ttaactccta 300
   agcgttacat ggttgtgact atggaaactc cagttaagac aggatcttat cagaggtgga 360
   cagcgtgaag atttcctttt ccattttcaa taaactttgg aacaaccttc tcgtatctcc 420
   cctagagttt cgtgcccctc tgaactgtct gttattgcaa tgtagtttat caacagaatt 480
   tgtgtgtttt cgatttaagc taaaagataa tttaagaaca tttatttccc cttttcactt 540
   taaaaaatta tgattattcc tattattgtt atgaaccttc ttattttaca tttgagggat 600
   aaaggcaaat gatttgtgag tcttctagtt actggaccga gttttctgct ggatctggtg 660
   ggaaggcagc tcggtaaagt ttccctcctg ctccccccgc ccgactttga ctctgaatca 720
   gcatttggtc ctattcagag gactcttacc acaacgtttc tgttctacac ttgggtggag 780
   accagttgac catagagcat ttgcagagcc tcattgtttg atttcttgtg actattctaa 840
   gaatgaatgc aatcagattt taaaagtaac taaatatact tcagcacttt tttgctttaa 900
   actagatcat cttagacttg tttatacctt ccagatttga ttgttttact cccaatgact 960
   gcactatatg tatgcataag accacttttg agcgctgtgt tcccccttct gagtagtcct 1020
   ttgacgacgt gttgtgtttt ctgatgttga cttgagttcc atttagtagc atctcttcct 1080
   tccatgtctt gatgttatgc aggaagtaca gacgtacttt aaatttttgt tatgaaataa 1140
   aaaaaagatg ggttttgtaa aaataaaaaa aaatattttt agcagaacag gacttacagg 1200
   gtcattgtcc ccacaatgtg ccagtcgact atttgcactt accttgtcct atatatccgt 1260
   acggaggtgt gcaattcctc gtgtcagtag ccttgtgaca ctgaacctgg atggattata 1320
   gaggagccct cacggctgat caataatgtt gcaaagggag actacaggga tctcacgacg 1380
   aatattctga tacaatactc aacctcggta tatatatatg tgtataaata tatgtatatc 1440
   ccagcggcac tttatactgt tcactgtaca aaagcttaca gttttccaca aggactttaa 1500
   taactagctg ggaaaagacg atgtaattat ttcggggctc tgcggaacca tctctgtaca 1560
   gcgccccctt tctgttgtgc tattggttgc agctgccatg ctcagaatgc gttttgagag 1620
   ctgaagcaag gtgcttgcag tcacctgagg ccgtccgtgt ggcccagggc cccagctgcc 1680
   tttagggccc ccattgttca taacagcata tgcatttccc caccgcgttg tgtctgcagc 1740
   ttctttgcca atatagtaat gcttttagta gagtactaga tagtatcagt tttggattct 1800
   tattgttatc acctatgtac aatggaaagg gattttaagc acaaacctgc tgctcatcta 1860
   acgttggtac ataatctcaa atcaaaagtt atctgtgact attatatagg gatcacaaaa 1920
   gtgtcacata ttagaatgct gacctttcat atggattatt gtgagtcatc agagtttatt 1980
   ataacttatt gttcatattc atttctaagt taattcaagt aatcatttat taagacagaa 2040
   ttttgtataa actatttatt gtgctctctg tggaactgaa gtttgattta tttttgtact 2100
   acacggcatg ggtttgttga cactttaatt ttgctataaa tgtgtggaat cacaagttgc 2160
   tgtgatactt catttttaaa ttgtgaactt tgtacaaatt ttgtcatgct ggatgttaac 2220
   acatcttact ctaaataaac aaggtgttgc caaaaaaaaa aaaaaaaagg ccacatgtgc 2280
   tcgagctgca g2291

<210> 73
   <211> 852
   <212> DNA
   <213> Homo sapiens
<220>
   <221> misc_feature
   <222> (1)...(852)
   <223> n = a, t, c or g
<220>
   <223> nbla-02890-f
<400> 73
   gagnagnnna gntggngngg anantgnnat ttgantcctc tncctgttgg cctactggag 60
   atgcttctgg gtcgcggtgt gctaagcgag gagtccgagt gtgtgagctt gagagccgcg 120
   cgctagagcg acccggcgag ggatggcggc cacngggacc gcggccgccg cagccacggg 180
   caggctcctg cttctgctgc tggtggggct cacggcgcct gccttggcgc tggccggcta 240
   catcgaggct cttgcagcca atgccggaac aggatttgct gttgctgagc ctcaaatcgc 300
   aatgttttgt gggaagttaa atatgcatgt gaacattcag actgggaaat gggaacctga 360
   tccaacaggc accaagagct gctttgaaac aaaagaagaa gttcttcagt actgtcagga 420
   gatgtatcca gagctacana tcacaaatgt gatggaggca aaccagcggg ttantattga 480
   caactggtgc cggagggaca aaaagcaatg caagagtcct ttgttacacc tttaaagtgc 540
   tcgtnggtna atttgtaagn gatgtcctgc tanttncana aaantgccna gtttttcaca 600
   aanagcnnat ngangtntnt tagaatnaca nncttgnaca ccgnnntnaa aanncntntt 660
   ttnntnangg antnaccttn ttnnnttccg nttctgttcc ntttggntta accnnntnnn 720
   tngnntnnaa tatntnnnnn nnccntnann annaanttnt gttttnnnna nataannntn 780
   atnnnntnnn tnnntnatnn nntntnnnan nnntnnnntn nnnctntntn nnnntntntn 840
   tntntntnnc cn                                                 852
<210> 74
   <211> 862
   <212> DNA
   <213> Homo sapiens
<220>
   <221> misc_feature
   <222> (1)...(862)
   <223> n = a, t, c or g
<220>
   <223> nbla-02890-r1
<400> 74
   gaggngttgn anntttgtgg ccttttttnt tnttcttttt ttggtggaag ttaaagtatt 60
   tattgatgtg tttaaactgt gtacattctc cacagatcat attaaggagt ttgtaggtga 120
   agtttaatct gtgcataggg ggtagagaca tgaatagggt caaaggggag gaaaaaggaa 180
   aaaaacaaaa caaaaacagt cacaggaaaa taaaaataca ccacaggtta ccagaacctt 240
   cagatttaaa ataaaaagaa agaaaaagca gaagcagtga gcatcgacat caactgtaca 300
   agcattacaa agactcctgt gacgaaaaca caattgttca aagctttcca cagtgtccac 360
   agactcagtt tcagggacag cctgnactac nttcctttca cacaaacaaa cctncccgtg 420
   ttccctcctg ggccagcggc cgncttcttg gtgtggtctt ctttgagtga atgtcacaan 480
   ggcccgtnac atggaagggg ttggnngtng atgggcaaag tttangccca nggntnaant 540
   gccttttnan aaaagnccan anangatgaa ccctgcncnt ntggacctta nttgnntggg 600
   gnattgaccg nttaaaanct tccnnnnnng tnatcatctt ncntttnnat ntcaaattta 660
   ancttcnngn tnatnncttn ngaatcctgn nttctnnnna nnttcntttn tgnctccctt 720
   antttttncc tttntttttn aanactntgn attnatncnt ttntcgtccn tngntnnttg 780
   actttttttn nacccnctgn tttcntcctt ntaacttnnt ntttnnaann ggnccttnan 840
   ntcttttnnt ttntnnntna nc                                      862
<210> 75
   <211> 851
   <212> DNA
   <213> Homo sapiens
<220>
   <221> misc_feature
<222> (1)...(851)
   <223> n = a, t, c or g
<220>
   <223> nbla-02969-f
<400> 75
   nnnnttnnnt ttgaattccc gttcctgttg gcctactggc aaattagaaa atgaattgag 60
   gaccacaaag agtgaaatgg cgcgatacct aaaagaatac caagacctcc tcaacgtgaa 120
   gatggctttg gatattgaga ttgcagctta caggaaactc ttggaaggcg aggagacccg 180
   actcagtttc accagcgtgg gaagcataac cagtggctac tcccagagct cccaggtctt 240
   tggccgatct gcctacggcg gtttacagac cagctcctat ctgatgtcca cccgctcctt 300
   cccgtcctac tacaccagcc atgtccaaga ggagcanatc gaagtggagg aaaccattga 360
   ggctgccaag gctgaggaag ccaaggatga gcccccctct gaaggagaag ccgangagga 420
   ggagaaggca aggaagaagc cgaggaagaa gaagcagctg aagaggaaga agcttgccaa 480
   ggaagaatct gaagaaacaa aagaagaaga agaaggangt gaaggtgaag aaggagaagg 540
   aacccaagaa gcttgaagag gaggagaaag aaangttgaa ggtgcttggg aaggaccagc 600
   nactttagaa gaaanattgg accccccntt tccttaatta tttcagggat aatcttcccg 660
   aattaggtna accccattan ccaaccaacc aacccnttga gnttccnaat tttttgngga 720
   attaaaaagc natttttgnt tactttgnaa tgcttaggtt ggcnattcaa ngtgggcttt 780
   naattccnct ttntccaaan tttgntnctt caaaaggctt ttggcttntt ncaacctngc 840
   atggncaccc t                                                851
<210> 76
   <211> 817
   <212> DNA
   <213> Homo sapiens
<220>
   <221> misc_feature
   <222> (1)...(817)
   <223> n = a, t, c or g
<220>
   <223> nbla-02969-r1
<400> 76
   ggnggngntt tnctnnngan gcccttttga aannccctta ggttttttac tattttatta 60
   tggcacacag gatagaggat ggtacagttt tcttacttca accaagtaat tctcaaagca 120
   tccagctatt tccatttggc taaagttact ttttgcacat agcttgcatc tgtttgagac 180
   ttaccatgta catcaaccca ggtctagtaa gcagaaatgt gaaaagtttt gtttctgagg 240
   agacgcctca tctttacaga agccaataca ctgagagcct tcatagttcc aatccattac 300
   catcatggca aggaagcact ttacctattt gcatagcaac atatatttaa ctagaaatag 360
   gtggtcaaag ggattaagta actttaaatg gagaccactt tggtttcagg ttaaattaat 420
   aacttataga gatcgctaaa aaacaaatat tgaatgaaat tagctgacaa agcaattggt 480
   tcaagaacaa aggcagaata gcagatagta atatcatcta tatttattnc acatcaatgc 540
   cagaaccgtc ttaactttac gacngaaagg atcntggggc cgggatttga tgcaatgntc 600
   aancnggcaa gctntcattg gaatccaatn tttccagtng ancatgccna caatggcaag 660
   gacntacagc ctttaccttc cccttaaggg ctataattca ttactgggct ngaccnttaa 720
   aggttaaaag gggnncntta aggggnttac tgnggaccna ttcccttana cccctaatnt 780
   ggcccaggat gggggactnt tccnggangg naanacc                    817
<210> 77
   <211> 866
   <212> DNA
   <213> Homo sapiens
<220>
   <221> misc_feature
   <222> (1)...(866)
   <223> n = a, t, c or g
<220>
   <223> nbla-03002-f
<400> 77
   gngnnnnnnn nggngnnnnn nnnnnggcng natcccgagc acgtgggcct actggaaaac 60
   ttatgagtgt gttggatgtg ggaaatcctt taggcacagt tcagcacttc ttcgacatca 120
   gaggcttcat gctggagagt aaaatttgga atataatgag tatgggaaga tttgtatgga 180
   agcacctttt ttttctccta aattcctagg aatgtaagac tcaatctgta gctagtatga 240
   atattttgta ttttgaacaa gaaatgttgt gtcctaatgt gtcctagtct ggagtttgat 300
   gccttatctg ctgctcagtg ggtgtaacct tatcccttct gagcctcagt ttccccattc 360
   cccccaatag aaaaatgggg atatttccta gggttgtcct gagaataaaa tgagttctgt 420
   gtgacagtta actgccatta attaattagc cgttctaaaa gtttatttac aatatcaaaa 480
   tctgacaagt aaatcagcat tgttgtttgg tctcataatg tattgtttct taagaacatt 540
   ttgtaattgg gctatgattt tatgatctac agacgtctat ttaaactata atgtcctggn 600
   ttaaatagga taatacaatc taagactggt ttgtccttga gaaatttcag atttaatcag 660
   agaaaaatgt actgattggg atttgagtgg acattgtggg ggtgtattct ggatancctc 720
   aagatctcaa ttaaantttg ntacnttcca atactagatc taaattgatt ttaagctcca 780
   agttcacttt tccaattaaa cgggaagcct ccctgacata actgaataaa acctcanggt 840
   tgtgggactc cgggtgngtt taaggt                                      866
<210> 78
   <211> 872
   <212> DNA
   <213> Homo sapiens
<220>
   <221> misc_feature
   <222> (1)...(872)
   <223> n = a, t, c or g
<220>
   <223> nbla-03002-r1
<400> 78
   gngntgnnnn tgtggctttt ttttngnttt taagacgtag tctcgctctg tcgcccaggc 60
   tggagtgcag tggtgcaatc tcagctcatt gcaaccttca tctcccaggt tcaagcaatt 120
   tctcctgcct cggcctcctg agtagctggg accacaggtg ccctccatca tgctggctaa 180
   ttatttttgt atttttagta gaggtggggt ttcactatgt tgaccaggct ggtctcgaac 240
   tcctgacctc aaatgatcca cccacctcgg cctcccaaag tgctgggatt acaggcatga 300
   gccactgtgc ccagcctgag aattacctta taaatatctt gcacatgtgc ctaaagacaa 360
   ggatgctgta tatgcagttt tgcttacaac agaaaaataa aagccattaa gtgtccacca 420
   atagggaact aataatggtc tattaagaca atgacacact gggcaagggt gggggaaaga 480
   tcttatttac tgatacaaga acaatctgtg agatggttaa tccttaaaaa atggncaact 540
   gtgtatatta tgctaccatt tcnttaaaaa aagaaaaaag aaaaaaaaag tnaagagaag 600
   ggatcaagga ttgagaaaat aagccacaac ctgggangga aatatttgca aaagacctat 660
   ctgataaagg nctgttatac aaaatatnca agggacactt naaattcaac aataagaaaa 720
   tgagcaatcc aattnaaaat gggcaaaacc tccaacaagt acctcaccaa aagctttcag 780
   atggnaaaca tntgaaaaga tgctaaattc aaatattant ggggaactgc agnttaaaac 840
   aattnatncc actacattcc tatttnatan tt                                872
<210> 79
   <211> 853
   <212> DNA
   <213> Homo sapiens
<220>
   <221> misc_feature
   <222> (1)...(853)
   <223> n = a, t, c or g
<220>
   <223> nbla-03037-f
<400> 79
   ggnngnnnnn nnggnnnntt tnnnngggcc nggatcctct agcactggcn gcctactggg 60
   ccttcgcgcc ggcccttccg cgggtgatca gctggtctgc gctcccctga cgtgggctgg 120
   ggcacgtcac cgccgaatgg cagcctccag aaagccaccg cgagtaaggg tgaatcacca 180
   ggattttcaa ctgagaaatt taagaataat tgaacctaac gaggtgacac actcaggaga 240
   cacaggtgtg gaaacagacg gcagaatgcc tccaaaggtg acttcagagc tgcttcggca 300
   gctgagacaa gccatgagga actctgagta tgtgaccgaa ccgatccagg cctacatcat 360
   cccatcggga gatgctcatc agagtgagta tattgctcca tgtgactgtc ggcgggcttt 420
   tgtctctgga ttcgatggct ctgcgggcac agccatcatc acagaagagc atgcagccat 480
   gtggactgac gggcgctact ttctccaggc tgccaagcaa atggacaaca actggacact 540
   tatgaagatg ggtctgaagg acacaccaac tcangaagac tgggctggtg agtgtgcttc 600
   ctgaaaggat tcaaggttgg tgttggaccc ccttgatcan tcctacagat tantgggaag 660
   aaaatggcca aagttctgaa aaantgccgg catcaacctc antcctgtca aaggagaanc 720
   tcgttgacaa aatcctggca anaccgtcct taaacgcccc ttgaancctc tcctcaaaat 780
   ggggcctggn tttnaaaagg aatctnctgg aanggcaaag gtncaaaccn ttcggttnaa 840
   aatggttaaa agn                                                    853
<210> 80
   <211> 836
   <212> DNA
   <213> Homo sapiens
<220>
   <221> misc_feature
   <222> (1)...(836)
   <223> n = a, t, c or g
<220>
   <223> nbla-03037-r1
<400> 80
   ggntgnnnnn tgtggctttt tttggccttt tcacagttct gacnttttta ttcactgatc 60
   ataaatatag tctcatgagc attaaggtga tcatgaatga tgtactagca cctggaacat 120
   gaccatcatg gagcactcac tgattcccca tcactggcca aagaagtcgg ggcatcttcc 180
   tttcaccaag tgttcaactt tggagggacc ctccttctgg tgcagcaatt ttattcttgg 240
   cttgttctca acaattaaaa aatcataaaa gactgagtgt ttgcaataaa atatcaaaga 300
   ggataagaag attttctgtt cttcttaaag tctaaagtaa aaaggggagg tagggaagaa 360
   ggaaaggaaa ggggaaagat gtcagggatc tgccacgttt cttccttcct ccagagcatt 420
   ttacaaaaac aaaaccgggg aggtatttat taatgctgtt tggagatggg ttgcgtctct 480
   ctgatgagcc actcgagagc ttcctggcgg ccctgtttct gcaattcctt cccaatcaca 540
   tccctgcang tcaagtggta attgttgagc caatcgcact ccttgtccgt aagagaatcc 600
   acatctatca atttggtctg aattggganc aatgttanan ggtcaaaggn caagcttccc 660
   cgggntatta aaaattatac ttggncctca aanggaccac aagggcaaca atctcaatgc 720
   gaanttcaaa agcccaacct caaaataacc cggntcaaca antggcaaat aatgccggcc 780
   tccaanggnt caacaaanaa atgttttgta actgatgccc caaggncccn catggn     836
<210> 81
   <211> 1895
   <212> DNA
   <213> Homo sapiens
<220>
   <221> misc_feature
   <222> (1)...(1895)
   <223> n = a, t, c or g
<220>
   <223> nbla-03066
<400> 81
   gaagtgttac ttctgctcta aaagctgcgg aattcctcga gcactgttgg cctactggaa 60
   accaataatg attgttctgt ttttctcacc ctactatgtg aatatatatt ctgttagaga 120
   tgggctgtta tctgcaatgt ggactcaata ccatgggctt gagtttggtt agttcatcat 180
   tagagatttt ttttcagcag ttggtttact ttatgagaaa ggatcatgag tttaattttc 240
   ccactgatat atttgtgttc tcattgctga gatataagtt tcttaatgat ttgttacctt 300
   tttctatttt cctagactag tattaaaatt ttgatggcat atgggtttgg ttccacttgt 360
   agagaataaa tgttcacaac ttactggtat tagcacacac acatcacatg cagaaatggt 420
   gttttcttgc cacttactct ttctcataag atgcttatat tcctgtgggc atttgtcttc 480
   cacactacag cacataattt gatgaaactg attttctgtg caagagcttg tgtttctcca 540
   actctggttt tatggaccac ttttcattgg tatataggag gcggttaaaa cgtggtggta 600
   gaatgcaggt tttaggttag ctgggtgtga agaatcccag gtctttggga tagtcttggc 660
   aagttactca actcctctaa gttagtttct tgatctgtca gatgacagta aacctcagag 720
   ttgtgaaaat taaatggata taatgtgtgt aaagcactta gcataatgct ttatacatcg 780
   taaaactttc aaaaatagga gtagtaatta ttacaaagaa atgtgaagaa gagtcctagt 840
   gggctgcatg ttaaaaatag tttttagtta atgaaaatta aatcacatct tagatgttta 900
   tcatctgcat ggtacttttc tggttgctgc tttaaccttt gtatagttga aagtctgtaa 960
   actttctagt attctttgat ttctggccca tttccctcct accattcacg cttatatttt 1020
   ttggttcaag tcaccattcc ttgcaaaact aattaaatac attcctaaat acataatatt 1080
   gccaatattc attcacatcc accacgtcta gatccaagtc ttcatcttct ttatgctggt 1140
   ctaatttttg ctgagcctta gaagcaaaga cagaccccgg gggttgatat gggacctcca 1200
   gaatggggat tgcacagaat aatcttattt ttcctctatt cttctagtct gttatgttct 1260
   gaacccacac atgttttcgg aaggaccaga tggctgacac tggctaatgg gaaccaaaag 1320
   acgagtgaaa gtaacctggt tgtcacatac ccccagctta gaggtgcttc accctgcccc 1380
   aaggtcaatt tcttttttct ctagagactt taccccatta tatcaaaaac ctttactaca 1440
   gcctgacttt atctgatgtt agagtatcat taagtcaagc agcagcattt attgagtgaa 1500
   aaaagattgt acacaggact ggaagagaag catctgcctt taaaatacag tagaaggccg 1560
   ggcatggtgg ctcatgcctg taattctagc actttgggag gccaaggcag gtggattgcc 1620
   tgagctcagg agttcgagac caccctgggc aacatggtga aaccctgtct ctcctaaaaa 1680
   aaaaaaaaaa aaattacaaa aaatttgccg ggcatggtgg tgcacacctg tagtcccagc 1740
   cactctaaag gctgaggtac aagaattgct tgagcccagg gtcagaggtt gcagtgagtg 1800
   gagatcgcgc cgctgcgctg cagcctgcgc gacagagcga gactatgttt tttttaaaaa 1860
   aaaaaaaaaa aaggccacat gtgctcgagc tgcag1895
<210> 82
   <211> 842
   <212> DNA
   <213> Homo sapiens
<220>
   <221> misc_feature
   <222> (1)...(842)
   <223> n = a, t, c or g
<220>
   <223> nbla-03096-f
<400> 82
   gggnngnntg ntgncgcntt ttnnnngggc ccgantcctc gagcangggt ngcctactgg 60
   tgtcatgtag agcttcacat gaggtgggtt ttgtcgatta cgcccccttg gaggtgttta 120
   tcatgtgcct ctgtccccgg tatttcctat aaattgacag ttacatctag gtgcctggtc 180
   agattctggt tcagctttta cttttacttt tttggcaaga gcagttcacc aagggaggtg 240
   ctaatgtcca actcgccttc tgtgatgcca gcagcttcct gatcagaggc tgtggaattg 300
   actgtattgt gttgatgttt tataaagagt cattcattac cttttagtct tgcgcttgct 360
   ggtagaacaa gatgttgtag gctcgttgga aacatttttc cccagacccg gtcatttatc 420
   caaagacctc cagatcttct cagtgggaat gacatgagga gaccacaact gggctctctg 480
   tgttcagttt tttccatgga caggtctaga aaccatgtat tttacgataa aagacactgg 540
   acttatacag atgcttccaa gttcaacatc aagactagaa gactattaaa aaattgcaaa 600
   taactctatt tctgccaaaa gtacttttgg gacagattcc taaaagtgga attgctggtc 660
   aaagggcaaa cgtgtctatg acctcgccag atactgccaa gttcacatga tgaggtcatg 720
   ccanttgccg caanccatgc cacgggagaa ttcanccctc cccaacttct ccagctaaac 780
   ttctgantta ccaagaggca ntatccaatg ngcctttaan tcaaanttcn ccggtaagtg 840
   tg 842
<210> 83
   <211> 839
   <212> DNA
   <213> Homo sapiens
<220>
   <221> misc_feature
   <222> (1)...(839)
   <223> n = a, t, c or g
<220>
   <223> nbla-03096-r1
<400> 83
   gagntgnngn tgnngctttt anggnccttt tttttttgaa aanttaaacg tactttaaac 60
   tttgcatgag aagaaaacaa gaagcaaagt gaaaagtcct gtgacagact ggaaaaaata 120
   ttggcaatta tcaaaatgaa aggttgctat ttaatatatg gtttctaaag taaagaatag 180
   cataattttt atccctatag aaaaaaaaat ctagagatat caaccagtag ttcacagaaa 240
   aagaaatcca tcacagtagc caagatatgg gatcggtcac agatgagggg ataaacagaa 300
   catggtccgt atccacagca gagggctgta cagcctggtc tgtttgcaca gcggagtgcg 360
   gtacggcctg ctctatatac acagcggagt gcagtatggc ctggtctata tgcacagtgg 420
   agtactgtac ggcctaatct atatacacag cggagtgcac tatggcctgg tctatataca 480
   cagcggagtg ctgcatggcc tggtctacgt acacagcgga gtgcantacg ggctggtcta 540
   catacacaac ggagtgctgc acggnctggt ctacatacac agtggagtgc aatacggcct 600
   ggtctacata tacaacggag tgcaatacgg nctggtttct atacacancg ggagtactgt 660
   ncgggctggt ctatatacac agcggagtgc tgcacggnct ggtctacatg cacaagcgga 720
   gtactgtacg ggctggtcta natgcacaag gggggtgctg tgccacctgg tccatatgca 780
   cancaaaant gctgtgttgn ntcaaccnat atacacacaa atngnggtgn cggtnnntc839
<210> 84
   <211> 846
   <212> DNA
   <213> Homo sapiens
<220>
   <221> misc_feature
   <222> (1)...(846)
   <223> n = a, t, c or g
<220>
   <223> nbla-03107-f
<400> 84
   ggnnngnnnn gngnngnntt tnnnngggcc cgnatcctcg agcactgntg gcctactggg 60
   agtagctcag ctcctattcc tgggaagcct ggaacgggga cttttgaaaa taactgcagc 120
   ggcattcggg ttagggtccg tgctctccgc ctgcgccagg acagggtgaa gtggtcgggg 180
   cgagcagagg gtgcgaaggt gcgggtgctg gtgcctcgca gcaggaggga gccccggctg 240
   cgccgcgcga ctccctcttt ggccctcgga gcgcagcacc cggcggacaa gcggcgggac 300
   gccaggacgc ggcgagcaag atctctcgtg gaagaggaag accaacacat gaaattgtcc 360
   cttggaggca gcgaaatggg cctctcatcc catttgcagt cttccaaggc aggacctaca 420
   cgcatcttta ccaagcaata cccacagttc tgtggtgtta cagggctttg accagcttcg 480
   acttgaagga ttgctttgtg atgtgaccct gatgccaggt gacacagatg atgctttccc 540
   tgtgcataga gtcatgatgg catctgctag tgattacttc aaggctatgt tcacagaatg 600
   aaagaacaag atttaatgtg cattaaactt catggtgtga gcaaagtcgg tctaaggaaa 660
   attattgatt tcatttatac tgcaaagctt tctcctaata tggacaacct tcaagacacc 720
   tggaanctgc caatttccta cagattctgc cagttttgga cntctgtaaa gtgttcccaa 780
   aaccggggtc actttaacaa ctgtgttnaa tttggccggn ttgcaaanac tacaaatcta 840
   accgnn                                                  846
<210> 85
   <211> 836
   <212> DNA
   <213> Homo sapiens
<220>
   <221> misc_feature
   <222> (1)...(836)
   <223> n = a, t, c or g
<220>
   <223> nbla-03107-r1
<400> 85
   ggntgnnnnt gggctttttt tggnctttga cattaaaagt ttttattggn cacaaaaaga 60
   taaaacatgg aagttgaatt tactgagcaa aagcagctct ccaggtgaag ctgctatact 120
   ttgtgctaaa taaccttatg aactgagtat acagaataca tataatatgc aagttacctc 180
   aacagcaaag gagaaggagt agaatacagt ttttgaagat aaaatctggt caagtgacaa 240
   attttgttgc tcaaaatttc tagcccttat ccacctaaat tctgtatggt tctacatata 300
   tgcattcagt atgtgcatac tgaattccca ttttaatgga agctgctttt tggaagaatt 360
   ctttttaatt tcacatttct ttgatgtgcc actcaatttt taaaaaaatt atatttgaca 420
   tatgtgcatg tgtgtatgtg tatgtatgta tacacacttt aaaaacacca aacccttgtt 480
   tataagtaga gggttcatgc tgctttttaa attaatatta gtgaatttaa gctacttctc 540
   ctgtgtgtct aggaaacttt gtgttctcaa tgcacccaca cagtcaagtg ggttgacaga 600
   tatgtcaaaa atacnttatg aaaagaggga ggtagctcat gcgagttggc aaccttttgt 660
   gtaatggttc ctgttcaagc angctgcctc cctttgacat cctacagtca aagatgaaan 720
   gggaaacttt tacntgaagc ctantggagc acaagttgta canttacaat aatccacctt 780
   caacttggct tatggggntt acnaangtaa ggatgncaaa taccttacac caatan 836
<210> 86
   <211> 2896
   <212> DNA
   <213> Homo sapiens
<220>
   <221> misc_feature
   <222> (1)...(2896)
   <223> n = a, t, c or g
<220>
   <223> nbla-03113
<400> 86
   ttctaggcct gtacggaagt gttacttctg ctctaaaagc tgcggaattc ctcgagcact 60
   gttggcctac tggggaacag cattatgata gatttttcag tgaatatgaa aagttacttc 120
   attcagaaaa ttatgtgaca aaaagacagt cactgaagct tctcggtgaa ctactactag 180
   atagacacaa cttcacaatt atgacaaaat acatcagtaa acctgagaac ctcaaattaa 240
   tgatgaacct gctgcgagac aaaagtcgca acatccagtt tgaggccttt cacgttttta 300
   aggtgtttgt agccaatcct aacaagacgc agcccatcct agacatcctc ctcaagaacc 360
   aggccaaact catagagttc ctcagcaagt ttcagaacga caggacggag gatgagcagt 420
   ttaacgacga gaagacctat ttagttaaac agatcaggga tttgaagaga ccagctcagc 480
   aagaagctta atctccaata aacatctatg ttaaatccaa attcagcatt tgctgttagc 540
   tattcagcat caggcactca ttgattcatg aggaacatta ctgctaatct gctgttaagt 600
   gaacggtttt tcattttacc cttttgtttt tcagtccagg ttggagatcg tagctgctgc 660
   tgcttgcaca ctagggcaca tgtgggcttt ctcttgatct ttgtgtcatt tcagaattca 720
   aagactgtgc tacgggagtt ctgaacatgg ctgggttcat gaaggcaaat gtatggatga 780
   gagtgtggtt taggaaagag ggcactgata tcagattaga cctatgtgtt tgcacccatc 840
   tttgttggcg atctgagtgc agtgtggcaa gtgcacacct ggcatccctg cgtcagatcg 900
   cgcaccttca ggtcgcgcac cttcgctgaa ggaagatgac gcagagcttt atctgaaatc 960
   agaggggagc tatccaaaat gggagtttgg gggcagctaa agttgacatg cgaataaatt 1020
   gatactgaaa cttagcaact tcttaaaagt gtaaagaagc ctcataagat cataaggaaa 1080
   atgtatatat gcttttcaca gctttctaga attttttgac atttgatttt cttgagactt 1140
   gtaaacctgg atatgttgaa gggtatttgt taattttact tttcaaagat actttaaaac 1200
   agtagagcta gcaatgacac cttgcatttc atttcaacac tgcttcaagg tttcttttgt 1260
   atataattct tagaatgctc atttctttta aatggtttaa tttgtacagc agagaaatgt 1320
   tattgtagta gtatgtaact attacctaat actgagtttt tgcaaaaaac aatgaatgct 1380
   catatgtaat tgaaatactt cagatcacat gaaaatgctg atttaacatt taagtatcac 1440
   agcattaaaa gaaaaagaaa gtaaaccagt cctttgtatt cagttaccta atggggtgcc 1500
   atcaataagc tgcgatacag ccctggagct cagtcagcca caccttcctg catcctattg 1560
   gccttattca ttttaaatga gttaatgaat ctgccagatc tgtgaatgat agagattatg 1620
   ctaaattaat gctgattctt tgtgtgtgtg ggaaatctct gtagagcacc ttttctttct 1680
   tagactaagt aacccagtac aatagttgtg aactgaataa ttaaaacttt ggcttctctt 1740
   aggaaaagac gacttcctag tcataggtgt cctatgggga aatttatttt ttttaatgtc 1800
   ctgttcctta atgctgcaaa ttatcagtat ttataaagta actgattttg caccactttt 1860
   ttgttactgt gaccacggca gaacaatgtc ttctagacta tatctatgta aagttattag 1920
   aatggtatct gttcatttta gtgatatgaa gatcacaact aacaactgac aaatcagagt 1980
   ttgccagttc aaattcagca tggctgcagc tgattaagaa attgatatga ttattctttg 2040
   ctagcctctc ttactaatgg aattatatac tggccagtaa aatgggcctc ccaattgctg 2100
   tttcagcagg ttttaaacct tcaggaacac cagttaggaa aatagctcca gaaaatatag 2160
   atatatttta tttttattaa aatggcagtc tacatcataa ttggcatttc tcaagactgt 2220
   ctttaccaga atctgtgtga aataaggcaa tctagtctcc ttgaaaaaaa aatctcttgg 2280
   atgtttagga aggaagactt ggccgtgatg tggtgtcctg gctttgtggt gtagtgctgt 2340
   gtgtatggag ttagtgtaaa aacatggatt acaccaagtg gaagaaacgt cttcttgcca 2400
   agctcattct tagaacttac acatctagaa cagcttccac tttggcagtg aggtcgtagc 2460
   cttttaggtg gaagaagtga gggtgcagcg tgtcagacac aacattcatg ttactcttac 2520
   attggaatct gaaggtagtt cagacttcgt gggttttgtt tttaagcaaa acaatgtgaa 2580
   aacatttaag tttgaaatgt tgcatttgaa gttatgatca tttaatatat tcatattacc 2640
   aagactatta tactggaagt ggtttttgtg ttataaaggt ttaattttac ataaggcagt 2700
   tacttaatgt gatttttaac ccttaaaaaa gtggagatgt atacatttgt taacaatgcc 2760
   atgaaagcat tttctttctc ctaaggaaaa gtgaatttct tatcagaata tctggctggc 2820
   cctgtaattt aaattaaaat aaaattttgg agaaacaaaa aaaaaaaaaa aaaggccaca 2880
   tgtgctcgag ctgcag                                                 2896
<210> 87
   <211> 779
   <212> DNA
   <213> Homo sapiens
<220>
   <221> misc_feature
   <222> (1)...(779)
   <223> n = a, t, c or g
<220>
   <223> nbla-03119-f
<400> 87
   gagggggnnn nntttgantc ctcggcactg ttggcctact ggtttatctc aattttcaac 60
   agatttgctg caatagaaaa gttatctttt tttagattta agagctccaa atagcactgt 120
   agcctacatt aagcatcaag tgccccgttc ccttcacaaa agttttctgg ttcattcgag 180
   tcactgaatt ccaaataatg cacattgggt taccaacatt agtcgccact gtcatattca 240
   gaaattaatc ctaaatggtt tacagtcctt ctcatttttt ctctttcaat tatgtgacat 300
   agtccacact tttcattaaa gttattaata atataaggat cttaggtggc aattttctgc 360
   acagctgggg aaaagttgca tattcagtgt angtatgcag atgcttactg aatcccagag 420
   acataaatgg tttattccca ggaatagtcc taccttgcta ttggttaata aaggnctaaa 480
   ttagattggc atcagtncaa caaagcattc atttttattt atgcatctcg aatcactttt 540
   atacatatgt naaatgaaca cantttgaga gnttaataag gtttataaat tatacttcta 600
   gataaatgca ctttttgaga aagacaagaa aaataattat tttctgatgg atttccatta 660
   gccaaanggg cctanaacat aaccagggca aattattgga atgcccatna gggaggactt 720
   nccanattgc tttcctttgg nacaggaact ntttgaacgn nanggntttg aaaatnacc  779
<210> 88
   <211> 734
   <212> DNA
   <213> Homo sapiens
<220>
   <221> misc_feature
   <222> (1)...(734)
   <223> n = a, t, c or g
<220>
   <223> nbla-03119-r1
<400> 88
   gagttttgtc gcaatttcta gtagtgacaa gtagaagtaa tgctagaaga cttgtgccat 60
   tagtgcattc anatcactga atttttcatg atcctgcttc cattcacaag tcctttattt 120
   tacactggtt ccaactttga ttttccattg gtattttgtc agaccaagcc atgtgactct 180
   ttcttccttg gatgcaaaac caagtagttg gaaaaatctt tagggcatct gtaatctatg 240
   ggaatcaaat aattttgcat ccaatccgta aatttatgta tgtgtctgga gaattcaaac 300
   accccagaat gtttagaaaa ctagctgaca gctcggtaaa ggaagtatgc tttcacttgg 360
   tcacaaaggt ttgttcatct ctcaaatagt gtattaatag atggcaacag agtttaaatg 420
   cttttggaag atccttagcc aggtatgagg aaagcctcaa gagagtgcaa gcttattatg 480
   gngttataag gnttatcggg ataatacact ttngagagca atgttcatga tttatcttca 540
   tctgatgaag aaaagacatt ttctaacaca cattatcgag ttaaaattgg ggnaaaaaag 600
   aattttntgn ctcctatttt atagcaaatt tgggatcctt ttggctgaaa anttanttgg 660
   ttcttngatt aaagctggnt nttnattant tcnttaaatt ccagccnatn nnaaaagaat 720
   tantgntctt ttgg                                          734
<210> 89
   <211> 722
   <212> DNA
   <213> Homo sapiens
<220>
   <221> misc_feature
   <222> (1)...(722)
   <223> n = a, t, c or g
<220>
   <223> nbla-03169-f
<400> 89
   gggaatagaa aaaatcacac aggttngcat ggcctcattg nttgaactag atcaaagact 60
   ctttgaaaat tncntcgagt tgaaagcana tcccatcgtt ggctccttag aacctggaat 120
   ttatgcagga tattttgatt ggaaggactg cctgcctcca acaggtgtca gaaactattt 180
   aaaagaagca ctggtgaata taattgccgt gcatgcagag gtgttcacca tttccaaaga 240
   actggtccct cgggtactat ccaaggtgat agaagcagnt tctgaagagc tcagtcgact 300
   gatgcagtgt gtttcatcct tcagcaaaaa tggagcttta caggcgagac ttgaaatctg 360
   tgctttgagg gacactgtgg ctgtttacct gacacccgaa agcaagtcaa gttttaagca 420
   ggctttggaa gccctgccca actttcagtg gagcagataa aaagttactg gaagagctcc 480
   tgaacaagtt caagagtagc atgcacttgc agctnacctg tttccaagca gcttcttcaa 540
   ccatgatgaa aacataaata tctggcacat aaaagaagtc caggaaaata acacgtaata 600
   agactggtca ctctctaagt ccctaaangn attttggngn attaaacatt ggggnttgnc 660
   atttttctct tttttctttc tctgactttg aaaaatggtg ggatacattt naanccaaat 720
   gn                                                            722
<210> 90
   <211> 714
   <212> DNA
   <213> Homo sapiens
<220>
   <221> misc_feature
   <222> (1)...(714)
   <223> n = a, t, c or g
<220>
   <223> nbla-03169-r1
<400> 90
   gctgaatgtg ctgtagtaga tattttnaat atcacaaacn ttcatgattg cagggggtaa 60
   acttagctnn nagttgagtt agatacaaca gttaacatac ttaggaaaga caattcttaa 120
   cagctttatc aagcacattt gtgaaagatt aaactttcat ttccagttac catgatgtcc 180
   ccccatacac caaatgaacc aacacattta tttattttaa ataatggaaa ctgtcatggc 240
   tacactgtgc tctaaaatta cttgcattaa tgaggacatt aattttagca tttaaaaaaa 300
   gagatttaaa aataaccata gaaaaacttg aaaaccctga caagccgact acaaagagaa 360
   ttcctctctg aaaattttca gatgcgacgg tatgagggag ttgggtgggc tgctgactct 420
   gagagaagtc ccggtgacct gaacctattc cagtgaagcc agtcagcagt gaatgtgggg 480
   caagtgcatg cggntggcta cagttccatt gcctggtgac caaggagcat cctgaagttt 540
   cagagaaaaa actgtgtgac agcagcattg gtncaactac tgagcaagat ccttancagg 600
   taaaaaagtg gcanaaacag tcacctgctg aaggaacggg canggagttg gccgtcacgc 660
   ggagcctggg cntttccgta ngggaccctg aaatttgnga acggcccgcc aaac       714
<210> 91
   <211> 1803
   <212> DNA
   <213> Homo sapiens
<220>
   <221> misc_feature
   <222> (1)...(1803)
   <223> n = a, t, c or g
<220>
   <223> nbla-03199
<400> 91
   cactgttggc ctactggcag atgccaaaaa agatgccaac agacactaca ttaccctaat 60
   ggctgctacc cagaaccttt ttataggttg ttcttaattt ttttgttgtt gttgttcaag 120
   cttttccttt cttttttttc ttggtgtttg ggccacgatt ttaaaatgac ttttattatg 180
   ggtatgtgtt gccaaagctg gctttttgtc aaataaaatg aatacgaact taaaaaataa 240
   aagctggtat cttaaaatgt aagagagtaa gactgtaaag cctaaaatga ctggctgaga 300
   atgaaccaga aatgccattt gccaaacagt tgtaactaga aatttgattc tcacggtcca 360
   ttcttttctt tgtccttaag atgacattgt tagtgttcac gtcccatgtt cagtgtccaa 420
   accggcaatg taaaaagtat cctgtgtggt ttaacaggaa atctgtttat gtctctttat 480
   ttgaaaccag ttttactctc agtggttctt taagttcaat gaagtctgcc aggaacattg 540
   gttggtagta ttattccgac acctttaatt tccaaaatct gaagttcctg ctagtttacc 600
   accttcatga tcttcttgaa ctggtaactg attaggttga acttatggaa gatttgtgga 660
   cttaactcaa aagtaacctc tcagtgttct atagaacatg tatttgtgta actgaaccta 720
   ccaggagaaa tgtttggaat tctatatgtg caatttttca acaaatgcaa aaaaaataca 780
   gcacatgtat tgacaagctt ctgtcaagca gcttgagttg aaatttgatt taagaaaata 840
   aatcatgatt gttcaaagct gctgggacgt tagaattagg ccatgatact ggtctcattt 900
   taactacagt ggtatttggc actagtgtaa acttccatat aaatcactct tttggaacaa 960
   caaaggggga gggagaaaaa tcacggcctg ttaaatgagt accaaagccg cccaacagta 1020
   atgagatgtt ctcatccttg attctcccag cctcaaacaa cacagcttac tttttttttc 1080
   ccttgctcag aaagtacctg taatttaaca aacagactgc ctgtaggtat agtgcaatta 1140
   caaatgctct aatcattgta catacatctc tcttgatatt gcagcatcca tactggcttt 1200
   gtaatcatta attttttggc agattgaatg tgctgtattg atatgtatct atgtaattgt 1260
   attgtatgtc tatagctaat tcacgttttg aataatgtta ttttatttac ttttttaaga 1320
   gaggagaatg taaatttgtc agtttatttc tgactaggga tattttcttt ccatttagaa 1380
   aagaagaaaa aaaaaaaacc ttactgtcat acagagcggt actagcgtcg tgctgtataa 1440
   aatcatttgc acattcctga gtagaggtat actgattata agacccaaag gtaatttcat 1500
   agcaaaatac ataaaatcag tcggagcttt tatacaaaca tggaaaccaa ctttgtagaa 1560
   cttttgccat ttgatctagg attggaatat gagcttttat acaattcata ttcttatttg 1620
   gcaaatgcac agtttagtat tacctctctg atggccttta ctagaaaggc agttttagaa 1680
   gctattgtga tccactaagg aaatgtttta acagctagag accactgctt gcctgaaagg 1740
   gcgttcttaa atttggtgca gcaaaaaaaa aaaaaaaaaa ggccacatgt gctcgagctg 1800
   cag                                                               1803
<210> 92
   <211> 2295
   <212> DNA
   <213> Homo sapiens
<220>
   <221> misc_feature
   <222> (1)...(2295)
   <223> n = a, t, c or g
<220>
   <223> nbla-03212
<400> 92
   gcctactggg ctgtgattta taagatgtat atgctttcta tgtgaatata agcttgtgca 60
   caatgtttaa aagaaaaaca atgaattaga agagatcccc cgtcccccag tctgacatat 120
   ttcatacaga atgtttaaaa gaaaaactct gctagtcttg gcaaacattt ggccatgtga 180
   ggcatgtggt cagtttccat tttctattac ctggaaaggg ccataaggaa gcaggcaggg 240
   cttaggtagc ttggttacct aagttagaaa gtaagagaat catggttcgg atatgaattg 300
   aagtggttgg tacatggata cctgttcttt aaaaggaatg tgggagatga gtcaagggtg 360
   aggccgattg ttctgcccat gccctaccat accaatttca ataacaatct tgcctgtcat 420
   tgcactcaga atcttaagat catgtttcaa atatgtcgat tcaaacttgc ttctaagcct 480
   gagtccaggg atagcaatgg tactgaccag ctatggactg ggagaacatt gagtcctgtt 540
   ttccattggc ttcagggcgc atccatgtgg atataggaaa agcctctttc cacagactta 600
   ggaagccccc cgaccacgcg gacgctcgct gggctgcact cagcaggctg cttgagtggg 660
   agctgactcc aacatgccct gccctggctg ggactaggca tgcttccagc ccttcccgtg 720
   ctctccctgc tgtcaacact tgcgtacaaa cctgcctagc tgttgtaagt tttcatcagt 780
   agcttaagta ctagtagcaa aaacttaaat tcattcagaa agttgccact tctgaatgag 840
   tagagcagga aaagtaaact ttactgaaac ataatttgct gcaacagtca acattgttat 900
   gctgaatgtg ctctcaactg ccctttgctc atcgccagat ttattatcca gtgtattcct 960
   aacagtcttt tgttgtcgtt gtttttaaaa caaaggtacg gaccatgtgc tatatataaa 1020
   tcgaatggct taaactaatt tgctatgatc ctctaacacc gaaatttccc ataaacttaa 1080
   gtgcctacac ttaggacttt tgaactttca gtcatctttt tcgtggtgtc tcactgaacg 1140
   atgtgttaga acatgtgttc catttggagt tttgggcctg tgcacggtca accccaatta 1200
   ccaatttagt tgaggctgga ttcgattgct cagttcacct gccacgttct ctccaaggag 1260
   tggggtctac gtgaccttta gtcctctacc tcagagcccc gagtcatttc tgaccacccc 1320
   gttctgtgtc ctcactcttg tccctgaatg ggtccctgtg tggatctcag tgtgtgtgtg 1380
   gtttctccac tcctccccgc tcatgtccca cacctgccat attgaaccgt ttctgcacta 1440
   atcttctcca cgggcacgga gtggagggaa cgtcttggga aaggggagag cttgacctcc 1500
   atctaggttt cttttatctg gagaaaaaga acacttttga actatgtaat gcttcgccct 1560
   gaaaggcaag ctaacgctaa cttcccaggt gacagtagca ggaacaagga agggtaatgt 1620
   ttccatgaca gacacttgct tcccttggga caagtcccag aagaactacc tgaagcacca 1680
   aagctcccca ccccagcctg gtggcagagg aagcaggtgg caccaactca ccaaagggac 1740
   ttgggagcca ccttctcact ctgtggaagt actttagtga gagctgactg gcaagataaa 1800
   aaccctgaaa ttcccagaag ttacagatgc cttaaagact ttctttggaa gatcattgcc 1860
   tgtactattc cttttttgtg tggcataaat aatatattaa cctgttacaa ctgacaacat 1920
   tggttttctg cagctaggga gtcttgagtc gagactgctc gccttgatga cttgctcttt 1980
   tttggcagat gtgtggagtt ggctgttacg acatccctgt gtgatcccag aggaggggca 2040
   gtagctgggc agaaactgca ggcacacatt tgggtggatc acctggcttc cagccccttc 2100
   ggggtggtgc tcagtgtggg gtgatggtat catagaggac cacggcgctt atcagcattg 2160
   tttctttgga gtctgtgtaa tggaatgtat ttttcaaata ctgatatttg cattttctgt 2220
   aacaattcct tataataaaa tgaagtaaaa ttgtgaaaaa aaaaaaaaaa aaggccacat 2280
   gtgctcgagc tgcag                                      2295
<210> 93
   <211> 750
   <212> DNA
   <213> Homo sapiens
<220>
   <221> misc_feature
   <222> (1)...(750)
   <223> n = a, t, c or g
<220>
   <223> nbla-03213-f
<400> 93
   gttggaagca agacnataca gaaagctgct ctaaaatctc actntttgat ttcaccttcg 60
   nttactatga aatgtngnat cagacctgac atgnctgaga taaccccgtg attcaggatt 120
   tgatcaaaag aagtctagta agaattaatt acacagaagc ctcctttcat ttctatgggc 180
   caaacaaagg ccatggataa ccctacccgc tttatgtcat tacccattgg gaaacacaat 240
   ggctacttct gttagggtac attgaccttg gtcaagcatc ttaaagaagg caaccctaat 300
   tgagagctgt cttggctaat actctgcacc acaattgtga tgtcctagtc ctaccactag 360
   agggcatggt acagcctggc aaaagttaaa aggggtgtgg cagctcccat caggtctgga 420
   ggtggtctat aagcacagtt gacagttgtg cattgggatg ggtggagaaa gacgacaaga 480
   gagcagagaa tctgctgatg tggctgcgct tacttttagt gactttatgt acttatatta 540
   acagctggaa ataggntgnt gggttttgag cangctggta tagtgaggaa tgntcatttt 600
   taaatgncct aacagatttt gctttgaaaa atgcttggtc atgaataatt tgnggccagg 660
   gatgctttnt gaagcagnnt agggaacatg aatnttcaag angaaatcca aaaatntgtt 720
   aagggcatan ngntaannnc nncnaggaan                              750
<210> 94
   <211> 743
   <212> DNA
   <213> Homo sapiens
<220>
   <221> misc_feature
   <222> (1)...(743)
   <223> n = a, t, c or g
<220>
   <223> nbla-03213-r1
<400> 94
   tgcttgatgc atttagagac aattccaagg gcaagcactg gattttgata ttatctgctt 60
   tgtagatatt tgtacgnaag actgttaaga ctacatggcc agtccccact gcaggaaaca 120
   gaatgatttt cttttttttt tattattatt ccctgccaat agcattttcc atctaacaca 180
   tttaaattct cacagcataa ttgcaaacat gtacatattg gttcccacga gaaatctgca 240
   ggtgacattt cgggatgttt ggagtcaatc agataaagca cattatgaca gaaaccataa 300
   gaggggagct gggacctggg actgtgcaga atgaaaagcc acggctacgg gaagcataag 360
   cttgtgctgc aacggcatgt gcacatgacc tgctgctcct ggcctctacg aagcatgccc 420
   agcaagggaa cctcctncgc cctnctcacg tgcttccttc ctaaggctga gagctcaact 480
   gaagaccttc ccaataaatg acagatggct cttcagtcan gaatgcagnt agtgggttca 540
   tgacagacac actggnccca cccggcttcc tgntgaccac cantctgaat aacacctgnc 600
   caatgnggga tgacatcagc taggaacaaa gnaattggcn gggtgatgat atttttatcc 660
   naangatnnc ccatntnaag gaaccccccn nttanggcta acaggnnnta ccnggnctgg 720
   nagntannan aaananccnt cnn                                     743
<210> 95
   <211> 3049
   <212> DNA
   <213> Homo sapiens
<220>
   <221> misc_feature
   <222> (1)...(3049)
   <223> n = a, t, c or g
<220>
   <223> nbla-03219
<400> 95
   gaattcctcg agcactgttg gcctactgga aatttaaaaa tagctacgaa acggtgtttt 60
   taaagttgag gtctggaaga cctggctcgg ggtctgggaa ggtgggtctt ttgtgatgtg 120
   gtccccgggc ggtgcacttg ggagccatgg cggggccagg acctctggca gcgcagggat 180
   ggagcccgca ggtgatgagc ttgggaggtg agttgtggag gctgcgctca catcaatgcc 240
   cagtgccctc cccgaggggc ctggttctct ctccacaggg gcgggggaag cacacagggg 300
   acagggaggg gtgctgggtt ctctctcctc gggacaggga gcgcagccag gttctctctc 360
   ctcgggacag ggtggtgccg ttgcgtgcat tccccagctg cagccacgag aaacaatttg 420
   gagcggaacc cgggctctga cctcccctca tcctcagcct tcccccaggg atgggccgtg 480
   agatgaatgt ggtcaccggc ccaatccagg ggtctatggc caaaccgcag acccggagga 540
   agcaggccag gccatctggg gagccggctc ccctcctctc ctcccctggc tcccacaaag 600
   ctgtctcatc cagaagccag gcccgcctgt gagcaagggg aggctgcagg tgttccttca 660
   gcctgaagcg tgtgaaagcc aacagccccc accctggtct ccagccgcag ccccctcccc 720
   agactcaggg ggccaaaccc acttttcaca gccatctgta acccaaacgt ctgcccacag 780
   ctttgttcga gctcagtcca cagtgggccg cgtggcctgg gagcgtccag tggcagctgg 840
   ggcctgccag gactgctgcc cccagatcgc tgtttgctgt gtgtgtggct ctgtggggcc 900
   ccggctccac gataccctca agcatctgcc tcccgctctt cccagattgc agggcatgcc 960
   tggcaagact gcgcagtgcc gctctgacgg gcctgatctg aacctgggct cggggggaca 1020
   agggagggga ctgagcacgt gggatggaca gagatgcaca cccctcccag gcaggagagg 1080
   cagagctgct tctgacacac aggcagttat cagggggaag agaaggtggg cagaggagcc 1140
   accccatctc aaggcagcgt gcagggcaga gcctaagaga gggtcctcct gcaagggcca 1200
   gacaaggagg agggcatggg caggaaccgc gtccaaatca acagcaaaga caaaggtcac 1260
   ctagaaaggg ggactccacc agactcttac ccagcagcac gtccctcctg cctgggcctg 1320
   gtgagcgagg ggctcaaggg gaaactgagg caggtgctgg cacttctgtg tgggtgagga 1380
   ggacaccaat ctgaggcccc gcccgctccc tgcttcggct cctctgtgtt cccgggctgc 1440
   cgactctcag gaggctttgc cacctgaaga acagggaggt ccagcaaggt aagaagagct 1500
   ttccagctcc tgttccgacc tgagagccga gaggcaccat cctgaagaat ggccttggtc 1560
   ccaggcctcc atgtgcgctg tggcagacaa tgccagtcga tcacctctct ttcctgaccc 1620
   agacacagcc tcatgctctt cagccaagtg ccccagggaa gcccgccacc gatcagtcaa 1680
   gcctggagta aaagatgaag cctatttgct tcgctgtttt cacgtggggc ttatcaagta 1740
   tttactaagc accttctaga agcttggaaa gggctacaag ccatggtccc tgagcccaag 1800
   acacttacag gtttgctttc tgggaggatt ggggtggatt agagactgcc ttccagtcag 1860
   tgaccacagg atgggccatc ttggtctctg tccgtatctg gtttgggggt tttcgcagga 1920
   ctaggcctag gctaaggaca gggagggcac aagttctggc tgggtccagg gtcctgggac 1980
   ccagggccat ttgggacctg tgagaatgga tggacagcat gggatggggc atgtggtctg 2040
   agccctcagt agattccctt caccctcttg ttgttggggt cgaagggcga cttcaggtga 2100
   gcctgggcac cataggtcac ccccattctc tccagggcat agtccccgct cttcacaaag 2160
   tccagcgaga cctaggagca gaggtgggga tgaggatcac tctctgttgg taggtacagg 2220
   gctcacgtct gcctgcttct ctggaggagg gggtcttggt cttgctacct gccctgtggg 2280
   caaactcatc cctgtgccag ggactccttt ctgaacccag gcattgcccc agagcaggtg 2340
   cagttcgtgt ccatgtgatc ttgagttgga catgggcatc tggggtccgg caaagccaag 2400
   gtctggcagc caatatgact tccaccggac aacacacctc ccaccccagc aattccagat 2460
   gcctgggaaa cagcagcaac acggtggctt ccccggaccc gggcaagcac agcggccaga 2520
   gcagcgccga ggctgggggc caatccggct cgaatccctc ggggcagggg cggctggaac 2580
   agccttctag acagcttggc ctctgcatgc tccccatccc acaccctggc tcgggctccc 2640
   cagcaagccc tcgggagcca gtcagcccca ggtgcaggtg ctcttaggca ggaggagggt 2700
   gtcacctcca gcctggcaca gctggtgacg cctggcactt ttgctgcagc tgctatgagg 2760
   agggcaggct aggccactca gggaagctgt tcttccttta cagcaattaa acccaaggtc 2820
   agggccacag gagggagtct cgggctccgc tctgggagtc agagggctcc ttcttgttcc 2880
   tagcagctca ggactggatg aaaaaaataa aaaacaaacc aagctttttc aaaaaaaaaa 2940
   aaaaaaaaaa aaaaaaaaaa aaaaaaaaaa aaaaaaaaaa aaaaaaaaaa aaaaaaaaaa 3000
   aaaaaaaggc cacatgtgct cgagctgcag gtgcgcggcc gctagacta 3049
<210> 96
<211> 754
   <212> DNA
   <213> Homo sapiens
<220>
   <221> misc_feature
   <222> (1)...(754)
   <223> n = a, t, c or g
<220>
   <223> nbla-03223-f
<400> 96
   cttctttgat atggataatg gaccgagcct ctgtttgcaa attatttnag tatgcttgcc 60
   ataagaagtg ctgtctgnna accacagcca agtgctctaa aaagtatgat ccagagctgt 120
   catctcgaca atttggggtt gaactgtccc gtttgaccag tgaagaccga actgttcctt 180
   tagtagtgga aaagctcata aactacattg aaatgcatgg actgtataca gaaggtattt 240
   atcgaaagtc tggttcgact aataaaatca aggagcttcg gcagggtcta gatacagatg 300
   ctgagagtgt aaatctagat gactataaca tacacgtcat tgcaagtgta ttcaaacaat 360
   ggcttcgaga tttgcccaat cctctcatga cctttgaact ctatgaggaa tttcttcgag 420
   ctatgggcct tcaggagagg aaggagacaa tccgtggtgt atactctgtg attgatcaac 480
   tctnccgaac tcatctcaat acactggaac gcctcatctt tcatctagtc aggattgctc 540
   tgcaggaaga cactaatcga atgtctgcta atgctttggc cattgtgttt gcgccctgca 600
   ttctccgctg cctgcaccac tgcccactac aaagtgtcag gacatcagta agactaccac 660
   ttgtgtggaa ctgattgtgt ggacaaatga ataatncang ntcgctcaaa gattcagtac 720
   cttggaattg ctgagaataa gcaaaancnc gttg                            754
<210> 97
   <211> 761
   <212> DNA
   <213> Homo sapiens
<220>
   <221> misc_feature
   <222> (1)...(761)
   <223> n = a, t, c or g
<220>
   <223> nbla-03223-r1
<400> 97
   ntaacatgga gtgttttttc agaaattttt ttaatagagg gattantttt ttttgtttgt 60
   anggnagttt ttggaaaann attatnttct accctagctc ctaactatcc caaaataaac 120
   ccaaaggctt ttgctttcac ggntaanaaa gatttatacn ttttcttcaa atgtcagaaa 180
   tganagggtc cctnaggaca gcaatatccc ccctagttca acacccacct ttgggaaggg 240
   aaaagagggt gggggagagg caactacaac tgacccaaat ccccaggccc taggtggctt 300
   tgtatagtaa aaatctcaat tcaaatncaa cagccaaggc acagctggca ccatncccag 360
   caggctttct gcttntgcag gaggcccagg aattcagcac atacagtctt agccatatgc 420
   ttagaaaaga ggcaggacca caattaggat tgctattgtg gaccaagtga tgaaacccan 480
   ccccaaaggn gagatttggt taccactntg taccaccgag ggaacacatt tgccggntcg 540
   gccataaatt cattattttc aaaaaggcta nctgntggtt ggaagngcaa tctnggtnca 600
   caagtttttt tgnggccaac atgactgtcc ctntngggnc tctttgggtt tctganggga 660
   gtttttacaa tctttnaatt ttgggttgnc cttttccggg ttaaaggtcc tttgntgatn 720
   aactgangct tgnntttcaa gaatttggct gggccaaagn c                    761
<210> 98
   <211> 763
   <212> DNA
   <213> Homo sapiens
<220>
   <221> misc_feature
   <222> (1)...(763)
   <223> n = a, t, c or g
<220>
   <223> nbla-03224-f
<400> 98
   ctcctccatc attgaaaagt ggtcgatggt tcgattgcag gcacctttta aacccgattc 60
   agggntgctg gatggatngg ngacgactga tgctgctcta atgtatgatg ctgtgcatgt 120
   ggtgtctgtg gccgttcaac agtttcccca gatgacagtc agttccttgc agtgtaatcg 180
   acataaaccc tggcgcttcg ggacccgctt tatgagtcta attaaagagg cacattggga 240
   aggcctcaca ggcagaataa ctttcaacaa aaccaatggc ttgagaacag attttgattt 300
   ggatgtgatc agtctgaagg aagaaggtct agaaaagatt ggaacgtggg atccagccag 360
   tggcctgaat atgacagaaa gtcaaaaggg aaagccagcg aacatcacag attccttatc 420
   caatcgttct ttgattgtta ccaccatttt ggaagagcct tatgtccttt ttaagaaatc 480
   tgacaaacct ctctatggta atgatcgatt tgaaggctat tgcattgatc tcctcanaga 540
   gttatctaca atccttggct ttacatatga aattagactt gtggaagatg ggaaatatgg 600
   agcccangat gatgccaatg gacaatggaa tggaatggnt cgtgaactaa ttgatcataa 660
   actgccttgc aattgcttca ctggctatta cctatgttcg anagaaggca tcgacttttc 720
   caagncctta tgacacttgg nataagnatt ttgacccgaa gcc               763
<210> 99
   <211> 767
   <212> DNA
   <213> Homo sapiens
<220>
   <221> misc_feature
   <222> (1)...(767)
   <223> n = a, t, c or g
<220>
   <223> nbla-03224-r1
<400> 99
   caaacatgag tacataacaa gtactggttt tttataaaag aggaanttgt atttgccttg 60
   ggggntaaga cttagctnng aatcaaccat taaattgnta atattttccc agtttgctca 120
   gttttgatgg atttattcat aagatatcct atcaatcata atctcaggac acagctcaga 180
   gcaagtgagg aggggatggg cctgtcaaat attcgtccct cttcctttat ccattgaatc 240
   acttatgcat tgacaatttc ttgagcacct atcacaggcc attgattgca ttatgtgctc 300
   aggggaaaca gatgactaga acattcaggt cctcaacata tcctgttata aaagttaana 360
   tctgaacaaa tnactaatta gaataaagtg gatttaagta atatgcttat tttcacagac 420
   ttcaaattta tgactgatgc tttggtacta acaagtaatg gcaaatatac tgngaaatca 480
   gaagtntaaa atgncaatac attattgggg cattcaaaaa caattgngtg tgataacatg 540
   caaggagaat atgttttcac tgagctatat cttacataaa ttattaccaa aataaacctt 600
   acttttttat taagactaat gngnaacant ttacagangn nttttttaac ccaaggacaa 660
   actgcaaaat gaggggagaa ccatgaccga taaagctgaa natagaccct ntgacttggt 720
   ataaaaaagg cttatnagaa gtgnnanatt tgggtttctt gnaaaaa 767
<210> 100
   <211> 804
   <212> DNA
   <213> Homo sapiens
<220>
   <221> misc_feature
   <222> (1)...(804)
   <223> n = a, t, c or g
<220>
   <223> nbla-03298-r1
<400> 100
   tntactgtat gctgtatgtt acagtctact atagtcattg ctctattttt agaatatgtc 60
   agtacacact ggctgaattc cacattttct cattagtaac tttttcagtg gtctttatta 120
   cactacattt tatttttgaa aagaaaaact caccttttac tagggtagga cagaggaata 180
   ggttgaatat ttacagattt cataccataa acatgatctg gtctattttg gccttggaaa 240
   ttaagataat tttaattaag ttttgccttt ggctagcatc agtgacatag aaatgtcctt 300
   ttctgtagtt ttctttggaa atgcaacaaa ctaaaaaatg cagaagtact atactttgta 360
   tgttaaggca ttaccttaag tgttggttca ctttgntact ataaaaaagg ttgatatttt 420
   tggctgccct taagtgaatt taataacaac tactaataaa aatatttgca aaagtatttt 480
   acatatatct aatcataaat atccacaaaa ttccggtgag aaaggnaatt catacccatt 540
   ttaaggaaga taaattactt attcacgtaa catagaaaaa atgaaagtct agtttgactg 600
   acttcaaaga ctatattttt nccctagaat attaactacc tttaatttct ccaactaaga 660
   actggggaca ttttttaaat atntattggt atggaatcta ttncccnaag nccaaagggg 720
   aactgaaccn aggaaaagat ttgaaacttn attccanata ctaactcann ggnncctgac 780
   tnaaggaatn tttccggcct ctct                                        804
<210> 101
   <211> 730
   <212> DNA
   <213> Homo sapiens
<220>
   <221> misc_feature
   <222> (1)...(730)
   <223> n = a, t, c or g
<220>
   <223> nbla-03401-f
<400> 101
   gnagnncngg gtngttgcta ntnatcnttt gaactgccng cctactntac atttgtgttt 60
   caggtgtaga gaggagagag agtgaacagg gagcggggct tttntctgtt ggtctccctg 120
   gactgaagag agggngaata gaagcccaag actaagattc tcaaaatggt ttattaccca 180
   gaactctttg tctgggtcag tcaagaacca tttccaaaca aggacatgga gggaaggctt 240
   cctaagggaa gacttcctgt cccaaaggaa gtgaaccgca agaagaacga tgagacaaac 300
   gctgcctccc tgactccact gggcagcagt gaactccgct ccccaagaat cagttacctc 360
   cacttttttt aatcgtaaca cctccatttg tattacatat ggtgtatggg tattgatgag 420
   gtcatggtat catatatggg atttttttct gtgtaaatca tcaagtataa gaagaaacta 480
   tgggactctg agccttgctt tagagaattt acagtggaca aataggtgtc atcaaaccag 540
   tttttaatca ttctgactca agtgaaaacg ctcagaattt cacactgtga atccacgttt 600
   acaaccctta cangtgggcc ttnaagcctg gttcgctaca acaatggctt ncacaactna 660
   aactccaccg gctnacacaa cccggccact cctggctttt nacttacaca gcttccgact 720
   gnttcttgcn730
<210> 102
   <211> 736
   <212> DNA
   <213> Homo sapiens
<220>
   <221> misc_feature
   <222> (1)...(736)
   <223> n = a, t, c or g
<220>
   <223> nbla-03421-f
<400> 102
   cactgttggc ctactggata atgaagataa taatatcttt attctttatc ccccttcaaa 60
   gaaattacct ttgtgtcaaa tgccgctttg ngnggagccc ttaaaatncc acctcctcat 120
   gtgtaaattg acacaatcac taatctggta atttaaacaa ttgagatagc aaaagtgttt 180
   aacagactag gataattttt ttttcatatt tgccaaaatt tttgtaaacc ctgtcttgtc 240
   aaataagtgt ataatattgt attattaatt tatttttact ttctatacca tttcaaaaca 300
   cattacacta agggggaacc aagactagtt tcttcagggc agtggacgta gtagtttgta 360
   aaaacgtttt ctatgacgca taagctagca tgcctatgat ttatttcctt catgaatttg 420
   tcactggatc agcagctgtg gaaataaagc ttgtgagccc tctgctggcc acagtgagga 480
   aagtagcaca cataggatac agttgtatgt agtcattggc aacaattgca tacaatttta 540
   ctaccaagag aaggtctcnc atggaaagtc caaatgactt ccttgatgga tgttaacaag 600
   ctgactggtg tgagacttga ggttcatcta gtccttcaaa actatattgg ntgcctagat 660
   tctctntgga aactgacttt ncaaataaat agcanattgt anngctgngn cnnttnggac 720
   antactgctt ttatnn 736
<210> 103
   <211> 752
   <212> DNA
   <213> Homo sapiens
<220>
   <221> misc_feature
   <222> (1)...(752)
   <223> n = a, t, c or g
<220>
   <221> misc_feature
   <222> (1)...(752)
   <223> n = a, t, c or g
<220>
   <223> nbla-03421-r1
<400> 103
   gtcccttttc ctttttnttt ttntttcctn nttttttttt tttttttttt tttttttttt 60
   tttatnccct ttttaaaatt taatttnnaa aaacnccttt ngggtaaccn ttcnnantta 120
   aannggaaan ngccnaantt tttaggnnnc cntccctgga atgntttcat ttttncaagg 180
   gnncccaaaa aagggccntt taaaancncn nttccnnntt ttnancccgg ggancnncnn 240
   tnttttccca tgnnactttt aacttnntgg aaaatccccc nanaaaaaaa gntcaaancc 300
   natnnccttn nnttaanccc ctnggttngg ctnngttttt ttnnaaaatt ccnaaanggg 360
   ngnctanttt tttcccanaa nttncncntt tcntttaggc cnccngnggg gaatttnaat 420
   ttttnaggtc cnaaanggtt ttaatttcnc ancttttcca anagngccct ttgggggatt 480
   nggccccttt ttttttnnnc aaaaattttt taatngaagg aaggttnctt tnnaaaacac 540
   cncccnnggt ttttccttaa nacttnanca tttttggatn aaactggcaa aatttntaan 600
   ntnccaaang ggcttaaggg cccccaaggn nggaaccttc ttttnggggt tttnccnctt 660
   tannaccgnt ccctgttttn aancccttac tncccttttt ttnaaggggt taanancccn 720
   cccntcccnn ggnnnncccc ccaccnccaa nc 752
<210> 104
   <211> 732
   <212> DNA
   <213> Homo sapiens
<220>
   <221> misc_feature
   <222> (1)...(732)
   <223> n = a, t, c or g
<220>
   <223> nbla-03439-f
<400> 104
   gncngcctac tgttnaacca aggtctgtgg ataacagacc acaagcacca ctggtttctg 60
   cgtctgctgt tantgaagaa gtcagcnggn ttaaatctac aggnnaattt ccagaacaaa 120
   aattgtttgc tgccctgttg attaaatgtg ttgtgcagct ggaactcatc cagactatcg 180
   acaacattgt cttcttccca gccacaagta agaaagaaga tgcagaagac ttagctgcag 240
   cacagagaga tgcggtggac tttgatgttc gcgttgatac tcaagaccaa ggaatgtacc 300
   gctttttaac atcacaacaa ctttttaagc tactggactg cttattagag tcacatagat 360
   ttgcaaaagc gtttaattcc aacaacgaac agaggactgc cctgtggaaa gcaggattca 420
   aaggcaaatc caagcccaac cttctgaagc aggagaccag cagcctgcct gtgggctgcg 480
   cattctcttc cggatgtaca tggatgagag ccgcgttagt gcctgggagg aggtccagca 540
   gaagcttttg aatgtctgca gtgaancact aagttacttc ctactctaca tcaagaaagt 600
   catcgagaaa cctggctaac ttactgcttt tggttctaac tnaagttctn aagataagtg 660
   ataatagggt taaagctcat gcatcattct actacccttc ttatgngaaa ntatgcaatt 720
   tgacttgatt cn                                                  732
<210> 105
   <211> 713
   <212> DNA
   <213> Homo sapiens
<220>
   <221> misc_feature
   <222> (1)...(713)
   <223> n = a, t, c or g
<220>
   <223> nbla-03439-r1
<400> 105
   anctttattg acctataacc tgattagaat atgccagatg ggaatcaata ttttgcagaa 60
   agttgtacag aattttttac anggnaaact ttacatngng taccatatac attttgtcca 120
   tctgaaaaaa ttttctacat ccactgttaa tacggaatgc ttgacaatct tgtcttttaa 180
   ccatcagagc acaattcaca gtatgaatac atttccagta aatctaacct ccgcaaacca 240
   tgccagattt gttattttaa tatattcaac gttaaattct gtacatagag taaaatctac 300
   atcaggcccc accacccaaa agaaaagaaa atgacagcaa tctggattca ttttgcagtg 360
   attcaagttt tggtccataa aggaccattc tattttaatg gctcatcttt aaaagtccta 420
   agagaaatat gtgacaggta gttactgtcn gtagggatat tttctacctt tntttcccta 480
   ttggtgaaat gcngccacaa nanctttggg catgttcaan ctcaaaattn ccaccttccn 540
   aaaanggngn aacttaaaan cctctttaat aaaaaanaaa gtttncccca tgtttntgcn 600
   tttttcaaaa natttngggt tttcnnngnt tntaaaattt aaaaaacacg ncggngaaaa 660
   ntttatggng taaaaanttn tnttnnancn cntntntnna ganttttata  ana   713
<210> 106
   <211> 2231
   <212> DNA
   <213> Homo sapiens
<220>
   <221> misc_feature
   <222> (1)...(2231)
   <223> n = a, t, c or g
<220>
   <223> nbla-03461
<400> 106
   cactgttggc ctactgggta actgtcagtc aaattatccc atttcctagg tctgtctctt 60
   ttttctttgc agtttaatta ttagtaaata agaaatttta agtctcaaaa ttttggtcag 120
   agataaactc agacactgcc tcgatatcac gaagttctca tttataccaa ctcttatctt 180
   cacgccaccg tgaattctca tcggcataag gaggaaaaga gatggcacca aaggggaaaa 240
   aaatctggtg gtgtaatttg gcatcttcat taagcaagcc atgagcagct tgtgaaatgc 300
   ttcatttatg gggccgccag ctgggagaga gaggcgttct cacaatgcct tgaaaatggg 360
   aactttgcat cctttaaatt tttccaaact gacttagttt gtttaccttg aatttctggg 420
   atggggcaaa tgtgaccttc atgctatagg gcccacgttt ccagatttgg tatggaaaga 480
   aggaagaaag tctgaccctc ttgtttttaa gataggcaaa aggaagatga gatagtccat 540
   ggttcaccac ccaaggtcct tcctgggcac tggctgggct gacgctgggc ctggttccca 600
   gctatgccta cctttctctt gccataccac accgttgctt tatgagcatt cttttgttaa 660
   ggccaagatc aagataactt ttttccttga ataataggac agtcaccttc cccagtggac 720
   tttaagtggc atctgaatgt agaaaggaaa acaaccatga cctgtatttt tagagtagaa 780
   ataaaattcc tcattccacg tatataatcg tggataagca caaataccta gctatagaaa 840
   tgtttgtaaa agtggaaaat tggcagcaac ccaaaagccc aacagcagac aattagttaa 900
   atcagttatg gtctataatc gcagtaggat ttatgttccc ctttaaaatt ctggaaagaa 960
   aaatatagac ttagaaaggg catgtgatgg gggaagggga aaaacaggtc tgtaatgtaa 1020
   taacttttgc aatatttttc cattttatca gaagagtatc agctattatc tttgagtaat 1080
   aaggttatat tttctaaatt tgttttaaag ttttgaatga aatgtctgtt acttttatga 1140
   gattaatttt tatataccga tatatgaaag aaaaatatta acctttagat ccattaaggt 1200
   cctccctggg gaatggcaaa tacatagcat atgtctctcc tcaccttcct tctccgtcta 1260
   tagcaaaaac tttctatcaa tctgtgatca ttccctctgt gcctggtaga agacctcaga 1320
   atgctctcaa ccactatcaa tccatgttcc ttggtatata catgggaact caaatggcat 1380
   tgtcctaagt ctaagtcatg gaaaagagag tgctctaggt gtgaagatgc cccaattaaa 1440
   acaagttcag cgctaattca aagtaaagca tttgggcctt cctgggaagc tgtagtagca 1500
   tgcagaaata ccaaggcaga gttatgatcg tgctgatttt ttcctatcag tgggagggtg 1560
   cagtctgcat atattttacc cctgctctca ctgcagtgtg gggtggatcg ataggtggat 1620
   gtgtaatatt aactggcaca ttgacaccct aatttttgca aatggtaaag ccattccaca 1680
   tatgcatttg ttcaagaagc taatgtttat tgaaaaccta ttgtgttcta agccatgaac 1740
   catccccagg taagtgacct cagctctgcc attacgagtc ctctggggct ggccagggaa 1800
   ggtaagagcc caccctcctc atctgtggag ttgctatgga gatctctgca cctctagaca 1860
   gatactaagg actcaatgca actggcagaa catttttttg atagcacaag atgaaaaaca 1920
   atttgcaagg tatttctgaa aagttaatga aaattgtagg atagttgaaa gcaaaagtga 1980
   acattatgag gcatatgata ctgttaaaat catcatacac tgctgtaaag cagtttcaat 2040
   atctgtatgt ctgccccagg aataatcaac caggtattac ctagaatagt gtttttcaac 2100
   ttctgcaact gtaatctaga gcaagagatg acaagttttg cattatggcc cagtacgcac 2160
   atgcctatgc ctagatacag ctgaaacaaa caaaaaaaaa aaaaaaaagg ccacatgtgc 2220
   tcgagctgca g2231
<210> 107
   <211> 737
   <212> DNA
   <213> Homo sapiens
<220>
   <221> misc_feature
   <222> (1)...(737)
   <223> n = a, t, c or g
<220>
   <223> nbla-03477-f
<400> 107
   ggcncgcccg cccaccgctc tccacgtgct cgctggaggg cggtgcgagg ggccgnttnn 60
   acaagatgtt cttgctgcct cttccggggg nggtgcgagt agtcgtccga cgtctggccg 120
   tgagacgttt cgggagccgg agtctctcca ccgcagacat gacgaagggc cttgttttag 180
   gaatctattc caaagaaaaa gaagatgatg tgccacagtt cacaagtgca ggagagaatt 240
   ttgataaatt gttagctgga aagctgagag agactttgaa catatctgga ccacctctga 300
   aggcagggaa gactcgaacc ttttatggtc tgcatcagga cttccccagc gtggtgctag 360
   ttggcctcgg caaaaaggca gctggaatcg acgaacagga aaactggcat gaaggcaaag 420
   aaaacatcag agcagctgtt gcagcggggt gcaggcagat tcaagacctg gagctctcgt 480
   ctgtggaggt ggatccctgt ggagacgctc aagctgctgc nganggaacg gtgcttggtc 540
   tctatgaata cgatgaccta aagcaaaaaa agaagatggc ttgtgtcgca aaactctatg 600
   gaagtgggga tcangaagcc tggcanaaaa ggagtcctgt ttgcttctgg gcaaaacttg 660
   gcacgccaat tgatggagac ccancccatg agattacgcc aaccagattt gccnaaatta 720
   ttgggaaaaa tttaaaa 737
<210> 108
   <211> 750
   <212> DNA
   <213> Homo sapiens
<220>
   <221> misc_feature
   <222> (1)...(750)
   <223> n = a, t, c or g
<220>
   <223> nbla-03477-r1
<400> 108
   gaccccatca agttttnatt aattgtccct gacaagtgca tagtaanntt cannttgctc 60
   atgttcctag ntttncaaat ctganttaca attttgcttc ggggggaana acatatactc 120
   tgaaaagtgn cnaggaagtt ctaaaaattt taatcattta tagagtatct taaaaatcct 180
   tatcaagtaa gatattaact ttacctttat aaatctttgt gtgaaatgaa aaaaaaatca 240
   aggcatacaa atttcattgn gttctacatt tttaaatacc atcctttgtc tccgttaaaa 300
   nattttcatc catttattca aaaacctttt aagttcaact gtccaattta agacagagtg 360
   aagacatttt tgagtatctg aactaagcat tgtcttgact gaaacgaagt aagaactcaa 420
   tgagagtcct tgtgggcctc cagtcatgcc tttccgtaga tagggaactt catctttgtt 480
   ggcatcacgc ctgctatgtc taaatgtncc cacttaggat gagttccaat tctttcanga 540
   atgctgcacc tgacatgctc ctgcanatcn ggattttcca atgntgttaa catcncnang 600
   cttgtaattt acaaccngnc nttnnnaant gtccaaaaan aggcntcctt caaacaccgn 660
   nccccnnttt cnaatgctnc ccntnaaant ntnttncnta acccctgatt taatttnnaa 720
   aaaccccntn gcncctntnc ccaagttcct 750
<210> 109
   <211> 2342
   <212> DNA
   <213> Homo sapiens
<220>
   <221> misc_feature
   <222> (1)...(2342)
   <223> n = a, t, c or g
<220>
   <223> nbla-03526
<400> 109
   ttctaggcct gtacggaagt gttacttctg ctctaaaagc tgcggaattc ctcgagcact 60
   gttggcctac tggcagattg ttttatattt caatgtatta tgtggagagt gacttttcat 120
   taataaacta caaagaaatg ggttataagg aatattcatg aacatgctct atagaaatat 180
   aataaaaatt cattgatgca aagtcctttt ttaaaaataa ttttcaatct ttttgataag 240
   ctggacggaa gcatctgtct tttactatga agaaaacatt tttgaagtga actgacaaca 300
   taaggcaaat actgggtaaa aatttaagca tctccaaaat cattttatta acccatttgc 360
   atgcaaatac tagggaatgg ttcttcaata tgtagctagt tcttgtggtg gttaatagtt 420
   cttcgtgtat ttgaaagtaa caaaactgca tttgtgttga actcttaaga ccaattaatc 480
   aggtcaaatt gctcttaaga ccaataaatt aggtcagttg ctcctaacaa aggcaaattc 540
   atcagattta atttctaccc ttttctaata taaattttta gaaattttgt ttctcagatg 600
   tgaggcaaat aggttgatct atttgtttat tctttgacag agagactgac tagagttaga 660
   cagcttttta ataccccttc cagtcaaaaa gtaacattgt tatattagta tttagtagtt 720
   ttaaagtctg atgacataag aacagtaaca atttgttaat attcaaaaga ttgaaattga 780
   aaaaaagggg tcctgcattc tgctctccta gaattttttt ttgtgttgaa atcttctttt 840
   ccatattgtc attattgctt ctttatccca tgacattaat tttctggtga ggtgaccctg 900
   aatttagtca ttttcgtcag taattttaga tttaagtaac ttatcatgct tgttttaaag 960
   tctctctcag ctcacccatg aagaaacaaa ttttttttca tttttttctt caaaaatcag 1020
   caagtattgc tgaatctaac aagtatcagc attcaggttt aaccgtgtta tttgattgaa 1080
   ataaatcaaa ccatggaaat aatgggcact gtgtctgctc aagagtaaaa ttacccaatg 1140
   aatgattatg tcaactggat gtcttttata tcaattgatg actttttaaa aaatctgtat 1200
   tttaaataaa atatggagag aacagtattg gtccctacaa cattccaata gctttgtcat 1260
   gacagccatg tcatatgaaa taacttactc tgaatggcta ataattcagt ggagatgaat 1320
   tctaaagatt tttatttgca cttaaaatgt taaagtagta ttgttcattt gatcaatgtt 1380
   tgaatactac tcagtattga aaactttaac tgcattctga aaggtcattg cgccgattgg 1440
   cttacctaag atgtcattta gcttcctttt ctcatgattt acacaaatgt tgttacctta 1500
   aatgcagaaa gatgtgatat tctttggagg ataaaaaacc taagtgaatg aagctagaca 1560
   caaaaggcta catattatat gatttcattt atatgaaata tgcagaatag gtaaattcat 1620
   agagataaat cagattcatg gttgctgggg gaatgggagt gactgcttca tgggtatggg 1680
   gttttctttt ggattgatga aaatattgta gaacaagata gtggggatag ttgcacattg 1740
   tgaatgtgcc aaaatgccag tgaattgtat actttaaaat ggtgtatttg atgtgtattt 1800
   tattacaatt aaaaagaaaa ggatttgata tttgcaatca ttaccaaagc caaaaactag 1860
   tgttctcaac gtttgtgaaa tgcccaaacc gtggtttaga attaatttat agcattagta 1920
   acatgacaaa tctattcagc ttaaagttct agtaaaattt gtgaatccca tcatagaagt 1980
   actcctgaca gcagcagaaa taaagctgta attcatcaaa atttccttat atttaaaact 2040
   tctgtgactg agtgattgac ttctgtgtca atgtttattt tttatctttt gaaatttatt 2100
   atgattcctt gtggcacatc ccaatcacgc tttttaaaga tgtgttttta aaaccatgct 2160
   ttgaaaaaaa tttgtataat tttcaaggtt tttttttata gttttatact ttgtttcttt 2220
   taaagaacaa atgcaacttg ttgtattaga gtaagactaa ataaattgta ctgatgtgta 2280
   aaaaaaaaaa aaaaaaaaac aaaggccaca tgtgctcgag ctgcaggtcg cggccgctag 2340
   ac                                                                2342

<210> 110
   <211> 739
   <212> DNA
   <213> Homo sapiens
<220>
   <221> misc_feature
   <222> (1)...(739)
   <223> n = a, t, c or g
<220>
   <223> nbla-03539-f
<400> 110
   tnaaaccccc ctgctgttaa gacttgacat tcaatatttt ttgatcacat tcttttttat 60
   agcttagcta nnggcaacat ttgtggncat ataaattgca aaagaagctt tctcgngtac 120
   atacattttt aaaagcttga aattgatgtg aacttttaaa aacacgtagg atctgtatta 180
   cattctacat ctcaaaacaa atttaattaa agtgaatatc attccagtat atacaatatg 240
   cctaagaccc agaattggca cactgattta ctagttgaaa atataacagt attcaccaaa 300
   cttcaatgta tactttttgg agagaatgaa attacagtat ttcttaattt actgnaatgt 360
   catctttgta attatgaatt aacaattcaa tgagaggaga cttggttgat taaattaatg 420
   ctggtcctac acattatatc taaaggatct tcgtatatga ctactatctt cttggattat 480
   tttaacaggt aaaatatcaa agtggccatt aaaaacagag ttgacttttc accattgctg 540
   gttttctggt gagacatgtg gaaaggaagg acaggtggac ttttcaacta actagctctc 600
   tgatttttaa taagatcctc aantcttttg gnctnagnta cctatctgtc caanggtaag 660
   catatgctta atcactaana cnggtanatc ctgccnttaa naaccttatn aaccaaatnc 720
   tggaccntan ggtacaaaa                                              739
<210> 111
   <211> 747
   <212> DNA
   <213> Homo sapiens
<220>
   <221> misc_feature
   <222> (1)...(747)
   <223> n = a, t, c or g
<220>
   <223> nbla-03539-r1
<400> 111
   aaaacctcca gnaatatttt cacactacct tctattttaa agttcacact tttnattcca 60
   gagcagggna tggtcaggcc ngggtgggct ccccnccctc tccccttggc nntggtaacc 120
   actggcccca gggactcagc ctgctttcct atccatcccc tcagtagctg tcaccatgca 180
   ggttacccct tctgtttctt ctaccactaa ctccatgtct gactgcaagt gaaaggaaca 240
   gaagcccaaa cctttgggtt ttaaggagtt tattgctaat ctgtaaaaca gaaagagaca 300
   ggagataagc atgacaaaat atagggaaga aatgactttt gcctaaactt ccaattgtgt 360
   acaattgaag cctctgcttt atagctctta gcacacctct caaataagaa ggcaagtact 420
   gggaaagctc tgaacctgtg gcanaaccac tgatagctgt ggagctattc aaggagtctg 480
   ggaatcaagg ggattatcaa nacattgnta gaataaatta atcttactgg atatatanca 540
   naaanttttc aagcatatgt aaatgctact aataccaaat aattacacct tgttttcttt 600
   aaaccggaac tcttaaanat gnctctacaa aantttttga atnggaangg ctgnatgctc 660
   naaaaacttn aaaacactac tgganaaaaa aggtctcngg aaggngatga aanccntnac 720
   attggaacnt tnatnantta aatnggg                                     747
<210> 112
   <211> 725
   <212> DNA
   <213> Homo sapiens
<220>
   <221> misc_feature
   <222> (1)...(725)
   <223> n = a, t, c or g
<220>
   <223> nbla-03587-f
<400> 112
   ttgacagtgc caaagctcgg tactggacac aacgagggac ctgggtctac gataacgnnc 60
   ttttgctcct cctgaagtgt ctttggtcca acgttgttcc aggtttgtca ccgtttctct 120
   ccggccggtc agggcaagag ccagagtgta ccatggcttc cagtagcact gtgttgatgc 180
   ggttggtagc ctccgcatat tctattgctc aaaaggcagg aatgatagtc agacgtgtta 240
   ttgctgaagg agacctgggt attgtggaga agacctgtgc aacagacctg cagaccaaag 300
   ctgaccgatt ggcacagatg agcatatgtt cttcattggc ccggaaattc cccaaactca 360
   caattatagg ggaagagctc gtggtctggg ttgatcctct ggatggaacc aaggaatata 420
   ccgaaggtct tcttgacaat gtaacagttc ttattggaat tgcttatgaa ggaaaagcca 480
   tagcaggagt tattaaccag ccatattaca actatgaggc aggaccagat gctgtgttgg 540
   ggaggacaat ctggggagtt ttaggtttaa gcgcctttgg gtttcaactg aaagaagtcc 600
   ctgctgggaa acacattatc acaactactc gatcccatac aacaagttgg gtactgactg 660
   ngttgctgct atgaaccccg atgctgtgct gcnagtagga agagcaggaa ataagaatat 720
   tcann                                                        725
<210> 113
   <211> 734
   <212> DNA
   <213> Homo sapiens
<220>
   <221> misc_feature
   <222> (1)...(734)
   <223> n = a, t, c or g
<220>
   <223> nbla-03587-r1
<400> 113
   gatcaaatct ttatttaaaa aaaacttttc tcataaatct gnttcataag catntataat 60
   aacatcatnt atattcttaa ttggngtaga aacgttttta aaattactgt gaaaaacaag 120
   agtgagattc cagaaaaaat tgtgccctaa agaaatctgg tttaggccag gtgcggtggc 180
   tcacacctgc aatcccagca ctctgggagt ccgaggcaga cagatcacga tgtcaggaga 240
   tcaagaccat cctggccaac acggcgaaac accctctcta ctaaaaatac aaaaaattag 300
   ccaggcgtgg tggcggtgcc tgcagtccca gctactcggg aggctgaggc aggagaatgg 360
   catgaacccg ggaggcggag cctgcagtga gccgagatca cgccactaca ctccagccta 420
   ggctacagag cgagactccg tctcaggaaa aaaaaagaga aatctggttt agaagatagg 480
   aatgttcatt gaacattgcc tgaactgtca attggaacta aagctttttc tcatttctnc 540
   acctaaatac aggaaaacaa atntnacata ttgaggttat aagttcttca nacgtcaaaa 600
   ttggnctggn tgattaatca ngttaaatta tttaattcta ttttctattc ctaaatataa 660
   tancttaatt taaatcctgn tnaacaatga ataaaagngg actntaattt acccattaat 720
   cttaaaangn tttn 734
<210> 114
   <211> 805
   <212> DNA
   <213> Homo sapiens
<220>
   <221> misc_feature
   <222> (1)...(805)
   <223> n = a, t, c or g
<220>
   <223> nbla-03598-f
<400> 114
   ccccgnnngg ccaaaaaaac nggncaaacn ncaanttnnn anaanttttt nacanctnac 60
   gancngaang aaagacgggg gggnnnttna naaaanngaa anncncnccc ccagnaagan 120
   aancccaanc cacancanaa canncnncga tanaccanca naagaancca aaacacccac 180
   tacactcaca acgnacanca aagcaagncc tttaaacaan gaacacacca ccccnaaaan 240
   acccccnnnc taccataaac acaaaacact ataantgaac caaaaacaag naaacnaaca 300
   nctaanaann aataccgaan tccccnnacc gaaaacaacn taacnccata tnnnccccnc 360
   aannnncaac tcanaanaca ancnacataa nnaacccaac aaacacancc naanaaccnn 420
   caannantnc cctaaaaaat nnnaanacnn nanancnaca cctccacnac caaccnacga 480
   angcaanaac annaaancac naaccnacca acaacanaaa ccanncnnac anngaaaccc 540
   cnaaanccaa aacnncaata cnncctaaaa ccnncnccan aaaccannaa cctngctctc 600
   ncccanatca acccccntaa aacaaannac atcccannac acctaanata ccaangcacc 660
   tnaaancann ccnnanaaan ncaccaaanc canncaccgc aaaannnnnt ccccancnnn 720
   aanaaaccnn aaanccnann ccacananca cnacncaanc canaacntan naanacccac 780
   ncaaacactc ttcntcacan ncncg                                  805
<210> 115
   <211> 752
   <212> DNA
   <213> Homo sapiens
<220>
   <221> misc_feature
   <222> (1)...(752)
   <223> n = a, t, c or g
<220>
   <223> nbla-03598-r1
<400> 115
   tnagtttggg tggtagcctg tgactacagt cccagctaat caggaggctg aagcagaagg 60
   accntttgag cccaggagtt ccaggttaca gtgagngntg atcttgccaa cacactctag 120
   gctgggtgag acagtgagac tccatctcta aaataagaat tataaaaata atagcttcat 180
   atgtcaacac attcaccaat gctgaggccg atttcctcca aatcatttta gtatattaaa 240
   ccgttataat acatggattt tgaggaaagt ccttcaatta ttaacagtag ctagtgaatc 300
   ttttatgtga atattttcgc aaccatgagg agagaacacc aagcttttgc ttcccaccac 360
   tgctccactg gaataagccc atctcattaa ggacattacc tgccctgcca taggacggtg 420
   agcagcttca gcctctgcct ctgnttttcc aaattactcg gngaagatca atattccagg 480
   tgtgaagatc ccaggacaag cagtaaagat gtgaagaggg ttncattcac ngggattcaa 540
   cgtttaagcc ttcattagca caacaacctt taaanggccn ccacccaaat tgccaatcca 600
   aattgccaat ccaaattggg gtgtgggctg gtgaatcaaa aggncangtt ccagctactt 660
   taagggaata gangggtttt cttaangggg aagagnccna aaggttaact ctattgnaac 720
   canggttaaa tcccnagccn tanaagnact gg                               752
<210> 116
   <211> 3168
   <212> DNA
   <213> Homo sapiens
<220>
   <221> misc_feature
   <222> (1)...(3168)
   <223> n = a, t, c or g
<220>
   <223> nbla-03614
<400> 116
   ttctaggcct gtacggaagt gttacttctg ctctaaaagc tgcggaattc ctcgagcact 60
   gttggcctac tgggaaaatc aagccatata catttttttc cttcgaaagt gtctaagcaa 120
   caaaataaat aaatgtatta ttcattctgg gttgtgttaa tttttcctgc cagaggggaa 180
   aaatgaattt acagggaatt cctggaagaa tatcttggga cctaaaaata aaaatcatgg 240
   ttttatttta agcttgtcaa caggggtgac aagtgtaaga attgtagccc agatgtgcgg 300
   gaagcaactc ataaattcta ttttccacta tgttttatct tgcctgccac tttccctaaa 360
   atggaaatca tttttagtat catagagcga gatgggaaac gtggaagttt atttgtaaaa 420
   gtggcactat ggtgagcaag cattcacagg gacactggcc tattttagca gctggcctct 480
   gggagagagg gatgccctac agcttggcac tggagctctt tgggatgctg ctgagcttgt 540
   cattgctgtt cctctttggg gtctccaggc tgcttttaaa ttctctagga ggtgaccatg 600
   ggaccttatt agatgagctt taatttctct ttctaagttg gcttgtgcaa atacagatat 660
   ctagtttttt ttcagtcaga ggggagagga aggatagagt tgaggagtgg acagtaagca 720
   tccccattca gaggcagatg cagcctggtt tggggagacg gtagaggact agaacatatt 780
   agtgtgtatg gagctggcct gggcgctgag atccagagtg aggaatgtgc cagagattcc 840
   atctgtttga gctggcatga atcatagtgt gaatcagtag atctcttttc ttagcaaaaa 900
   ccagcaaaag cccttcttgt gagggtcata tgaagcggaa ggagatgaga ttctatattg 960
   atttagtact agttcatgag ctaccaaatc taaaaactag gagtcaggtc agataaaata 1020
   gcatatttct agcttaaagt tttatgatca tgaatcttat agaaacatag ttaacttaga 1080
   aattaaagat ttaagattaa gttagaaata gaagttgcta gaagttctca aatgtatact 1140
   tctgacaact tggttaaatc cctggcatat gcagaaaagt ttctgtgatt aaaaaataac 1200
   agaagtatga gagtttttcg ttgtccagcc aattttgtag tctatttgag aaggttaaaa 1260
   acaactgtaa aagctaagta acaaggaaac aaaaaaatga tgagtatata accagaagga 1320
   gcccaggtac tataaaagct aagtaacgag gaaacaaaaa aatgatgagt atataaccag 1380
   aaggaacact caatttgatt cccatatcag gaagttcttg ggttcagtgc aagacttgaa 1440
   acctgacatg gtttcattag attgtagaaa acgctttttt tttttttttc tggacaacag 1500
   atggaaccat gttgtcggtt gttcaaagat ggacaggaga cagaaatcta tcaacctgaa 1560
   gtcagctcca actgcagctg tctgttctcc tgaggactgt gctagtggtc cacagaggtc 1620
   accttgctta tcacatgata ttttcttgac gttagttgaa gcacatcacc ttaggtgaca 1680
   catgagggcc aaaggcctct ttctcaggga tgtatgtgaa agtgtggtgg gttggggacc 1740
   agctaaggaa aaaaagacat gaaggagaat gcaagcatct tacagtcatc attcagtaag 1800
   aaaactttat gactggacaa aggaaaacat aaacgccctt taacctagaa tgcaaatttc 1860
   tagtgatttt tactttttta gtgtatcaga agtcactgag caaggtaagt accagcactt 1920
   acagagacgg agtcataaag gctgctctct aaaagtagag gatgtgcaaa acctgtagta 1980
   ccatttaaca gatttttgac cttgttaatt atgtcatttt gaaactggaa taaatatgta 2040
   attcgaaggt tgatttttct gaacaaaagt aagtcaagtc aaagttttct cagagtctac 2100
   tgtttgattc ttgagggtat tcagaattca agattgatgt gcatttcaac atttcagttt 2160
   ctgcctaagt gaagcgtcac ttaaagcctt cgaaattaca gatgtaaaat gaggttgcac 2220
   gtgatgtcag tagctataat tcctactctg gaggtagctc gatgtgtctg aaggctgagt 2280
   gagctgcggc aggatgaatg aatctttgac ttcacgagat gctgttctag tctgtaacct 2340
   tgtcgtgtgg aatcagccca tctgatgctg aacactaact tgaaatggct ggccgaatgc 2400
   agagtaacac atcagagcac tagaccggtg gaaagaacct agattgcaag tccagtgaat 2460
   tatctgcatc ttcattttgt taggtgtaga tttacttttg ttattggttg caattgcaat 2520
   attttaaaat catgattatg cgttaactcc atctatttct aaaacattgg tatttgtgtc 2580
   atttttagtg tagcttgatt tttgttttct tctttctggt acaaaaacga gtttctccct 2640
   ccaacccctg ccatgtatat ctgtatcatg tatatctgta tctacattcc tgtctactat 2700
   caatgcctac tatttacaag gtagttcagt tcgtttcaga tcaactagaa tttattgagt 2760
   actctgtatg ctaatccctg taccaggggc ttgggatata acagtgaata agttcctggg 2820
   gcttacatca gtcaatatag ggttactgtc tattgaacaa gattattttt gggtttctat 2880
   ctatttgggg atatgataat gcatgtattt attcctaacc atcacacaat aatattatga 2940
   aacaatgtta gtaggaggaa gatgagagcc ttcattttaa ctgggaggac ttggttctgt 3000
   ctaagatgtt gcttttgtta tgggttagtg cctattagat aacattctgt ttctgtgact 3060
   gattatttct ttttgacctt ctgagtttta gtttgtccac tgagttttag gtaaaaaaaa 3120
   aaaaaaaaag gccacatgtg ctcgagctgc aggtcgcggc cgctagac         3168
<210> 117
   <211> 758
   <212> DNA
   <213> Homo sapiens
<220>
   <221> misc_feature
   <222> (1)...(758)
   <223> n = a, t, c or g
<220>
   <223> nbla-03615-f
<400> 117
   actggnttct ttaaaaaaat cccaacaaaa gtaagataat gcctttctta aactctcttc 60
   ataaatctgt actattacat tcctcttttt aaccagtatc ataagttggt tttcatttat 120
   ttaaagctta ttggaaaagt tagaattata ggagtagtag gcatatacac tcacatacaa 180
   ggcaaaacct atattggtgc ctaactttat tatacatgag attgggccag aaatgggaaa 240
   agacaggtat ttaccatgaa tttttattat tgntctttct acataagatt gctaactttt 300
   agaacataaa ctgtataatt ataagccata acctcttcca ttttgtaaaa ttaatactct 360
   gtcaatcaga attaagagac agccacattt gggccataag tgaatttaat actgcttttc 420
   aacacaaaga aatggtttcc aaatatttgn attaagttcg ttgtaggtca gtatacattt 480
   tatgggtggt ctgctaatgc cactttgtaa accctgnata atagtcatta ctaaacaaac 540
   ttcagntatt tttaagtaaa tcatatttcg acaattattc caacaagttt aaagccttaa 600
   gtaaatcatc ttttgacaat tcttcaacaa agcttgncag aatttggata aactgataaa 660
   agtcaatatt tacaaatttt ttaaaatcag tggcactnaa tattttcaac tggaattttt 720
   aacccattaa ggnaagtgga ataaatctat tttagtcn                        758
<210> 118
   <211> 778
   <212> DNA
   <213> Homo sapiens
<220>
   <221> misc_feature
   <222> (1)...(778)
   <223> n = a, t, c or g
<220>
   <223> nbla-03615-r1
<400> 118
   catttnacat ttnaaagcta taattttcaa ttatgtcctt ttgtttttcc tgagatgata 60
   aanntgaaag gttacttgac attcattgag attatgaaac attctaataa aatatacttt 120
   tcagtttagt tatggaagcc caaaatgtat tcagatggaa ctgaaatatt tctcaatcag 180
   taaaatgaag tatttcacaa gtttttctga tgtgaataac tgatattatt aggttactga 240
   aaaatgtgat tcataatgat tattatttgg ttgagtgtct acaatgtgct aggcactgac 300
   aaacataata taaattcatt accttcttga atattcctac caaatgaact tggctgctat 360
   cctcaattaa caaatgaaaa aacagacaaa agctagaaag caaactcatg ctctgatcca 420
   tagcccaagc tcttgagctg ntatataaag aaatctatgc tatgcagatt tcagtctgga 480
   gtctctgagc atagacagtt catgaagatc aagcctggag aatagtcttc ttataccggg 540
   gaggtccggc atactgncta ccatcaaatc actactnaag cagttcaaat catttctaac 600
   caaacacttt ggatggattc tagaaaatga tacctccaaa ccataccctg gaancctact 660
   ccnttttact atttaataat cccttttaaa aaggnggcct tttntaacag caaagangga 720
   aacancntga tgggtnaaag gtttggntta ataacagatc cgntttgncc ttctagat   778
<210> 119
   <211> 757
   <212> DNA
   <213> Homo sapiens
<220>
   <221> misc_feature
   <222> (1)...(757)
   <223> n = a, t, c or g
<220>
   <223> nbla-03636-f
<400> 119
   gcctactgtn acagccaagc agtggnaaga gctttaaact cagcatcacc aaacaggacg 60
   atggaggggc ccctattttg gaatacattg tgaaatatgg gnagtaaaga taaggaagac 120
   caatggctag agaaaaaagt gcaaggaaat aaagaccaca tcattttgga gcatctccag 180
   tggaccatgg gtatgaagtt cagattacag ctgccaatag attgggatat tctgaaccga 240
   cagtttatga attcagcatg ccaccaaagc ccaacattat caaagataaa tgctgtgaag 300
   cgaataaagg cgaaaatgga agccagtcgt ggcagctgaa tgctgctggg ttttctttca 360
   ttatagccat aagtttgtct tgcgtgtttt aatgtcattg gtatatcata tggtatatcc 420
   tcttatgccc tgaaaattaa aaaaaaaata gtaatagcaa ggcttatcta aaattataat 480
   aaaatatttc ctaaggncca attaattatg catgnatctt cacccatgaa tgattaaacc 540
   ttttctcctt ncccaantat caancctttt aagtggatan tttgncagaa aatcaaatgg 600
   aaggaattca atcngggatt gngtactttc aagttaccag ttcactgnat accatcctta 660
   atctaaaatt tccattaatt cctaaaagtg anccttgtaa atacctggta ccttggcctt 720
   gnaacttaan cctttggtac atntaacctt cattatt                     757
<210> 120
   <211> 751
   <212> DNA
   <213> Homo sapiens
<220>
   <221> misc_feature
   <222> (1)...(751)
   <223> n = a, t, c or g
<220>
   <223> nbla-03636-r1
<400> 120
   tttttttttt gagacggagt ctcggtctgt tgcccaggct ggagtgcagt ggcgcnatct 60
   cggctcactg caagctccgc ctnccgggtt cacgccatgc tnntgcctna gcctcccgag 120
   tagctgggac tacaggcgcc cgccaccacg cctggctaat tttttgcatt ttttagtaga 180
   gacggggttt taccgtgtta gccgggatgg tctcaatctc ctgacctcgt gatccacctg 240
   cctcggcctn ccaaagtgct aggattacag gcgtgagcca ccgcgcccag ccggccanaa 300
   gtgtgtttta tcggttgatg ggttgcttct gcagtctaca ggtctattac tagattacaa 360
   gaaaggatag canttgacag ctaggagttt ggaaaagata agttaaaaga aaatctacta 420
   tgaaagaaag gcctaccctc atatgaaaag attctgnagc tatctcttta aaaagagaca 480
   cgaaaaatat ttctggtcaa caaaaatgat agcttttgat gaatgaatca cagaagtcca 540
   gaacccaatc attggattta nccaagtntg tatgggcact tttatgcata tatgtcatnt 600
   ngggtggttc ctattggcct aaataataag tngtaaaagt nccttgttaa aatggggttc 660
   aagtgaaaac ccaacctntt tacttggttt gaattcctta ggnaaagatt ncttaagnga 720
   aaatgngtnt tnccttanng aaatttgcaa a                                751
<210> 121
   <211> 4219
   <212> DNA
   <213> Homo sapiens
<220>
   <221> misc_feature
   <222> (1)...(4219)
   <223> n = a, t, c or g
<220>
   <223> nbla-03646
<400> 121
   cactgttggc ctactgggct ggcggcagtg acaggaggcg cgaacccgca gcgcttaccg 60
   cgcggcgccg caccatggag cccgccgtgt cgctggccgt gtgcgcgctg ctcttcctgc 120
   tgtgggtgcg cctgaagggg ctggagttcg cttatcttcg atatctacta ctacgtgcgc 180
   gcctgggtgg tgttcaagct cagcagcgct ccgcgcctgc acgagcagcg cgtgcgggac 240
   atccagaagc aggtgcggga atggaaggag cagggtagca agaccttcat gtgcacgggg 300
   cgccctggct ggctcactgt ctcactacgt gtcgggaagt acaagaagac acacaaaaac 360
   atcatgatca acctgatgga cattctggaa gtggacacca agaaacagat tgtccgtgtg 420
   gagcccttgg tgaccatggg ccaggtgact gccctgctga cctccattgg ctggactctc 480
   cccgtgttgc ctgagcttga tgacctcaca gtggggggct tgatcatggg cacaggcatc 540
   gagtcatcat cccacaagta cggcctgttc caacacatct gcactgctta cgagctggtc 600
   ctggctgatg gcagctttgt gcgatgcact ccgtccgaaa actcagacct gttctatgcc 660
   gtaccctggt cctgtgggac gctgggtttc ctggtggccg ctgagatccg catcatccct 720
   gccaagaagt acgtcaagct gcgtttcgag ccagtgcggg gcctggaggc tatctgtgcc 780
   aagttcaccc acgagtccca gcggcaggag aaccacttcg tggaagggct gctctactcc 840
   ctggatgagg ctgtcattat gacaggggtc atgacagatg aggcagagcc cagcaagctg 900
   aatagcattg gcaattacta caagccgtgg ttctttaagc atgtggagaa ctatctgaag 960
   acaaaccgag agggcctgga gtacattccc ttgagacact actaccaccg ccacacgcgc 1020
   agcatcttct gggagctcca ggacatcatc ccctttggca acaaccccat cttccgctac 1080
   ctctttggct ggatggtgcc tcccaagatc tccctcctga agctgaccca gggtgagacc 1140
   ctgcgcaagc tgtacgagca gcaccacgtg gtgcaggaca tgctggtgcc catgaagtgc 1200
   ctgcagcagg ccctgcacac cttccaaaac gacatccacg tctaccccat ctggctgtgt 1260
   ccgttcatcc tgcccagcca gccaggccta gtgcacccca aaggaaatga ggcagagctc 1320
   tacatcgaca ttggagcata tggggagccg cgtgtgaaac actttgaagc caggtcctgc 1380
   atgaggcagc tggagaagtt tgtccgcagc gtgcatggct tccagatgct gtatgccgac 1440
   tgctacatga accgggagga gttctgggag atgtttgatg gctccttgta ccacaagctg 1500
   cgagagaagc tgggttgcca ggacgccttc cccgaggtgt acgacaagat ctgcaaggcc 1560
   gccaggcact gagctggagc ccgcctggag agacagacac gtgtgagtgg tcaggcatct 1620
   tcccttcact caagcttggc tgctttccta gatccacact ttcaaagaga aacccctcca 1680
   gaactcccac cctgacagcc caacaccacc ttcctcctgg cttccagggg gcagcccagt 1740
   ggaatggaaa gaatgtggga tttggagtca gacaagcctg agtccagttc cccgtttaga 1800
   actcattagc tgtgtgactc tgggtgagtc ccttaacccc tctgagcccg ggtctcttca 1860
   ttagttgaaa gggatagtaa tacctacttg caggttgttg tcatctgagt tgagcactgg 1920
   tcacattgaa ggtgctgggt aagtggtagc tcttgttgct tcccgttcag cgtcacatct 1980
   gcagtggagc ctgaaaaggc tccacattag gtcacctgtg cacagccatg gctggaatga 2040
   tgaaggggat acgctggagt tgccctgcca tcgcctccat cagccagacg aggtcctcac 2100
   aggagaagga cagctcttcc ccaccctggg atctcaggag ggcagccacg gagtggggag 2160
   gccccagatg cgctgtgcca aagccaggtc cgaggccaaa gttctccctg ccatccttgg 2220
   tgccgtcctg ccccttcctc cttcatgcct gggcctgcag gcccacccca gccaccactg 2280
   agtccactcg gagtgccctg tgttcctgga gaaggcattc cagggttgaa tcttgtccca 2340
   gcctcagcct gggacaccta ggtggagaga gtggtctccg ctctgaattg gatccagggg 2400
   acctgggctc attcttcttg gctcaccaac cctgcaggcc tcatctttcc caaaacccac 2460
   tttgtcttgg tgggagtggg tccgcgctgc tctgcagcag gggctgggga gtggacagca 2520
   tcaggtggga aagtggagtc caccctcatg tttctgtagg attctcaccg tggggctgga 2580
   agaaaagagc atcgacttga tttctccaac cactcatccc tctttttctt tcttccacca 2640
   ctccccaccc cagctgtagt taatttcagt gccttacaaa tcctaagctc agagaaagtt 2700
   ccatttccgt tccagaggga agggaacctc cctaggtcct tccctggctt gttataacgc 2760
   aaagcttggt tgtttatgca actctatctt aagaactgcc cagcctcagc tgaaaacccg 2820
   aatctgagaa ggaattgcgt catgtaaggg aagctggaat taagggagct gagccagtca 2880
   tggttgtggc gtgtgagtca ggagacctag gtttcagccc ctctctactg tcagcgagct 2940
   gtgcaacgtg ggcaagtcat tgtcctctga gctgcagttt cctcatctgt cacatcgcta 3000
   cagacaagac ctccctggaa cccttctgat tgtcttagac actgtggttg caaaacccac 3060
   ggaaagcctc atttgtgtgg aaagtcagag gaaaaatgat ccagtggaca cttggggatt 3120
   atctgtcatt caagatcctt ccttcaaccc caaggtcagc tcccatctca tttccagaaa 3180
   ggctcatacc tggcttgcag ggaagcatct gtcttgtcat tccaggtgcc agaatcctct 3240
   cagagtcatt gaagggtgtt cacccatccc acccaaggct tggcacactg ccagtgtctt 3300
   agcagggtct tgtgagggct gggggcatcc aggcactcag aaggcaaagg aaccacccta 3360
   cccatttggc ctctggaggg ggcagaagaa agaaagaaac ctcatcctat attttacaaa 3420
   gcatgtgaat tctggcatta gctctcatag gagacccatg tgcttccttg ctcagtgcaa 3480
   aactgatgat tctacttgct gtagatgaat ggttaacacg agctagttaa acagtgccat 3540
   tgttttgcca gtgaagcctc caaccctaag ccactgggac ggtggccaga gatgccagca 3600
   gcctctgtcg cccttagtca tataaccaaa atccagacct tatccacaac ccggggcttg 3660
   gaaaggaagg tattttggaa tcacaccctc cggttatgtt gctccagtaa aatcttgcct 3720
   ggaaagaggc agtcttctta gcatggtgag ctgagttcat ggcttttttt tgtagccagt 3780
   cctgtccctg gccatccatg tgatggtttt ggatggagtt aaacttgatg ccagtgggca 3840
   gtgcatgtgg aaagtatcag agtaaggctc tcccctccag agccctgagt ttcttggctg 3900
   catgaaggtt ttctttagaa tcagaattgt agccagtttc tttggccaga aggatgaata 3960
   cttggatatt actgaaaggg aggggtggag atgggtgtgg cagtgtatgg tgtgtgattt 4020
   ttattttctt ctttggtcat gggggccaag gagaaaggca tgaatcttcc ctgtcaggct 4080
   cttacagcca caggcactgt gtctactgtc tggaagacat gtccccgtgg ctgtggggcc 4140
   gctgcttctg tttaaataaa agtggcctgg aaaaaaaaaa aaaaaaaaaa aaaaaaggcc 4200
   acatgtgctc gagctgcag                                              4219
<210> 122
   <211> 726
   <212> DNA
   <213> Homo sapiens
<220>
   <221> misc_feature
   <222> (1)...(726)
   <223> n = a, t, c or g
<220>
   <223> nbla-03652-f
<400> 122
   ngttgcccta ctggctcttg gcggcggagg ctttgtctcg tgggctggtc cccagcggct 60
   ccctccccga acagctgctg ctccagggag gaagcggcgc gggtgctgct ccagcttccc 120
   ggtgctgaaa accggagggc tcgtcatcca ccactaccat gtaagggcca tgagaagggc 180
   tcatcctggc gcagcgcgga catggaggag gacttattcc agctaaggca gctgccggtt 240
   gtgaaattcc gtcgcacagg cgagagtgca aggtcagagg acgacacggc ttcaggagag 300
   catgaagtcc agattgaagg ggtccacgtg ggcctagagg ctgtggagct ggatgatggg 360
   gcagctgtgc ccaaggagtt tgccaatccc accgatgata ctttcatggt ggaagatgca 420
   gtggaagcca ttggctttgg aaaatttcag tggaagctgt ctgttctcac tggcttggct 480
   tgggtggtct ttgtaggcat gatgtccagc tccacgctct ggggaaatat ctcagaccag 540
   tacggcagga aaacagggct gaaagatcan cgtgctgtgg actctgtact atggcatcct 600
   tagtgcattt gcgcccgtgt atagctggat cctggtgctt ccggggcctg gtgggctttn 660
   ggaatcggag gagttcccaa tccgtgacct tnatccgagt ncttccatga aagcnnactt 720
   aatgtt                                                            726
<210> 123
   <211> 749
   <212> DNA
   <213> Homo sapiens
<220>
   <221> misc_feature
   <222> (1)...(749)
   <223> n = a, t, c or g
<220>
   <223> nbla-03652-r1
<400> 123
   tgaatccctt tttttggtcc aggtcatact cttgggtgag ttcttgatgt cggcagtgac 60
   aatcagtgcc ccagttgggg cctgccagcc ccccaagctc tgnagcctca aagaccagct 120
   cagtccccca tcggtcacta ttcctgagag ccagagttcg acctggtaac acctgcaccg 180
   tgcattcctc ggccgaccat ctcctggccc cactcccggt ggctggactc ctgcagtcct 240
   cggcctttgg tctcaatggg caaaaagcag gaggccaggg cagccaggag gcagcagcca 300
   ctgtaaactg ccagagtcag gtacacagag gattccagca tcacctgggc gatgaacgga 360
   gtgatgagag cacccactct tgccatgccg ctgcaggtgc ccaggccgag ggcccgcgtt 420
   gccgtggggt agacctcagg tgtgtaaaca tatgccgctt gaaagcctcc agaaataaac 480
   gctcttgcaa tgaagagtaa cagagtgagc acatttcttc aacacagata aacagcagga 540
   ggcttgcaga aggagaagat gacaaagcac anggccatgg ncttcttgcg ccccaagcgg 600
   ncaataatnc acagagtcac aagggcacct ggnaactcan aaaagggtgg gtccacaagn 660
   aagtccatgt aatccttctt anttaagnac ttcgnaggnc aagcttggaa ttttggcctt 720
   taaagccttt tttcgacttg gagatgccc                                   749
<210> 124
   <211> 1990
   <212> DNA
   <213> Homo sapiens
<220>
   <221> misc_feature
   <222> (1)...(1990)
   <223> n = a, t, c or g
<220>
   <223> nbla-03755
<400> 124
   ttctaggcct gtacggaagt gttacttctg ctctaaaagc tgcggaattc ctcgagcact 60
   gttggcctac tggcaaaaaa aagaataatg acccggcccc cgccctctgc ccccagctgc 120
   tcctcgcagt tcggttaatt ggttaatcac ttaacctgct tttgtcactc ggctttggct 180
   cgggacttca aaatcagtga tgggagtaag agcaaatttc atctttccaa attgatgggt 240
   gggctagtaa taaaatattt aaaaaaaaac attcaaaaac atggccacat ccaacatttc 300
   ctcaggcaat tccttttgat tcttttttct tccccctcca tgtagaagag ggagaaggag 360
   aggctctgaa agctgcttct gggggatttc aagggactgg gggtgccaac cacctctggc 420
   cctgttgtgg gggtgtcaca gaggcagtgg cagcaacaaa ggatttgaaa cttggtgtgt 480
   tcgtggagcc acaggcagac gatgtcaacc ttgtgtgagt gtgacggggg ttggggtggg 540
   gcgggaggcc acgggggagg ccgaggcagg ggctgggcag aggggagagg aagcacaaga 600
   agtgggagtg ggagaggaag ccacgtgctg gagagtagac atccccctcc ttgccgctgg 660
   gagagccaag gcctatgcca cctgcagcgt ctgagcggcc gcctgtcctt ggtggccggg 720
   ggtgggggcc tgctgtgggt cagtgtgcca ccctctgcag ggcagcctgt gggagaaggg 780
   acagcgggta aaaagagaag gcaagctggc aggagggtgg cacttcgtgg atgacctcct 840
   tagaaaagac tgaccttgat gtcttgagag cgctggcctc ttcctccctc cctgcagggt 900
   agggggcctg agttgagggg cttccctctg ctccacagaa accctgtttt attgagttct 960
   gaaggttgga actgctgcca tgattttggc cactttgcag acctgggact ttagggctaa 1020
   ccagttctct ttgtaaggac ttgtgcctct tgggagacgt ccacccgttt ccaagcctgg 1080
   gccactggca tctctggagt gtgtgggggt ctgggaggca ggtcccgagc cccctgtcct 1140
   tcccacggcc actgcagtca ccccgtctgc gccgctgtgc tgttgtctgc cgtgagagcc 1200
   caatcactgc ctatacccct catcacacgt cacaatgtcc cgaattccca gcctcaccac 1260
   cccttctcag taatgaccct ggttggttgc aggaggtacc tactccatac tgagggtgaa 1320
   attaagggaa ggcaaagtcc aggcacaaga gtgggacccc agcctctcac tctcagttcc 1380
   actcatccaa ctgggaccct caccacgaat ctcatgatct gattcggttc cctgtctcct 1440
   cctcccgtca cagatgtgag ccagggcact gctcagctgt gaccctaggt gtttctgcct 1500
   tgttgacatg gagagagccc tttcccctga gaaggcctgg ccccttcctg tgctgagccc 1560
   acagcagcag gctgggcgtc ttggttgtca gtggcggcac caggatggaa gggcaaggca 1620
   cccagggcag gcccacagtc ccgctgtccc ccacttgcac cctagcttgt agctgccaac 1680
   ctcccagaca gcccagcccg ctgctcagct ccacatgcat agtatcagcc ctccacaccc 1740
   gacaaagggg aacacacccc cttggaaatg gttcttttcc cccagtccca gctggaagcc 1800
   atgctgtctg ttctgctgga gcagctgaac atatacatag atgttgccct gccctcccca 1860
   tctgcaccct gttgagttgt agttggattt gtctgtttat gcttggattc accagagtga 1920
   ctatgattga aaagaaaaaa aaaaaaaaaa aggccacatg tgctcgagct gcaggtcgcg 1980
   gccgctagac 1990
<210> 125
   <211> 2180
   <212> DNA
   <213> Homo sapiens
<220>
   <221> misc_feature
   <222> (1)...(2180)
   <223> n = a, t, c or g
<220>
   <223> nbla-03771
<400> 125
   cactgttggc ctactggcag ataaagcctt atgctgccca ccagcccact aaatgtatta 60
   aatacctgtc tctatgtagc ttatgtaaaa actcaatgtt gactgtcccg tgtctgctgc 120
   atttaaaagc tcattgtgat tctatcatct tgctatgcca atgccttatg ttatggtgtc 180
   atgtatatag gccatggtac aaaagtgact gtcaactgct tactcaacat ctagtcagaa 240
   aaggtctgag gcagtgcaat aacgctttta gtcaaactgg ctcactgttg gagtcattta 300
   catctgtgta ttctttaccg taaatactga aatagtattt tttaactgtt ttttcaggct 360
   tgtaataaat atctgtgtca tatctacata gtcaaaatac attgagtaat tcagtttaaa 420
   agtgttgcct actaacaaac taaagagaaa catctactga ttttcccata taattgctta 480
   ttttccattg ccaatgtaga cctgcctgga atggtgtctt tcaccactat catgtgtaaa 540
   ataaagggag gctattgtgg tgaattttca cctgtctgac attagctctt tcactagtca 600
   aaggatgtac atacctcaga ggtgacctga ctaccgaggt ccagttttca aaagtgcatc 660
   ttattttaat ttttgtctcc aaaaattaaa attggatgtc tacaaaacta acatatgtag 720
   actttcaaag gccagcattg gtggggaagg gaaaagaaag gatggaagga tggaaggaac 780
   gaagggtgat gaggagatga agggagaggg aggggaaaga aaagaacaga aaaaagctaa 840
   tagaggaagg taaatctcta aatgagttaa ataaggctta aataaagttt gcatggtata 900
   ttctctaaaa gttgattggc gtggaggagg ctgtatgtgt ttgtgtgtgt atgcacatgg 960
   gcatgtctat gtgtaaggtc agaaggggga gcctgaatta tctactgctt ttttggttaa 1020
   ctagtcctat ttttaaaact ttgtctaact cccaaaactt attaatgcga catgtagtac 1080
   tgaaattaga agattattgt taaactcagg acagctctgt ggagattcac aaagtaattt 1140
   catgaaaact tatgaggaag ttgttcagta ctgtcactaa ttaacgcgca caaaaaaaaa 1200
   acttgtgagt aattatgggt gatttcgctg caaaacaaat tagtatatga attattattt 1260
   tttctcaaat tgtagaggtt ttgtttctat taagtctcta acatttccca gattaaaaat 1320
   accaaaacta ttttttaaac ttaaggaata gagtatcaga aattaaccat taaattaata 1380
   gttcttaaag ttgctatgta tatggtaagt aaaacaattt cctatttcta agttatacat 1440
   ggtattagat ttctaggtac agtcgcatga agcattattg acttttatta acttcagtgt 1500
   tttagagggt tttcatacta gtctatgaat gctctgaatg ctgcagggaa aaagaagcac 1560
   aatcagtatt tcattattta tgaattctca attgaattat cctatcctgt ccacacacat 1620
   aatagcatta gcatttcttc ttataccatg ttgaaatact tttaatacaa actctcaata 1680
   gaagcttaaa cataagtggt taagtcttgt tgtctagtct cattcacctg cctcaacatg 1740
   ctttctttca ttctatttgc atacaaaatg ttcttatttc agttttgtag acaggatatg 1800
   agttagcata ctcgtgtttg ttcagctgtc catcctgcat cgttactaca atgccttttt 1860
   ctgccattta atggtgtttg tatcaatgtt cccatatctg ctgcatttta actccataaa 1920
   aaggaaatgt gatttcgtat taatagtttt gttgatcaac tcaatatttc tgcaccaatc 1980
   agcataccta tatgcatgta gtagtctgta caattgttca acatcaaaat acttgtttac 2040
   tttatgtcaa aatgtctata aaattgctgg cattgttctc catttcagtc tggtagaata 2100
   agcgagatag aaaataaatg tgtaaatgaa aaaaaaaaaa aaaaaaaaaa aaaaaaaggc 2160
   cacatgtgct cgagctgcag                                             2180
<210> 126
   <211> 2078
   <212> DNA
   <213> Homo sapiens
<220>
   <221> misc_feature
   <222> (1)...(2078)
   <223> n = a, t, c or g
<220>
   <223> nbla-03777
<400> 126
   cactgttggc ctactggttt aatctcattt tctcctaaag ttcttctctt caatatgatc 60
   tgcaaagggt tgaaagtgct gtcctccaaa ccaggcgtaa gaaagtggtt acagataaag 120
   caagtaaatc cattaaagaa gaaatgggaa gatctgttgt gtgccttatc tgacccagca 180
   ttatcgctgg aggggaagtg cttataggga aggaagagaa gtttggtcaa ttgatgcagt 240
   ctcagtatga tgacagctgg tgacttcctg agggttattc atcacacttc agcagggtga 300
   tgttttcaaa gcctgcgtgt gaccatgtca ctcctctgtt ctccagcgct ttcaaaataa 360
   aactgaaatc cgtctcagag ggccagtctc agagtatccc ctgccagccc catccttcac 420
   tgctcagcag tcacctcctt ccccctcctc atccttttga ggtctcttct gatccttcag 480
   gggccagctt ctctccgtcc catggttgtt tgcctatgct gatcccctac atggatctcc 540
   ggtcacctcc atcactctta cctggttagt ctctccctgg cctttatccc aggtcagctt 600
   cgcttctgca ggaatgcctg ccttgaaacc tcaggtcctc ctgttccccc tcttagagca 660
   ctgggctatt ctcctgggag gcagtaattc cagtagtaat ttgcatacat ttgtaagcag 720
   ttttgattaa tgcctgttgt ttccactaga ctctatgctc tgtaggggac aaatcttgtc 780
   tgctttttgc tcatcaattt ttccccagca cagagctcaa tactggcact ggtaaaccct 840
   tgagaaaatg attaaaagcc tctaagatgc atgcagttat gaagaaccaa cagaaggtac 900
   aggtggcatg ggacagcagt caggaagcaa tttggcttaa ggaggaaggc cagtgactca 960
   cagatggagt ctcagcactt cccttgcccg tagaagtgag aaacaataca ggtagtcaca 1020
   gcatagtaaa aatttcaggc caggcacggt ggctcacggc tgtaatccca gcactttggg 1080
   aggctgatgt gggtgggtca caaggtgaag agatcaggag catcgtggcc aacatggtga 1140
   aaccccatct ctactcaaag tgcaaaagtt aactgggcat ggtgacacag gcctttagac 1200
   ccagctgctc gggagactga ggcgggagag tcgcttgaac ccaggaggaa gaggttgcag 1260
   tcagtcgaca tcacgccatt gcactccagc ctggcaacag agcgagacgt gtctttaaaa 1320
   aaaaaaaaat cagatagcag tttcatatga ctagaggctg tgggctgatc agatcctgaa 1380
   aaaccgggca tggaccaagc tggctaagac caagtggacc caacatggtg ctgaatttga 1440
   cctaggattc acctaggacc tcgtgatgtg tttgttaaca tcctaagtca ctgtgacttg 1500
   ggatgttaat gagtgactta ccatgttaat gagcatatca cgaggtccta ggtgaccagc 1560
   accatgaatt ccgagaatac ctacgtttgg tgtaaaaatg gatggcatca caattctaag 1620
   aaatttccac cctttcccag gaattttcat aagtattcca ccccttggct aaagaaacac 1680
   ataaaggtag cagccccaaa tccccttagg catggtgctc tcttgagtcc gcctacactc 1740
   ccccttcctt gagtgtgtac ttttcccgtt gttataaatc tctgtacttc cactgttgcc 1800
   tgactcatcc ttgaattcct tcttgagacg gtgtcaagag cctggacacc tgccgggatc 1860
   agggtcccac cagcatttgg ggacctcccc aagcccacta gtatcagtag agtccctata 1920
   ggtaattgtc taataagtgg aaggtgggtg tagaagtttc aagaaaccaa agaaatgtca 1980
   gatgtgtcat ttgacccaaa agaaaagcgt tttaaaggaa gttaatgaaa tgcagtcaga 2040
   aaaaaaaaaa aaaaaggcca catgtgctcg agctgcag                        2078
<210> 127
   <211> 1957
   <212> DNA
   <213> Homo sapiens
<220>
   <221> misc_feature
   <222> (1)...(1957)
   <223> n = a, t, c or g
<220>
   <223> nbla-03779
<400> 127
   gaattcctcg agcactgttg gcctactggg atcctgtttg acatttttat ggctgtattt 60
   gtaaacttaa acacaccagt gtctgttctt gatgcagttg ctatttagga tgagttaagt 120
   gcctggggag tccctcaaaa ggttaaaggg attcccatca ttggaatctt atcaccagat 180
   aggcaagttt atgaccaaac aagagagtac tggctttatc ctctaacctc atattttctc 240
   ccacttggca agtcctttgt ggcatttatt catcagtcag ggtgtccgat tggtcctaga 300
   acttccaaag gctgcttgtc atagaagcca ttgcatctat aaagcaacgg ctcctgttaa 360
   atggtatctc ctttctgagg ctcctactaa aagtcatttg ttacctaaac ttatgtgctt 420
   aacaggcaat gcttctcaga ccacaaagca gaaagaagaa gaaaagctcc tgactaaatc 480
   agggctgggc ttagacagag ttgatctgta gaatatcttt aaaggagaga tgtcaacttt 540
   ctgcactatt cccagcctct gctcctccct gtctaccctc tcccctccct ctctccctcc 600
   acttcacccc acaatcttga aaaacttcct ttctcttctg tgaacatcat tggccagatc 660
   cattttcagt ggtctggatt tctttttatt ttcttttcaa cttgaaagaa actggacatt 720
   aggccactat gtgttgttac tgccactagt gttcaagtgc ctcttgtttt cccagagatt 780
   tcctgggtct gccagaggcc cagacaggct cactcaagct ctttaactga aaagcaacaa 840
   gccactccag gacaaggttc aaaatggtta caacagcctc tacctgtcgc cccagggaga 900
   aaggggtagt gatacaagtc tcatagccag agatggtttt ccactccttc tagatattcc 960
   caaaaagagg ctgagacagg aggttatttt caattttatt ttggaattaa atactttttt 1020
   ccctttatta ctgttgtagt ccctcacttg gatatacctc tgttttcacg atagaaataa 1080
   gggaggtcta gagcttttat tccttggcca ttgtcaacgg agagctggcc aagtcttcac 1140
   aaacccttgc aacattgcct gaagtttatg gaataagatg tattctcact cccttgatct 1200
   caagggcgta actctggaag cacagcttga ctacacgtca tttttaccaa tgattttcag 1260
   gtgacctggg ctaagtcatt taaactgggt ctttataaaa gtaaaaggcc aacatttaat 1320
   tattttgcaa agcaacctaa gagctaaaga tgtaattttt cttgcaattg taaatctttt 1380
   gtgtctcctg aagacttccc ttaaaattag ctctgagtga aaaatcaaaa gagacaaaag 1440
   acatcttcga atccatattt caagcctggt agaattggct tttctagcag aacctttcca 1500
   aaagttttat attgagattc ataacaacac caagaattga ttttgtagcc aacattcatt 1560
   caatactgtt atatcagagg agtaggagag aggaaacatt tgacttatct ggaaaagcaa 1620
   aatgtactta agaataagaa taacatggtc cattcacctt tatgttatag atatgtcttt 1680
   gtgtaaatca tttgttttga gttttcaaag aatagcccat tgttcattct tgtgctgtac 1740
   aatgaccact gttattgtta ctttgacttt tcagagcaca cccttcctct ggtttttgta 1800
   tatttattga tggatcaata ataatgagga aagcatgata tgtatattgc tgagttgaaa 1860
   gcacttattg gaaaatatta aaaggctaac attaaaagac taaaggaaaa aaaaaaaaaa 1920
   aaaaaaaaaa aaaaggccac atgtgctcga gctgcag                1957
<210> 128
   <211> 775
   <212> DNA
   <213> Homo sapiens
<220>
   <221> misc_feature
   <222> (1)...(775)
   <223> n = a, t, c or g
<220>
   <223> nbla-03794-f
<400> 128
   gnggngnggn ggttnnnntn tgaantcctt tagcactgtc ggcctactgg gttgtggttg 60
   gtgtgcgggt ttcggttgga ggactcgttg gggaggtggc ctgcgcttgt agagactgca 120
   tccccgagac gatggcggag ggagataatc gcagcaccaa cctgctggct gcagagactg 180
   caagtctgga agagcagctg caaggatggg gagaagtgat gctgatggct gataaagtcc 240
   tccgatggga aagagcctgg tttccacctg ccatcatggg tgtggnttct ttggtgtttc 300
   tgattatcta ctatctanat ccatctgttc tgtccggcgt ttcctgtttt gttatgtttt 360
   tgtgcttggc tgactacctt gttcccattc tagcgcctag aatttttggc tccaatagat 420
   ggaccactga acaacagcaa agattccatg aaatttgcag caatctagta aaaactcgac 480
   gcagagctgt gggttggtgg aaacgcctct tcacactaaa ggaagaaaaa cctaagatgt 540
   acttcatgac catgatcgtt tccttgctgc ggntgcttgg gtgggacaac aagtncacaa 600
   cctgcttctt acctacctga tagtgacttn cttactattg ctttctggac taaaccaaca 660
   tggaatcatt ttgaagtcat tggaatgggc caagaaggag ataaaccaac ttntcaaacc 720
   aaaagaaaag aaaaacgaat gatcatctgc nttaatcann gngaataatg cacac775
<210> 129
   <211> 784
   <212> DNA
   <213> Homo sapiens
<220>
   <221> misc_feature
   <222> (1)...(784)
   <223> n = a, t, c or g
<220>
   <223> nbla-03794-r1
<400> 129
   gnggnggggg nggttnnnnt ttgtgtggcc tttgtttttt tcnttttttt tttttttttt 60
   tttttaagat tccagctttt tttatttttt ccccttttac acaaaacaaa gtagaagaaa 120
   taatacagga ttaaaactgn aaaagtagtt aatcagtaga atatgtatgc acacaaaaaa 180
   tccagatagg aagacaggct tatttacaat gaaagtaagg taaaattaac gtagttttct 240
   tatgaaataa taaaaaaaaa tcaaacatat gctacaatgg gcaccactgt tcacagcaat 300
   attaaagagg agaaaacaac acttatttaa tagggacaga tataccacaa ggtacaacat 360
   cagtgcaata aattcacaaa actatattac agctagttaa tcagtttaag aattgttccc 420
   gtcagtcaca ttttttggcc ctcagaagtt cattcctaaa gatttcaact actctaaatt 480
   tctagctacc aagaagttaa gaatgattat aagaagcttt ccaaggagtt atgaaatctt 540
   tgtagaccag aggccaacta tcatcacctn gagtctgntn ttaccaacag cccttgnatt 600
   tttcanggag aaatctctag gaaaaagcag acaccagtgt agtactatct tccatgtcaa 660
   acctanggga ctaaaatggn cagtattacc nttaaaatgg tactttgang gttaccctaa 720
   aangnttatt ctgggctcaa aaantnggtt aggaatatct tttctggaaa gaatattggc 780
   ttaa                                                    784
<210> 130
   <211> 772
   <212> DNA
   <213> Homo sapiens
<220>
   <221> misc_feature
   <222> (1)...(772)
   <223> n = a, t, c or g
<220>
   <223> nbla-03804-f
<400> 130
   cactgttgcc ctactggaga tgaaaaaaaa caacatccca gagccagttg tcatcggaat 60
   agtctggtca agtgtaatga gcactgtgga atggaacaaa aaagaggagc ttgtagcaga 120
   gcaagccatc aagcacttga agcaatacag ccctctactt gctgccttta ctactcaagg 180
   tcagtctgag ctgactctgt tactgaagat tcaggagtat tgctatgaca acattcattt 240
   catgaaagcc ttccagaaaa tagtggtgct tttttataaa gctgaagtcc tgagcgagga 300
   gcccattttg aagtggtata aagatgcaca tgttgcaaag gggaagagtg ttttccttga 360
   gcaaatgaaa aagtttgtag aatggctcaa aaatgctgaa gaagaatctg aatctgaagc 420
   tgaagaangt gactgaattt tgaaactaca ccctcagtaa agcaaacagg agttgtagat 480
   aaaatgtcat gtctcatgtg tcctggttct tacatcttct accttcctgt atcaagcatg 540
   atataagggc tttcatggca aattttattt taactggttc tatggntgct ggaaatgttg 600
   ggtttaagtt ctaaaaccat ggtttaagta actacaggag ctatagattt gaatctaatg 660
   gtgcattaag cctttcaagt atcttctacc ttctggattt ctactggaat aatgnaaatt 720
   aaggccttcc caatgnacag gttactttat tcctgggttt tataacagnt ta    772
<210> 131
   <211> 792
<212> DNA
   <213> Homo sapiens
<220>
   <221> misc_feature
   <222> (1)...(792)
   <223> n = a, t, c or g
<220>
   <223> nbla-03804-r1
<400> 131
   tgganncntt aagtgtaagt atggacttta tttttttaaa aaaaagtctt ttgagaacta 60
   caaattacaa agtaaaagca gatcactacg tagaaatcta gtgaatacat tttgcctgtg 120
   catgaaaaac tttatcaaag ttactcattt taacaaattc accccattca caacaatcag 180
   ttataatgtt ctttttaagg ttactatacc tttatttcaa attatattac atgttattcc 240
   ctcaaaatga acttttaaat gacaaacttt tggctcataa gaatggcacc ttcaaaaggg 300
   gcgatttcca ctcagtctct ggtggcatta ttccaggcta gtttcaccag ttaaaaacct 360
   tcaaaatgtt cacttttaac acaaagtctt cccataaaaa atgtggctac attttccccc 420
   caatgactac atacatagat gggtagcagc aagaaaagat gaaaatgaaa aggcagccat 480
   taaaataaat ggatcaattg natccaagaa atatttggct catggcaatt aaaaaacggn 540
   aactagggta tggtaatagc cagaagcttg agtcattact gagtatatta agagaaccga 600
   agccngtnca gtatgcccga gtaggaatat ataattgnca ntgnccttca tttggtatag 660
   caaatacttt taagnccatt gggaatctaa gctttatcta aattnccaan tngaacatgc 720
   tggcccttna caatttnaac aatttttact ggggttaaga cattaagcat tttctttact 780
   naaactccat an                                              792
<210> 132
   <211> 1230
   <212> DNA
   <213> Homo sapiens
<220>
   <221> misc_feature
   <222> (1)...(1230)
   <223> n = a, t, c or g
<220>
   <223> nbla-03831
<400> 132
   cactgttggc ctactggctt tttttcagcc caggggccgg cgcacgaaac ctgtttggag 60
   gttatggatg ataaacccaa tcctgaagcc ctaagtgaca gttcagagcg tcttttctcc 120
   tttggcgtca tcgcagatgt tcaatttgca gacttagaag atggctttaa tttccaagga 180
   accaggcggc gatactacag acatagtctt cttcacttac agggtgccat tgaagactgg 240
   aataatgaaa gcagcatgcc ctgttgtgtc cttcagcttg gagatatcat cgatggatat 300
   aatgcacagt ataatgcatc caaaaagtcc ctagaacttg ttatggacat gttcaagagg 360
   cttaaagttc cagttcatca tacatgggga aaccatgaat tctataactt cagtagagag 420
   tatttaacac actctaaact taacactaag tttctagaag atcagattgt acatcatcct 480
   gagaccatgc cttcagaaga ttattatgct tatcattttg taccattccc taaattccgg 540
   ttcattttac ttgatgcata tgacttgagt gtcttgggcg tggatcagtc ttctccaaaa 600
   tacgagcagt gtatgaagat attgagggag cacaatccaa atacggaact gaatagtcct 660
   caaggacttt ctgagcccca gtttgtccag tttaatggag gattcagcca agaacagcta 720
   aactggttga atgaagtgct aacattctct gacacaaacc aagaaaaggt ggtgattgtg 780
   agccatcttc ccatttaccc ggacgcctct gacaatgtgt gcctggcctg gaactacaga 840
   gatgccctgg cagtcatttg gtctcatgag tgtgtggtgt gtttctttgc tggtcacacc 900
   catgatggtg gctactctga ggatcctttt ggtgtatacc acgtcaacct agaaggagtt 960
   attgaaacag ctccagacag ccaagccttt ggcacagttc atgtctatcc tgacaaaatg 1020
   atgttgaaag ggagaggcag agttccagat agaattatga attacaagaa agaaagagcc 1080
   ttccattgtt agtctaattt attttaactt gatagaaaat gagctttgtg tttgtccctc 1140
   ctaaacaaaa aaataaaaat cctctgtctc attgtttagt aaaaaaaaaa aaaaaaaaaa 1200
   aaaaaaaggc cacatgtgct cgagctgcag                                  1230
<210> 133
   <211> 2376
   <212> DNA
   <213> Homo sapiens
<220>
   <221> misc_feature
   <222> (1)...(2376)
   <223> n = a, t, c or g
<220>
   <223> nbla-03862
<400> 133
   cactgttggc ctactggctt cacaatattc tttatttctc tgtctctgtc tctctctctc 60
   aagtcagagt gtacaacagt aagcaaagtt tggcctctgt tctcgcgtga aatcaagtta 120
   acatgctcca cctgttgata tgtttgtaag agaaatctca tgtatatgca catatgcaga 180
   atttctgctc tttgcttctc aggaaatctc ttttctccaa tgtaggaaga acacattaaa 240
   atgaataagt catgttattt ttagaaaaca gaaaagcaaa taaatgtgtg aatagaatat 300
   gcactgtttc tgtgcttgaa acattgaaca ttgaatattg attgaaaggc caccatgaac 360
   tttgaaagac cactgtgttc agagaactgt gatagaaact aaaagagtat aaaaagatgt 420
   gatactttca tttttgagag gtttacagtg ggatgcagaa aaaaagaaac ctgtaaatgt 480
   gaatggcagt gtgtttggtt agtgcctact ggctatataa aattgctttt ggatgtgttt 540
   catgattcct tataaaacga agacttaata agtttacttg gcagctgatg ggcaaagttt 600
   taaaaaaaat caaatgagtt ttttgttttc ctttaagcag ttcctggcaa tgctttcttt 660
   ttttttattt caaacagatg agtttttaaa acaatgattg catttagaac cttcaagaag 720
   gcttgttaac catggatttg atgagccggg tcctgagatc tgaactggga gctagcttgg 780
   tgcacacatg gcagggccaa ggaggtcttg gatttccatg taagggatac acagacaaat 840
   aaacgtgata agggcaaacg ttagccttgc accctttgag tagcaaatca cagcgtatat 900
   tgtgtagtcc atgaagactg gtgtcatcca tcccagggcc tgggtactgt gtacagggtg 960
   agcaatgact tagatggaga aaatatttat catttgtttt gatcactttt tcattgcaac 1020
   cacatttcct aaggaaatgg gtataccagt tcaaatattt gctcttcatt tcatgctgct 1080
   tggcagtctt tgcatccttt gctctctgac ttgttcgcct ggctgtgcag tctgtttccc 1140
   aagagtgctc ttgatgagat attttgtttt tatcaggaaa ttgatttctt gcatcttttt 1200
   ctgagattac tgaatccaat attggagtag aatgactcaa ttcttggaaa aatgtctaat 1260
   ttgggactct ggtggttgat ttttattttc cagaggttca aagccgaatt tgagcatgtt 1320
   ggtgggagag gcagaggaga tgccttgcgg cccacctggt tcttccttgc atgaagctct 1380
   catcctcctc cagcagtgtg cctcatcaca ctgggctctg ggtgtatctt gcttccactg 1440
   gagagagcat cctgctccag tgtagctttc cactgtttcc ctagttctaa atcttgactg 1500
   aggtttcttg gtcttattct ttaattcctt aagcatcctg gcagttcctt actgggctgt 1560
   ctgcctttac aattgtgcgt tgttgcattt ttggtgagat tagcctaaat tcaccactct 1620
   tccttggtgt cgagaggtgg cttagtgccc taagctttgg gttaataaag gctggactct 1680
   tgagtccagg actagcacag ctgtctgtac tctgtgtctt cccataagag atgcaccaag 1740
   cagttttatt atgtgtaagg gcctttgcca tgatactttt gagaagggcg atgtgtatat 1800
   ttatatttca ccttgatgtt ttattcatga ctgatacttg tttagtctat gatggcagta 1860
   gggagtagga aagcagtctg tgtgtctgag tctggttcat ttaggtctta aactcagaac 1920
   tggtttaatg cagcacgtgt atgtagttat tataaacaat gatggcagga atgcacaatt 1980
   ttctgcaatt aaaatgccca gtatattcaa ttcactggat tttaccctgt gaccactgtg 2040
   ctgtttctct ctatgtgaag aaatatactc aattaagtgt tgcataatgt gagctttgtc 2100
   agtccaaaaa attttttcaa gttgcttgtg gtgtcattgg tatttattct gcaacgtcag 2160
   cagtgcttgc gggccaaatg cagagtgcag gctgtggttg aagcaacttt tttttgttgt 2220
   tgttcacatt ccaacattct taagaactgc ctgaaaagaa atgttggaac tgctttctaa 2280
   aagtactttt cagagccgtg cactattgga gaagttaaga accttacagt tccgaaaaaa 2340
   aaaaaaaaaa aaaggccaca tgtgctcgag ctgcag                           2376
<210> 134
   <211> 2145
   <212> DNA
   <213> Homo sapiens
<220>
   <221> misc_feature
   <222> (1)...(2145)
   <223> n = a, t, c or g
<220>
   <223> nbla-03898
<400> 134
   cactgttggc ctactggaat tattcagttg cggagacctg tttgagaaaa aaaactcttt 60
   gtcttcttta atcaagtgtt gtattgtctg tggcactgtt ttaaatgaaa gacaattaaa 120
   ttgctttgct gttttataca ttgttgtctt taatcactag tctaaactct atgtttttat 180
   gaaagcatct ttaaattttt tttcttagct gttctttctt gtttgtggta taacctttct 240
   gtaccatctt ttggttctgt ggaaatgccc ttaataacac ataggattag gactaaattt 300
   tggagatggg taagtttgag caaagagtca gtcaacacag gggaggattt ttgaaatttt 360
   atctctaaaa acagttttcc aattcagagt ttttaaaacc cttttaaaaa tatagttagt 420
   tttcagtggt ttcttttact tttagtgttt ttacacttgg aagtcagata tctaaaaata 480
   gggaatgttc ttttgctatt tttagatctc tactaaaatg taatctgtag tgttttcttg 540
   tttcagagca tatcttaaaa gattcagaca agtggcattt ggggacctct tccccatcca 600
   ctggctttca ctcaaaggaa aataagactt cttggttctg gcagatactg tctctggcag 660
   aattggtctc actgttttcc ttggggagca ttttaggtag tatgttgaaa gacagatata 720
   catcagttga agacaggatc agatgctatc tggttaataa agcttatgat cagggaaggg 780
   gcaaagaaga cagataccac taccattttg ttctttctgg ttttactaat atgaccataa 840
   tgagtcattt tttatgcata taggctatgt gtttcaggtt gcctttcctt ttcctcctac 900
   agatctattg agctttgtgt tctaaacaag atagtgtgct tatctgaatg tttcccatct 960
   gtctttgatg aaaaagctcc cagttaaact aatttggatt tatttatttt tcctgtctat 1020
   tccagttctc tgctatgtgt gggcaagtgc ctgttttatc ttgaggggta gattttagca 1080
   tttgaactct ctcccttttt aaaatcacct tgttacttac agatcatctc agtccagtaa 1140
   cttttcttta taaaggttaa aagattgttt gctttcttct caggtagtct cagtgttctc 1200
   agccttgaga gggaaaggga catacttaat attttcttgt cttgcttgct aagagctgtt 1260
   tttccttcgt catgtgttgg gcagggctag ccacccatct gttggaccag ctacttcata 1320
   aaactttcaa aggatgatag taggtgaaat gaaattgaca agagtgttgg atgcaggtag 1380
   aatgaagggt ctgctgtagc gtgtatgtgg acttctttct tttgtttatg ttcgtaaaag 1440
   tggagagact ctggatatag aaagggtaat agcaaactga tatctccagt acctgtctcc 1500
   tatatgatca aaaacattaa caatgtgttg gttttgtaaa attgctactg ttttgttctg 1560
   aagtgctgta gccattagct ggattgtaac agtaatatga cagctgtata gtaaaatact 1620
   gtctctcttt atgataggaa atgaaaaagc atctgttatg aagcctcagt gaactaaaag 1680
   ccattctctg aaaagtcaag acttttgggc tttatcagta gataaacatg agccataggt 1740
   tttctagcaa tagaatattt taacctatat gaatatatgc tttataggtg agactgctat 1800
   ttaatgagag ttttaaagta actaaacctt gttgacagaa ttcaggatgg aaagttttac 1860
   cctaaataaa acttcaggat attgaatatg atagcaaagt tccagggtat gttttatatt 1920
   tatgaacaat tttcatttga atatttggag cttgggggtt ttggtgagac atgttcatgt 1980
   atgttatata caaactttcg ggcctggcat gtggctcaca cctgtaatcc caacacttca 2040
   ggaggctgag gcgggaggat cgcttaagcc caagagttca agacccatct ctacaaaaaa 2100
   ttaaaaaaaa aaaaaaaaaa aaaaaaggcc acatgtgctc gagct 2145
<210> 135
   <211> 2592
   <212> DNA
   <213> Homo sapiens
<220>
   <221> misc_feature
   <222> (1)...(2592)
   <223> n = a, t, c or g
<220>
   <223> nbla-03914
<400> 135
   cactgttggc ctactggatc gtataaaatc ttatgtggaa gccaaacatt aaactggtaa 60
   aaatcatttc aggttgaggg tgtatgttgg tgggtacgaa gtggtttcag agcttccctc 120
   tcagttttcc cagtgttccc caaagactcc taggacacct cgggggagct caggggaccc 180
   aatgcagcac aactagaggc cccagcctcc acactgcctg gtgggggggt ctagactgaa 240
   tcgtgaaatc accctatcta tgggctgtgt gtccagttgt tggggtgagg tctggggagt 300
   gggggatgca agtggtggag ggaatgaaag gagggaggga aacttccagt gcctcatcat 360
   tcaccctccc catagatggc acctgggctc cccggggctg ggtcaggctc tgagtgacag 420
   ccattgaaga gaagccagcc tccaggaaat ttctccagca tgactgggca tcctctctcc 480
   tagccaaata tatcagagct ttgaggaaaa tgggcttctg gccaggccac actcgtcctt 540
   aggaagagct gtttccatct gaggaatctt tttgtagaca ggtgctggtc cttgaagggt 600
   aggtccgctg agcttgcgcc atagaatgcc tacaccactg gcatccttta gtcctgctga 660
   ggggagggac taactcctgg taattttcgt tttgttgatc aataaaggtt gttgtattgg 720
   caagtgccac ctgcatcact actacagatt cttagggacc taccctcaca tgtgaatctc 780
   gggttacatt ttgcaaagcc ctttcacata ttttgcaaag cattttgaca catttttcac 840
   atattccaca tattttgcaa agccctttca ctgtatttcg atgcacaccc acctgagatg 900
   caggtgctga gagtcttact ttacagataa ggaatcaggt taaagaggtt ccctgactca 960
   ggcaaggtca caaaactggt tagtgccaga gtggagaccc agcaatcaac tcttctcatt 1020
   ccaagccgga cggcatctcg ccatgccaca ggggccttct ggtggtatag ctggtgactg 1080
   aggacagaag agaagtaaga caatggcaga ctcttaattt ctagcaggtt ctctgcagct 1140
   cccccctgaa tctcccctaa tattcccact tacctcctca cccttccctg gtgagacaga 1200
   gcaggactct tccagagcag atttccattg ttatttttcg cgtctcagca ccacgtgtat 1260
   tgctattttt atcctgtact agaaactatt aagtggggag aaatgttaat tggtattatt 1320
   acagcaaact gtgtcctagt aacattgcaa ttcagaggta agctctccac tcccctcccc 1380
   tgccttttag gcaaacatag gaattttatt cattttgctg acagccagcc ctggtttcct 1440
   aggctggtgt ggggagaagt acttgggacc ctgagatgta ttggacaggc tcctgggcct 1500
   ctgctctgtg gctagtttat gaaggaagga agttggggtg ggtggaatag cgatgaattt 1560
   agttctgttt tatccactga tgctggactg gaaggaataa gatgtctgct caggaagccc 1620
   ttgtgagggg tggcttgcag catctttgtg aaagaatcag gggcatgtgg attcatagtc 1680
   cagctgaaag taagagacag tggccctcat ggtgtgtccc cgtaagccag agggaagaat 1740
   gagcagatta aaaaataata gcagtgaagt ccacacaagt tctgcctaac ttccagaagg 1800
   aaggggacac attattatta gggattcaac aaacgggctg gtttctaacc ctggcagctt 1860
   gccttcccat ctatgtaacc ttaagcaagt ctcttatcct ctctggcatc aatttcctca 1920
   tctgtcaaat gggctaaaaa tacttacatc tctgggttgt tgtgagaata aagtacattg 1980
   cacaaagcac acagtaaggg ctctggaaat taaagcttaa tagtgatttt ttttcctcat 2040
   gtgctctaag aatactgagg gctgagtgtg cactggtaga tggaaaatcc agtttccaac 2100
   ccagcagcat ccactgactg atcaaaccta acctgtgctg gataatacct tccaaatcct 2160
   ctctggccct tcctcctccc ctgggaagaa caatcagctt ggtttcatgg taccctacaa 2220
   tttttatttt ttataatgtt gattgttttt aagaattatt acaggctggg cgcgatggct 2280
   cacgcctgta atcctagcac tttgagaggc tgaagggggg tggatcactt gaggtcagaa 2340
   gttagagacc aacctggcca acatggtgaa accccgtttc tactaaaaat atagaagtta 2400
   gctgggcacg gtgttgtgtg cctgtaatct cagctactcg ggaggctgag gcaggagaat 2460
   cacttgaacc tgacaggcgg aggttgcagt gagccaagat tgcaccattg tactccagcc 2520
   tgggtggaaa gagcaaaact cggtccccct cacaaaaaaa aaaaaaaaag gccacatgtg 2580
   ctcgagctgc ag                                                2592
<210> 136
   <211> 754
   <212> DNA
   <213> Homo sapiens
<220>
   <221> misc_feature
   <222> (1)...(754)
   <223> n = a, t, c or g
<220>
   <223> nbla-03932-f
<400> 136
   gcactgttgg cccggtgaaa gccttcgagg tgacattttt caaaaattta tggaaagtga 60
   caagttcact agattttgtc agtggaaaaa cgttgaatta aatatccatt tgaccatgaa 120
   tgagttcagt gtgcatagga ttattggacg aggaggattc ggggaagttt atggttgcag 180
   gaaagcagac actggaaaaa tctcacagga agtaaaagag cacagctttt tcaaaggtgt 240
   tgactggcag catgtctact tacaaaagta cccaccaccc ttgattcctc cccggggaga 300
   agtcaatgct gctgatgcct ttgatattgg ctcatttgat gaagaggata ccaaagggat 360
   taagctactt gattgcgacc aagaactcta caagaacttc cctttggtca tctctgaacg 420
   ctggcagcaa gaagtaacgg aaacagttta tgaagcagta aatgcagaca cagataaaat 480
   cgaggccagg aagagagcta aaaataagca acttggccac gaagaagatt acgctctggg 540
   gaaggactgn attatgcacg ggtacatgct gaaactggga aacccatttc tgactcaatg 600
   gcagcgtcgn tatttttacc tctttccaaa tagacttgaa tgganaggan aaggaaaagt 660
   cccggcaaaa nttactgnca atgggaccan atctntctgg ggaagaaact caaattaagg 720
   ccaaaatgcc tttgtcanaa taaaaggggg gaan                         754
<210> 137
   <211> 759
   <212> DNA
   <213> Homo sapiens
<220>
   <221> misc_feature
   <222> (1)...(759)
   <223> n = a, t, c or g
<220>
   <223> nbla-03932-r1
<400> 137
   tttttnnntt ttaagactga tgacatgggg gatttcgctg gttctcataa cccagcatct 60
   atttgggggt atcagtgggg cctggaggca gtgggagaaa acaaacttca cagggcagat 120
   gctgctggga ccaggaaaaa ggcttcatgc cctaaggtcc tcactgtcgt acagcccagt 180
   gcaaagtatc attcattcat gacagatgca tgacgacatg aaagtcaata tacacagtta 240
   acccattttg acacattcat tcctcagcat tttttcaata caattcatct gaaattatac 300
   taagacataa aacagacctg cttttgtaca tccattactg atatgtctcc cagggtcagg 360
   cagaaggaac cagaatgact gtcacacaac tgctaattgt cgagacctga gacggactca 420
   cccatgagtg ctcctgcatg ccaggtactg cttgtcgact cgtgcacact ggactcaaag 480
   acttttgaaa actgntcttg ccctgaaacc taaaacttca cggcctgtcc ttcagaatca 540
   ctgnaaatct ttncaatgat ggacggatga atgccttcaa tgattaagtt aatcccatac 600
   acttggatag gaccgtgtcc gcanttccac ctgctgaagg ggncccggac aaactggcaa 660
   aagcanggca cgcatcgtgg tgccggntcc aaagganggn ccaagtgcna naaatatctt 720
   naacttttac ntcntnngaa aatancctta actttaacn                    759
<210> 138
   <211> 756
   <212> DNA
   <213> Homo sapiens
<220>
   <221> misc_feature
   <222> (1)...(765)
   <223> n = a, t, c or g
<220>
   <223> nbla-03949-f
<400> 138
   cactgttggc ctactggacc ctaattgagg gagtgaggaa gagagcagct cgcttctaac 60
   tggactgcac gttggtgaca gcgtcccaag ctggtgacag acccactctg taactttcag 120
   ctagattcag ccaccagatc ccagaaacat gacccttgct gcctacaaag agaagatgaa 180
   ggagctcccg ctggtgtcct tgttctgctc ctgcttcctg gccgatcccc tgaataagtc 240
   gtcctacaaa tatgaagcag acacggtgga cctgaattgg tgcgtcattt ccgacatgga 300
   agtcatcgag ctgaacaaat gcacctcggg ccaatccttt gaagtcatcc tgaagccacc 360
   ctcctttgat ggggttcccg agttcaacgc ctccctgcca aggcggcgag acccatccct 420
   ggaagagatc cagaagaaac tagaagcggc tgaggagcga aggaagtacc aggaagcgga 480
   gctcctgaaa cacctagcag agaaacggga acatgagaga gaggtgatcc aaaaggccat 540
   tgaggaaaac aacaacttca tcaagatggc taaggaaaaa ctggcccaga agatggaatc 600
   caacaaggag aacagggang cccaccttgg ccggcatgtt ggaacggttg caagagaang 660
   acaagcacgc cgangangtg ccggaaaaac aaggactgaa ggaagaagcc ttcaggtaaa 720
   gcctagaggc caagaacttt cangtaagcc ggncan 756
<210> 139
   <211> 761
   <212> DNA
   <213> Homo sapiens
<220>
   <221> misc_feature
   <222> (1)...(761)
   <223> n = a, t, c or g
<220>
   <223> nbla-03949-r1
<400> 139
   tttttttttt tttttatacc aaagggttta tttgcaaagc tgtaaaaaac tacacagtgt 60
   gcagagcaga gtgggaagag gtcctgggac ccggttaaca caggcagccc ctgcactcat 120
   gtttgcaggg gaagggagga ggagagcttc ttttccagcc atggttcagt gtcctggaga 180
   ccaagactgc tgggtctgga tgcctccttt gccttctact agcaaacact ttatttcctt 240
   gctcgtattt tctaatctac ctagaatctc aaacccccaa agcatggagg acttctgcta 300
   tggtgtgaat gttgctgtct cccccaatat acaggttgaa tcctaatccc gacactacag 360
   aattaagaga tagggcctta agaggtgaga tcatgaggac tccaccttca tgaatgggat 420
   tagtgcctta aagaaaaggc tcaaggaagc ccttttgcct tcccttctgc catgtgagga 480
   tgcagcaaaa aggcatcatc tttgaagcag atggcaagcc ctnaccagac actgaatctg 540
   ctggcacctt gatattggac ttnccagcct tcaaaactgt gagcaatcaa tttctggtat 600
   caattactta atctaangna ttttggtaca gcagtctgaa cggttgtgac taagacaaat 660
   tctttcttac aggcatcttg gggaccatat tanggatgtc aggtcattcg caagggaacn 720
   ccgcgtttcn ttttctnatn aaatttangg cntgctatcc t 761
<210> 140
   <211> 693
   <212> DNA
   <213> Homo sapiens
<220>
   <221> misc_feature
   <222> (1)...(693)
   <223> n = a, t, c or g
<220>
   <223> nbla-04021
<400> 140
   cactgttggc ctactggatt aatttactcg cagttgctgc tgctcaggaa gagagacaag 60
   gaatatttta acagaatcaa ggcatagaag aatcaccatt ttatttgagc ctctaatcag 120
   agtcagacca gtagagaaat taaataagat tagaaaactc tgtactgaaa gctgctgatg 180
   cttcaaaaat gaaaacaaga tctcacaact ctccctgtta gttgaaaata tatcaatttg 240
   ctctgaaagg attcagctgc ctagtgttgc cattactaac ataaacatat ggctcatatt 300
   tccatccaga gaaattaatg ctaaattggt gcctcgctaa catcagatac actgtattat 360
   gcttaaatat attcagtaaa atgtggaaag gggtattaac aacgacaaca aaaagatgga 420
   tttttttttt ctcacaatca cagttgctaa tccagtggga gatgtttgag agagttttgt 480
   tcaacatcac agtgagagtg cctagggaaa tcagaaaatt acaatggatt cccctttgat 540
   tgtaataagt gttgattttc tccatgagtt gtttatcctg tctagtgatt tgatggtgaa 600
   cttttctaaa taaatagccc tttcccctcc ggtgtcggta aaaaaaaaaa aaaaaaaaaa 660
   aaaaaaaaaa ggccacatgt gctcgagctg cag                             693
<210> 141
   <211> 2851
   <212> DNA
   <213> Homo sapiens
<220>
   <221> misc_feature
   <222> (1)...(2851)
   <223> n = a, t, c or g
<220>
   <223> nbla-04055
<400> 141
   cactgttggc ctactggcag atgaactagg tcagatccct tggaaagatt gaatatagaa 60
   ttttaatggc atcaaatagt tctgtccttc catattagac aattatattt caaccgaagt 120
   cacattttgg agaagactct ataccagaat cttagtaaga gctttttatt ctctgtgtag 180
   tagtaggata gctttttggg ggtgttttcc tggtttttcc aaattgctac aattttaaca 240
   attatgatca tgaatagcaa aaagaaagaa aacatcactc agaagtgaag aaaagcgctt 300
   ggtcagacac aaaagcccag tcacaaaggt taaaataacc atcattttgt gagccttttt 360
   acaatgcact agacaccgtg aggtgtgcat catctccatc cctcacagca gcactgaagg 420
   gtagatgata ttattcccag catcctattg ctatccagag ggaaaggagg cttagccaac 480
   gggctgcaaa cattccaatt ccttttcctg agatggacgc atgaactctc ttggcccaaa 540
   ggcattaaat attccgccca tgtaaccgat gccccttctt ggaattcaga gctcaccctg 600
   caacctgctg gttatcattt ggcttctatc acaggctgcc aacggtgaga gtacacatgg 660
   gtcacgctca tgtaaatatt ccagaccata tggcagttgt atttctcact gtaaatgaac 720
   acattggctt tgttctatag aaaatcaatt tcgcaaaaac ttgagttcag ttacttttgg 780
   actggaagga taaaaatttc ctaagccttc actgattgct gaaccttcta taccaagtct 840
   acatgagttc cacatccctg aatgctctct ttcagttgat tcgcttgctt tactaatcca 900
   aggactcaaa cattcacaca gctgtatatg gagaaaatat aaaaacaatt cacctttttg 960
   gcaactgagc tacgtaacca ataaaggtgg tttcacttca ggccattacc cagattagtt 1020
   ttactaaact tagctggaat gccatcctgc gcctttgagg aatgtgcaca atatgctttg 1080
   gtactggcat tgctaccact gtaattctct cacacaaata gaggcactac agtcgtaata 1140
   ttattccagt gaaaaactgg acctctctct ccctgggata ccacaaagct aacaatggca 1200
   aagcctccca aaaccaaagt gcacagaaga agacagtgct taagctaatt aaatctgctg 1260
   tggaaccagg agaattccaa gaacagcagt aacacatttt tattttataa aaatgtcccc 1320
   attgaaacaa cagatctgaa tgcaaatgta aaactaagca ttcgataatg ctcactttca 1380
   catctgtcaa cattttagag taacttcaag aggaatacat ttgtggttct agaaattctt 1440
   agtttatgca tagaatacat ttgagaggac tttgttttca caagtatcca ggtatcaaat 1500
   aaaaaaatac catggaatca cacacttttg aagctggaag ggatcatgag agtcatgtat 1560
   acctgcccct catttaacag gtgtgatgaa agttcagaga ggttcagtta tcgctcaaga 1620
   ttactcacat gactggattt caggaaattt gaaggcaaaa aataaattag ggatagattt 1680
   ccctctgtac ccttagcatt taaatggcaa atctctcagt ctgagataga gggctaaccc 1740
   agaccaagat tcactggtca cctgaccttt ctgtattttt acaaacaaaa ttaaatggag 1800
   aattgaaaca tttgcccaaa ttgctttgga taatttactg agctttgttt accttaaaaa 1860
   aaaatgaaat agagtagaat aaaaagggtg acacatctta cagtatttga atattaataa 1920
   aatcatattt gatgttaaat ttactgtttt tattcttacg tgagtatatt tcatgacact 1980
   tttcatgttt cagttataac tatatgtgtg tatatatatg tatgtgtctg tatgtatata 2040
   tattagatat atgctaagtt ataatatata actttttctt accatgaatc acagtaaaaa 2100
   agaccaaaat tcacttcagt ggaaatttca cttttgcccc actcaccatc tttaaaaaca 2160
   aaaaaccaac aatgaaaata aaatgtaaaa tagaagcttc tcataacatt ttgggggaac 2220
   cttgcattaa actatcactt tcttgtcact gcagcccaaa atgttctttc tccttcaggc 2280
   ctagtgcaat gtcctatagg ttactaaaaa agaaacatta aacttaagaa actagaaaga 2340
   gggcaaatgc acaaaagatg aaaaaggaaa cagatcttcc tttcgttgtt ttaagataca 2400
   tgtttttttc tttcctaata tcaaagtttg gggtaaacaa caaatgtaat ttgctcagtt 2460
   cctaggacag tagatgtaca ataatacgtg ttaaaggaac accaaaataa gggtaaaaca 2520
   gcagacttta gactctgcta ctaacctgta caaaacttgg tggagtttat aatttctctg 2580
   agcttcaact ttctcaaccg taaaaggtga tgactgggca agagctgcat ttctttcagg 2640
   gatctttgca gaactctttc catgggctct taataaatga cccatgaaaa tcatttctaa 2700
   atgataggtt aatagtgggc ccttcaccga tttctacctt cttatgtatt catgatcatc 2760
   caatgagtcc taactttcca gctcagaaat ggtgacctcc aaagaaaaag aaaaaaaaaa 2820
   aaaaaaaagg ccacatgtgc tcgagctgca g 2851
<210> 142
   <211> 2157
   <212> DNA
   <213> Homo sapiens
<220>
   <221> misc_feature
   <222> (1)...(2157)
   <223> n = a, t, c or g
<220>
   <223> nbla-04072
<400> 142
   gaattcctcg agcactgttg gcctactggc ttgataatcc ccctgattcc cagactcctt 60
   ggaatagctc cacagctatc agtggttctg ccacaggttc agagccttcc cagtactgcc 120
   ttcttatttg agaggtgtgc taagagctat aaagcagagg cttcaattgt acacagtttg 180
   gaagtttagg caaaagtcat ttcttcccta tattttgtca tgcttatctc ctgtctcttt 240
   ctgttttaca gattagcaat aaactcctta aaacccaaag gtttgggcct ctgttccttt 300
   cacttgcagt cagacatgga gttagtggta gaagaaacag aaggggtaac ctgcatggtg 360
   acagctactg aggggatgga taggaaagca ggctgagtcc ctggggccag tggttaccaa 420
   agccaaggag agagcaaggg gagcccagtg ggcctggcca tggactgctc tggaattccg 480
   agtgtgaact ttcagccaag aaggtagtgt gaaaatatta ctgtgaggtt ttaaaagtac 540
   acaaataaca attgtttttt gtaaaaagaa aaaaaaaaag ttagaaaaag agatatgcca 600
   aaaagaagaa agtaaaaaat acacgacttc tgtcttctgt taacatatta aaatttattc 660
   ttttagattt ttatgtgtct atatacatgt aaaatatata tgcatcccca aattgtatta 720
   tactcttttg agacctcaca tttttttcac ccaagaatat aacattaatt tctttccttt 780
   agaaaagaca tgagactaaa gaatgtttat tgagttgaga gtttcccaat caaggagtat 840
   gactctgtat aataataaat ctaatgtttt attagaaatt ataataaaat tagaaattta 900
   atatttggca ggatgtgagt tgcctgcata tgtacaccct ttattatatg actagacact 960
   cttttgtgtg gtcatggata tattggcatc ttttgaagtg tatccttttt gtttcaggag 1020
   cttctgtcta gatgaatagc aaatgtgaca ctgataatag taatcgaata ttcctccaag 1080
   tacaggctcc ttcctctttc tcctggacct ttgcacatgc tgcctggaaa actcacccac 1140
   aacctacttt ccatcctctc cttcggctca tccctgctca gccctctttg ttgagatatt 1200
   tgccaagcca gattctctga gaagcctttc ctggcatctt cgtctatata attctgctag 1260
   ttgtttctaa aactatgttg ttagtgtata ttgtaatcac ctatttatgt ctgtttgact 1320
   gtgagcctct tggagatggg aattctctcc tatgcattgc ggtttcccca ggccctagca 1380
   cactgtctag cccagtattc cttctcagta agtaggtgtt gaatgactta caaaatgaat 1440
   gctaaaagac ctggtagaca agaaggaatt tcttagccta agtgaccaga tgtggagaag 1500
   atactcaaac acttcagggt ccccgatctg gtgatgtcac ttgcagtgga atttcatatg 1560
   aatccagtgt gacagaagtt gcctagtcaa gttgtaagtc ctcttctctg ggtcacatgt 1620
   tcaaatactt ttaggagaca gataatgcaa tatttaagat acaggaagtg atgaagactg 1680
   tggcacactg gagagtttgg actccaagag taattaattc agttgaaaaa atcaacagca 1740
   ctctgttgat caagcaaact ttctaggggc cctaatccac ctgggcctgc agtatgtaat 1800
   tccataactc atgggatgcc atcaagagag acaatctact tgattgctaa agggcctttg 1860
   ttttctttgg agttccccag gaaggagaaa tgtatgtgtg tgcaagggtg aggagggggg 1920
   agagtgaaga aacaatacta tccccttaaa aaggtttgtc agttttcatt atgaaagaag 1980
   accaaacgtg cccactccag aaatcttggc atatgcagtt attttgaagc cattcagcgt 2040
   tcttcaagat gtcacaatga aaagagtgat ggttttttaa ataaaaaatt aacatgtttc 2100
   tcttggcaaa aaaaaaaaaa aaggccacat gtgctcgagc tgcaggtcgc ggccgct    2157

<210> 143
   <211> 2579
   <212> DNA
   <213> Homo sapiens
<220>
   <221> misc_feature
   <222> (1)...(2579)
   <223> n = a, t, c or g
<220>
   <223> nbla-04099
<400> 143
   gaattcctcg agcactgttg gcctactgga agttagaaga aaacaatcac aaaactgaag 60
   cctaagagag aaactgtcct agttgtccag agataaaaat catatagacc aattgaagca 120
   tgaacgtgga ttgtatttaa gacataaaca aagacattga cagcaattca tggttcaagt 180
   attaagcagt tcattctacc aagctgtcac aggttttcag agaattatct caagtaaaac 240
   aaatgaaatt taattacaaa caataagaac aagttttggc agccatgata ataggtcata 300
   tgttgtgttt ggttcaattt tttttccgta aatgtctgca ctgaggattt ctttttggtt 360
   tgccttttat gtaaattttt tacgtagcta tttttataca ctgtaagctt tgttctggga 420
   gttgctgtta atctgatgta taatgtaatg tttttatttc aattgtttat atggataatc 480
   tgagcaggta catttctgat tctgattgct atcagcaatg ccccaaactt tctcataagc 540
   acctaaaacc caaaggtggc agcttgtgaa gattggggac actcatattg ccctaattaa 600
   aaactgtgat ttttatcaca agggagggga ggccgagagt cagactgata gacaccatag 660
   gagccgactc tttgatatgc caccagcgaa ctctcagaaa taaatcacag atgcatatag 720
   acacacatac ataatggtac tcccaaactg acaattttac ctattctgaa aaagacataa 780
   aacagaattt ggtagcactt acctctacag acacctgcta ataaattatt ttctgtcaaa 840
   agaaaaaaca caagcatgtg tgagagacag tttggaaaaa tcatggtcaa cattcccatt 900
   ttcatagatc acaatgtaaa tcactataat tacaaattgg tgttaaatcc tttgggttat 960
   ccactgcctt aaaattatac ctatttcatg tttaaaaaga tatcaatcag aattggagtt 1020
   tttaacagtg gtcattatca aagctgtgtt attttccaca gaatatagaa tatatatttt 1080
   tttcgtgtgt gtttttgtta actaccctac agatattgaa tgcaccttga gataatttag 1140
   tgtttttaac tgatacataa tttatcaagc agtacatgaa agtgtaataa taaaatgtct 1200
   atgtatcttt agttacattc aaatttgtaa ctttataaac atgttttatg cttgaggaaa 1260
   tttttaaggt ggtagtataa atggaaactt tttgaagtag accagatatg ggctacttgt 1320
   gactagactt ttaaactttg ctctttcaag cagaagcctg gtttctggga gaacactgca 1380
   cagcgatttc tttcccagga tttacacaac tttaaaggga agataaatga acatcagatt 1440
   tctaggtata gaactatgtt attgaaagga aagggaaaac tggtgtttgt ttcttagact 1500
   catgaaataa aaaattatga aggcaatgaa aaataaattg aaaattaaag tcagatgaga 1560
   ataggaataa tactttgcca cttctgcatt atttagaaac atacgttatt gtacatttgt 1620
   aaaccattta ctgtctgggc aatagtgact ccgtttaata aaagcttccg tagtgcattg 1680
   gtatggatta aatgcataaa atattcttag actcgatgct gtataaaata ttatgggaaa 1740
   aaaagaaaat acgttatttt gcctctaaac ttttattgaa gttttatttg gcaggaaaaa 1800
   aaattgaatc ttggtcaaca tttaaaccaa agtaaaaggg gaaaaaccaa agttatttgt 1860
   tttgcatggc taagccattc tgttatctct gtaaatactg tgatttcttt tttattttct 1920
   ctttagaatt ttgttaaaga aattctaaaa tttttaaaca cctgctctcc acaataaatc 1980
   acaaacacta aaataaaatt acttccatat aaatattatt ttctcttttg gtgtgggaga 2040
   tcaaaggttt aaagtctaac ttctaagata tatttgcaga aagaagcaac atgacaatag 2100
   agagagttat gctacaatta tttcttggtt tccacttgca atggttaatt aagtccaaaa 2160
   acagctgtca gaacctcgag agcagaacat gagaaactca gagctctgga ccgaaagcag 2220
   aaagtttgcc gggaaaaaaa aagacaacat tattaccatc gattcagtgc ttggataaag 2280
   aggaaagctt acttgtttaa tggcagccac atgcacgaag atgctaagaa gaaaaagaat 2340
   tccaaatcct caacttttga ggtttcggct ctccaattta actctttggc aacaggaaac 2400
   aggttttgca agttcaaggt tcactcccta tatgtgatta taggaattgt ttgtggaaat 2460
   ggattaacat acccgtctat gcctaaaaga taataaaact gaaatatgtc ttcacaggaa 2520
   aaaaaaaaaa aaaaaaaagg ccacatgtgc tcgagctgca ggtcgcggcc gctagacta 2579
<210> 144
   <211> 728
   <212> DNA
   <213> Homo sapiens
<220>
   <221> misc_feature
   <222> (1)...(728)
   <223> n = a, t, c or g
<220>
   <223> nbla-04106-f
<400> 144
   cactgttggc ctactggttt atgtcatgtt tagaattgtg tgtgatagag gctgtacata 60
   cacgttttac aggaagcgcc tggcactgtg tgaggctggc ggttgaatgg tcaggagtgt 120
   agtagcggct ctaagccagc ctcacagccc tttctcattt ctcgctccat ccccctcccc 180
   gttcatttcc aggcctttcc acgaagagtc ctcagccttc tccctccagc cggaagagcc 240
   ccctgagtct gagccctgca tttcaggtgc ctttgggccc agctgaacag ggctcatccc 300
   cactcctgag ccctgtcctc agtgatgctg gcggagccgg gatggactgc gaggagccca 360
   gacacaaggt gggaccctcg aggctgagaa gctagcagag ggttgcaggg tgggcctgtt 420
   ttcactgttc cttcgggtgg tgctggtggg gaccctgggg cggggcaggg aagggctctg 480
   cactcacagc cgcttaccag cctcctgagc agagaggagg tactgggccg ccttcctagc 540
   aaaaccttct gagactttcc tcttatctct gggactccag gttccttcgt acaaaaacga 600
   agtttcctta ttcatttagc aaacggtccc tggaacccac aggcttcatg tgtcaagctg 660
   gatctttcag cctncccact cttccttcct tccttccacc ttcttcattt ctccttcctt 720
   cctcctcc 728
<210> 145
   <211> 746
   <212> DNA
   <213> Homo sapiens
<220>
   <221> misc_feature
   <222> (1)...(746)
   <223> n = a, t, c or g
<220>
   <223> nbla-04106-r1
<400> 145
   nttttncttt gacccaaaga aacagtganc taaagagatt gtcattaggt ttccaaaata 60
   aaataaaaag acatcttttt tggaaacagt cttgctgcca gggcccagaa ctagcnctca 120
   cttgcaggcc tcgtgcagca gtacgtttaa tatcctttgc tgcctccaga ccagggggct 180
   ccttccactc cagtgggatc cagggggacc ctcagggcac caggaaggag gagactgccc 240
   tgcctgagcc atcctacctg ctgagagtcc cagcatccca cgggtagccc tggcatctgg 300
   aaggcaggcc ttggacagaa tatcctctgg ccaattcggg ccaaagcagg agtgctgagg 360
   tgtcacaatg acatacctcc ccctgtacca gcactgactc cctccctcct agaggcccca 420
   tggcctcttc catncagctg ccantcaggg accccatgtn tnccttctct gnggactttc 480
   anncncnata tantttacan acttccttgg acnctgagtc tttcttttta nggtccntcn 540
   gcnttnccgn atnntgcagg nananttncc tgnncttngn atagtgnang ccacctttga 600
   ggcncagnat tncctgnctn tggttntcct ggttnttgnc tgnangantc tgtttgnttg 660
   nnncntttnt tcttggttan ttntntntta nnccttatnn nttattnttt gnntnttngn 720
   aancatnncg ggncttcnnn ntnntc 746
<210> 146
   <211> 2378
   <212> DNA
   <213> Homo sapiens
<220>
   <221> misc_feature
   <222> (1)...(2378)
   <223> n = a, t, c or g
<220>
   <223> nbla-04137
<400> 146
   gaattcctcg agcactgttg gcctactgga attaaccagc agagccagct ggctgaaaag 60
   ctgaagcaac agatgttgga tcaggatgag cttttagctt ccacaagaag agactatgag 120
   aagatacagg aggagctgac acgtctccag attgaaaatg aggcagccaa ggatgaggtg 180
   aaagaagttc tccaggccct ggaggagctg gctgtcaatt atgaccagaa atcacaggaa 240
   gtggaggata agacccgggc caatgagcag ctgacagacg agctggccca gaaaacgact 300
   acatcgacaa ccacacagag agagctgagc cagctacaag agcttagcaa ccaccagaag 360
   aaaagggcaa ctgagatcct gaatttgctg ttgaaagatc tgggggagat aggtggaatt 420
   attggcacca atgatgtgaa aactttggca gatgtgaacg gagtcattga ggaggagttt 480
   accatggccc gcctgtacat cagcaagatg aagtcagagg tcaagtccct ggtgaaccgc 540
   agcaaacagc tcgagagcgc ccagatggac tccaacagga agatgaatgc cagcgagcgg 600
   gagctggcag cctgccagct gctcatctcc cagcacgaag ccaagatcaa gtctctgaca 660
   gactacatgc agaacatgga acagaagagg aggcagctag aagagtccca ggactcgctc 720
   agcgaagagc tggcaaagct ccgagcccag gaaaaaatgc acgaagtcag cttccaggat 780
   aaggagaagg aacatctgac gcggttgcag gatgctgaag aaatgaaggt gtgtgtggct 840
   gccccttcca tcaagcccag tgtgcatttg atgcatttgg attggaaatg cttgttctag 900
   gaaaatctgt caccttcaga tccgtatatg gcaagggaca ggggaggggc tgttgttggg 960
   cattcagagt cgatctcaga gtatagcctg gcttagaaat gaacaagtcc agaaacccca 1020
   agatgtctgt agagaatggt taagttgcgt taataccttt gtcttcacca ttcctgatga 1080
   agagggaaag ggtagcaccc ccaatcactg catgggctct tttgtcggag ttaacttcta 1140
   aggatttctt cctccaacct ttacgcagaa cacaccaaat ttgagtttct atctcaaaca 1200
   tagagatttt tcttttggtc acattttaaa ctgtaaagga aaattgattg tggagtaaga 1260
   cttttaggtg aataaaatga gacaggtgaa gatgtaggat tttgattagt tgtaggcaaa 1320
   ctatgagcct cattgagtca aggtccacag ttaaagacgt ccacggcatg cccttgctcc 1380
   agccagcaga ataaaatcat gcgtcgtggg agtccctttc ctgttttcag agtgctgttg 1440
   ccaaaaggtc tcctttggac atcatcataa atctgtgcag tgggcagggc aggggctttt 1500
   atctcagttt tacatgtgag ggagtgcagc tcacacaggt tacaggctta tctggaggtt 1560
   acgtggatag ggagaggttg gtgccctctg actgacctgg ctggagaagc tcaggctggg 1620
   ctgtaaaagg tcaagggaac actttttatt tagttggtgg gctaattggc ttctagggcc 1680
   aacttggttt gataacagat cttgggtttt gttatcccaa taaagcaagg ccgggtcctg 1740
   ggggcaaaac agccagaaat gcccccagtt ttgggatctg acatctgagg gacacaggga 1800
   aggaggtagg tccagatcca gggcttgtca cagagtgggc tgagtgccaa cgagtagatg 1860
   acatgtttct cttggcctgg gatgcagaag gcgctggagc agcagatgga gagccaccgg 1920
   gaagctcacc agaagcagct gtccagactc cgagacgaaa ttgaggagaa gcagaaaatc 1980
   attgatgaga ttcgggagtg agtcggccca gggcaccagg ggtgtggggg tggtcatgcc 2040
   tcggtcctct tggggaagcc tggaaggatg tggctcttag tcgagggccc tgctcacctt 2100
   gccctgtggg cactgccctg gtgacacagc aggctgggcg ggctgcttcc aaggtctgtt 2160
   ctccgatctg gagctgagcc tcctggagcc ctggcatggc aggtggcagg cgggcccagc 2220
   tgcctctcct agtccccggg ggccagggtc acataggtga ttccgctgga tggacgcatg 2280
   ccccagtaga tgggggggag catctgtaat gaaagcacca aaaaaaaaaa aaaaaaaggc 2340
   cacatgtgct cgagctgcag gtcgcggccg ctagacta 2378
<210> 147
   <211> 4684
   <212> DNA
   <213> Homo sapiens
<220>
   <221> misc_feature
   <222> (1)...(4684)
   <223> n = a, t, c or g
<220>
   <223> nbla-04145
<400> 147
<210> 148
   <211> 4125
   <212> DNA
   <213> Homo sapiens
<220>
   <221> misc_feature
   <222> (1)...(4125)
   <223> n = a, t, c or g
<220>
   <223> nbla-04171
<400> 148
   gaattcctcg agcactgttg gcctactgga tatttctata aacttttggg aagttgcacc 60
   aaacccaata aaggtgcagt atataattaa agttttggtg tattaattag acaatctgag 120
   agctcacttg aagataaaag gacaccagat gtgtcaggga gaaaaagttg acatgcttta 180
   gataaaattt aaaatcaaaa gctttcaaat cgtcttatgc tctattatag cagaatgatt 240
   gcttagttca aagtcttgct gacaatttaa cacattcagt ttgatcattt gggcttagaa 300
   tttttttttc tctttcatat ttgcccctcc cttgtctcca aataattttt gagatgatag 360
   aagaatatac agtatatcaa atatctgaca gagggttccc tatcaggttc ctggctttga 420
   aggtcgagtc tcatttatat ttagttctct tgtttacaag atggaaaacg ttctttcagt 480
   tttatcactg tagtctactt aggtcaagct tttaatatga gcaaaggcaa cttattaata 540
   tttataagtt gtaaaattta ttgactttta aagaataaaa tctcagtaaa atctcacatg 600
   cctggaactt gatatttagt ggtgacattc tctcttcttt gtatttatta tgagtagaga 660
   agtagtttta cttcctgctg ggttgtattg atggagaata atgaagttaa cagtcacaga 720
   gagcaagtgt gcaggggcac gctcatcctt ggctgatatg agccccctaa tagagcagca 780
   ccacctcctc ctcctccttc ctgccatctg taaaagctga gacgaaatga caaccattgt 840
   accctcttgg tactgagttg atttaacctc acctttgaaa tctggtaggg aagaggggtg 900
   tcgtatgttt tgatttttcc tcccctctct tcacatctcc tcatccctaa ttcacttcat 960
   taatgaaaat tctcttttct tcttttctca gctcttccaa ctaaggcttt gggatataat 1020
   tacaagacat aaatcttgca gggaaagaac ttttggtttt aaagggggaa gccatcaaac 1080
   aaacatctca atgtcctcat gcctttgttt tgtcattact ggtcattgtt gtgttcagca 1140
   ttgtgggttt gattttcctt tttttttcta tccagatccc cccacaacta tccctcctcc 1200
   cacaacaacc accaccacca ccaccaccac caccaccacc atccttacca tcatcacagg 1260
   tatggtatct tcattaccat tgaaaatgtc ctgaatgtat attgtctttc tggtatgtat 1320
   aaaaagaagc cgaaaagttc cctttaaatc cttggaattt aatcgttcct ttattttgga 1380
   actccaacga cattagatac tatgttctta agttgaatct taaggacctc agaaacatcc 1440
   cagcatttaa cagtacgtca gatattttgc agcttatgca taaaagaatt cttttttaag 1500
   catcactatt caaagcaaaa tgctagggaa aatggcagct ggctactaga gaacacgcct 1560
   atagcttcta cttagacatg tttgatgaaa tggtaagtac tgtggctttc aatcttgtct 1620
   ctgtcttagt ttcatctcag tccatgtaag ggaaaatata cacacctaac ttcaatgtgc 1680
   ctatttgggc catgctacta aatgcatagt catccttcct gctccagaga gtacccgctg 1740
   acaagtgata ctacctgttt gtgctgccta ggtagtcaca agagatcaga gggagtcact 1800
   gtcttttcca tgctgagcac tcatgtgccc ctccctgccc tcggtcacat attggaagag 1860
   tggaagaggc agccactgtg gttctaactc tgcggcttta tctttttgga ctgtctgaaa 1920
   ctcttattca gcatgagttt tactgtctct tgtttctgtc actgagggag gtggcatgcc 1980
   atccagccct tcctggcatc cagtggctct ctcatactgc ttgatgtggc ttgtctggga 2040
   gccatggggt ttcttctgcc tacctaagta ggaaagcttc tggccacact atgtccactt 2100
   gacatgaagg atttccctag ataaggcctt cccaacctgt gtttacaggg acttaacaca 2160
   aaaacatgtg aaagtaatta ttcagtgaga aaaaaatatc tcttgaaaga gtaacttttc 2220
   cgctgcattt tgttagctgg tgagactgca aggtatctga accgtgtgcc ctggtaccca 2280
   cccctgtctc tggacttctt tatgaacact gagtatttga ctccccaccc ttcatggtca 2340
   gctcctttta acaccctaga tatcattgct gtccttttca ttgacatgct agagacattt 2400
   ccaattttca tgggagtcag taagatagag atgatgaaaa ttcagagtgt tcaaagaaag 2460
   aggaggcctc atcagttgat agaatataca acagattgtc taatctcagt tttcttcact 2520
   tttctaccca ttacagtgga aaaaagaacc cttaaggaag ccagtgcttc tcaacattgg 2580
   ttacaaaacc atcagtgcca gcacttaatt taaaatcttc tgaaaatcca tcagttggtt 2640
   ttcatgtctc actctaaact cagctccaac caggctgctt cctagattct tgtctgtgtg 2700
   tgtttctgtt ttggctcttt gttacttcct tctgtaaact taagtaaact cagtttgttg 2760
   acctagtgaa ttgattcttt ccattttcta tactattcac cagcatatca aagatgcttc 2820
   ttcctaactc tgatttccag agtaactgtg ctatcagctt ttcatactaa gaactaggtt 2880
   tttacctttt cacatttctt gcctcctacc cagttcgtaa gccaaattag actcgtactc 2940
   ttgtgaagtg ccctttagat actagtgtaa aatatactgc gtaattctcc attgccctac 3000
   atatattaac cctttaagaa aatatatcac cagtctattt tatcttcgca gaacttgccc 3060
   gggtatgagc cagcatttat taccactcat tctagaaggt gccagttagg tagctatcca 3120
   ggtttacatt ttaaggcagt cgagacctta ttcacgtggt atataaacaa ccacatttct 3180
   cttttatatg gaacaacacc tttcatttgc ggctggcctt ctcactagct tatgcttttt 3240
   tttttaagac ctttcttagc actcgctgaa ctcctccccc tcacaatcaa tctcagcaac 3300
   tcagcagagt cgcttcagtt cacagctctt aatccttcac tccagactaa agctaatctt 3360
   atgtcctcaa acagctacaa ctcactgtta tcacagttcc gactgcagag actccattta 3420
   agaggaaatt taaaaaataa acaatgctct atttctgtcc acatcaaagg tacctctaac 3480
   agaaacttgt ctttgcttct gtctctctgc tactggactc tcagctcaag gtaatgtaag 3540
   atgatctcat agatgcactt tagcctcttg atgttagcag ttgagtcctc cctgcgcaca 3600
   aaatacattt gtggtaatga atgcccagaa tctcactggc acacccaaga gtagttaaac 3660
   taaaattgct actaacacaa cattgtagaa ttcgttgcct taatggatgt gatcttctag 3720
   gcttactgtg tcttaacaag cagtcttata tatatttatc ttgcccagaa agtacacttg 3780
   tagaatttaa catttgctcc tttctccaaa cagattaaag gttatgcagc tgaatgattc 3840
   tgtgtcccat tttaaaggtc caggctatcc cctagattta gattagttcc taaaaaatct 3900
   tccagtctga atcctgacaa atggatagct aaattaattt ccactcctct tctctcttgc 3960
   actgtttggt acattacagc caccattagc cttaaaagct gcagcagcca aattcggtga 4020
   cagtaacata aagcctgagc ttaaaaaaaa aaaaaaaaaa aaaaaaaaaa aaaaaaaaaa 4080
   aaaaggccac atgtgctcga gctgcaggtc gcggccgcta gacta             4125
<210> 149
   <211> 739
   <212> DNA
   <213> Homo sapiens
<220>
   <221> misc_feature
   <222> (1)...(739)
   <223> n = a, t, c or g
<220>
   <223> nbla-04178-f
<400> 149
   cactgttngc ctactgggtt accaccacca aatctttacc ttctgaccaa gtcatgcttg 60
   tttatgatca acaagaagtt gaagctatat tcttggtgat cttggtagtt gtcataatag 120
   gagaattttt cagtgcctct tctgtcacaa tcgtagacac ggtcacactc cagtatctgg 180
   gaaaacacag agatcgctat gggttgcagc gcatgtgggg ctccctgggc tggggcctgg 240
   cgatgctgtc tgtgggcatc ggnatcgact acacccacat cgaagtgctc atcgatggaa 300
   aggggtgtaa gccccccgag tacaggaatt accanatcgt cttcatcgtc ttcggcgttc 360
   tcatgaccat ggccttgatc cgttgccact cagttccggt tccgctacaa ccatttcaaa 420
   aacgatgatt ctaaagggaa agaggtggan atcccgcagg tggaaaggaa caactctaca 480
   gagtcctctg aggagacacc aaccaccaca agccactcgc aggccttcaa cttttgggac 540
   ctaatcaagc tgctctgcan cgtgcagtat ggctcaatgc tggttgtggc ttgggtcatg 600
   gnttttggat atggcttcgg gttanctttc tctactggca tttggaagaa cttaatggac 660
   tcaacccttt ttggggtctg gtcaatcctg aatcatgtgt ctgactgaca gcatattntt 720
   tagtccaagc ttattgaat                                        739
<210> 150
   <211> 748
   <212> DNA
   <213> Homo sapiens
<220>
   <221> misc_feature
   <222> (1)...(748)
   <223> n = a, t, c or g
<220>
   <223> nbla-04178-r1
<400> 150
   cnttttaaaa atggtattca tttttatttc aacatgtcaa ctgtgcattt ccaaaacagc 60
   aggcttttca aaggaataaa tcagaactgt aaacacaaga tacagtacaa gtttttgact 120
   tcctacagtc agtttcacaa atccacatac tgtacattca taggtgaggt taagcctgtc 180
   acccatttct ttatttctat aattacacaa gcataataaa tacatctgat tttaaaggtc 240
   acttaaaatg agtcataatt tacagtacag tacgtttcag ttcaagtgca aaaaataact 300
   atttgctgaa ttctatttct ttcagttatt ttatttttaa gctgtgtttt attgtgaagc 360
   gagacgtcca agtgtagaat ttcttatccc aaatgcagta ttgggcagaa ctatggtgtc 420
   atggagaagc cctcacttta caattgatcc tgctaatcag ggtcagaggg taaggtgaag 480
   ttgctcattt cgactgntct tttctttcct ctctctctgt gctcatccac agctattata 540
   aattatataa ttcaccttta ttcaagctaa aacaggtttt ccctttttca cacttccccc 600
   aggtttnctg gtaacctctg ctttgntttt taagtggcaa gctttacgtg cgatccancc 660
   anccatggtc tggctggann tgnaacgttt gcagttgcat acattacatt gnccantggc 720
   tgactgggct tcatganaac gaatttnt                                    748
<210> 151
   <211> 747
   <212> DNA
   <213> Homo sapiens
<220>
   <221> misc_feature
   <222> (1)...(747)
   <223> n = a, t, c or g
<220>
   <223> nbla-04183-f
<400> 151
   gcctgttggc ctactgggat gcattcattt aaaatatatt gagcaatcat ataggttttc 60
   tgatgttcat aagccaagta ctattctgaa ggtgctgagt gactgctctg gttatttgat 120
   tgaaaaaaag atttttattc aatctttttt tgaaaaatct agtttctaaa caagttagtc 180
   taccttgtta cataatgtag gagaatgtta tgttttagag aattaggaat aattactatt 240
   gcaaaatcat tagattatac tcaataaaat ggntttaaaa cactcctaat ggtatgtgtg 300
   ccatctccta aaataaaaaa atgcagttca agaactttac tattaaaagt gtcctgaacc 360
   aattttttag ttgtcaatca atctacaatg atttcctgaa agaactcaga gaatcaagta 420
   cttgaaggat tataatatcc caaaatctta acatttattt tccagtaaca tttaagtaaa 480
   cattcctacc acaaatactg gacagagaat aatcttttgc cacctattat tctttatttt 540
   aagttgcata aattagcctt tngtctgtaa aaagatgaan gccnaaagat ctgggnnant 600
   tacnaatcta aagnttntga agngctantt cttgntattn nnttnaggat tncaatttta 660
   canntttttt gaccannnnt gntnnngctg nnnannttca ncnttgnnnn gatnntnann 720
   gggntnctan ggntnnnttn nntntgg                                    747
<210> 152
   <211> 748
   <212> DNA
   <213> Homo sapiens
<220>
   <221> misc_feature
   <222> (1)...(748)
   <223> n = a, t, c or g
<220>
   <223> nbla-04183-r1
<400> 152
   tttttcnttt tttgtacatt cgaacctttt taatgaaaat tacaggtcat cgcctggtta 60
   agtgttaaat agcatgcatc agaccatgag aaagctgaga cactccatcc cccatccaca 120
   ctcccgtcat cctcttccac tctcattaga atcaatgaga tgtaaggtga tgcactgggc 180
   atcttaataa gtgaaataag aaaaaagtag gaataagcta tgtgagtctt atacggcaat 240
   tttgctgact ggggaagagg ctcagtttaa ggaaggaaca tcatgtccag ttttaggcat 300
   tttaaacatc aaaccttgcg cctactgtgt aatgcagaaa ttcgactatt gagttgtttc 360
   aatgaaaatt tacaagactg agagcaaaac acattcacta ttaagtgcaa atgaatcaca 420
   aacaggcaga aaacacttcc acatagtaat tgctattttg agttgagatt ttaacttcta 480
   atctaaaact cctctgtttt gtgtcctcac ccttgctcaa ccacagaata aagacaatat 540
   tttggatctt ctggactaaa gaaagaactg gcagagaaaa atctcctcat actcagtcac 600
   tgnacaggaa gatggcctgt agtctagatt gggggggtgg gttctggcan tacaaccctg 660
   tcttccatnc caaagatctg aatttaaatt taagcaaagt aagactgagg gaaaaattaa 720
   cctaaagtaa atgnatggna atatatga                                   748
<210> 153
   <211> 741
   <212> DNA
   <213> Homo sapiens
<220>
   <221> misc_feature
   <222> (1)...(741)
   <223> n = a, t, c or g
<220>
   <223> nbla-04193-f
<400> 153
   gcactgttgg cctactggag agtatgatga tgttaatttg ctgactgctg acttccagct 60
   tctttttaac aatgcaaagt cctattataa gccagattct cctgaatata aagccgcttg 120
   caaactctgg gatttgtacc ttcgaacaag aaatgagttt gttcagaaag gagaagcaga 180
   tgacgaagat gatgatgaag atgggcaaga caatcagggc acagtgactg aaggatcttc 240
   tccagcttac ttgaaggaga tcctggagca gcttcttgaa gccatagttg tagctacaaa 300
   tccatcagga cgtctcatta gcgaactttt tcagaaactg ccttctaaag tgcaatatcc 360
   agattattat gcaataatta aggagcctat agatctcaag accattgccc agaggataca 420
   gaatggaagc tacaaaagta ttcatgcaat ggccaaagat atagatctcc tcgcaaaaaa 480
   tgccaaaact tataatgagc ctggctctca agtattcaag gatgcaaatt caattaaaaa 540
   aatattttat atgaaaaagg ctgaaattga acatcatgaa atggctaagt caagcttcga 600
   atgaggactt catccacttt ggctgcagcc agactgacag gtccttnaca cagtaaangc 660
   agccttggtg aagagagaaa tccactngca gnattancgn aataaagagc cgtccaggag 720
   gtcgttatna gcaataccat g                                          741
<210> 154
   <211> 793
   <212> DNA
   <213> Homo sapiens
<220>
   <221> misc_feature
   <222> (1)...(793)
   <223> n = a, t, c or g
<220>
   <223> nbla-04193-r1
<400> 154
   nnaagaaccc tttggactgt ccgcctactg ttgagtatga tgacnttaat ttgctgactg 60
   ctgacttcca gcttcttttt aacaatgcat ngtcctatta taagccagat gctcctgang 120
   ggnaactctc ttnncaaacn nnggcctntc nccangcnca nntaanntan gtanctntnn 180
   ttganacngc gtttnaccct gttgnncang atggnnttga tcttcnnacc tcntnatcct 240
   gcntcgncct cccaaagtgc tgggattaca ggtgtgagcc actgtngccc attcaactat 300
   gggattttta taatctnnag nttatgcann atgaaacnna ngcttacctc gtncatnanc 360
   tgnnnantnn cnacaaangt aantnttaac cacaatccnt nctncctgct naataatnaa 420
   tntanggtan ancttntctn aatatatttn gnncctnnng atnaatncaa cttgnaatnc 480
   cttatttctc gntaccnana accngtgcac ttnnannntn tanactaatt gtngcnannn 540
   ntcannnnnn gcggacatcc anaannctat tagacanntt ggttccnntn ncnnaanaat 600
   cctnctcnnt tngcgaatna anncncnnan nntntanntn ttnnttgcct tnnancttnt 660
   cncnaacnnt natnatnntn naacgtnnnt atnaatcntc nctgggancc naaatnnttc 720
   nntanccnnn tcnttcctaa ggnnatccct tttntctctc taccttncnn naaatttnaa 780
   tngcngnann ncc793
<210> 155
   <211> 2753
   <212> DNA
   <213> Homo sapiens
<220>
   <221> misc_feature
   <222> (1)...(2753)
   <223> n = a, t, c or g
<220>
   <223> nbla-04196
<400> 155
   gaattcctcg agcactgttg gcctactgga tcgtaatgct catcggaaga ataagccgtg 60
   tactgatttg gaaagtggag aggaaaagaa aaatattatc aatacccctg tgtcttcatt 120
   agaaatactt ttacaatctt tctgcaaact tggcctgatt atggcatatt tctatatgtg 180
   tgaccgtgca aatctgttca tgaaggaaaa caaattttat acacattcat ctttctttat 240
   tccaattatc tacattttgg ttttgggagt attttataat gaaaatacta aagagactaa 300
   agtattaaat agagaacaaa cagacgaatg gaaaggctgg atgcaacttg tgattttgat 360
   ttatcacatt tctggagcaa gtacattttt gcctgtatac atgcacattc gagttctggt 420
   tgctgcatat ttatttcaga cagggtatgg gcatttctca tacttttgga taaaaggaga 480
   ttttggaatc tatagagtat gtcaggtttt atttcgtctc aatttcctgg tagtggtgtt 540
   atgtatagta atggatcgac cttatcaatt ctattacttt gtccccttgg tcactgtatg 600
   gttcatggtc atatatgtta ctttagcact atggccacaa ataatccaaa aaaaagcaaa 660
   cggaaattgt ttctggcatt ttggcttact gttgaaacta ggctttttgc tgttattcat 720
   atgttttttg gcatattctc agggtgcatt tgagaagatc ttttctcttt ggccattgtc 780
   caagtgtttt gaactgaaag ggaatgtata tgaatggtgg ttcagatgga ggttagaccg 840
   ttatgtagtt ttccacggaa tgctgtttgc ttttatttat ctggctttgc agaagcgtca 900
   aatactttct gaaggaaagg gtgaacctct tttttcaaac aaaatttcaa attttctgtt 960
   gtttatttca gtagtttctt tcttgaccta ttccatctgg gctagcagtt gtaaaaacaa 1020
   agcagagtgc aatgaactcc atccgtctgt ttctgtggta cagattttag ccttcatcct 1080
   aataagaaac atccctggat atgcccgttc agtttacagt tcattttttg cttggtttgg 1140
   aaaaatttca ttagagctat ttatttgcca gtatcacata tggctggcag cggacacaag 1200
   gggtatcttg gtactgatac ctggaaaccc tatgctcaac atcattgtca gcactttcat 1260
   atttgtttgt gtggcacatg aaatttctca gatcactaat gatcttgcac agattattat 1320
   tcctaaagat aactcatctc tcttgaaaag gttggcatgt atagctgcat ttttttgtgg 1380
   actcctcatc ttatcatcca ttcaagataa atcaaaacat taggttccaa aaattctaaa 1440
   aaacctaaac tcttcaggct acctttgtgt gtctctagaa gagaaaagca tctatctgga 1500
   gatataaatg tgtatgtaaa tataaacgtt tgtggcaaga ggacagttct gtgacatctg 1560
   ttgaacatat gtggttgtat atattggaaa tgtacatatc caatatgaaa tactaaaaca 1620
   aacaaacaaa caaaaaacca gaatgcattg tataggattg catgtgaagt cttttctact 1680
   gaatctatat ttccatttat aagtgatttt aagttaacat atgaaggcag ggaaatgatt 1740
   acctttccag taaaaagtat agataattta attaacttag tgacaccacc aagtgttttg 1800
   atataactaa atttgtggta ataagactgt ctgcacctgt attcattgtg gaacttcctc 1860
   tttcattgga actttcttta ctcaagaatg acggcagtat tgttttctta tatgtgcaat 1920
   gaagtggaat gataaacagt atgcctttaa tttatatgtg ttcttgttct gatgttgttt 1980
   cctgaaatga tttttcttcc taactgtggt tttcgggtat gcaagcctaa atctttgtac 2040
   actttgtctc acagaatagt tctgaggctc catgacaggg ttttgtcatt gttgatgtta 2100
   ttgttgcttc gttttataaa aaagccaaaa tttttttcca atccaaacgt tcacctgttt 2160
   cctttcctca agctatacca gtgtaatacc agttaccctg tggatccatt taatatgtta 2220
   tccccactaa ttaattttcg tatattattt ccaatatttg gaaagctctt tatagccatt 2280
   tggtatttcc tattacccac ctcctatttt aaatatttat cagtctaaac ttgtgcagtg 2340
   tagtaaacat gcaagttgtt atgattgagc tgtattacca taagtagaat tttaagtaaa 2400
   ctggtgaatt tgggccatag atgtttttgc tttttgtttg attttttttt acaagctaac 2460
   tgttagaggt atacatttat ttatctgttg tacagatttg attatgattt taatgtttga 2520
   aagattgcac ttgtttgctt ttactatatg tggggtaaaa tatattttct gttcacagta 2580
   tatgaaaata tggagtaatt taaacagtaa ataaacattc tgtggatgct tatttttgta 2640
   ttggcaaagt atcaattaaa ctatatgtgt tctttttcaa aaaaaaaaaa aaaaaaaaaa 2700
   aaaaaaaaaa aaggccacat gtgctcgagc tgcaggtcgc ggccgctaga cta   2753
<210> 156
   <211> 809
   <212> DNA
   <213> Homo sapiens
<220>
   <221> misc_feature
   <222> (1)...(809)
   <223> n = a, t, c or g
<220>
   <223> nbla-04219-f
<400> 156
   gnagatgnnn nnttgnnnnc cnttntttga annccctnga gcactgttgg cctactggtt 60
   tatcaatgtg actccacggg ggagtccatg gtgatgatga tgaggaggag gatgatgatg 120
   atgagacacc tctaaacttg gaacaagttt aagactttat gagagaagaa aaaaaatcac 180
   caacaagaat tgtttgagga aaaattataa ctatcctgtg ttcatttttt ttttataaac 240
   aataagaaaa agttgttgga ttttttttta atgatttctt ttttggggga gggaattttg 300
   ttgcagtttt atggtggaaa atgcaaaaac cagagccagg tgcataatct tgtaatctgt 360
   ggatatccct ggagcaggac tgagtaccag ttaaaatact ttttggggat acacatgtga 420
   gatactaagt acttgcagaa gatttttgtc tctcttttta aagtctcttt ccttggaata 480
   ttgtgagaat atttgtggcc atttaaaggt ttgtgtgatt tttgctaaaa tgcatcacca 540
   acagcgaatg gctgccttaa ggaccgacca aagaactnag tgatttactg gattcagtgc 600
   natgttttca cctcctgtga gcagtgggaa aaatggccac ttcttttggc aagtggacat 660
   tttactggct caaatgtaga agacnnaagt tagctcaagg tcctgggggg aatggaggac 720
   atccaagccc cntccaggaa cctntgggng atgggacttc cntttgaccc ccatgaccag 780
   cangggacct ttgggtcaca ttgacattc                               809
<210> 157
   <211> 769
   <212> DNA
   <213> Homo sapiens
<220>
   <221> misc_feature
   <222> (1)...(769)
   <223> n = a, t, c or g
<220>
   <223> nbla-04219-r1
<400> 157
   gagnggntnn ntttgtggcc tttttttttt nnntttgttt ttctgntttt tgataattgg 60
   gaatgctgaa acctcttgcg tctgcgattc ataactactc agacttgtct tatattacaa 120
   aaatgggggt taaggagaag tgtttatgtg ggtttaagat aatacagctg ttaaggaagt 180
   ggtctcttgt tttaatgaag caatgtggca acttggaccc ttttacatct gtcccatgtg 240
   attcgatgcg tctcccattc cagggtgtgg gccggccaag gagagagggg gaggctccga 300
   ggacaccttc tcttcctccc ttcttttcag acacgcagct ttcggattca gattcctttc 360
   tcggacttgc tgctccagac tgaggatgac ggccaccgcc tggtggagga tcaggagctt 420
   ggtctggggc ttgcactctt gaggtggagc tgcaccatgc ggncgagctc tttgaaaagc 480
   ctcgttgatg tcacggaccc gaaacgctct cgggcattgt gggcatcttc ggtcctttta 540
   cgctctgcct tctgntctgg nggcaaggnc ctcatcgcan tattgntaga tcttgaccta 600
   ntaattgatt tgatatcctc ttggcgcatc taattcttgn cctccgaaaa attcgggctt 660
   tgaaggtctt ataaccttgg cattggattt gactcaaact gcanangaac actttgccct 720
   gtagcctggg gcatccttgt aaggncctgg ctgggttagn cagggaant 769
<210> 158
   <211> 768
   <212> DNA
   <213> Homo sapiens
<220>
   <221> misc_feature
   <222> (1)...(768)
   <223> n = a, t, c or g
<220>
   <223> nbla-04241-f
<400> 158
   gnnnnnnnnn nnnnnnnnnn nnttggattc ctcggcactg ttggcctact gggagtaatg 60
   aagggtgaat ttcgagctgt gaggcagtaa acttattgca gttcctcaaa cttagaaatt 120
   gggttgaatc ccaataccct aatttcctgt tccacatcac atttcaacta atacttgcct 180
   tttatcacag caaccaccaa caacctagct ttgcaaagaa atgacatcat caaaaggaaa 240
   ataacacaat atgttaaact gtgaattcgc ttgaaatagt cattcatgca agcatctgag 300
   aaatgttgtg tattgctttg tttccctcaa agacaaaata tcctgcacac ctctgtgaat 360
   ttgctgtagt gccctgaggt acactggcac ataatttggg gacctcagaa ctaaaatcat 420
   gtctttttca gcaactcttg accgcatcac tgtaacatat gagattgtaa gatgccaatt 480
   tggttaagac tacgagtaat gtgtaaagtg aaaattgtat ggggtcaagt attattttaa 540
   aaagcctctt angaagttac caggttggag gctgatatac catctnctat taagtataag 600
   acttactggc actggttcct tancattgaa acaggttgct attctttaag ancagatcct 660
   ananttagct tggctttgag cattttgcta anaaccaatn ccttggcnta aacccaacat 720
   cattntcagn gngngaaaag ctnacanngg aaaagnccat tccnagnn 768
<210> 159
   <211> 3553
   <212> DNA
   <213> Homo sapiens
<220>
   <221> misc_feature
   <222> (1)...(3553)
   <223> n = a, t, c or g
<220>
   <223> nbla-04246
<400> 159
   gcactgttgg cctactggtt atgccaaaga acaaacagtt cctgcagctc tcatgttgta 60
   ttcaccacaa attcaggaag tggctgaaga aggggattac atgcctgcac aaatctttag 120
   tgctatcgag agtgtcaagc tttgaaaccc tttgaatact tctgaagagc aagcgcccac 180
   tgatgctgag gtcaacaaaa tcagagaagc tgacatttcc attttttgcc aatacttcag 240
   gtgacctcat aatgaaaccc ttgctgctct acagaaaatt gtgcccaaac cctctcaggg 300
   gaaataaatg agccaagttt ccagtgtact agcaagcaaa cagaaaagcc cagatgaatc 360
   ttcctctcct taagggatgg tttgaacagt actttcttgt ggatgttcaa gactacttaa 420
   aagaaaaaaa aaataccttg aattcaaagt cctgctgatt cttcagtcta tttggtgctt 480
   caggtacatt tgccaatatg catcctcatg gtaaggttgt ctttataact agccacatgt 540
   ctgagattct tgagcctttc agtcagtgtt tgatctggcc attcaggaag gcttattata 600
   aactaatgta taactttgtt cacaatctcg caaagtttcc actgtctgaa aatcctagtg 660
   catgagactc ctacatcgtt attaatggca tatccttaat aaaagtttgg cttttgattt 720
   ttaatgggtt ttcaggagat aacttcccaa agaggcatta gatagtttaa cagagcctgt 780
   cattaatgtg acctgtgaga agacttggct agaggtggtg aaatatcttt cctctatccc 840
   tcccaaagac aagaaaaacc tatggatgag gatgaaaatt tggcacaaga gcaatcattg 900
   gcggaagttg aatctgaaac tgttgacacc aattcaagtt aatgctgcta gaggctgatc 960
   ctcaggaagc tttcttgact ccagaggtta ttatcataag tgatgatgaa gacaattagg 1020
   aggctgtggg actggaaaca aatacagcaa taagaaacag gagcaaaatt tttagaacaa 1080
   gattaaaacc tccctaagaa ggtaattaaa attggcatct ttacatgtgt cagatattac 1140
   ctgttcaaag tttgagtgac ttagagttct ataaagaggt gctatgatgc catcaaacat 1200
   aatcatattg gacagaaaca atcttcaata gaacttaaat catgtgccat ttattactgt 1260
   tgctggacag ctgataaaac taccttctga caaagtttga tttaattaga ctctaataaa 1320
   aggtcctatg agactttcta aaagactata ttgggaagaa agaaacctca gaaaagtcta 1380
   aattatcaag tagtaccatt taaatactct tactggacag ctaataagct accttcagac 1440
   aaagattgaa tgattaaatt gaactccata cagaactgct aaggtgtctt caaaaaggac 1500
   ttgagaagat gaaagcatct ttagaagggc cacttaaatt cacttgcttg atagaaataa 1560
   agcctcaagc aagttgttat aacttcagga ttcgacttca ctgactctaa gagtatagac 1620
   atccataatt tgaactaatg aatagtccac ttctgttcat tgcttctctg tcacccccat 1680
   ttgccactac cataatgagt gatagataca tcttcatcac ctctggaaat catctcagga 1740
   tctaaatgga aactgtataa agcctatcat ttttactgat ttaaactatg taaactcatt 1800
   attcttttta tgtaatgtgc tgttgttatt gtttacctgc ataaaaatat ttatgagggt 1860
   tttcaacagt ttacttgaga cctcattttt gcccattttt ttccttcccg atatcatgat 1920
   cccctcagct gaactttctt accttggggg ttgttcagga actgactctc atggggaaag 1980
   agggattact atttctgtgt tcctatctct tggcaactgc ttaaccacag tcagtcttga 2040
   actaatggaa ggagcactgg acttgggttc ttgagacctg ggttcatgtt cagttctgcc 2100
   actgattatt gtgacattgg gccagtcact tgatttctct gagcctcagt ttcatcacct 2160
   gttaagtgag gatagtaata cctggcacaa atatcacaat attagtgata attgaatata 2220
   attataagta cccaatggct attaaaagta aaactaggaa gtgctgaata accataatat 2280
   cattatattt gtagcatttt ggaccttatc aatgaacaac tgagaaaact aggtttttga 2340
   attcttttac tttttaaagt aacttcctcc catttttatg tcaattatag aaaattttaa 2400
   aaagaaaatt aaatgtgcct ataattttat aagccggagg taactaagtt ggtatttttc 2460
   ttcttagtac ctctttgtct catcataaat tgttcatcaa tgtcaaaaac ttggaaaata 2520
   aagataagca tatagaaaaa aataaaaacc acccataatc acaaatccca gaagcaatgt 2580
   taatattttg gtggatttat ttccagtctt tttctatggc tatatgtgca catatataat 2640
   ttttacatag aaaaagtcat aatgcataca gctttgttgc ttttagcatt tttatcatga 2700
   atattttcct acatttatgc aaagtatttg taaatatcat tttcaatggt gtataatatt 2760
   tcatcatagg atgacatcat ggtttagtta accattttct tttgttggat atttgagggt 2820
   ctttccaaat ttggccattg taatttcaca atgtcttttt cattacctaa ctgaaaatat 2880
   ttgctttggt gaaagcagag gattttttgt tgtttgtttg tttgtttttg aagaagtcct 2940
   tttaatagct acatttcatt gactaagtgg aacttcaaga gacaggtaga agaaaaaaaa 3000
   aaagaaacag tagatgtaat ttcaagattg aggatttatt ttgttagtga ctgttccaga 3060
   agctgaattt tggtgttaga gcaattcagg agggacagtt tgccaccatt ttatgatact 3120
   ttactgtaga aaagttttca ggatttagac caggaaagag acatcctaac catatgggtt 3180
   gattttattt tatggaccct gtgaagtctg ggactgatca ggtttctctt ttgttggcta 3240
   ctagaaagct tggagtcaaa tgtgtggtca atgcatagca cttgtaatgg gactctacgg 3300
   tatgtatgca ctttgtatta gctttctgcc aggctccatt tcgtgttcct atctttattg 3360
   tttttgtttt ttccttttac tttcttatct actttgaatt tatgctatca tgttgtattt 3420
   tgtgtattct tgtaagccac ctgacatcca tcttggaaca tggtggggaa taaacacact 3480
   aataaataaa tacattaata aaaaaaaaaa aaaaaaggcc acatgtgctc gagctgcagg 3540
   tcgcggccgc tag 3553
<210> 160
   <211> 1971
   <212> DNA
   <213> Homo sapiens
<220>
   <221> misc_feature
   <222> (1)...(1971)
   <223> n = a, t, c or g
<220>
   <223> nbla-04261
<400> 160
   gaattcctcg agcactgttg gcctactgga tctgtcacta acgtcactgc gaccgacgct 60
   gatgacccag tttatggaaa cagtgcaaag ttggtttata gtatattgga agggcagcct 120
   tatttttcca ttgagcctga aacagctatt ataaaaactg cccttcccaa catggacaga 180
   gaagccaagg aggagtacct ggttgttatc caagccaaag atatgggtgg acactctggt 240
   ggcctgtctg ggaccacgac acttacagtg actcttactg atgttaatga caatcctcca 300
   aaatttgcac agagcctgta tcacttctca gtaccggaag atgtggttct tggcactgca 360
   ataggaaggg tgaaggccaa tgatcaggat attggtgaaa atgcacagtc atcatatgat 420
   atcatcgatg gagatggaac agcacttttt gaaatcactt ctgatgccca ggcccaggat 480
   ggcattataa ggctaagaaa acctctggac tttgagacca aaaaatccta tacgctaaag 540
   gtagaggcag ccaatgtcca tattgaccca cgcttcagtg gcagggggcc ctttaaagac 600
   acggcgacag tcaaaatcgt ggttgaagat gctgatgagc ctccggtctt ctcttcaccg 660
   acttacctac ttgaagttca tgaaaatgct gctctaaact ccgtgattgg gcaagtgact 720
   gctcgtgacc ctgatatcac ttccagtcct ataaggtttt ccatcgaccg gcacactgac 780
   ctggagaggc agttcaacat taatgcagac gatgggaaga taacgctggc aacaccactt 840
   gacagagaat taagtgtatg gcacaacata acaatcattg ctactgaaat taggaaccac 900
   agtcagatat cacgagtacc tgttgctatt aaagtgctgg atgtcaatga caacgcccct 960
   gaattcgcat ccgaatatga ggcattttta tgtgaaaatg gaaaacccgg ccaagtaaat 1020
   atctccatgt tgttaatgct gaatatgttt gtatacaact gtctcatatt tgactaacct 1080
   gcattatatt gtgcatttgc atcatttatg atctgcaaag tctcaggcaa gaaacacatc 1140
   caaatgggaa cagagaggga attttttttc tccatcagat gttatttctc ttcctactta 1200
   tgaccaaatt ggctcctaaa gtggaacaac ctgctgtgcc acaaacccag agccaaatca 1260
   ctgtcttaaa atatcttggg atatggatga tagcaggaga tgtcttagag tgacgggggc 1320
   tgaattttta ctttcaaatg acaagaagca aggtaaaacc tcgtggagag gagacatttg 1380
   tttcacagca aacatatgaa taccaaataa tagtgaaacc caagaaggcc agtggtttat 1440
   agaaagttgt aggaagtgaa tattgaaatc tgtaaatatg taaatatatt tagataaaca 1500
   taagatgaac aacacgtttt acatgtgtgt attagtagaa cattttttgt gcctcatatt 1560
   gcatagatgg gtcaatgaaa acagaaaaaa aagaaacata aagaaagtga gagagaggga 1620
   gaaggaaagg gagagggaga gggaatggga atgggagtgg gaggaaggga gggagggaga 1680
   gagaaaaaga gggaggaaga aagggaggaa aatagttaaa caagcaagat gaacagaaac 1740
   aaatatctgc tatactctga atgtcaaaat ggaaatagcc tgtgtaaagt tctgcaaaac 1800
   cataaatgta atagatttat tttaacaagg taccaaagta ctgtatgttt aagaaattta 1860
   tcatttttca acttcctaat ttatttctgg atggtgacat tttaatttaa ataaacagca 1920
   gctgacagta aaaaaaaaaa aaaaaaaagg ccacatgtgc tcgagctgca g     1971
<210> 161
   <211> 785
   <212> DNA
   <213> Homo sapiens
<220>
   <221> misc_feature
   <222> (1)...(785)
   <223> n = a, t, c or g
<220>
   <223> nbla-04264-f
<400> 161
   gnnnnnnttn nnnnnnttnn nnttggattc ctcgagcact gttggcctac tgggatagaa 60
   atttatgctc tctccatttg tctgatgttc aatgaaagac acaaacaact aaacaggtag 120
   ttacaaatac tgtgtaataa gttgttgtgg aaagtacatt gtgctatgag aactcaggaa 180
   taagacatag tccagatttg gaggatcagg aaagacctcc atgggaagag atgtataagc 240
   taagactaga aatgggacct ggaaataaaa cttgggctca ttacggtagc aacaagacct 300
   ggagacttag atgtaagata gaagaagaaa ggctccttga gaagcttaag gagacttaga 360
   tgtaagatag agggaaaaga aagcctcctg gagaagctta aggaggggca tgggatgcat 420
   aatgggcaag gtggggctgt ccatgtatta actaagagga gagctggaaa agacgatgaa 480
   gggccttgac atctaaatta cacacaaaca tgtaaaatta catgcaatgn attttctttg 540
   caaagaagac agagtgatgt ctaatatatt tcagatttct gncataaaag cattttaaaa 600
   ggtcataaaa cttaatattt aaaatggtan ttttttttag aaaaatacct tgngtgactt 660
   aagnatttcc tgaataaatg agctttnctg ggaatcctca tantatcaag cctttggtct 720
   taanaaangg accaaggtnn tgtnggaacn nggaaaaaaa tggnttnaan aatttaataa 780
   tcntt                                                   785
<210> 162
   <211> 784
   <212> DNA
   <213> Homo sapiens
<220>
   <221> misc_feature
   <222> (1)...(784)
   <223> n = a, t, c or g
<220>
   <223> nbla-04267-r1
<400> 162
   nngnagnnnn nnttntgtgg cctttttttt tttnnttttt aagtaatggg taaagtttat 60
   ttcattttta ataacaatta gaggacaaaa tgtttaaaat ttgcagttta aaacatgcac 120
   attcacaaaa ggccaatgac acataacact gcatagaaat aatattactc aattttaata 180
   actataaaac acagtgcatc aaacatcaag ataaacaaac ctgagaaaac tactataacc 240
   acagattcaa tactctccac tcatgcagct tcacaatttc tacagcagtt tcaggaggaa 300
   tggtttttca gggagctgaa aatactactt tatctttaac gcaaaactgc agttttctgt 360
   agtagctgcc ttccaaggct gccctgtttt tcttaaccta ataaacttga aaatgtaaaa 420
   aatgacgatt aaagtagtta aacaacagat agtatttact gcatttatgg cttccattta 480
   gaaccatgaa acataaaaat attattttta actattctgc tcacatcttt gcaagaacag 540
   atttacctgt ggaaaggtgc tggtaattca aagtaagcaa atatgaaatc taagtttcta 600
   cttaagggag attattgctg agaagtttgg gagccttant aagaaaagtc ttaaangcag 660
   cttaacngga agatcaantn aattggtcaa aattctttgg aaataatcta cttattaaaa 720
   aagtgggccg anggnccant aattggctct ggaattttaa attcaggtag gccgggaaaa 780
   actt                                                 784
<210> 163
   <211> 793
   <212> DNA
   <213> Homo sapiens
<220>
   <221> misc_feature
   <222> (1)...(793)
   <223> n = a, t, c or g
<220>
   <223> nbla-04271-f
<400> 163
   gnnggnngnn nttgnnnccn nnnntttgan tcctcnncac tgttggccta ctgggttcat 60
   gatgtccaag aanattgaga ggagctactg aattgtgttt ccctaagttc atttacactt 120
   tagcagcaaa tcttgatgat cagagcctaa ggatgcctaa gtttacatgg agtggtaatg 180
   gttaagtaaa ttgatttggg taatttttca acctcatcta caccacacta agaccaaagt 240
   tctctaaaaa tttctgaaat aatgattttt atatagnttc attaaaagtt ttaaaagtat 300
   ctatcagcat tttctgtaaa agtgtcaggc tgaaaagata ccactaaaca gtcagctcaa 360
   aaaagatagt agataaaata tgatgaattg ccacatctgc atttcctatg tttgcaagcc 420
   accagtaaga ctgcatgatt tccaagttgg aggacagagt ctaggaatgt gaaggatgaa 480
   gggtttcttg tgcaccgttc tcttgagcac gtacgccttc agaaggtaga taggctaacc 540
   tgcatcactg gataaaaact ggatttctct tactttttca tgtgtatgtg gctcatgtag 600
   attttggtat ttttcttgga aaatatgttc ccgctgncta ctgaagtaac tgngtaataa 660
   tttncaattt tctgntgggc attacctcat ggacaccatg ccacccttta aacttcattt 720
   acccaaaacc atactcaant attcccccat aanccttttc acctatggtt cnttatcaaa 780
   atcacctttc tta                                                     793
<210> 164
   <211> 795
   <212> DNA
   <213> Homo sapiens
<220>
   <221> misc_feature
   <222> (1)...(795)
   <223> n = a, t, c or g
<220>
   <223> nbla-04271-r1
<400> 164
   gaggggtnnn nttggtggcc tttttttttt tnntttgcca gtttttatgt tgaagaagaa 60
   aaaatatgaa taaacaaata agtaacctat ttttggtatc aagtaaatgt gcagcctaga 120
   gaagggggat aattctagaa attggaaaca aagttggccc tttcaagtta aaccactaga 180
   aatatacaag ggctgacatt tctgcagata ctctaagaga cggtgagttc caagggaaaa 240
   ggggacctcc caaatcaaat cagggcatta caaaatctaa tcaaggtgac aaggatatat 300
   ccccagcata agactgtaac taaagtggaa gggggcagga tcattcactc attcattctt 360
   gcatatattc atttcaccat ttattattaa tagcagcaat ctgctttgtg taaagtgcta 420
   cacagagata aacgagaaat accttgcatt tagacagcaa cttaaaattt caaaagcagn 480
   tttatcattt agagtccgtg gcagggaaca gatggcaaac ctgactttgg taatttaagg 540
   agcctttaat aaagggactg gttattaang ngtgggtaga gtacagggag accacanggg 600
   ccagtactct ggggncttta ctacctctan ggcctaagga gccncatgaa ggangtnatt 660
   gnttcaacag aacctggaga tatgaaaact gtnnaaangg nccacttgca caactgggnc 720
   cttaatcaan ggactctntn attctaaggn gacatgttgg aangggatgg ggaatccttt 780
   tnaccnccnc tntnt                                                  795
<210> 165
   <211> 3028
   <212> DNA
   <213> Homo sapiens
<220>
   <221> misc_feature
   <222> (1)...(3028)
   <223> n = a, t, c or g
<220>
   <223> nbla-04300
<400> 165
   cactgttggc ctactgggat gcatgggaaa agcactgctg tgctagaaat ggactattca 60
   attgttgatt ttctaacgct gtgtgaaaca ccatacccaa atgattttca agaaccatat 120
   gctgtggttg ttcttctaga aaaggattta gtacttatag accttgcaca aaatggatat 180
   cctatatttg aaaatcccta ccctttgagt atacatgagt cccctgttac atgttgcgaa 240
   tattttgcgg attgtcctgt ggaccttatt cctgcacttt attctgttgg agctagacag 300
   aaacgtcaag gttacagcaa aaaggaatgg cccatcaacg gaggtaattg gggcttgggt 360
   gctcaaagtt acccagaaat aattattaca gggcatgctg atgggtcagt taagttctgg 420
   gatgcttctg caataactct acaagtatta tataagctaa agacatctaa agtatttgaa 480
   aagtcaagaa ataaagatga caggccaaac acagacattg tagatgaaga tccatatgcc 540
   attcagatca tctcctggtg tccagaaagt agaatgctgt gcatcgctgg agtttcagct 600
   catgtcatta tttatagatt cagcaagcag gaagtaatca cagaagtcat tccgatgctt 660
   gaagttcgat tattatatga gataaatgat gtggaaactc cggagggtga gcagccacca 720
   cctttgccaa cacccgtggg agggtccaac cctcagccca tccctcctca gtctcatcca 780
   tctaccagta gcagttcatc tgatgggctt cgtgataatg taccttgttt aaaagttaaa 840
   aactcaccac ttaaacagtc tccaggttat caaacagaac tagttattca gttggtttgg 900
   gtgggtggag aaccaccaca acaaataacc agcctggcag tcaattcttc ctatggactg 960
   gtggtttttg gtaattgcaa tggcattgct atggttgact acctccagaa agcagtgctg 1020
   ctcaacctgg gcactattga attatatggc tctaatgatc cttatcggag agaaccccga 1080
   tctcctcgta aatctcgaca gccttcagga gccggtctgt gtgatattag tgaagggact 1140
   gttgttccag aggatcgctg caaatctcca acctctgcaa agatgtcaag gaagttaagc 1200
   ttacctactg acctaaagcc tgatttagat gtaaaggata actcctttag ccgatcacgg 1260
   agttcaagtg taacaagcat tgacaaagaa tcccgagaag cgatctccgc tcttcatttc 1320
   tgtgaaacgt ttactcgaaa gacggactcg tccccttccc cttgtctatg ggttggaaca 1380
   acgctaggaa cagtgcttgt cattgcactg aaccttcccc cagggggaga gcaaagactt 1440
   cttcagccag taattgtgtc tccaagtggt actatattga ggttaaaagg tgcaatcttg 1500
   agaatggcat ttctggatac cacaggctgc ttaataccac ctgcgtatga accctggaga 1560
   gagcacaatg ttcctgaaga aaaagacgaa aaggagaaat tgaaaaaacg gcggcctgtc 1620
   tcagtatccc cctcctcttc tcaggaaatt agtgaaaacc agtatgcagt gatatgttct 1680
   gaaaagcaag caaaagtaat ctcactgcca acccagaact gtgcttataa gcaaaatatt 1740
   acagagacct cgtttgtgct tcgtggagat attgtagcat tgagtaacag tatctgcctt 1800
   gcctgtttct gtgccaatgg acatataatg acttttagtt tgccaagttt aagacctctg 1860
   ttggatgtgt attacttgcc ccttaccaat atgcggatag ccagaacgtt ctgctttacc 1920
   aacaatggac aagcattata ccttgtttca cctacagaaa tccagagact tacttatagt 1980
   caagagacct gtgaaaatct tcaggaaatg ttgggtgaac tcttcactcc tgtagaaaca 2040
   cctgaagcac caaacagggg attctttaaa ggcttatttg gaggtggtgc acaatctctt 2100
   gacagagaag aactatttgg agaatcgtcc tcaggaaagg cttcaaggag ccttgcacag 2160
   catattcctg gccctggtgg cattgaaggc gtaaaagggg cagcatctgg agttgttggt 2220
   gaattagcac gagccaggct ggcactagat gaaagagggc agaaacttgg cgatctggaa 2280
   gaaagaactg cggccatgtt atcaagtgca gagtcatttt ctaaacatgc tcatgagatt 2340
   atgttgaaat acaaagataa gaagtggtac cagttctgac aaccagaatc caataagtcc 2400
   aacttcagcc agaaggaaaa aagttttcca tttttattac attctttagg aaagttaacg 2460
   ttaaagggat gttcgtcact gaatactgtt ctttcctagc acagtcatgc actgttttac 2520
   ctcagtcatg tggctttaac tgaggagtgt tcacacgcac tcgaaatgga gtatatggtg 2580
   tgtgccagtt atgagttgac catttgggaa ttaaacaggt cacacgtgac agatgaagaa 2640
   accaaggggg ctgctgagga gacctggtgc agggactaat cctggatcat tcctgtatta 2700
   aactttcata tgccaaaagg gtttgtgccg ttttatctgc catcagtgtt tgacctgttt 2760
   agggcagagg caataagtca gaagtcttga agttgaaata gttatatgtg tgtcattgga 2820
   ctggattata aacagctgtc ttggactttc cctctcttaa cactgactgg tcatcagtat 2880
   cattagtgaa aaagaaacaa attgtttgtt atcatctctt tagacagata agctgaatgg 2940
   tgggctttaa ataataaaaa catacacata gttgaaaaaa aaaaaaaaaa aggccacatg 3000
   tgctcgagct gcaggtcgcg gccgctag                                  3028
<210> 166
   <211> 784
   <212> DNA
   <213> Homo sapiens
<220>
   <221> misc_feature
   <222> (1)...(789)
   <223> n = a, t, c or g
<220>
   <223> nbla-10010-f
<400> 166
   gggnnnggtt gngnnnnnnn tttggaattc ctttagcact gttngcctac tgtnaaaaaa 60
   anattggaaa atatttaaag gagagtaaaa ggaataaaca cagaattata atagattgat 120
   aagatatacc ctccaaagta caagatttct ataattttaa tccatgggta agagaaatgt 180
   ctaactaaat tttgtgtgtg tgtgtgtgtg tgtgtgtgtg tgtgtgtgtg tgtacatgcg 240
   cgaatgtttc caatcagatc ctcgggaata aacaaggaga agaattaaat atcagtactg 300
   gacccttgat ctttcctttc accttaggct gcctttagat tcccttttgg agctatgaat 360
   atacccctta gttacaggtg tacattgcca atgtatgtat cttagcctgg gatagctgaa 420
   gtgatttgtc tagttatttg aattttagca tttgagagat tttttaagtg aacacctagc 480
   ctttaacacc ataagaaaag ccttgtgaaa tgatagaagt atttttaatt tggacagttg 540
   aggggaagat atcctaacag caattttttt ttaagtgttt ctgcttttat tcttctagaa 600
   atttagaaac cgtttcaagt gaaatccact ttttttgtgg ggcagaagac tatatataga 660
   ttaatttggg catttttttc taacgtggag tcttcttatn cagaaaaatg gatgtcttcc 720
   caatttttaa aaatanttca acttttattt taaatgcagg ggggtcatgt gcanatttct 780
   taca                                                             784
<210> 167
   <211> 781
   <212> DNA
   <213> Homo sapiens
<220>
   <221> misc_feature
   <222> (1)...(781)
   <223> n = a, t, c or g
<220>
   <223> nbla-10010-r1
<400> 167
   gnagngtnnn ttggtggcct tttttaannc cctttttttt tctgtgacgg agtctttctc 60
   tgtcacccag gctggagtgc agtggtgcga tcttggctca ctgcacctct gcctcctggg 120
   ttcaagcgat tctcttgcct cagcctcctg agtagctggt gcatgcgcca ccgcacgtgg 180
   ctactttttt ttgtattttt agtagagatg gggttttgct atgttagcca ggctggtctc 240
   aaactcctga cctcaagtga tccaccgaac ccagcctcag atgatattct taacagaaat 300
   attaaaggaa ggtgtaagag aacatgtggt gacttgagtt gacaaaagaa catactggtg 360
   aaaacgtggc accagagttc tgaagagatt ggcactccga gctgtgcatt aggaaatcat 420
   gttgccatag aggtggtaac tgaagtcttg ggatttttaa gaacacgctt actgcctata 480
   tagaaactga aaaggtaggt ggtggataaa tgtgtgtctg gagttgttta agaatgagtt 540
   aacaaaaaga caacatgctt ctgttcaaga gtcaatattt cacaagtttt cagctcatgg 600
   aaaagactga aataaataga aaaaggaatc tagaaagaag tactccttat tctcttgaat 660
   actctttgaa gatgcagaaa atattctatc taagaacact cttaccctta atggatcctt 720
   aaanctttaa tctatgctga ttagaaacac attacactgg aatcattgat ttgccttttt 780
   t                                                                781
<210> 168
   <211> 1475
   <212> DNA
   <213> Homo sapiens
<220>
   <221> misc_feature
   <222> (1)...(1475)
   <223> n = a, t, c or g
<220>
   <223> nbla-10070
<400> 168
   ttctaggcct gtacggaagt gttacttctg ctctaaaagc tgcggaattc ctcgagcact 60
   gttggcctac tggtttcgct aaatgctata gctttcttca aataaaaatt agccagaaaa 120
   ataccttaga aaagattttt tttttcattg tggattccag gagactttta agacatggaa 180
   ttctcggtga ttatattcaa atggcagagc cacatttgct aacagtattt tctaatttta 240
   tctttcttat aagaaaaaaa gggtaaaaat cggatctgaa atactgacag ggttccccaa 300
   agttagatat taacacagct tttcatttct cttatgagaa ctttactgta aatatacctt 360
   tatcatagaa atgagatatc caatacacat tacaaaatgt catatttaaa gatgacaaat 420
   ctattcttaa cccagattct tcatccacag aagtttaatg actaaaataa cacaacagaa 480
   tgaacaaatg ttttattggc atgttcattg ttgtaaacag catctggcat gaaaaagttt 540
   taccaaaacc ttttttattg atttttataa attagatggt atgtcaaaat ttatgtagaa 600
   ttgtgttcat tgagtacttt tatttagcaa agcaaaaact tgctgatact aataaactct 660
   gcaatgaaat catgttgact gcatcagtct gtaccatgac ctgacctcat tagcaagttt 720
   attcagaaca atgttaagac gtttcccaaa agttaaacta catgtaacta caaattgact 780
   ctaaaaatta aaactggact tacaagcatt ctagaagatt caaacttgaa gtaaatcttc 840
   aaaactagat gtacaaagag aaaaaaattg aagccgctga acagagaaac ctcattaaca 900
   agggcaacga caatcctatg ggacctagca ctgaggaacc tcactgagga ttacatttac 960
   atagattgct atacttctca atgtacacaa atgtacagaa tgaatgatac gtgaaaagaa 1020
   taatcatggc agatagttga actgcaatta tgataactca acaggatcaa ttttatttac 1080
   atgctgaata aatgaatgaa acatattctc tacatccata acttaagtac aatcagttat 1140
   aattacaaca ctgcagcaat ctgagctgcc acctgctggg atcggtctaa gggaaacgtt 1200
   ctgatgttcc agaataatgt tcttaatact ctgcaaaaaa tgaaacaaaa taaaaacaca 1260
   acttcacaat tccttactgg gttacggctg gttccaagaa tttcttttct tcagctcctg 1320
   tttttgctgc tttccaagag tcaacacacg ttggatttac ttctgaattg tcaatgaggt 1380
   aatgtgtgca tcagtgaaat aaacagaaaa ttcattcaaa aaaaaaaaaa aaaaaaggcc 1440
   acatgtgctc gagctgcagg tcgcggccgc tagac                           1475
<210> 169
   <211> 1557
   <212> DNA
   <213> Homo sapiens
<220>
   <221> misc_feature
   <222> (1)...(1557)
   <223> n = a, t, c or g
<220>
   <223> nbla-10071
<400> 169
   gcctgtacgg aagtgttact tctgctctaa aagctgcgga attcctcgag cactgttggc 60
   ctactggctt tcatgcagcg aggacaatag aattttactc tgggataccc gctgtcccaa 120
   gccagcatca cagattggct gcagtgcgcc tggctacctt cctacctcgc tggcttggca 180
   tcctcagcaa agtgaagtct ttgtctttgg tgatgagaat gggacagtct cccttgtgga 240
   caccaagagt acaagctgtg tcctgagctc agctgtacac tcccagtgtg tcactgggct 300
   ggtgttctcc ccacacagtg ttcccttcct ggcctctctc agtgaagact gctcacttgc 360
   tgtgctggac tcaagccttt ctgagttgtt tagaagccaa gcccacagag actttgtgag 420
   agatgcgact tggtccccgc tcaatcactc cctgcttacc acagtgggct gggaccatca 480
   ggtcgtccac cacgttgtgc ccacagaacc tctcccagcc cctggacctg caagtgttac 540
   tgagtagatt ggatttaaga caaaaagcaa gtcccccatg agtgtccact tctttgccct 600
   gccctctcag cttgtgagac aacacaggag ccttctatag tatgttgata tgctagatct 660
   gtgccgttaa taggcatcgt ctctcagcct gagggaggct ggattctggg ttcctgtagt 720
   cacagggagg aaaagctttc ttaaaaatgg acatgtatgt gcgtgtgagt gtgtgtgtag 780
   atttatagtt tttggtagtg gcaggaataa aaaaaatcca tcctacatct tccctaagca 840
   ctgcctctct ctcacccccc aaaacaagtt gacgaaaggg ttttatgtag ctgtctatga 900
   ggaattggcc gtgtctgggt gggttatggg atgtgggcat ccctgggttc ttggaagcag 960
   ctcttatgct actcatagag atgggattga ctttattttt ttatagtgct taattcacca 1020
   ttatgagaaa tgcttccagt cacaaaaatg cagcccagct cactctgagg aagaagcagg 1080
   acttggtacg gttttacaca actccttacc attaaactga atcagaaatc cattttctgg 1140
   ctgaataaaa agtttggctt gcctgtgtaa tgcccactcc cttccccctg gctccctagt 1200
   gatgggacat atatgagaga gaagtgtttt tctatcatag acaccatagg ggaaagtttg 1260
   gggatgaagg agagcttaaa ggtgtttcaa ttaagttaga aaactgacac aggctgttga 1320
   gaattctttg ccacttttcc caccccaaaa cagcatgggg cctgacatct tctgccctgg 1380
   tcccctttct cttgatgtgg aaagtctgaa tgcagtattt atagacttct aaggttttaa 1440
   aatccagtat caagaagaaa atcagaaata ccggttggtg aaataaagag tttaggcatt 1500
   gttaaaaaaa aaaaaaaaaa aaaaaaaaaa aaaaggccac atgtgctcga gctgcag    1557
<210> 170
   <211> 1783
   <212> DNA
   <213> Homo sapiens
<220>
   <221> misc_feature
   <222> (1)...(1793)
   <223> n = a, t, c or g
<220>
   <223> nbla-10111
<400> 170
   gcctgtacgg aagtgttact tctgctctaa aagctgcgga attcctcgag cactgttggc 60
   ctactggtta ctatggatca gtcggcactc gggaacagca gcaaggaaaa aaagcaaatt 120
   tcattcacat gttctgtgtt catacctctt ctctacctaa ttgttcattt aaatttcagc 180
   cttattcctt gataagggat tttaccacat gaagtcatcc agtgacccta gctcttattg 240
   tgaagttagt ggagtatact tagaaatgtt acaactttaa aatgttacaa aacattcatt 300
   aaagctcata tttaaagtag agcatctagt ttgagaaata gaaatcaatt attaaagatg 360
   tcttttttct acccatttaa ctagttaaaa ccatgacatg taaatgtaga agtagaataa 420
   tcatagaatt ccctaaaata tttctgttta ctaacatata ttgaccaagt acatcaagca 480
   ggagagatct tccttcattc tgttatagtc cacatcattc taattttgct cagttgttat 540
   taagagcata ttcctaaacc atacactttt gtttcaataa agttttattt tgttgagatg 600
   aataaaataa caaagttata agctgcataa gacaaaagtt caattgttca aaaaaaattt 660
   actgggatag ctttctatta caggtattgt tagattatat tgtgctgata agattacttt 720
   ctaaaaaatt tgtacttttc tgtaaattaa aagaatatgg agtcataaaa tggcaagtgt 780
   tttaggatta gcctaaaatt ggacattgtc attgatttca aagaaggtat gaactagcag 840
   tcttacagcc taattcttct ttggactggt ccttggcagc agttcctttt cagactcgat 900
   aaacagaatt cagatgatgt aagtcaaaac aaaactttac aaagccaagc gtattatctt 960
   ttgcattaac ctattttttt ccatcataca tgctactagt atgtgcatta gcatgatatt 1020
   ctcatataca ttgcattaaa aattaaaagg tggcagctca gggtgagctc ttctgttgct 1080
   catttgttcc taaattttta agggcttttt ctcagtcaat agtttgtaca aactggttag 1140
   tttaacttca ttacccattt cattaaagtt gatgggtcgt gtgatgagat gcatttaagg 1200
   ccgatagtga tagatgtttt ttttatttct tgaacacagg ctttgtctga atgatgttct 1260
   tttatctctt gaacacaagc tttgaatgat aactacaggt tttaagtgct gttacattaa 1320
   taccataatg tgatgtgtta gaaacaaagg gatatttcaa aggtagatat ttgaaaattc 1380
   tctagtctca atatgtatgt gtattgaata tactctaaaa ataaatgtgc aatttgctag 1440
   taggacaatg cagtgactga ctagcattag gtatgtttct tttatatcct ggctatgtcc 1500
   cactttcttc taagtgcaat cctttcatgt tcacttgctg ttttacccca tctactctaa 1560
   cttcatttgg aaggcttgtc tagagtatag catgtatttt tacctttgca gtgaattgca 1620
   tgtgctaatt gtaaccacag ctatttttat gttgacataa ctccaaatgt tatattaaat 1680
   gttctattat atattagctc taatccctta agtaaatttt aagaaataaa tacttgttca 1740
   aaaaaaaaaa aaaaaaaaaa ggccacatgt gctcgagctg cag            1783
<210> 171
   <211> 2121
   <212> DNA
   <213> Homo sapiens
<220>
   <221> misc_feature
   <222> (1)...(2121)
   <223> n = a, t, c or g
<220>
   <223> nbla-10120
<400> 171
   ttctaggcct gtacggaagt gttacttctg ctctaaaagc tgcggaattc ctcgagcact 60
   gttggcctac tgggttactc agcgtagatg tgtgttcaca caaattgctc tgcattcagt 120
   gttcattgtg aattgggagt gtgagtcttt ctgtagggta gaaagaagcc tcctacccag 180
   caaaccagta gacccaaaag ttgaaaaaaa ctgatgacag acaacaagca tgaagatggc 240
   atatttgatg tcactttggt tctttttccc agaaggctta tacagtgact cagtcgggaa 300
   gctttccagc ttccagccct tgaatgtgaa gtgtcattgg catgtctggc agtagtctct 360
   cattcactcc tcaataaaca acattgaata caaaagaggc ttgtgtaaaa actcagtact 420
   gtctggcttg gattcatttc atgtttttaa tataagaatg atctaatatt tttttaaagt 480
   aatagctatc agtaatagct gagtgttttt tcccctaata ttttccttgt gcaattcaga 540
   cttaagcatc gagtttttac catcttccac tttaagctaa gttatgatac ctattccatt 600
   cacaattggt gttcttttta aggtttgcaa atttcagcca attttgtagc taagattgtt 660
   ctgatcagct caaaaagatt tggcttagtg ttttcattgc aaattataat tgctgtagag 720
   ccacacacaa cttttgaact tttaattata agtgttatgg ctaaagttat ttactgaaaa 780
   tttcagtaaa atgtgtgaat gtttctttat gtattaacct catagcagta aatgacttgc 840
   tgttgtttaa tttttctaag gcatcttaat agacttctgt tgaaaacttc agtgttaaca 900
   tttttatagt ttgtactaaa tttaaccgtg atataaaaat gaattttatg catagatcag 960
   aattttaaat taaaggtttt ttctttaaat gatttgtatt actttattaa aactaaatct 1020
   gaaatggaat agaaaataga atggattaca tacagatggt ttccttgtta gcagatgccc 1080
   ttcaaatata ttttacgttt gcagcacaac accattgccc ggaggcttcc gaatctagca 1140
   aagcagcata ttaaatgact ccacctgcgc agtccttgtg ttcagtggat gaatacctgt 1200
   gggctcttgt aatgtgtggt aatatttgga gctttatagc ctgtattcct tgggagaaaa 1260
   aaatgggaat tggagtaaaa agtgtgtcaa gacaaccact ttctgctttc ttatgtagca 1320
   tctgcaaagc cgattcatgt actgatgcca atcagctaga gcaatgtagg ctttttttaa 1380
   tttaaattat tactacactt tattactaca cttgcagaaa agaaacatgt taaaatcatg 1440
   gcacacctgc agaatttcag atgacagtgt ggtcagaaga tgttttttgg gagaacctag 1500
   tgtttgctgg gcactatgct aagcactctg ggagcaagaa cctcctacct tccagtttaa 1560
   tgataaaagc agaaaagcaa agaggtaatt ggagttgaat caagttaata gggaaggttg 1620
   gtagtaaatg ctgttagtgt gacatgattc tgaaacccag agctcagaac taactgtgag 1680
   atgtaccatg aactaacaat tctttgaaag gacctgcaga agcattttta atacctcatt 1740
   ctgtctatgc aagatgaaaa tccatggagt tttttcttgg cttttgtacc aaatttaggg 1800
   gtcttgcatg tcagcaagtg atatatattg tttataaatg caagctattt ctggagggga 1860
   aaaatgtgtg tggctcttac gttttctggg aattttgtaa caagttacac gcacaagtgt 1920
   tagaaacttt ggcataactc aatttggagt attttttaaa tgctgtatct ttaaagaaaa 1980
   gtaaaattgt cttaaatcta attgacactt ctgtaatgag aggcaacctt ataactctgg 2040
   agttgtagtt tgacagaacc atcagtaaag acataagcaa ataaaaaaaa aaaaaaaagg 2100
   ccacatgtgc tcgagctgca g 2121

<210> 172
   <211> 801
   <212> DNA
   <213> Homo sapiens
<220>
   <221> misc_feature
   <222> (1)...(801)
   <223> n = a, t, c or g
<220>
   <223> nbla-10130-f
<400> 172
   nnngnngnnn nagnnngtgg gggggggttn nnatntgant cctttgcact gttggcctac 60
   tggcgtcttc agcaacctgg acatggaggc cgcactgcag ccactgggcc aggccaagag 120
   cttagaggac cttcgtgccc ccaaagacct gagggagcag ccagggacct ttgactatca 180
   gaggctggat ctgggcggga gtgagaggag ccgcggggtg acagtggcct tgaagcttac 240
   ccacccgtac aacaagctct ggagcctggg ccaggacgac atggccatcc ccagcaagcc 300
   cccagctgcc tcccctgaga agccctcggc cctgctcggg aactccctgg ccctgcctcg 360
   aaggccccag aaccgggaca gcatcctgaa ccccagtgac aaggaggagg tgcccacccc 420
   tactctgggc agcatcacca tcccccggcc ccaaggcagg aagaccccag agctgggcat 480
   cgtgcctnca ccgcccattc cccgccggcc aagctccagg ctgccggcgc cgacttggtg 540
   acgtctcana gcggctgcag acggatcggg acaggcgaac ttgcctgagt ncaaggcttc 600
   tgcctggtgt tgtccccaag gcccactgaa ctgttcaagc cgntcaaccc ttgggcccgg 660
   ggcttgangc acgaacagtg acgccttgtt ggcctctgga ccggtnagac aanctggtca 720
   ggcagaacct tccgtaaggc ccgcaacccg atgtngcanc ccattaaccc ccaattaant 780
   tccccttgnn gggaaccacc c                                          801
<210> 173
   <211> 800
   <212> DNA
   <213> Homo sapiens
<220>
   <221> misc_feature
   <222> (1)...(800)
   <223> n = a, t, c or g
<220>
   <223> nbla-10130-r1
<400> 173
   gagggngntt nnnttgtggc tttnnnnntt gntttctttt tttttttctt ccccaagatg 60
   attttattga atgcacacaa agttcatcct tgggtttgca aaaagtccca caagtgaaga 120
   ggcagcagtg ctcatgtgaa catggagcgc tcaccccagc ccctcagcac agccaggggg 180
   ccttggggta cacaccctcc ttccctgggg ccgccagcac ctcctctgcc ctatcccgga 240
   tggggcctgg gggtctgccc agggtgcgaa actggaatct atgctgaaac acctaagtgc 300
   ccaggaggtg cccccatggc ccaggagtga cacggntccc ccagcagcca gagcccattc 360
   ctgagccaga caggtcacgg ttgacccagg aagagccatg tgccaggatg gccgccaagc 420
   ctactgagca tgtgcagcag tggcagcctn tagacataga gggggctccc tgggtgacat 480
   nttcagagac ccccttgtcc ccagacaccc ctgggtagac tgngtctgac ctttacaaat 540
   nggaaatgag aacttgggtc gaaaagntta caatttnctg ggngncttaa aagggtggtt 600
   tcttaaaagg gcnggccata ctttaactgg gttatgggaa tgaatcnntt naagaattat 660
   caaantaatt agggaaaaag ggaaaagaaa gatnttaacc nttaatctaa ctttggngac 720
   cttggcctaa gaaattctgg gctggcctgg aactttggca agttggggag gacttgggtt 780
   ttaaccttct tctngnttcc                                            800
<210> 174
   <211> 1698
   <212> DNA
   <213> Homo sapiens
<220>
   <221> misc_feature
   <222> (1)...(1698)
   <223> n = a, t, c or g
<220>
   <223> nbla-10143
<400> 174
   gcctgtacgg aagtgttact tctgctctaa aagctgcgga attcctcgag cactgttggc 60
   ctactggagt cgtttatgct ccactttcaa agaagcagga gatcttttat acagccattg 120
   tgaaccgtac aattgcaaac atgtttggat ccagtgagaa agaaacaatt gagttaagtc 180
   ctactggtcg accaaaacga cgaactagaa aatcaataaa ttacagcaaa atagatgatt 240
   tccctaatga attggaaaaa ctgatcagtc aaatacagcc agaggtggac cgagaaagag 300
   ctgttgtgga agtgaatatc cctgtagaat ctgaagttaa tctgaagctg cagaatataa 360
   tgatgctact tcgtaaatgt tgtaatcatc catatttgat tgaatatcct atagaccctg 420
   ttacacaaga atttaagatc gatgaagaat tggtaacaaa ttctgggaag ttcttgattt 480
   tggatcgaat gctgccagaa ctaaaaaaaa gaggtcacaa ggtgctgctt ttttcacaaa 540
   tgacaagcat gttggacatt ttgatggatt actgccatct cagagatttc aacttcagca 600
   ggcttgatgg gtccatgtct tactcagaga gagaaaaaaa catgcacagc ttcaacacgg 660
   atccagaggt gtttatcttc ttagtgagta cacgagctgg tggcctgggc attaatctga 720
   ctgcagcaga tacagttatc atttatgata gtgattggaa cccccagtcg gatcctcagg 780
   cccaggatag atgtcataga attggtcaga caaagccagt tgttgtttat cgccttgtta 840
   cagcaaatac tatcgatcag aaaattgtgg aaagagcagc tgctaaaagg aaactggaaa 900
   agttgatcat ccataaaaat catttcaaag gtggtcagtc tggattaaat ctgtctaaga 960
   atttcttaga tcctaaggaa ttaatggaat tattaaaatc tagagattat gaaagggaaa 1020
   taaaaggatc aagagagaag gtcattagtg ataaagatct agagttgttg ttagatcgaa 1080
   gtgatcttat tgatcaaatg aatgcttcag gaccaattaa agagaagatg ggatattcca 1140
   agatattaga aaattctgaa gattccagtc ctgaatgttt gttttaaagt ggagctcaag 1200
   aatagctttt aaaagttctt atttacatct agtgatttcc ctgtattggg tttgaaatac 1260
   tgattgtcca cttcaccttt tttattatat cagttgacat gtaactagta ccatgcgtac 1320
   ttaaatagat ggtaattttc tgagccttac caagaacaaa gaagtatcca tattaagttt 1380
   agattttcag ttaatttttg agactgagta gtattcttgg atacaggctg atgtgtactt 1440
   aaccacttcc agatttatac agtcttcctg tggaagttta gtaaatgtct ttttccctcc 1500
   tttcttctag taatgcagtt catgggcttt aggtacttca gttatgaagt aggcttttca 1560
   tggggagaga ttgggattat gctttctgtt gtttaagaaa ctgtttgatt ttagagtcta 1620
   tttctatgag atagtttacc aaataaatgt tccttataaa aaaaaaaaaa aaaaaaggcc 1680
   acatgtgctc gagctgca                                               1698
<210> 175
   <211> 793
   <212> DNA
   <213> Homo sapiens
<220>
   <221> misc_feature
   <222> (1)...(793)
   <223> n = a, t, c or g
<220>
   <223> nbla-10198-f
<400> 175
   gnnnnngnnn nnnnntnggg ggcnttnnna tttgattcct ttgcactgtt ggcctactgg 60
   tcattttgtt ttggacaccg agcaggagct ggcggccgct gcagacgaaa ggcaggaaag 120
   ggcaggccgg gtgagcagac ggatcggccg actagacagc caaccagcaa caacgaactg 180
   agctcgcata ctaccgctta cgcatctaac caaccgccca tctagctaac ccgagcccct 240
   ccaccgtcaa ctcaggttcg gccggtcccc ggcccgcctg ccggagccgt ggtggcagcc 300
   ccgggaggag cactggcgtc tgtttccttc gattctcggg attcgaagat ggctgcacag 360
   tcagcgccga aagttgtgct aaaaagcacc accaagatgt ctctaaatga gcgctttact 420
   aatatgctga agaacaaaca gccgacgcca gtgaatattc gggcttcgat gcagcaacaa 480
   cagcagctag ccagtgccag aaacagaaga ctggccagca gatggagaat agaccctctg 540
   tccaggcagc attaaaactt aagcagagct taaagcagcg cctgggtaag agtaacatcc 600
   aggcacggtt aggccgaccc ataggggccc tggccagggg agcaatcgga ggacgangcc 660
   taccataatn cagaaangct tgccananga ngactacgtg gggacgtgcc cancagaacc 720
   ctacttaagg gccgggatgt catttcgagg tcaaaanctg ttccgaggtg gacnagccgt 780
   acttcccgaa tgg                                                    793
<210> 176
   <211> 784
   <212> DNA
   <213> Homo sapiens
<220>
   <221> misc_feature
   <222> (1)...(784)
   <223> n = a, t, c or g
<220>
   <223> nbla-10198-r1
<400> 176
   gagnggggnt gnnttggnng ccttnnnttg acnncctnag cgaacagctt gttataatga 60
   accagaaata caagattcca ctgaaactga acagttgaca gaatatggtt gaacttaaac 120
   cttcaaggga aacaagggca aaacaaagct aatgagtgga aaagtccaag attagtttgg 180
   gataaacatg aggatataat tgcattttag catggctatc ttctgacctc ttccagcagt 240
   tcgtctgcca tcattctccc ttctgacaca cctaccaaat caaatggctt ctgatcctct 300
   atattgcagt ataaaccaac cctatagtac ccctctggtc atgatacaaa cccagaagga 360
   aagtctgtaa atctcataca aaggtagaaa gtgcaattca acccaacacg ctggttatgg 420
   tatatgtgct gtcaatgtac atggtagcaa cttgaatcaa accagagctg ctaccacatg 480
   taagcaacct cttatgctgc cctgtagtga tgtcaatgtt ctcagtttac taaattatca 540
   atgtggaaga acgcattttt gagctccaga ccttctaaac attttaaaca ggctatcgaa 600
   gaggatgtaa cagaacccta tttcagaaac ctaaggatca nggaggaang aacactgacc 660
   gggttttctg ggatggttct gtgggggcac agggatgaac aaaagaaatg gtancatgtc 720
   anaacatttg caagtgaagg ggagccgaag tnggaaaagg gngtctannc ccaanggcca 780
   tgct                                                  784
<210> 177
   <211> 1880
   <212> DNA
   <213> Homo sapiens
<220>
   <221> misc_feature
   <222> (1)...(1880)
   <223> n = a, t, c or g
<220>
   <223> nbla-10236
<400> 177
   gtacggaagt gttacttctg ctctaaaagc tgcggaattc ctcgagcact gttggcctac 60
   tggaagatgc acatgttgca aaggggaaga gtgttttcct tgagcaaatg aaaaagtttg 120
   tagaatggct caaaaatgct gaagaagaat ctgaatctga agctgaagaa ggtgactgaa 180
   ttttgaaact acaccctcag taaagcaaac aggagttgta gataaaatgt catgtctcat 240
   gtgtcctggt tcttacatct tcctacctcc ctgtatcaag catgatataa gggctttcat 300
   ggcaaatttt attttaactg tttctatggt tgctggaaat gttgggttta gtttctaaaa 360
   ccatgtttta agtagctaca ggagctatag atttgaatct aatgttgcat tagtcttttc 420
   agttatcttc tacctcctgt attttctact gtaataatgt aatttaaggc cttccacaat 480
   gaacagttca ctttattccc tgggttttct ataaacagtt ttaaggatat gatttggtta 540
   aaaaataatt tgttataaaa attctgtttg caaattaaac tggaaaagta tccagagtct 600
   caaaaggcaa tgatttgtga gataatatgg catgcccgga gccctgctca tcaatgaaaa 660
   acccatatgt aataatcgaa ttcatttaac atgaatcttg agtacgtgga ccattgcttg 720
   catgttaact ttttgttttg ttttgttttg ttttgttttg catttttaac tccagatatc 780
   ctaaagctca attgtttggt ctctggtttt catccttaga gaagccatgg agaacagact 840
   tgaaaagttt aggaaatcat aatgtggcag aggtggtggg aagaagaaag ttgagctttt 900
   tccccttgag aaacttctgc atttagtttc tatctttcca ggcaaaacaa atgggtattc 960
   ttttcataca accattttca aatgaacctt agaaaagtct taacatttaa ggtattttat 1020
   gcacagaata cacttagatt gataggaaag aactcgtaat ggagtttgag taaagaaaat 1080
   gactgatgta ctaaacccag taaaaattgt tgaaaatgtt aaaggtcagc atgttctaat 1140
   tgggaatcta gatatagctt agatttccta ttggcttaga gtatttgcta taacaaatga 1200
   agtgcaatga caattatata ttcctactcg gtcatactgg actggcttcg ttctcttaat 1260
   atactcagta atgactcaag cctctggcta ttaacatacc ctagttgccg ttttttaatt 1320
   gccatgagcc aaatacttct tggtatacaa ttgatccatt tattttaatg gctgcctttt 1380
   cattttcatc ttttcttgct gctacccatc tatgtatgta gtcattgggg ggaaaatgta 1440
   gccacatttt ttatgggaag actttgtgtt aaaagtgaac attttgaagg tttttaactg 1500
   gtgaaactag cctggaataa tgccaccaga gactgagtgg aaatcgcccc ttttgaaggt 1560
   gccattctta tgagccaaaa gtttgtcatt taaaagttca ttttgaggga ataacatgta 1620
   atataatttg aaataaaggt atagtaacct taaaaagaac attataactg attgttgtga 1680
   atggggtgaa tttgttaaaa tgagtaactt tgataaagtt tttcatgcac aggcaaaatg 1740
   tattcactag atttctacgt agtgatctgc ttttactttg taatttgtag ttctcaaaag 1800
   actttttttt aaaaaaataa agtccatact tacacttaaa aaaaaaaaaa aaaaaaaggc 1860
   cacatgtgct cgagctgcag 1880
<210> 178
   <211> 2075
   <212> DNA
   <213> Homo sapiens
<220>
   <221> misc_feature
   <222> (1)...(2075)
   <223> n = a, t, c or g
<220>
   <223> nbla-10241
<400> 178
   gtacggaagt gttacttctg ctctaaaagc tgcggaattc ctcgagcact gttggcctac 60
   tgggatagtg ttaaatgaga tcactggatc ctgctaaaca tgttctagtg ctcttgcctt 120
   ttgctgtatg tttggtgtca ttcatcagca gggaatggct gaaatgtgag gtgtaaattg 180
   acagagtaga gagaacctat acagttttaa caccatagcc acagacatcc aggcatccta 240
   acattttgtc ctagatcttc ctgaacctgg acggcaggat gagaacacta gtgattttat 300
   tggaatgaag ccaagacaaa taaccttttt ttgtgctctt tataatatag ctctcaagaa 360
   tgaacaaata aaaagaatgg ggtcaccata tatatcattg tttagcaagt gacaagtgac 420
   acagtgagca atccttagtc agtggcatga caaggaaata ctcaagacag acactgacct 480
   gtattgtgaa tgaggtgcct tggtgaagca aaggtttggg cccaatagtg agtcaaggag 540
   ccatttcaaa gatgggctac agtcactcat taaaagggat agaacacaac tgtgctgatc 600
   gtagaattaa ctgtgacttg tccataaaat agagggagga tgcaatctgt tttatagaag 660
   gcatatcttg cagggccttg tagatttgtt aaggatagat gatagtcagc aggctgaaat 720
   tttctctcta gtggaccact ttgacacatt ctcaaggttt tatttgtata cattaccaca 780
   agacatggac ttcagttaca atggcagcaa atataataca tgctactaac tatgaatttt 840
   taggtaagat gaaatgcttc ttaaaagatt gttggtcaga aatctggaga ttggtacttt 900
   ttctcagaaa gaaaatcagc tcaacctaat ggctgtatac tgcattgtaa catttatttt 960
   tatttaaaag ttggattttt atgatttaaa aaagtaatgt gagtcttcat ttaaaagttt 1020
   aaatggcata aagacagcaa gttttcatat agtagtaaaa ttgtatgaat tttagacaat 1080
   gattgatggc ctctcttgac tgtatcttaa acaaaaggac ttactgcaat tttttcatta 1140
   tcagtgttat gcaaatatac cagtaaacga ctgctttctg agatcttagg gaagtaatga 1200
   ttaatgaagt ataaaaatgt aattgtaatt atatcaccta aattatgcca aagagataca 1260
   ttggtgacat acttatgcta aaaagagaga gagaaaaaaa gactccctct ctccccaaat 1320
   cttacaacac attaaatctg tcttctgttg catgttgaat ataacagtat caggaactag 1380
   atgaaagttg gttattcatt tatgatgatg atgaggctgc tgaagcagtt ggtatagttt 1440
   tgtgcattac agagaatgtt gtatgccagt taacaaatag acagccacta tgtgcccagc 1500
   acagagagaa atgagtcatg gtttgtgacc tccatggatc ctaacatttc ttctcattaa 1560
   ttccttcact tggtatttac tgaatgccag tatgtaccaa atattgagca tgacactggg 1620
   aataagggtg tccagaagac aaaatctttt tcttgatgga gttgacggtt taatggagtc 1680
   atggcataag cttccgaaac agttgaagga tgagtaatac acagaaaatt gtcaggagaa 1740
   aattatgtgg ggtataacca ttgcactaga ttgttttgta gaggagacag attggcactg 1800
   aactttgtgg ctaatatttt agaccatgag ataggaaatg tattcaaaag gctgaaaatt 1860
   acatgggaga gagtaaatat gatcagagat ttaacccata ggctgttacc tggagaatgc 1920
   ttgggtatac attgacttat aactaagaaa gatgaatttc aattttctgc ttaggtatgc 1980
   tgaagaaaaa ttaaaagtgt aactccagag aagtaagttc agaaagttag caaaaaaaaa 2040
   aaaaaaaaaa aaggccacat gtgctcgagc tgcag                        2075
<210> 179
   <211> 1754
   <212> DNA
   <213> Homo sapiens
<220>
   <221> misc_feature
   <222> (1)...(1754)
   <223> n = a, t, c or g
<220>
   <223> nbla-10242
<400> 179
   gcctgtacgg aagtgttact tctgctctaa aagctgcgga attcctcgag cactgtggcc 60
   tttttttttt tttttttttt ttagacaagg tctcacttta tcacccagct ggagtgcagt 120
   ggtgcaatca tgacttgcta cagcctcgac ccccaagggc tcaggtggat cctcccatct 180
   cagcctcccc agtagctggg accataggcg tgccccacca cacctggcta atttttgtat 240
   tttttgtaga gacagggttt caccatgttg cccagaccgg tcttgaactc ctagactcaa 300
   gcaatcctcc tggctcagcc tcccaaagtg ctgggattac aggcgtgagt caccacaccc 360
   acccaagaat tttaatttat gtggactacg cttacacctt gctgcccttt gattttcttc 420
   caaacagcaa tgtttaatca ttttcttgct ctcataggac taggttaact taaactgact 480
   cattcactga tacagggcct acagcaggaa gcccaggacc tcccacccct caaagccttt 540
   ttctggcaag actgtccaaa gcccctttgt cacgaactca ccttccaggc atggtatgtg 600
   cccgaggggt cagtctgata gagcctagga gtgccatcaa agtcgaaacc cacgatgagg 660
   gcagagatgc caaacggcct gcgcccattg ctctgcgtat aacgctagca agaaaagaaa 720
   caggtcagtg ccatccaatt aagaaactgc atgtgaggaa agaaccgtac ttagtgaaga 780
   tacgaacatt ttaaaccatg cctgagcccc ttctcaggtt atcagcagtt caaatgatac 840
   attagccaat agctttcaac atccttttct gcattttcac tcctagaaaa actcacctag 900
   gaccagaatt gtgaactcag gttactggaa attactttga caggtagaca cacattctgc 960
   ttacatacta atggcatgtc ctcatagggt ggcttccagc ccccttcaac tgctttgtga 1020
   ctggtcactc aggatggcaa gacaaaagct atcatttccc tccagacaca ggaatttgga 1080
   atatcccatg ccaggaactg agaacagaac aatgttactg aaattggtga gctaggtcaa 1140
   gttacacttg tgcaagtcaa gattaggatc acgccccaga ataaatcagt tttctgattc 1200
   acctgaagtg acatggcagg accctccatg gcggagccgt gactgccctg ctgccagagg 1260
   tgagcatgca agcgggcagg cacccacctg cttcagactg gcgatgtagc gggtgatgta 1320
   ctccacagtg accgggtcct ccacagtcag ccggtggctc tggcactcca cccgggccct 1380
   gttgatgact atccttgcat cggcggtgag gcctgcaagg aaagcagaga ggcttctgct 1440
   agagagacag cacaaactac agggtgggga cagacacgat gacccagcat ttaaccttcc 1500
   acagaccccc caaaccggca gcaaggctat tctaactgac tggcagcgga acctatcagc 1560
   ccctgggaac ttccaggaaa ttgggtacag atgcttcttg atgtgcagtg gggtggtatc 1620
   ccgctaaact catcgtaagt caaaaatacc tggctgaaaa tgcatttact accccataaa 1680
   cccattgtaa ataaaaaatt gtaggctgcc gtgcaaaaaa aaaaaaaaaa aggccacatg 1740
   tgctcgagct gcag                                                  1754
<210> 180
   <211> 1668
   <212> DNA
   <213> Homo sapiens
<220>
   <221> misc_feature
   <222> (1)...(1668)
   <223> n = a, t, c or g
<220>
   <223> nbla-10283
<400> 180
   gtacggaagt gttacttctg ctctaaaagc tgcggaattc ctcgagcact gttggcctac 60
   tggctgtgtg aatcctgtac accctgggtt ggctgcagtt ttcctttcaa tctcgtggat 120
   gctgtgtcct tcttacttgg tttttcccag gcagcccttt cctgtagagc cagtatgagg 180
   tagccaggtt ctcagctgtt tcgagtgctt tgccttctct ggttatctca agcatcagga 240
   aaattatatg ttgcatacat tccacagagg tcccatggtg atggggaaag gtcagctcag 300
   gcattccctc tgcttagcca cacttcatca tactgcttgg atacatgttg ctgcctagga 360
   gactttctta acccaaacag gccccactgc ctttagcagt ttcttcatct tctctgacat 420
   acaattcagc tttggcccag agtggagatt aagatatggg tctgactctg cctctctttt 480
   ccctgtgtga ctgctccagt tgctcctaac tgaatgccct gtagctccca agcttgcagg 540
   ctctgccatg ggatgcctgg gaaagtaagg cacttgtact cttccatttt tgtctggatg 600
   ataccagtgt ctgcctcttc tcccattatt catgtaaatg caagagaaaa aaagcacttt 660
   aataattttc aaaaatttct catattacat tctctattgt gtctggctct gaagttaggt 720
   ttttgtgttt ttttcccaca attattccta ctgtatgtcc ttgctgcaat ccaagtgctg 780
   accttatttt tttagagctc gttctttcta aagttaaact ttactatgca tagaagtaat 840
   tttgaatctt tcaaaatgca gattctcatt gagtaggtct ggggtggggc ctgagattct 900
   gcatttctca taggctcccg gggtgcttgt attgctgagc cacagtccag tccatctcag 960
   agtcctagag gaggtaatct tctctggttc ttttccattt atccatttca taagcatttc 1020
   tcacatctta tctggccttt gccacattca tgaaacctat cactttatca gtaaggtatt 1080
   taaaacactg ccttgcacag agtaaacatt catcatgttc tagatattca catgtacatg 1140
   aacacacaca tatctatcac acttaatatt attgctgttt tatagttaaa cttcttctaa 1200
   tgtcaatcta gaaacttgtt ttagtagtgc attttgagag aaaccatttg gtatcatctt 1260
   gcatggaaac atccgcttcc ttaggaatga ccaaaaacta taattagtga taatgaatct 1320
   tttatgattt ggcaagagtg ttttaagtgg agttcttttg atgatacgat tgttcaggtc 1380
   aggctgaagg agccccaata ataatgtttt tctgactgtt ttcctctggt aatgtttgtg 1440
   ataaaattcc tttgtcataa ataaatattg ttgctggtgc agactgaata tttgtattcc 1500
   cccaaaattc acatgtcgaa accctaaccc ccaatgtgat agggtttgga gatggggctt 1560
   ttgggaagta attaaggtta gattaggtaa tgagggctgg tccttcatga tgagattggt 1620
   atccttatta aaaaaaaaaa aaaaaggcca catgtgctcg agctgcag         1668
<210> 181
   <211> 2161
   <212> DNA
   <213> Homo sapiens
<220>
   <221> misc_feature
   <222> (1)...(181)
   <223> n = a, t, c or g
<220>
   <223> nbla-10300
<400> 181
   gtacggaagt gttacttctg ctctaaaagc tgcggaattc ctcgagcact gttggcctac 60
   tggccatatt gctgctctca ttgcagagta tctgaaaaga aagggttact ggaaagtgga 120
   aaagatttgc acagcatccc tgctctcgga ggatacccac ccctgtgata gcaactcatt 180
   actaacaact cccagtggag gaagcatgtt ctctatggga tggccagctt ttttgagcat 240
   tacaccaaac attaaggaag aaggagcgat gaaagaggat tctggaatgc aagatacacc 300
   atacaatgag aatatcctgg tggagcagct atacatgtgt gtggagtttc tctggaagtc 360
   tgagcgatat gaactcattg ctgatgtcaa caagcccatc attgctgtct ttgagaaaca 420
   acgagacttc aaaaaattgt cagatctcta ctacgacatt catcggtcat atctgaaagt 480
   ggcagaggtg gtgaattcgg agaagcggct gtttggtcgc tactatcgtg tggcatttta 540
   tgggcagggc ttttttgaag aagaagaagg taaagagtat atttataaag agcctaagct 600
   gacaggtctg tccgagattt cccaaagatt actcaagctc tatgcagata aatttggagc 660
   agacaatgtg aagataatcc aggattccaa caaggtaaac cccaaggatt tggaccccaa 720
   atatgcctac atccaggtga cctatgtgac gccgttcttt gaggaaaagg aaatcgaaga 780
   ccggaagaca gatttcgaaa tgcaccacaa catcaaccgc tttgtcttcg agacaccctt 840
   cacgctgtcg ggcaagaagc acggtggggt ggcggagcag tgcaagcggc ggacgatcct 900
   gacaacgagt cacctgttcc cctacgtgaa gaagagaata caagtaatta gccaatcgag 960
   cacagaactg aatccaattg aagtggcaat tgacgagatg tccaagaagg tttctgagct 1020
   taatcagctt tgcacaatgg aagaagtgga catgatcaga ctgcagctca aactgcaagg 1080
   aagtgtcagc gtgaaggtta atgctgggcc aatggcctat gcacgagctt ttcttgaaga 1140
   aaccaatgca aagaagtacc ctgacaacca agtaaagctt ttgaaggaga tcttcaggca 1200
   atttgcagat gcatgtgggc aggcccttga cgtgaatgag cgcctcatca aagaggacca 1260
   gctggagtac caggaagaac tgaggtccca ctacaaggac atgctcagcg aactctccac 1320
   agtcatgaat gagcagatta cgggcaggga cgacctgtca aagcgcggag tggaccaaac 1380
   ctgcactcga gtaattagca aagcaactcc ggccctaccc acggtctcca tctcatctag 1440
   tgctgaagtc tgaggactct gcagcatcag acccacctct aagagaactt tctgaatttg 1500
   cagctaatct cggggaagag aaagataggt ttaatttatt tgaagttttc atggtgttaa 1560
   tatttttgtt tacctcgcta gcttcagaat tttgccaacc tctgaatttg cacattttgt 1620
   ataatttttt ttttctttga gcagtgttga tcaagccaag ttgaatattt gccatgaaat 1680
   tccagtgaat gtgtagctca aatgcaaacc ctaagtttgc tgtcagttat tgtatggtca 1740
   gtaccccagt cctagtacac atattttaaa ggttaaagtg aatgtttttg taacatttaa 1800
   gcatatttca gatgtaaata aaagattgta aaatatacgg tttttaccaa atttaaaaga 1860
   tcctttttag ttaatactat gacagtacta aaaatatatg aataacattt cagataccat 1920
   tatattaaaa tatttgtgta tgtgtacaaa agcgttgata aatactaatc tttaaagttt 1980
   gtggagttcc tttatttgta atatatgtgc tcttaaaagc aatgggatgt gaaattatga 2040
   aagtatttta ttgttcatag aaataaaaaa cacagttact ttgcaaaaaa aaaaaaaaaa 2100
   aaaaaaaaaa aaaaaaaaaa aaaaaaaaaa aaaaaaaagg ccacatgtgc tcgagctgca 2160
   g                                                             2161
<210> 182
   <211> 777
   <212> DNA
   <213> Homo sapiens
<220>
   <221> misc_feature
   <222> (1)...(777)
   <223> n = a, t, c or g
<220>
   <223> nbla-10313-f
<400> 182
   gngnnngtgg ggnnnnnnnt ttggantcct cnagcctgtt ggcctactgg agatgtttgg 60
   ataaatacta aaaaagcaaa aaacaaaaac aaagattaac agtgcatgtc agagatagct 120
   gaggagagaa cagctgaaga atttagttag atagaaagcc aagttatggg cctataaagc 180
   tggaattcaa gatatggaaa ccagaagggc agaacaatga tgcagaactg tataacaaaa 240
   taaggcattg gagtctttct tgtattctct ttgtatctta gatgcctcag cctccaatgc 300
   tgcttgctgt tacaacatag aaaaacattg gagtgatttt agagatgatt tttttgtgaa 360
   tatgtcagta aagtttgtgt tgactgttgg taacattgtt catctactga agcactctaa 420
   taataagaaa agtgaattgg agcttgaaaa aattgctgtg acattggaag aagagagaat 480
   gtaaatgaag gggttaattc ttgctgccac tttcttctgg tgttgctgat gcctnaatca 540
   ttcactcaag aaaatggtta ctaaagtacc tcttatggtt tggacattaa actgggtaca 600
   aaaggatgta gaagttgnac ttgcanancc gcgccngaag aaaccacgca ctgactttgg 660
   cttctcggac gactgtacag aagtcttcaa agccttctnt tttctgngca aaaatggcaa 720
   ctcttaangg aaaactcatt ggaccanttg cgggaagaag aagcaacaag ttccaac    777
<210> 183
   <211> 788
   <212> DNA
   <213> Homo sapiens
<220>
   <221> misc_feature
   <222> (1)...(788)
   <223> n = a, t, c or g
<220>
   <223> nbla-10313-r1
<400> 183
   gntgngggtt nnatttgang gccctttgtt ngnnnccntt ttacactaca atagttaatt 60
   ttatttgttc aagagctcag attgcaagca ttaaaccaag cataggcttt gattctgtga 120
   gcccaaattc acatattgaa gaagatcaaa gcaaactgtg atccatgtac atggatgaaa 180
   actaaaggct cgagttaatc acattgtagt ttttaaattt ctacagccta gagctcacta 240
   gtcacaggtc ttttaggtcc ttctggatgt cccacagggt atctgcactt ttcttgagct 300
   gagcaacctc atcatccttt agcttctggt tgataacgct ggttaatccc cgggcattga 360
   ggatacatgg aaggctcagg aagacttcat tctcaatgcc atacatcccc tttaccattg 420
   ttgacacggg atgaatcctg gatagatttt tcaacatgga ttcaataaga tcagccacac 480
   ttaatccaat agcccagttg gtatatcctt ttaacttgat gacttcatag gcactttcaa 540
   ccaccatctt atgcacttnc tttcaatttt cactatcatt ggcaggtccc atttctggat 600
   tcaattcctg gagagaaaca cctggcacat tcacaccant tcaaacaggc acactttgag 660
   tcggcatgtt ccccaaaatc catncatggn aagcttnttg ggatgaatgg ccaagttttt 720
   naagccntaa ggaacgnaaa tntagcngaa tccanattac attcactttc aatacgccgg 780
   gggttggg                                                          788
<210> 184
   <211> 2324
   <212> DNA
   <213> Homo sapiens
<220>
   <221> misc_feature
   <222> (1)...(2324)
   <223> n = a, t, c or g
<220>
   <223> nbla-10314
<400> 184
   ctgtacggaa gtgttacttc tgctctaaaa gctgcggaat tcctcgagca ctgttggcct 60
   actgggatat tcaaaaatcc atggctgcct tcaaaaacct ttcagcccaa tgtcaagaag 120
   tccggaagag gaaagagctt gagctgcaga tcctgacgga agccatccgg aactgggagg 180
   gcgatgacat taaaactctg ggcaacgtca cttacatgtc ccaggtcctg attcagtgtg 240
   ccggaagtga ggaaaagaat gaaagatatc ttctactctt cccaaatgtt ttgctaatgt 300
   tgtctgccag tcctaggatg agtggcttta tctatcaggg aaagcttcca acgacaggaa 360
   tgacaatcac aaagcttgag gacagtgaaa atcatagaaa tgcatttgaa atatcaggga 420
   gcatgattga gcggatatta gtgtcgtgca acaaccagca ggatctgcag gaatgggtgg 480
   agcacctaca gaagcaaacg aaggtcacgt ctgtgggaaa ccccaccata aagcctcatt 540
   cagtgccatc tcataccctc ccctcccacc cggtcactcc gtccagcaag cacgcagaca 600
   gcaagcccgc gccgctgacg cccgcctacc acacgctgcc ccacccctcc caccacggca 660
   ccccgcacac caccatcaac tggggacccc tggagcctcc gaaaacaccc aagccctgga 720
   gcctgagctg cctgcggccc gcgcctcccc tccggccctc agctgctctc tgctacaagg 780
   aggatcttag taagagccct aagaccatga aaaagctgct gcccaagcgc aaacctgaac 840
   ggaagccttc agatgaggag ttcgcgtccc ggaaaagcac agctgctttg gaagaagatg 900
   ctcagattct gaaagtcatt gaagcttact gcaccagcgc caaaacaagg caaacactca 960
   attcaacatg gcaaggcact gacctgatgc ataatcacgt cttggctgat gatgaccaac 1020
   caagcctaga ctccctgggg cgtcgcagta gcctttctcg tttggagcct tcagacctct 1080
   cggaagactc tgactatgac agtatatgga cagcccatag ttacagaatg ggttctacat 1140
   ctcgtaagag ctgttgctca tatatctctc accagaacta atgcacttct gagcttttct 1200
   taacaaatgc ttgttgtatc gcagcctgct tttcttagat gttttcttgc tgttccttgt 1260
   ctcttttatg tgatatttta acaacttttg agagtgttcc tgatatttcc cattgtactt 1320
   tgtggaggct taactgccga tgcgagtata cattcgaatg actgactgtt ggagggaggt 1380
   ggtgtgttca cgtgagccta catctgaggg tgctcttcgt aaggggtagt tgttgtttta 1440
   tagctccaaa tggttttttt attctcagtg tagcaactgt ttgatttctt tctggataca 1500
   gctgtatgaa cacttttcta ttttatactg aaatcacaca ggtttgtgat ctaagtgcca 1560
   gaaccatgga ggagactctg gccttatctg tagggaccta aattttttaa gtgctatgaa 1620
   cactgatcaa gtagttgtgg agagatgaga agaatatatg cagcaaaggt gctgtgagcc 1680
   cagttccaac ggctgtgttc tggtaatcgt gcagaacagc atgtgggctc tgggccttgg 1740
   caggtgcaca gacgggcggg cgtcacgtgt gttcagagct tgtctccacc tcacggctct 1800
   gtgctcttct gtgtgctgcg ttgtggccgt gaagtgggag ctctcctttc caaaggcaga 1860
   gcttactgtc atggctcaga tgttcactcg catcttcacc ccaaacttcc ctgaattgca 1920
   gtgatttctg ttataaggca catagcaagc ctgtaccact ctacagtaaa atctctacac 1980
   taacagttta aagcccgaaa gctgaagcat tcagcctctg caagtgtaga ctactggact 2040
   gtagtagagg catttggaat acttacaaag tcaagtgctg tggcttcagt tcccccggct 2100
   ccccacccct gacatccata cttatgttgt ctggaagagc catggacaca tcattatttt 2160
   cagcaaccag ctgcagtgaa actccattta cagcaacccc atagtataaa accacaatgc 2220
   ctcattgcaa tgggatattg aagttcattg aagaattact aggttgccat gtggctcact 2280
   tatttaaaaa aaaaaaaaaa aaggccacat gtgctcgagc tgca                    2324
<210> 185
   <211> 783
   <212> DNA
   <213> Homo sapiens
<220>
   <221> misc_feature
   <222> (1)...(783)
   <223> n = a, t, c or g
<220>
   <223> nbla-10327-f
<400> 185
   gnggnnnnng ttgggntcnn nttganncct ttgcactgtt ggcctactgg ttacgatgca 60
   gctgatgaag agggagaaag gggtgttggt ggctttgccc aaatccaaat ggatgaatgt 120
   agaccatccc attcatctag gagatgaaca gcacagccag tactccaagt tgctgctggc 180
   ctctaaggag caggtgctgc accgggtagt tctggaggag aaagtagcac tagagcagca 240
   gctggcagag gagaagcaca ctcccgagtc ctgctttatt gaggcagcta tccaggagct 300
   caagactcgg gaagaggctc tgtcgggatt ggccggaagc agaagggagg tcactggtgt 360
   tgtggctgct ctggaacaac tggtgctgat ggctcccttg gcgaaggagt ctgtttttca 420
   acccaggaag agtttgctgc aacagggtgt gctggagtat ctgtctgcct tcgatgaaga 480
   aaccacggaa gtttgntctc tggacactcc ttctagacct cttgctctcc tctggtagaa 540
   gaggangaag cagtgtctga accaaacctg angggttgcc agagcctgtg atgacttgga 600
   gntagcagat gacaatctta aagaagggga ccantttgca ctgagtccag ccagcaggaa 660
   cccatnaacc aagtcaagct ttacacgcct taacaanttt tccttggtac tacttttacc 720
   aaaccggaan atggacagnn tatggtcctg nanccctggg aatgnnccct tgctcnnncc 780
   gga                                                               783
<210> 186
   <211> 775
   <212> DNA
   <213> Homo sapiens
<220>
   <221> misc_feature
   <222> (1)...(775)
   <223> n = a, t, c or g
<220>
   <223> nbla-10327-r1
<400> 186
   gnnngnngnt tnnnttgntg ccctttttnn nnnccntttc ctcctttctc ttcagagagg 60
   ggatctgaag tagctggtgt gtccagtttc atgaaggctg cttcaatagc ttggctgaag 120
   gaattttgaa aactgggcac aggaacacgg tctgaattat cactctcccc gtcgctgtcc 180
   acaggggcag gaggaactaa gctgttctca tctttctttg gagcagtttt gggccacaca 240
   tctgcttttg cttttccaac cctcagcatc tgggcaaagg aagggaaggg agagtcttct 300
   tccagactcc caacgcagaa tgagggactg ccctgactgg cagtgggtga cagaggggtc 360
   agcagaaagt ctgcatggga acctggactt ctgctgagag gagaaatgga gagggcccca 420
   tggccctcgg tgctggtggg acccaaagca gaatcagagg agcaggtata agaattgaag 480
   gcaggaaact gctgtagatt ctcgagggga atgtggactt ctgggtactt gccctgtttc 540
   ttgtctcctc tatctcaatc ctgcgctctc ggcggccgtt cctccgagcc ttcttttggc 600
   gctgaccgtt tcccttntca atgncatctg agaacatctc tangggttcc ttaaagacca 660
   caggaggttg caagccngnt cacagatctg aactcacagg tgaatgcaag tgaaagagat 720
   atctgngacn ctgncaacat ncttaaacat ggggtaccnn caatttcacc ccagt      775
<210> 187
   <211> 2671
   <212> DNA
   <213> Homo sapiens
<220>
   <221> misc_feature
   <222> (1)...(2671)
   <223> n = a, t, c or g
<220>
   <223> nbla-10329
<400> 187
   gcctgtacgg aagtgttact tctgctctaa aagctgcgga attcctcgag cactgttggc 60
   ctactggctt ttgatgagtc cttttccctg cacggagctt ctggtgaagg tggtgggagt 120
   tgttcttgca gagcacatgg gagtccaacg gcctggcagt gggccaggat cttgttgcca 180
   tggtgactga cactgttttc gctgaaattt tgccagatcc tgaagatctt cgtggacaat 240
   ctctttgcta tcgtcctgct gaagcaggcc acagctgtgc gctgcttgga catgagtgcc 300
   tcccgtaaga agctggccgt ggtagatgaa aatgacactt gcctggtgta tgacatcgac 360
   accaaggagc tgctttttca ggaaccaaac gccaacagtg tggcctggaa cacccagtgt 420
   gaggacatgc tctgcttctc gggaggaggc aacctcaaca tcaaagccag catcttccct 480
   gtgcactggc agaagctgca gggctttgtg gtcggctaca atggctccaa gatcttctgc 540
   ctccacgtct tcatttctgc cgtggaggtg ccacaggtaa ctgggagcac ctgtccactc 600
   tcagcactgg caaggccagt aagaccaggg aagccgggcc cctggctggt gctttgagga 660
   gagagtagca aggagagaag aatggcagct gtgatgtcgt gggaggcagt ctgggcttgg 720
   aacctgcagc cctatctcca ctccttgcaa cctgtgtgac tgggggcaag gagctcaacc 780
   tccctgtgcc tcaatttctt cctggtcccc cttctggtaa ggattcagag gggtatacag 840
   tgttgtcagc agagtgtatt ttacagagag cccagtgtgg gccaagtgga gagagcagca 900
   aagccgggag tccagcaggg tgtgtgtgtg tgagcacatg tgtgatggtg gtgatgtgtg 960
   tgtgagagag ggggaagaga gagagaatga gtgggtgggg ggggtgaggc ctgaggtctg 1020
   tgagccaata agggagcatg ggactctgca caagccccct gaactaggag gtggccacag 1080
   cagaaatggg gcactgaagt gtggagccac agaatgcggg agggcagaac cacagacagg 1140
   aggctgagat tgacctcctg agtgcaagct ggtctcccct tcacctcctg caccccacgc 1200
   agatggtgct taccatagga ttgccgtaaa acagagacac gcaccagcga gaaactttag 1260
   cccttagtat cccatcctca ggacagaatc actcttaaac atgttgaaat acatctgctt 1320
   agagcttttc tatgtgtcta tataatgtat gcataatata caattagaag catgtgattt 1380
   tataacattt ttaaacaaaa gtggaatcac actgcaaata ctgtttcatg acttttatgt 1440
   gtgtaagtat tgctacctat agatggaacc tcagatttac tgtattcccc aagcctggac 1500
   tgtaaacaag acttctcatc ccttcctcta tggccatctt ctcatttcac ctcacatttg 1560
   gttatgacag ccccatcact gtctatgaag tgctggctgt aaactcgtca tctggccttt 1620
   tcactcagca atctcctgaa gcatggttct gtgtcagtaa atgttcttat ccagcatggc 1680
   aaaatgttaa ggtaattgta gactcacata agttgcagga aataatacag agatccctta 1740
   taccctttgc ccagtttccc gcaatggtaa cagtgtgcaa agctccagga cgattttgcc 1800
   acccatgttg gcatgtgtaa ccgcccccca cagtcacgac actgaacatg gccaccacca 1860
   caagtgccta gtgttgtgct acccttttac agccagcgcc cctccctcct gcccggcccc 1920
   cctgcgttgt atttatggct gcattttgtc ctgtcttgtg gatgttccat ggcggacaga 1980
   caggctgaac aaacaaaggg tgtgcaaaga agcccagagc aggagagaag gctccccagc 2040
   ctggctcttg gtcagcacag tcacaggctg gctctgcctc tcttggctgc actgtcctga 2100
   agcaggaaat atgagaaaaa caagtaaagg gaaaacaagt ccagttggag ctatgataac 2160
   attatctgca aggccaggca ggggccccaa agaaatggga tccaagagca gggacgagaa 2220
   aagcaagttc ttagtctccc cctggaattc tttccccatg ccatcattct ttgttctgct 2280
   cttataacta tttttgtaac tatttctgca agtttgcaag gattttataa gttcatgttt 2340
   tcccatctat gcaggatggc aaaggtcaca agacatgcct gaattgcaaa acctgtcaca 2400
   gtttgattaa ctgcctttgt tctgcttctg taagctcgct ttccccaccc cgcaagtttt 2460
   gcaccactgg ctagccactc cccttcagtc gcctgaataa aagtcaagcc tgatctttgt 2520
   tcgttgctca gcctctggat attaatccgc tgggccagtg gccacctgaa taaaatcctc 2580
   ctatttcaaa aaaaaaaaaa aaaaaaaaaa aaaaaaaaaa aaaaaaaaaa aaaaaaaaaa 2640
   aaaaaaaagg ccacatgtgc tcgagctgca g                                2671
<210> 188
   <211> 3129
   <212> DNA
   <213> Homo sapiens
<220>
   <221> misc_feature
   <222> (1)...(3129)
   <223> n = a, t, c or g
<220>
   <223> nbla-10363
<400> 188
   gtacggaagt gttacttctg ctctaaaagc tgcggaattc ctcgagcact gttggcctac 60
   tggatttgaa aacacgctcc gggagctaga gcctgaggtc ggcggcgcac gctgttgccc 120
   cgtgggcttc tgctccctcg cttgtcttct cgggcttctc gccccggccg cggccgggtc 180
   ctcagtagtc gagaatgact aaaaaaagaa aacgccaaca tgattttcaa aaagtaaaat 240
   tgaaagttgg taaaaagaag cccaagttac aaaatgctac tcctacaaac tttaaaacaa 300
   agactataca tctgcctgag caactcaaag aggatggaac acttccaaca aacaatagaa 360
   aacttaacat aaaggatttg ctgtcacaga tgcatcacta caatgctggg gttaaacaaa 420
   gtgctcttct tggacttaaa gaccttttgt ctcaataccc atttataatt gatgcacacc 480
   tttcaaacat attaagtgaa gtgactgctg tgtttacaga taaagatgct aatgtacgat 540
   tagcagcagt tcaacttctt caattcctgg cccccaaaat acgagctgaa caaatttctc 600
   cattttttcc tttggtaagt gcccatctct ctagtgccat gactcacatt actgaaggaa 660
   ttcaggagga ctctttaaaa gttttggaca ttctgctgga acagtaccca gctctaatta 720
   ctggccgtag cagcatattg cttaagaatt ttgtagaact tatttctcat cagcagctgt 780
   ccaaaggact gataaataga gacagatccc agtcctggat actttctgta aatcctaatc 840
   ggagactcac ttctcagcaa tggaggctga aagtcttagt gagactcagt aaattccttc 900
   aggccttggc agatggatcc agtaggttga gagaaagtga aggacttcag gaacagaaag 960
   aaaatcccca tgccactagc aactccattt ttatcaactg gaaggaacat gccaacgacc 1020
   agcaacacat ccaggtttat gaaaatgggg gttcacagcc aaatgtcagt tcacagttca 1080
   ggctacggta tctggttgga ggactgagtg gtgtggatga aggcctgtca tctactgaaa 1140
   acctgaaagg atttattgag ataataattc cattgctaat tgaatgctgg gttgaagctg 1200
   tacctccaca actagctact cctgttggga atggtataga acgagaacct ctacaggtta 1260
   tgcagcaagt tcttaatatt atttcccttc tgtggaaact ctctaaacaa caggatgaaa 1320
   cccataaatt ggagtcatgg cttcgaaaga actaccttat tgattttaaa caccatttta 1380
   tgagtcgttt tccatatgtc ttaaaagaaa taaccaagca caaaaggaaa gagccaaata 1440
   aaagcatcaa gcattgcaca gttctctcca ataacataga tcatctctta ctgaatttaa 1500
   cactgtctga tatcatggtc tccctggcaa atgcgtcaac tttgcagaag gattgcagtt 1560
   ggatagaaat gataaggaaa tttgtaacag agacccttga agatggctct aggctaaata 1620
   gtaagcaact gaacagattg ctgggagtat cctggaggtt aatgcaaata cagccaaaca 1680
   gagaggacac agagactctt attaaggcag tttatacatt atatcagcag aggggcctta 1740
   tccttccagt tcggactttg ttattgaagt ttttcagtaa aatctatcag acagaagaac 1800
   tgagatcttg tagattcaga tatcgtagta aagtgttatc ccgttggctg gctggcttac 1860
   cattgcaact tgctcatctt ggctcccgaa atcctgagct ctctacacag cttatcgata 1920
   tcattcatac cgctgcagca cgagcaaata aagaattact aaaaagttta caagcaactg 1980
   ccctccgaat ttatgatcca caagaaggtg ctgtggtggt tctccctgca gactctcagc 2040
   agcgtttggt tcagcttgta tatttcctac ccagtctgcc ggctgatttg ctttctcggt 2100
   taagtcgttg ctgtattatg ggaagactca gttcaagttt ggctgccatg cttatcggga 2160
   tactgcacat gagatcatca ttttctgggt ggaagtattc agctaaagac tggttgatga 2220
   gtgatgtaga ctatttcagc ttcttatttt ccacacttac agggttttcg aaagaggagt 2280
   tgacttggct tcagagcctt cgaggagttc ctcatgtcat ccagacacag ctttcccctg 2340
   tgcttctcta ccttacagat ttggatcaat ttttacacca ctgggaagta acagaggcag 2400
   tttttcacag tttattggtt attcctgccc gaagtcagaa ctttgacatc ttgcaaagtg 2460
   ccatcagtaa gcatttggtt gggttgactg taattcctga cagcacggct ggctgtgttt 2520
   ttggtgttat ctgtaagctc ctggatcata cttgtgtagt tagtgagact ctactgccat 2580
   ttctggcttc ttgttgctac agtcttcttt attttctgct cactatagag aaaggggaag 2640
   cagaacatct aagaaagagg gacaagctgt ggggggtctg tgtctccatc ctggctctct 2700
   tgcctcgagt cctcaggttg atgctgcaga gcctgcgggt gaacagagtt gggcctgagg 2760
   agctgcctgt tgtgggccag ctgcttcgac tgctgcttca gcatgcaccc ctcaggactc 2820
   atatgttgac caatgcgatc ttggtgcagc agatcatcaa gaatatcacg acattgaaga 2880
   gtggaagtgt tcaggaacag tggctcacag acttacatta ctgctttaac gtgtatatca 2940
   ctgggcatcc ccaagggccc agtgcactgg ctacagtgta ttgaagaggc catagtacct 3000
   cctgtttgaa gttgtttatt cacatctatc ttatttgaag aaaaagactg atgtaataga 3060
   tctttgtcat taaagctgaa cttttaaaga aaaaaaaaaa aaaaaaggcc acatgtgctc 3120
   gagctgcag 3129
<210> 189
   <211> 589
   <212> DNA
   <213> Homo sapiens
<220>
   <221> misc_feature
   <222> (1)...(589)
   <223> n = a, t, c or g
<220>
   <223> nbla-10367
<400> 189
   gcctgtacgg aagtgttact tctgctctaa aagctgcgga attcctcgag cactgttggc 60
   ctactgggaa gatgcaaata aatactttga tctatgtttg attttgctaa taatatttga 120
   aggagattgc ctaccaagga caaaacaata aatttaaaaa tcaaacgatt tctccatacg 180
   ctcatagtca catatggaat tttgagaaaa taaagcatgc tgtctttagg aatttttata 240
   cttctttgtc tttcttcctt aatatttgct tctagctgct cttggcaatg atgaattgtt 300
   atgtatgcat taatgttttg cagcccaaaa gttgttcaca tttttcctat ataagatctg 360
   tggagtgtgt gtttcaaaga gagaactaca gaaatgttaa agcgggaaaa cctgaatgtg 420
   atgtgcacat tttcatccca catggacaat gtatgtgttt taataaatgg aattttcaga 480
   ttcaaaaaaa aaaaaaaaaa aaaaaaaaaa aaaaaaaaaa aaaaaaaaaa aaaaaaaaaa 540
   aaaaaaaaaa aaaaaaaaaa aaaaaaggcc acatgtgctc gagctgcag 589
<210> 190
   <211> 788
   <212> DNA
   <213> Homo sapiens
<220>
   <221> misc_feature
   <222> (1)...(788)
   <223> n = a, t, c or g
<220>
   <223> nbla-10371-r1
<400> 190
   gnagngggtt nnnttgatgg ccctttnttn ntncntttta ttgctgatat gttggtttta 60
   attcaaaaga aacgatgatg ccaatggttt ggattgacag cagcttaagg aggaaaaaaa 120
   aaaaaagaaa gaaaaaaaga aaaggctggt gacacccccc tggcgtggtt gacctggtcc 180
   cagtcgcagt ccctatggga cagcagagct gtcaaaacag aacccaatga tgacggctga 240
   aaatcaagcc agactgctgt gcggtcagct tgccgccgtg gaatgccttg tgtcttggcc 300
   atgtccagcc aagggtacaa cgtgcttgtt cccgatcacc caggtttgcc attggaagtc 360
   aaagacagaa tcgcttcatg gcactacaga tgtggaaaaa taaaaatctc agctagaaag 420
   aacgtccgat ttggagatag cgggaggaca cgaaggagtg ggggccattt tggtgctgag 480
   aggagggtgc cccaacttca ggaggaccat gtgacggctg tggttgtttt cggggtcact 540
   tgcagcacac acagcgtccc cttgatgctc gatggggacc gcaaacggcc tgcagaccta 600
   acccctggct gtggacagga aaagcttgct tcaagcttct ttccttctca atgctttaca 660
   cangatttcc ttcatttggg ctttcctttg ggttaacaag ttaaaaaaga anatgagggg 720
   gcnaaatggg ttggcacttt gtccaaactg gaaanaanag gtggaantgg gccccnaaat 780
   ttctgggg 788
<210> 191
   <211> 2935
   <212> DNA
   <213> Homo sapiens
<220>
   <221> misc_feature
   <222> (1)...(2935)
   <223> n = a, t, c or g
<220>
   <223> nbla-10383
<400> 191
   gtacggaagt gttacttctg ctctaaaagc tgcggaattc ctcgagcact gttggcctac 60
   tggctcttac agtggtccct gctggccctt ggtgacgggt cgcgtcagtt ccgacccgga 120
   cccgtacgct gctgcgctga cgtggctcct ggaagcaggg ctggcgtagg gccgccatgt 180
   tgcagcagga tagtaatgat gacactgaag atgtttcact gtttgatgcg gaagaggaga 240
   cgactaatag accaagaaaa gccaaaatca gacatccagt agcatcgttt ttccacttat 300
   tctttcgagt cagtgcaatc atcgtctgtc ttctctgtga gttgctcagc agcagcttta 360
   ttacctgtat ggttacaatt atcttgttgt tgtcgtgtga cttttgggca gtgaagaatg 420
   tcacaggtag actaatggtt ggcctacgtt ggtggaatca cattgatgaa gatggaaagg 480
   gccattgggt gtttgaatct agaaaggagt cctctcaaga gaataaaact gtgtcagagg 540
   ctgaatcaag aatcttttgg ttgggactta ttgcctgttc agtactgtgg gtgatatttg 600
   cctttagtgc actcttctcc ttcacagtaa agtggctgga atctgaacct gtgatgttaa 660
   gaaatcagta aatattaaaa agaagatgga tgcaagaagg atgaagaaag aagaaggact 720
   cacagaaaac actggacttc cccggaagct acttgaaaaa catgacccct ggccggccta 780
   tgtcacctat acctctcaga cagtgaaaag actcattgag aaaagcaaaa ctagagaact 840
   ggaatgcatg cgtgccctcg aggaaagacc ctgggcatca aggcagaata aaccttccag 900
   cgtcattcag ccgaaaagga ggaagtcttc caagtcttct ggcaaagctg tgttcaggga 960
   cacgctgtca gaatccacgt tatcaatgtg gggcgcttat tcggtattgg ccatggctcc 1020
   taccatgatc ccagaaccca cacacttaca tgcggattcc agagactgtc caactgaaaa 1080
   ctataacaag atcatctttg cccgcaagcc tatgatgagg atgctcccta cagttcgcta 1140
   ctgaccaaca aggagaaaca tgcaaatgtt ttagccaggg gtccctgcag ccattcccca 1200
   agaacgtaca aaagttaagc tccatttacc actcacttga cagtgtcctt taaatctgaa 1260
   cttgctcttg ctcatgcgga gcttggctaa gagaaaccag atcagaatgc aagctgtagg 1320
   tgggtgggtg agtgtgtccc ctcccacact gctctctggt gggtttgggc agtccctggc 1380
   tctagtctct gctgcagatt ctcaggacca aaatgtgggt gatttgtctg ggtccaggca 1440
   ggagacagaa atcacacaag ctcttttatt agagataata taaagaatga ttaaccaggt 1500
   gacagtaaag ggcaggaagg gaatacaaag gtatccatgg aggtagcacc acaggaagca 1560
   ggcatcacac ttgaggcttg ggaacaaggg gaagatgctg gactcattga aaggtggttg 1620
   cttggagaag ggacatgggg ctgaatctca gacttctgag gtgggggagt ggggatgcca 1680
   ctcaatgcag ctggtatctc tgagctctga ggagagtcct gtggggatgg tgccccctcc 1740
   tctgaatagg ggagatgttt tgttggtgtc tctgagggga cccccatgag gctgcttttg 1800
   tgagtgttgg aaaaaccaaa aactgcattg aaatgtttct gggaggagtt gccactgcct 1860
   gggtgaagaa aaagcactgc tagggttagc aacctgtctg gcaagggaca ggaagctgat 1920
   agaaagtgaa caggaagcaa acaggaagtg agcaaggggc cacttcctac ttcctccttt 1980
   agcctagcag attccttcta gctccccctg ggggctgagc ctatcagggc acaaagccag 2040
   aatgtggttt gtagaatccc agccccagca tctccaagca taggatggat tttgaagcca 2100
   agagggaatt agctctcggc cgggttctga gtgatcccat ccagctgctc ctggacctgg 2160
   tattgagagg tttttccctt ggagggcaaa caaagtggca gtggggacca gggtgtcccc 2220
   tgctgagaaa tggagagctc ttttcccctg tacttctctg gggtttgcct tgtggaacca 2280
   agtgcttggg ggaggagctc ctggcaggac tccagctgtt atttgtcaga ggacagctag 2340
   gattggttca cccttgctct gagttggccc caaagcgagc tatgctgaac tcttctccat 2400
   ctccaagcag acaacctttg tctttacatg caagagggat ccaattatga caggctgacc 2460
   cagctggttt catgtttggg gtttatctac tgagtaaggc tgaccttacc tgagtttctg 2520
   tatgtgtatt tgcaagacag ttaatactaa tccatcatcc ctcacagaga tgtagaggat 2580
   gagatgtagt aacttatagc agtgtcattt ggaaaagttg aatttgccct acaaggcatg 2640
   ttctccattc aagggtcttg tgtaaacgtt tgcagacaca ggtgcagctt tggagaactc 2700
   acctctggct cttgtgcttt tatgttggga cagctgccta aattaggagc tctagacctg 2760
   ttattggttc ccaactgttg gccagaatga attcttcgaa aaatataggt ggtttccagg 2820
   gaatttgaat gattgtattt tgtgtccatg gaacgtttat agacatggag gcaggcaata 2880
   aattcctcca ataattaaaa aaaaaaaaaa aaaggccaca tgtgctcgag ctgca      2935
<210> 192
   <211> 2139
   <212> DNA
   <213> Homo sapiens
<220>
   <221> misc_feature
   <222> (1)...(2139)
   <223> n = a, t, c or g
<220>
   <223> nbla-10388
<400> 192
   ttctaggcct gtacggaagt gttacttctg ctctaaaagc tgcggaattc ctcgagcact 60
   gttggcctac tggaaatgta gatagcctta caagctttaa gtccattaaa aatggtaatg 120
   ataaggacat cactgctgaa ctttctgctc ctgtgaaagg acagtctgat gttttacttt 180
   ctaccaccag caaagaaata atttcatctg aaatgttagc tacttccatt gatggcatgg 240
   aaagaccagg aaatttaaat aaagtagtgg agcagagaca gaatgttgga acacttgaaa 300
   gtttctctcc aggagatttt agaactaata tgaatgttgt tcatcaaaac aagcagttag 360
   agagctgcaa tggtgaaaag cctccttgtc tggagattct aacaaatggg tttgcagtgt 420
   tggaaactgt aaatcctcag ggaacagatg atctggacaa tgtagctgat tcaaagggac 480
   ggaagcctct tagcactcat agcactgagt ataatttaga ctctgtacct agtcctgctg 540
   aggaatttgc agattttgcc acattttcca aaaaggaaag gatacaatta gaagaaatag 600
   aatgtgcagt tttaaatgat agagaagcac taaccattcg ggaaaacaat aaaattaata 660
   gagtcaatga actgaattct gtaaaagaag tggctttggg tagaagcttg gataacaaag 720
   gagacactga tggagaggat caggtttgtg tttcaaaaat aagcatagtg actaacagag 780
   gtttcagtgt tgaaaaacaa ggccttccaa cactgcaaca ggatgaattt ttacagtcag 840
   gtgttcagtc aaaggcttgg agtttggtag actcagctga taattcagaa gccattagga 900
   gagaacaatg taaaactgaa gaaaaacttg acttacttac ttctaaatgt gctcacctat 960
   gcatggattc tgttaaaact tctgatgatg aagttggttc tcccaaagaa gaaagtagaa 1020
   agtttactaa tttccaaagc ccaaacattg accccacaga agaaaatgat ttggatgatt 1080
   ctttaagtgt aaaaaatggt gatagtagta atgactttgt gacttgcaat gatatcaatg 1140
   aagatgattt tggtgatttt ggtgactttg gctctgccag tggctcaact ccaccttttg 1200
   ttactggtac tcaagattca atgagtgatg ccacttttga agagtcttca gagcactttc 1260
   cacattttag tgaaccaggt gatgactttg gagaatttgg ggatataaat gctgtttctt 1320
   gccaagagga gacaatatta acaaagtcag acctaaaaca gacttctgat aatttatcag 1380
   aagaatgtca attggcaaga aaatctagtg gaacaggcac tgaacctgtt gcaaaactta 1440
   aaaatgggca agaaggtgag attggacatt ttgattctgt gccaaatatt caggatgact 1500
   gcaatggttt tcaagactct gatgattttg cagacttcag ttcagctggt cctagccaag 1560
   ttgtagattg gaatgctttt gaggatgaac aaaaagatag ttgttcttgg gctgcttttg 1620
   gagaccagca ggctactgaa tctcatcatc gaaaggaagc ctggcagtca cataggacag 1680
   atgaaaatat tgatactcca ggaaccccca aaacgcacag tgtaccttca gcaacttcca 1740
   aaggagcagt tgctagtggc catttacagg aatcagccac ttcagttcag gtatttacta 1800
   attttctcta ttttgtatga tgtattaatt gttgtggagg cttctaattt cagcttttca 1860
   aaaaatattt tttcaactga gtacttgtta aaggagtctc gttatgatgt ataatgcctg 1920
   tctgatttgt gggttggtta catttttcac aaccctttgg gaaactacaa atagtcctat 1980
   agtaaagcgc agtaagattt ttctttatct caatgagttt cgttaatatt tacagttact 2040
   catcaacttg gatttcttgg tgactataaa taccatatat gttaaaaaaa aaaaaaaaaa 2100
   ggccacatgt gctcgagctg caggtcgcgg ccgctagac                        2139
<210> 193
   <211> 821
   <212> DNA
   <213> Homo sapiens
<220>
   <221> misc_feature
   <222> (1)...(821)
   <223> n = a, t, c or g
<220>
   <223> nbla-10390-f
<400> 193
   gngnnggggt gggntncnnt tttgaanncc cttgcactgt tggcctactg ggctctcgag 60
   tgtctccaag caagatggcg gaggagccgc agtctgtgtt gcagcttcct acttcaattg 120
   ctgctggagg ggaaggactt acggatgtct ccccagaaac aaccaccccg gagcccccgt 180
   cttccgctgc agtttccccg ggaacagagg aacctgctgg cgacaccaag aaaaaaattg 240
   gttgtgaatt cactccttaa accaaaaacc aaattcttat gcagaatgaa gactgctaag 300
   ggaaagctaa aggcatagat gttacctttt taagaaacac attttggaac ctttttgaca 360
   ttttgctaaa ggctgtggga gacactccta ttatgaaaac aaagaagtgg gcagtagagc 420
   gaacacgaac catccaagga ctcattgact tcatcaaaaa gtttcttaaa cttgtggcct 480
   cagaacagtt gacccaagag gtataggtcc tattattttc catatcttac aaataaagag 540
   gatgagagat tatttgtaaa gccaggattc aaacccagtt tatttatgtg aatcaagtcc 600
   tttgntnctt cccagaccaa gaagttggaa ctctctatga gtggtttggc agtgatggna 660
   aactgggttt acantactgn aagtctnaag cgtggggatg aaccccaagg aaaatcaact 720
   tgttctacat gaaaaggatt tcacgggaag aaacagttnt gaaagtttga tcttgtggaa 780
   gaacttacag atgngaccat taagatgggg tacttatggt n                   821
<210> 194
   <211> 821
   <212> DNA
   <213> Homo sapiens
<220>
   <221> misc_feature
   <222> (1)...(821)
   <223> n = a, t, c or g
<220>
   <223> nbla-10390-r1
<400> 194
   gnaggggnnn nntttgtggc cttttttttt ttntttgaga tggggtctca ctctgttgct 60
   cagcaggaat gcagtggtac aatcacgact cactataccc ttgaacttct gggctcaggg 120
   gatcctcttg cctctgcagc ctaataattt ttttttaatt tttatttttt tgtagagttg 180
   gggatcttgc cttgctaacc aggctggtct caaacttgtg gcttcacgca atccccacac 240
   ctcagccttc caaagtgctg ggattacagg tgtaagccac catgcctgat ctggaatgct 300
   ttctaaaaat atggcattat aaacataata ctaaagagaa aaaaaggtta aatttcactt 360
   caagaaatta tcagtctaat agcatcagta aaaatggcac ttttagtcat ggcataaaat 420
   atacaataag gctatttttc aattattgnt ttcaagttgg aaaatgcatt atgaaacact 480
   caaaaagatg ctactgggtt tactcatcaa gtgcttttga caccatataa atgacagagt 540
   aacaccacag ttatgtgatt gggactcaag aatccttaaa tggtaggact gaaaacaggt 600
   atatgactta ttgnccagaa attatatttt tctggatcca ataaaataat taagccctgt 660
   gtattgggna aaaatacagg ctttcaatct ggcctatgng cttgntnttc tggctagata 720
   ttagcttaaa antagcaaaa cagccatttt ccctatattt cccancaaaa tgggaaacca 780
   aacgcctaac ctttttgcct gggggctgcc ttactggctn t                   821
<210> 195
   <211> 2131
   <212> DNA
   <213> Homo sapiens
<220>
   <221> misc_feature
   <222> (1)...(2131)
   <223> n = a, t, c or g
<220>
   <223> nbla-10396
<400> 195
   gtacggaagt gttacttctg ctctaaaagc tgcggaattc ctcgagcact gttggcctac 60
   tgggacaaag ctagcgatgc tttatgctag gaaacttgtt gacagtaacc tgtgcgactt 120
   tatgcagaag acaaatgcta gtaattatta tgcacagagg aaaaatcatt ttaagtatgt 180
   ggtaaagcag cttcatcttt caaaattgat ttgctctggt ttttctttag tccattagat 240
   tccagaatgt ccttttactg ggaatttagt tatgtattaa gataacctgt tttcagttct 300
   ttttgaaaag aagacattat ttatattgaa ccaccttatt ttaaaatttt taacttttat 360
   ataccacttg tgtgattcca gtgtcatgtc ttgggtttga tgtcgttgga cagaaaagtg 420
   tatcaattat tttaaatgaa tttttcccca tgtttgaggc ttagtctgta aatgtgttgc 480
   tgtaacagaa aatacttggg tatgcattac ttgaatactt gaaagctgaa attaataaga 540
   tgtattacat aatgaattag atttctctga acagttttta cactgaaaat cttcatttct 600
   ggattgcagt ttgaaatgga atgaagacct gaattatttg ggtagaaaaa attatgatag 660
   tgcttataag aactgtaaac tgttttaaac tattttgtgt ttgacgcatc aaacttcaag 720
   ttttttgtaa gtttctctcc tgaaattttc tttctcttct atactttatg cacttactat 780
   actactgatg taataaaaga gcagggttaa aaatattgta tctgtattca ttgtgaatcc 840
   tgtagctttt ctagttaaca aaaaatcgct ttctaaaata ctcttaatcc cattgttttg 900
   gttaacatct tacccatttg ttgtatttca aatgccatta atcattttag tacaacacct 960
   atgtttataa aaatttgaaa acattacata ttgtatttaa aactaattag tgaagagtaa 1020
   gaaaaaaact agccaacaga attgtaggtg atgcattagt taaatttcaa aactcataat 1080
   aaaggaactt tcagagattg gttgaaaccc agtggtatcc ctgtaaatta gctcctgtga 1140
   ctggaaaaga ccccaaaaag gcagtagagg agattagtgt ttacttgctg tggttgtggt 1200
   gtgctgctac ttaattatag gtagtgacac actgaaattc ttatttgtcc aataatctga 1260
   agtagtttcc tatatttatc tgtactaaat tgactataaa ttgagtctgc aaagaggaaa 1320
   ctttttgact gtactgtatt taggagcctt tgtacagctt ggtcaaattt ccatgatatg 1380
   aagtatttga gttttaaaat atactgttat taaaaggaaa aagacatggc cattattcca 1440
   tgtgcttaaa tgataatttc cttattcagt ttcagaagaa aaagaatgaa attgggtaac 1500
   tgtcattgcg ttagctttat gttgaattgg gaaattgtgg cataaagctt aaattcgtgt 1560
   ttatcaaatg tgaaccatag tagtataatg ctgctttgta tataatgtaa gtgctacaaa 1620
   tagtctcagc actgaaaatg tattgatacc tcttaaatga atgcaacttt tgatgtaggt 1680
   gttttgctat gcctcagaaa atatctgtct gagaatttgt taatctgttt gataatgaag 1740
   atacttcctg ttttcttgtt tcatattttc atgttcaaaa tttaagtttt acatttttac 1800
   tactgttaat ttaaataaaa tttgttctgt ggataaaatg aggttggcag tgaagaaaat 1860
   taaaaacagc ctcattcatg taactggtta agtaaaaata cattttcact atgtgttcat 1920
   aaacttttaa tgaagctgtt tgtctttcag ttcaaatata agtgatgttt aggctttatt 1980
   tctgttaata aggcttttta ccattgatta aatgaaggaa tgtatctttt tgaagagatt 2040
   tatattctgt aaataaaaat tcgttgtaac aataaagttg agttctaact acaaaaaaaa 2100
   aaaaaaaagg ccacatgtgc tcgagctgca g                                2131
<210> 196
   <211> 775
   <212> DNA
   <213> Homo sapiens
<220>
   <221> misc_feature
   <222> (1)...(775)
   <223> n = a, t, c or g
<220>
   <223> nbla-10398-f
<400> 196
   ncctnggcct gttggcctac tggcattagc tcagagaact atctaattat aggtggacga 60
   gatcagcaac agaatgaaat acttgtgaaa agatacttga caccaggaga acccatcccc 120
   ccacggacct tgactgaagc tggcacaatg gcactttgct acagtgctgc ttgggatgca 180
   cgagttatca ctagtgcttg gtgggtgtac catcatcagg tatctaaaac agcgccaact 240
   ggagaatatt tgacaacagg aagcttcatg ataagaggaa aaaagaattt tcttcctccc 300
   tcatatctaa tgatggggtt tagcttcctt tttaaggtag atgagtcttg tgtttggaga 360
   catcagggtg aacgaaaagt cagagtacag gatgaagaca tggagacact ggcaagttgt 420
   acaagtgaac tcatatcaga agaaatggaa caattagatg gaggtgacac gagcagtgat 480
   gaggataaag aagaacatga aactcctgtg gaagtagaac tcatgactca ngttgccaag 540
   angatatcac tcttcagagt ggcagagatg aactaaatga ggactcattc aggaagaaag 600
   ctctgaagac gaaggagaat atgaagangg tagaaaagat caggattctg gtgggngaaa 660
   tgaaggntga anggggaaga gaccttaaat attctgntct nccatggcct ggnttacctt 720
   aancccnaag gtcattcnna aattggnttn aaaanaggaa cntttaattn nnggn      775
<210> 197
   <211> 791
   <212> DNA
   <213> Homo sapiens
<220>
   <221> misc_feature
   <222> (1)...(197)
   <223> n = a, t, c or g
<220>
   <223> nbla-10398-r1
<400> 197
   gngnnnnntt tgtggccttt ttttttttnn ttttggtgaa tatagcaagg caatgtttag 60
   ttcattttta taatgcggaa atcctgatac atggtgcttt catctttgaa cttccaaaag 120
   gctataaaat tggctcttct caaatatttt agaatttcat ttcagctatt tcctttttac 180
   gttcagaaga ttgggtgcag acactttcac tttgccagga atgtttcttg ataaatctgt 240
   gtcctttacg ctgcggaata agtctttttc tcttgctgtt gcttctttgg aatgcatgaa 300
   actattcaag gctgtttttg cagcttttcc ctttttctgc actccaggag taagtttcac 360
   tttatatttg tagtttgtca tggtggtgta aggggcacat attggaatgg caaacagtag 420
   tgcatcttca ggatgtggct ggcctgtcaa agaatcaaat aggttttcct catttccctg 480
   ntgatccana tcttgctctt ccttgcatca tgnggatcat ctacagcaaa gtcttgnaac 540
   tcatgagtta tacctcaagg aacggagntt ctttcttaat ggtgcagaaa ccctttgtcc 600
   acctntangt tctggggctg gtctttacan ggtcngccct tggttttcct tcttccctct 660
   tcctttttct tcttggttga accctgnaga acccnanaac ttnatganaa gtcacgggct 720
   tnttaatccn ggccttggaa ttnncnttna atttttcaat ttaanccttt ggccncgntn 780
   natngnnggc n                                                     791
<210> 198
   <211> 802
   <212> DNA
   <213> Homo sapiens
<220>
   <221> misc_feature
<222> (1)...(802)
   <223> n = a, t, c or g
<220>
   <223> nbla-10399-f
<400> 198
   gnngnnnnnt tnnnnnnnnn ntttganttc ctcnagcact gttggcctac tggaaatctg 60
   taaaagacct ccagcgctgc acagtgtctc ttgcacggta tcgagttgta gttaaagaag 120
   agatggatgc ctccattaag aaaatgaaac aagcctttgc tgaattggag agctgtttaa 180
   tggatcgaga agtggcgttg cttgctgaaa tggacaaagt gaaagctgaa gcaatggaaa 240
   ttttgctcag ccgacaaaag aaggctgaac ttctaaagaa gatgactcat gtggctgttc 300
   aaatgtcaga gcagcaattg gttgagctca gagctgatat caagcacttt gttagtgaac 360
   gtaaatatga tgaggatctg ggacgagtag cccggttcac ctgtgatgta gagaccctaa 420
   agaagagcat tgattcattt ggacaagtgt ctcatccaaa gaacagctat tcgaccanat 480
   cccgatgtag ctcagttaca tctgtgtcct tgagtagccc aagtgatgcc tctgctgctt 540
   cctctttcac ctgtgcctnt nctccacctt acaagtgcta acaaggaaaa actttgcacc 600
   gggagagact tctgcagcca tagcaaactt caatgggcaa gcctaccagn cctttnggan 660
   gnantggcag ggaacagacn angaggacag gctataggnc acaaggccaa agtccaatga 720
   cccattaacc aagggcggat tacantttng gncntacnna acannttngg ggattnaant 780
   ggttcaagnt taaaangctc cn                                         802
<210> 199
   <211> 811
   <212> DNA
   <213> Homo sapiens
<220>
   <221> misc_feature
   <222> (1)...(889)
   <223> n = a, t, c or g
<220>
   <223> nbla-10399-r1
<400> 199
   gnngngtnna ttgtggccct ttttaatttc ctttgggcat acttatgcag ttgtttttat 60
   ttattcaact gtaaagactg cagcaatttt tttgatgagt aatgccttag attaacacta 120
   attatattgc tataaatttg gttcaggatc agaatagggt tcaggaaagg gagagaacaa 180
   aaaaaaagtc tccaaacatt aaaacagaga aatgcttttt tttttttttt gagactgagt 240
   cttgctctgt ctcccaggct ggagtgcagt ggcacaatct cagctcactg cagcccccgc 300
   ctcctgggtt caagcgattc tcctgcctca gcctcctgag tagctgagat tacaggcaca 360
   cgccaccatg ctcagctaat ttttgtattt ttagtacaga cagggtttca ccatgctggc 420
   caggctggtc ttgaactcct gacctagtga tccacccgcc tcagcctccc aaagtgctgg 480
   gattacaggc atgagccaca acgcccaacc tttttttttt taaactttaa ttaaatgcta 540
   ctttctcata gacttcccta nactgggttg caatctaagg gaatanggaa acaaggcatt 600
   gggcattttg aaaaaactgn agggntaagc tatggggccc agccnaanac ttaaccctgg 660
   tttgagggca aatnttcagn ncaatttggg gannaaggca gggtcttaaa aaggttccta 720
   agggncnttc ctttnactaa ataaaanttc ccttttttgg ggaanccngg ccnnnttnca 780
   accccctgga ttggcctttt tgggnnaaaa n                                811
<210> 200
   <211> 1941
   <212> DNA
   <213> Homo sapiens
<220>
   <221> misc_feature
   <222> (1)...(1941)
   <223> n = a, t, c or g
<220>
   <223> nbla-10454
<400> 200
   ctgtacggaa gtgttacttc tgctctaaaa gctgcggaat tcctcgagca ctgtggcctt 60
   tttttttttt ttttttcaaa cacttcttgt tctgatacgt tcaaacatct tggtgtttag 120
   gttggggaag ctgagttgtt gtttatgagt tatctccagt gctcagatat ccggcagctt 180
   tttcctagga agtcagcttt gcacatgaga gaaaggaaaa aaaaaaaaaa aaaaaaaaaa 240
   aaaccaaact gctggaggag aggtggggga aggttgagaa agttggagaa gggggcaaag 300
   atcagaaaga ataaagcctc gatatccagg attgtcaaag attaaagtca tgtggacatt 360
   tccatcccta gaaagttttc cttctttggc cgggagcggt ggctcatgcc tgtaatccca 420
   gcactctgcc cgactgcctt tatgaaatat ggtaatttaa tgcttaaaca actgtttcat 480
   ttgtgtactt tttgggagaa agtactacgt aaaaccagtg cttaatgatt ttgacaatta 540
   aattatatgt gattattatc ttcctatagt gttttttttt ttagaagagc tactcagata 600
   tagcagatta atatcactca ggcacctggg tttcctagcc cagcaattct gaatgtacag 660
   agtacaagca tataatgcca actggtaaag tcagagctcc agatgacaag gtacagcatg 720
   taatcttttg cagtctattg acagcaatct aaataaaagc aaaggaagaa gcttccagta 780
   ctacaaactt tatgggaact tagggtctct caaagccaca ctgtttgggt cactagcagg 840
   aggtacagta tattaaagca agcagctcag cctctcacta gtggtgagat gtatgaaaat 900
   cctggcaatg ccagggggaa aagaatctat gagtcttccc ttcggtcatc tctcctattt 960
   cttgcattag ttttttcagg gttaaatacg catctcacag aagagctagc tatatatggg 1020
   ctgggtggtc ttcagtgaat ctgcaaatgt atcaaacaaa aatagcaagc tggaactgga 1080
   ataaagggtc agggaataca ggaaagaaga aaacaaaggg ttctaggtgt tcagaagtca 1140
   aatacatctc agggctaaaa aaaaaaagaa agaaagaaag aaagaaagaa aaagaaatct 1200
   gagtgtgtcc tgtgttctac ctccctgtag cttatgaatt ctatagaact caaaacatca 1260
   aaccaccaaa gacagatgat taagaaagaa aagacagatg aaaatgtgac agtcattgaa 1320
   ctctaaaatt caaagcaatt attactcaga gttctagaga tttcctatga aaggtgagtg 1380
   gagataaaat tgcataaaat ggaattacga tcctcctctg caatctgacc agacagagga 1440
   agaagaccca agaaaatgtc aaataaactt ttgctcacca atcagcccat gccttgagac 1500
   ttttaatccc tctttttcct tctttacttt tattagaaaa aaaacagaaa aaacaaaaaa 1560
   aacaactata atggtaccaa gacacatttt aacaagaagg tttttgtttg ttgttttttt 1620
   ttcctctctc tctctctctc tttttttttt ttttttacta aggccaggtt tttgagtgat 1680
   tcaaaacatt cttttgtggt gtgatcatat tcattctgca ctaacacctc tagccttttt 1740
   tttttttttt tttcaaacac ttcttgttct gatacgttca aacatcttgg tgtttaggtt 1800
   ggggaagctg agttgttgtt tatgagttat ctccagtgct cagatatccg gcagcttttt 1860
   cctaggaagt cagctttgca catgagagaa aggaaaaaaa aaaaaaaaaa aaaaaaaggc 1920
   cacatgtgct cgagctgcga g                                          1941
<210> 201
   <211> 794
   <212> DNA
   <213> Homo sapiens
<220>
   <221> misc_feature
   <222> (1)...(889)
   <223> n = a, t, c or g
<220>
   <223> nbla-10456-f
<400> 201
   ggnnnnntgg ggnncnnnnn ttngaattcc tcgagcactg ttggcctact gggtcatgta 60
   gaacattatc cacgtggctg tggctgtggc tgtggctggg ccctggcagg tggaaaacca 120
   tctcccagaa acctgaaagc acctgccaat gacgcagata accctggccc tacagcctgc 180
   ttgctccgcc tataccacag agcacagcct ggacattatg gagggtgtgg cgggacggcc 240
   cacacctggg tcctccatcg ggaacttttc atgcttcttt ctccacctga ggtcttggtc 300
   tgaagaagac ctcaggactc acatcttcac tcctgggcct ttgcacttcc agacgacagg 360
   tcatcgttca agcagaatgc agacaggcca ttcacgagcc caagttgaag agaagagacg 420
   cccatccgtg aaggagcaga ccatccatcc gatcctcccc ttccctgtcc ttcttcgtgg 480
   attgtctcca ttgtccagac agtgccccca cctnccaccg ncttgcctac tggcaatctg 540
   gactcgatgg agaacatncc cccaccttca tttggcacta cccaagtgga gtgtaccctt 600
   gcctttcacc tgtaccaccc actncaacct tccccaactt gccaatgctt ctggggaatt 660
   taatagctac catgcaggcc acaaggaatt tgtgangctt ctttggcatc tttggatctn 720
   caagttggct tctttnttca tanccctgct ttactttcct tccttggaat naggggtccn 780
   ttanccattt gcnt                                             794
<210> 202
   <211> 807
   <212> DNA
   <213> Homo sapiens
<220>
   <221> misc_feature
   <222> (1)...(807)
   <223> n = a, t, c or g
<220>
   <223> nbla-10456-r1
<400> 202
   gaggngtnnn ttggtggcct tttttttttt tttttgccaa gataaatcac ttttatctct 60
   ataggaaagg gaggatctaa aaaaaatata aattacatta gtaacacaac ataagaaaaa 120
   gacagggaca aaaacaacag agaagtctga atgatgctac cctaacctat ttataaaaag 180
   gccctgcatc agaaattcac aatcctaccc acttctaaaa atatatttag acatgtacag 240
   aagcggtggg cttgttttta aattgtttgc tttttttgta aaaatatatt aaaggtgaat 300
   agaaatcctc tctcccttcc ccctgtccag cccccagcta gggactggag atcaggggta 360
   actatctcat ggtgttctaa accttgatta ctaacactcc caacccctcc ccaactcact 420
   tcacttagaa cctgaaacat taaaaaaaac aaaaacaaag acatcagcct ctttggaaaa 480
   gcataactct gagggtaaac ttgcttttca ttggaaacaa cacaaaacaa caaacagggg 540
   gctctaagaa cccaactaag ctaaatgaaa cccagtcaaa cagaggctgg gtttcaagct 600
   ccacagaaaa cttctaacaa agaaatagag gggaaaaggc cggaatcggg gggatttgct 660
   agcaattagc tgnctttgaa agatcaagaa ggggtgttaa tggacaaaaa ggctggggac 720
   ctgacttggt tcagcagtca ccgcatagtc aggcagttgg ctatacagga canagccttt 780
   tctggtntnc cttantaccc ttggtgg                                807
<210> 203
   <211> 2180
   <212> DNA
   <213> Homo sapiens
<220>
   <221> misc_feature
   <222> (1)...(2180)
   <223> n = a, t, c or g
<220>
   <223> nbla-10457
<400> 203
   gtacggaagt gttacttctg ctctaaaagc tgcggaattc ctcgagcact gttggcctac 60
   tggctgagca tcagactaat tctgaagcat atttgctaga ggagctactt tgcttttcac 120
   aatggggtgg agaggattct ttcacttgtc ccattaaccc tcttctagtc tagatgagat 180
   gaaatctgtt aatgtgtgtg tagaagaaaa cgtatgttct tctactcagc attgcccttt 240
   tccacctcct cacttcacct ccgagtagct tgtttatcaa gaatgaatga atgtctttgt 300
   cttaaatttt gcccatgtgt taaaagatgt aattctcaga atgggagaga aatgactacc 360
   tttgttccta ctcttttata taattatcct tttagggaaa gacttggtca actctaatat 420
   atctagaagg aagactatat ctggtgtaga ctaatatgag atgttttaga agagttaacc 480
   tgaacacttt gagggagaga ttattcttgc cagcaaaaag ctagccagga atgagcctac 540
   cacattattt gagaatatca aacctcaggc ctggggggat gaggggaaga agattaccag 600
   aagtgcagga aagagaagtt tgaggaacac ccttggctta gcaacatgtg ataatgcaaa 660
   gctgttataa cctgttaatc ctacgtacta tgtgttctgt acctttacat gtttttaaat 720
   ttaagatagt ttgtaagaac tgtacaaaaa aatgcttctg gagatttctt tggcagaaat 780
   gcctttcatc tataatttca tggagaactg ctttaattag cctaggtgaa aagtagtcct 840
   agcagtgtaa atatgtataa ttagagtttt ctaatttcac tgtgagatct ctaacttttg 900
   agtggcaaac agatcaagtc ttttgctcat agacttttct gtggggttat taaaatgcaa 960
   aagctttatt ttttttaata atgccatact ccattagtgt cagatgatgg tatggaattt 1020
   gttcccttgc tttcccccac tgttactgct tcagtttata gattgccagc agagttcaga 1080
   aatagagcag ggatttaccc gttctttgct tggacatccc attttctttt gtccagaccc 1140
   atgttggcaa tcatgtatga actgtgttat acttctcagt gctttctttt ttctttttga 1200
   taagatggat atcaaaaata gttgctgtgc aaaagttagt agtcttcttc aagaagaaaa 1260
   tcaattcttt ttctaataat atcctgtgaa attgcttcat tcattcattt atttttaagc 1320
   caaatgtcag cagagtgctg ctgcttttat ctagtaattt tgatatgtaa gtattaatgc 1380
   atttttaaaa gatgtctaca ttgaaacatg ttcttcccag tgtcctgctt atgatgcttt 1440
   gttcagattt tttgtaagag accagttagt acactggggg tgtatattgt gtacatgtgt 1500
   cattttagtt aggcattgta ggccaaatgt gattataaat gaagttgatg aacattaatt 1560
   ttgttattag tgagtttttt gaattgtaaa tggatttcca gtttaccttc tgttgtctac 1620
   agctttttta attttaaggt ttgactaatt gtatccatct cattgtacag tgttttagtt 1680
   gcaagcagaa agtagaattt ggtataaagc aggttatttc tatattgaaa ggagtacagt 1740
   tgaaattgta gatttaagat tgttaaaatc atgacaattc taacttgtct attctaacct 1800
   attgtgtaca atctgatttt ttaaaattgt aaacatgtat gatcttggtt tcatgtgttt 1860
   ttgaaagtgt tattgtttaa aaaatgaaaa aagcatatct gctaaagagc tgtcagtttt 1920
   cattactgac tctgtaaaat acactgttct ttgtgtactg tgtgttattt tgccagctgc 1980
   tgcattagcc ttcaaaagta tttggaaact taagatgaac tacatttctt gcaaagtaca 2040
   ttcctttctg tggtattttg tcctgtaact gaagtatagt aattatttta tggaaatgtt 2100
   agcaattctg taccaacttt gaataaaatg aaaaatttat aaaaaaaaaa aaaaaaaggc 2160
   cacatgtgct cgagctgcag                                            2180

<210> 204
   <211> 805
   <212> DNA
   <213> Homo sapiens
<220>
   <221> misc_feature
   <222> (1)...(805)
   <223> n = a, t, c or g
<220>
   <223> nbla-10473-f
<400> 204
   nnngnngggn nngttgggnn nnnnntttga nnncctttgc ctgttggcct actggttttt 60
   attaatctac tgtatcaata aaattctgta atttgaatga gtttttaata gtctagaatg 120
   ttattgtgta tagatatttc ctcttgaacg ttatgttcag aaaatgcaaa ttacactata 180
   atataaaacc tgatatatac acattagaaa tactccagtt ctccgtaaca gtgtaaagtt 240
   aatcagaaag aaaaaatttt attgatgcag tgtgtcttta taaagctgtt cttgaaagcc 300
   aagtgttttg tattttatag tgagttgcat gtttttgaaa aaaagtgctg aaaatgggat 360
   gtgctgtttt ctgtaaattc atatttagcc atagtatatg atagattgcc ccataacata 420
   gtttttcatc aagagtttat tattgnactt tattttggag ccaaaaaatt gattctgggg 480
   ggtgggggca gcgtagaagt ggtatatcca agtatatcag ctactgnagt tgcacagctt 540
   gtgaactttg aatgaaattc cactttgcca gattgggaga agtggaaatt atttggattt 600
   agagcangnc tttttttttc ttcattgnaa tctgcaaaat gtangaaata aattacttaa 660
   ngcagtatcc tttatacagg tgnataaact ggattttgga cccaaacata tagggtacta 720
   cttaatgctt acctaatttc attttaagnn tttaagngct ggtaaggttt ttcatgggta 780
   caccngangg ggaaaaatca ttttt                                      805
<210> 205
   <211> 808
   <212> DNA
   <213> Homo sapiens
<220>
   <221> misc_feature
   <222> (1)...(808)
   <223> n = a, t, c or g
<220>
   <223> nbla-10473-r1
<400> 205
   gnagggngtt nnnttggtgg ccttttttnn nnnccttttt tttttttttt ttagaaagcc 60
   atttaacctt taatacctgg aataagtttt acagccacat ttgtaattta catatacagt 120
   gaagatcact gaaaaagcct cctgttgcac tgcagcaaga aattttatta taatgtagat 180
   taaacaaatt tgctacagag tgcagcaaga taacctcact gaggaataag attttcataa 240
   tggaacttgt tacagggagt taaatatttt cttaatgtta tacagtacat attttcaaaa 300
   tgaacattat cactgaaata gtttgctaac aattgttagc aattaatgnt ttatacacac 360
   ttaattttac tgtaaagtat gtggctctcc aaatcttact acatgaaatg ttaagcttaa 420
   acgtaagata atcccctaag atggagactc aagacatcaa aactttgatc attattgcac 480
   ctatttttct acacaaaaaa aaaaaaaaga aagaaagaaa agaaaaattt acatcgtaat 540
   ccacccaaaa aaagaacaga caggcgtaaa attcagactc attggcattg aacataacaa 600
   aatctacaat gaacattttt ttgngaactg ctgtcagcat tacaaaaatg tgaaatgaaa 660
   tttgcttaac tataatcatg ccaataaaaa tccttnacaa tcactttttc gnatggttta 720
   taaaangtct taatatttcc ataaacantt ntggggcaat ttaatcaatg gccaaaatcc 780
   aaggttttnc ttaatcntta tggtatag                                   808
<210> 206
   <211> 802
   <212> DNA
   <213> Homo sapiens
<220>
   <221> misc_feature
   <222> (1)...(802)
   <223> n = a, t, c or g
<220>
   <223> nbla-10474-f
<400> 206
   gnnnnnnnng gnnnnnnnnt tggnttcctc ngcctgttgg cctactggcg cattatttta 60
   ttcactgaag atccaagggt atttaaagta acatatgaaa gtgagaaagc agagttagca 120
   gagcaagaaa ttgcagtggc attacaagat gttggaattt ctcttgtcaa caattacacg 180
   aagcaagaag tagcctatat aggcattaca agttctgatg tggtttggga aacaaagccc 240
   aagaagaagg caagatggaa gccaatgagt gtaaagcacg ctgagaagtt agagagagaa 300
   tttaaggaat atactgaatc ttctccttca gaagataagg ttattcagtt ggacactaat 360
   gttccggttc gcctaacccc tactggtcat aacatgaaaa ttctgcagcc gcatgtaata 420
   gctctacgaa gaaattatct tccagcatta aaagtggaat ataacacatc tgcacatcaa 480
   tcatcattta gaattcagat ttacagaata cagatccaaa atcagataca tggtgctgta 540
   tttccctttg tgttttatcc tgttaaacct tcaaagtcgg tcaccatgga ttcagcacca 600
   aagcccttta cagatgtcag tattggcatg agatctgcag gacattccag atatcacgta 660
   ttaagtattt caaagtattg attcaagaaa tggatctcan gttacatctt ggggttatct 720
   atgctttaac agaccttatg acagaactga ggtgactgaa atacngaggt gggccttttc 780
   ataaaggatn tngaancttt cn                                          802
<210> 207
   <211> 810
   <212> DNA
   <213> Homo sapiens
<220>
   <221> misc_feature
   <222> (1)...(810)
   <223> n = a, t, c or g
<220>
   <223> nbla-10475-f
<400> 207
   nnggngnnnn nttgnnnncc ntntttgnnn nccnttgcac tgttggccta ctggcttttc 60
   agtcgggcgc tgagtggttt ttcggatcat gtctggtggc tccgcggatt ataacagcag 120
   agaacatggc ggcccagagg gaatggaccc cgatggtgtc atcgagagca actggaatga 180
   gattgttgat aactttgatg atatgaattt aaaggagtct ctccttcgtg gcatctatgc 240
   ttacggtttt gagaagcctt ccgctattca gcagagagct attattcccg gtattaaagg 300
   gtgtgatgtg attgctcaag ctcagtcagg tactggcaag acagccacat ttgctatttc 360
   catcctgcaa cagttggaga ttgagttcaa ggagacccaa gcactagtat tggcccccac 420
   cagagaactg gctcaacaga tccaaaaggt aattctggca cttggagact atatgggagc 480
   cacttgtcat gcctgcattg gtggaacaaa tgttcgaaat gaaatgcaaa aactgcaggc 540
   tgaagcacca catattggtg ttggtacacc cgggagagtg tttgatatgt taaacagaag 600
   ataccttctt ccaaaatgga tcaaaatgtt tggtttggat gaagcanatg aaatgttgag 660
   ccgtggnttt aaggatcaaa tctatgagat tttncaaaaa ctaaacacaa gtattcaagg 720
   ttgggntgct ttctggcaca atgcccgact gatgtgtttg gaagtgccca aaaatcatga 780
   ganatccaat tcgaatctgg ngaaaaaagg                                  810
<210> 208
   <211> 756
   <212> DNA
   <213> Homo sapiens
<220>
   <221> misc_feature
   <222> (1)...(756)
   <223> n = a, t, c or g
<220>
   <223> nbla-10484-f
<400> 208
   gnngggnnnn nngntgggnt cnnnttgaan nccttggcac tgttggccag gctggtcttg 60
   aactgctgat ctcaggtgaa ccacccacct cggcctccct aagtactggg tttacagtca 120
   tttttttaaa aaataaatct tgntctttgg aagaccatta agaatagtca taaaatatcc 180
   ttacaaatag aactgctcaa aaaagaaact caaaaactaa atttgagttc agtggcttct 240
   gagggtatac cacgagtgaa gtctcttatg ttctcaacaa gggagcgaac ggcaaagccg 300
   cacacctgag ggctcagtgc tctcaggatt catggcctgg cttcctgcta agtggtagca 360
   ggtccctctc tttcatttgg actgagtgtc caaagtgcaa gtggattagg agccagggag 420
   atcctccatt tttggtgttt tggtttaacc aatagtccta ggagcttagc acacctgggg 480
   tgtcaccttt tgcttgtgtt tgtgaacctc tcttatcagg gacaagaacc atgtgtccat 540
   ctagctgcag gcctctaggg tggggacagt atccatcaag acagggattc ccatggtgca 600
   aatgatggct cttctncttc gacacatctg tgctgnactc aagccatttg tcaaggntag 660
   cagagcctgg gggaagcant gtggaaagag ctgcttcgga gatagcttgg gcccaagtct 720
   gaatcctggc tntgntgcac tttccanncc aatgac                           756
<210> 209
   <211> 751
   <212> DNA
   <213> Homo sapiens
<220>
   <221> misc_feature
   <222> (1)...(751)
   <223> n = a, t, c or g
<220>
   <223> nbla-10484-r1
<400> 209
   gnagnggggt nngtttgggn ctttttgann ccntttttag acaacagaaa tttatttctc 60
   acagttctga aggctggaaa gtgccagatc aggtggccag cagatctggt gtttgatgag 120
   ggctctctgc ttcctaaata atgttttctt gctgagtctt cacatgggag agggaggcca 180
   ggcaactccc tccaacctct cctataaggc tctaatctca ttcatgaggg ttccaccctc 240
   attccctaag caatttaatg ccttcccaaa ggccccacct cttactacga tcgctttggg 300
   tattgggttc tgacacatac attacctcat ttactttcaa aatagtagtg tgggcatttt 360
   taaaaactat aaaatatttc atgcataaaa aacacacact atatatatta acagttaaag 420
   aataaaatgt ctgcttccac cacttagaaa gagatggtta caaacacctn tgcaggctcg 480
   cgtctgttcc caaacacatc ctttccacac tcatcccaga caaagttact gccttaaatt 540
   tgagattatc aggactcact gntaggagct ggagacaagc agcgggcttc ccactcatgg 600
   aagaggctga aacaaaacaa aaacaggtat ggatccctga caactacnga gtggaggctn 660
   acagcaaact gnaggcccan ggtgggggca ccgtaccatg ggcctntgaa aagccagtca 720
   ccctggcttn tggacctgaa tctgcaagng g 751
<210> 210
   <211> 2038
   <212> DNA
   <213> Homo sapiens
<220>
   <221> misc_feature
   <222> (1)...(2038)
   <223> n = a, t, c or g
<220>
   <223> nbla-10485
<400> 210
   gtacggaagt gttacttctg ctctaaaagc tgcggaattc ctcgagcact gttggcctac 60
   tggaaagatg cttgaaagat aaatacgttt aattaggggc agaggatgat cattaaagga 120
   agtctgactg ctgcagaagt cattaacaat cttaaatgaa atttttattt ttatgatagc 180
   catttacatg tataataaga gccaatgatt cttgggagca gtgtgacaga aacagagtgt 240
   ggcgagaact catgtaagac atactattta aatgagctgg tgttagctat tcatatttgt 300
   taatgaacta gatatgcagg gctataagaa tgaatgttct gatgctttaa attttaatat 360
   ctagaaatca agggtaagga ccaattacta gaatttccac ccttagaaga ataattaata 420
   acaatgtaac aaatgtacag ccccaaacct cttttaaaaa agtttaattt aaaaaattta 480
   atatcaatag ttgaaaaaca gccaaacagc aatgtcaagc tgtagtagtt tgtgattgtg 540
   gcagcttttt gtgttggtca tggatcataa ttctaaacgt tttaatatac agtaatccat 600
   atgaatgcat tgcagaccct gttatcacct gtagttaatg gtgcctgtga tacaagtgaa 660
   caggctgttc tttgctctta cctttatggt aaataaacca gcattctgga gtcacaataa 720
   ttaaataaaa tgtaaatccc taatctcatt tggctatttg gaccaagaaa cttacccggc 780
   tattacctgt ctagttctaa tgattactaa tgatttattg ttcttttttt ttattgttct 840
   aaaagtgctc tgatgtggtg ccttgatgtc taatgaaaag aaattggtct taaaaagcta 900
   ctcattgttt atcctgactc tgagataaca catttttctt ctgataaaat atttttctta 960
   tttattgttc aaaacacaca atatagacaa taactctaat tctggtgcag ttttatttag 1020
   tagtgtttta tccccggtgc ctggctgtct acagtcatac ctcagcacat agctttactc 1080
   tgagataagc cctctctcct ttgctctttc tcccatgctt tctggagttt gtctgcagga 1140
   tgcaggtttt gaactgaaga gcgcagtgga gttcttactg tgcctttccg gttccagcac 1200
   aatcaaagtt ttgtgtttgg aattgtcctt gacattcttt cgtcccacaa tggggtggac 1260
   acagtgtgct ggaaaccatg acatcattat cattgacaca gacttaaata aatcacctgc 1320
   atcttggcct ctttctgctt attcaaggat tcttcatctt aaggaagaaa gtgctcttta 1380
   tttttacaaa tcttcatccc aggggtgact ggaagacgca aacttttaca agtgttttaa 1440
   aaataaatat actatgtggt tttagacaca gtatgctgag tcagaggtgt cttagagaat 1500
   gtttaaggtt agacataagg cagaaagaca gccagattgc ctttaattcg gaggcaatat 1560
   tcctaaactg tgtatcatta tgtccttctg aatatggatt cacttgaaac ttaattaaat 1620
   aggcatatac ataacatttc aaatacatac ctgaaaaggc ttagtagtgt ttcattttca 1680
   cacattgagc ttgatattca aaaaagaaaa agattgatgc tcagtgaatc aaagcaaagt 1740
   actttctagg taaaagtgac accatctcat ccaatgtctc tgaaagatct cagttgcaca 1800
   gtctttggat aaaaagggtt ataactaaat aatactacaa aatgcaattt acacaatgtg 1860
   ctgactacac atttgtatac ccagcattta ttaatttggg aagcaaccaa ctactgtaac 1920
   ttctctttac tttttcataa gaaaatgaca atgaggaagg aagtgcagca gaaatcttga 1980
   acattaccac tattatgcaa aaaaaaaaaa aaaaaggcca catgtgctcg agctgcag 2038
<210> 211
   <211> 1690
   <212> DNA
   <213> Homo sapiens
<220>
   <221> misc_feature
   <222> (1)...(1690)
   <223> n = a, t, c or g
<220>
   <223> nbla-10500
<400> 211
   gtacggaagt gttacttctg ctctaaaagc tgcggaattc ctcgagcact gttggcctac 60
   tggagtgttt tttttttgtt gtttttttaa acatttcatg ggtttgtaga tttttggaaa 120
   taatttcagt taatctcagg gcttcccaaa ccttgaaatg gttaaacacc aaccactgct 180
   taacttggtt gttgtaaaca aagcccgaaa tgaaacccct gtgtatttgc tgcagattcc 240
   caggtgcctc ttaagtgtga ggataagctc agaaaaggct taataggttt attaagttgt 300
   catccattaa aaggttcaca tgctccttgt gtgttgcata aggacaggtg tctgccacct 360
   cattgcattt ctcactgagc attgcctagg agagggtaag ccctaggatt tagatccctc 420
   tcagatgttc aggcagaata attttgtcag gacaaaagta aggtcacgga actatatgaa 480
   aatggattat gaatcctaag acatcagcct attctcccct atgtctaaga agaatattcc 540
   tcagcttatg actggaatgt cttgctaggg gtgctgggta agttttgtgg aggtccccac 600
   cagcagaaaa aaatcattga agaggaacac ttaattcaca atggctgctg attaaaaaga 660
   aagcctcaat attcacctat aaccctaaca tccttaacag cttagcagtg tggtaactgg 720
   cacaaactat ggcgtcttgg tcaaatccaa cctcagtgaa ggtagttcac atcctagaga 780
   tgagctgaat gctgatagct ttgaggactt gtttatttgg gggaagggaa agagatggtg 840
   agggtagaga taggaatctg tctgtcgtaa taggatttgg tctgtgtatt aatggaatga 900
   actggccttt tttgctctgc ttcttttgtc cttgctgaga agggaggctg aagtttccaa 960
   gagttatgga tgaagttgga gagtattaga ggaactgttc tctgaccttc ttgatggtaa 1020
   tgcaagctgt tgggcgagtt tggagaagag cgcagggtgg tgtgaggacg agggccgtat 1080
   taacattttg ccttaaaaca ggaatacaca aattaaagca ataactgcta ctctgctttc 1140
   ttgtttctgc aatcttctgt catttgacat tgttttttcc aaagcaacat gcgaccattt 1200
   tcttcaggat tttcttttca gttatcatat gggtttgttg ttggattgag aaaaaaaaat 1260
   ctgcctcatt tgagcaggcc tctctagatc ttttcttatg actgtgtaga gggtacttcc 1320
   tcatttttct tcctctcaaa gacatcaaat tcccgagcat ttaactaaaa ttattatcaa 1380
   ctaaaaatgt tttttttaaa acagttctta gaggtaactg tttagactta attattgaaa 1440
   tgcatgaagg ccttctgcct tgcccccatc ccactctttt gcctgcctct ctcaccaaca 1500
   aaaacatggc agaggaacag tttattaaca atagcctgtt tgaaagggaa gcttcagttt 1560
   ttagctaatt atttgctaat aaaagtttta aaaaaatttt cttttaaaag ttgtaaaacc 1620
   tctgtctata tttcaaaacc cctcaatacc aaaaaaaaaa aaaaaaaggc cacatgtgct 1680
   cgagctgcag 1690
<210> 212
   <211> 1965
   <212> DNA
   <213> Homo sapiens
<220>
   <221> misc_feature
   <222> (1)...(1965)
   <223> n = a, t, c or g
<220>
   <223> nbla-10512
<400> 212
   gtacggaagt gttacttctg ctctaaaagc tgcggaattc ctcgagcact gttggcctac 60
   tgggagagaa ttcatacagg taagaaaccc tatgaatgta aggagtgtgg gaaagctttt 120
   agtgtatgcg gacaacttac ccgtcatcag aaaattcata ctggtgtaaa accctacgaa 180
   tgtaaggaat gtggaaagac ctttagactt agtttttacc ttactgaaca cagaagaact 240
   catgcaggta agaaacctta tgaatgtaag gagtgtggga aatcatttaa tgtgcgtgga 300
   cagcttaatc ggcataaaac aatccatact ggtataaaac cttttgcatg taaggtgtgt 360
   gagaaggctt ttagttatag tggtgacctc agagtacatt ctagaattca tactggagag 420
   aaaccatatg aatgtaagga atgcgggaaa gcctttatgc ttcgttcagt ccttactgaa 480
   catcagagac ttcatactgg tgtgaagccc tacgaatgta aggaatgtgg gaagaccttt 540
   cgagttcgtt ctcaaattag tctacataag aaaattcata ctgatgtgaa gccctacaaa 600
   tgtgtacgat gtgggaagac ctttagattt ggtttctacc ttactgaaca ccagagaatt 660
   cacactggtg aaaagcccta taaatgtaaa gaatgtggaa aggcctttat tcgtagaggg 720
   aatcttaaag aacatctgaa aattcattct ggtttaaaac cctatgactg taaagaatgt 780
   gggaagtcct ttagtcggcg tgggcagttc actgaacatc agaaaattca tacgggtgta 840
   aaaccataca aatgtaaaga atgtgggaag gcctttagtc gtagtgtaga ccttagaata 900
   catcaaagaa ttcatactgg tgagaaaccc tatgagtgta aacaatgtgg gaaggccttt 960
   agacttaatt cacaccttac tgaacatcag agaattcaca ctggtgagaa accctatgag 1020
   tgtaaggtat gtagaaaggc ctttagacaa tattcacatc tttatcaaca tcagaaaact 1080
   cataatgtaa tttaatataa gaaaaggttt ccatgtcatg ctctatttat agaatatcaa 1140
   aatatttatg gccagaagtt ctgtcaatgt gttgatgttt ttttacacat attaacttaa 1200
   taaatgtatg agtcttaaat acctcttagt tctcattaaa tttaggaaaa ttcacactag 1260
   aaaataaagc tgttaatgta acagttgtgg agaagtgttc tagcaacagc atatacttat 1320
   catcattgcc tttccactac tctactatct gtgtgatatt agacaaaata tttgcttctt 1380
   ggtacctcag ctgtaaaatg aaacacacct aaaagtgtgg ttgtttccaa catgtataat 1440
   acagcaacaa ctatctggcc caaactgctt tggattaata ttggatatta ctgtttttat 1500
   tatcatcaac attattatta gtggatttct taataggaag atgcaatgga gatgacaaat 1560
   ttggaaaaac cactcatcac ttacatttca tgaagtactt ctttgataaa atctgttatg 1620
   ggctgaatgt ttgtgttccc gtaacaattc ctatgttgaa acacgaatcc caaggtgatg 1680
   gtatttgaag gtagggcctt taggaggaaa ttaggtcatg agggtggagc cttcatgagt 1740
   ggaattactg cctttataag aagaagccaa agagccagct agctctttca accacatgag 1800
   gttacagcaa gaagtcagca gtctacagtg caaaagaggg ccttcaccag aacccaagca 1860
   tgctggcacc ttgaccttgg actttcagcc tccaaaactg tgagaaataa acctcagttg 1920
   tttataaaaa aaaaaaaaaa aaggccacat gtgctcgagc tgcag                1965
<210> 213
   <211> 1980
   <212> DNA
   <213> Homo sapiens
<220>
   <221> misc_feature
   <222> (1)...(1980)
   <223> n = a, t, c or g
<220>
   <223> nbla-10520
<400> 213
   gtacggaagt gttacttctg ctctaaaagc tgcggaattc ctcgagcact gttggcctac 60
   tggcaaagca ggttattcag tgattcctcc ccaccattct taagaacgtt aaataatgct 120
   gttgtgttag ctctgagtag aaaggaaaaa gtaaaacctc tgtttggagg taatattggg 180
   ttgaattctg actgcccctt tctagctgga cctttaacaa atcacccaat cttttttgtg 240
   tttctctaaa gtcatttata cattaaatgt aattatagca actgtggggt tctgttgaga 300
   attaagagct aacactatat atgtaaagtt tccagtacta gtcccagaat ttagaatatg 360
   ctcaacacaa agtaaacagc attatataag tttatatttt tgtgagttat aaagtacttt 420
   gatatattct cattaaatct gtaaatcacc tctataagta agtggtaata ataaagcaga 480
   tatttttgtc cccatttaaa aaatgaagaa attaatgctt aatagggtgg taccctggaa 540
   aggatctggg aagtggtaga atttctggtc tgtactttta caaatggagc ccttgggagg 600
   tgggttaggt aaaagaagct ttttacttaa cgttgtctta tttccagtct aattttacgc 660
   tgtagcagaa ccagatggct gagaaaattc tggaactatg gatcttgacc ccaaggatat 720
   attattttat tccaagaaag atcaggtagg cgaaaagatg acaggataca gagtcaatcc 780
   ataaactaaa tatttataac tgttctgaat tatacagagt ctaaaaatat gtgtcagcta 840
   cttcattcct gtaaatactc ttgctgtgtt ataaatatgg caagaaataa acatgaccaa 900
   tatcaataga cttcttgagg ctactataag ttttgagaaa taaggttcaa aaaataagaa 960
   tgctaacact taagcacaga ctagagcttg cttgggtttc ttcctgcatt acaaggtaaa 1020
   aatttgttaa tgtttgtttt tattcagctt gggaaagctt tgtgccatga atacgtcgca 1080
   tttaataaca agcaacacac ggcatataga aataacttta attaaaaaac ttacatagaa 1140
   gattataata tcagacgtga caaagatttg agtttatttg cctggacaac ttgggtttgt 1200
   ctggcttttg ttttcttttt ctttaaaaat aaatgtacag taaaactaca agcaaaagtt 1260
   tgtcagtatt gaattgaatt ttttacccct taaaaggact agtataattt ccaatctcta 1320
   acaaaaactt agtgtcaaat ctcacagata aggccaaatg gccaatattt tcagttatgt 1380
   gggtagtaca acttgagtaa ccttttttac atgacaaaaa gtgagttata taaattgtcc 1440
   tcaactttca cataggaaaa aaatggttta atagcttcaa aaggaatttt ctttcatgta 1500
   tactcttcag tatccaatat tgaagctttg ttctttgaaa aattttaatt tccaatctag 1560
   gatgcaagca agaatatatg tttatttgaa tagagtaagc tatggcaaag aatgaccaaa 1620
   ttagctagaa atagaaatca gccagaatta actaatttct tgctaatcta gaaatacaat 1680
   catctttttt ttttttcaaa ttttatactg atagggcttt actgtttgtg gctcatttta 1740
   aaactggtgt cttctcttca tgagacacat taattggtaa aactcaaatt gagttttcaa 1800
   agatgtgata gtattaaagt gcaccaatat ttgactcaaa tttgcttgct ttattttgtt 1860
   aggagtaaac agaaagtagc ctgtgtttag tcccaaagat agcagtgatt ttgaataaag 1920
   gagttttgtg ttgcctggaa aaaaaaaaaa aaaaaaaggc cacatgtgct cgagctgcag 1980
<210> 214
   <211> 772
   <212> DNA
   <213> Homo sapiens
<220>
   <221> misc_feature
   <222> (1)...(772)
   <223> n = a, t, c or g
<220>
   <223> nbla-10522-f
<400> 214
   gnnngnngtg ggntcnnnnt tgaanccctt ggaatgnccc ctactggact gaaatatcag 60
   ctcatagaaa gtaacaaaat ttgctgtcac cttaaataag acattttaat tttgttataa 120
   tgtacaattt agaagtttga ttaattatat tatctattta ggcattaata taaaagaggt 180
   aggagtctgt tatttaaaaa aagcattaaa tttaaaaaaa aactgtcttg tctactttta 240
   gcttcattct cccatatttt gaagggtgtg taacttcagc tctgcaggat tgcatggggt 300
   aaaacttgtt accaacacat gtgaaccatt gctacattgt aggttgtgat cattttgccc 360
   cactgaagcc catgtatctg accttacgtg ccttttgaac taggagaatc gggctaattt 420
   attaatgatg ataattataa tgtatctgta cagcactttt tacatttgcg aagtgctttc 480
   caatccatgt tagttactaa gttattacag ctgtaaggat aaaacacgtc atgtggattc 540
   attttgaatt ggtgctattg gtatttcctc tgntattgct aataaatgaa aatggtggta 600
   tgaaaaaaaa aaannnnnaa aaaaaaggcc acatgtgctc gagctgcang tcgcggccgc 660
   tagactagtc tagagaaaaa acctccacac cttcccctga acctgaaaca taaaatgaat 720
   gcaatggtgg tggtaactng gttattgnag cttataatgg gtacaaataa ag         772
<210> 215
   <211> 769
<212> DNA
   <213> Homo sapiens
<220>
   <221> misc_feature
   <222> (1)...(769)
   <223> n = a, t, c or g
<220>
   <223> nbla-10522-r1
<400> 215
   gagngggntn natttgancc cttgtnannc cttttttttt ataccccatt ttcatttatt 60
   agcaataaca gaggaaatac caatagcacc aattcaaaat gaatccacat gacgtgtttt 120
   atccttacag ctgtaataac tagtaactaa catggattgg aaagcacttc gcaaatgtaa 180
   aaagtgctgt acagatacat tataattatc atcattaata aattagcccg attctcctag 240
   ttcaaaaggc acgtaaggtc agatacatgg gcttcagtgg ggcaaaatga tcacaaccta 300
   caatgtagca atggttcaca tgtgttggta acaagtttta ccccatgcaa tcctgcagag 360
   ctgaagttac acacccttca aaatatggga gaatgaagct aaaagtagac aagacagttt 420
   ttttttaaat ttaatgcttt ttttaaataa cagactccta cctcttttat attaatgcct 480
   aaatagataa tataattaat caaacttcta aattgnacat tataacaaaa ttaaaatgnc 540
   ttatttaagg tgacagcaaa ttttgntact ttctatgagc tgatatttca gtccagtagg 600
   ccaacagtgc tcgaggaatt ccgcagcttt taaagcagaa gtaacacttt ccgtacaggc 660
   ctanaagtaa aggcacatca ctgnggagca gtctttgatt tgaccaccac cggaatccgg 720
   gcctggaata aaagaccaaa ngactaaact tccagttaac ttttgggtt          769
<210> 216
   <211> 2755
   <212> DNA
   <213> Homo sapiens
<220>
   <221> misc_feature
   <222> (1)...(2755)
   <223> n = a, t, c or g
<220>
   <223> nbla-10535
<400> 216
   ttctaggcct gtacggaagt gttacttctg ctctaaaagc tgcggaattc ctcgagcact 60
   gttggcctac tggcgtacgg tgttttcttg tgcattgact gttccggggt gcaccgctcc 120
   ctgggcgtcc atctgagctt catcaggtcc acagagttgg attccaactg gaactggttc 180
   cagctgaggt gtatgcaggt cggcgggaat gccaatgcga cggctttttt tcgccaacat 240
   ggatgcacag ccaatgatgc caacaccaaa tataatagcc gagctgccca gatgtaccgg 300
   gagaagatcc ggcagctggg gagtgcggcc ctggctaggc atggcactga tctttggata 360
   gacaacatga gtagtgccgt tcctaatcac tccccagaga agaaggactc tgatttcttc 420
   acagaacaca ctcaaccccc tgcctgggat gcgccagcca ctgagccttc agggacccag 480
   cagccagccc cgtctacaga gagcagtggc ctggcacagc cggagcatgg ccccaacaca 540
   gacctgcttg gcacctcacc caaagcctca ctggaactga aaagctccat cattggcaag 600
   aagaagccag cagcagctaa gaaagggctg ggtgccaaga aaggcctagg ggcccagaag 660
   gtgagcagcc agagcttcag tgagattgag cggcaggctc aggtggcaga gaagctccgt 720
   gagcagcagg cagccgatgc caagaagcag gcggaggagt ccatggtcgc ctccatgcgt 780
   ctggcctacc aggagctcca gattgatcgt aagaaagagg aaaagaagct acagaatctg 840
   gaagggaaga agcgagagca ggcagaaagg ttgggcatgg gcttggtatc ccgaagctct 900
   gtctcccact ccgtgctgtc tgagatgcag gtgattgagc aggaaacccc agtgagtgca 960
   aaatcctctc gctcgcagct ggacttgttt gacgatgttg gtactttcgc ctctggaccc 1020
   ccaaagtaca aggacaatcc cttttcctta ggggaaagct ttggctcccg ctgggataca 1080
   gatgctgcct ggggtatgga cagggtagag gagaaggagc cagaagtgac catctcaagc 1140
   atccggccta tttcagaaag agccacaaac cggagggaag tggagaaccg gagctcaggc 1200
   ctcgagtcta gtgaggcgcg tcagaaattc gcaggagcca aagccatctc atctgacatg 1260
   ttctttgggc gggaggtgga tgcggagtat gaggccaggt ctcggctgca gcagctctca 1320
   ggcagcagtg ccatcagctc ttcagacctc tttggggaca tggatggagc tcacggagca 1380
   ggaagtgtat ctctggggaa cgtgctgcct acagcggaca ttgcccagtt taagcagggt 1440
   gtcaagtctg tggctgggaa aatggctgtg ctggccaatg gtgtgatgaa ttccttgcag 1500
   gatcgctacg gttcctactg atccgagctc tgtgactcag gcttacgatg gtgacggcaa 1560
   caagaactcc acagttccca ggctggggat gctttgcctt gtggaagctg gggaggattt 1620
   gttacttcgt atgtgtggtg tgtgtgtggg gtggcctttg aggcgctcac tcctgtgagg 1680
   ggaatggtca gtaccagccc ttgtcctctg cctgtggact gagcccttta ttccctctca 1740
   caccaccctc cgtgtgttag actcttgtcc ttctgtcctg cccccacagc tgctgctcac 1800
   ttatcctgcc atactgggaa agggggttcc cccacgatgg cttattctgg gtccagactt 1860
   tccccaggta gggaaagcgg aaggtagaag gctttttttg ctggctctag ggttcttcta 1920
   gttcgaggcc ttgggtcccc atcctctgga accaggggga ggcctggaag gagttcactg 1980
   tagacccgtc ccatggggaa agaggctgcg gacttgctgc tgctgctgct gccagtggcc 2040
   tcttctgggt gccaggagag gggaaggacc tttgtctggg cgttaccaag ggctggaaac 2100
   tttacctggt acctaaaggt ttcatttggt atcagaccgg agacccttgg gttctcccgt 2160
   ctcaccaccc ctttctacag taagcacttg gaagattgtt tcagggtgtc tcagggtccc 2220
   tctgtaccat ctgctgtgga atgcaggacc ctctgtgaca ttctttatcc cttcttcccc 2280
   gggttggtgg ccatggaggg tcttgtctgc tgtgattcga ctctggatgc tgtgagcttg 2340
   atgctggcca gggaagcaga ggatgtgaga ggcagaggca ggctcctggg gctgagctcc 2400
   ttcctctgca tcattctggg cttggcctgg acagcacccg ccagtgagag ctgtgggcct 2460
   caccctctgg cagctgagcc aagcactgtc attcttggtg ccatcttccc ctgccgcacc 2520
   ggcagtctca gcccagcccc cacctttggg ttgtaggttg ggctcccaag caacacagac 2580
   cactcttccc cttgcccctc ccccagaggg acttgacttt ctttctggac tgtttgtatt 2640
   gaaacaaagt ggtgtcaaaa taaagcccct gcagggcctg gctccctgtt ggtctgagtg 2700
   aaaaaaaaaa aaaaaaaaaa aaaaaaaaaa aaggccacat gtgctcgagc tgcag      2755
<210> 217
   <211> 2100
   <212> DNA
   <213> Homo sapiens
<220>
   <221> misc_feature
   <222> (1)...(2100)
   <223> n = a, t, c or g
<220>
   <223> nbla-10545
<400> 217
   ttctaggcct gtacggaagt gttacttctg ctctaaaagc tgcggaattc ctcgagcact 60
   gttggcctac tggcagcttt ttgtttttgg cctgaataga acaagacaat ttacctatgt 120
   gaaaacctga gtgaaagctt ggggtgtgat atgttaagta gaacagtatt ttaaagccac 180
   tttgattcat aagtttcttt aacataaaca ggttgtattg cctcttgaaa aatgataggt 240
   tctatggata acatttaatt agctttataa tacatatgat gatttaacct gaattaatgg 300
   tactactttt tctgacttcc ttttgatctt ggccctttct ctgtaaagtt ttttgttaac 360
   ttattacaaa ctgaaccctg gcttgctcct cttatcttta gtctcctttc aatctgtctt 420
   tatttctgct gaggttatat tctatttgtt tttcagatga gacccttttt gctcttcctg 480
   ctgcatctga gcctgtgata cgctcctctg caggcaagtt ctttctgcca gccttttatc 540
   ctctctgtat tctaacttta actctttagt gtgttaacac tatgcagagt agtttgggtt 600
   tactaaaaat tagcatggtt ccgagaatgt tttgtctact ggaaaagttg aaatgtgcat 660
   tttatatggt atgcccaagg gaaaaatgag caaaatctga tgaatacttg gaagtacata 720
   aggaattctt ggacccttga attacattta catatttacc attccctttc tccaaataca 780
   gtttgagtat agccagaaag gctttcctgt aaataaatac cagtctatgg catttaccca 840
   gtgccccttc tttcctatgc tgtcaccgaa actcttttca cagatattct tctcttctct 900
   aaaactagct attagtatct tagttgataa catttgaggt ttactcagac ttgatttgtc 960
   tttgcacagc atgtaattgc ctctgtagcc atgcatatgt tatatttaca atagcttaat 1020
   attttcagta atgattgaag tctactctat tgttacttat taattgctat taacatttca 1080
   ttttagtaaa cagtaagtct ttactaacac tattttggat ctgtcataaa acttgcatta 1140
   actctgagaa cactggcatt ctctaacttt cctttctgtc catggtcaca gaaaatatgg 1200
   acttgaagga gcagccaggt aacactattt cggctggtca agaggatttc ccatctgtcc 1260
   tgcttgaaac tgctgcttct cttccttctc tgtctcctct ctcagccgct tctttcaaag 1320
   aacatgaata ccttggtaat ttgtcaacag tattacccac tgaaggaaca cttcaagaaa 1380
   atgtcagtga agcttctaaa gaggtctcag agaaggcaaa aactctactc atagatagag 1440
   atttaacaga gttttcagaa ttagaatact cagaaatggg atcatcgttc agtgtctctc 1500
   caaaagcaga atctgccgta atagtagcaa atcctaggga agaaataatc gtgaaaaata 1560
   aagatgaaga agagaagtta gttagtaata acatccttca taatcaacaa gagttaccta 1620
   cagctcttac taaattggtt aaagaggatg aagttgtgtc ttcagaaaaa gcaaaagaca 1680
   gttttaatga aaagagagtt gcagtggaag ctcctatgag ggaggaatat gcagacttca 1740
   aaccatttga gcgagtatgg gaagtgaaag atagtaagga agatagtgat atgttggctg 1800
   ctggaggtaa aatcgagagc aacttggaaa gtaaagtgga taaaaaatgt tttgcagata 1860
   gccttgagca aactaatcac gaaaaagata gtgagagtag taatgatgat acttctttcc 1920
   ccagtacgcc agaaggtata aaggatcgtt caggagcata tatcacatgt gctcccttta 1980
   acccagcagc aactgagagc attgcaacaa acatttttcc tttgttagga gatcctactt 2040
   cagaaaataa gaccgatgaa aaaaaaaaaa aaaaaaaggc cacatgtgct cgagctgcag 2100
<210> 218
   <211> 1815
   <212> DNA
   <213> Homo sapiens
<220>
   <221> misc_feature
   <222> (1)...(1815)
   <223> n = a, t, c or g
<220>
   <223> nbla-10570
<400> 218
   gtacggaagt gttacttctg ctctaaaagc tgcggaattc ctcgagcact gttggcctac 60
   tggatgcatt tcgaaagcaa acagaagaaa aaagtatatt ctcacagttt ttccccagga 120
   agtctggaga aaaagtcctc atgtcaccaa tttctctgtt gcatgaattt tagtgttttg 180
   ctataagaca gcatgagggc tatcaggcca aaattcatta ttgtatttcc aatacagttt 240
   gtcttgaatg tctgtctctt aaaccagttt ttacttgtca gttttctctc agcaaacagt 300
   cttactatta tgtggaattt aacttttcag agaaaatttc caaagtcctc taccttattt 360
   ggcttttatc tgtgtctttt ggaaaaggag ctgcatggcc tcagtgcctc cataaaaagc 420
   ttaagttgag ctcatgggag aacattaaga tcgttttttt tcccttttag ctcggtaatc 480
   tcatgtacca caggtaatga ccaagcatct ctgctagagg atgtacctct cataactcct 540
   gtgtaaaatg aggatcctgt tggtgatcct cattccagtt tccaaagcga gaggagcctt 600
   ctgagcagaa gtatgcaaga ctgattctgt ttggaacagt ttcctatttg gctgcattct 660
   aggaggcctc ctgacttatt catagtattg tcgcctggtg gcattggtgt ggcgtagctt 720
   gtcctgctac ttgctaaagt tctctttctt tgagaaaact gaggaaccag aatgcttaga 780
   gttcataaaa taatctctaa accatgttag gttaaaacaa aatgaccatc tcacaaaaac 840
   gagtttagtt gaatgctcaa ttccttacaa aatcttcatt taaggctgag tgtggtggct 900
   catgcctgta attccagcac tttgggaggc cgaggcagga agactgcttg agcccaggag 960
   ttcaagacca gcctgggcaa cataggaaga ctctgtctct acatagaaat tttattataa 1020
   aagtagctgg ggatggtggc acacacctgt agtcccagct actcaggagg ttgaggtggg 1080
   aagattgctt gagcccagga gtttgaggtt gcagtgagct gatcatgcca ctgcactcta 1140
   gcctaggcta cagagcagga ccccatctcc aaaaaaaaaa aaaaaaaagt catttaaaaa 1200
   ctgaagaatg ttctcttctt gctcagttcc ctaaattagc tggggggatt gggatacttt 1260
   acaattttta catttttaaa tttgaagaat ttgttttaaa cttacttggt aatggcactt 1320
   ctacttactg accaactttt cagctctttt tgaccaggat ggttgcaaat tagggaaaat 1380
   atctttatac tatacatcca attcaagagg actttaatta cttaaaagca tttaggtcat 1440
   aattaaaatt attttcactg tttccataaa ataagcatta tgattgcaca ctccttctac 1500
   tgtctgaatt tatgtaagag gtatagcttg cttaaaatac atatatatgt aaagttatat 1560
   tttcttagaa acatggatgt ttacagccat tgctttcaaa gagattatta ctgtgtattc 1620
   tccctgtttt acaatatgtt ttcatgaaga ctatgttaca ccagaggctt ctgattatca 1680
   aagtgataac cagttttaac tgccggtatg taccagattt gtgaactaat ttaaaaaacc 1740
   acaaactatg aataaaatgg ggtttgcaaa ctaaaaaaaa aaaaaaaaaa aaggccacat 1800
   gtgctcgagc tgcag                                                1815
<210> 219
   <211> 730
   <212> DNA
   <213> Homo sapiens
<220>
   <221> misc_feature
   <222> (1)...(730)
   <223> n = a, t, c or g
<220>
   <223> nbla-10594-f
<400> 219
   cnnnngcctg ttggcctact ggttgacttt ctatttgaca agtagttata attggcaagc 60
   agtttgagtt tatactcatg acatactgaa agcatctctt tggggtcaag aaagaatccc 120
   tagtggattt gggattctag aggaggtgtt ataattaatt attcagaaaa atgcatgtat 180
   ttacctcttc aaccaaaagg gcagtaaaga atagggataa tatattttaa tgtaaatttg 240
   gaagaaggat aaaatcaccc acagttactc tccctattat tttcactggg tcaagtttta 300
   atcttttggc ttcatgagtt ccactccctt tgatgatata tgttgtatat ttttagttgt 360
   aaagcagtaa tctttaactg tagcattcct ctaattataa acagtgaaaa atcaaaatca 420
   gtccagagtt cttttgttga cttaattcct gatcagtata gacaggacta tatatattta 480
   atcagagaat tacattatat ctgaagatgg agctcaggag aatgcaccaa gactttgaat 540
   tgnatttaca ttcactgtca gtaaagacct ggctacgctt tgagaaatca ggatctncta 600
   ggccattgct tcgcaactgt gattactgaa atcctnctca taggagataa agtccgangg 660
   gtagagtcca agatgtcacc ttggaatttc tttggaagtc acatgtttat ccttaaaata 720
   ttcttattta                                                        730
<210> 220
   <211> 763
   <212> DNA
   <213> Homo sapiens
<220>
   <221> misc_feature
   <222> (1)...(889)
   <223> n = a, t, c or g
<220>
   <223> nbla-10594-r1
<400> 220
   gnngnngnnn nnnnttntgg gcctttttnn nnnctttttt tttttagcac tttctacaaa 60
   aaatgcttta tttaaaaata tgatcttttt aaaaaggaga cttttataca cacttacttt 120
   agaaacagtc aaaaacacaa cagaatcaat ccagatttag aaatgatgat ttgtaacata 180
   caatattggt atgctgaaca agacagttta aatgtatgat tctttttgtc ccttcacatt 240
   tcaataattc acatttgtta aaactagtgg ttgggactaa agaaaaggtc atatctgaat 300
   cctttcccct tcagaaatct gtcacccttc tgggcagtta cagaaatggc agcagctagg 360
   caatgtttgt atttctttgg gtgcctctgt gtcaaaaaat atatctccgc ttttattctt 420
   tttctatgaa atttatattt atgtggctat agggcctggt cgtatgtccc acgcctttta 480
   aaatctaaat ttctaaaaac agacactccg taagggcaag gagcatacac tgctatgacc 540
   tcactggact cagagcttaa tgctcttagg ttatcagcaa gagacatgat tttgatcttc 600
   cagttagcac tccaagctgc tatcaaccaa cctttttcag cacggtagtg aaatatttca 660
   aaccatattc aaacatctaa tatagattta taatnaaaca ggaaaaatgg cttgncaaat 720
   taaagngcta tgagctttta cattcaanan ggtatcttga ntt                   763
<210> 221
   <211> 797
   <212> DNA
   <213> Homo sapiens
<220>
   <221> misc_feature
   <222> (1)...(797)
   <223> n = a, t, c or g
<220>
   <223> nbla-10623-f
<400> 221
   gcactgttgg cctactggcg atgggcgagt agtcagcaac ttcatcctgc aggcgctcca 60
   gggggagcca ctcacggtat acggatccgg gtctcagaca agggcgttcc agtacgtcag 120
   cgatctagtg aatggcctcg tggctctcat gaacagcaac gtcagcagcc cggtcaacct 180
   ggggaaccca gaagaacaca caatcctaga atttgctcag ttaattaaaa accttgttgg 240
   tagcggaagt gaaattcagt ttctctctga agcccaggat gacccacaga aaagaaaacc 300
   agacatcaaa aaagcaaagc tgatgctggg gtgggagccc gtggtcccgc tggaggaagg 360
   tttaaacaaa gcaattcact actttcgtaa agaactcgag taccaggcan ataatcagta 420
   cattcccaaa ccaaagcctg gcagaataaa gaaaggacgg actcgncaca gntgaactcc 480
   tacttttagg acacaagact accattgtnc acttgatggg ganngtattt tttggctntt 540
   ttttgntgtc gtttaatgaa angactttac aggcgtcatg aagaacnaac tnggantttc 600
   attctgaagt tggctttaat gaaatggatg tgccctaana ncntcccttg aanaactnna 660
   gaattttgcc ntgnancntt tgnaattttc ctnttttgta aaatagcntn antccatntt 720
   ttgcgnnttt gtaannnttt ttattttgct ngnnanagca ttttnngnnc tnccgttnac 780
   agtcttattt aantgnn                                            797
<210> 222
   <211> 811
   <212> DNA
   <213> Homo sapiens
<220>
   <221> misc_feature
   <222> (1)...(811)
   <223> n = a, t, c or g
<220>
   <223> nbla-10623-r1
<400> 222
   gnaggggnnn nnttgggcct tttttttttt ttcntgactg gtgagtttca atattttatt 60
   aaaatagcat atttaaccat ggttaaccaa gaatatgtta actttgtgcc ccccaacctg 120
   cccccgcccc agatacgcag caaaacacag tagtgatttc aaatatcctt gatcatgacc 180
   aacaggaata tttcaaagtc aagtttgcaa ctcaaaagta gaccttggaa aaactctgaa 240
   ttaaaagaga aacataaagc tctcacacag aattctttta tctgccctaa aatcaatgcc 300
   ggcgcaccac cacctgacaa tactgctagg atttttcttt atgcatagta taacgactta 360
   tttattaagt acccactttt ataaataaaa tcggcatttt ttgtcccgct taatgttcct 420
   tttcagcttc acaaagaaac cagtaaataa aactgtcaac gacagtcaca acatatgctc 480
   tcacagcaag ataaaaaaac ttgaaaatac gcagagatgc atctacgcta ttttacataa 540
   aaagagagat tcaaaaggtg caaggcaaaa tctgcagctt tttgagggga gcttttangc 600
   acatccattt cattaaagca agcttcagaa tgaaattcca gtttggtctt catgacacct 660
   ggtnaaggct ttctttaacg accaccaaaa aaagnccaaa atncttccat aagtgtncat 720
   ggnagncttg ggnctaaaag tgaggagtca actgtggcga gtccgccctt cnttattctg 780
   gaggctttgg nttgggatgt ccgatatttg c                                811
<210> 223
   <211> 825
   <212> DNA
   <213> Homo sapiens
<220>
   <221> misc_feature
   <222> (1)...(825)
   <223> n = a, t, c or g
<220>
   <223> nbla-10624-f
<400> 223
   nnnngnagnn gnnngtgggg nnnnnnnttt gattcctgtg cctgttggcc tactgggaag 60
   aaaggacaca agataaacaa taatttggat aattcgtaaa ttggtatttc attcttgact 120
   tcttgagaat tcattgtata cgctactcct caaaaataag gaaaaccata aacaaaaatt 180
   ctggtctgaa atatcttggc agtaatgtgt ttgctctccc ctgtcctgaa tattttaggc 240
   taactttcaa ttaaaatgga ttgcaattaa attattttgg gcagaaatgt gtcttaacct 300
   ttgacaaagt tgatcatttc agcattttca aatgcttaaa ctatttctta aaatgacaca 360
   aattgtggcg ttgccattta ccgaggttta catgctcttg atgcttaagg attcggcttt 420
   cttttttccc ccgcatttat tcactgaaaa catgattgat tcagttttgt acctatgcag 480
   aatgccactg acgtgatgcc agatatgccc tatttagaaa aaataaaaat taaaaggata 540
   tttttctgca cagtcaaaat tatgcagatg tggatttaaa agactacatg tcaaaaaata 600
   cacangtaaa ggttacttac tncatggttc cttttacact cacagacagc agatttacag 660
   gcaccaaaca gccttcatca aaacaatgaa atgcccaatg tggctctttg atgacttcat 720
   tctggctttn acgtatgccc aaaccctnat aattgcaggt gctttttctg accaattatt 780
   tgggggctct ggggtatttc caggcatgcc tangncantc tggtt                 825
<210> 224
   <211> 829
   <212> DNA
   <213> Homo sapiens
<220>
   <221> misc_feature
   <222> (1)...(829)
   <223> n = a, t, c or g
<220>
   <223> nbla-10624-r1
<400> 224
   gngtgggggn nnnnntttgg ggccnttttn tnnntttttc ttgctaggaa tgagtctaca 60
   aaatgcagga gagcaaacca aatagacaaa tgtaagggaa tattgtctaa ggggactacc 120
   agatctccaa aatgccacta ctttttgtat cctactattc tcaacattct gtgtcagtta 180
   cctgtgggct ctaagattca cgtacagaga agaaaaaatg gatgagcatt aaagacaaaa 240
   aatgaagatg tcatagtaga aggagaaata gtaagttata aggaatagat tagaaataga 300
   tttaccatga ttaatctatt atgctagcaa acttatatag agccatgcat tagtagattg 360
   attagataaa tcttatgaat ctctttattt gaaaaccaaa agtattacat tgccactcag 420
   ttttggacct ttactcacga gtatctgatt cttgctcctg gttctgaatg atacttctac 480
   ttagaactgt gcctcagatg tcacacagct cccagataaa tgagggtaat caaagttctt 540
   gaacatcagt gtttctgatg ttcaagatag taggtgtagg acagtcagga agggaacaca 600
   gaccttcaca aggcttataa gagtttctac atatgaactg cagagattga ggtgctttcc 660
   ccaaccacac gtgggttcct taaagacaaa gacctaaaaa atgaggcact ggcaaaatca 720
   taaacaggct ctttcggaag atcagtttat cagngnacaa atacacttac tggttaacta 780
   tatctttcan ggnattcttt ctctatttna aatgggactt gantttaac           829
<210> 225
   <211> 2547
   <212> DNA
   <213> Homo sapiens
<220>
   <221> misc_feature
   <222> (1)...(2547)
   <223> n = a, t, c or g
<220>
   <223> nbla-10635
<400> 225
   ttctaggcct gtacggaagt gttacttctg ctctaaaagc tgcggaattc ctcgagcact 60
   gttggcctac tgggaaaatg cattgacttt gtagtcttta ttctcctagc cgcacttcgg 120
   gtatatccag aaggaagagg aaatgcaaag aggaagaaga tgtaatagtt cttctctcca 180
   gctaggtgca cttgaggttg ttcataaatg taaaattatg tcaggtttct aacatgggac 240
   actgcacaca gttgtctgac ctgatgaacc atcccatttg aaagtataga ttattattat 300
   ttcttgtagt atttggttgt tttccatctc attcatgaac aactcaacct gatagtagta 360
   tccaataaat gcctttcagg gctcaggaat gaattgacat cctagttaag aaatgagact 420
   taataatgga gactgaggcg gtttgtatta aattatatgc catgaagtgt tcattttagc 480
   tttaacctaa ttatgactgt accaccatga agtacagaat gaaaaattat atatatgggg 540
   gggaaacaga atgaatatct gattcttttg aatgcttgtg gaaatctttg agatcgtgca 600
   gggcatacca caaaatagcc tttagaacag atacccaatt ttacagttca taggacaaca 660
   tcaaacatta gtaagtctaa ataagatgaa tagaattttt gttatgtaaa ttttgctaga 720
   acagtctatt ttcttgcacc cctcaagtta acctcttaaa aaaatgaatg tataatttct 780
   accgaaagaa tatcagagag aatctctctg gcctatagtg ttaaaatatt gttcacaaat 840
   cctgattagt taagtgcata cattatgaaa cttacagaat aaaacttatt atacatctct 900
   ttcttaaatt aatatcttta cacattttca actggctccc caagtctgat aaggaaggat 960
   taaaagaaaa aagaaatgta ttagttgggt ggccaaggag tttcctttgt aatgttgaga 1020
   gacttccgct ttctgaattt cgctggttct ctaaggtaaa agagttaaat agtacccttg 1080
   ttcaccaagg aaagtgatcc aaactatata tctagtgcag atatttcctt tgcattattt 1140
   agtcttctct ggagagaaaa tacagtttcc ccttcctctt tctcttcaca tttactcttt 1200
   tcaacccaaa ataagagaca tagaaagcaa accacagcca gtttggcatc ttctcagtgc 1260
   tactagtata ggcacataca catacacagt ctcagcaagg ttataaagaa ccctgtcagg 1320
   tccacttgca acatggcctt gctacttgga ttagctcctt taagcctgaa aataactttc 1380
   ctggtcatgg aagaactgga cgcatctttt aacttatgaa atagaagttg aacttgaaaa 1440
   ctctttttaa aaaatcctgg ttttgcagga cagctacata atgaatgtat atattaagac 1500
   tgtagctgaa ttgcacatga aatcagattg ccaacttctt gactttcaat gttagacatt 1560
   tatccttaag ttgtgagcga tatatgtagc atgctgtgaa atgtctgtta tagctcttta 1620
   attcatcagt attaatacag aattatcatt tgcgtttctt ggtacttttt attcaatgta 1680
   atcagaagct gtgatgtttt gcctttgtag tcctgtgctt tgttactgta attttttttt 1740
   ttttttttta cgaagcacgt gactggacta atgtaaggca gatgacgtga tctttaagac 1800
   tgctatatat atcagtctct tactctataa ggttttaaat tagaataagc ttttatcaaa 1860
   tagataattg atgcaattta ggattcacgc aagtttcagt gtcaaatggc ggtcttatag 1920
   tttcaattct gaaaatagca aacttaataa acagccactt taaacttgtt ctggcaaacc 1980
   agaccctgct gtagatatag tctaaggtag ttaaccatat aagccttttc aactcttaat 2040
   gccctccaca tgaatcagca gttaagaagg ttctagaacc catgaaagct tttgtatgta 2100
   ttactaggtt ttgtttttct tatgtttgct gattttacag ttctgactaa agctgaccta 2160
   aatggatcag tttatgtgta atattctagt gctttaatga ctcttttttt ctttggaggg 2220
   agggtaacat tatttggaca gatgcagaag gaactgttag tgagtcaaga caaacacatc 2280
   tgaaataaag gaactgtgta ttaacatgtt aacaattcat aactgcactt tttatgacat 2340
   tttgaaaatc tatttatagg tacagaacaa tgggttttgt taaactgtat cacatttata 2400
   cttgcagaaa tttatttcat tgttattagt aggaatttta ttggttcaat aaaattggca 2460
   aaactgaaca ccaaaaaaaa aaaaaaaaaa aaaaaaaaaa aaaaaaaaaa aaaaaaaaaa 2520
   aaaaggccac atgtgctcga gctgcag                                      2547
<210> 226
   <211> 823
   <212> DNA
   <213> Homo sapiens
<220>
   <221> misc_feature
   <222> (1)...(823)
   <223> n = a, t, c or g
<220>
   <223> nbla-10667-r1
<400> 226
   ggggggntnn nttganccct ttngannncc ttnnnttttt ttttttttag ttacatagca 60
   tctaagtttt ctgatcacta ccaggtaatt ttcaaccaat aagaaaaagg aaccaacact 120
   cagctctgta gaaatctacc ttcttttaga aacctcaggc ctctgcaccc ctttagacaa 180
   ctcatttaca accacaacct tccatggctt ctcacatgcc agccagggca gggtaaaaac 240
   ggcaatttct tacaccgaaa ggtccttttt atgtaacaaa cagacctncc caaaccacaa 300
   cttttttgnc ctattgatga ctcgcatgag atcaaatctc tagtgtcccc tgactgcccc 360
   gacagggtct aggacaacac aggttaaaaa ggacagcagg gtccgttcaa ctcccacact 420
   gnctcaggtg ttttctcaaa gcacgacaat tctgcaatgg agacttacac gtggaaaaga 480
   atcaaactgt gcaggtaccc tcagtcctnt gncactacca gctcaatagc ttgagctcag 540
   aggatcaagt caagnctctg ctcactttta atctagcaca caagangcca actcagcagt 600
   acacggnatt gaagcatgca caggcattgg tacaaaactc agttagatta agagtccnta 660
   aataaacctt gnagntgagt agaaatccca aacagntggt gangacaggc tgtangctaa 720
   ctttacattc aaggttttat cncccagnag gcagattcca aatgntagat attggnccgg 780
   aaaattttcn gggnaaagaa atcntnttnc nctttatggc cng                    823
<210> 227
   <211> 838
   <212> DNA
   <213> Homo sapiens
<220>
   <221> misc_feature
   <222> (1)...(838)
   <223> n = a, t, c or g
<220>
   <223> nbla-10672-f
<400> 227
   gngngggtng nnttnnnatt tgaantccnt ttnctgttgn cctactggtt caaaaccttc 60
   aagtgatatg tggaaaaaag tgaatgagac ctctgatagg gggttttcag aaccttgttc 120
   acaccaaaat gtgacagttc tttcatgttt tcctaaacca agttaaaatt acatgtatat 180
   tttggtgtta aggttgattt ttaagatact tctgatttgt acaaaaggaa tgtttccttt 240
   ataaatcaca gaagaaaatg acaatatctg ttggatattt gatataattt aatggtgtta 300
   taaaaccttt aagaggattc atggtgaata tatgtgataa catctttata ctttgaaaaa 360
   tgttccactt acccttcaga tatttgttgt aagttaattc aattcttaat actttaattt 420
   tgctccaaca agggctttat gttgctggta agagaattta tttactaaat gcactatgta 480
   taaagtgaaa gatagtttac ttatctgact ttgatattag atggctgaca ttagtgcaca 540
   taatgcagag tttaaccttg attcttcaac agagtccaga tttaaatgtc tacttagtta 600
   attagttagc tgatattctt ncacaattaa tatattcaat ttcccatcag tatatcactt 660
   taaattttat ggttttctaa ngnaactttt cacagaattt taaacaactg atgcatncat 720
   actcanggtg tagggaagaa tactttgcan ttnaaaaccc tgtcaacctg caaccagcnc 780
   angagaattn naacttcant gagaatttag aaaaattttc taaagngaga tgcntttt   838
<210> 228
   <211> 882
   <212> DNA
   <213> Homo sapiens
<220>
   <221> misc_feature
   <222> (1)...(882)
   <223> n = a, t, c or g
<220>
   <223> nbla-10672-r1
<400> 228
   ggccgnntgn atcaattgnn anccctttga ancacccaan cnnatttcgc caaatcnccg 60
   nnttgnnacn nnatcacnnt cccnnccact atncactana ttttcaannn nancanctnn 120
   ttntatnntc ggggggcatt tntaaacacc tacanncccc cccnctnant tcatngcnna 180
   tactacncnn tnantncnnc anncacatta ccntctcatc anngannnnc ntnannntna 240
   nncccttnan atcncantaa cntncccnat tacancnnac tactgncnna nntncaancc 300
   ntnagnaant ctntntccnn nnnnattacc cnntaatnga acnnntcnna ancaatctna 360
   atncttactn aacgncttac nctntananc ctannctctc atanncanna nnncnnnnna 420
   ctntcttnnt nntnttnntn acatcatcct atactantcc natacnncta ancctcnant 480
   tntttnttct gcannncnaa nanntnanct attcancaaa nnctnccnnt nntcacttan 540
   ncacnnntnn ntnnaancct tnnttanana nacnnttnan tatnncnant ancnanccnt 600
   ctancncntc natncnanta nnttnctcnt anantnctnn ctananntnn aaantannnt 660
   cactcacctn cctcacttnn aatnnganna tntnannnnn ntntaaacan tacnntntat 720
   aatnnntnta ntnnntcctc cncntnanat aatnctatnt nnntatatac acccatttat 780
   ctnncnnnct nnnccctcna nntntnacnc nntctantnc tactataatc cannaatctt 840
   canaactcat ntncatctnn tnacatancc cntnccatnn cg                    882
<210> 229
   <211> 2570
   <212> DNA
   <213> Homo sapiens
<220>
   <221> misc_feature
   <222> (1)...(2570)
   <223> n = a, t, c or g
<220>
   <223> nbla-10677
<400> 229
   gtacggaagt gttacttctg ctctaaaagc tgcggaattc ctcgagcact gttggcctac 60
   tggtacttct aaataaatga attactcaat ctcctatgac catctataca tactccacct 120
   tcaaaaagta catcaatatt atatcattaa ggaaatagta accttctctt ctccaatatg 180
   catgacattt ttagacaatg caattgtggc actggcactt atttcagtga agaaaaactt 240
   tgtggttcta tggcattcat catttgacaa atgcaagcat cttccttatc aatcagctcc 300
   tattgaactt actagcactg actgtggaat ccttaagggc ccattacatt tctgaagaag 360
   aaagctaaga tgaaggacat gccactccga attcatgtgc tacttggcct agctatcact 420
   acactagtac aagccgtaga taaaaaagtg gattgtccac ggttatgtac gtgtgaaatc 480
   aggccttggt ttacacccag atccatttat atggaagcat ctacagtgga ttgtaatgat 540
   ttaggtcttt taactttccc agccagattg ccagctaaca cacagattct tctcctacag 600
   actaacaata ttgcaaaaat tgaatactcc acagactttc cagtaaacct tactggcctg 660
   gatttatctc aaaacaattt atcttcagtc accaatatta atgtaaagaa gatgcctcag 720
   ctcctttctg tgtacctaga ggaaaacaaa cttactgaac tgcctgaaaa atgtctgtcc 780
   gaactgagca acttacaaga actctatatt aatcacaact tgctttctac aatttcacct 840
   ggagccttta ttggcctaca taatcttctt cgacttcatc tcaattcaaa tagattgcag 900
   atgatcaaca gtaagtggtt tgatgctctt ccaaatctag agattctgat gattggggaa 960
   aatccaatta tcagaatcaa agacatgaac tttaagcctc ttatcaatct tcgcagcctg 1020
   gttatagctg gtataaacct cacagaaata ccagataacg ccttggttgg actggaaaac 1080
   ttagaaagca tctcttttta cgataacagg cttattaaag taccccatgt tgctcttcaa 1140
   aaagttgtaa atctcaaatt tttggatcta aataaaaatc ctattaatag aatacgaagg 1200
   ggtgatttta gcaatatgct acacttaaaa gagttgggga taaataatat gcctgagctg 1260
   atttccatcg atagtcttgc tgtggataac ctgccagatt taagaaaaat agaagctact 1320
   aacaacccta gattgtctta cattcacccc aatgcatttt tcagactccc caagctggaa 1380
   tcactcatgc tgaacagcaa tgctctcagt gccctgtacc atggtaccat tgagtctctg 1440
   ccaaacctca aggaaatcag catacacagt aaccccatca ggtgtgactg tgtcatccgt 1500
   tggatgaaca tgaacaaaac caacattcga ttcatggagc cagattcact gttttgcgtg 1560
   gacccacctg aattccaagg tcagaatgtt cggcaagtgc atttcaggga catgatggaa 1620
   atttgtctcc ctcttatagc tcctgagagc tttccttcta atctaaatgt agaagctggg 1680
   agctatgttt cctttcactg tagagctact gcagaaccac agcctgaaat ctactggata 1740
   acaccttctg gtcaaaaact cttgcctaat accctgacag acaagttcta tgtccattct 1800
   gagggaacac tagatataaa tggcgtaact cccaaagaag ggggtttata tacttgtata 1860
   gcaactaacc tagttggcgc tgacttgaag tctgttatga tcaaagtgga tggatctttt 1920
   ccacaagata acaatggctc tttgaatatt aaaataagag atattcaggc caattcagtt 1980
   ttggtgtcct ggaaagcaag ttctaaaatt ctcaaatcta gtgttaaatg gacagccttt 2040
   gtcaagactg aaaattctca tgctgcgcaa agtgctcgaa taccatctga tgtcaaggta 2100
   tataatctta ctcatctgaa tccatcaact gagtataaaa tttgtattga tattcccacc 2160
   atctatcaga aaaacagaaa aaaatgtgta aatgtcacca ccaaaggttt gcaccctgat 2220
   caaaaagagt atgaaaagaa taataccaca acacttatgg cctgtcttgg aggccttctg 2280
   gggattattg gtgtgatatg tcttatcagc tgcctctctc cagaaatgaa ctgtgatggt 2340
   ggacacagct atgtgaggaa ttacttacag aaaccaacct ttgcattagg tgagctttat 2400
   cctcctctga taaatctctg ggaagcagga aaagaaaaaa gtacatcact gaaagtaaaa 2460
   gcaactgtta taggtttacc aacaaatatg tcctaaaaac caccaaggaa acctactcca 2520
   aaaatgaaca aaaaaaaaaa aaaaaaaggc cacatgtgct cgagctgcag            2570
<210> 230
   <211> 1991
   <212> DNA
   <213> Homo sapiens
<220>
   <221> misc_feature
   <222> (1)...(1991)
   <223> n = a, t, c or g
<220>
   <223> nbla-10679
<400> 230
<210> 231
   <211> 2473
   <212> DNA
   <213> Homo sapiens
<220>
   <221> misc_feature
   <222> (1)...(2473)
   <223> n = a, t, c or g
<220>
   <223> nbla-10687
<400> 231
   gtacggaagt gttacttctg ctctaaaagc tgcggaattc ctcgagcact gttggcctac 60
   tggagagcga gggcgggcgg gtgtgctccg ttcggcctgg gaatccgatt ggttatcgat 120
   tcccgcggaa atcaccccgg tgcctctcgc gccccctggc cggcctatgg cgataacttc 180
   acggagacgg gcgtggctgg agttttgctt tattgaagtt gaacggttgg ggccaggctg 240
   cgaaggagac tgagagcgcg acaaggccct ggacagatgc ccgcgcgggg aaggaaggct 300
   ggcttaggag ggggcgtcag ctatggttta gcagcttcca gcgaccgaaa cctacatcac 360
   agccgcagca cacaattcca ggaaacctgg tgctcaatat tgggatctca ggaatcagga 420
   ccggcggctg gggtgaaagc ctagcggtca gagcttagga gtgaggcaga agctgttaga 480
   gactcttcgc aagtgacccc ggggctacat atggatgatt caagagggag tctcctcgcc 540
   agaaagttca catgtttcac tccttctggc cttcatacca acccattgtt aaaactggaa 600
   gtccctccac ctggggtcac tgactccact gttttcagat aggatgggga ccctgcactg 660
   gagactgctc tctagcccct gggcaggaga ggcacagaca ctgctacgca cgcggagcag 720
   ggatcaccag cgctttgact gactttgctc gtccaggagg ctggggacat gcgtgcccat 780
   gctcagccca ccccctctgt agacacaatt ggtcgcccat tctgtttata ggaagtcttc 840
   catcctgcac cagcagctgt ccctagacag ggattgggag acatgcaagt tgcttggctt 900
   cctattacct tgtgttgaaa acacaatagg ctttgggatc aggagttcat tccagttatt 960
   ttaatgttat ttactagcag tgtgccctcg tgcacattat caaactcctc tgactcttgg 1020
   aatcctcatt tttcaataat aactcacaaa gctgtcaagc ctaagggttc taacagctcg 1080
   aacagcacct ggaacagaag caatagccgc cactcagagg ttacttcttc cttctccgcc 1140
   ttctctggat cacctcccta cctcatcctc aatctaacgc tggaaatatt ggatatgggg 1200
   taggtcacca aatgttgcca gaagactcaa attatgccac ccatatctca gctgggaagg 1260
   gcttaccgtg gcctgactct cctcagggtc caagtcccca tttatgatgt ccaggcagaa 1320
   acacttgttt ctgacacata gtaggcgctg gaaaatgtta gtcaaatgaa cgaggtctgg 1380
   tgtggagaga agagaaatct tgggagagtc atttaagggg gtgcccagtt gtcctcataa 1440
   aaaacactat catcatcagg gtgaccaaag cagacatcat aggcaggggg tggagtcaga 1500
   ggctctaatg tggggactgc cgccaagctc tgcagatcag gagcagtgga cacagcccgt 1560
   ggtccccgaa gccgaaggtt gattggagct cttccagggc ttccttcctg ggccatggac 1620
   ccctctgagg ccattcgcct ctgttccagt ttggctctcc tggacccctc tggatggcta 1680
   ggttgttcct cctcaggtgc tgggggtatc acaacagtgc tgtagggggg tggttggtcc 1740
   aactcttggg ggcggcactg ggattctagt cccaccacgg cctcttcata gactggcact 1800
   tcaaaggctt cattgtccct gagggaagag aaataaggcc attggtggct tccatgaaag 1860
   aagccctttt tccctttttt ttgtctgctg tgggcaaaag atcctctgtt ggcctctgac 1920
   ccctcctccc gctgatgcca atgacaggaa accagagact ctctgcctca gagactacag 1980
   ggagagaggg aggatgggcc caaggtgtat tcctcttacg atgcccaccc ttaactcacc 2040
   gtgcattgcc tgagggccct gggctctcag tctgcattga ttggagctgg gaccgaagcc 2100
   cccattccag aaaccggacc aggagatagg caaacaagaa gaagccaccc aatgtgagaa 2160
   agaaataagc aacgggggtg atgtccgtct ttatgcccag caaagccagc cccagtagga 2220
   aggaggcgca gcacaggagg agaagcggct tctccagtct ccccagtaag tgcattggca 2280
   ccatggcccc tccccaggct cctgcaaaaa gacacacagc tattgtgtct acactagcaa 2340
   ttggtgtcat ggctgtgtca gataccaaag taaccatatg atgtttcttt gtttggtttc 2400
   taggttcttc aatctcaaat aaatcatctg taaaaaaaaa aaaaaaaaaa ggccacatgt 2460
   gctcgagctg cag                                                    2473
<210> 232
   <211> 1945
   <212> DNA
   <213> Homo sapiens
<220>
   <221> misc_feature
   <222> (1)...(1945)
   <223> n = a, t, c or g
<220>
   <223> nbla-10696
<400> 232
   gcctgtacgg aagtgttact tctgctctaa aagctgcgga attcctcgag cactgttggc 60
   ctactgggac aatccccaga gccccgcggc ccggcctcgg ggcagccatg gactcgcagg 120
   aattgaagac tttgattaat tactattgtc aagagagata tttccatcat gtattactgg 180
   ttgccagtga aggaattaag aggtatggaa gtgatccagt cttcaggttt tatcatgcct 240
   atggcacatt aatggaaggt aaaactcaag aagctcttcg agaatttgag gctattaaaa 300
   ataaacaaga tgtatcactt tgttctctac ttgcactgat atatgcccat aaaatgagtc 360
   ctaatccaga tagagaagct attctggaat cagatgccag agtgaaggaa caacgtaaag 420
   gagctggaga gaaagcctta taccatgcag gcttattttt atggcacatt ggtcgccatg 480
   ataaagcaag ggaatatatt gacagaatga tcaaaatatc agatggtagt aaacagggac 540
   acgttttgaa agcatggctt gatattacaa gaggaaaaga gccttacact aaaaaagcac 600
   tgaagtattt tgaagaggga ctccaagatg ggaatgatac ttttgctctg ctgggtaagg 660
   cacaatgcct tgagatgcgc cagaattatt caggtgccct ggagactatg aaccagataa 720
   tcgtgaattt tccgagcttc cttcctgctt ttgttaagaa aatgaaatta caactagcct 780
   tgcagaattg ggaccagaca gttgagacag cacaaaggtt gctgctccaa gatagccaaa 840
   atgtggaagc actgagaatg caggcactct actatgtgtg tagagagggg gatatagaga 900
   aggcttccac caagctggaa aacttgggaa atgcattgga tgccatggaa ccacagaatg 960
   ctcaactttt ctataacatt acactcgcct tcagcagaac ttgtggacgt agtcaactta 1020
   ttcttcaaaa aattcaaacg ttacttgaga gagcttttag tttaaaccct cagcaatcag 1080
   aatttgctac agaacttgga taccaaatga ttttacaagg aagagttaaa gaggcactga 1140
   agtggtataa gaccgccatg acacttgatg agactagtgt gtctgcccta gttggattta 1200
   tccaatgtca gttgatagaa gggcaattac aggatgcaga tcagcagcta gaatttttaa 1260
   atgaaatcca gcaatccatt ggaaaatctg cggaattaat ctatttacat gcagttcttg 1320
   ccatgaagaa aaataaacga caagaagaag ttattaattt gttaaatgat gtcctggaca 1380
   ctcacttttc acaattagaa ggtttgcctc ttggcataca gtattttgaa aagctaaatc 1440
   ctgatttctt gttagaaatt gttatggagt atctgagctt ctgtccaatg caggtaagta 1500
   attatgggac ttactttcaa ggatgcgttt acttgatgtt ttatgaaaga acttaaaagt 1560
   tttctttgat gcattagact atatcggcat cttaaaatgg tagtatttgt acctatgtta 1620
   gttgaaatcg cttttaatca gactgttttt agtaatgact gctgtcaact actgaaagta 1680
   ttggtttgca aatattcatt tgtatttcat aaaatcttaa aatcttcagt ttaaagaaat 1740
   attaaaaggc atctaattca gacacctacc ttatatttgc taccagtctg taacatcact 1800
   gtcaatgtct atcccatgtc tggacacact aaaatgaagt tcttgtcata taactatgat 1860
   tgttagcctg tgtttcctgt gttaagtcaa actctgcttt tggttaaaaa aaaaaaaaaa 1920
   aaggccacat gtgctcgagc tgcag                                  1945
<210> 233
   <211> 2489
   <212> DNA
   <213> Homo sapiens
<220>
   <221> misc_feature
   <222> (1)...(2489)
   <223> n = a, t, c or g
<220>
   <223> nbla-10712
<400> 233
   gcctgtacgg aagtgttact tctgctctaa aagctgcgga attcctcgag cactgttggc 60
   ctactggcag ttagaatagg taaagcatta ataatggtag gctatatatc ataatcacta 120
   tataaagaaa catcagaatt tggaaacccc ttaagcaaaa atatatctta gaaatcttaa 180
   ggacacttaa tggcaagcaa aaaagtttaa atttagtttt ttataaatta catatagcac 240
   aatttatgtg tacatgtaga atcttttagt ttttagtgtt tatgtaatat attagacaat 300
   aataaacatt tacatttatg tttacattta tatttaattt ttcaaattta atttaagttt 360
   ctaaatgctt ctgtggattt aaattttgga ataatctttt cttccccatt gatactggaa 420
   tcttgggaag tttcttactc atatcacaat atgttttatt taaatggatg cagcttacat 480
   tgcagagttt atataaataa tcacataaca agccaattcc ttagtcattt tctgtattaa 540
   aaaactcaga gatctagaga gcttttggat tgaaatattt ccttggtgat tttggatgca 600
   aacctgattt ctgtgttcac accatggcca gagatgggac tgttagcacc acctgcttct 660
   ttaatgtcgt ccatatgatc agcatcagca ccagaaactc caggtgtccc aaacttaaag 720
   tgaaagccat agagtccttt ggtgactccc atgttctgtg ctggttctag aacatgctgg 780
   taaatatcag agtttctatg cttatggctg ataagtagac atttttaaaa atcataaaaa 840
   ctgtattttc tatatagttc agctaagttt atgaaaaaga tgcagactca gtatttggag 900
   cattgctatc gctccatgat gtgtttaaaa attattttta aattgacaaa attttatatc 960
   tacatatata tatatcttgt aacacatgat gtttgaaatg cttaattcta gccaattaaa 1020
   aatgctttac ctcaatagtt atttttattg tgagaaaggt caacatggtc ttggcacttt 1080
   aaaaaaaaaa acaatatatt ctcaataact gtattcactc tgcactacaa taggtctctt 1140
   gaactaattc ctcctagcta acaggcattg tttatcattt aacagataat tctctaagcc 1200
   cccttctcct aaaacttcag catctcttaa ccttctttct agtctctact tttatgagat 1260
   caacttgttt agattccacg tgagtgagac catgaggtat tcatctttct gtgttctgct 1320
   ccattggtct atttatctgt ttttaatgcc agtattggac tgtttcagtt actgtagctt 1380
   tgcagtatat ttgactgtca agtagtgcag tgcttccagc cttcagagtc ttttactgta 1440
   ctatatgaat ttttgttttt tttttgtgaa aaattttatt gatattttga tagacattat 1500
   gttaaatata tacatcattt tgtgtaacat agacttttta gcaatattaa tttggccaat 1560
   ttatgaataa gggataactt ttcatttatt tatgtttaat tttatacctc agttttcttt 1620
   agcttttaat gtagagatct ttaacatttt tgaataattt tatttggaag taaactttct 1680
   gtaactattg caggtgtcaa ttgttttctt tatttctttt tcagatagtt tgttgttagt 1740
   gtatagaaat gctactgacc cttatatctt gattttgtat cctgcaactt tagtgaattt 1800
   attctaagta tttttaaagt tttctattta taagactgtg atatctgcaa acagggacaa 1860
   ttattttctt tctttccaat ttggttgtct ttggttttgt tttctaattt ctctggcctg 1920
   gaaattcagt agcatgttaa ataagagtga tgaagttaga tatatttgcc ttactataac 1980
   tcttagaaaa aaggcttaca acttttgaag gtttagcatg atgtcagatg tccatttgtc 2040
   atatatggcc tttattggct tgaggtatgt atgttctata tgtaatttgt tttgagattt 2100
   tgtcataaag taatgttgaa ttttctaaaa ttctttttct gcattcactt aaaagttata 2160
   attggttctg agatccttca tctttctcag aatctaggag gatgcatctg tacctagagg 2220
   aggacacaaa ggcatcatat gttacattct cgtgacatat aatgtctact gcttataatg 2280
   tctactgtga gtttctgact ccccaaatga aattgaggcc cagaaacttt caactgcttt 2340
   gttcgttgaa taatgccaag tgtctggaaa tgtgactatt cagattaaat gcttaatgtg 2400
   tgtgtgttga ctaaataaat ctcaaccaga accttctcag aaaaaaaaaa aaaaaaaaaa 2460
   aaaaaaggcc acatgtgctc gagctgcag                               2489
<210> 234
   <211> 2385
   <212> DNA
   <213> Homo sapiens
<220>
   <221> misc_feature
   <222> (1)...(2385)
   <223> n = a, t, c or g
<220>
   <223> nbla-10751
<400> 234
   gcctgtacgg aagtgttact tctgctctaa aagctgcgga attcctcgag cactgttggc 60
   ctactggatt tttggcctgt caggtccatc cggcgatgct gggtctggac gagctcggga 120
   ggagtggttg tggccattgc acacaggcgg atctgaggtt cggcgacgcc gctggtcgcg 180
   acccgggaca ggacaacgac aggaacaccg ccgagcccgc gttccccccc cgcccagagt 240
   catggcggca gcagccgctc tgagggcgcc tgctcagagc agtgtgacct ttgaagatgt 300
   ggctgtaaac ttttccctgg aggaatggag tcttcttaat gaggctcaga gatgcctgta 360
   ccgtgacgtg atgctggaga ccttgacact tatatcctcc ctgggttgtt ggcatggagg 420
   ggaagatgag gcagcacctt ctaagcagag cacgtgtata catatataca aagaccaggg 480
   aggtcatagt ggagaaaggc cttatgagtg tggggaatat aggaaattat ttaagaacaa 540
   gtcctgcctc actgaaccca gaagagatca caaacacagg aatgttcgca ctggagaaag 600
   gccttatgag tgcagcaaat atgggaaatt atttcaccaa aagcctacac tccatattca 660
   tgagagattt catactgggc aaaagaccta tgagtgcagt gagtgtggaa aatcatttca 720
   ccaaagctct tcactcttgc agcgtcagac acttcacact agagaaaggc cttatgagtg 780
   tattgaatgt gggaaagcct ttgctgaaaa gtccagtctc attaaccaca ggaaagttca 840
   ctctggagca aagcgttatg aatgcaatga atgtgggaag tcctttgctt atacatctag 900
   tctcattaaa cacaggagga ttcacactgg agagaggcct tatgagtgca gtgaatgtgg 960
   gagatccttt gctgaaaact ccagtcttat taaacacttg agagttcaca caggagaaag 1020
   gccttatgaa tgcgttgagt gtggaaaatc atttcgccga agctcttcac tcttgcagca 1080
   tcagagagtt cacactagag aaaggcctta tgaatgtagt gaatgtggga aatcctttag 1140
   cttaaggtcc aacctcattc accatcagcg agttcatact ggagaaaggc atgagtgcgg 1200
   gcagtgtggg aaatccttta gccgaaaatc tagccttatt atacatctga gagttcacac 1260
   tggagaaagg ccttatgagt gcagtgactg tgggaaatcc tttgctgaaa actccagcct 1320
   tattaaacac ttgagagttc acactggaga aaggccatat gaatgcattg attgtggaaa 1380
   atcatttcgc cacagttctt cgttccgtcg ccatcagaga gttcatactg gaatgaggcc 1440
   ttataagtga agcaaatttt ggaaattctc ttgcccaggc tttttgctcc ttcaaggcca 1500
   gagagttcac accggatcaa ggtgttatga gtgtgacaaa tggggaatat tctttagcta 1560
   gaatgctagc ttctttacat aaaagagtgc tcccactgaa gaagtgcctt ttgagtgcaa 1620
   tgaatgtgag aaagccttca gccctctctc attggttacc acaatattta catgaggaaa 1680
   ataccacaga tgtggaggta atattatttt ttcttcaata taacactgga ggaaacccct 1740
   tatgaggatg ccatctgcct aaattgaatg tcatacatca aatagctgca tacattccag 1800
   gtatgtggga actgcatagt atttttcgac ctgcccaggt ctcttgctag acttctgtga 1860
   cttacagttc tcttctaaaa agcatttcac ctttaccact tggcaggtgc ccacagtaca 1920
   tcattcgcct aactcatcat gatccagaag taactttgat tttgtttcat tcaacagagg 1980
   gattttttag tatcctcagc actcattcta ttctcatttc tttctctcca atgagttagg 2040
   ccatgaacct gactcatttt tggcacagag gactcttctg ataacttgaa aaaatggact 2100
   acctttttca gggatctttt ggatctcaca gagttcttaa gatggaattt tccagcctgc 2160
   cagtcatgaa tctcagacag cctgccacct attgccctga tttgtgttat aataaaggcc 2220
   ttgttgctta aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa ggccacatgt gctcgagctg cagtcgcggc cgtag 2385

<210> 235
   <211> 758
   <212> DNA
   <213> Homo sapiens
<220>
   <221> misc_feature
   <222> (1)...(758)
   <223> n = a, t, c or g
<220>
   <223> nbla-10759-f
<400> 235
   gnnnnnnnnn tnnnnnnnnn nntttggant ccttgagcac tgttggccta ctggtttatg 60
   aaataagttt gcattgcaca tccgtgcaga aatctttctg actttgaaat ttttagggcg 120
   tcagctgtca gatacgaaag gtagatatca ggtaagaatc tggacttagg aaatagtcac 180
   aaaactgtca taggttgtaa ttttatcaac attcgcttct agtaaaatta aagtcaatta 240
   agaaatagaa cttgggtcaa agttctgtta caaagcttca taatttgtcc cgaagcatat 300
   ggtggagcat tctgagaaat ttgctttttg tgtgtttgac attcctaatt tgggagtcct 360
   tcagctgaat tactattctt ttagaagttg agacagcagg taagcaaagg acctagttca 420
   tgtaaacatg gacatcatga tggctattta aaaaatattt gttctacacc ttctcccctg 480
   aggcttgggg agtgtgttca gccgctgcag tttctctgct catggaggtc ttgtttggat 540
   ctgtgctggc ggctgagcat ttagtgtgag ccagtgaccc atgaacttgc cgctctgtga 600
   gggccagagt cagggccagt catggtaatg ggcctgaagg cacttncaga accttttatg 660
   tctctcgtga gccatctgtt aagaacgtct tcttggtgtg gnttgtagcc tacctggcng 720
   ctattctggg aaaccttctt gagtgctatg caaaagtg                         758
<210> 236
   <211> 767
   <212> DNA
   <213> Homo sapiens
<220>
   <221> misc_feature
   <222> (1)...(767)
   <223> n = a, t, c or g
<220>
   <221> misc_feature
   <222> (1)...(767)
   <223> n = a, t, c or g
<220>
   <223> nbla-10759-r1
<400> 236
   gnngnnnnng gngnnnnnnn ttgtgtggcc tttttttttt tncttttttt ttttttgcaa 60
   cacaagtcaa tctttattga aaactgcagt attaatacat aacaattctt gttacaataa 120
   acgtgctttt gagattttta aatctgagct catctcatca gattgcataa aaaattaaaa 180
   tagtatcaat tgacacctaa ctgaactggc tcaggatgga aattccattc cttggcatgg 240
   atacgtaagt tcaatgcaga ggtgagggat gcctttaaca ctggaagaca atgctgactt 300
   agcttaaaaa aagtaccgag agaacggtgt aaaaaacggt atttaaaaat catttttaaa 360
   aaaacaaaaa ggaaccgttt cttctttagt tacaatccat gaggctctct agggcctctc 420
   cgtgtggcca gcacagcaac cctgctagga gcacaaacgg ctggcctgag atctggccca 480
   gctgccttgc ccactggtct gcacagggac tcatgggcac agcctgtggt gaggaggaga 540
   cacctgtcat gccagtcctg ggagcacacc acccttctgc aggcccgggg gggggtncct 600
   acacagacgc cggtcatctc tgcttccctt tccaagtgac ttcacttcag tcctgtggac 660
   caagataang ctattcccac aggctnttct tcttcaaata atgagtngga aaaagagaca 720
   aactttntga aacatgcaan tttcgancca aattnggcca catcccc               767
<210> 237
   <211> 785
   <212> DNA
   <213> Homo sapiens
<220>
   <221> misc_feature
   <222> (1)...(785)
   <223> n = a, t, c or g
<220>
   <223> nbla-10773-f
<400> 237
   gnnnnnnttg nnnncnnnnn nttgnantcc tcgagcactg ttggcctact ggcagaatag 60
   acatttgata atggtcttta aattaccctg tacttttagg ggggttatat gtcataggca 120
   cagacatctg actctctaat gtcattatga tacacttttt ttatatatat attttctttc 180
   taaatcataa aatgagttta cagcagcaga ccccagcttt tctgagaagt acctacactg 240
   ttatattgtg taatcgctgt accaactaat atttttcgtc agaatatgta caatacagag 300
   ttgaagaatc tgtatcaaaa tgatcagctg tgatgagtct gagctagagg gctttttcac 360
   aaagcatgtt ttgttctctg gtgaacttat attagaccat ggctgccaac ttaaaattcc 420
   tttcttttaa ggatgcaatg ttgcataatt agtctgctat tgcagtcagg atgcttgcac 480
   cctttatttt actcctggta tagaatgcaa tagactatca gagttgaaag gaacctctga 540
   gaccatctaa ttcaacttgc tcgtaatata gatgagaaaa cagcctctgt cagttttaat 600
   gtcgcacagc aaggtagctg ccattaagaa tctcagaaat aagtttcatt ttagaaaatc 660
   tcaagtattt tctggataac aaatagaaat agatnaaaaa aagtagctta aaacaatact 720
   attttttcta atgatcnttt cantgacaga acangccttt gnttcacgtg ggaccaattg 780
   ggggt                                                             785
<210> 238
   <211> 797
   <212> DNA
   <213> Homo sapiens
<220>
   <221> misc_feature
   <222> (1)...(797)
   <223> n = a, t, c or g
<220>
   <223> nbla-10773-r1
<400> 238
   gnngnnggnn nnnnttngtg gccttttttt tttttcnttt tctaagaaga aagggatagg 60
   aatcatgaag tcatttccag caaatgtcct taataggcaa gcttgccaaa atatcaccgt 120
   gcccctgtct ttcccaaaac cttccaacag ctatcagcaa cagctgacaa gcactagcta 180
   atttgaaatt gtatgttctc tcctgtcaac accagaccac acacacagct ttgaaaaaca 240
   cccagtgaat accataattt ttaaacagag ggctcatcat gttccatttt cttgtgccta 300
   aaaagtaata tgtactttct attcagaatt taaaaacctt caccaccctt tgaagtttta 360
   gatttaaaat tttattcttt ggcatctgaa ggaagaggac atcaaatatt agaggcaata 420
   aagcaagtag aagtgacaac ttcccctaag gttggtggta ccagacactg gcgcaaattc 480
   atccatctgc atgtctcaag gcatattccc acatgaggat ctaggaggga aagtgtcacg 540
   gaacgaacag atgggcttcc ttgcctagac tagcctaatg gagcttttag gcaatttgca 600
   tactagctgc aagtgacaca ggtgtgtgtc aagacttgag agaagctaaa agaaacctta 660
   tatagggnat actttcatgc tgattcatgc ttgncttcat tgngaatgct aatangngat 720
   attttggtta naanggcaac anttcaatgg ngggcccttt ggttttttct ttntaagtac 780
   aggcttgagg canttnn                                                797
<210> 239
   <211> 2438
   <212> DNA
   <213> Homo sapiens
<220>
   <221> misc_feature
   <222> (1)...(2438)
   <223> n = a, t, c or g
<220>
   <223> nbla-10797
<400> 239
   ttctaggcct gtacggaagt gttacttctg ctctaaaagc tgcggaattc ctcgagcact 60
   gttggcctac tggatgtatc tttatgctct ttcactgctc ttctgtgcgt tggagtttag 120
   caattcagtg agactgggga gtgtctcaag aatggaacct ttgccttcaa ggttgggtca 180
   tcaaaaatcc ctctaaagga tctaactgta atgaactgtt tttatttttg ccattagtgc 240
   aagtatcaac tggcagcctc aaacagcttt agactatgtc tcaggtctct catagttata 300
   cttagaactg ctctttcttg aagctaaaag aggtttttgg agtaaagatc tgcttcatgt 360
   ctgctagcat ggtatctgct gttcaaccta gtatagagca tgtgaaatgc aactgtcctt 420
   taatggaatt cagccaaact ccaaaaatac tgagttgctt ctagttatct aaagtagtac 480
   agaatcacta tcccgatgaa gaagtggggt ttttcagaag attgtaaact catttccatg 540
   gcacagatac aaatcatcgt caggacttgt tagccagtcc atgtctcatt gataggatgt 600
   agcctgccat ctgcatagat ggtttaacat ggtgctgttt ttgaaaactt gctgtggaaa 660
   taaatttgac agacgagtag ttatttgtaa agctgtccta gaaagaggtt aattcttttg 720
   gtatatgaat atttctcctc caatcctttt aattaggtgg ttcagcttat cttgtatttt 780
   ctgagtcata actatactct agggccaaga cctactataa aatctggggt cctaaccaga 840
   gagttgttta gatcaatatg atatgctttg ccaacatgat ctaagaaggt attcattctt 900
   tctgggtttg agaagagttg acgaaagtgt aagattatac tctcatgcta aaataaggta 960
   taggctaagt gaaatactgt aaatgggttg gttagaatta aagtttagaa ctctaaatct 1020
   tatgcttatt tttaaagtaa aaagttgcct cactgtcatg aaatcctgtt actttcatta 1080
   aaaaaaaaaa tcctgtaaaa tggtcaaatt ggataatgta agagataatg atggctttga 1140
   ggcatttcat actttaccag tttacatttg gctagcagac tggtaaggtt tagggttctc 1200
   tccactaatg gaaaattaaa tgctaaacgt cctaagaatt atgttgtttt caaggctttg 1260
   actcagattt gtactttgat gtatgaaagt gaatatcaaa cttgtttata gaaaagacta 1320
   tatagaaaaa taagtctgtg ttgtgtatag tgatacatgt tttatgttta cccaatgcct 1380
   gtactaaatc ttgtgcttgg ctttgaatta tatatgtgtt tacctatgac ttttttttga 1440
   aataaacaga acagtcatct aaaaacataa ggttttggaa aggagatgct ttttcaattc 1500
   ttaccatctg attaggtgga atgttttata gacttttggt gttgaaaaaa aaaagtttga 1560
   taacaatgca ttttgcttgg aatacccccc aatccagaaa agttggtgac ttctctccat 1620
   tgtatatatg tacatagttt tcattactag attgtaagct cctcaagggc aggaattgtt 1680
   ttatctttgt tgttcctgca gcacctaatg taaagtgcct tgcacagggt aggtgctcat 1740
   gacgtgtaga atgagtgagt aacttcctgc ctcatcagga tgaaatgtgg ttcacttccc 1800
   ctgtgttcaa cactacaaag ctactcctgc cagaagaggg cagagttctt tcttgggttt 1860
   gaactggctt ttttgggttg aaccagatca tcccacatcc ttagcactac ttaaccttct 1920
   tttcctgaag attacatatc agtgtcctgc gtttttatct aatattagag ctgaggttcc 1980
   taggaaaatt ttgaagtggg gaagattatg gagaaaatga aatggaaatt tggggagggt 2040
   ggtttgtcag tataaggccc tgtggggtaa tcatatttta gttattactt gttaaaaggg 2100
   tagcagaacc tctgtttctt taccaagtcg ctggttggcc atttctccaa ttgatgaact 2160
   accattggtt ttagctcttt ctcatttggg attcccctca attgcttaca tgattctggt 2220
   acttagagtc taatagagtt gctgtgaata tgtagtgctg tacataatgg cagttttgta 2280
   aatgtgagat aaccttgaat atgttgtggt ttgtatacag agccattagt attgttattt 2340
   tggccacttt tttattcaaa ataaataaca taattatttg taaaaaaaaa aaaaaaaaag 2400
   gccacatgtg ctcgagctgc aggtcgcggc cgctagac                        2438
<210> 240
   <211> 1739
   <212> DNA
   <213> Homo sapiens
<220>
   <221> misc_feature
   <222> (1)...(1739)
   <223> n = a, t, c or g
<220>
   <223> nbla-10798
<400> 240
   gcctgtacgg aagtgttact tctgctctaa aagctgcgga attcctcgag cactgttggc 60
   ctactggtta gatgtttctc aggatcccct cctgcgcagg ggttctctga ttttcgtgtt 120
   ctctgcccag atgggctggg ggagttgaga gtgtgcttat tttcactgcg atcatgagac 180
   cacagttctg ggttatctcc tctcatacat caagccccag aggaggcggc aagaggaaca 240
   gccacaaaca agtactttac cccacagctt agtggccagt aaacaccctg gggactagga 300
   aaaggaacca actgtaggca cctctccagg gcctagggag acaagtgtcc tctcttctgc 360
   atacatttgg gctcccctta cagagccctt tgccctggct ctctggtcct tgttgctcta 420
   acagtccaga tgtacaccca gcctcagggg gaaggcagct ctctccagac agagtctcag 480
   ggcccagcaa ggtcaggtta tctgctttca ttcagggcaa caaatgatac aaatggtgcc 540
   agggagtggc aaggccatgg gggtaggtgg gggtgtcttt ttcttttcat aaagtaacaa 600
   cagacgagac tgaggttaaa catcagaaaa aaacctctgg aatgaccttc ctcattccag 660
   gaggccctgg aataaggaag aggcttcttt ctgagggagc tttgaggaat tttgacagct 720
   gttgacatgg gatttgggaa aggtgaagct gtgactggag gggcaggaga tggtccaagt 780
   gtccatccag agatgagact cttagaatca aagtgttcag cccaggaagt cttggagatc 840
   ccaccttctg tggccctgca ccttatggga agccattaag ggggctcatc taggaattct 900
   ggttacagcc cagtgctcat cccagcgtat gctgcctctt tagggcagcc ccaagggcca 960
   gccagcctgt actctgggca agagcccaaa atggctagga atgtttgact cccttaatct 1020
   cttccccagc tacagaggaa tcttttctct gcctggtctc agaatgggac tgccaactgg 1080
   ctcattggtg ggagacacag tatcctcaaa cctgtggcca ctggcatgac agtggtgctc 1140
   tgtctccctg ggtgacaccc accctaggct tcctcctgga tgtgatgggg attgccagag 1200
   aggctcttag cataaaaggc attaggtggg catttttctg tgtgccccca aaaagctcca 1260
   tggaaacagg cacctggtag ctgcggaaca cccgtggact tgtgtatatg gtcataggct 1320
   ttgggaagac aggacgtaaa ggaaaatgag agaaacaaaa tgggtcagat agctttggcc 1380
   acagccccag gcagcctttg gggcctatga cacttagtgc ccttagatgg gatacatctc 1440
   gcctcggccc caagactcct ccaacttacc cgtcccatcc aaggcctgca cagcttagag 1500
   aggctcacag cttggcaaat gctagggctt catcagacca ctgacttgac tcagtgtttg 1560
   ttaaaatgga accactcccg ttagcctact gtttctctcc tgtacttctt gtaatgatag 1620
   ttatttattg actctggtag caggcagttc ttaaataaag atggtttctc aacctgttgg 1680
   ggaaaaaaaa aaaaaaaaaa aaaaaaaaaa aaaaaaggcc acatgtgctc gagctgcag  1739
<210> 241
   <211> 794
   <212> DNA
   <213> Homo sapiens
<220>
   <221> misc_feature
   <222> (1)...(794)
   <223> n = a, t, c or g
<220>
   <223> nbla-10805-f
<400> 241
   nnngnnnnnn nnnnnnnnnn nnnnntttgg aattcctcna gcactgttgg cctactggtt 60
   aaatttggct ctaaagttgg tgtagcagca gttgatcaga ctgaaaaacg gtttttagtc 120
   tctggaaaaa gactgatttt gcttttttta taaatattat tagatttatt aattnttctg 180
   tgctcaatgt gtaaattgta ttataattca ttgtgattta tttcactttt aatttgctgg 240
   tgttttaata aatgggggtg ttactgaatc tttcttccca cttccatttc ttttgaccac 300
   cccttaaccc tcaactgtga cggtagtagt attatcattt gtaccaaagt tttgcatagt 360
   ccctgttgac tttgtaatgt taacggagtc ataaaagcac taggcaagag aaagatagaa 420
   atttgctttt aatctttttg ccttttattt tgcacattat gcaaaaggaa aaacattaaa 480
   gaacactttn ttttaagtga gtgaaaacat ggtaaaagac atacaaggct tttatgcaca 540
   ttggtaagct aaatcaaggn cattataatc atttcctttt tattaagatt taagtaacca 600
   aatttanaat ttcagcattt caaaatgatt tatttttcag gcttaaatca tatttacacc 660
   taggttgagn taaagatntg aaatatntaa ggatgatnca gggtttcaaa tatctaatac 720
   ttttggaaat aactctggtt tngntttttn ggggnggntt taccaaattt aaggttccaa 780
   actgnaaagt tatc                                                  794
<210> 242
   <211> 803
   <212> DNA
   <213> Homo sapiens
<220>
   <221> misc_feature
   <222> (1)...(803)
   <223> n = a, t, c or g
<220>
   <223> nbla-10805-r1
<400> 242
   nnnngnnggn nnnnntttgt ggcctttttt ttttttnttt aatttaaaga tagaatttat 60
   tctctgatgg tttactcatg caaactgcac ataaaagaaa atagtacagt ttgtgtaaca 120
   ttgcaaatat aaggactgaa aatgctaggt acagaagtag tatgacatcc tgaaagtcaa 180
   aatggaattt gtgtttatac aactaataat gatttttatt tgctcagtac agactgattt 240
   acaatgaaag ttttgctaac cttggtaagc ttgttaaccg tttacatgac ttcttacatc 300
   tatgtagttt tattttacag ttgcaagaat tctgttacca aagaacctta acttgcataa 360
   gtaataagat gaaagaaaaa tgtactgaag gacttaagag tgaaaaattg aggcttttcc 420
   taacccattc aaactacaat aaaaaatagc cttcttgcag aattcatatt ttgngccttt 480
   gagataactn cttagaataa tgatatcaaa atcatgattt gaaaaactga ttactttaaa 540
   ataatgaaat taggtaaaaa ttaaaaattt aaaaaaatta tcaaataaaa atgctctcat 600
   tttaaaaagc caaaccatta aattnccant atcttcatac tnaagtctgg aatggcattt 660
   atataattgn atggttccca ntttggacnt agcaggagga tanttccntg gattatcaag 720
   gttttccata cgacctttaa aangcagctc attngaaatg gccctggnac tttnccaaaa 780
   gggganntng atccggacaa ctn                                       803
<210> 243
   <211> 789
   <212> DNA
   <213> Homo sapiens
<220>
   <221> misc_feature
   <222> (1)...(789)
   <223> n = a, t, c or g
<220>
   <223> nbla-10808-f
<400> 243
   ntttgaattc ctcgagcact gttggcctac tggaaaaaac aataggacgg aaacgccgag 60
   gaacccggct gaggcggcag cagagcatcc tggccagaac aagccaagga gccaagacga 120
   gagggacaca cggacaaaca acagacagaa gacgtactgg ccgctggact ccgctgcctc 180
   ccccatctcc ccgccatctg cgcccggagg atgagcccag ccttcagggc catggatgtg 240
   gagccccgcg ccaaaggcgt ccttctggag ccctttgtcc accaggtcgg ggggcactca 300
   tgcgtgctcc gcttcaatga gacaaccctg tgcaagcccc tggtcccaag ggaacatcag 360
   ttctacgaga ccctccctgc tgagatgcgc aaattcactc cccagtacaa aggtgtggta 420
   tctgtgcgct ttgaagaaga tgaagacagg aacttgtgtc taatagcata tccattgaaa 480
   ggggaccatg gaattgtgga cattgnagat aattcaagac tgtgaaccaa aaagtaagct 540
   cctaaggtgg acaacaaaca aaaaacatca tgtcttagaa cagaaaagac cctaaggact 600
   gggtgcgtaa canccgtnaa agaggagaaa atgaagaacc ntaagttaga agaagaattt 660
   gagtggctaa agaaatntga agnctggcta cacttgnnan aaaaagggaa tttaagttcc 720
   aantttaacc tttaaccctt ggancntgaa tgtanccnna acngtnccng nnaatgaagg 780
   nnaatgcan                                                        789
<210> 244
   <211> 804
   <212> DNA
   <213> Homo sapiens
<220>
   <221> misc_feature
   <222> (1)...(804)
   <223> n = a, t, c or g
<220>
   <223> nbla-10808-r1
<400> 244
   gnnggnnnnn ntttgtggcc tttttttttt ttcntttttt acaataaaca aaaagatttg 60
   tattagaaca tatacactca gggaagaaag aggtatcatc atcaaatgtg gaatgttgaa 120
   gaaatagtta aaataaataa agactccaag cacagctggg actggctcag gctggggctc 180
   acagaggcca ccgcacatca gctccaggct gcaggagcca ccacctggcc atactggctt 240
   cctccctgac acagcacagc tgtgcctggg acacagagtc gctctcaagt actggagcag 300
   ctagcaagct cactccccac tctcctcact tatctctgtg acaatgtcta tcaggctctg 360
   gagcccgaag atatagccag catcctggcc ctcatgcacc acggtgtcct cgccatacag 420
   cctgcaggtg gtgtgtgcaa agtcgatcat gcgcacatct acagagctgg cgccgatggg 480
   tttgtaggca taggcaccag cagactcatc aagctgattc ctctgacagg tcttcaaatc 540
   tcacatctga gtncaggacc acttngggcc cggtccttgc catcataaat gaccagcang 600
   gaacttgagt agaaacggta ggactnctgg cgttcaacac tggcttgact cagtcagctt 660
   cttgacacaa gggccaagaa ttcacggggg caagtcccgc cattggggga aaactgggaa 720
   aagngcctcc ttgaancctt naccgatagc tttcgnccgg ggacctggtc atgaacatga 780
   cttgccaatg ctgcttggnc acct                                       804
<210> 245
   <211> 1916
   <212> DNA
   <213> Homo sapiens
<220>
   <221> misc_feature
   <222> (1)...(1916)
   <223> n = a, t, c or g
<220>
   <223> nbla-10830
<400> 245
   ttctaggcct gtacggaagt gttacttctg ctctaaaagc tgcggaattc ctcgagcact 60
   gttggcctac tggggtacaa agtcatgatg attccctgag taacaaaggg aaatgaaaac 120
   tgtttgtgat cttccgtatt cactgaaaaa taaatgcttg tttagtatca aattttattt 180
   cacgaaagtt tttttaaaga acagaaactt ttccaattaa aaaaaaaaag cagacttctg 240
   gttcaagata gctcactgga atacatgttt acctctttct ttcctaaatt gcattgaatt 300
   gataggaagg atggcggaat cttaaagtga tacatgctaa ctgtagaaaa aaatagaaaa 360
   tgcacataag caaaaggaaa catttaaatg ctatctttca aagataacta ctcttaaaac 420
   cttgagtatc ttttcagacc tttttcttgg caaatgaatc catattgaca tatttgattt 480
   ttttaaaaac atggaaacgt actgttttgt aaattctttt taactgcaca tctactgttc 540
   ataaatatac ctctgtaaca taactttttg tggttctaat gtactctgtt gtatagctaa 600
   tacaagttag gatgcttttg gccagaggta acagtgtcca aatataattg gcctaagtaa 660
   cctaggaaat tgtttgacat aacacagggt tcaggggtgt cattaaagac acactttttt 720
   tgccttgacc tcagttggtt tgttttgcct taggctattc cacctctcga tcacaagagc 780
   tgctgctata acttccagtt ttacatcttt gtaaaattgt atctcaaagg cagaaaaggc 840
   catttcgtct ctcatttgtt ttatccatga ggaagatttt taacaaaagc ctccagaaga 900
   tttcccctca gtttccattg acttagatca ggttacagag aaaggcaatg tctgacattt 960
   ttggtctctg ttagaagtag actctgttga aaagaaagaa gctaagctag gtgtgaagaa 1020
   tggaattgga agcccactgc cttcccataa gaaaggttta ccataattta ctcacttttt 1080
   tctgtgttag acattttgat tatctgcagt ttattactac aagcagtggc agagtgaatg 1140
   tccttgtaca ttttgagtta catgcttaat tatgtccttg agaaagtttc taaaagtgga 1200
   atgattggtt tgactggttc atagggcttt aattatacaa tttacccctc taattagtac 1260
   tatatgtatg tgacttccct ccccctgcca gaatactcct tggtcaattg taggtattct 1320
   ttttggttta atttttgcca atgtaattaa aaaatggtat gtcattttta aaatttgtat 1380
   ttctttcatt acaaataaga ttgttatgtc agtattgtta ttggcttttc gtattcctct 1440
   taacgtgaac cgtctgttca ttgtttttac ctgttttcgt tttagcaagt agtacttaat 1500
   ttaaagtgtg aacttaatat ataagatgcc aggaccatca tattgatgac aaaaatctat 1560
   tatgtggttg tagtgcatgt cttggagtta aactcttaag tatcaagaag taaaataatt 1620
   tcattgtttc tgttactaac attaaaagtg tgcaaattgt ataaaattag gtacttcgat 1680
   tagtaaaaga aaaaaatttg aatgtttttt tttttatttt attaagcatg cccagttatg 1740
   ccaagcatag taaataaagg tcaagtagca tttataatag aggaagtatt gttatcccta 1800
   gcatgagtgt aatggtgata tgaaaaactt tgtcttgtca ttataataat aaaaaaatga 1860
   acatttatta tggaaaaaaa aaaaaaaaaa aaaggccaca tgtgctcgag ctgcag     1916
<210> 246
   <211> 1858
   <212> DNA
   <213> Homo sapiens
<220>
   <221> misc_feature
   <222> (1)...(1858)
   <223> n = a, t, c or g
<220>
   <223> nbla-10833
<400> 246
   gcctgtacgg aagtgttact tctgctctaa aagctgcgga attcctcgag cactgttggc 60
   ctactgggtt caaaaaaaat gtatagaatt atccatgcaa gggattctgg tataggaggt 120
   gtgggcggtg agcaaaaaga gatagtggac gtagactact tgtttaaaaa gcctgcagtg 180
   tatagaagga aaaaaaaata agctgaccat cagttattgg ctgggagatg cccaggaaag 240
   acatgccttg gctcacaccc tggggcagat tctgagggca ctgctgctgg aggctttcag 300
   ctaactgtgc ccatcacagc tgagtgacaa aatgtcttgc agggagatct gagcggcaca 360
   tgtccttggt ataatttatg ccactataaa caggacacta acactgaacc atttatgcca 420
   ctataaatag gaagctaaca ctgaacctga tgaacgaatc catcaggaac aaaagacaga 480
   cagacacgca cacacacaca tgcacacgca cacatcagca tggctgtatt tggccatcta 540
   tcagtagtca acggttaatc agactgtata ttgggaacat acatggcttt gttttcttac 600
   agacttaaat ttgaagaaca gtgatgatga actcattcta tatcactttg gtttaaaagt 660
   tgcttagcaa tgggacaaaa acaaaagcaa ctaggtttat atttagtgct ctatctatat 720
   gcatctggag cacatttcgg aaataattca aaaaggccaa ttcatttcat gccttctgct 780
   actaaatgaa tgtgggagtc aagctgctat tttcacagtg caagacattt attgttgact 840
   taaaaaaact ctcccacttg cacataatca ctatcctgac aaatgacact atagagtttc 900
   agctccttaa gcataaggta gaacaatctt taagaaataa atattaggcc acaggagtta 960
   aacataaagt atcatccaag cacagattca aagcatttaa acacatacat tattctcaag 1020
   tggaccagtt cttcactgag tatgtgttct ttaggtttgc aagactaatc ttatgtctca 1080
   cccctcctca attgaacagt attggcactt tgattattca gtcagtgtag gactttttaa 1140
   ctccgtgcca ggctcatttg cagctatcag tgctgataca acttcatgcc atataatact 1200
   tttaacagac tacttccaca cccctgcaaa aaaaaaaaaa gttaataagc aactgatcaa 1260
   ttaaggttaa tggtagattt aggcctataa attgtaccca tcttagtcat ttctgcttcc 1320
   taaaaattta tacaagaaca gtttttttct ggggtaagag ggtgtgattg acaccctaat 1380
   tctgtttctt ctgtttagat tgtggaaata taatcggacc gtttcagggc tctatgagtg 1440
   tgatccttgg acatgaaaca gtttccgagt acagagtatg aagtgctggt gagcttttgt 1500
   caccaacagc acttaagaag tattttggcc aggtgcagtg gctcatgcct acaatcccag 1560
   cactttagga gactgaagag gcaggatgat tgcttgagct caggagttca agaccagcct 1620
   gggcaacata gtgaggcccc atctctacta aaaatttaaa aaaaaaaatc agccaggcgt 1680
   gatgtcacat acctgtagtc cctgctactt gggaggctaa ggcaggagga tcacttgagc 1740
   ctggatgatt gaggctgcag tgagctgtaa ttgtgctact gcactctgcc taggagacag 1800
   aaggagaccc tgtctcaaaa aaaaaaaaaa aaaaaggcca catgtgctcg agctgcag   1858
<210> 247
   <211> 1934
   <212> DNA
   <213> Homo sapiens
<220>
   <221> misc_feature
   <222> (1)...(1934)
   <223> n = a, t, c or g
<220>
   <223> nbla-10857
<400> 247
   ttctaggcct gtacggaagt gttacttctg ctctaaaagc tgcggaattc ctcgagcact 60
   gttggcctac tggagtgggt gtgagtgaat gggtgggtga aagagtatac atgcgtagat 120
   ggataggtgg atgggtagat agatggatgg atgaaagaat gaatgggtag gtgggtagat 180
   gggtgagtgg aagagtaaat ggatggatga cctcaaatgg ccccaaggga ccactgggct 240
   ctgccttgcc ctgtgtattt ggtgtctatg gtttcaggcc agggctctaa gtggcacgag 300
   atgctcccca aacctctcgc tcttccactt tccaccaccc tttgctgttt tgccaccttc 360
   tgaaagatac caaagagaat gaaggttaaa tctgaccctt cctcctccct gccccaccct 420
   gggcccaaca ttcagagaag agttaagtga cagagcctcg ttctcctgta tgaattaata 480
   attgattcag taacaaatca ctaatattgc agtagtcgtg gtgaataaga taatgaatgt 540
   gttctgtcca gggagtgtca tgttgtgagg attcagctga aattattcat aaatgtgcta 600
   cttttcctca tctccaacta ctgctagttt agctcctctt tttaatcttc ccaacctgtc 660
   acattcgatg gtagaaatcg tcaggctcca tatacagcag aatattaagg gggaactgcc 720
   aaggcattat gtcaaatttc aataggtaat aaaaacaaag tgcttgaaga ccattttcca 780
   cttctcttaa agttaaaaaa agaagacttc caaaaacctt tctagatggg cactttctcc 840
   catcattggg gggaaaaaaa cctgagttca aggtattcag aaacttgggt atgatcccca 900
   ctttaccaca aagttgctgc gtgactttga gccagtactc aaccatcctg ggctaagtct 960
   ccaatctcta aacaaggggt gcctccctgc ttttctcctg ggtgtcatga tgtaattgtg 1020
   aggtgatggg aaagaaagtg ttctaggcca ctaatagata gctaggctgg attttctcca 1080
   ctatcacacc ctacaccaac caggtcagca ggttcactat tctctaaaca tgccttccat 1140
   gtggccacca cagagagggc ccctgccctc ctgttcaccc tccatgtctc ttcccttctc 1200
   catctgaccc aaccagcact ctgggctcag ttcagccacc acttgcttca tgaaaccttt 1260
   cccgatcctg cctgacagac atattttctc ctactccaca tctccccaga gcaccctggc 1320
   ccactcttac acatacctct tacactgctt tccacatacc atcttcccta gtagactgca 1380
   agcatctcaa gaatggaagt tgtcttctac atctttgagt acaataagaa aggagaaata 1440
   tttgtagaat gagtccactg gtggatgagt ggggagatgg atagatagat gagtaggtgg 1500
   gtggatcggt ggtggatgag tggatgggta gatggatgag tggatgggta gatggatgag 1560
   tggatgaatg ctggatggat ggatgaatta gggccgggct ttgtggctca cacctgtaat 1620
   cccagcactt tgggaggccg aggtgggtgg atcacgaggt caggagattg agaccatcct 1680
   ggctaacacg gtgaaacccc atctctacta aaaatgcaaa aaattagccg ggcacggtgg 1740
   tggctgcctg tagtcccagc tactcgggag gctgaggcag gagaatggca tgaacctggg 1800
   aggcagagct tgcagccgag atcgcgccac tgcactccag cctgggtgac agagcaagac 1860
   tccgtctcaa aaaaaaaaaa aaaaaagaaa agaaaaaaaa aaaaaaaaaa aggccacatg 1920
   tgctcgagct gcag 1934
<210> 248
   <211> 779
   <212> DNA
   <213> Homo sapiens
<220>
   <221> misc_feature
   <222> (1)...(779)
   <223> n = a, t, c or g
<220>
   <223> nbla-10860-f
<400> 248
   gngnnnnnnn ttgnnnncnn tttggantcc tcgagcactg ttggcctact gggtgctttt 60
   gtgcacttta tctggggtaa agttgtaaat gttcagtttc ttggtaaagg atattatgtt 120
   ttaagttttg gtggccattg tccaattttc tttaaagagc tgccccaagt tacagtcaga 180
   ttaacagtgt agagcaccag ctttctcatg tccccagcag cactgagagg cggaagatag 240
   tgcctcactg atggtttatt tcacacttcg ttacttatgg ttgagagaga atatttttca 300
   gattttattg gacattgata tttgtaaatt gttcattcct tttgcccagt tttctattga 360
   gtggttcata gtttctcatg ggtatccaag agttctggat atgtagaggt ggagggtcaa 420
   tctcatcact tccttgtttt aaaaatcttc catggttttg tcatcactat gggctcaaac 480
   gccgtggtgt ggcatacaaa gccctcactt ctgaaccacc ctgcttccag cctcatctcc 540
   catgatccct gccacgcggt gccgtttgcg ctttcatgtc caagtgcttc cttcatgttg 600
   gtttcttcct tacaatgccc tttccttatt gccaacagag tgcttctaca ccttgaggat 660
   cataagaacc tgngtttcca ttttattntt tatcataatt ttggggccta tcttcccaaa 720
   gactttgatt cantggncan tgtgangnct gatctcattt tctttatctg aacgatgac 779
<210> 249
   <211> 788
   <212> DNA
   <213> Homo sapiens
<220>
   <221> misc_feature
   <222> (1)...(788)
   <223> n = a, t, c or g
<220>
   <223> nbla-10860-r1
<400> 249
   gnngnngnnn nnnnttngtg gccttttttt ttttccnttt tttttttaag acagggtctt 60
   gctctgtggc ccaggctgga ctgcagtggt gaaatcacag ctcactgcag cctctacctc 120
   ccgggctcgg gcaattctcc cacctcagcc ttccaagtag ctgggactac aggcacacac 180
   caccatgccg agctaatctg tgcatttttt tgtaaagaca aagtgtcact atgttgctca 240
   agctggtctt gaactcctgg gctcaaacaa tctgcctgcc tctgcctccc aaagtgctgg 300
   gataataggc atgaacaatc acacctggcc aaaatctcca tttctccctt atgaaacata 360
   ttctcataga atcttaggaa taataggcct taaagtctat aaataaggga aaaaatccat 420
   tgacattagg aacctgcgaa aaaaatacat aacaaaacaa tcagtgtttc aggcacctaa 480
   attgtgaaca aatcacacta tctacacgta gctatttcca tagacccgta gaaatatctc 540
   tgcctaaaga cacagacaag tatgttctct ttctcacata tcaaggagaa aaacagtaga 600
   acgttgttgt acactgncta agaaacaatt ggtctttaaa acagactcaa taaaagcaaa 660
   atgnctttnc aaaagggtct aatacttcct gccttctaaa ggggtctaat ggcatttttt 720
   tttaattaag caaaaagtga atagaatgga aaaatttggg gtcttcatat taactactna 780
   catntacn 788
<210> 250
   <211> 789
   <212> DNA
   <213> Homo sapiens
<220>
   <221> misc_feature
   <222> (1)...(789)
   <223> n = a, t, c or g
<220>
   <223> nbla-10875-f
<400> 250
   nnnnnnnntn nnnnnnnnnt ttgnnnncct ngagcactgt tggcctactg gactttgcac 60
   atgatgtatc acgaagctac agcttgccat gtgactggag atttagtaga acttctgtca 120
   atatttcttt cggttttgaa gtctacacgc ccttatcttc agagaaaaga tgtgaaacaa 180
   gcattaatcc agtggcagga gcgaattgaa tttgcccata aactgttaac tcttcttaat 240
   tcctatagtc ctccagaact tagaaatgcc tgtatagatg tcctcaagga acttgtactt 300
   ttgagtctcc atgattttct tcatactctg gttccctttc tacaacacaa ccattgtact 360
   taccatcaca gtaatatacc aatgtctctt ggaccttatt tcccttgtcg agaaaatatc 420
   aagctaatag gagggaaaag caatattcgg cctccgcgcc ctgaactcaa tatgtgcctc 480
   ttgcccacaa tggtggaaac cagtaagggc aaagatgacg tttatgatcg tatgctgcta 540
   gactacttct tttcttatca tcagttcatc catctattat gccgagttgc aatcaactgt 600
   gaaaaattta ctgaaacatt agttaagctg agtgtcctaa gtgcctatga anggtagcca 660
   cttcatcttg cactggtncc caaactttgg actgactatg ccagactcaa gctgctatgt 720
   caaaaaactg gatnaagctt ttgggggaaa ancctggttt cgnanaatnt attaaatgga 780
   tcctaangg                                                     789
<210> 251
   <211> 791
   <212> DNA
   <213> Homo sapiens
<220>
   <221> misc_feature
   <222> (1)...(791)
   <223> n = a, t, c or g
<220>
   <223> nbla-10875-r1
<400> 251
   nnnngnngnn nnnntttgtg gccttttttt ttttntttta tttttacatc aagtgtgctt 60
   tatttcctcc acaggtattc tgttaaataa agcaccattt atatactgcc aggccacagc 120
   taaagaggat tctttacaga atcaaatttc ttgtggttgt tccgtataca agtaaactta 180
   attttgataa taagaaccac agcgatcgga ggcaatctgc ctctataagg tacaaaactg 240
   gcacagagga caccatatca tacacagtaa aaatgctgta agtttaaatt acattgtaca 300
   gggctaggca accctgttct tcccagacag ccatattaaa tgaaagccac taaagtgaac 360
   tcttaattac ataaaacata tccattatct gattgccctt taggaagtat actgaagatg 420
   caagtttttt tcatctggag ttctgcctga ccaagaatta agcctataaa tctatcttgc 480
   cattcaagca gagagcactg gacaaactga agcacaaaaa caaataagca aaacttatac 540
   aaacagcatg gggggttggg gtgagggact taaaagtaga catgctacac ctaatgtcaa 600
   gaagcanctt ggtttccttt gccagatatc cttgtgacga catggattgn agattcaatg 660
   gtcctacaca nggtatctaa aatgncatgg tgcttcatat gactaaagag tctttgggaa 720
   tgggcccact gagagataaa gcaccttaag gtcctgggan ggcaaaactt gggcattaca 780
   acttttnaan n                                                  791
<210> 252
   <211> 789
   <212> DNA
   <213> Homo sapiens
<220>
   <221> misc_feature
   <222> (1)...(789)
   <223> n = a, t, c or g
<220>
   <223> nbla-10881-f
<400> 252
   nnnnnnnnnn ttgnnnncnn tttngantcc tcnngcactg tggccttttt tttttttttt 60
   ttcaaattcc aaccagaagc taaatacaat tggaaactgg taagcactag ttttactcca 120
   aaggagtagg atcattcaga tttactccaa taaaagtatg caacccttaa gcaaagcttt 180
   tcttcattta aaaggagaaa aaaaaaaaac ctatacagta gtctttcctt atgttcattg 240
   cacaaaatga gttctgcttt tagaactttg acactcaatg gttaatttta caatttaaga 300
   ttccaacttt ataacctttt ttctactcca aaacaccctt gtaaagtttt tctttaggat 360
   ggtgtaaaaa ccagcatttc tgcacaattc actggaattt ttttctttgt aataaaaatc 420
   tcttctctgt aaaaccaaaa acaaaacaaa acaaaacaaa acaaaaccaa aagaaaagtc 480
   ctctacctat catggtttct gcagctatgc atgtatttct gttttatagc tgctttatag 540
   ctacttcaga ctccagatct gctttaatgn gtataactgc atccacacgc agcagaatac 600
   tcttacaata gcaacttggg gaaagagatc tggaaaaaaa aaatcatgag taccaggaaa 660
   caaacatggc ccagtaaaat atgaggcaaa aatgcctaca atgagatgcg tntttcattt 720
   aagatttctt tccatgggtg gaatttcctt tttttaaacc agtntgggca ttanaaagna 780
   ttgacagng                                                     789
<210> 253
   <211> 784
   <212> DNA
   <213> Homo sapiens
<220>
   <221> misc_feature
   <222> (1)...(784)
   <223> n = a, t, c or g
<220>
   <223> nbla-10881-r1
<400> 253
   gntgnnnnnn ttngtggcct tttttttttt cnttttggaa agcaaggatc acacttcccc 60
   ctccctgttc cttaatccct tttctaaaaa ggggggaaaa tccggatgga ttttagggat 120
   tggtctggtg tcagctgtgt tttattgcac acctaaatcc tgattatagg cttttcattt 180
   ctccgcaaag cctttatttt ggcagttaag ccaaatgtgt tttccagaaa gttagttatt 240
   ttctcctctt tctttccttt ctttcccccc tttttcccgt ctgaccccaa acgttattgt 300
   ccaaacatga ctggacagca gcttttgttt cttgaccctg taatatgaca gtctgctaat 360
   attgacagaa ggtgcagntt ttgggttata gtcgtgattt tcgctaatca atcatattag 420
   caggaaaaaa aatgacttgn ttctgttgta cttgagtctt aagaaaaagt gcccatagtt 480
   taagtgacaa tttccaaagg ctttagtacc acctgtattt caaaatgggg gacccaaact 540
   cccggaagaa acaagctctg aacagactac gtgctcagct tagaaagaaa aaagaatctc 600
   tagctgacca gnttgacttc aagatgtata ttgcctttgn attcaaggag aagaagaaaa 660
   agtcaagcac tttttgaagg nctganggta taccagcatg acaaataatt atgaagaaaa 720
   tatcctgaaa ggtgtgcgaa aatncagctt ttccttggna agtncctaaa cctttacaga 780
   agga                                                         784
<210> 254
   <211> 2690
   <212> DNA
   <213> Homo sapiens
<220>
   <221> misc_feature
   <222> (1)...(2690)
   <223> n = a, t, c or g
<220>
   <223> nbla-10895
<400> 254
   gcctgtacgg aagtgttact tctgctctaa aagctgcgga attcctcgag cactgttggc 60
   ctactggaaa ctccgagtga gccagtattc ataaaaaatg ttaaaaagca gaagaaagga 120
   cacaagataa acaataattt ggataattcg taaattggta tttcattctt gacttcttga 180
   gaattcattg tatacgctac tcctcaaaaa taaggaaaac cataaacaaa aattctggtc 240
   tgaaatatct tggcagtaat gtgtttgctc tcccctgtcc tgaatatttt aggctaactt 300
   tcaattaaaa tggattgcaa ttaaattatt ttgggcagaa atgtgtctta acctttgaca 360
   aagttgatca tttcagcatt ttcaaatgct taaactattt cttaaaatga cacaaattgt 420
   ggcgttgcca tttaccgagg tttacatgct cttgatgctt aaggattcgg ctttcttttt 480
   tcccccgcat ttattcactg aaaacatgat tgattcagtt ttgtacctat gcagaatgcc 540
   actgacgtga tgccaaatat gccctattta gaaaaaataa aaattaaaag gatatttttc 600
   tgcacagtca aaattatgca gatgtggatt taaaagacta catgtcaaaa aatacgcaag 660
   taaagtttac ttactccatg tttcctttta cactcacaga cagcagattt acaggcacca 720
   aacagccttc atcaagacaa tgaaatgccc agtgtggctc tttgatgact tcattctgtc 780
   tttcacgtat gcccaaaccc tcataattgc aggtgctttt tctgtaccca attatttggg 840
   gtctctgtgt tattcccagg catgcttagg tcattctggt ttcccagggc tccctttagg 900
   gcctctagat atccaagttg tcacagtctc atttctcagc ttagtctttc cagactctcc 960
   aacataccca tgttcaattc tacatcacat ggcatcaaga cagggactca acattgccta 1020
   aagcagtttg ggtcactaac ttatttgata aactaatttc tgttaaggct ctttgttctt 1080
   tcattctcag ctgaaatgtg tgtatggcaa caggggcctc ccatggacag tccacttttg 1140
   gaagctcttg gatggtgtca ccatgaaaga gtagagaaag atgtttagga atgtgcccac 1200
   ttgatgcgag aattgtgaga acagtccttc atagatggac aatggtgggt tcattaaata 1260
   gcactctggg aataaagtct cttggtaaag taagcaagag aatattcata agctggccag 1320
   tcgttcattc ttccatgcac acatttgttc agtacatttt attgtgctag gtcctggaga 1380
   tacagaagag aggagttaga cactccatct ccatgtgtgg tttacagtct agcagggatg 1440
   catccatgtg agagagctgt gcacctgttc agttttgctc acctctagcc cagttgaaaa 1500
   caaaattatg gacaacaatt aatctaataa aaaaataata agttagtcaa gtgttgattc 1560
   agatcacatg cctctcattg ttcgtctctg gacaaagttg tgaagaatgc tgcatctcag 1620
   actgcccatg ttattgactt tttttaaaat cacctggtaa tccctttctc ctcatatcaa 1680
   atgcaaacac agcactctct cagactcact tataagagtg cgtttttatc cattcttact 1740
   cttgcttttc cttttattct ccctctgccc agctataaca aattggattg tgattttgtc 1800
   cacaattatt attcctaaaa tgacatttat atttggaatt gtattttcat tacagagaaa 1860
   caggttaaaa atcaagtcac aattgaaata gggaaagaat accctgaaag atatagttta 1920
   gcagtaagtg tatttgtaca ctgataaact gatcttccga agagactgtt tatgattttg 1980
   gcagtgcctc attttttagg tctttgtctt taaggaaccc acgtgtggtt ggggaaagca 2040
   cctcaatctc tgcagttcat atgtagaaac tcttataagc cttgtgagag gtctgtgttc 2100
   ccttcctgac tgtcctacac ctactatctt gaacatcaga aacactgatg ttcaagaact 2160
   ttgattaccc tcatttatct gggagctgtg tgacatctga ggcacagttc taagtagaag 2220
   tatcattcag aaccaggagc aagaatcaga tactcgtgag taaaggtcca aaactgagtg 2280
   gcaatgtaat acttttggtt ttcaaataaa gagattcata agatttatct aatcaatcta 2340
   ctaatgcatg gctctatata agtttgctag cataatagat taatcatggt aaatctattt 2400
   ctaatctatt ccttataact tactatttct ccttctacta tgacatcttc attttttgtc 2460
   tttaatgctc atccattttt tcttctctgt acgtgaatct tagagcccac aggtaactga 2520
   cacagaatgt tgagaatagt aggatacaaa aagtagtggc attttggaga tctggtagtc 2580
   ccttagacaa tattccttac atttgtctat ttggtttgct ctcctgcatt ttgtagactc 2640
   attcctagca aaaaaaaaaa caaaaaaggc cacatgtgct cgagctgcag           2690
<210> 255
   <211> 2457
   <212> DNA
   <213> Homo sapiens
<220>
   <221> misc_feature
   <222> (1)...(2457)
   <223> n = a, t, c or g
<220>
   <223> nbla-10909
<400> 255
   gcctgtacgg aagtgttact tctgctctaa aagctgcgga attcctcgag cactgttggc 60
   ctactggctt gcaataaaat aatgcaaatg aaaacacatg agggtaaaat aataaaataa 120
   gataaaaatt atttaaagca gaagccgcta gtcagggtta gtaaataagc ttagtggagt 180
   tcatgaaccc actggaattc catgtatatt tttgcatatc tgttttatgg aggtggtcct 240
   tcatacggcc aatcaattca ttgattctga gtgatttgat tatgatttgc ttgcctaaaa 300
   gaataatgtt tagatgattt tgagcatcta agaaaacctg atagttataa ttttgaactg 360
   gtttgcctta aagttcttga atataattta ggaaggtatg ttaagacaca catatgtgtg 420
   ggtgtgtaca gggggagtac aaaaaaaacc acatttttaa gttcagaaaa aaaatcattg 480
   caatttgttg taaacagcat ggactaatga tacaggatga tgttggttga attttcagga 540
   ctagcaatgt aactttgcaa tggatacgta gatgccattc aaataagtga ttctgttatt 600
   tatcctgttt ttttaaagta aaaatattaa acataactta gtttgtataa gaaaaaataa 660
   ttgcaggagg taaatgtaac ctgtctgaga taacacacaa aactctgatg attgtatttt 720
   ggagttaaga ctatgaagct aaaaaatgtg tgtgcacata atttcaaata ttaggcccaa 780
   gtaattttat tttcggaact gctcattaat tatgggagca ctcagtgttt caggaagtgt 840
   taagacttca gggtttcagc aatgaaattg ataaggctct tccctagatc taagaagaga 900
   cagacaataa acattcaaaa gcaagaacat aagatactga taaattctaa gaagaaaacc 960
   cagtaggatg atatacaggg gtgtgactag gaggtcagag gaggttgctc tgaggaggtg 1020
   atgtttatgc aaatctgaat gataggaagc ccagcaagag atctgggagc agagccttcc 1080
   agggaaaggg aaggacctgt gcaaaacccc agaggcgaag tccatctagg cttgctcaaa 1140
   gacaagaaag aggacaagag cattaagtgt ggggagagtg gcaagaggca agatcatcag 1200
   gcaagggcgc ctcagacaag accacgccaa ggggagagca cagggcagag caggactgtg 1260
   tggaaattcc aacgtgaatg acttccaaaa tcaggacaca ggctctctcc ccagcctgac 1320
   ctcttctggt gcttaactaa cttgttagca aaactccttg ggcacagcca ctgagtcctc 1380
   cagccaggct gcccctttgt attgacatgg cagggataca ggaggcacga gagactgtaa 1440
   ctttctagag ttagaatgtc tctagtaact ctagagacat tttagtgcta acttacaatt 1500
   gatctggcaa aggaagatag gcagaactat taaagtgttc aatttccttc cagagagatt 1560
   cttccatttt ctctcattac aaaaccagaa gatcagctgt gtggggccat cagctcccag 1620
   cctaaggtcc tataacctga agcttgaagg caatcagtac ctctgcttta taattgatca 1680
   ctttgaggag ccaaaggaaa gagtgaaaga ttgggactgc tttgagtgga gatggcactg 1740
   aactcgttgt aataactaca aatgcaattt aaagtaaaag catgagtata taaattgaaa 1800
   gggcaggtgg acagaaagaa gagactgact cctagacagg tgctgagaaa gcagtgtaat 1860
   taaaaagata aggaagggaa aggagctaca acatatacca cacacacaca cacacacaca 1920
   cacacacaca cacgttatca gacattcaaa aaattagatc ttagactcca caatacaaat 1980
   cccagaggac aatggattac agtgttgaca ggggagaaat attgtgataa aatcattgca 2040
   tacttagtta tgttttcatt gttaaagaaa taaacagacc attttgaggt agttaaacct 2100
   cagagaagaa tagcatgtat ttactcttct tgaaatctat gttggcttta tgccccagct 2160
   gagataggaa tcaaaggtga ggttgaaaat aaatagggat aatataaacc gtccaccaga 2220
   ttgtgttaaa tctaaagaat cgttcagtat tttattgtat ctcactgtat gtgaaaagaa 2280
   acaagtttca ccaaacaata cttagcctta ttttgtatat gcagtgcatt ataatatttt 2340
   ctattttgtt ctgtctcttt ttttgttcat gcttgacaca aaacattaaa ttggttttgc 2400
   aacctacaat ttgaaataaa aaaaaaaaaa aaaaggccac atgtgctcga gctgcag    2457
<210> 256
   <211> 821
   <212> DNA
   <213> Homo sapiens
<220>
   <221> misc_feature
   <222> (1)...(821)
   <223> n = a, t, c or g
<220>
   <223> nbla-10919-f
<400> 256
   ttnnnnncnn ntttgaattc ctctgcactg ttggcctact gggagatatg actgtcagtt 60
   gcagtgagtt ctgcatccca agtggaggga attgggtttg tggcaaagag cttgacccag 120
   aaatagatgg tgccccccaa attgtctcca catgaagatg tactgatgac gccccagaaa 180
   tgctgcttcc atatcagctg ctgctagcgc cagcgcagac tctcagggag tcaccacagc 240
   ttgtcttgtg cttggtgagt gagggtctct ctactcagtg tcagacatct acaggaaaga 300
   aacaactggt ggaaaagagc aataaattgc ccggtgctct gcagggctgg aatttcaaac 360
   agaaagaggg aataagatcc tgtgattttt ctcacctgct tttccacgca ctgtggtcat 420
   cactgtgcaa tctacatcta gtatgaaatc cacacatagg agagctgggg cacaagggga 480
   ctggaggcag ttgctttgca agatggctga ngagaaagca cactgggaac acaatccaga 540
   atggtctaac aataagtttt caagtgaata aaccactggc aagacaattn catgtgcacc 600
   tttanggtac ctatatagnc tnctaagaag atcaggatga aagacctaga tgatcccctg 660
   aggataaaac cttcatccct aaaatgattt ttttaaatcc actgcnttac tgtccaggag 720
   gcaaaatgtt tttctggctt tgggccaant cctgctgana cnggatttta ctcttggtac 780
   caaatctatn tncctaangc anggnttcaa gcctganttc t                  821
<210> 257
   <211> 837
   <212> DNA
   <213> Homo sapiens
<220>
   <221> misc_feature
   <222> (1)...(837)
   <223> n = a, t, c or g
<220>
   <223> nbla-10919-r1
<400> 257
   gnngnagnnn nctttgnggc cccttttntt tttttntggt ttcagtagtt tattcacatt 60
   tttttttctc tgnttatttt aatatgcacc acgtgtaagt tagatgaggg ctacggnaaa 120
   aacatttttg atgactgaaa tcttactttt ccttctaacn atctggagca aatcacatat 180
   tcattgntca tgacgaataa aatgatttct gntacaatgn tgagaaaaaa tgngntcatt 240
   aatatgaaca catgtctaca gagatgattt acacnatgca agtaatgaaa gagttcttcc 300
   ttaactagnc tactatcaaa ctgaaaagat gaatcttaaa attcccttga aaataccata 360
   tgattggtca tgngtcagta aaagaaagac ggtccccatn accaggccac caccattaga 420
   gagctatntt aaatacttgg gaaaaaatat ggaaacaccg gtggtgatga ttaaattgat 480
   aattacntag atncactgnc ataatgatac atatttggtc tatgataagt gaaatcaaaa 540
   ataagatcaa nttggntata gaaattcctt aaagaacacc accacttaat tatttcaaac 600
   ttggactatt ccaagaatgn cnagaaaaaa atttgaaagc nctggcttaa agccaagcaa 660
   tctggatccc caatgnagtc agcatgnctt gggcttggct ggtaaccctn tggggacatt 720
   tttgacattc aggacttggg ancctaatgg tnnaaagaat ggcnttcatt tngntactna 780
   aganttttac ttgctnggna cattcactgg tattnncntn nacnctcaat tttcnng    837
<210> 258
   <211> 1788
   <212> DNA
   <213> Homo sapiens
<220>
   <221> misc_feature
   <222> (1)...(1788)
   <223> n = a, t, c or g
<220>
   <223> nbla-10927
<400> 258
   ctgtacggaa gtgttacttc tgctctaaaa gctgcggaat tcctcgagca ctgttggcct 60
   actggttgct aaaagctgga agttgggcta aaaagattgc tgtgaaaaga gctcttaaat 120
   ctgaagatct tagcataaat ctaataagtt ctgaatttgt tgggcttgat attcagcaga 180
   aacttacagt cttttactta gaacccaaga ggtttggaaa tcaaatcact atgcaaagaa 240
   aatctgaaac acagatttcc cattctaaac attcagacat atctacaata gcaggaccta 300
   ataaaggaac gactgccagc aaaaaacctg ggaaccgaga atgcaatcat ctttgtaaaa 360
   gtaaacatac atgtggacat gactgctgta aaattggagt tgcacagaag tcagaaatta 420
   aagagtcaac aatttcctca tatttatctg atttaagaaa caggaatgct gtttcatctg 480
   ttcctccagt taaacgtctg aagatacaga tgaataaatc tcaaagtgtg gaccttaaag 540
   agtttggttt tactccaaaa ccttcccttc ctagtatatc aaggtcagaa tatttaaaca 600
   tatctgaatt gcctataatg gagcagtggg atcagcctga aatctatgga aaagttagac 660
   aagaaccatc tgaatatcaa gacaaagaag ttttggatgt gaactttgaa ttgggaaatg 720
   aagtttggga tgattttgat gatgaaaact tagaagttac tagcttttca actgatactg 780
   agaagacaaa aatatcagga tttggaaaca ctttgagttc aagtaccagg ggaagtaagc 840
   taccccttca agagtcaaag agcaaattcc aaagagaaat gtcaaacagt tttgtttcat 900
   cacatgagat gtcggatatt tctttatcaa attctgctat gcccaagttc agtgcatcct 960
   ccatgacaaa attacctcaa caagctggaa atgcagttat tgtccatttt caagaaagaa 1020
   aaccacaaaa tctgtcacca gagattgaga agcaatgctt tactttctct gaaaaaaacc 1080
   caaattcttc aaattataaa aaagtggatt tttttattag aaacagtgaa tgtaaaaagg 1140
   aagttgattt cagtatgtat catcctgatg atgaagctga tgaaatgaag tctttattgg 1200
   gaatatttga tggtattttc taaaacaaac aaatactttt tatattgata agagaaagaa 1260
   taaagacacc taatcacaaa gcatgctttt tatttttaaa aagtagttca taattgttcg 1320
   gatttttttc tcctgtattt gattagcttt gttcactttt tgcaacaaaa tgtatgggcc 1380
   gatttatttt cccattatat tgtaacttct tgaaaagctc ttttacagtg gctttgtttc 1440
   aataagccct taagttaagg gctgattgca cttagaaata agttgcactt agaaataatt 1500
   atttggctat taattggatt ttgacaagaa ccaatgtaaa atttcatttt tatattttct 1560
   taacaagaag aaaaatattc tatgaaagca aagtaatgct aacttaatga atctgcttat 1620
   tgtatggatt aatcactaat attattccca tgtattttgt tttactaaag atttttaaga 1680
   atccagtggc tatttgaacc tactgttatc taactttgag caacaataaa tgaaacatta 1740
   atcagtataa aaaaaaaaaa aaaaaggcca catgtgctcg agctgcag             1788
<210> 259
   <211> 815
   <212> DNA
   <213> Homo sapiens
<220>
   <221> misc_feature
   <222> (1)...(815)
   <223> n = a, t, c or g
<220>
   <223> nbla-10934-r1
<400> 259
   gagnngnnnn nttgtggctt tttttttttt ttactttaca aatagtttat ttccccttca 60
   caaattcatg cgcctctgac cattatgcaa ggcaacatga attaaatact tatgtcagaa 120
   catatttgtc ttacattatt caagataaag tggattttaa aagcaagtgg gtaccaatga 180
   tggggagatg aggagaatag ggcaggcagc aacagggcaa catgcatttt tcaagagtgt 240
   ttattaaaat aggcagtaat cagtacatgt acatcatatg agcagttttt caaaattagc 300
   acttccagga gaggggctac atctcagttt tttctgtctg tacagtaaaa tgccaaaagt 360
   acttccctaa agtacaaagg catttcccta gtagtcttgg taccagtaac aatatgatta 420
   ctaaacatct ccaatgtggt tttcattaca aagaaacatg tttcacataa aagcttcata 480
   atataatcca gagacagaat ttgtgcatgc ttacaatttg aagccaggct gaatatattt 540
   gatatatatt ttttcatttc tccagtgttt tttattttta taaatatgca cctagtttcc 600
   taccagttta aaacacacac gaaacactgn ttttgagcac catgattgcc ttacagtagc 660
   ctctttggga ctncaatcct agttcaagga ggggagtcct aatttctatt ctgacttgnt 720
   tcnttcccca aatnatgtta gnggttgccc aaatccatca attttnagat gccaacaaag 780
   ggttttgggc naangggcca atnataattt tgant                           815
<210> 260
   <211> 835
   <212> DNA
   <213> Homo sapiens
<220>
   <221> misc_feature
   <222> (1)...(835)
   <223> n = a, t, c or g
<220>
   <223> nbla-10939-f
<400> 260
   gnngnagngg nngtngggnt tnnnntttga ttcccttgca ctgttggcct actgggcaag 60
   attgcactac ggaaggacct ggataacgct gaggaaaagg cagatgctct gaataaggag 120
   ctgctgatga ccaaacagaa gttgattgat gcagaagaag agaaaagacg gctggaagaa 180
   gagtctgctc agttaaaaga aatgtgccgt cggaaactcg acaaggcaga atctgagatt 240
   aaaaaaaaca gttctatcat tggtgactat aagcagattt gttctcagtt gagtgaaaga 300
   ttggagaagc agcagacagc caataaggtg gaaattgaga aaattcggca aaaagtggat 360
   gactgtgagc ggtgccggga atttttcaac aaagaagggc cgtgtaaaag gcataagctc 420
   aaccaaggag gttttagatg aggacacgga tgaagagaaa gagacgctca agaaccagct 480
   gagagaaatg gagctagaac tggcacagac caaactccag ctggtggagg ccgagtgtaa 540
   gatacaggac ttggaacacc atttagggct tgccctcaat gaggtgcagg cagccaagaa 600
   gacgtggttt aacccgaaca cttgagcttc ataaagacag caacccgggg ttcaagggaa 660
   agagacttgc tgagaacagc ttgncggctt ccgcaccttn agaaaacacg acaccttttg 720
   gtgccttctt tggcaagatg tgtgaatctg tgacttgtcc angancagaa tgtacctaag 780
   tcaagatcca taacgcatgt tggtaaggtc acttggacca naactttgtg aacan      835
<210> 261
   <211> 824
   <212> DNA
   <213> Homo sapiens
<220>
   <221> misc_feature
   <222> (1)...(824)
   <223> n = a, t, c or g
<220>
   <223> nbla-10939-r1
<400> 261
   gnagtggggt tnnnttngng gccctttgtn nnnttcntta aaggtgctaa aatccattcc 60
   atagctttga agtgagaact tacagcccat ctgaacagtg aggtttgtac ccaggcatac 120
   tctgaggagg gcccaaaggc ccagcccatc tgggagggac aggcccggag gatggagacc 180
   tcgcagcatg tgcaccctgc gcagacattt acagacacgc aactcagtct ttctcacttg 240
   gacaacctgt tgtgcaaaaa cactaggaat cttgaagtga gggagctctg tccacagctc 300
   ccgtaatggc agggaggaga gcagagccca atgaaccttg agtgaaatta agtgctcgaa 360
   taaccacata attaaattgt gcagatcagc agtcctagtc tggaatttaa acactgtcaa 420
   cggggcatat tgggaaagat atttatatat atatatatac atacacacac acacacacac 480
   acacacacac acacacacaa acacattaag caaaataaga tttgtccatg ttataatcca 540
   gtctcacagt caagtcaccc accccagcct tttgaagtca tttggcagct aatgctaaaa 600
   agtaacagga actatgagag ttgcatagaa ttgatcagaa acatgaataa tgccaaaccc 660
   aaaacctgna ccatcagtta acagaaccag gtggcttcag atgcactatg aaacccttct 720
   atttaaaaca aagngcttnt tcatgtctac ctgggggacc ttttgntggg cattgaacag 780
   aaaagaanta tttgaattct ggatccttgg ccaaccccac tttt                  824
<210> 262
   <211> 816
   <212> DNA
   <213> Homo sapiens
<220>
   <221> misc_feature
   <222> (1)...(816)
   <223> n = a, t, c or g
<220>
   <223> nbla-10946-f
<400> 262
   gnggnngtgg ggnntnnnnt ttgaannccc ntgcctgttg gcctactggt tgtcttgctg 60
   ccttctttgc cagagatgaa ctaaaaggtg acaatatgta cagttgtgaa aaatgcaaaa 120
   agctgagaaa tggagtgaag ttttgtaaag tacaaaactt tcctgagatt ttgtgcatcc 180
   accttaaaag attcagacat gaactaatgt tttccaccaa aatcagtacc catgtttcat 240
   ttccgctaga aggcttggat cttcagccat ttcttgctaa ggatagtcca gctcaaattg 300
   tgacatatga tcttctgtca gtcatttgcc atcatggaac tgcaagtagt ggacactata 360
   tagcctactg ccgaaacaat ctaaataatc tctggtatga atttgatgat cagagtgtca 420
   ctgaagtttc agaatctact gtacaaaatg cagaagctta cgttcttttc tataggaaga 480
   gcagcgaaga ggcacaaaaa gagaggagaa ggatatcaaa tttattgaac ataatggaac 540
   caagccttcc tcagttttat atttctcgac agtggcttaa taaatttaag acctttgccg 600
   aacctggccc tatttcaaat aatgactttc ttttgnattc atggaggtgt tccttcaaga 660
   aaagctgggt atattgaaga cctgggtttg atgctgcctc anaacatttg ggataaccta 720
   tatagcaggt atggtggagg cccactgcaa ncatctgnac atttggcatc ctggccaatt 780
   gaggcggnga aaattggaaa angaagaaaa ctggat                           816
<210> 263
   <211> 826
   <212> DNA
   <213> Homo sapiens
<220>
   <221> misc_feature
   <222> (1)...(826)
   <223> n = a, t, c or g
<220>
   <223> nbla-10946-r1
<400> 263
   gnnagngggn nnnnntttgn ggccntttnt ttgnnncccn ttttnttttt ttttacattt 60
   taaagacatt tttattgagc taattttaac aacattgctt tagctggtga cagctgcccc 120
   aaaccaaaac aaagccatca tgaatgctat tcaacatcct caatgtaatc cagtatgttt 180
   ttgtacttgg aatatagtta aacttttgac attacataat caagcaaata gcagtgcata 240
   ctatattatt caaaaagact ttatctattt catttaaaaa atcaagttgc aagtggcctc 300
   agctttatca acaatcgtag tgacacattc cacacttcat gctctcaaaa taaaaagtgc 360
   cctaaaacta actctaagtt ttttagtcac tgacattaat actaaccagg ttacaggaat 420
   tgaagtttaa cattgtacaa tataagcggc aataagttac tgatatctgc tgacaaattc 480
   cactcaaact aaatatatcc ttgatcattc aaaaagacat tttgtaaatt ttaataagct 540
   agttagtgna tatgctgcag tgcaaaaaaa gtgacaccct agtttcctga gctttaggaa 600
   attaacaatt ttctgctact acttttaact taaaataggc tctttattat atgaagcctg 660
   gcaaaactnt ncaattaaat tgggcttttg ggagaactga aagactggtc agaagtcang 720
   ggggatttan gacaatggaa aaagnttcaa gncaaagttt ttccacactn ccaaaggttc 780
   tntgggnnct catgtnnngg gaagattaaa gataaccncc tggttt                826
<210> 264
   <211> 1735
   <212> DNA
   <213> Homo sapiens
<220>
   <221> misc_feature
   <222> (1)...(1735)
   <223> n = a, t, c or g
<220>
   <223> nbla-10949
<400> 264
   gcctgtacgg aagtgttact tctgctctaa aagctgcgga attcctcgag cactgttggc 60
   ctactggtgt tttaacactt gtttgtgtga gattgtgtga gaaattcagg acctgtacac 120
   tgcagtacat ttgaggattc ctggccctca cctcctaaat cccaccagct acccttcccc 180
   cattattttg agaattaaaa cacctacttg tttccagatg ccacctgcct ctgtgagtgt 240
   gggcatgagt atgtgtgtgt tgcggaggtg ggtgagggag ttggaggagt tttgttacac 300
   atctgagtta tttcatcatg gtaagatgtt attgttcttt catttcatta aaaaaatctg 360
   tctaattctt taaaaataga gttaaagaaa gaagatattt atgcagtgga gatagttggt 420
   ggtgctacac gaatccctgc ggtaaaagag aagatcagca aatttttcgg taaagaactt 480
   agtacaacat taaatgctga tgaagctatc actcgaggct gtgcattgca ggtgaatatt 540
   ctttctttta taggtaacca tttataaatc attgtaacaa gatggcagtg gtttatgtat 600
   ctaataacct gggattagct cagggtgact tcagggtgtt gagattccaa aacctttgtt 660
   ttaaaataga ggtgatgttc ctaaaaataa ttgtactcta tagcacataa gatgaattaa 720
   ttgactagaa acataggtaa ctctgagcct gaagtatgac tggaagtttg gcctgttatg 780
   tatgtctgta tgaaatagtg tacattaatt aaataagggt tggggttaat agaaaaataa 840
   ggaatagcta gattatatag gtaattgaca tgatagaagt ttgtaaaact gttcagacac 900
   cattaaatta ctaatataaa tattttaaaa agaccattga ttttctgaag taatttgtat 960
   gtttaagtga tattatcagt ttatattgtc tatacttgtt aaacggaatt tttgaaaatt 1020
   attattctag tgtgccatct tatcgcctgc tttcaaagtc agagaatttt ctatcactga 1080
   tgtagtacca tatccaatat ctctgagatg gaattctcca gctgaagaag ggtcaaggta 1140
   tcatgtttat taatcattta catatctaaa gttaattgac tattaggtct gagtaatatg 1200
   ttgcttttca tttcagttta cataaatctg taacatttta tcatagtgca tgaaggaagt 1260
   ggtcatttat tttagtcaga ataaatttgt gtccttaatt agtttctcac acttgattct 1320
   gctgaaatca tcggtgcctt ttatgtgcag attgacgtat attccttttt tactgacttt 1380
   atatataaag attcttggga actcttataa tctggaaata ataccaattg aaaagagaga 1440
   gaaactaaca ctgtacaata attacaaacc tagaacacat gggtaaaagt gttaggaata 1500
   tcctgaattt gtgtttttgt ctttttcctc cattcagtga ctgtgaagtc ttttccaaaa 1560
   atcatgctgc tcctttctct aaagttctta cattttatag aaaggaacct ttcactcttg 1620
   aggcctacta cagctctcct caggatttgc cctatccaga tcctgctata ggtaagtaaa 1680
   gagttggaaa ttaaaaaaaa aaaaaaaaaa aaggccacat gtgctcgagc tgcag      1735
<210> 265
   <211> 725
   <212> DNA
   <213> Homo sapiens
<220>
   <221> misc_feature
   <222> (1)...(725)
   <223> n = a, t, c or g
<220>
   <223> nbla-10967-r1
<400> 265
   gnngnggnnn nnnttgannc cnttnnttnn cntttttttg tgattcaaag atttactaag 60
   gtactctatg cattctatct atacagaaac acctatttat tattacagtt gatttatgca 120
   ggattaacat ttttggtgtt aaaattgtta aacatcatgc ttactgcaca tcaactctcc 180
   ataatgaatc tgaacttcac aatgatttac caaacacttt acttaaatta ctaattaaat 240
   aaatgaaaaa tgcaccgagc caaacaaaac ttaactgggc tataaagaaa aaaacccttc 300
   aaaacagtcc atatacacat ctatttcaaa actacctttg ctagctagcc cctttcccaa 360
   agttttagcc tgaaaaaaca gtaaaatcta cacaaaattt tattgcaatc atacaagggt 420
   tacattaggt caacaaatac tatgatgcaa ttttacattt attaaactac agttcaaagc 480
   acaaatttac acattctaaa tacactaaac gttatctaat gaagtcacac tggcttctaa 540
   catttgatat atctgggtga aagacatgaa ctttacaaga ctttaaacac aaatccttag 600
   tataaaaact gngttcttgn atgtaaacga tttaatgggg aacccaatta atgggcttnc 660
   atcttcacta agcatncatt tggtgnatat ttattttaaa cgcttaangg gtaggacaaa 720
   ctggt                                                           725
<210> 266
   <211> 710
   <212> DNA
   <213> Homo sapiens
<220>
   <221> misc_feature
   <222> (1)...(710)
   <223> n = a, t, c or g
<220>
   <223> nbla-10977-f
<400> 266
   ggnnnccntt ttgaganccc tcgngcactg ttggcctact ggaagtagtt ccttgaatgt 60
   ctgttgatga aatagtcact gtttaaggaa aaaagtaatt atgaggtgta gcagattgca 120
   gaaaaacagg attagaaaca cacttaaaaa gaacacacat ttagagtctc tcttcctcct 180
   cagcgaacca ctaggccccc tttttaaaaa cacctttaga gcctaattac tccaataaaa 240
   gtaactagag gtttggagtc tggttaaata aattctgagt aaaattctta agccaaatgg 300
   aaattcttaa tgcaatcatg aggacttcta ttgtctctta ctgttgtatt anatcctata 360
   aattgaactg atttttccat aaggaaaatg cttcttttga gattaattct aataacgtat 420
   ttgctattgc agtgcagagc ccactgcaac tgctaggact gaaagcagag gctgggtgcc 480
   agagcacgtg attcttaaca tcatttccac agacccctct gccctgaccc tctgcattgg 540
   atgcaggaag ctgggaaaga ctgatgttga nttggaaaca tgggctgaaa atgaaggccc 600
   atagtgcata ggaacaagta aagccagggt gcttgacgtg tgtgtgtgtg tgtgtgtgtg 660
   tgtgtgtgtg tgtgtggngn tgngngtgnt tgnncctgca cccctacann            710
<210> 267
   <211> 717
   <212> DNA
   <213> Homo sapiens
<220>
   <221> misc_feature
   <222> (1)...(717)
   <223> n = a, t, c or g
<220>
   <223> nbla-10977-r1
<400> 267
   gnngngngtt nnntttgtgt gncctttttt tttctttttt gaattaagtc tcatttattc 60
   tagggcaaac tcaaaagggt tttttttttt tcttttttag caaatcctag cacattatta 120
   acaaaaaaat ctgtacattt gccatgatac acttgaaagt gaaacaaata agatataaat 180
   acagatatgg atgtaacatg aaaaagcccc ccccccaaaa aaacccaaaa aacaaaaaaa 240
   cccacccaac ctgggggtgt ggggaccatg cgtgtgaaca cagtgccaac ataactattt 300
   tggtggctga tattttttca gagcacttct ttatttccaa acattattgt tagcaaagtc 360
   ctggtgggat tctacaaagt tcttatttgc tgtcctttaa agtgtaacat tacgcttgat 420
   aaataaaggg tggcagggaa agaaaaagac tgatgagaca aagtcagttg cattacctgc 480
   acagggtggt aagctagtga ggcgcataga aaaggcaggc ccgtggcatc gccgcaggtc 540
   cgggaagcac acacagtgta cttgaaggaa gctgcgtgcc ccgaggaccg gntgnaccct 600
   gcccanaccc tggctgcaag ggaggcaaca acttgganga acagggagtc aancccgcca 660
   gcccgnatgg ancaacgggg gtcttgggcc tggcagtccg gtanacaggg angcaaa    717
<210> 268
   <211> 1979
   <212> DNA
   <213> Homo sapiens
<220>
   <221> misc_feature
   <222> (1)...(1979)
   <223> n = a, t, c or g
<220>
   <223> nbla-10993
<400> 268
   ctgtacggaa gtgttacttc tgctctaaaa gctgcggaat tcctcgagca ctgttggcct 60
   actggaggat gcgctccccg gcgcccagca gcagaggcca ccgctcccag aaatgcatgc 120
   gaccgatccc cttctcccgg accccaggag ccggcgcccc cgccctgtag ggttacgact 180
   cactgattaa aaagagggac tttttcaaat actttgcact tttgattgtg tattatggat 240
   accaaggaag agaagaagga acggaaacaa agttattttg ctcgactgaa aaagaaaaaa 300
   caagccaaac aaaatgcaga gacagccaca gctgtagcta caaggactca tactgggaag 360
   gaagataata atacagtagt tttagagcca gacaagtgca acattgctgt ggaagaggaa 420
   tatatgactg atgagaaaaa aaagagaaaa agtaatcagt taaaggagat caggcgtaca 480
   gaactaaaga gatattatag tattgatgac aatcaaaaca aaacacatga taaaaaagag 540
   aagaagatgg tggttcagaa gccccatggg actatggaat acactgctgg aaaccaggac 600
   accctaaact ccatagcact gaaatttaac atcactccca ataaattggt ggaactgaat 660
   aaacttttca cacatactat tgttccaggc caggtccttt ttgtgccaga tgccaactct 720
   ccttccagta ccttaaggct atcatcatcc agtcctggtg ctactgtctc tccttcatca 780
   tcagatgcag aatatgataa attgcctgat gctgacttag cacgaaaggc cttgaaaccc 840
   attgaaagag tcttatcgtc tacttctgaa gaagatgagc caggtgtggt gaaattttta 900
   aaaatgaatt gtcgatactt caccgatgga aagggtgtgg ttggcggtgt tatgatagtg 960
   actcctaaca acatcatgtt tgaccctcat aaatctgatc ctctggttat tgaaaatggg 1020
   tgtgaggagt atggtctcat ctgccccatg gaagaggttg tttccattgc gctctacaat 1080
   gacatttctc acatgaagat caaagatgcc ttgccatcgt aagacattta tttgtttacc 1140
   aggaaaaaag gggtgttgag agagctaaat gtagcttaaa aatgagggca tttgcatgat 1200
   tgagggattg tgtagaggtg attttgaaga tggaagactt gtgcactgaa gaaaatgaga 1260
   aaaatgagaa gaaatgaaaa gaataaaatc aatgatggga aaagttgaac atataacgat 1320
   taaaggagaa aaacaaagaa gccgtcatgt aaaaatagta tttgttgggc ttatttttct 1380
   aaaaagcagt gcacgttctt aatgaaatta tgaaggaaga aaggcagttc tctgaaagaa 1440
   gtttatccaa ttatcaataa gagaataatg ttttcttctg ggtttaatta aggagagtta 1500
   tgtttgtctt catttaactt ctaggaaaag cagtctccct gattcatgtc ctccctcagt 1560
   cctgcatgga gagaggtttg ggtctacagt gtagtggtag ccaccttctc atgctgtgaa 1620
   gagggagtaa taccagtttg ctttttccct gaaatacaga tgaatatgac ttcagtcctg 1680
   attacttttg ccttataatg ctggatttat tgtaaaaaag agagggaagc tccccaggaa 1740
   aaaagagaaa gcattaagaa agctcaggaa attgattaac tgatacagat aatctgattt 1800
   ttactgtcct ttcgctctac tgtgtctgtt tctctataaa agccagcagt aaaaaacttt 1860
   aaaaaccttc agtgatggga agaggcaaag cagtaggtcc taacagtaaa gagggaaact 1920
   agcccttggg gcttatatga aaaaaaaaaa aaaaaaggcc acatgtgctc gagctgcag  1979
<210> 269
   <211> 727
   <212> DNA
   <213> Homo sapiens
<220>
   <221> misc_feature
   <222> (1)...(727)
   <223> n = a, t, c or g
<220>
   <223> nbla-10999-f
<400> 269
   gnnnnnnggg ggggnttnnn ntttgannnc ctttgngcac tgctggccta ctggctgttc 60
   cagaaaaact gttactcccc ctcacccact ccctccttcc ccagttgttc ccttcagact 120
   caggggctcc accaatgcca tcccaaaaca gggtcagaca ctgcccagct tccctccagg 180
   aggttcttgt ctctgtgtaa gggcttgtct ccctcccagt ttttcttttg ctccacgtca 240
   ttttgtcagg ctggttataa gccggaggca gctttaacca gcccccaggg atgattgtga 300
   aggaggcccc tccccttgtg aggagggggc actcctctcc agcccctggt accacagtcc 360
   tcacgatggt gcagtgattt ctagccaggc gtcaagatgc gctgctttcc ctctcctgcc 420
   tcatcccttg ttggcagctc cagttcaggc cgtggaggga cgtgatgctg ggctgtgttt 480
   actaaaccca cgggttttca gcctcttaag cccagctccg atctccaatt aagttgagag 540
   cgctgggttg actaacctct ggtatctgag cacagacaga gggtgctgtg ggtctgctgg 600
   gtggcagaaa tggttccttc cggcttggcg ttctctcctg gcactcttnc tgctgcctct 660
   gactactcag ccttggtttc ggngtgaagc cccaactgcc actgggaact ggcggctnat 720
   gcttgct                                                           727

<210> 270
   <211> 731
   <212> DNA
   <213> Homo sapiens
<220>
   <221> misc_feature
   <222> (1)...(731)
   <223> n = a, t, c or g
<220>
   <223> nbla-10999-r1
<400> 270
<210> 271
   <211> 1834
   <212> DNA
   <213> Homo sapiens
<220>
   <223> nbla-11030
<400> 271
   ctgtacggaa gtgttacttc tgctctaaaa gctgcggaat tcctcgagca ctgttggcct 60
   actggtggag tcaagctaat agaatatttt ctgaggaaca ttattatttg tggaatattt 120
   tttctaccct catccatttc cttctctgca tttttctttc ttactggtct ttgtacagat 180
   tttattcaat tttctgttaa agttgtaata gtatgcataa gacctgtggc attgagtact 240
   tttttaaaat cccacataga gtcaaaaatc ccaattactt gcatttgtta gttgctgaga 300
   agacagacct tataaagagc tgaattggtt aaagtccaga gcaccaattc atgatgttgt 360
   gaccaattta caaagacatt cagacacagc tggataccat agacaaagat gaaattatga 420
   taatgctgtt caaaattaac ggtccataac tatcaaaggc aggtgcaagg caaattgaat 480
   ttggatattg atgaggctga aatgggatag ttcatacagg gaccttggat ttatactgtt 540
   gcttttttat ggccatgtta aaagcatcta ctttccccat gggaggaagg tgcatgctga 600
   gtgatcctgt tgagctgtca ctgctctgtc aggagattct tgttgatgga catatgtctg 660
   accacttgag aattgtgttg gagtgaaata cacttgcata agtcaattat taatgacagt 720
   tcctttagca actcccagag aaggtggggc atgacttctt ccctggagct gacttcagac 780
   aaattcacag atgctaaacc ctggcttttt tttttttaac attttaattt cctctcatag 840
   aatcatcaca aaataagaaa acacttcttt atatcgtaat cataattcca gtgttttcag 900
   ttttgtttcc tttttccact aaaatcattc ctgtgtttca atcagtaaag tgggcttctt 960
   gatttcattt gggatttgta tttgtgtttt tgttttccat tcgtttatgt ttctttgaaa 1020
   ggcgctgtga aacgcctgca ggtatggtgc tagccaagtg atctctagag acctagattc 1080
   caaaaatcca agccattgtc catctgaatg ctataaactt catggacatg ccctcacctc 1140
   atgagtgtcc agtgcctctc agatgcaccc tgtatattta ctgttcatcg tggaactcgt 1200
   gccactgaaa atttttaagt gactatattc aaaaacagca ggttgcatga cagtttctca 1260
   gtgaagaggt tcaaaaaagg tgagatgcta ttgctttgtg aatttacaaa ggaaagaata 1320
   atttaactgc tcagaattac atgtccggtc actgcttttt aatttaaaaa ataatagagc 1380
   atcattagta atcttgtttt ctctttgata cataggtaaa gggtgttttg tgtctggatg 1440
   cctaaggtga ttccagggga ggggatggaa gatatgtgac atcttccctg aaatttatat 1500
   tgatatgcaa tgctttgtca tttaaaacct aagctaatgt tttctacaat ccataactct 1560
   gagtttatct ttttggaaac atagaagggg atgacattga agatgaaatg gatacagcaa 1620
   ttgctgaatg acagtttgcc caaattagtg cagttaaaat atgctgacgc ccctgcatgg 1680
   ccaggaagac ttctgctcca tgcacacaag caccaagtat caagcgacca ccaacacgtt 1740
   cccattcctt taggcctcca tagctttgct tttgctttct gtttcctgaa ctaaaaaaaa 1800
   aaaaaaaaaa aggccacatg tgctcgagct gcag                          1834
<210> 272
   <211> 1871
   <212> DNA
   <213> Homo sapiens
<220>
   <221> misc_feature
   <222> (1)...(1871)
   <223> n = a, t, c or g
<220>
   <223> nbla-11042
<400> 272
   gcctgtacgg aagtgttact tctgctctaa aagctgcgga attcctcgag cactgttggc 60
   ctactggttc aaaagaactg ctgcccagtg actccgtgct gcacggaaga atatcagctc 120
   cagaaacaga aaagatagtc ctagaggcag gaaatggatt accatcctgg aaattcaatg 180
   accagctttt tccctgtgac gtgtgtggga aagtgtttgg ccgacagcag acattgtccc 240
   gacacctctc gctgcacaca gaggaaagaa aatacaaatg ccacttgtgc ccctatgctg 300
   ctaagtgccg tgcaaatctg aaccagcact tgaccgtcca ttccgtgaag ctggtgagta 360
   cagacaccga ggacattgtc agcgccgtca cctctgaagg cagtgatggg aagaaacatc 420
   cttattatta cagttgtcac gtgtgtggat ttgagaccga gctcaatgtc cagtttgtca 480
   gccacatgtc actccacgtg gacaaggagc agtggatgtt ttcgatctgc tgcactgcct 540
   gcgacttcgt caccatggag gaagcagaga taaagactca cattggcacc aagcacacag 600
   gggaagacag gaagaccccc agcgaatcaa atagcccctc ttcatcctcc ctctcagctc 660
   tgagtgattc agccaacagc aaagatgatt cagatggctc ccagaaaaac aagggcggga 720
   acaatctgct ggtcatctct gtcatgcctg ggagccagcc ctcactgaac agtgaggaaa 780
   agccagaaaa agggttcaaa tgtgtttttt gcaactttgt ctgcaagacg aagaacatgt 840
   ttgagcgtca tctgcagata cacctcatca cccggatgtt tgagtgtgat gtgtgccaca 900
   agttcatgaa gacccccgaa cagctgctgg agcataagaa atgccacact gtccccaccg 960
   gtgggctcaa gtgcccattc tgcatttatt ccaccaaccg ccccgctgcc atggagtgcc 1020
   acctcaagac ccactacaag atggagtaca agtgccggat ctgccagacg gtgaaggcca 1080
   accagctgga gctggagacg cacacccggg agcaccgcct gggcaaccac tacaagtgcg 1140
   accagtgcgg ctacctgtcc aagaccgcca acaagctcat cgagcacgtg cgcgtccaca 1200
   ccggggagcg gcccttccac tgtgaccagt gcagctacag ctgcaagcgc aaggacaatc 1260
   tcaacctgca caagaagctg aagcacgccc cacgccagac cttcagctgc gaagagtgcc 1320
   tgttcaagac cacacaccct ttcgtcttca gccgccacgt caagaagcac cagagtgggg 1380
   actgccccga ggaggacaag aagggcctgt gtccagcccc caaggaaccg gccggcccgg 1440
   gggccccgct cctggtggtc gggagctccc ggaatctcct gtctcccctg tcagttatgt 1500
   ctgcctccca ggctctgcag accgtggccc tgtcggcagc ccacggcagc agctcagagc 1560
   ccaacctggc actcaaggct ttggccttca acggctcccc tttgcgcttt gacaagtacc 1620
   ggaactcaga ttttgcccat ctcattccct tgacaatgtt ataccccaag aaccacttgg 1680
   atctcacatt ccaccctccc cgacctcaga ctgcgcctcc cagcatcccc tcacccaaac 1740
   actccttcct ggcctatctc ggactgagag aaagagcaga gactgtctga gggcagccat 1800
   gttctgtacc aaaaacagag agacaaaaga caaaaaaaaa aaaaaaaagg ccacatgtgc 1860
   tcgagctgca g                                                      1871
<210> 273
   <211> 2169
   <212> DNA
   <213> Homo sapiens
<220>
   <221> misc_feature
   <222> (1)...(2169)
   <223> n = a, t, c or g
<220>
   <223> nbla-11051
<400> 273
   ctgtacggaa gtgttacttc tgctctaaaa gctgcggaat tcctcgagca ctgttggcct 60
   actgggtctt aaatttgtta agctaaatat atgttggttc tttttatttt ggaatccttt 120
   aagcatctta aacatttttt ttttgaagag aagttacaaa taacatttct atcaggtagt 180
   acttgtatga aaccaccttt cttattctat aattttgatt tttcaatttt atatacttaa 240
   tatactcact gtcttactat cagaaagtta ttttgaccaa gatttttatt atcttcatag 300
   attcagaaag agatgctaat tctgtaccaa tgtcttcctg gttactattc tcttccctct 360
   aatatatact ggccatttgt aaaaccattg tgttgttggg atcacttagt tatactatac 420
   gcagatagag catctcaact ctgtcatagt gttcgctgaa cagttttcag tgtcatgcac 480
   ctttggctgc taattgttcc tgacgtgcac tcttccgagt tggtaaaggc acagtgtgtt 540
   catgccagac ttctaagaga aacaccagcc tcttaaatca gaagcctaca cacaaccccc 600
   ttaacaatcc aaagaagctt gatggtgtgc aaagaagcat cctgccagcc ttgtcattgt 660
   tctgttctat gctaatcctg ctgtgttgtc taaaagatgg agggaagagg acatcagtgt 720
   ctgatagtga aatcatcagc aggaaagtga agctctttcc ttggttacag ataagacttg 780
   gtttacacta ttggccagta tctgctaaac atatgaagac ttaactattc agtgttgcct 840
   aggcattcgc ctgcacaaca ttttgaggtt agaacataga atattttcag aaatactgtt 900
   gtagtttgtg agtgttgttc attagttaca cattagctat agagtggatg catgaagccc 960
   catgacacca gtaaacttct cttaccagta ggtaaaccaa acaccattct gtcattagca 1020
   gccctcttaa atgttgcctc tccgtatcct gttgcatttt tgtgtgcatt gtgtttctac 1080
   tgatctctct taggttttta cggaatcaaa ggaaactaat ttttccttaa tagcaagaaa 1140
   gatgaagagg taaagggcat tgaagcagaa atgtatagtt tggggtacga ttagaaaact 1200
   cgtaaggaaa acagaagtcc taatttcaaa ctgactgctc ttcgttaagt gctcttaagg 1260
   agagtctagt aacagtaaca ctttctggcc atttctagtt tagattctct tcgttactga 1320
   aacttttgag aaatattacc tgtggattaa ttttgcacaa tgttctattc tcataatgac 1380
   ttacaaatta aactaggttt ttactgaact acctcacact aattttctat gctttcccaa 1440
   gtaagctgtt gccctgttgg atctttactg agtgaattat aaatgtgtgt taaatacttt 1500
   ctagccaatg ttgacacaat accagtaagt atgtaaagta tataccttac atcagtaaga 1560
   gacacgtgta aaatctttga ctgtatgtct tgcaaaattg tgctcgttga cattattact 1620
   gtttttgtaa gtagaaacct gctcgtgata tcggtccatt tacattttac aaaaggagta 1680
   aatcttagta aaaattttac gaagaaataa attacttttg taggcccaat atttggtata 1740
   tttttgagaa gctgttaatc ttttagctga ataatgaagt tagactgaat tacgtgtctc 1800
   cctggactgt gacatctatt ttctcattac agtttatcct ggtcagcagg gtgtcacacc 1860
   tggaaacctg agtatgatag ctgacatttg cttttctccc tctgcgatgt cattcctcct 1920
   ccattcctct ccttccctgt gttccgttcc ctctcctttc ctctagacaa aacaaaatgg 1980
   ggcacttttt agggaatgct gagaacatta ttgtggtttt tcatcattca tgccctagtc 2040
   attaaacatg caccactgga atgtaaacaa tgttatctag tatgtcaatt ggttataata 2100
   ttttaaataa aaaagaaaaa agtggtatga aaaaaaaaaa aaaaaaggcc acatgtgctc 2160
   gagctgcag                                                        2169
<210> 274
   <211> 733
   <212> DNA
   <213> Homo sapiens
<220>
   <221> misc_feature
   <222> (1)...(733)
   <223> n = a, t, c or g
<220>
   <223> nbla-11067-f
<400> 274
   gnnnnnnggg tggnttntnt ttganncctt tggcactgcc ggcctacttg aaacacttgt 60
   acttctggaa atggactgga gactttttaa atttgagtcc actattgaca tggaaacccc 120
   agtggaatca gatnntccct caaagaccat gatggtatcg gactagtttt cagacactgc 180
   ctgttgctgt ccatcagcac ttggtctctt atcttcagtg agaaggtgac ccgccttctt 240
   cccatggtgg ctgcctaaag tgcttctttt ctaacccaaa acagttctac tcacttcctt 300
   ttacagaatt caccggccat tttcctgtta cctgatcctt ctacaggatt tttaaaaagt 360
   aagagagttt cagagaagcc gatcccataa tccccagtgc agccagggtt tgtgtgtcat 420
   cgtactggag tagagggccg actcttccca tgaaggtgag cacagctgtg agtgagtgag 480
   ctcatatctc catttgtcag tgctggactg gtaccagatc gtaaccttcc cgttggtcgg 540
   aaacttttcc atctgtcgcc tagaaaaaga gaaagcttac catcgagggt gtggntgatc 600
   cttgaagctg cttggtaaag tatcatttct caagcttttc ttttggactc ctatcatgna 660
   tcattaatat ctaccagtcc cttttcattc taagacaaaa catttnttct aaatctctga 720
   ataaaatcag ggg                                                733
<210> 275
   <211> 736
   <212> DNA
   <213> Homo sapiens
<220>
   <221> misc_feature
   <222> (1)...(736)
   <223> n = a, t, c or g
<220>
   <223> nbla-11067-r1
<400> 275
   gnnggnnnnn nntttgagtg ccctttgaan ncctttgtta ctggcgaaac tcatctttat 60
   taaacttaaa cagagaagct acttctgcac atgttaaaca cacatggaac caaacagctc 120
   gatggagaca ccctgcactg cttatggtga tgctgcagga atggcggcag cgatgcccgc 180
   tacactccat ggcctgctgc acatgtgaca ggacagttat ttcatctatg cttacacctg 240
   caggtccctt accctagaaa cactctatat cttcttaggt acttcccttg aaacaggatg 300
   agttctagag gaaataaaac tgctgcactt acaaacctag aaatattcag ggaccaagat 360
   tagttgttca gtgacctagg aaaccttaga atcctgtagg ttcctcagga gaggtgcttc 420
   caagtacttg agaacccaca ggcagagatg cttccctttc catggtggat gtgtatgtca 480
   ctccaggaca aactgcggag tcacggagtg tggaacacat ctggttgtgt acagtacact 540
   atcccagagg aaaatcacaa agcaacacaa ctctgcagca aaaactagtc ccaaagtcag 600
   aagataaaac tagcaaaaac tttgccaata tttagagaaa agctctangg tgtggaaaag 660
   tccgggcttc ctttttctnc atgcccaagc agagccagta tcattcctta agctntaaca 720
   aaaacccttt gnataa                                             736
<210> 276
   <211> 719
   <212> DNA
   <213> Homo sapiens
<220>
   <221> misc_feature
   <222> (1)...(719)
   <223> n = a, t, c or g
<220>
   <223> nbla-11090-f
<400> 276
   ggnnnnnnnn tgnnnnccnn tttgagancc ctcgagcact gttggcctac tgggtaaaaa 60
   gactggctct ccaggaggcc ctggtgtgag tggtggtagc cctgcaggtg gggctggagg 120
   tggaagcagt ggtttaccac catccaccaa aaagtcaggc cagaagcctg tcttagatgt 180
   tcatgcagaa ctagacagaa ttacacgaaa accagttaca gtttctccca caacacctac 240
   atctcctacg gaaggagaag cttcttgaag ataccaaata aagccattta ttctgttttc 300
   tgggataatg taaacatgcc tctgcctttt ccttcaaaag tggaattaga aagctggagt 360
   gcttcttcag atggactaaa tttatgtcgt gtgtgtgtgt gtgtgtgtgg ttacccattt 420
   tttagaagga gccgtacaga agaaaaatta ttctacatta tgtaggattg ctgtttgcat 480
   tgccattttg cataagaaag taatttttga ttttgaaaat ctcaaaactt ttanatctga 540
   aatacagcca tgtgatcgat catattctaa aggctattta aaacatgtaa aaggatttgg 600
   ggaacggcag aaaacatgca gtttgggcat ttgactgact tggaaagtct aagcttattt 660
   taagttataa ctattaaaat catttttaaa aatttggtaa gtttgctgac agagaaaaa  719
<210> 277
   <211> 715
   <212> DNA
   <213> Homo sapiens
<220>
   <221> misc_feature
   <222> (1)...(715)
   <223> n = a, t, c or g
<220>
   <223> nbla-11090-r1
<400> 277
   gaggnnnnnt ttntgtgccc tttntttttc cttttttgaa cctgaatgca nagaataaat 60
   actttattaa ctgattacag ttgaaagtca caaacaaaaa agggggtata ttaaggcaaa 120
   gccatatata tatatatata tgtatagaca aatccaaaga ttgttacttc tttacatttt 180
   aataacctct gcatttttct tttacacatt taggcagtgt gctttctttt ctcctcagtt 240
   aaagcaacac cttggccaat atttctaaat catattttga tgacaactta caggatcagt 300
   gattgagaca atgactagtg tatcaaacgc tatctgatag ttttcttagc atattccgtg 360
   tgtggtggta catttattgt ggtgtgcaaa atctgacaac tgacgatttt tctctgtcag 420
   caaaactaac aaatttttaa aaatgatttt aatagttata actaaaataa gcttagactt 480
   ccaagtcagt caaatgccca aactgcatgt tttctgccgt tccccaaatc cttttacatg 540
   ttttaaatag cctttagaat atgatccgat cacatggctg tatttcacat ctaaaagttt 600
   tgagattttc aaaatcaaaa attactttct tatgcaaaat ggcaatgcaa acagcaatcc 660
   tacataatgt agaataattt ttcttctgta cnggtccttt taaaaaatgg gtacc      715
<210> 278
   <211> 2961
   <212> DNA
   <213> Homo sapiens
<220>
   <221> misc_feature
   <222> (1)...(2961)
   <223> n = a, t, c or g
<220>
   <223> nbla-11110
<400> 278
   ctgtacggaa gtgttacttc tgctctaaaa gctgcggaat tcctcgagca ctgttggcct 60
   actggagttg cagggtcctt tttggaagct cttgccgtca tcgctgactt gggcaatggg 120
   gccggtgggg tttgggggcg gaagagaccc tcggggttga gaagtatgtg gtggcctttc 180
   gtcccctgta aaacattgtc acacggtgtg gggcggcagc gctggatctt tgcaagttaa 240
   gtaggcgctg gcgttgttta cttgtcacct tctctgtatg ttttttccct cattttccct 300
   acccttgggg ctagtgagtc tccaacctct gtgcccattg tgtgattctg ccttgatgac 360
   gtgagttgct cgttctgact tccaggtgtc acattctctt ctaatctttt tttgattggt 420
   gtagatacct gtggagggcg gagctgtagc cctctttatt accagtatcg gagtatatac 480
   tactacctgt ctgacctcag gtgctttctt ttccaacgat cttatttctg catattcagt 540
   gagcaaagtg tagatagaaa tgtctcagag tggcctggct attacttccc tgttcatatt 600
   aaaacccttt ggtttatttg ggagttcaat ttatttcctt tcataacttc tctaccttct 660
   caaatcttct cttttctcat cgtccgaggc tgtagttact ttctgttctc acgtattttg 720
   acctttttgc attgcaaaac aaaaacctcc agttctggaa atcaggcttg tttggtttta 780
   ctttaattgt cagatccagt tggtgtctgg tacctatgta gtatgttcta gtagattttt 840
   gcagtagaga ctaaaaaaag aaaataaaag aaaagaatgg tgtgaaaatt taagaaaagg 900
   atcctcttgt tactgccatc actgtcacag ataaaaacac tgcctttacc ctggaacatg 960
   ttagtcctct cttggaggaa atgaaaccct aaaggttgac tgtggtctct gcagccaccc 1020
   agtcctgtat ttggagagcc ccctggcttt tccagtccat cagatttagt ttgtttagaa 1080
   aaaaataaat aagcatgtaa tgtatgaaag ttacgtgaga ctatcttggc agttataaat 1140
   taataaacat ttacggtaca gaagtggtgt gaattgtaag aggatgtaaa ccaaacaaaa 1200
   tacggcttta aagagggcct gaagataagt gagaaaactg aaattcttga aacatgtatt 1260
   ttatggaccg aaagccaaag tgtgactata aataaagcag gacttaagag aatagacaac 1320
   aatattggaa gaaaatgaga attgtggtag agtttggata gatgaagaag agaggccatt 1380
   taagttggca atattacagc aaggacaaat aaaatcagaa ttgaatgttg tcgtggataa 1440
   aagcattagg aagaaggact tttgtttaaa tttctgcaaa tctctttaaa tcctggttta 1500
   ttagaagaaa ggctggtttc ttttgttgtc ttctacattc agtcttttgg ggtatgttat 1560
   tttagtcgaa gtgtatggag aaaatacagc tgcaatgata ggtagtcaga aacagaggag 1620
   cgtttttcag ataattgtgg atgttttttg acactacacc aaagctcaac aagtggaaga 1680
   ttcatgtcag ctgcaaggtg gaatctgaaa ccctatcaat gagcatttca ttcattgtta 1740
   cattaaaatc cattagccca tcttgcactt tgaaaggctc ttttacccat gcatggtttt 1800
   ataacatgat gccatggcca cttagaaaat gttggtttac tgagttatgc acatctttta 1860
   aatatggata cgtttcatta cacaatatca aaatatcaca tttcattaaa ttacaactga 1920
   tcttgtcaga aatgtctctg aaaattctaa gtattgaaga gttgtcaagc tcatacttgt 1980
   ggatgtaagt ctttcaaaat ccaattttca tttgaaagtt tgactcataa ttatcattgg 2040
   tatcaaatac tgtcagtttt tctttaagtg atagattccc tttgttcatt tgacaaaatc 2100
   tgccaaaaag cgaagtctga ataaccagtt tgacaattgt tctttcaagt aaaaatggtg 2160
   ttccttgaag agtggttggt ttagctttta actctaaact tttcctggag atagccatca 2220
   catcttggta tgcagcagga ctttatgtat gcatgcttca tattttaccc cacaaagtat 2280
   taaaaagagt caagggttga atttcaataa aatgaattta tgctgctttg tgaagcacag 2340
   tcttaattta aattgtccct ttttgaactt gtggtgaaga atacaatgat tactggtgca 2400
   gtttggtgcc actgtcttga ttcatgctaa tgcaccagta gttttaccca ctgtggctat 2460
   gaaccatgag tgcaaatgtc agcacagtga aaaaggcaaa tagcatctta gtatttatta 2520
   tgagaatagt tttcaacttc tgaatctcca gaaaggattc tggggactac cagggcctag 2580
   ggatttcaca cttggagaac ttctgccaat catctactaa atattcagga catgggccag 2640
   gtgtggtggc tcacacctgt aatcccagca ctttgggagg ctgaggcagg tggatcagtt 2700
   gagctcagga gttgagacca gcctgggcag catggcaaag ccccattcct actaaaaata 2760
   caaaaaatta gccgggcgag gtggcaaatg cctgaggttc cagctactca gaaggctgag 2820
   gtgggaggat tgcttgagcc caggaggtgg tttgtggagg cagttgcagc aagctctgat 2880
   agtgcctggg caacagaggg aaacttcgtc tcaaaaaaaa aaaaaaaaaa aaaaaaaagg 2940
   ccacatgtgc tcgagctgca g                                           2961
<210> 279
   <211> 701
   <212> DNA
   <213> Homo sapiens
<220>
   <221> misc_feature
   <222> (1)...(701)
   <223> n = a, t, c or g
<220>
   <223> nbla-11176-f
<400> 279
   tngnnngntn nngtggggnt cnnnttgaan nccttgggca ctgtnggcct actggggaaa 60
   ggagtgttcg ttctacccag ggtaccacag ttccccacag tcaaaaccca aaagcaagga 120
   gatgagttga aagacagttt ttctttaagt catcagtatg ggatgtcagc agaacaaaaa 180
   ttaaaaagat taattttcct tttgatctaa aacttcctta gtttgagcag taggtgctac 240
   aaaattattt acatatctta gtatcatagt taaatgtaat gtgtttagga gaggaaaaca 300
   aaagatacat ttgctttaaa ttcattaaga aattttcaaa ttcactttgt agcccatgct 360
   gatagaattg ggctgtgttg gtacatttga aacactgttt atgttgcttg aaacacttat 420
   ttatttaatc gccgatgtga tgatgcctat ggccgagatc aaatatagct agattggcta 480
   gactacttat ttgtttactt aaactatggg aagaagcata ttattgtgtc attctgttgt 540
   gtgtgtatgt gtatatacaa tataaatata tatatataaa gttatttttt ctttgggtta 600
   atttattata agttgtaaca cttggctagt tttgnttgna tatgtcttaa aatgttttct 660
   tatgatattt aagtgacagt taaagaggta tcaaggtaac t                     701
<210> 280
   <211> 704
   <212> DNA
   <213> Homo sapiens
<220>
   <221> misc_feature
   <222> (1)...(704)
   <223> n = a, t, c or g
<220>
   <223> nbla-11176-r1
<400> 280
   gnagttggtg gggttnnntt gatggccttg ttgnnccctt tctacacact ttcatttatt 60
   gtctggacag ataaagtaca aataattcaa acacaacaag attatgaaaa aaaaacattt 120
   acaatacttt cccttggaaa tcatctaaac tagcaagatt aacatgaagt cattcattaa 180
   taactaaagt atacaactgc ctggtcctaa gatttttttt cctttttttt tcccccaaga 240
   ttgaacacat tatagagaag attttggcat gcctttccca ctaatgctta gcaaatgcat 300
   caaaactttg ccagcaaact acgttcattc atagcaggtg acgtaaaaag ctttccctga 360
   atgttgaacg agtgttctct gaactgaaat ctggtgttat atccaaaacc gaaaagagga 420
   aaaaaaaaca gaaaaaaaat ttaaaggagg agtctatgat gcagccttgg tattcgttat 480
   ctgtctacca ctacaacgac tactacaaag tttgcctttt cagtgtgaaa acaacagtcc 540
   tgtctacatg cacttagtgg gttcagcatg ggaaaaaatg cacaaaataa gcacangtta 600
   taaataacca taacgtttta ttaccattaa gactaaactt gnattgaaaa gattttatat 660
   aacangacan gaaaagcaat agcaaattta actatcaact agat                  704
<210> 281
   <211> 2035
   <212> DNA
   <213> Homo sapiens
<220>
   <221> misc_feature
   <222> (1)...(2035)
   <223> n = a, t, c or g
<220>
   <223> nbla-11189
<400> 281
   ttctaggcct gtacggaagt gttacttctg ctctaaaagc tgcggaattc ctcgagcact 60
   gttggccggg tggtgcttgg gtggtcgcca ccaagaagac tttggtgggg tagtctcggg 120
   gcagctcagc ggcccgctgt gcccgtttct ggcctcgctc gcagcttgca cgtcgagact 180
   cgtaggccgc accgtagggc gagcgtgcgg gtcgccgccg cggccgcctc ggggtctggg 240
   cccagccgca gcctcttcta ccgcggccgg ttgggagtcg ccgcgagatg cagcctccgg 300
   gcccgccccc ggcctatgcc cccactaacg gggacttcac ctttgtctcc tcagcagacg 360
   cggaagatct cagtggttca atagcatccc cagatgtcaa attaaatctt ggtggagatt 420
   ttatcaaaga atctacagct actacatttc tgagacaaag aggttatggc tggcttctgg 480
   aagttgaaga tgatgatcct gaagataaca agccactctt agaagaattg gacattgatc 540
   taaaggatat ttactacaaa atccgatgtg ttttgatgcc aatgccatca cttggtttta 600
   atagacaagt ggtgagagac aatcctgact tttggggtcc tctggctgtt gttcttttct 660
   tttccatgat atcattatat ggacagttta gggtggtctc atggattata accatttgga 720
   tatttggttc actaacaatt ttcttactgg ccagagttct tggtggagaa gttgcatatg 780
   gccaagtcct tggagttata ggatattcat tacttcctct cattgtaata gcccctgtac 840
   ttttggtggt tggatcattt gaagtggtgt ctacacttat aaaactgttt ggtgtgtttt 900
   gggctgccta cagtgctgct tcattgttag tgggtgaaga attcaagacc aaaaagcctc 960
   ttctgattta tccaatcttt ttattataca tttatttttt gtcgttatat actggtgtgt 1020
   gatccaagtt atacatgaat agaaaaagat ggtgttaaat ttgtgtgtag gctgggaatt 1080
   cttgctgaag gaattggaga aaacctgttg ctgcaaaatt ttacatgttc cagatggaaa 1140
   gggaagtcta agcgcttttt aaaacaattt ttttttgtat ttaattaagc aattgcagtt 1200
   atctgggatt tttgggtcag aattttaaat tctgtttgat tctccatatt ccagtgaata 1260
   aaatacaaaa gcattgtgtt tttaagattg tgtcgatatt cacctaaaaa cttgtgccaa 1320
   aagcacctgg attggtaatt atatttcact taaagggtaa atttgacaat atcttgataa 1380
   tcaaaagtgc aatttttttc ttcaaaatgt tttctccagc atcacagatc ctgcagatat 1440
   atatttatat ttatacatat atatttatga aataattctt actcacaaaa tatatttctg 1500
   ataaacatta agatattaaa tctgatgcac aaactttaat ttggccatta atctttttta 1560
   tttaaaaatt taaatttgtt tttaaaattg tatatagttt ttaaaatctc acacatgctt 1620
   cgatacttcc ttgttaagaa ttcttaataa ctactaaaac tgatttttaa tagttgctga 1680
   tatatatttg gtttgtttgg gtatactttt caaaaccatt tttgaatgtc caaacatctg 1740
   atttaaagtt tctgtttatc tttctgacca aaggagcaag aggtataatg gatatggcat 1800
   tcattaaaat ctttactatg tacaaaaaca gtaatattta cagcatcagt aaatattttt 1860
   aagtggtact tctaaatcat aaaagttggg gaaagagacc tttaaaatct tgtggtgttg 1920
   aacaatgtta tatgaagtag aaaaaataaa atacttccca gttgtgtgtt ttgttttaca 1980
   aaaaaaaaaa aaaaaaggcc acatgtgctc gagctgcagg tcgcggccgc tagac      2035
<210> 282
   <211> 726
   <212> DNA
   <213> Homo sapiens
<220>
   <221> misc_feature
   <222> (1)..(726)
   <223> n = a, t, c or g
<220>
   <223> nbla-11201-f
<400> 282
   gngggnnnnn gtgggntcnn nttgannccn ttggcctgnt ggcctactgg aggttggaca 60
   tccaacaaga tagactctaa ttcagcctcc ccagtctctg gccacccagc tcactcaggg 120
   attcagctac ccttggctac ccaagtagtc taaacatttt atctttatca ggcacatata 180
   cagtctggtt ctctgtagcc aaatttagat acccatgaca tttatctaat gatttcaaac 240
   caaacaggtt cctaaaaaaa cattcagcct acttctagaa cttccccatt ttgcttggct 300
   gtgaaaatgt atttgtccca tgcaggccct tgctccagcc cagtggaagc attagtacct 360
   cttcctagcg ggaactcagt ccagctacac accagagcca ggccagtggt tgtctgggaa 420
   atcttcaaag gccgtgacca ctcagcaagg gtcagtggca cctctcccca ctgtctcctc 480
   accaggtgca ttcatcccat ccatccatgc attcactcac ccttcgtgtt gtccagcact 540
   atgtatagtg ctgcagaaac aagaaggata tagtgcttgc cttcaaagac ctccaaagtg 600
   gggagatagt ctggatgaca ggtgctgtaa tagaagtgtg cccccggatg ctataggagc 660
   acagaaagan ggcaactgat gaacactggg gtgaaaagtg gaagangaga cctgggaaga 720
   ctcctn                                                         726
<210> 283
   <211> 727
   <212> DNA
   <213> Homo sapiens
<220>
   <221> misc_feature
   <222> (1)...(727)
   <223> n = a, t, c or g
<220>
   <223> nbla-11201-r1
<400> 283
   gnggggngng tggggggcct nttggannnc nttttcaaga caggagtgca gtggcacgat 60
   ctcagctcac cacaacctct gcctcccagg agaatcaagt gattctcctc tctcagcctc 120
   ccgagtagct gggattacag gcacatgcca ctaccgccca gctaatttat gtatttttag 180
   tagagacggg gtttcaccac gttggccagc tggtctcaaa ctcctgggct caagcaatcc 240
   gcccgcctca gcctcccaaa gtgctgggat tacaggcgtg agccaccacg cccagccaag 300
   gttcttcctt aaatgccgat gctctctgaa gtttcagctt cagcccttgc atctttctgg 360
   tgggattgct actcagggct tcaagggtcg cccctaggag tgactccaag gtggatctct 420
   ccatctctgc cttgcctatt tccagccagc tgtactcctt ctcctgggga acgacaggct 480
   ctccatgctc aacacagtaa ctgctctcct ctgaccacac cgagatcaac ttctattcca 540
   tcttccatgt ccagcagact ggagagtctg ggagtcaatc tcgaatcttc tcttactcct 600
   tactttatta aggcactggg acctatagct ttcacttctt taatatctca tattggggtt 660
   ccttcctttt caacttatcc tacagacact ttacttnang cttngccatt ctgncctgaa 720
   tcaaata                                                         727
<210> 284
   <211> 704
   <212> DNA
   <213> Homo sapiens
<220>
   <221> misc_feature
   <222> (1)...(704)
   <223> n = a, t, c or g
<220>
   <223> nbla-11212-f
<400> 284
   gatccctcgg cactgttggc ctactggttc ttccttctcc tctacccctc cttcccccct 60
   ccccattaga gtagtgtgga gataaggctg gactggtcta tcagattgaa ctccaagaat 120
   gatcacacaa aatgtttagg gagatgttcc ccgtggtgta tcctcatggt aacaacgaca 180
   aaaaatgccg gttgtctttg ttctcttttc actattccta acatgtgtac atgatagctt 240
   tgattctgca agtaaaagta aatcctgtgt tgtgactggt gctttcatat atttgtgaca 300
   atttttgagt aatattgcat gaaaatgtcc ctatgttaca tccattcaga agttttgttg 360
   ttttactcta aagctgggaa aggaaatgag agggaaaaga ccccggagag ggaagaaaat 420
   ctcagtattt tgaaaattga gattacttca gagccttagc cacacctaaa atacctccta 480
   gttaatagtg gtataaatgc ctcttcaata cgttttccag aatccaaagc attttggttt 540
   atccaggacc canggcagca cagctgtcac caagcaggag agttaaggat tcaccatgag 600
   ctgggaaatg ctttggcatg agtatgagca aattccctct ttcctgaatc atggacattc 660
   tagattaaaa gaacattttt gggctcttaa caagaaaacc ntgg                 704
<210> 285
   <211> 720
   <212> DNA
   <213> Homo sapiens
<220>
   <221> misc_feature
   <222> (1)...(720)
   <223> n = a, t, c or g
<220>
   <223> nbla-11234-f
<400> 285
   tgttggccta ctggtgtggt cctaagacct ggagaggaca ggctaagtga agtgtggtcc 60
   ctggagccta caagtggtct gggttagagg cgagcctggn aggcagcaca gactgaactc 120
   agaggtagac aggtcacctt actacctcct ccctcgtggc agggctcaaa ctgaaagagt 180
   gtgggttcta agtacaggca ttcaaggctg ggggaaggaa agctacgcca tccttcctta 240
   gccagagagg gagaaccagc cagatgatag tagttaaact gctaagcttg ggcccaggag 300
   gctttgagaa agccttctct gtgtactctg gagatagatg gagaagtgtt ttcagattcc 360
   tgggaacaga caccagtgct ccagctcctc caaagttctg gcttagcagc tgcaggcaag 420
   cgttatgctg ctattgaaga agcattaggg gtatgcctgg caggtgtgag catcctggct 480
   cgctggattt gtgggtgttt tcaggccttc cattccccat agaggcaagg cccaatggcc 540
   agtggtgctt atcgcttcan ggtangttgg cacaggcttg gactagagag gagaaagatt 600
   ggtgtaatct gctttcctgt ctgtagtgcc tgctgtttgg aaagggtgag ttagaatatg 660
   ttccaagggt ggtgaagggc taaaatgnac gcntttaagg ttgnancccc tgtgcaaggg 720
<210> 286
   <211> 758
   <212> DNA
   <213> Homo sapiens
<220>
   <221> misc_feature
   <222> (1)...(758)
   <223> n = a, t, c or g
<220>
   <223> nbla-11234-r1
<400> 286
   gngngggnnn nnttgnnncc ntnnttnnnc cnntttggat tctaacactt tattaagagg 60
   tcacaagcca caggacttta aagtgcatga aatttattgg caatgaagcc gcatgtatac 120
   caggctcccc tagtccccac caccttgccc catccatcat gacgtggtgg ggaggggttg 180
   agacccatct ttaaaagtgg gggacagcag ggacaaagga tgggaaactg gaggaacagg 240
   agtattcatg aactgaagct gggacaggag actgaccaca gtgattctgc tcactgaagg 300
   aagggtgggg tcagtctagt ctctcaaagg ctggtgaaaa gttttttttc acctattcct 360
   cacagcgagc ccctggcatt tcaaatagac ctaactcctc ctcttcatct ttctctggtt 420
   ttctcctcta tgagtaattt ggaacccaca gtccagcttg agggtatccc tgatatgcac 480
   aatgaataca aaccacggcg gatgcagatt tcaggatcat gctggggcct ggcccatttg 540
   ggtgggagga gctttctgtc aggagttgct gggtgcgggg taccgtgtgt gcccttcant 600
   gtggtgaagt gagaacatgt gaatgtgtgt gcatgtncca tccaaaccgt aagaggcaac 660
   ttgggtcang aggaccccac ttnttccttt atgggacaac agtggantgg gaanaaacng 720
   gggcttttga ntccanaaag aaagganagg nctncttc 758
<210> 287
   <211> 714
   <212> DNA
   <213> Homo sapiens
<220>
   <221> misc_feature
   <222> (1)...(714)
   <223> n = a, t, c or g
<220>
   <223> nbla-11269-f
<400> 287
   gtggcctact ggagcatcct ccttgctaga catggacatg acatttttaa aggaattatc 60
   tggttacagc gttttaagca aactgttcta aggagtctgt acattgcatt attttttcta 120
   ttcttttttt cttttgcata tatgtaattc tggaggtaaa gaaaaagcct gccagggatt 180
   cagaaggcat cccactagcg atcagctgac attcctaact gaaggctgca atgtgttgct 240
   tattcatttt gtaccgtggg agctgcgggg actagcagag agctaaacta tgcatttcaa 300
   acagcagtgc ttgtgcagaa agaggggtga gagagaggca gccggcgagg aaagagcaca 360
   gctggacttt ctccttgntt ttatccattt ctgcaggatc atgtattcat aagggatgag 420
   gcgggccacg gcgatcccag gcctgagccg cggctaccca gtcagttcag agccaggccc 480
   tncactaccg gaacagagag cgctttgcac gatcaaatca gcatctttgg ttacacgaca 540
   gatccatgag catgagcagg agaacgaagt tgcgggaaca agatgtcang gtataagccg 600
   gatgccgcgg ccagcaccag aagcagctga tcgcctggag aacaagctga angctgagat 660
   ggacgaacac cggcttcaag ctacagaagg aggtggagac gcatgccaac aact 714
<210> 288
   <211> 715
   <212> DNA
   <213> Homo sapiens
<220>
   <221> misc_feature
   <222> (1)...(715)
   <223> n = a, t, c or g
<220>
   <223> nbla-11269-r1
<400> 288
   gaaatggcaa aaaatttaat ctcatctgta gtcctcctta ggaaaatcta atgtaaccaa 60
   attcccaaat cccattctga ggctctccat gncaaaagtt tnaatctctc gctcttgcct 120
   ttccaatagg ttctttattc tctcgctgcg ttccttccga agggcagcca gctcctcttc 180
   aatcttctgc tcaaggtgtg ctctgcgcag agacactctc tgctctagct tctggagctc 240
   acgttcatgt tgtgcctctg tttgcatctt gattttgctc tggtaggccg ttgagcagct 300
   ccatttcctg ctggagctgt agcctcaagg cctggcattc tgcttcttga gcctcatcta 360
   gccgtaacgc ttgagaggcc atcatttcat ttatactctg tcatactgct ctgcaaaatg 420
   gcaagttttc ttgtctgctc atctttcagt gtctttaaga ttgntttgtg ctcattcttt 480
   ggagtaactt ccaactggtg attcttgagt gctttatact ggttggnctg gactttgcaa 540
   gtgcctgaaa ctgnttttta atttgcattt ncatggcctt aagctttttg gctgttgccg 600
   aaagtncatg acatgccttt ctgtgcagnt ctctttttnn gcctcttatt gnacttcaan 660
   ctggttttcc nnttccgctg ggtgctgnna aaccngatca caatccatgc tancc      715
<210> 289
   <211> 746
   <212> DNA
   <213> Homo sapiens
<220>
   <221> misc_feature
   <222> (1)...(746)
   <223> n = a, t, c or g
<220>
   <223> nbla-11270-f
<400> 289
   gnngnnnggg gggnttnnnn ttgaanncct ttggcactgc cggcctactg gagatctgct 60
   tctccccggc tcggggcgcc gaggcggcgt ccgggaggtg tcttctgcaa aggttgcctg 120
   gcgctgtcca acatggngga ggcaccggca gcgggcgtta cctgcgagcg ggactaaccc 180
   ctgcctggcg ttccgggcgg cagtgcctgg tccagagtcc agggactcgg ctgcctgcgc 240
   gccccgaggc ggaccccggc tctgactgct cccctggccg cgggcttctc gactgggacg 300
   cggcgcgagg agggagcgcg gcggccccga gtctccctga gccatgggca acgaggcgag 360
   cttggaaggg gaagggctcc ccgaagggct ggcggcggcc gcagcggctg gaggaggagc 420
   tagcggggcg gggagcccct ctacaccgcg atcccggccg gcatggaggc ggatttgagc 480
   cagctgagcg aagaggagag gagacagatc gccgctgtca tgtcaagggc gcaggggctg 540
   cccaagggaa gcgtcccccg gccgctgcgg agtcgccttn catgcacagg catgcacagc 600
   atgatgtata tgtccgggca tatatgtaca gcttccgtgc acatatgtgc caacctgtat 660
   gtgccaacac ggcggtacat atatgtcgnc tccgtcattt attcttcccc antggggtgg 720
   ncataagaga tcctaagctt gtgatg                                    746
<210> 290
   <211> 761
   <212> DNA
   <213> Homo sapiens
<220>
   <221> misc_feature
   <222> (1)...(761)
   <223> n = a, t, c or g
<220>
   <223> nbla-11270-r1
<400> 290
   gnngnngngn nnnntttnnn ngccttnttn nnnnncnttt tataaagtgg aggataagga 60
   tctacaccta tatatccagg tctttcgtgc ttacagtatc tcaaatgaaa aattgtaggt 120
   ttgccttcag ttgtcataaa agaggaattg tttcagaacc acggacagaa aactgttctc 180
   tactagaggg tgcaaagtta aagggtttgt ttacagtaag aaaggcaagg caggcacaat 240
   gattataccc ctctcagtac tctaattaga aaacaactaa tctattggga tgaaggaaaa 300
   tggagagtta caggcggaaa tgaaaacatt catattactg attagctctt atacttgcct 360
   atgcaagttg ccatttattt gctttgtttt taaaagtatg tattgagaat tattttctaa 420
   ggtaataaaa ttcacatgtt cttccctaaa ctgaaggcca cttttgcata aaacagagca 480
   gtggcttaaa aatagcatat gctaatttag aaatatatgt acagcacctg ctctgctgtg 540
   accaaaccat ctgtttaatg cccacatatt ttagggtcct caatgaatat aaaaggnata 600
   ttcattctat tccttctacc taaacagctt ctgctctcct ctgggggtgg ggtggcaaag 660
   aaanggcaaa tcacccacaa nggggattaa agttacagaa atatttagac atcattcaca 720
   gagaaaaaag acccccaatg ggccaaaang tttaaacaag a                    761
<210> 291
   <211> 731
   <212> DNA
   <213> Homo sapiens
<220>
   <221> misc_feature
   <222> (1)...(731)
   <223> n = a, t, c or g
<220>
   <223> nbla-11279-f
<400> 291
   gngnngngnt gnttncattt gaannccttg gaacactcct ggcctactgg catgtgaaag 60
   cactttgaaa attgtaaagc gctatgtaaa tgtaaggtat tatngaaaca tctttaacat 120
   atagtttcat accattcatt ttttaacaaa gaaagggaaa agtctgcttg taagctggtt 180
   gaaaaagtta atcttgatat aaatttgtgt ttgataaata tcctctcagt gttttatctt 240
   ccatgtttca acaactattg aaatatgaaa tgcctgtgaa ctcttaaagc ttcatgagca 300
   gctgcttgag ttcgggaagt tcactgttag aaataggctt tgttagctga ctagggtcag 360
   ggaaactttt ctcttcaaat ttgaaagctg nttctgtttt cattttacat tattattcag 420
   aaatggtagc tattctatac ctatggttaa gtaaatattt ctgaataagg cttcaccata 480
   ctgtaagcat tttaggtaga ttgccttaaa ggttatggga gggcatgagg gaacacttct 540
   tatgagaaaa catttataaa caaaagaaac atttataaac taaagaaaaa ctaaaagaat 600
   gacagaacaa tcatcttaac accctttctn acaataatat aaaaatatta aaagaacata 660
   ggcaggcttt ttttaaattt ggcttttttc tttccttttt tcaaattgac ttttataggn 720
   atttnctgga a 731
<210> 292
   <211> 734
   <212> DNA
   <213> Homo sapiens
<220>
   <221> misc_feature
   <222> (1)...(734)
   <223> n = a, t, c or g
<220>
   <223> nbla-11279-r1
<400> 292
   gnagnggnnn nnttgannnc cnttganttc cttttttttt ttgggtaaaa tcaaaatata 60
   ttttacttcc cattagattt taaaacaatc acaataattt atcaacagtt gccatttcac 120
   agcaattaga aaagaggagt tgatacaagt tgaacagaaa ggtatctttt cttcttttag 180
   gaaatatata tatatatgta tatatatttc acatacacac ataacattat ctttaaaatt 240
   ccaagtggtt acttttgtat tataaaaaaa ttatctccct atggtcaaaa tactgaaaaa 300
   gcgcaaaggc tgaaaaaaga aaaccacatt tgccacttta aaaaaataca ttattgtctc 360
   agtggttcta tttatttaat tgattataaa gtattatgac tcctagtact gtactggacc 420
   tgatggaatt aaatacaccc ctttctcagt ctccagaaag gatactccta acccacccta 480
   aaatatgata aaataaatgc ccctattctg aactgttaaa ctgggaaata aaccatgccc 540
   acctggaaac aatataagtc ataaaagttt atgaatctgt gggagagact acacgaatca 600
   gaaagcaatt ctttggatat aagttaggag cttttaagaa gtatataaac tcttttangc 660
   atctagagta gagacnccaa gaggatttct tcataaataa tgatttancc agngcaatgg 720
   ntggttgcca tggc 734
<210> 293
   <211> 761
   <212> DNA
   <213> Homo sapiens
<220>
   <221> misc_feature
   <222> (1)...(761)
   <223> n = a, t, c or g
<220>
   <223> nbla-11307-f
<400> 293
   gaggnnnnnt tgnnnccntt nttgancccn ttgnncnncn ncnntgnnng atnnggataa 60
   gctgctctca ttgagtagta cctcagnttc ttcatcttta aatgtaagta tctgccttac 120
   ttacagagta tatgtgaatg taaagtgggg taattacatg aaagtattct gccaatgcag 180
   tgtgatttta ttaatactag tttccttgtc tcacattatg ctcatttcca tttgtgtatt 240
   tctgctactg ctacctacaa ttgtcttctt aaattttaca taatcaaagt cagtctctga 300
   gcttctagct acccacaact gtgcaactcc tgtacaactc ctcctgcgtg cccacaggtc 360
   tgttacaagt actcagggtt acactggtaa ctcttctctc agtccctatt ctgcatgtag 420
   tcaaccggnt tcctatttag tgtctaatgt cttggttttt ttttttttat tttcttcatt 480
   agatttgaaa tgacttgatg gcaaggaata tatactttat atatggttta tacatttcat 540
   acattttctg gnatttttat aggcctggaa tggaccttgg agctgaaaaa aatagacntt 600
   acttggnatt actataagct agggaaatag aaagggttaa tcccaatata ttnctttata 660
   acctggnggg taaccattcc tttctggatt ctgaanagnc cctacctttg gatacctata 720
   ctaaccgaan ttttcagggc ttntgaacct taannaanat c                761
<210> 294
   <211> 751
   <212> DNA
   <213> Homo sapiens
<220>
   <221> misc_feature
   <222> (1)...(751)
   <223> n = a, t, c or g
<220>
   <223> nbla-11307-r1
<400> 294
   gnagngggtn nnttgngtgc cttnntnnnc ctttttgntt tttttgagac ggagttttgc 60
   tcttgttgcc caggctgaag ggcaatggca cgatctcagc tcaccgcaac ctctgcctcc 120
   cgggttcaag caattctcct gcctcagcct cctgagtagc tgggattaca gacatgcacc 180
   accacacctg gctaattttg tatttttagt agagacgggg tttctccatg ttggtcaggc 240
   tggtctcgaa ctcctgacct caggtggtcc acccgcctcg gcctcccaaa gtgctgggat 300
   tacaggtgtg agccaccaca agactgagtt tatctatctg tttcatctct ttgatttgta 360
   tttgtgtggn tttacgtagg aggcagtgat atggcgagta taaggtagaa gaaggcataa 420
   ctgtgagagt ggatatggta tgcaatgcat acagaagagt catgaatggc tacactagaa 480
   taaaacacct accaagatat gccatgctaa aaagctggga agtctatcag gaangcaaca 540
   gaaaacagtg gacagacatt caaatggntg catataagca tttnctntca gttaaaaagc 600
   nccactttaa angcnggaaa tccngaccta aatnccactt ggtactactt tttggttaaa 660
   gcacaagctt nttaaaactc ctaaaattat tggataactg naaaanaggg taattgnncc 720
   cttttaaaac ttggttnaac ttaaaaaaaa a                           751
<210> 295
   <211> 735
   <212> DNA
   <213> Homo sapiens
<220>
   <221> misc_feature
   <222> (1)...(735)
   <223> n = a, t, c or g
<220>
   <223> nbla-11378-f
<400> 295
   gnnnnnnnng ngnnnntgnn gnttgtnttn ganaccttgn gcctgctggc ctactggtat 60
   aaagtggtgg caagataggg aagggataat ctgagaattt ttaaaagatg atttaatgag 120
   aagaagcaca attttgattt tgatgagtca ctttctgtaa acaatcttgg tctatcttta 180
   cccttatacc ttatctgtaa tttaccattt attgtatttg caaagctagt atggttttta 240
   atcacagtaa atcctttgta ttccagactt tagggcagag ccctgaggga gtattatttt 300
   acataacccg tcctagagta acattttagg caacattctt cattgcaagt aaaagatcca 360
   taagtggcat tttacacggc tgcgagtatt gttatatcta atcctatttt aaaagatttt 420
   tggtaatatg aagcttgaat actggtaaca gtgatgcaat atacgcaagc tgcacaacct 480
   gtatattgta tgcattgctg cgtggaggct gnttatttca acctttttaa aaattgngtt 540
   ttttagtaaa atggcttatt ttttcccaaa ggtggaattt agcattttgn aatgatgaat 600
   ataaaaatac ctgtcatncc cagatcattt aaaagntaac taaagtgaga atgaaaaaac 660
   aaaattncaa gaccttttta aaagaatggc tggcctacac acttttatgg attggttttc 720
   ttacatccca tcttt                                                  735
<210> 296
   <211> 737
   <212> DNA
   <213> Homo sapiens
<220>
   <221> misc_feature
   <222> (1)...(737)
   <223> n = a, t, c or g
<220>
   <223> nbla-11378-r1
<400> 296
   ggnggnnggt tnctntgagg ccnttgtnna nccttttact aagaaagcca gtaactttac 60
   tgaattgtct gcaaaataca aattatacct tatttccagc aggagaagct tttgaaaatt 120
   tcccactttt attcactaca gacagctgaa ttaagtccct tgagatacac agatgtggtt 180
   taacttagtt ataacatctt gtcatctagt ggcgacaaag tccagcttat gccgctgtga 240
   gcctctattt gaatatttgt tgcaaatgct tgtatgccct catttacaag cgattttaat 300
   tactctcatt taatggaaac aagtccccag gaaaagaaaa agaagaaaaa tgatacagca 360
   aaccatctga agcaatccaa gtcctatgcc tacagtgacc caggtctact ttgctcgaag 420
   agaacactgg tactgcattc aaaccacaac agaatccgtc acttaactga cctctctgaa 480
   ttaaactgag gtatgaatca atttgaggta tgaacaggga aggcaaaatg caataccaga 540
   ttatgaaaat aaccaaaatg gaaaaatttg atgtagaaga tatgtggctt ggattgctcc 600
   acatctttgg gttaaaacat attaagaaaa gcctttaatt acatctgnag ctgaagcatt 660
   ctaacagaca ccagtatttc agcaatcttc aaaagatctg tantggcagg gaaattaacg 720
   ccataaaagt gacgaca                                                737
<210> 297
   <211> 723
   <212> DNA
   <213> Homo sapiens
<220>
   <221> misc_feature
   <222> (1)...(723)
   <223> n = a, t, c or g
<220>
   <223> nbla-11379-f
<400> 297
   gggnnnnnnt ngggntcnna tttgaatcct ttgagcctgc tggcctactg gcagatgctt 60
   tttctttttc tgttggaaac tgaacacact acagacagtg aaaaaaggta catattccat 120
   tttctcattg cctgaagatc tctgctgatg ctcctggaga atgactttgg gggctttaga 180
   aagaatattg ccagtccgtc tcggcaagga gatgatggga gcgctttata tggaggcttt 240
   acatgacttg taaattaaat gtgaatgagg gcagttgatt aaaattggta ttacagaagg 300
   gccctgctga ggtttgaaaa cagctgagct gctgatgtct caggcctttc cctgaattag 360
   cactgcggtt ctccaggata tcagcaaaga gggcaagtaa tagaagcccc tgataaggag 420
   cgtcagccga caggcaagct tgggaggctg tgggaatggg tctgccccca gcttcacaga 480
   cctcttcctt cagcctctga atcccattag ccacagccta gaacattagc tgagctgcac 540
   aagctnaccc acccctgtgc caaggggccc tgacctcctt catgccatgt ttttggctgg 600
   atctacggna cttaacaata nggggccttt tattttcata cagagatatt ttgaaaaatt 660
   taaaaagaca tgaactnaca ttaacngnta tggatgatag gtnaaaagag aaaccggtgg 720
   agg                                                               723
<210> 298
   <211> 718
   <212> DNA
   <213> Homo sapiens
<220>
   <221> misc_feature
   <222> (1)...(718)
   <223> n = a, t, c or g
<220>
   <223> nbla-11379-r1
<400> 298
   gaggggtnng ttgtgtncct ttgatccttt ttaatatatt cagagcacag aatttattga 60
   acataatatg gagacagcta gtacaaaagt ttatgccttg gcagggcagg tttcatcagg 120
   ctgataatgg aacatgggag aacatgctgt aaatggctct tttggtctgg accctgcaga 180
   aatacatctc cttctctctg ggaaacatca tgtcagctgc caagccatga atttattaca 240
   tatcagaaaa ccaaaatgtg caacagtaaa gagtcctttc ctgcaccaac tttttaaatt 300
   acttccatgt caaaacagca aatacaatga aaaatacata aaggacaaat tatgaaaact 360
   aaagcattct aagacatctc ttaagatact cagttcctaa catgttaaca tacattgaag 420
   actataaaca cacacatcag aaactgtggg aatctttttt caacataggg agaaagacgt 480
   agaataggga gagacagcat gggttactga aaccatttat tcagaaagca gaggaaaggc 540
   gaaaggacac tgaaatanac atttttaggt tcttatagga agttccaaat ggcagcagtg 600
   ataaatcgnc tttaaaaang gaacgacagc accngaaatg agaacccaat ntattttgca 660
   tttttctctc ggngnatcat cttgaggcac ccttacttaa gcttcacaac gaatggaa   718
<210> 299
   <211> 726
   <212> DNA
   <213> Homo sapiens
<220>
   <221> misc_feature
   <222> (1)...(726)
   <223> n = a, t, c or g
<220>
   <223> nbla-11469-f
<400> 299
   ngngnnnggn gggnttnnnt tgacnccttn gncctgctgg cctactggaa atatggggct 60
   tcttggtctt tatatgcaca tctgagcatg ctactgaagg cagaggaagg gagatctctt 120
   agggagtcag cacatgcaaa atgctgtgcc ttcagactgc ctctgctacc tcttacaagg 180
   agcctcacac cgcaggctgc cctttagctg ttcccttgga atgcagcctt gttaagtcac 240
   tgccagcgtt tcccctcttg gcgtcccttt ctcttaccta agttgttaac tccagacttc 300
   gctgctttct taaaggctaa agtaatgggc agagtaaaac cctgaaatgt ttagaagcta 360
   tgcagtatta tattgtaaac ctgtaaaaat tcttaacaaa gttagtggct tggaaagatt 420
   ttcatgagaa cttcagctaa aaggcagtaa agtaaatgcc ttaaaataac taaactttgg 480
   cttataacaa ggccattgtt tggcaagtgt ccacttttta aagacttttc agaacttgga 540
   ggaggaaatg atcctgagac aaagtagtga tttaaaaaat gaagacagta aattaacatt 600
   gacattattc gncaacaatt gattcttggc tttcaacttt ttaccctgca ttctgaataa 660
   atggaccatg tgaaggacta gaagattact ttaagggttt ggtttgattg gaaatgggnt 720
   tggaaa                                                             726
<210> 300
   <211> 3121
   <212> DNA
   <213> Homo sapiens
<220>
   <221> misc_feature
   <222> (1)...(3121)
   <223> n = a, t, c or g
<220>
   <223> nbla-11473
<400> 300
   ctgtacggaa gtgttacttc tgctctaaaa gctgcggaat tcctcgagca ctgtggcctt 60
   tttttttttt tttttttttt tttttttaat gtgcagagga ttgacctgtg catgcttttg 120
   atctctcatt caaaggatca atattaaata aaattgtcat gagctgtgtt gaagacaggg 180
   tgctttcaaa tagaggtaat ttgctcttgt gttgtaagag gaacatgtca acaaagatag 240
   gaaatgaggg tgatcgtgca gatggcttgt atcttatata tgcaaaggag ccaatctcag 300
   aagcacaaag aaaaaagtgt gcatacctta ttttgtacag ataaagatga tgtctttttg 360
   ttattgtctg tctgttttgt atgtgtctga gataagggat agagaggaaa catccgtcag 420
   gctaatttaa ctacatttta ttttaaaaat agagaaacat aacctctaga tgggacagca 480
   gaggacagtt agtagaggcc acaaactgtt atgggctgct gtgttttgtt ctaaaatcaa 540
   tatggttgga gcatgtatat cttaggtgat catttcacat cttaggaatg cctactcatt 600
   ttattttatt ctagtgatgc tcaattcact atttaattta ttatattttc tcttctgtgg 660
   cacttataca aaatatctct tcacctactt agttctacag ggttttaact ttggagcaac 720
   atgaataaaa tcatcgagaa ggccaatatt gtttagcaac atgaatacaa tacagtttaa 780
   agttgtacac atcctgctca actttattca tatacatttc ctttctgtgg ttttcttttg 840
   cttcttagaa attctgttag tggttagtaa agaatttgaa agtactttct ccttgctgtt 900
   tttttttttt tttaagacat tcctcccaga atactccagg gggcagtgtt ttataacaca 960
   ttttccccac tgggtgattg aaggatggag gatttttgaa aatttgacag ctacatgaaa 1020
   catgagaaaa cattttcctc acttctgaag tcggtttgca gctggtaact tgttcatcca 1080
   gaaaacattc taaagcaatg agactttgtg agctgtgctt acagtttggg agaatcatga 1140
   agattctttc tatattttgc atttacttcc cagtgcttca tagctgcatt ttgtttgtaa 1200
   ctaagacaga agaatttcgt aatccttgaa attgaaaaaa aaaaaattgt gtttttaaag 1260
   agtgaaaaca gttagaaaac aagtagaact gtaatcagaa cgctgcttca attgatatta 1320
   aaaataacct caataataat gtaaaggttc ctttctcttg tgtcagttat attcttaggg 1380
   atagcctaga aggaatatat ggttagaact aagtgtgact aatcatctga gccttgaaga 1440
   gaaacttcag tgcctctaaa cagatcatct acaaaacaac aggtaaacat ttatgccagt 1500
   taagtgggtc atgtttttgt ttcttgggtt tttcctaaat ttaagtgagg ttgggcttac 1560
   cttgtagata aaattatgtt ttctttttgg taaatacttg aacgtggata acgtcaaatc 1620
   agaatatttt gtgaggaggt gatgatttga aattaagcta gatttctagg gaggtgttgg 1680
   ttccaatgaa ggatgggaag aaattaaaat agtcttcaaa cttcttcctt attatatttg 1740
   gttgctttgg aaaagattgg tcctatcctc aatctaattt attcactatt aatattttaa 1800
   aaaacattcc tgagatactt aaaaagaccc acttagcgat tatagttgct caatgaaaca 1860
   agaatttatt tatgcataga tttttctctg tatcttacca aaatccactt tacttagata 1920
   acactaaatt gttcttaaag actactcatt tcccaataat cctttatgat ttcaaaattt 1980
   ctagtggctc agaagtgaat tttattttat ttgtctttca cttgaataaa tgagaaccca 2040
   gaaattaata atgttgttta ttgcttactg tcaggactat ttcaaagact aagaagagtt 2100
   tcttctaacc cctccctctc aaaggaatcc taaattatta gttgttagat aagttttgta 2160
   tgctaagata ttcgggttta tagtttatgt atgtgtgtat atatataaat atatatgtat 2220
   atataaatat tatgttcagt ttggagtctg gcacaactcc attatgtgga ttagagagta 2280
   agatattatg ggtgataaag tactaaatga aacataatat ttatttataa aagtgtgtag 2340
   attgttaaat cacaaaaaga gtgctatgac cattatgtat gaggaaacag gcctttgacc 2400
   tcctggaaag cactgctcaa aagtcattag tgcccatttt tgaattcccc aaacagaaag 2460
   cttcttagaa aacatgctga gattttattt acagggaatt ctttgacaca tttcaattgg 2520
   tgtgtagtca agtatagcaa gtacttaata atgactgaat ttcatgttcc tacagtcata 2580
   catattcatt agaagtttta tgttgttggt ctgatctgat tcttctttgt ttgtgggtgg 2640
   aacggcactg agagaagtat agttttttaa acttgaacat gttcagtagt tacattgcct 2700
   tagaaaaccc agacacatag cagtggaaat gaaagaaatg gcatcagaag tgacttaatt 2760
   tagcaattgt gattcctctt gtaaaacaaa acaaaaaaac aatgccatat tttttggaga 2820
   aaagttggca atataggggt ttcgttgtct gtttcacaag aagactcatt tgttcttttg 2880
   ggggaaccag tgccttacag attttgtata tactgtaatt attcaggact agggaacaaa 2940
   caattgtatt gtatttgtta cagattgtat atggctttgt tttaacattc ccctaaataa 3000
   aatggcttca ttctcccctt ggaaaaaaaa aaaaaaaaaa aaaaaaaaaa aaaaaaaaaa 3060
   aaaaaaaaaa aaaaaaaaaa aaaaaaaaaa aaaaaaaagg ccacatgtgc tcgagctgca 3120
   g                                                            3121
<210> 301
   <211> 718
   <212> DNA
   <213> Homo sapiens
<220>
   <221> misc_feature
   <222> (1)...(718)
   <223> n = a, t, c or g
<220>
   <223> nbla-11483-f
<400> 301
   gnnnnngnng ngnttnnntt gaganccttg agcactgntg gcctactggt ggaccaacaa 60
   aggggccaga tgagtgaatt gctgaaagca tcttgctgta agacagggtc acaggacaca 120
   acattccaag tcatgtctct tcgaaacatg caataactta gaatcagcat ggtggtcata 180
   tgctcagaca gttcagggag caccatgact cttgggcgga gcacatttta aacttggtga 240
   atttaaattt cttttctttc ccctagcctt aaatggcctg aaatcctgaa gtgaataccg 300
   aagacatctt aagagcgtgc ttttagtgat aaatgcctat gtttggggtg ttttaaataa 360
   tgaggttgtt tgaaaagtag tgagaagtct ttccataaga atggtttgtg actggatgtt 420
   atatattttg gtttacaaaa ttgtctgaaa acctttctgg aataagtcag ggtctatgta 480
   aataaatctg cataaggaaa gtactgtgct ctttctaccc attcacccta gagactgcag 540
   gtttttaaat tttatggact tcacttcana gcagtttang gtcacagnac anttgaagac 600
   tgcancgggt ttaacacang tgncttcctg ggctcttntc ataactgttg caacccaang 660
   gaatggcttt ctaaggagac ntaaaatctg naaggctgnc ctgtgcttaa nccaatgg   718
<210> 302
   <211> 703
   <212> DNA
   <213> Homo sapiens
<220>
   <221> misc_feature
   <222> (1)...(703)
   <223> n = a, t, c or g
<220>
   <223> nbla-11483-r1
<400> 302
   gngnnggggn nnnnttgagn cccttttgan tnctttttta aacaattgaa tcctaagggt 60
   atttttcttt tggcgctgaa acgatattaa aatttactgc aactttattt aaaaaaatag 120
   aaaactaatg tttctctggg tcttccattt ttgaataaaa ctgactctta ccacccaaca 180
   cgtggatttg gaaggatagc agtacaccac cttcttagct cacttggctg gaacatgagc 240
   tgatgaagtc ctagtctcaa gtttggcctt caagaaaact agataggctt gccctatgcc 300
   ggagccaatc tagacctgcc atcagttctg tttcttcttt gtgtgtgtgt gtgtgtgtgt 360
   gtgtgtgaga gagagagaga gagagagaga gagacagaca gacagacagg gtctcactct 420
   gtttcccagg ctggagtgca gtggcatgat catggctcac tgtacctnaa ccacccaggc 480
   ccaaagtaat ccttccacct caccttcaga gtagctggaa ctcaagggca tgctccacac 540
   ccagctaatc ttggattttt tatagatcca ggatcttgca tggtgctaag ctggctnaaa 600
   tgcccaactg gccttgactc cnggctcaag aatcatccnc ttggcttcac annggtggaa 660
   tcaagggngg caacccacct ggccatnant ntgtttttga cca             703
<210> 303
   <211> 2650
   <212> DNA
   <213> Homo sapiens
<220>
   <221> misc_feature
   <222> (1)...(2650)
   <223> n = a, t, c or g
<220>
   <223> nbla-11485
<400> 303
   ttctaggcct gtacggaagt gttacttctg ctctaaaagc tgcggaattc ctcgagcact 60
   gttggcctac tggcctgctt ggtggtggaa ggttatatag taaacttcac tgtcattaag 120
   aaagcgcttg tccaaaatgt ttaaagtact agaaaaggga taattcagca ctttttctgt 180
   tacaacacta aagtcttaat ttatcttcag agtcttaggt actgtaagtt tttggatgat 240
   tatttaaaaa tataaaacat actacataat atggggtcag atcacattta tataaataat 300
   tcttatttta aaaaagacaa taacattttg tacctttcta gcaattccaa gtgattgcct 360
   cattttctct tgttaagcat ttcaagctta agaatttgga atttgagaga aggtgagggg 420
   gaaaattccc taaaataaaa atttgtttca aaccatttta gaaaaataaa tcactaggaa 480
   aataggtagg gattctctcc ccctttaagt cttcagggaa tgagcagtat ttagaatctt 540
   tggctgaaat tcaagccttt ttactttgta aatttttaat attaattcac tgacatgctt 600
   tttacaccag aggactgaaa atggatctta atgtttttaa gttgtggaag ggtatatttc 660
   taaaatgagg ggggatgatt aactaggtta gtattaccag cattagtaag tttaaggaat 720
   tttagtatgt aatagcaaag tgtttgatta aaagtctaat cctatattac tagtcaaaat 780
   tagttacatt ctgatctagg aaagataata gtcattggat attgatatgc tgtgctgtca 840
   gaaaaccaga ctaagataga aacctttatc tgactcccac atgttgcttg ttgtgatggg 900
   atgtatactt tacagcagct tattgctgct ttgaaaaaaa atgtataaac tatacatttg 960
   gagtgtttgc atataattct ttataacctc cacttaaagc tgtcagacat tggtatttta 1020
   tcagtccaca ttgttgaata aaactaatgt tcttaggaat ccagcttgta cacactgttt 1080
   aaaaaccctc agggacagtt tacacactct tctcactcaa ttcaggtact ttgatgctat 1140
   tcttaaacct aacagtgact tgtatttttc tgttttgctt ttatttcaac gtgctggtag 1200
   cacatctgat gaatgtcaac ttaaaaaact cagttcaggt atccaggtat aactcagcca 1260
   aacagatttt aaagctgcat ataactctcc agcacatggt gcctcacact cttatagtgg 1320
   cattctatat attcagttat tactactgag cagataatat gggggttcct gttaacagtg 1380
   tattttttaa aaaaagcgca taaatgtata gccagcacac tacacacaca cacacaaaca 1440
   tgcacagtta aactatatat ttttaaatgc cactaatagc cagcacaact aaaacaaaag 1500
   acattcctaa ctgctctgta agctgttaac agatgcagtt ccttcttgat gtggctcttg 1560
   cttcttcacg ataactacta ctaaattcaa gcactggtcc ttgggtgtct gacctctaca 1620
   ttctagttta tgcaatgtct ttagagaatt ttgtgcactg gccactgtga tggaaccatt 1680
   gggccaggag tgctttgagt ttatcagtag tgattctgcc aaagttggtg ttgtaacatg 1740
   agtatgtaaa atgtcaaaaa aattagcaga ggtctaggtc tgcatatcag cagacagttt 1800
   tgtccgtgta ttttgtagcc ttgaagttct cagtgacaag ttttttctga tgcgaagttc 1860
   taattccagt gttttagtcc tttgcatctt taatgttaag acttgcctct ttaaaattgc 1920
   ttttgttttc tgcagtacta tctgtggtta acaaaataga ttatttctct gctttaatat 1980
   ttgatatctt acatctaaaa taaattctct ccacatataa aacccatagc ctttggagat 2040
   atggaaaatg gtatctttca gatttctaga agttcaagtg tcatacaaca aaacaggaac 2100
   cccctttact cttatggacc tcatttcaat atactgttta cagtttgatg gaattgtata 2160
   atttaatatt tctcttgtac tgtagtttat atttatttac agattttttt gtactgtgtg 2220
   atttgaactt tttgttcctt gctatgatca atgtttatgt agtagagcac ttatgatcac 2280
   aaattaagtt ttttggtttg attgcactac attaaatttt ttaatgcagt tctgattttt 2340
   gactggacta aaactgtgtc ttaatgtatg tgatgagtac ttaaaatttt aatccatgtg 2400
   gtcccccccc tttttttttt ttttgcattg tatgtcaaaa gcgcttgttc tttcgtgcat 2460
   gtgtaagatt taatggttcc attgtattat ttgaccatga cattttggag aaacattccc 2520
   agctgtaatg ttgtgtatgg tagttctcac tggatgctag agttttcaaa accactattc 2580
   ttctaataaa ttttgttgtg aaaaactgtt aaaaaaaaaa aaaaaaaggc cacatgtgct 2640
   cgagctgcag                                                    2650
<210> 304
   <211> 743
   <212> DNA
   <213> Homo sapiens
<220>
   <221> misc_feature
   <222> (1)...743)
   <223> n = a, t, c or g
<220>
   <223> nbla-11519-f
<400> 304
   ggnnnngngt ngnnncnnnt tggnttcctc ggcactgttg gcctactgga atcgctgcag 60
   taacttcagg gctgtttgct ctgctgctga cctcactgta cgcaaattag cccttcttct 120
   gaggctcaac tatgctatgt ccattaccct agttactgct cacaatctac ataatgtgac 180
   agatcatgaa acatgaacta gtaaaatgtg tatttataaa gtaggcaata ccgttattgt 240
   tttcagagga aatgacagta tgtctagaga gctcatgaaa aatagaagag atagaaaatg 300
   ggtattccaa agcctagacc ttttaaaagc aattatcaat cataagctat aatgaaaaga 360
   atgtgattca caacagtggt tcttctgtga gccagatggg tgtggattcc attcagaagt 420
   agtgcatagt ccccaagctt gcacctcact tgagccctgn atccccaaat cattctgttt 480
   ttgtccagag tttcacaagg ttgattttga aaatgttccc accagctctg gctatgggaa 540
   tcacagtggg aaataaacgc catagcatct tccccatcat ccccgtctcc tcctgagaca 600
   aaacacattt tcccctaaat gctaactggg ctgctgcctt cagcatagac aatcttttac 660
   atttttacca caaagtagtt ttaactgcct ggccacatac caattgaata gtatgatgtc 720
   tgggtaaggg cagaatantg gnt                                         743
<210> 305
   <211> 755
   <212> DNA
   <213> Homo sapiens
<220>
   <221> misc_feature
   <222> (1)...(755)
   <223> n = a, t, c or g
<220>
   <223> nbla-11519-r1
<400> 305
   gnngnngggn nnnnttgtgt ggcctttttt ttttnttttt ttgaaagtaa caatatcatt 60
   ttattgcagg gtggcatagg tagttgtact tattaaatac atgacaaata ggtaccagca 120
   gatgagttag atgtagggat acagatggaa ataaacttcc atggtcccca ttcatcaaat 180
   ttattgtcct ctgagaaaga catgagttca tctgatcatt acagaggaga tgagtgtttt 240
   gaaggagaag cagagggttt tgtggatgtg attagcaggt ggacctaacc agcacagtgg 300
   gtcagggagg gattttctca ggaaataaca ttcaggctgg agtctgaatc ttgagctctg 360
   aagcaggtgt caggacattc agagcaaagt ggattatatt tgcaaaactt cacatgctaa 420
   aagatgagag aagtccagtc agtagcaagt tgttctttgg aggtcctcag aatatgtcac 480
   agcttcttga ttcctcctta catgcctgta agagatattc actatccaat caattgttta 540
   tcacagaccc tactatgaat gagattccat agaggataca aaggatgtgt aatacacttc 600
   aaagtaattt aaggctcang agcaagctac gaatgaatta agaaaataag gcaaagacaa 660
   gtaaatattc aatgncatta aagagcatgc cggaatataa gaaatggtgg tcaaactagg 720
   tccactcaat gggtcaagtt gangggcata tgtca                          755
<210> 306
   <211> 731
   <212> DNA
   <213> Homo sapiens
<220>
   <221> misc_feature
   <222> (1)...(731)
   <223> n = a, t, c or g
<220>
   <223> nbla-11530-f
<400> 306
   gnngnnnngn nnnnnnnnnt tnnnnttcct cgngcactgt tggcctactg gcttttcttg 60
   aaacatcttg attgcttttc ctcggcagct ttcaaaaaac caaataataa tagttatccg 120
   tcttctactt catggaagat tgttttggtg ccctgaccct ctgaagtgcc cagttcctgc 180
   catctgaaac ctcggcctga tctgatctca tgttggaatc tgcctgtctt tcacacaggg 240
   ctggtcttgg tcctttacat gccagttttg cttgtgaatt cttgcttttt tcctctcatc 300
   agccttaagt ttaggcgttt gttgttctcc agtgatgtag acagttccct tcacaagtca 360
   cagttcttcc cataaatgag gcccgctgac ctctgcggga ctttaaaaat ctattcagat 420
   atttccgagt aagtggcttg tttaaattct tcctgtgtct ttctttattc cttaattggt 480
   tggtggaaag aagagatgct tgggaacctt gggttcttag gtttggattc tttaataata 540
   tctaaaaagc taaattttaa ataccagctt tacataaatg attggtgact ctggtctgnt 600
   tctgacacct ttccagaaaa aagcaattgg tcaagtacac caaagaggaa gaagaactgt 660
   ggangccacc ctntacaaag ctttatagaa cttctggatc taacttacaa acaagctttc 720
   agaagagact n                                                      731

<210> 307
   <211> 766
   <212> DNA
   <213> Homo sapiens
<220>
   <221> misc_feature
   <222> (1)..(766)
   <223> n = a, t, c or g
<220>
   <223> nbla-11567-f
<400> 307
   gnnnnnnngn gggnnnnnnn nnnttngnat tcctcgngca ctgttggcct actggatcca 60
   gttcaggccg atcagggctg gtggaaacaa ctaccaagtg tctctctgtc tgctttgctt 120
   tgggagaaga aagtggaggg agccccagca tgttgtagag gaactggcct gcggaaacga 180
   gaggaggagg gtgcaggggg cagcggaaca gactgaacct cagagcatca gttggaatta 240
   cnatctgagg ctgtcagaga tgactctggt tctgtccatg aatagattct gcgagcccat 300
   tgtctcggaa ggagctgctg aaattgctgg gtaccaaaca ctatgggagg ctgacagcta 360
   cggaggccca agccccccag ggccagcaca agctcctttg cagggagacc ggggagctgg 420
   tcccccactg gcaggatcac attacagggg aatttcaaat cctataacaa catccaagat 480
   cacatacttt aagaggaagt atgtggaaga agaggacttt cacccaccac tcagcagctg 540
   tagccataaa accatctcaa tttttgagga acgagcccac atnctttata tgtccttaga 600
   aaagctaaag gttatcgatg atcctgaagt gtaccttcga agatctgncc ntataaacaa 660
   tttgatgaaa aggatccatg gagaaattat catgcnngaa tactggngct ttccttgctg 720
   gtctttaatg gnaccttttg ccaaantggt tatgntcaag actgnc 766
<210> 308
   <211> 772
   <212> DNA
   <213> Homo sapiens
<220>
   <221> misc_feature
   <222> (1)...(772)
   <223> n = a, t, c or g
<220>
   <223> nbla-11568-f
<400> 308
   gnnnnnnngt ggngnncnnn nnttggnatt cctcgagcac tgttggccta ctggagctct 60
   gtcttcatct tcaaaagatt tagaaacaat agttactctt aagaagacta tcagaatctc 120
   accacacagg gagagtgacc attctagtct aaataacaaa tatttgaatg gatgtggaga 180
   aatatcagtt tcagaaatga atgaaaagtt gacaactctg tgttatagga agtataatga 240
   tgtctctgat ctttgcaaat tagaaaataa acaatattgt aggtggtcca agaaccaaga 300
   tgacagtttt acaatttgca ggaaacttac atatccttta gaaactcttt gcaatagtcc 360
   aaatagaagt acaaatacat tgaaagaaat gccttgggga catatcaata acaacgtaac 420
   acagagctat tctattggtt atgaaggtag ctatgatgcc tctgctgatc tctttgatga 480
   tattgctaaa gaaatggaca ttgcaactga gattaccaaa aaatcacagg atattttggt 540
   aaaatgggga acatctttgg cagaaagtca cccttcagag tctgattttt cactgagatc 600
   actttctgaa gacttcattc agccttnaca aaaattatcc ttgcaaagcc tatctgactc 660
   tangcattca agacatgctc ttcaacacct nattttcaat cagattcang aatatnattt 720
   tgnaaatagt caagactttg gtccatgttc acagncaact tccaatttca ng 772
<210> 309
   <211> 771
   <212> DNA
   <213> Homo sapiens
<220>
   <221> misc_feature
   <222> (1)...(771)
   <223> n = a, t, c or g
<220>
   <223> nbla-11568-r1
<400> 309
   gagnggngtt nnnttgtgtg gccttttttt tttcnttttg gtttgcaaag tctttatttc 60
   tggatgtata tgtgatttta acaaacatta atactttcaa aaacaatctt aacaatatta 120
   ttaaagtaca ataaacagta taagataaag tgctcaacta cctaatcata gtattctaga 180
   taaaaccctg gggaaaacaa tggctttata ttttaaaagg cacagcagct cccagcagag 240
   tcctgttttc aatttgcttc tctccaagtg ttcaaaatgt aaagaatgct ttccgtaccc 300
   ctgaaggcaa acatttccaa acagatttaa aagttcagga attgggttca ggtgactttt 360
   tatgaaaaca attcaggtga ccaagcactt cgagtttcac acactgaagg tgggcctttt 420
   tctgaaaatg gaaggcaaca tttgacttct gaacaagaat caggtccaag aatagggtgc 480
   agctgaggtg ctgctaagac ttgaatgtct ggacatttgg aaatccagcc tgaactttta 540
   caggtatatt tatgagtttt ctttttcgta acaactgntg caagcatatt ctttaatttc 600
   ttcttaatta aacctttatt gggtccttgg ctaatatggt tcaanttctt tcttggnaac 660
   tcttggtgga tcaagggaaa tgattggcag caaggcattt ncctaganct atgncttggn 720
   atccagcntt tntgaaggga atattttttg gggggtagga ngganccntt n 771
<210> 310
   <211> 753
   <212> DNA
   <213> Homo sapiens
<220>
   <221> misc_feature
   <222> (1)...(753)
   <223> n = a, t, c or g
<220>
   <223> nbla-11600-f
<400> 310
   gnngnggnng nnnnnnnttt gaannccctt ntttgagatc cctcgagcac tgttggccta 60
   ctggaattag ctttagatac agcagtagcc ctttccttat gtccttgtga tgcctgagta 120
   aagaacatag tttttacttg ctaagctatg agattttgcc gcctgtagta gtttttaaaa 180
   tattgagtgg cctatctcca gatctcccaa acagattcat gaacaccatc ttgcttttga 240
   acatgtcgat tttaaagtac gaggccaata tgatattgcc ccaagctcat cattgttctt 300
   gatgagaaat gtagtccagg attcttagaa actttcttaa tgtgatcttt catttaatgt 360
   tctgtgtcat tttaaggact agaatatgct attttcttaa gtgttttttt ctcccttgac 420
   agttttaaac atattgtctt ttgcttccaa gcccttccat tgctgaacag tttttgtagg 480
   acagtcttca gctttatcat taagctgaat ccctatcaac atctagctgt ccaagtgagt 540
   tatagatggc cagtggctct gctttttact agcctgcacc ctcttagcta accttctttt 600
   tttagggttt gnatttttag ctataactta tttaacacta tcaggtgtta gatgccatgc 660
   caactggttt atgtacttga gaagcangtg ttaacttcct cagttacaaa tgagaaaacc 720
   cagcttaang ggattgactc atttgncaat agg 753
<210> 311
   <211> 751
   <212> DNA
   <213> Homo sapiens
<220>
   <221> misc_feature
   <222> (1)...(751)
   <223> n = a, t, c or g
<220>
   <223> nbla-11600-r1
<400> 311
   gngnnggngg ttnnattgag tgccctttgt taatnccctt tttttttttg agacagagtc 60
   tcgcactgtc acccaggctg gagtgcaatg gcaggatctc agctcgctgc aacctccacc 120
   tcccaggttc acacgattct cctgcctcag cctcctgagt agctgggatt acaggcacat 180
   accactacac ccggctagtt ttttgtattt ttagtagaga tggggattca ctatgttgac 240
   cagactcgtc ttgaactcct gacctcgtga tccaccccac tcggattccc agagtgctgg 300
   gattacaggc atgagccacc gcgcccagcc agcattgtat actcttccaa aggtacttct 360
   gaagtttgaa ccacatgaat aaattttcta acataaaaag taaatttatt gttcaaaagt 420
   tcattatcaa gtggcccaaa gctataaaac ttatggntaa catatttagc ctcaggaaac 480
   ttagctttag gaaagtcaat ctggagagac atgaatgaaa aatgattgcc tgctgaaaat 540
   taagcantta taatggttaa cgataataac agaaaaactt ctatggcatt tactatgtgc 600
   catttgaaac tttaccaaaa ctaactcatt taatccactc aagaatccgc ttaagtaggt 660
   actggtggta ttctttncat tttncanaag angagaaact gaggggtaag acattcagna 720
   ntttggncaa aggtacataa taagnnangg g                                751
<210> 312
   <211> 736
   <212> DNA
   <213> Homo sapiens
<220>
   <221> misc_feature
   <222> (1)...(736)
   <223> n = a, t, c or g
<220>
   <223> nbla-11618-f
<400> 312
   gngnnnnnnt ttnnnncttt gananccntt gggcactgtt ggcctactgg tctcatctat 60
   gatgtctttt gggtattttt ctcagcctac atcttcaata gcaacgtcat ggtgaaggtg 120
   gccactcagc cggctgacaa tccccttgac gttctatccc ggaagctcca cctggggccc 180
   aatgttgggc gtgatgttcc tcgcctgtct ctgcctggaa aactggtctt cccaagctcc 240
   actggcagcc acttctccat gttgggcatc ggagacatcg ttatgcctgg tctcctacta 300
   tgctttgtcc ttcgctatga caactacaaa aagcaagcca gtggggactc ctgtggggcc 360
   cctggacctg ccaacatctc cgggcgcatg cagaaggtct cctactttca ctgcaccctc 420
   atcggatact ttgtaggcct gctcactgct actgtggcgt ctcgcattca ccgggccgcc 480
   agcccgcctt ctctatttgg tgccatttac tttattgcca ctcctcacga tggcctattt 540
   aaagggcgac ctccggcgga tgtggtctga gcctttccac tccaaagtcc agcaagcttc 600
   cgattcctgg aagtatgatg gatcacgtgg aaagtgacca gatggccctc atggnccttt 660
   tctctnaact catgggttgg ttcctcttaa agctggnctg gnacttaaaa atgtacctgn 720
   ggttaaagga actgcc                                                 736
<210> 313
   <211> 733
   <212> DNA
   <213> Homo sapiens
<220>
   <221> misc_feature
   <222> (1)...(733)
   <223> n = a, t, c or g
<220>
   <223> nbla-11618-r1
<400> 313
   gnnnnnnttt gagggccttn nnnntttttt tttttttttt ttgctttttt cctttttttt 60
   ttttttctta aaggagtgag ggcatggaaa atatacagca caccaatgaa acaaaatgtc 120
   aaaaaaaaaa tacaaaaaaa tagaaaatag aaaaatgtat ttacaagtag tacgttacta 180
   tgatgagaaa gcacaaagac acctgctgct ataatacatg cattggctcg agaagaaact 240
   acggaacacc ctgcgaggga naagcctaga aaagaaagaa agggccaaaa ggtttgaact 300
   cttcatcccc aatttgctac actgatcaaa accaagtaag ggctcctgaa gtccatgagt 360
   ctatcatcaa tcagcacaaa tgctatacta gtttgtaact gcggggtcag ttgtgaaggg 420
   gaaggacagc agcttatcca tatacaagga agccacagta aactgctcga catgctcaaa 480
   acgacagcaa gcccctgtca gctacacagc gtgatcttag caggatttca tttttgntta 540
   ttcatatata tctatgtgtg ngtttatata tatgtccgta tgtataaaaa ccgtgccctt 600
   gttcactggc aacacaacat ntgggtggac atgaactcat tcaacaaatt cttatgtgga 660
   tgttgtaaga agcactcaat gnctggggat ttaataagca ctttacaccn cagaaaatnt 720
   ttaataaatc cca                                                    733
<210> 314
   <211> 2036
   <212> DNA
   <213> Homo sapiens
<220>
   <221> misc_feature
   <222> (1)...(2036)
   <223> n = a, t, c or g
<220>
   <223> nbla-11652
<400> 314
   taggcctgta cggaagtgtt acttctgctc taaaagctgc ggaattcctc gagcactgtt 60
   ggcctactgg ctagtttgca tttgcggaag atcccatgca gaaacagatg tcacactgca 120
   gctgcaagga cagtgaggag ggagatggcc atggtgcctt ggtccccaga gtgacggcat 180
   gcggaggcgt caatgcatct acctccagca caccaggcat gatgtcaggt gcagcaggag 240
   gtacctggcc ctttgctaca cagaccacat ggtcttgctg gggacaagac ctagggaaca 300
   gctcattttg gtacagtgtg gttggttcct ggagagggag agggaatagc ccacgggcta 360
   agcagcccac tgcaggtacc taatgcaacc aggaaggtca gggaaggaga tggccagcca 420
   cgcggtggag tttgaacatc atgtagcagt tagccaggtg aagaggagat gctggggaga 480
   cagggagagg ccactcctgg ctgagggacc tgtacctgca aagactctca ggggaggagg 540
   acggctttct gtcactgttt ctgtgtgtga gggaaatcag agggtaggcc cggctgtccc 600
   ctgcctttcc tgtggggcct gactgcatgt accccctctc cccaaaccct ccaggagttc 660
   tgagtctcta cctggatctt gattccactg gcatgaaatc tgtgaatctc acctcgagaa 720
   tcctccctct gctgccgcag gatgggacca acagttccct gcagtggcaa gggagctgtt 780
   cacacactga gcgagcagtc acagctgccc tgagctccac attctcctcc tcgggggact 840
   cccctggaat tacctttgtc attttacttt ctcaacccct gtgtttcctc tgatatctta 900
   cttatctctg gtctttttgt ccagacagct cttaagcctt gggaggaacc ttttcctctg 960
   atcccttttt aggccattat caagttcagg gtgggagggt agagtcactg tctttccagg 1020
   ccttgctgca tcccttagag accttcggag ggggcacttt gtgaccagtg gggatggggg 1080
   aaggatggct tggtgtgtct gtcctcaccc actaccctct gcccacttct gcctgccctt 1140
   cctctgtgtg gcatatacag tactgcaccc ctgagcagga cattccccag tgagagcccc 1200
   aggcccgaca cttccaggct gtgtggcctt gatcaagtta gtttcccttt cgtgattcag 1260
   attcctctca ggctgcttgt gcaaactcaa cataagaaag tctgcgaagc tcttaaccca 1320
   gggcctgggc catgctaggg ggatcagtaa gcagtttctg ccacctctgt tactgccgct 1380
   ttcacctttc cttgtggaaa agagaagcag ccagaaaagc tgggttttag ccccagctct 1440
   gcctcttaac tagctaagta aatgtgggca gaatgcttga cttctctgaa tctcattttt 1500
   tttttttttt tttgtcaaac aagatgaatg aacaggatcg gtcttttttg atggggactt 1560
   gaatggtgtg tggggggaca gttctttgtg tgggacggtc cacactaaat gccagttatc 1620
   cccacccatt gttacaaccc aaatgtcacc ccccaccaag tatttctcca tgtccctatg 1680
   ggttggtggt ggaactgtcc ctgactgagg accactgaac tagcttaccc taaacttcac 1740
   tcctgctccg gtgtcctgtg tgggtggaaa agtgaggacc cagtggagca ggtagacaaa 1800
   gatcagttca acagggccct ctgctccttt gatattggga cagcttcact tcctctgaat 1860
   tagaagcttc ttgacctcca tatattgagg aaggaagggg tttctaacaa atgaagaaac 1920
   aggctgggca cagtggctca tgcctgtaat tcccctttgg gaagcccagt tccatctcta 1980
   aaaaaaaaaa aaaaaaaggc cacatgtgct cgagctgcag gtcgcggccg ctagac     2036
<210> 315
   <211> 728
   <212> DNA
   <213> Homo sapiens
<220>
   <221> misc_feature
   <222> (1)...(728)
   <223> n = a, t, c or g
<220>
   <223> nbla-11674-f
<400> 315
   nnnnnngnnn nnttgnnnnc cnttgggnnc cctcgagcct gttggcctac tggaaaaagg 60
   aagcaaaaga agtttttcca aagatgttat gaagggctct aatccaaaag taccacagca 120
   aaacaacttc agtgactgtg gtgtatatgt attgcagtat gtagagagct tttttgagaa 180
   tccaattctc agttttgaac tacctatgaa tttggcaaac tggtttcctc caccaagaat 240
   gagaacaaaa agagaagaaa tccgaaacat aattctgaag ctacaggaag atcagagcaa 300
   agagaaaaga aagcataagg acacttactc aacagaagca cctttaggcg aaggaacaga 360
   acaatatgtc aatagtatct cagattgacc atttctgtta cttgtcattt ctactttcag 420
   aaactaaatg actttcaaat ttgggtatag acaataaaga actgaagtgc tcactactca 480
   gtgatttgga aattttgatg cttgtataaa tgtcagataa ttaatttcca aaggcgtatg 540
   tattaagtaa aagtctgtaa atatgttaat gaggccaatt tttccagcat ttataattat 600
   ttttttcact tggtaggaag cttttggtat gnatttctgg taatagtacc taaaattgca 660
   acttctaacc caaatnaaaa ggaaaatatt tataggagga aatgattaat tggtatctta 720
   gggaactg                                                         728
<210> 316
   <211> 729
   <212> DNA
   <213> Homo sapiens
<220>
   <221> misc_feature
   <222> (1)...(729)
   <223> n = a, t, c or g
<220>
   <223> nbla-11674-r1
<400> 316
   gnnngnnnnn nnnttgtgtg ncctttgttt ttcntttttt ttttttttat cccagtttta 60
   ttatagattg aaaagcattt tgccatgttc agtaaataat aaaattaaac ttgnttttca 120
   tatgagctaa ttaagatatc tgagctaaca gctctaagtt attactataa agagttaatc 180
   agaaatatag ttcaaagtaa caaactacag ttgatatcac taatggtgac ctttcttaca 240
   ttttaatatt tctcttactg agactctact gcgtctcaat agatattttt tggttgttgt 300
   taatactccc attcaccctg aacccaccca tgggacttta ttaaattcaa gaaaataccc 360
   taagatattg actgcaatgt cagagatggc caaattaaaa ataaaataat tgctgatgat 420
   taggctttct ttttattctc aactattttt gatatatgtg tatttaaaaa ttcaatattt 480
   aacaaaaaat gactaaaata tgtttcaagt ctcaaagttc atttgaaaga agtatacacc 540
   atttcatttg aaaaataatg aagtttctgc agatatatgc aagatcccta tatgtgctat 600
   aaatctgnca tggaaaccat caaattttag ttattttaaa ggggaattca aagatttttg 660
   ctggattatg caactggtgg aatacgtact ttacatgtaa aaaataatta ccctggagna 720
   actcaagcc                                                        729
<210> 317
   <211> 776
   <212> DNA
   <213> Homo sapiens
<220>
   <221> misc_feature
   <222> (1)...(776)
   <223> n = a, t, c or g
<220>
   <223> nbla-11679-f
<400> 317
   gnagnttggn nccctttttg ngantcctcg agcactgttg gcctactgga agttagttat 60
   tttctcctct ttctttcctt tctttcctcc ctttttcccg tctgacccca aacgttattg 120
   tccaaacatg actggacagc agcttttgtt tcttgaccct gtaatatgac agtctgctaa 180
   tattgacaga aggtgcagtt tttgggttat agtcgtgatt ttcactaatc aatcatatta 240
   gcaggaaaaa aaatgacttg tttctgttgt acttgagtct taagaaaaag tgcccatagt 300
   ttagtgacaa tttccaaagg ctttagtacc acctgtattt caaaatgggg gacccaaact 360
   cccggaagaa acaagctctg aacagactac gtgctcagct tagaaagaaa aaagaatctc 420
   tagctgacca gtttgacttc aagatgtata ttgcctttgt attcaaggag aagaagaaaa 480
   agtcagcact ttttgaaagt gtctgaggtt ataccagtca tgacaaataa tttatgaaga 540
   aaatatnctg aaaggtgtgc gagattccag ctattccctt ggaaagttcc ctannagctt 600
   ttaccagaan gatgtgggtc cagntncatg ctnctngata tcaagtccta tgnnaanggg 660
   anngaaattn gctgtactca ggnagatgga tttcantcnt ttggnccttc ngnantgann 720
   tttgaaaaaa atncgntggn nntcctggtt tctaaggggn taanaattct natgng     776
<210> 318
   <211> 753
   <212> DNA
   <213> Homo sapiens
<220>
   <221> misc_feature
   <222> (1)...(753)
   <223> n = a, t, c or g
<220>
   <223> nbla-11679-r1
<400> 318
   gnngnngntt ggtggccttt tttttttttt tttccctggt tgtattttct ttttttaaac 60
   cagttctggt cattagaatg tatctgacag tgctctggaa caactgtgat tatagcaaag 120
   ttattgtttt taaaaatgtt atttatacct gttacattca cttctcaccc tctaaatact 180
   gatgacagat gtcaaagagg caaaaaccag cacaaatggg aagaccataa aaatgagtat 240
   caccttgtgc tttcagatac atttaagcat ttcagtgtac aatagtcctt tcatttcaca 300
   tttttagtaa gatactgatg cagtgcagca aatatgcaaa gcatcttctt tcacacagtc 360
   cgtgcacgac atctaatttt tgtttaagtt ctgagataaa aatgatttaa aaaaatccag 420
   gatgaacaag tttcaaaatg catagtgttc tgtgcatgag tccattttct ttaaactctg 480
   aaagaataaa gtggtaagaa aacaagaaaa aaaactgctg ctgcattctc tgatcttctc 540
   cattttgctg gcagtactct actctggcat ataaggacng aggtggatcc tctatggngc 600
   caactggcca ntgggtgaac tggtcctgtg gcangtagaa gcaanatgct gataacttgg 660
   aagattggaa ctttggtttt tggagtctgc aagtgctgct gtcantgaan aagaaacact 720
   gactgggtag gattggtggn cacgattcca ggc                              753
<210> 319
   <211> 799
   <212> DNA
   <213> Homo sapiens
<220>
   <221> misc_feature
   <222> (1)...(799)
   <223> n = a, t, c or g
<220>
   <223> nbla-11753-r1
<400> 319
   gntnagntgt gccttttttt ttttttttaa gtgtaaaagt tatattttat tgccatgcta 60
   caaaatgtat gaagttggca ctgataggga gaaatagaga acaaagggtg ggaagggata 120
   gagggaaaat tatgttgtta catatacaac aaggttttat tttaattaac agtggttacg 180
   ttttgccaat attaaaaatg caaaccaaaa tttaaaatgc tgatctgaaa cagcattaag 240
   atacaatgta tgcatagtac agtatcactt atgtcttttt attagagaaa tatggaatgt 300
   ttataaaaga aattaaccat gggggtaaaa ttcatatttc atatacaatt tggcaatggt 360
   agtcccactg ttggacaatt ttttataaaa gaaaaaatta aaaatctaat aagctacctt 420
   tatacaaagt tgctatattt atgcctttac gtaggaaaaa aacatttata atgcaaatta 480
   ggacatacaa tagtcttaca atattataca atgtaatgaa aataaaacat aacacaaagt 540
   ttgcctttat aaaatgnata ttttgcatta ctaatgcaaa tgtggcacac tggtgactac 600
   tgnactattt atccagtctg tttccatgct ataactgtcc aattgccata gaactgcaat 660
   tataatgatg cttcaaaaga actgngttat accaaaaatg aggaagtagg gatggggaac 720
   agggatgtaa agaacaggaa agatagatag ggtgcctggt ttgggcanag atactggggt 780
   ccgtataaaa aatggtnat                                          799
<210> 320
   <211> 759
   <212> DNA
   <213> Homo sapiens
<220>
   <221> misc_feature
   <222> (1)...(759)
   <223> n = a, t, c or g
<220>
   <223> nbla-11763-f
<400> 320
   ttggnttcct tggcctgttg gcctactggt gtgaatattg gaaattaatg ggtttcattt 60
   tcatttattt cccttgcctc ttcttgagga ggattcgaca ggcgatgaaa tgggtaaaaa 120
   ggcagtcaga ctaacaggaa gaaggaaaga taggctagct gtcaactgat gagcctgcct 180
   gaataatctc aaatgggctt ttgaaaagac cttaacaaac actgttgcac tcttgtcaaa 240
   tatctttatc tacaccctac ttataagtac caaaaagaca gcatttgatg ccaagtttga 300
   agcagttagg tcctatgcag gctgtgtatt aacatacagt gaattgcact ttaaaattca 360
   gaaagctttc atttggtcac atcaaaaata aacactacaa aatatataaa aagttaaatg 420
   ataggactct ttttttggta ttaagagtaa aatagaagaa gatctaagaa atacaaatac 480
   ttacattatt tctccttatg acattaactt tgggactaga gccacaccac ccccttcctt 540
   cttcacttac tgattggatt gcatataatt ttcatgccca cataaatcct gattcatata 600
   tttgnaactg natatgacaa attgagangc angaataaga aaanggaaga ttatngangg 660
   aagatgttaa gaaagancag ttcttctaan tactctggca gatcgggtgg ctgaaagagg 720
   tnattagcat ttgaaatgga tgggctgatg nggggctca                    759
<210> 321
   <211> 759
   <212> DNA
   <213> Homo sapiens
<220>
   <221> misc_feature
   <222> (1)...(759)
   <223> n = a, t, c or g
<220>
   <223> nbla-11763-r
<400> 321
   ttggtggcct tttttttttt ttttttaaaa ttttttttcc cattcttgag ttttacaatg 60
   tctcctatcc aaacttcaaa tttcattaga gccggctaca tttgttttag tgccagatac 120
   aggtatcaag tgatgacaga cacttacgtg aggtatacaa tcaattaaag actggaaatt 180
   ggggtattat catgtaagac atttgagagg ccaaaagtca tataatttta agatacttca 240
   tgatgttaca gtaataattc atctatataa caattatttt gacataagtg gcatatcaga 300
   atttaactta tttacttaga tcttaaggaa tattagatgt attctcacat gaacactcaa 360
   atcatgctat tattatcagt cactgggaga acagccaaag taatcaggca gtctaaggca 420
   catatagacc caataattta gaaattttca ccattatgag gtccctgttt ttaataaata 480
   tgacaataac aggcgcttga aatgggggca aggtgaataa gtatccagtc aattgnatac 540
   ttttataaag tagtgnattt gagacataaa ctgagatcaa aangaatttn cttactttna 600
   aaaaaggngc ttctatctta gnagattctt ttaaatactt ancntatatt naaaagtagt 660
   cnttacatac ttaagaaatc accgtaatct aaaaaantaa angcagtttc ttggnaatga 720
   acctgngatc caacaggcca caatgnggga attactcag 759
<210> 322
   <211> 778
   <212> DNA
   <213> Homo sapiens
<220>
   <221> misc_feature
   <222> (1)...(778)
   <223> n = a, t, c or g
<220>
   <223> nbla-11767-f
<400> 322
   gttnnnnttn nnnnnnnttt gaattccntt agcactgttg gcctactggg agagaccaca 60
   cgtagagctc ggtcaccaag gaatgcccaa catcccttta cctctcacaa tgagcagcca 120
   cgctcaccgg gaagcagtgt ggggacttca gtggggtgtc ccttagtgga gaacaccttt 180
   catcattttt atagagacaa ttcccttgag aatgtaggct tttcatctta ggaacaatta 240
   ggaagaatct ccagctggga gtgttcttat gtatacagaa acaatgggtc gcattggttc 300
   atacttaggt gatattaggt ttggctatat tgatgtttgg tgccacttac cttctagatt 360
   ttcccaaaag ttgggtcatt gctatgggaa attggggcct gcagatgggg gaggaatgat 420
   cattattggt gcagggaagg ggttcaggca gagctggagt ctgaggctca gtgtgagaaa 480
   gcactggcag ttttctagta gagtcaggtc ctgggctcct ctcctnaccc actgtgtccg 540
   aactgaggcc agctgtgatc ggcaaattgc ttatctctag gnaggttaaa gaaaacgtaa 600
   gaatttgggg ggtggtggga gaataactgt agaaagacct aaactcttnt ttccccanca 660
   aattatcttg gataatggtt aaaaacccca aggccnnact tcccttttac cgngaatcan 720
   aaggaggatg gcantgggcc canggcnnaa tggcccactt tgaacncatt ttaaaang 778
<210> 323
   <211> 800
   <212> DNA
   <213> Homo sapiens
<220>
   <221> misc_feature
   <222> (1)...(800)
   <223> n = a, t, c or g
<220>
   <223> nb1a-11767-r1
<400> 323
   gnaggngann nttggtggcc tttgaaaccn tttgnttttt tntttttttt tttttttttt 60
   tttttttttt tttttttttn ntnnnnnnnn tnnnntnnnn nnnnnngggn nnnnnnnnnn 120
   ggnnnggggn nnnggggggg ggggggggna ccnnnggnng gnaanggcaa nagaaanaan 180
   cccncaaaac ccccnggggn ggggggggaa aacccngggg ggnnnnnaaa gnaaancnan 240
   anggnagggg nggcccnnan naanaaaaan ggaaanccaa nncnccncaa accccccccn 300
   caancccnnn naanccannc aaaaaaaaan nnaaaannnc nccnaaanan aaccccaaaa 360
   ancnnaccnc ngaacccnan nnncnccccc cccaaanggg anccaaagnn aannncnaac 420
   ncnanngnnn gggacaccca acccnngcgg ggggggcaan nccnccccnn gngggnngnn 480
   tanaaaaaac cncaaaccgg aacccnnnng cccccccnna ngnnnccncn nanacaccng 540
   anngggcacc nccnaaaacc tnngnnccnn aggnaccaaa gnannggncc cctncnngna 600
   nncgngccnc ccccnaggtt nanaannaaa ccncannnaa annaaanaaa nanancccct 660
   ntnnctcaan cngngnccgg nnnnanccna nccncnaacc caangnctca cccccngngc 720
   ccgnnanccn gnacaccana nccaccntag ggnggnncna cnccaacnan cngnncngtc 780
   aaannncgcg ncnnnagccg 800
<210> 324
   <211> 783
   <212> DNA
   <213> Homo sapiens
<220>
   <221> misc_feature
   <222> (1)...(783)
   <223> n = a, t, c or g
<220>
   <223> nb1a-11775-r1
<400> 324
   gnntnttgnn nnntgggtgg cctttngnnn nnncttttta tcctgtcatt tagcatttaa 60
   taacaaaact atacagaatt atgtgcatac caatgaatgt tttgtaccat ctagttaaat 120
   tttttaaata aagttttatg ggttaagcag aagacaactg tcatactgaa ttttattaaa 180
   agtatatata cttcaaattc aaagcatccc ttaggaccca cagaatatat taaaactacc 240
   acccttaaat tttatatttt tgctttaaga cagacaatgc aaaggtaact ggcaagaggt 300
   gagcaaatgt tttagaacat ttatattatt gcttaaaatg agatttgaaa ttgtaataaa 360
   attcttggtt atgaagtctg atgtcttctt tgagcaccag tttaaaagga aacatttcaa 420
   acagtaaaat aaatcagtgt gtactttatt ttgagccaat gttttacata agaccaacaa 480
   actaagtgtg ggcaaactgt tcaatcactt ttgagaaatt acatggttcc tatttaggtt 540
   tgaaaaagta gtttgttgcc ataacatcag accttaaggt gttttaaagt gtatcctaan 600
   ggtctgacag aagaaaaata cagggtatgg aagtatccca actcatgcca cacagtatag 660
   atcattcatg gngaataatg gtcatctggt ctccaaaggt agtggaaaat attaagtcat 720
   caaataatgc tcagaantac caanggacag gttaaaaagg accaggatcc aaacaggttn 780
   ttt                                                               783
<210> 325
   <211> 2676
   <212> DNA
   <213> Homo sapiens
<220>
   <221> misc_feature
   <222> (1)...(2676)
   <223> n = a, t, c or g
<220>
   <223> nbla-11778
<400> 325
   taggcctgta cggaagtgtt acttctgctc taaaagctgc ggaattcctc gagcactgtt 60
   ggcctactgg cactgtctgg aaaacttgaa ttattttacg ccgtgaaaga aaaaggaaaa 120
   aaaaaaaatc ttttctgttc ctagaaaatc tgaagtactg tgtttctccg ctagagggca 180
   gactgctaat gaaatttcag gaccctcact gactgcagta gcaggattgt tcagtactga 240
   gtggatgggt tagggtgtct gtgaacaaga tctagcccac acagaaacag gggatttatt 300
   ccacagttag caccacagat tgcgacttgg gaagaaacca tcagctagag gggtctagtt 360
   cgtatccaat cattattgac tgactgactg atactcaact agcaaaggca agaatttgga 420
   agcacgctct acccagatgg gactttggag ttccttccct tccagagtcc ttattgaggt 480
   gttgaagtgt tggcatgccg aagaatctta gtgacattta gccatgggtg tttctttaaa 540
   aaaggaaaag aaaatgtctc aacgaagctt tgaaatggga gagtgttgat ttctagttac 600
   attgctgggt tatgtagttg tatctgtggc taatttttct agtcctcaac aaatacacag 660
   acatgctatt atggggttta attcaattta caaataggtt ttccttcctc acagtggagt 720
   aatagaaaaa attgattttc tgctcctttg cagctgtgtc cagagacgga caatgaatcc 780
   acaatttatt aggcagaggc acaacttctc caccattcgt tttatccttc tcttttctct 840
   gtttccctcc acctctcttc ttcttctttc acactgagca gaaaacatat cttcaaaatg 900
   aattcgcctt tgccatgtgc atattctctt ccttaaaagg acatcaacag tgttggagat 960
   gaggagatga acattagatt ttggaatttc tgggagggag agggttaggg gtgactcagg 1020
   gcctcccttc gcccaaaggt gaatcagaaa aggcacccct ttctctggga tacaacctga 1080
   gcaggggtct cagctggcaa gtggagcagg gtgggggctc aacctcctca ccgcttgatc 1140
   agctgccgtt gtacagagca tgacctgtat aaccttgagg gcagccctgg gtagatctgc 1200
   agagtgaccc tctatatagt tagaatccag atgaaggcca taagaaatag tccatgccat 1260
   gccgttaggc ccactttcat gtgcagtact tgagaaagca catcgtagcc tccttttcag 1320
   acacacaaat ggcggctgga ctgagagcat ggcagagaag gggaggagga gaatgtacta 1380
   aacttgctca ctgaattgag cttgagttat tagattgtag aagagcttga tgtctggtga 1440
   ttttgttaca ggaagggggt cccgatccag accccaagag agggctcttg gatctcatgc 1500
   aagaaagaat tcagggtgag tccataaagt aaagtgaaag caagtttatt aagaaagtaa 1560
   aggaaggaaa gaatggctac cccatagtca aagcagctct gagggctgct ggttgcccat 1620
   ttttatgttt atttcttgat atgctaaaca aggggtggat tattcatgcc tccccctttt 1680
   agatcatata gggtaacttc ctgatgttgc catggcattt gtaaaccgtc atggcactgg 1740
   tggtagtata gcagcgagga caaccagagg tcactctcgt tgccatcttg gttttggtgg 1800
   gttttagcca acttctttac tgcaacctgt tttatcagaa ggtctttatg acctatatct 1860
   tgtgctgacc tcctgtctca tcctgtgact cagaatgctt tagccatctg ggaatgcagc 1920
   ccagtaggtc tcagcctcat tttacccagc tcccattcaa gatggagttg ctctggttca 1980
   aatgcctctg acaggttcag cacctgagaa aagtaccttg aatcaggcct tggggcagag 2040
   tcttcaagat tttaaggtga tagggcctgt ctcttaagaa tttattaatc gtaagtgctc 2100
   actgctggtt tagagttaga tacatttccc tcaaaacggc catgggcagg ttatgtactc 2160
   agaactgcca gggtgaaaac aatgagatcc agaaaaatcc cacaacaatc caggtttttc 2220
   actcctcttt tttttctcaa tcttccgata cttgtggtac ctcttccatc aacagatacc 2280
   tcatgtagtg acttccagta tcaagttttc aaaacttcct acccaccctc atcagtgcct 2340
   ccttcactac agagtcacaa acattggtgc tctcaaataa aaatcagccc caaacaatgt 2400
   caaagagatc ccttgagtag ttttcaggcg agagatatgt tctcatttca agtcttggag 2460
   aaaactggat ccatgttcac gtggaacttc tctcgcggag gtgctatctc attttgcatt 2520
   aaactttaag caggacagat tgctgaagcc atgatattta aggtttgact ttttaaaaat 2580
   ctcattactc ttaaaataaa tgctgccaca tcagaaaaaa aaaaaaaaaa aaaaaaaaaa 2640
   aaaaaaaaaa aaaggccaca tgtgctcgag ctgcag                      2676
<210> 326
   <211> 792
   <212> DNA
   <213> Homo sapiens
<220>
   <221> misc_feature
   <222> (1)...(792)
   <223> n = a, t, c or g
<220>
   <223> nbla-11822-f
<400> 326
   ctcggcactg ttggcctact ggctgactac ctagatgatt ttgcattaat ataataacca 60
   ttacctgcct tagtgctttg tacagtattg tggcaaaata gctaagccta aaggagttat 120
   acaaaaagca gaattccata atgaaacaga attttacttt ccacataaat agcatgcttt 180
   ttttttttat tttttttaag accgaaatat tatatcagaa gtgtgttttc tttcctgtat 240
   gatagttact tgcatggnac ctggttggtt cattttgttt tgttttttta ataccatgga 300
   gaaatagaag ctttctaact tttctgtttg ccaatacatc tgttgtatcc attgacttgt 360
   aattatgaaa acttgttttc cacagggggc ctgaggtgat gangcntaat tttcttctat 420
   ncaggctagn ttgnatangg gcatnanaaa ctcggnctag ttnntatttt tttnctcctc 480
   tntctcttna atggntgctn atntnctctg nctgnnntng ncgnnacttt ntgggancnn 540
   nntanttcaa atttgnagnt gntccggtta tntgcnttan ccagtggann nnngccntcc 600
   tngtcangct tgtacnnaag tncaggncnt gnatgctngn cngntctcta attnnntggn 660
   cccnttncat gnngtttttc ggnnngcann nnntaagnnn ttttnntntn gcantncant 720
   anntgnntcc gcannatnnt nnngcngttn ttcnnnatnc ntttntnttt cnnnctcnca 780
   gtanttcgng ac 792
<210> 327
   <211> 807
   <212> DNA
   <213> Homo sapiens
<220>
   <221> misc_feature
   <222> (1)...(807)
   <223> n = a, t, c or g
<220>
   <223> nbla-11822-r1
<400> 327
   nnagnnnntt tgtggccttt tttttnnncn tttttttttt tttttttttt tttcanattt 60
   catgccttct accttaacct ccttgagata acaacattca tttctgatgt tttctgtgct 120
   acagggttca tgccttaact gggagttcaa gctaattctt gatttctttc tcaaaagacg 180
   atttaaatnt aaggcaaaaa aaaaaaaaaa aatttccgat actgtaacta ccttaactgt 240
   ttgagtntnt anaaatgagt atgttttatg agttaacaat gaatcatana aggcaattta 300
   tgaanaacta gaatgattct gtctagctgc cttcagggat canaaattca aattaaactt 360
   ntggcanacn tttaaagaat gctagcatac aaaactgaat ttaaaaaaac gcacacagtt 420
   aacactcctn agnggagttt caagaggaaa tcatgaatta aaaaaacaca cggcntgata 480
   cactgttgac tgatnccaac tgggtgtggt cagatcccag gtgcacccaa acctgcggga 540
   atggcagaaa tacagctgng cttggccatc acagntntgc tagtatggcc tgctgtacct 600
   taataccagc aactgggaaa acggtgttag cactaaaact ggtggtaaaa gcccataccc 660
   actgntgggn aaatctgcct ganacgggtt tttntttaaa aangactctn tatcatntga 720
   anctgaaaga cttttaaaat atatgcccta ttatttcctt taacccccaa tggggaaacg 780
   tanatgatgg gaagggaaaa atttnaa 807
<210> 328
   <211> 835
   <212> DNA
   <213> Homo sapiens
<220>
   <221> misc_feature
   <222> (1)...(835)
   <223> n = a, t, c or g
<220>
   <223> nbla-11860-f
<400> 328
   gnngnnttgn nnnccnttnn tttgantcct cgagcactgt tggcctactg ggctatatcg 60
   aaggtttttc ccccagcagt atgggggccg tacaggtgat cgatcattaa tgtcattgca 120
   attgttattt tttaaaataa atttataaaa ataaccgaaa agtaatgtga cttttgagga 180
   atctgcaact cctgggttct agtttgtgtt tccggtgttg aaattttgat ctcccccgac 240
   aagacttgtc tgcatggacg gtgtctctgt aaacagtatc aaacttgtaa tgtctgagcc 300
   tcttagcttt atctcccctt aatttcttcc cttttggccc ttccaccctg ttgaggagat 360
   tttttctgtc acctgcaggc tgatcataca tttgatgcta ctactgaaag tgtttatcaa 420
   aatgtgatga agtaattcca tgaggaaaat tagccgttat cacaaaaaca gccttaagta 480
   ttgtagaatt tcaggtagtg aagatgtact atttacgttt tgttagattt tgttttggca 540
   gaagaccaaa acaaaagtac ttttctgatt gttaaaaatc cacagcatgc tcaccttggt 600
   actggtttac cttacggtcg ctgggaatct aagcaacaaa gactagaagc ctccgatatg 660
   ccattatcaa cacacaagag acaaacagcc cgaggaacac tcaatgatga gagcagtaga 720
   cctgncctgc agatgaaaga ngagaactct ccacaatgga aggaaaacac tgncggattt 780
   gatagcttta agcaaactgn gcttgagttg gcttaaatta tcccgtggac ctgnc      835
<210> 329
   <211> 849
   <212> DNA
   <213> Homo sapiens
<220>
   <221> misc_feature
   <222> (1)...(849)
   <223> n = a, t, c or g
<220>
   <223> nbla-11860-r1
<400> 329
   gnntgnnagt nnatttgngg cctttgaann nccctttgtt agaagagaat actttatttt 60
   gaaataaaat acaaatgtgc aaaggaaatt agctcctcct gccccccctt tgaacaatga 120
   gtcaatagaa tgtgagactg ggtagattag cagataatag gcaaaggtct agcttttcag 180
   tggcaacttg aagaaaccaa agatgaatga tgctaaagga atgacccttt ggtacctgtt 240
   ttaaagtact tctggccccc ttcttttata aacccccagg agcccagcac cacaccttgt 300
   taccctacaa tgatcactca cgctccacga tgtcactaat gtaataactg aagatatggg 360
   ccagtttgtc catgtcacgt tccgacttgt aggtcagggt gaacctgtcc attaatgctg 420
   taaaccctga ccaacaaagc agaaattacg atgttaactc caaatcctaa actttttggc 480
   ccctttctcc ccacacatca ggccagtgta aaggaaacag atcccttcaa gctgagtaat 540
   tcctttatcc attaacatgg tgtaacttag aaactactaa gggccaggtt ggttgctgct 600
   gccactggga atccaaaggc caccctntct gntttcaaga gctggagatg gtgccctttt 660
   catctggagc cagctntttn ccctactctc ttaagctgcc taactatngg aaacaatagg 720
   atccctccta atgtcttggc tagagaacca ctnacacagt gagggcaggc acctggnatt 780
   acccatcttn atatncctgg cacagantaa gnttttggta aatnaacgac ttncggagtg 840
   gnttgtnat                                                        849
<210> 330
   <211> 885
   <212> DNA
   <213> Homo sapiens
<220>
   <221> misc_feature
   <222> (1)...(885)
   <223> n = a, t, c or g
<220>
   <223> nbla-11874-f
<400> 330
   gnnnntttgn nnncccnttn nnttgaattc cccgtgcctg ttggcctact ggttgatgca 60
   ataccaaagt gcttcccaga gaggctgtgc caatgtgtgc tccctcccct cccacgggtg 120
   agagtgccca tccactgtat ccatactggt gctgagtgct atcattactt gacatctttg 180
   ccattttatt aaagagcata tggctggttg ctaaaggtaa ggtggcaaac aaaaaaaact 240
   gtgaagagaa gcagaagttt aaattccaga caataaatga aaattggtaa aggcatctgg 300
   ttcttctaga aacatccttc cattaatttg tagatgagta tctcctattt ctaggaggat 360
   gtgtcaacat ttcacagcct cctagagtat tagctgcccc tatgcctctc tctatatatt 420
   ctcatctggc attaatgcac attagatagg gatttggaat gtttaccacc ttgttagaac 480
   ttctgagcca tttctaattt ctttcctatg ctgaaccctc caataaaaag caagtgactg 540
   nttatataat attagagcat tttgaggtac ctctgcagct aactagttta aaaatgacaa 600
   attaagtcaa agtttgattt gctccatgat ctgatggttg catttcctga gatgtttcan 660
   ggaactgact aaaaatttga tggagacagt ctttgagaat ggtctaaaaa actccacgan 720
   ggcatctaat tcaacccctt gctctaggac aaaccagcca aacatgaatc catgaggaag 780
   gaaacaattt tcaattttca ntcacagnag ttaanngntt tgccaaatcc ccgacaccgn 840
   tttttcctga cnggggnttg ggancangna ataannngga gttgt            885
<210> 331
   <211> 888
   <212> DNA
   <213> Homo sapiens
<220>
   <221> misc_feature
   <222> (1)...(888)
   <223> n = a, t, c or g
<220>
   <223> nbla-11874-r1
<400> 331
   gnnntttgag gcccttttnn tttttctgnt tgtttttttt tggttgttaa gtctcccatg 60
   tttagtaatt aacagacccc gccagagagg aacagaacaa aaatgagcat gcaaatgact 120
   aatcaaggaa agctggaatc aacaagatat gggtaaaaag atgctcctct gagcctggcc 180
   atcatgcggc aggttctgcg cctctgatca cactgagctg cccaccttcc catgccctgg 240
   aggtaaagct atgtagtcct accctggtag cataggctgt gccactacac aattcagcat 300
   ttgacttgaa caagaatggg atactttgga tacttttttc tttaaaatgg ttccagtctc 360
   ccaaacacaa gtatggttgt atgatgcatt ttaggaattt atttatcaca ctgtgaattt 420
   ccacactgcc ttcttcataa gaattcaaaa tgcttgttca catttctaag gcatctaatg 480
   gggggcaagt attaaaatgc ctccatttta cagataaggt aaactgagca aagtaattta 540
   tcccaggtca gaggcaaagc aagtataagc cctgttccaa tctcaccaag tcataccacc 600
   tctgcaattc aaaatggctc ctttaatttt ctcaaggtac ccaaaaaaaa aacacatttt 660
   catatcttgn acaaactaaa aagctctaca gtggttctat ggcanctgtt ctttgcnaaa 720
   gggggtncag tggtagacat ctacattttn atctttcatt tttataagca atgctttggt 780
   gaacatctct ggatttttta cnaaggaaat gatgatcttt gcncagcaaa ctnttattaa 840
   agcncagggn cangcccata tttaacctgn tttagccaca gnaatttt 888
<210> 332
   <211> 885
   <212> DNA
   <213> Homo sapiens
<220>
   <221> misc_feature
   <222> (1)...(885)
   <223> n = a, t, c or g
<220>
   <223> nbla-11880-f
<400> 332
   gnnnnnttnn nnnncnnnnn nnttgnattc cncgntcact gttggcctac tggagtgtga 60
   caactgatcg ggtgaacgat gcaccactaa ccaccatgga aacaaggaaa aataaagcca 120
   gctcacagga tctctcttca ctggattgag agcctcagcc tgccgactga gaaaaagagt 180
   tccaggaaaa agaaggaatc ccggctgcag cctcctgcct tcctttatat tttaaaatag 240
   agagataaga ttgcgtgcat gtgtgcatat ctatagtata tattttgtac actttgttac 300
   acagacacac aaatgcacct atttataccg ggcaagaaca caaccatgtg attatctcaa 360
   ccaaggaact gaggaatcca gcacgcaagg acatcggagg tgggctagca ctgaaactgc 420
   ttttcaagac gaggaagagg aggagaaaga gaaagaagag gaagatgttg ggcaacattt 480
   atttaacatg ctccacagcc cggaccctgc atcatgctgc tattcctgca aatactgaag 540
   aagcatggga tttaaatatt ttacttctaa ataaatgaat tactcaatct cctatgacca 600
   tctatacata ctccaccttc aaaaagtcat caatattata tcattaagga aatagtaacc 660
   ttctcttctt caatatgcat gacattttgg acaatgcaat tgtggcactg cacttatttc 720
   agtgaagaaa aacttgnggt ctatggcatt catcatttga caaatgcaag catnttnctt 780
   atcaatcagc ttctatggac ttactacact tgctgnggaa tcctaagggc catacatttt 840
   gaagaanaag cttagataag gcatgccctc cnaattatgg cttct           885
<210> 333
   <211> 859
   <212> DNA
   <213> Homo sapiens
<220>
   <221> misc_feature
   <222> (1)...(859)
   <223> n = a, t, c or g
<220>
   <223> nbla-11880-r1
<400> 333
   gtggcccttt tttttgtttt tgttcttttt ggagtaggtt tccttggtgg tttttaggac 60
   atatttgttg gtaaacctat aacagttgct tttactttca gtgatgtact tttttctttt 120
   cctgcttccc agagatttat cagaggagga taaagctcac ctaatgcaaa ggttggtttc 180
   tgtaagtaat tcctcacata gctgtgtcca ccatcacagt tcatttctgg agagaggcag 240
   ctgataagac atatcacacc aataatcccc agaaggcctc caagacaggc cataagtgtt 300
   gtggtattat tcttttcata ctctttttga tcagggtgca aacctttggt ggtgacattt 360
   acacattttt ttctgttttt ctgatagatg gtgggaatat caatacaaat tttatactca 420
   gttgatggat tcagatgagt aagattatat accttgacat cagatggtat tcgagcactt 480
   tgcgcagcat gagaattttc agtcttgaca aaggctgcca tttaacacta gatttgagaa 540
   ttttagaact tgctttcagg acaccaaaac tgaattgcct gaatatctct tattttaata 600
   ttcaaagagc cattggtatc ttgnggaaaa gaccatccct ttgacataac agacttcaag 660
   cagcgncaac tagggtaggt gctatncagt atntaaaccc cttttttggg agtacgcctt 720
   tatatctagg gtccctcaaa tggacataga cttgctggca ggtattaggc aaaagttttt 780
   gacncgaagg ngtatccata gatttcagct ggggtctnaa gtactntacn ngaaaggaac 840
   atantccact tttcanttt                                         859
<210> 334
   <211> 2013
   <212> DNA
   <213> Homo sapiens
<220>
   <221> misc_feature
   <222> (1)...(2013)
   <223> n = a, t, c or g
<220>
   <223> nbla-11882
<400> 334
   taggcctgta cggaagtgtt acttctgctc taaaagctgc ggaattcctc gagcactgtt 60
   ggcctactgg tcagcaagac acatcagtac cacaagatcc aggtgtggcc ctccagaatc 120
   cagccggctg tctttgcttt cccatgtgct acacttatca ctgtgatgtt ggccttcatc 180
   atgtacatga ccctgcggaa tgccactcag caaaaggaca tggtggagaa cccggagcca 240
   ccctctgggg tcaggtgctg ctgccagatg tgctgtgggc ctttcttgct ggagactcca 300
   tctgagtacc tggaaattgt tcgtgagaac cacgggctgc tcccgcccct ctataagtct 360
   gtcaaaactt acaccgtgtg agcactcccc ctccccaccc catctcagtg ttaactgact 420
   gctgacttgg agtttccagc agggtggtgt gcaccactga ccaggagggg ttcatttgcg 480
   tggggctgtt ggcctggatc atccatccat ctgtacagtt cagccactgc cacaagcccc 540
   tccctctctg tcacccctga ccccagccat tcacccatct gtacagtcca gccactgaca 600
   taagccccac tcggttacca ccccctcgac cccctacctt tgaagaggct tcgtgcagga 660
   ctttgatgct tggggtgttc cgtgttgact cccaggtggg cctggctgcc cactgcccat 720
   tcctctcata ttggcacatc tgctgtccat tgggggttct cagtttcctc ccccagacag 780
   ccctacctgt gccagagagc tagaaagaag gtcataaagg gttaaaaatc cataactaaa 840
   ggttgtacac atagatgggc acactcacag agagaagtgt gcatgtacac acaccacaca 900
   cacacacaca cacacacaca cagagaaata taaacacatg cgtcacatgg gcatttcaga 960
   tgatcagctc tgtatctggt taagtcggtt gctgggatgc accctgcact agagctgaaa 1020
   ggaaatttga cctccaagca gccctgacag gttctgggcc cgggccctcc ctttgtgctt 1080
   tgtctctgca gttcttgcgc cctttataag gccatcctag tccctgctgg ctggcagggg 1140
   gctggatggg gggcaggact aatactgagt gattgcagag tgctttataa atatcacctt 1200
   attttatcga aacccatctg tgaaactttc actgaggaaa aggccttgca gcggtagaag 1260
   aggttgagtc aaggccgggc gcggtggctc acgcctgtaa tcccagcact ttgggaggcc 1320
   gaggcgggtg gatcacgaga tcaggagatc gagaccaccc tggctaacac ggtgaaaccc 1380
   cgtctctact aaaaaaatac aaaaagttag ccgggcgtgg tggtgggtgc ctgtagtccc 1440
   agctactcgg gaggctgagg caggagaatg gtgcgaaccc gggaggcgga gcttgcagtg 1500
   agcccagatg gcgccactgc actccagcct gagtgacaga gcgagactct gtctccaaaa 1560
   aaaaaaaaaa gaagaggttg agtcagcagg gacttgggtt ccctgtgtgt gaggggggca 1620
   ttcttgcctg ccagctgctc ccgaggtggc cttgagaagg aagaagcagg atgacagagc 1680
   ctgagcagcg gaaccagcct gcaccctccc ttctggccca gcgacctggg ctgtggctga 1740
   gacaataatg aggccagaag tagccggagc ctgtcaggaa gggcagggga ggactgtggg 1800
   gtctgggctc tgtcgctgta accatctgct cccaggctgt gtgcagaaaa tggcatttac 1860
   actattgtgc agctcattct catgaaatac tgccattgtt gctaaataaa gcttgtgtgc 1920
   tctgaatata gccaagcaaa aaaaaaaaaa aaaaaaaaaa aaaaaaaaaa aaaaaaaaaa 1980
   aaaaaaaaaa ggccacatgt gctcgagctg cag                              2013
<210> 335
   <211> 1907
   <212> DNA
   <213> Homo sapiens
<220>
   <221> misc_feature
   <222> (1)...(1907)
   <223> n = a, t, c or g
<220>
   <223> nbla-11895
<400> 335
   taggcctgta cggaagtgtt acttctgctc taaaagctgc ggaattcctc gagcactgtt 60
   ggcctactgg gaactaagct acagccacca gggattttgt ggggaagatt acctccatac 120
   tgggaggggc ccatgcaatt tgcatcacca tagctgccct ctccaggagt tcttgggaga 180
   gcaattctgt ttaggtacat agcagaagag caagctccta gagtggaagt taaaggggct 240
   gcttaatgtt tggtgtgtac tttgtgaggt tagggaggta agatgttaaa gtcagatgct 300
   gctctactta gtatgggatg tcaataaggt acttacttca aagtaggtac tggaacatac 360
   ctttcttcta agagactaag attttgttgt tgttcttcag agaagcattt gtggaatatt 420
   tctgtagtgc ttgaatggcc aaagatcaat aaacaaaata aattacagtc ctggtggatt 480
   ttaatattta aatttagata attatgatgt ccaaagagaa gagaaagaga tgctttaagt 540
   cattaccaaa aatatcttag tccttgagag ggatttgctg aaccaaagtg agaatgaagt 600
   ctgtgtgtcc ccttttctgc aaagaagctg acgccacatg atgcaaaggc ctgcccttaa 660
   cccagttaat atgaacatgg catggcacca aagaggagac agaatcgaca gggagagtcc 720
   cagttttaag ctcccaagca agatctttca gtggggatgg gaggggtcct tttacccttt 780
   cctaatgact aaattaaacc aaacctaccc aagtggcccc tcctctcctc ctgggagatg 840
   gtggtgccat tgtcaagata ggtgggacac atttgggaga gattggggga acacagtttc 900
   accttcacat ttaagaatcc aatttactcc tgtggccttg tttgaactct ccttccaaca 960
   ctcatataat aataattata tgtccttgcc ttctgtaagc tcatttcagt tggtgtagca 1020
   aatatatttc taccacacac acaaaaattc ttctcttagg ggttcatgtg gaaatgaaaa 1080
   cacaatgtgt tccactccac gtgaaaagca agtgctgtct ctgcagcaaa gtgcttcctg 1140
   acaaggactg agctgaagtg tcatgaagct actcacctgg agggggcatc agcatctcct 1200
   gcctcttaag tcgaccaggg aaagcttgga caagagcagc catgtaggtg ttacaggaaa 1260
   ggtcctgatc cagaccccta gagaacgttc ttggatgtcg cgcaagaaag aattcagggt 1320
   gactccacgg taaaagcaag tttattaaga aagaaaagga ataaaagaat agctactcca 1380
   tggaccagcc ccgagggctg ctggttgccc atttttatgg ttatttcttg gtgatatgct 1440
   aaacaaggga tggattactc atgcctcccc tttctagacc atatagggaa acttcctgac 1500
   attgccatgg cctttgtaaa ctgtcagggt gctggtggga gtgtagcagt gaggatgacc 1560
   tgaggtcact ctcgtcacca tcttggtttt ggtggctttt ggccagcttc tttactgcaa 1620
   cctgttttat cagcaaggtc tttatgacct gtattttgta caaacctcct atcttatcct 1680
   gttacttaga atgccttaac cttctgggaa tgcagcccag taggtctcag cctcatttta 1740
   cccagctcct attcaagacc gagttgctct ggttcaaatg tctctgacat gggggctaat 1800
   gacatcagaa ctggaaagaa agatgataaa ggaatgagtt tttttttaaa aaaaaaaaaa 1860
   aaaaaaaagg ccacatgtgc tcgagctgca ggtcgcggcc gctagac              1907
<210> 336
   <211> 777
   <212> DNA
   <213> Homo sapiens
<220>
   <221> misc_feature
   <222> (1)...(777)
   <223> n = a, t, c or g
<220>
   <223> nbla-11988-f
<400> 336
   gangnnnttg attcctcnng cactgttggc ctactggtaa ttgctattga tgtggagatt 60
   ttgcagaaca tggcagagtt tttgcgtctt tcacaagaaa tatataattg aatgatgagt 120
   gcctggtgtt agcttttcaa ccttatttct tttggtttaa caatgcagaa ttgtgaacag 180
   ctgcagcaaa taggcagcta caatatctag ctattatcaa ataaatgcag atctggcata 240
   tgagctgagc taccactttt ggatctgcac ctcttgaata aacattctga agtgtggaag 300
   catgaaaagt gaatgatttg ccctgaggtg tttaatcctg ccttgcattc ttgactgaaa 360
   ttcttcacaa ttagggtgac taacattaat atgtggccag atgcatttgc acagattgat 420
   ggattccccc ccacccttga tgttcctcga acaagtggtt aaaagtgtga gaggctttaa 480
   aggcaaagcc acgtgccttc aggtggctga tattttccca aagaccatca tcaaacttcc 540
   agccaggtgg ataatatcat ggcacccaac caggtgtcaa cactgaattc acttggcttc 600
   aacgaggacc tggccataag agagagcaaa caggcctggt ttcaagtatc cangacctct 660
   gctcagatcc ctcactgnaa ttcttaaggg aagcagcatt gtgaaagact ggctaggaga 720
   aaagagaagg taatggcaaa tatttcctta attctgngac tttgggcaag cttactt    777
<210> 337
   <211> 829
   <212> DNA
   <213> Homo sapiens
<220>
   <221> misc_feature
   <222> (1)...(829)
   <223> n = a, t, c or g
<220>
   <223> nbla-11988-r1
<400> 337
   gnnagnnnnn ntttgaannc cntttgttgt ggcctttttt tttttttttt ttccccgaga 60
   cgaagtcttg ctctatcttc caggctgcag tgcagtggca caatctcagc tcatggcaac 120
   ctccccctcc caggttcaag caattctctt gcctcagcct cccaaagctg ggactacagg 180
   catgtgccgc cacacccggc tcgttttttg tattttagta gagacagggt ttcaccatgt 240
   tgcccaggct ggtctcaaac tcctgagctc aagcaatccg cccgcctcgg cctcccaaag 300
   tgctaggatt acaggcgtga gtcaccgcac ccagccaaat gtccattttc ttctggctgt 360
   aatcgtaaag gtaactatcc actctaaata catatggctt tctgggcagc actgagcaac 420
   tatatagcta tcaactagct gaaagaagga aacaaaaaat atggccctag gatagaatca 480
   gcccttctgt gtcatattca taaggactgt gccggaagca catgggaacc tggtccggaa 540
   acccttcttt ctaaaatgtt agcaagactt ttttctttgg tagttcatca ttgcattgta 600
   ccagtcacct ttctaagctc tttagaatca ttaactctgn gaattctcaa taataactct 660
   aatgggtggg aagtattgca gcccattttt caagtaggga aattgaggat tgagaagtta 720
   acntggccaa agtcccagaa ttaagggaaa actttggcat taccttntnt tttttctaan 780
   ccagcttttn aaatgntgnt tnccttaaaa attgcantgn ggggactga             829
<210> 338
   <211> 812
   <212> DNA
   <213> Homo sapiens
<220>
   <221> misc_feature
   <222> (1)...(812)
   <223> n = a, t, c or g
<220>
   <223> nbla-12017-f
<400> 338
   gnngnnttnn tntgaatccc tttgtaagat ccctcgngca ctgttggcct actggtaggg 60
   gttggatcct agagtagatg cctgaggcca catctgaaca gacctgtcag ccaggcctgc 120
   caggtcttca cgttgaggat tcaactggcc aatcacagga caggtgtcct ggcctttctt 180
   cctgaggtct ctaggggagg ggcatgggta agggtgtttc ctcagcaccc tcctggggtg 240
   gggattatgt ctgctgtcat gtctgggtct ttagggtagg acaggctgtg gtatgagagg 300
   caggagtctc cacaaggctt catgtggccc cttatagggc aggccctgcc ctctgggaag 360
   gtcccttcat gctggaggca cacagcttta aggaagtagg ttgaagtagg actccttcgt 420
   cctctcactg gctttggctc cctcaataaa ctgtgtggga acctggctca gtgtctgtct 480
   ctctctctca ctctctggtt ttcctatctg aggtctttca tctctcactt cangaaaaca 540
   caagttcaac aaagtcttca natgccatcc tgtgtaggaa gaaaataccc ttctggtgcc 600
   ccatgaaaaa gggaaatacc aaaaccattt ggtcactgac ctttgcaata aggtgcttcc 660
   ttaccaattt caaaaagctt cccttgcaag tagatatcga gagagcacac tttncattan 720
   agcctggtaa tacccatatn acctttgntn tgaagctggg cctgcaactt gngaancntt 780
   tggaaaagtn ancccttgac cccnaancta aa                            812
<210> 339
   <211> 851
   <212> DNA
   <213> Homo sapiens
<220>
   <221> misc_feature
   <222> (1)...(851)
   <223> n = a, t, c or g
<220>
   <223> nb1a-12017-r1
<400> 339
   gnngnnnttg nnnnccnttn atttgtggcc cttttgattt ttccnttttt tttttttttt 60
   tttttttttt tttttttttt tttttttttt tttttttttt tttttttttt tttttnnnaa 120
   nntttnttnn ttnattnttt nttttggnaa nngggnnnna nttnnnnncc nnggnngggg 180
   nncntnggcn naannnnggt tnnccnnacc nttnnccncc gggcctnngg ggaccccccc 240
   nccnaanccn ccngngnngn tnggnctncn ggnnngngtn nccnnnttgg gnnaattttt 300
   ttttngaaan aaantttngn tttngtnncc naanntnggg ggnaagggcn nnanntnggn 360
   taacnggccn aagntancct tngtncccaa gttaaagngn tttnncggcc naaaccntta 420
   aaggggnnga aattnnnggc ccccnccncn aantcnggnt ngntttttng nnttttnaaa 480
   aaaaatgggg tncccccntn tnggccangn nggtntaaaa nnccngtntt nnggggaccc 540
   ccctntttgg gcccccnaaa nngncgggnt tncngggggn aaccattggg cccggccnnn 600
   ttttggnntt tttnggaaaa aaggggtttt gccattttgc cnagggtngg ttaaaacncc 660
   nggggntaaa aggganccan nanntttngg ttnnccaaaa gggcttggna ttnnnggnnn 720
   gggccccccn ccctagggtg ggtanttttt ttaaaaccgc cnttttgccc anaaaaaggc 780
   cccttnggtg gaaaaccctt ggaaccccng gggggggggn ccncttttaa aaacccgcct 840
   tanggggnng a                                                   851
<210> 340
   <211> 768
   <212> DNA
   <213> Homo sapiens
<220>
   <221> misc_feature
   <222> (1)...(768)
   <223> n = a, t, c or g
<220>
   <223> nbla-12022-f
<400> 340
   gntncctcgg cctgttggcc tactggctac ttaagtgtaa acaaggcaag cctcagacca 60
   gcaataaatt actcagtttg gataacatta ttttgtgcag ttaatcaaat ttgccaaagt 120
   ctttatctgc ccctttaaca agttgagtaa aaataaaagg tattttttag tcaatgtgtt 180
   ccatgatttt gcttaaatta atacttttaa gtaatggaac ttttttcaaa ggcaaattta 240
   aactatttaa gaaatagctc ctaatacttg ggatcttgtt tagagaatcc actttctgga 300
   agttctcagc ataattagtg ttgagagtgg ttcagttgtc tttaatgttt gtcatgtgga 360
   aatggaagta gcctcttttt gttctgaaat tgagtttatt caaagtgtaa aagcacatac 420
   tgcattttct gctgaaagat cattatgttt aacaggcact taatctcagt aaagtcagtt 480
   gccagttaaa gttcccccag tagtcagtcc cctttgtagt tagtgggatt atttgataat 540
   tgggtagatc atacttgaaa atttaatgct ttgggtaatt ggttggaaaa cagtggaatt 600
   gggaaacgca aacttttggn cctattcnag taagggnaat ggagtcctgn ggaacccaat 660
   tgnatacgna aatttgnagg agtctaggta cattangcca tccntcntaa ncctggtgag 720
   tngaatgcnn tttagaanga ntacctacat cctttggnaa nncctaaa             768
<210> 341
   <211> 847
   <212> DNA
   <213> Homo sapiens
<220>
   <221> misc_feature
   <222> (1)...(847)
   <223> n = a, t, c or g
<220>
   <223> nbla-12022-r1
<400> 341
   gnnagnnnnn ttgtggccnt tttttttttt nttttccctc agtaagtatt ttattgagat 60
   ttaaagcaca aaataggtga acaaatcggg aggaaggtgg taacacagga atgggaacgt 120
   aaaagtcaaa gtaggcagga ctcaaactgg ccataaaggc actacggcat gttcataaac 180
   accatcaagt aaaagaaagg ggaatcaaga tgttctcact tcttttctgc tccaccttaa 240
   tactccatct agccactggt atggagcatg tcatcaactc attcttctca caagctagca 300
   agggaattaa acattttatc agtcaaccct atgctcacca agtgagccaa tgttacacag 360
   tgattttgtg catccagtga ctgagaacta caggcaagga taggccctga ctgttaataa 420
   attggtcgtg gctattacta gaaaattatg ggtgcaggca tttacctata caatgacctg 480
   cttactgtgg cagctaacaa tatagtcaaa ccagtctatt accatatgat gtcctattaa 540
   caagagagcc cagttgaagg atgtttcata cttttagaaa gcctaatttc ttactgnaat 600
   atcctccatc cctcttttaa aaaggctgna gttcaaaaag cttttatgnt ctttcctgaa 660
   acaaagagaa ctttggtcaa aagccaattt ttaagaagca tcacttctgc tgagtaggca 720
   aaacaatgga ctntnaccac cagacatnat tttctttcct taaaatctga nggttatgct 780
   ggnaaaataa cccatttttt tttaaacatc taattaataa aggntcaang aaatnagata 840
   tggagga                                                       847
<210> 342
   <211> 914
   <212> DNA
   <213> Homo sapiens
<220>
   <221> misc_feature
   <222> (1)...(914)
   <223> n = a, t, c or g
<220>
   <223> nbla-12048-f
<400> 342
   ggnnagttgt tcctntgcct gttggcctac tgggttacta gcttgtgtga agacattctt 60
   ttctccttcc attcttgttt acagttagct gagcagatga cacagtcaga tgcacaggat 120
   aatgctgact acagattatt tcagaaaaca ctcaaattgt gtcgtttttt tgccaactcc 180
   cttttgcact acgctaagga atttcttcct ttcctctctg attcttgctg tactttgcac 240
   caactgtatc ttcagataca cagcaagttt cctccaagcc tttatgctac caggatttct 300
   aaagcacacc aagaggaaat agcaggtgct ttcctagtga cactggatcc acttatcagt 360
   cagctgctca catttcagcc tttcatgcag gtggttttgg acagtaaatt agacctgcca 420
   tgtgaactgc agtttccaca atgtcttctt ctggttgttg tcatggataa gctgccatct 480
   cagcctaagg aagtgcaaac cctgtggtgc acagacagcc aggtctcaga aacgacaacc 540
   aggatatctc tactcaaagc cgttttctac agttctgagc agtgttctgg tgaactctct 600
   ctacctgttc atttacaggg attaaagagt aaggggaaag ctgaggtggc tgcaccttgt 660
   atcaagcatg tttgtgttca tctgnggtac atttattact tcctttcatc cctcactggt 720
   tggtgaactg gatgctgctc tgctgaatgc tgtacttaag tgctaatatg aacacctctt 780
   tgttagctnt gggatgcatg gngcttcctt gctcgatatn gggactgctt gaactggggt 840
   gcaccaccat gttaaccata agtgggntcn atcttgataa aagncnatnc ccctgggnga 900
   aatgtttttn aacn                                             914
<210> 343
   <211> 995
   <212> DNA
   <213> Homo sapiens
<220>
   <221> misc_feature
   <222> (1)...(995)
   <223> n = a, t, c or g
<220>
   <223> nbla-12048-r1
<400> 343
   ggnnagttgg gccttggnnt ttcctttttt tttttttttt ttttttnttc aaactttaca 60
   aaattngttt tatatctgtt anaaaangtn cagacataag tnttttcagt ngacaaagcn 120
   tcaaacccag ttntgccagt tctgcctagn gataagtttc accctagagt atgtatgtaa 180
   cgttttagct tatccatcct ttntnggagc gcctccnttt ccattgaaag ccaggctgga 240
   ncaggaccct ttnggagtag ggactnagtt gnttccaaag cccctgntnt tgtatgcagc 300
   gctgacctgt actnttnttc ccaggggaac tcctgacgag ctntttttgc ntaaggctgt 360
   aaanatgacc tttttgnttt ttntacagtc acattagcaa agggttccca naaaatacct 420
   ttttcctgtt tnacacgttc cantttggca gnttcagttt natttacaaa cccagtnttt 480
   tccanaangg atacaacttt gttnttagtt tnttcanaac tganacactg gggaactatc 540
   tnttcatgat tngttccctc agcgaactga gtaaacgcct ccaaggtntg ttgttttagc 600
   agccaactcc tgtcagntag cagtaaggca aacatacagg gacaggttgg gcagaattnt 660
   tggcctggga tangcttcag gtataaaaag tttttccnta anggaaggat ccccaaattc 720
   cccaanattt ggccaaaccn naacattagg ttcaggggaa gngcttttta nttttaaaag 780
   ntaaggccgg ggggggtctt ngacaaaaag gtttnaacct tgcncctngg aanggttttt 840
   ccantttttt tanggggatc ttttaaagaa gtttttggga antgggaaaa aaaaattccc 900
   ccaaancagg ggngggggaa nggtaaaaaa aangggcctg ggaaaatggg tcttgggtng 960
   ggaaaaaaat ataaaggggg ggtggngatc ccncg 995
<210> 344
   <211> 961
   <212> DNA
   <213> Homo sapiens
<220>
   <221> misc_feature
   <222> (1)...(961)
   <223> n = a, t, c or g
<220>
   <223> nbla-12055-f
<400> 344
   nngttgttcc tctgcactgt tggcctactg gctgaaatat taataggtgg cctataagga 60
   tctttcatgt tttttgaatt tcttaattaa gtatcatttt gtgttaagct tagaagactg 120
   agagctgagg agagtggaca aataaatgtt ggaattagga aagttacatg aaggaaagtg 180
   ggtctgagct gggttttgaa agtaggcaga atttagatca gtagagcaaa gaggatagga 240
   tagtctaaat gtggagaatg ctgtatgaat ggaggcagaa tatgtattga acatgaggaa 300
   ccatcaagag gccggctcag tgagggaaga agggggcttc tggaggcacc ataggtagac 360
   ataagacatt aacagattat tgaccaacag gcaggggagg ttaatatgct ccaatgaata 420
   aagagccatc atatatttta taatggagac taacaggttg atgttgctga gaaaataata 480
   tggactttgc attcaatgat gtatcttttc acaaccatat ctttgataaa cacaatttgg 540
   gttatactgg cagggattaa gtagtggaaa atctcttaga agaaatgtgc ttctactgtc 600
   ccccttacca aaatagaaca gatgagccct aatcagagct ggtaactgta atggtgattg 660
   ctctgaggac ccaatgtata taaacatcac tatttgaata cctagatttg ggccaagtaa 720
   taagggaggg aaagtttttg acccctgaga ttacccccca gaattaaaag taggggaccc 780
   tttttggaga tggggcttaa aacangnttt ttcaaggtnc agttagncac cccttggctt 840
   tttcaagaaa ggaaggcngg accnggaaaa ttccccctnc ctttggaant ccanaatttg 900
   gtatngtttt cccgggaatt ttttaacccc gaccttaaga tctcagggcn ggggttcanc 960
   a                                                            961
<210> 345
   <211> 902
   <212> DNA
   <213> Homo sapiens
<220>
   <221> misc_feature
<222> (1)...(902)
   <223> n = a, t, c or g
<220>
   <223> nbla-12055-r1
<400> 345
   gattgggcct tttttttttt tttttactaa ttcagttcat ttaactttgc ctccagcagc 60
   tcaccataaa atttgggtcc aaaaaggcca aggaatgtat tatcagaaat gttagctatc 120
   tctaggaaaa tctgtcattc atttaagtga taatctttaa aattagcata actatattct 180
   ggatcatttt attacgagac tatcatgtga tttcaatgtc tgagtttatt accattgtac 240
   gagcaacatg ctaagttaca actgtaactc ccagggttgg tttagcagga aagtgatgga 300
   ggcagactta acagtgatgc gtgtagttca gcaaaggcca aatgacagca ctgggagcca 360
   taatggtggt ttcctagtaa ttcaaacaga ggaaagtagc aagaatacac tgatctcaga 420
   ataccaggct tgtcagacta aaaaatcctg tgctttaaca gtcagtggtt gacagcaact 480
   tatcctaagc aaaaaaaaaa aaatcatcta aaacagatgt catggtaatt tcaattttat 540
   agctttctga agtgttgatt ctatttaatt tgcatgtttg ttttgttttc atggctgtat 600
   aaagacctat tacacaagag ttcaaagcct gttttttaac atatacttta atctcatgca 660
   atattataaa gccaaaaaat taaatatgat tttagaggta ttntattcaa gatgattttt 720
   ccccttttag gaagggccct tttcatttct caaaatttaa cctngcagtt tacccattgn 780
   ctttnttttg gttattcnag gccncctatt aaccttttca aaattattgg gtttancttt 840
   ggnaaaaang gctttttaaa tcttttcctg cccaggctaa aaattctnga gaacaagcng 900
   cn                                                           902

<210> 346
   <211> 1532
   <212> DNA
   <213> Homo sapiens
<220>
   <221> misc_feature
   <222> (1)...(1532)
   <223> n = a, t, c or g
<220>
   <223> nbla-12074-f
<400> 346
   ggcccgannt acnctnngga aagncccccc accgtttngg gaaaaaggac ccgccggggg 60
   tggctgcgtg ccctngtggc ggatntccag cctnttgtat gtcggcccan gngcgctgaa 120
   ggctgagggn gctttntgcn taggaancgn cncctgcggt ggangaggcn gcggttgccc 180
   catgnagtgt tactacattt gnaattagct tnacgcattt tannatcgag acacttnngn 240
   aacatnaagg gagnanntcc ggggaccttt tagcaagaac ngggagcana antnnggact 300
   ntgctganaa aggaanatac caatngaana agcttctgga agatctgaan gnnaatttnt 360
   antgttgaac ttcatgnnac aatgnantac tataagcacc acgagttcnn caattacant 420
   taatttnata ngancatgct nacagncatg aggcttnant ggaaatnnaa acannangng 480
   aatntgctcn aaaatntagc atctacnntt tnnncgcnga cgtnnngann nagaaaanna 540
   gngtannaag ggctaccttt naaananacc cggncannan annntgnnna ttggaanctt 600
   ataantaaaa angggaaaca anatngnnga ntggnagggc acnacctntn nggtaanggn 660
   agngnnacan ccncannnga tnttnacgaa antctnnaan cnatancncn gtngtatatc 720
   cttggtcntg atancaaagc nataaaaaaa cnnctgnggt atatatntgt ggaaaaaaaa 780
   tatnttgtct cncatnaaaa ntagangatn tntcnagnct ccttgntang cgaaanctnt 840
   ntntgtatnn atgtntntan atagtatccn cccnnnaatc tntacacngc aaaatttcnt 900
   ttacaacaan aangannnat ggancntcnn ttcattttna tatgttnttn anaaaagtat 960
   tgatntacgt nncaaaaaag cttaaangna aatnctnnnc tcntctattg acncgcnncn 1020
   atctggtana ntgtaaangg ctcaagnaca ctntacagat ttanagnctt tttcagnant 1080
   nanngtaaaa aaannctctc nggtaataaa nantaaangt ggttttccnt ncagcnannt 1140
   attnagtntt ntngtttant nccccacnan gtnccacact atatntttaa tttnttaacn 1200
   cnncatnann ntgntntntn nntggggntn gtnttataan aaaaaatcct tctcccgaga 1260
   nnnggncgat atatttatnn caccncngta nannnncccn cgggtannta nanttnccag 1320
   tgtgntcgna gacagcgtcg cntataaagn gnaaaaaant nttnccttcn naannanagn 1380
   atanagngnc cncnanntca tanaancgtg aaatncncnt aantganaan atcnttatgt 1440
   atanaacnta cgcgtnnact ctcnttnntn ntctcntgtn tcttatctaa tnggcnaaag 1500
   nattcgaatn cnntctccnc annacaanag cg                               1532
<210> 347
   <211> 1007
   <212> DNA
   <213> Homo sapiens
<220>
   <221> misc_feature
   <222> (1)...(1007)
   <223> n = a, t, c or g
<220>
   <223> nbla-12074-r1
<400> 347
   gttggggcct ttgttttttt cntttttctt ttacttnaat ggaaccagta ttcatgggtc 60
   tctttcaacc ttattttgtt gtatttttna tctagagtgt cctgttttat ttgacttggn 120
   agtanaatta tacttttctg ctnctctgat cttacaggaa atgtttttat acctctgacc 180
   tgtgttctca ttttttccag aatcacaact actggagaga atatcatcag ccaaaagagg 240
   agtgacttgg ctgcatttat ttttgctact tccaatttca caatcagtga gtccttctgt 300
   agatacatcc ttgtaatctg aaagtggtcc catgcaaatg tcttcctgct tctgctgaga 360
   acaaagatct tttaagtctg gcttattttt atctagatta ttatttactt tagtagactt 420
   gaagtnaaaa catagaggat tacagctata ggaaattgat ggtttcgtag ntgtataaac 480
   cagcatttct gatggccact gaagaactgt agatctgtgn ttgttaagga caggtacaaa 540
   tggcntcaca ttgncttttg cangaaattt gctnttctta tgtantagnn atgttnaccc 600
   tcttcagtat ttttgaaggg ggcccaattg tctcggggac cattttttta ttttcaacct 660
   caanttgngg ganncccncg gtttgcttta acatttttcc ttctntnggg nannctctgn 720
   ccncannatt tngccantna attttnacnt tgtncaagaa acntggcggg attttntttt 780
   tccnntaacc aaaccttcaa aaancnatgt ttnntcccca nnttaaacaa ggggngcnag 840
   nntcctttcc aattttnatn ttanccaacc cncccttttn ttctnnngga gnggggnnaa 900
   tttttnngtn cccagcgncc tataacnngg gggggacccn cngaaanann ttttntgtga 960
   ccncaaantn ntntttctng tcgctttttn cagagaaaan ntaanca 1007
<210> 348
   <211> 963
   <212> DNA
<213> Homo sapiens
<220>
   <221> misc_feature
   <222> (1)...(963)
   <223> n = a, t, c or g
<220>
   <223> nbla-12075-f
<400> 348
   ggnagnnntt ccttggantg ttcccctact ggttcgactt atctatnagc atcttcaaca 60
   atattcctac agttgtatgg gtggcatgtt ggccatttgt gatgtagccg aatataggaa 120
   gngtgccaaa gacttcaaga ttccaatggt attacatctt tttgatactc tgcatgctct 180
   ttgcaatctt ctggtagttg ccccagataa tttaaagcaa gtctgctcag gagaacaact 240
   tgctaatctg gacaagaata tacttcactc cttcgtacaa cttcgtgctg attatagatc 300
   tgcccgcctt gctcgacact tcagctgaga ttgaatttac aaaggaattc agtgtcagtt 360
   cctttacaga ggaatgtctt atacttcagc agccctcggt tgatagaaag cacaggagat 420
   accttatgac acagccaaca ttttgtgaaa caatgactgg aacaaaacag cagccatact 480
   tacctttgag gttttattta aagtttggat accactagct atattttgct tttttcccct 540
   cacattgaat tttaattcca ttcttgaatg tagaaatttc agattctcta aaactacatg 600
   tcactgtttt tatcctagaa aatgttgctg tcagaaggca aaggaaatgt taccagtgtt 660
   ttccggttct tgtacttttt aacatattcc atttagaaat ttttgccatt ctgntttcca 720
   ttaataatag gnngaaatac agggaaaaac tacattttgg tnattccctc aagtttttta 780
   antgaccctt tttcagncat caaatttggt taantcaang aatnattttt taaggttttt 840
   tccnnaanaa taaatttttt tttggncttc nttttccaaa aagggttnaa acnaatttan 900
   ancattactt ttccttgncc annttngggt ttganttggg aanttttttt ttttttggng 960
   ggg 963
<210> 349
   <211> 977
   <212> DNA
   <213> Homo sapiens
<220>
   <221> misc_feature
   <222> (1)...(977)
   <223> n = a, t, c or g
<220>
   <223> nbla-12075-r1
<400> 349
   gttggggcct ttgttttttc ntttttggat ngcttaaaat gtnctctcat gttttatttg 60
   agattttttt tcgttttttc ttttttncaa attgacactg tacaaagagc ttaaanggcc 120
   agtnaaacta ctgccttaaa attgttggct ttaagtaaca gaggtaaaat ttgaattcac 180
   atcagagctt tctaaaatgg caatagtaca agcaaatgac tttctctagt atccaaaacc 240
   tgcgctggct ctcccttggc tagaaacaag acagccttct aaagctgcca taatgtccta 300
   aaatgctctg aaagaagttg gtatacttca ttccccagct taccttggta acactggact 360
   aaacagaaaa tgaagaaaat tcagtcaata gtaacttaaa cagtactaag ttagctctgg 420
   acttagaagg tttggtaaag ttgatgacat tcagaataag gttctcagaa gaaaagcagt 480
   tttccaaaaa taggctaaaa agcctatatt aaagaaaaaa tgccaagagt gaattgaata 540
   cttaagttta tctgttccta gaagactatg aacaccacaa tgggagctga aacactatat 600
   ataatttaga atgcattctc ccagagcaag tttataaggt ttatagaata gtaatggcct 660
   ttctaaattt tcacttggat ttttaaaaat ttttattaag gtgattttat ttantgtcca 720
   ttccaaaatt taaagcccna tttcccattt aaacttatta nttcccaagg gttngggaaa 780
   ttaaaccttt tcangaaaaa tggccntttt tgnnaaaaaa aggggaacca aattggaccn 840
   ngganccctt tttnangaac ncntgggaaa aaaggccnaa accnanggcc cnttttggca 900
   aaaagggcnt ncattctttc ccaaaaattt ttattcaaat nttccttcaa aattnctttc 960
   ctttanaaaa naaaaaa                                            977
<210> 350
   <211> 2698
   <212> DNA
   <213> Homo sapiens
<220>
   <221> misc_feature
   <222> (1)...(2698)
   <223> n = a, t, c or g
<220>
   <223> nbla-12092
<400> 350
   taggcctgta cggaagtgtt acttctgctc taaaagctgc ggaattcctc gagcactgtt 60
   ggcctactgg ggtgctttct gtttaacctg gaaagtgtga ttatattcgt taccttcttt 120
   ggtagacgga atagttggga ccacctttgg tacataagaa attggtataa cgatgctctg 180
   attagcacag tatatgcata cttctccaaa gtgatatatg aagactcttt tctttgcata 240
   aaaagcatta ggcatataaa tgtataaata tattttatca tgtacagtac aaaaatggaa 300
   ccttatgcat gggccttagg aatacaggct agtatttcag cacagacttc cctgcttgag 360
   ttcttgctga tgcttgcacc gtgacagtgg gcaccaacac agacgtgcca cccaaccccc 420
   tgcacacacc accggccacc aggggccccc ttgtgcgcct tggctttata actcctctgg 480
   gggtgatatt ggtggtaatc acagctccta gcataatgag agttccattt ggtattgtca 540
   cacgtctcct gcctcgcttg ggttgccatg tttgagcgat ggccctgttg atttcaccct 600
   gccttttact gaatctgtaa attgttgtgc aattgtggtt atagtagact gtagcacatt 660
   gccttttcta aactgctaca tgtttataat cttcattttt aaagtatgtg taattttttt 720
   aagtatgtat tctattcata tggtctgctt gtcagtgagc cagacttgct tactatattc 780
   ctttataata atgctagcca cttcctggat tctttagtaa tgtgctgtat gcaagaactt 840
   tccagtagca gtgaaggagg gttgcctctc caagcttcct aagggatgct gccctgtgtg 900
   gggatgcatt gcagaggcac tagtagcatg ggggctagag tggggagcga gatgtaaaag 960
   ggtgggggga taggagaatt ccagagtgct tccagcatta gggtcctgag aacttctgag 1020
   ttcagagaaa catgcaaagt gactaacaaa atagctactt acctttgcag ttctacagac 1080
   cctgggagct gctttgggag tgagaaaggc aaccctccaa tgtgtttcaa ctttaaaatg 1140
   ttgaattctt ttcagacatg gtatctcatt tattctcctt ttctagcgtt tgttgaattt 1200
   caggcagaat gtcttacaga atgtcctaga accagattat catttaatct gaaactgctg 1260
   aggaagggac agagaaggta caagggcaag gcagcacaaa gcagatcagg agaatgaaga 1320
   gggaatgctt tggttttttg ttttgttttg ttttttcttt ttcaagtaac taaaacagca 1380
   tctacatgta gagtgttgtg gagagctgag accagggtaa agtcaagtgc agcatcagta 1440
   ctgcgagacc caccagcccc tggagagggt cagccgagaa tctggtagtg aagcctgtct 1500
   agggtcccgg caccctcacc ctcagccacc tgcagagagg ccagggcccc agagactagc 1560
   ctggttctga agtgggcagg ggtgctgcca gagccctctg ccccttatgt tgagaccctg 1620
   ctttcaggac aggccagccg ttggccacca tgtcacattc tgagtgagtg tcacaggtcc 1680
   ctaacaataa ttttctgatc tggagcatat cagcagaatg cttagcctca aggggcctgg 1740
   aagctgtaat gtttgattta tgatgagaac tatccgaggc cacccttggc ctctaaataa 1800
   gctgctctag ggagccgcct actttttgat gagaaattag aagagtacct aatgttgaaa 1860
   acatgacatg cgctcttggg atctgctgtt ctctccaggg ctccagaacc tgatacctgt 1920
   taccaaagct aggaaagagc tttatcacaa gccttcactg tcctggcatg agaactggct 1980
   gccaggctca gtgtacccca ttaactgtga atgaatctga gcttggtttc ctttattgct 2040
   tcctctgcaa tatgattgct gaaacacatt ttaaaaattc agaagcttgt cactcctgtt 2100
   aatgggagga tcagtcacac atgtgtagta caaggcggac tttgtgtttg tttttggtgt 2160
   taatttttag cattgtgtgt gttgcttccc caccctgagg agaggacacc atggcttact 2220
   actcaggaca agtatgcccc gctcagggtg tgatttcagg tggcttccaa acttgtacgc 2280
   agtttaaaga tggtggggac agactttgcc tctacctagt gaaccccact taaagaataa 2340
   ggagcatttg aatctcttgg aaaaggccat gaagaataaa gcagtcaaaa agaagtcctc 2400
   catgttggtg ccaaggactt gcgaggggaa ataaaaatgt tatccagcct gaccaacatg 2460
   gagaaacccc gtctccatta aaaatacaaa attagcctgg catggtggcg catgcctgta 2520
   atcccagcta ctctggaggc tgaggcagga gaatcgcttg aacccaggag gcggaggtcg 2580
   cagtgagccg agatcatgcc agtgcactcc agcctgggta acaagagtga aactccgtgt 2640
   caaaaaaaaa aaaaaaaaag gccacatgtg ctcgagctgc aggtcgcggc cgctagac   2698
<210> 351
   <211> 968
   <212> DNA
   <213> Homo sapiens
<220>
   <221> misc_feature
   <222> (1)...(968)
   <223> n = a, t, c or g
<220>
   <223> nbla-12112-f
<400> 351
   gttgttcctt ggcactgttg gcctactgga agatatattg attgcctgcc gattgaacca 60
   aaaaaaaggg gcatatgata ttttcctgaa tgcaaaagag caaaacattg tgtttaatgc 120
   tgaaacctac agcaatctca ttaaattact gatgtcagaa gattatttta cacaagcaat 180
   ggaagtgaaa gcattcgcgg agacccacat caagggcttc acacttaacg atgctgccaa 240
   cagccgcctc atcataacgc aagttaggcg ggattatttg aaagaggctg tgacaacact 300
   gaaaacagta ttggatcagc agcagacccc ttctaggtta gcagtgaccc gtgtcatcca 360
   ggcattggcc atgaagggtg atgttgaaaa catagaagta gttcagaaga tgttaaatgg 420
   actcgaagac tccattggac tttcaaaaat ggttttcatc aataacattg ctttggctca 480
   aataaagaat aataacatag atgccgcaat agaaaacatt gaaaatatgc ttactttcag 540
   agaataaagt cattgaaccc caatacttcg gcttggcata cttattnaga aaagtaatag 600
   agggagcagt tggaaccagc agttgaaaag ataagcattc atggcggaga gattggccaa 660
   ttcaagtttt gcaatttatt aaacctgtcn ctgatttttt cctttcncct tgtnggattg 720
   caaggcaaag gtggattgat gccaagaagc tcttcctaca gganaatgtg gntgccaatt 780
   gcttgaacca aaaccccgga tttttgttgg ntggtccctc cttaaggaan ttcttaggga 840
   aaccnangga aagggccttn taancttgtg gaaantntng gngttnaaaa attgntttcc 900
   ctggntttaa attgaaaang gngngnangc cttccnnatt tcccnnatgn naaaagnntt 960
   tttnttct                                                          968
<210> 352
   <211> 913
   <212> DNA
   <213> Homo sapiens
<220>
   <221> misc_feature
   <222> (1)...(913)
   <223> n = a, t, c or g
<220>
   <223> nbla-12112-r1
<400> 352
   gtggcctttt tttttttttt tttataggga tcacttttat ttcaaacaat taaatacaaa 60
   ccaatatttt accccttcat agatgaaatc acatcttttc aggatatgag tataaagtaa 120
   caagcctagg gcagagcttg tactgacaaa gtcctgaaac tacaatgaga ggaaacacat 180
   tgctctactt cgggataagt catgaccgag actcaatttc agagacgctc tatgaacaga 240
   ggtgcttgaa gccacagtgg cagaagggaa agatggggaa gtgtgccgaa gagcctccag 300
   gcatgacaga cagtcccctg accaagcaca agtaacaggc cctttgggtc tctgcttctc 360
   actggaaaat gatgaagcct agatctgatg actcctagtg ccaacattta acaaagttcg 420
   aaagttatgc aggacttcac acatgtacgg aatggctgta tcacagaata ttatgcgtta 480
   gaaagttcac ggtcactata cctagctcta aaatttttca gagaaacagc agactaataa 540
   gtgaatctta aataaaggaa taacatttta atgaaataaa tgaaacagag cagggaaacc 600
   aaagagccaa ttaggggaag antcctgaaa aagtatggct tnacacagca gcagcattga 660
   agaaaacact atcgattttt nccagagaaa aaaactccaa aaatgtncaa taacccaagt 720
   gcccagagtt ntgggtcaaa taaagacttt tgaaccatgc catcaccaca taccntntgg 780
   tcccttaaag naaaattttt tcantttttt tttttcccnt caagntataa ctttaaaaaa 840
   taacccntgg tgggaccacc nngaatcncc aaaattattt tcccnaaaac caaaagttnt 900
   cgccntggnt tnn                                                    913
<210> 353
   <211> 1133
   <212> DNA
   <213> Homo sapiens
<220>
   <221> misc_feature
   <222> (1)...(1133)
   <223> n = a, t, c or g
<220>
   <223> nbla-12119-f
<400> 353
   gttgattcct cngcactgtt ggcctactgg aagtttaagg aattttagta tgtaatagca 60
   aagtgtttga ttaaaagtct aatcctatat tactagtcaa aattagttac attctgatct 120
   aggaaagata atagtcattg gatattgata tgctgtgctg tcagaaaacc agactaagat 180
   agaaacctct atctgactcc cacatgttgc ttgttgtgat gggatgtata ctttacagca 240
   gcttattgct gctttgaaaa aaaatgtata aactatacat ttggagtgtt tgcatataat 300
   tctttataac ctccacttaa agctgtcaga cattggtatt ttatcagtcc acattgttga 360
   ataaaactaa tgttcttagg aatccagctt gtacacactg tttaaaaacc ctcagggaca 420
   gtttacacac tcttntcact caattcaggt actttgatgc tatncttaaa cctaacagtg 480
   acttgtattt ttctgttttg ctttnatttc aacgtgctgg tagcacatct gatgaatgtc 540
   aacttaaaaa actcagntcn agggtatcca ggnntnactc anccnaacag attttaaagc 600
   tggcctntta actctncagc acatgggtgc ctcacacnnc ttataagtgg gcnttcctat 660
   ntaatncagg tcntttactt cnctgnnncc agnatnaatc attnggggnn nncccnngtn 720
   tacanaaggg nctatctttt ttcaaannaa anaaggccgc cttttantnt tgtttttngg 780
   tccaggncta tcaacttttc ancacnacnc tntctcttaa gaacccttgn gcncnntngt 840
   tttaaacaac tcttnnttnt ttttctanaa nantggggcc cncnntaatn tnangnccct 900
   angctnacan aactcatttg ttaaagcnaa tctgtnnntc tcttancctc tnacaatctn 960
   ngnnnctcct cgggnnaagc gncccgggng ngtaaaacca angaaaattn ngccacgngc 1020
   tntccctttt ttntatactc ggactntcat gtgggcctnn catctntgag ngtttattnc 1080
   ttcttcncta gcgtnngtaa nnacntccta ccccttnctt natanaaacc tct        1133
<210> 354
   <211> 952
   <212> DNA
   <213> Homo sapiens
<220>
   <221> misc_feature
   <222> (1)...(952)
   <223> n = a, t, c or g
<220>
   <223> nbla-12119-r1
<400> 354
   ggnnagttgg ggcctttttt tttttttttt tttttttcca acaaaattta ttagaagaat 60
   agtggttttg aaaactctag catccagtga gaactaccat acacaacatt acagctggga 120
   atgtttntcc aaaatgtcat ggtcaaataa tacaatggaa ccattaaatc ttacacatgc 180
   acgaaagaac aagcgctttt gacatacaat gcaaaaaaaa aaaaaagggg ggggaccaca 240
   tggattaaaa ttttaagtac tcatcacata cattaagaca cagttttagt ccagtcaaaa 300
   atcagaactg cattaaaaaa tttaatgtag tgcaatcaaa ccaaaaaact taatttgnga 360
   tcataagtgc tctactacat aaacattgat catagcangg aacaaaaagt tcaaatcaca 420
   cagtacaaaa aaatntgtaa ataaatataa actacagtcc aagagaaata ttaaattnta 480
   caattccatc aaactgtaaa cagtntattg aaatgaggtc cataagagta aagggggttc 540
   ctgttttgtt gtatgacact tgaacttnta gaaatntgaa agataccatt ttccatatnt 600
   ccaaaggcta tgggttttat atgtggagag aatttatttt agatgtaaga tttcaaatat 660
   taaagcagag aaataatcta tttttgttaa ccacagaata gtactgcaga aaaacaaaaa 720
   agcaantttt aaaggaggca aggtntttaa ccattttaaa ngatgccaaa agggacttaa 780
   aaacaactgg gaaatttagg aaacttttgc cattcannaa aaaaaaactt ttgttaacct 840
   tggaanaacc tttcaaangg ggttaccaaa aaataacccc cngggccaaa aaaacttggt 900
   cttggcttgg attatttgcc aanaccccta naaacccttt ttggttttaa tt         952
<210> 355
   <211> 957
   <212> DNA
   <213> Homo sapiens
<220>
   <221> misc_feature
   <222> (1)...(957)
   <223> n = a, t, c or g
<220>
   <223> nbla-12121-f
<400> 355
   gtgttcctcn gcactgttgg cctactgggt catgagactt attcactatc acaagaacag 60
   ttcaggaaag acctgccctc atgattcaat taccttccac caggtccctc ccacaacatg 120
   tggaaattca agatgagatt tgggtgggaa catagccaaa ccatatcaac ctctttaata 180
   aatatttgtt gaataaataa atgaatacat ggacgaacgt ttcttcagcc tttactgcct 240
   ggtgcaagtt gagtgggaat tctgcctcta gtgcatcctt gttgccaaga gagacgcagt 300
   tctgctgctg ccaccagatg catttctctg ccaatgtgtt ggtgcaggct aatggaaatg 360
   cactgctgct cctacatctt ccttgtggat cttcatcctc cttccccatg cttttcaaaa 420
   aatgagatga ctacttctct cttctatatt atttattctc tgcttggctg ggggcagtag 480
   aatgggttga attctcacag caagattact tggttcctaa attttttttt agcaattatt 540
   gtgacagctc tttcaatgaa tatctatggt tctattactc tccagggagc acacacacct 600
   ccatagtgtc ttcaaaagga tttgggtttg gatnagaaca agcagccatg ctcaagccnt 660
   ggtctacctc ttccttctga cctttatgca ttgtttataa agtcangntc actgtgacaa 720
   cattcatttc ntttccttnt tgatgaattt tctttcaang ggctttatta acccttccct 780
   tnacaaactg ggctttctgg ccaatnccan ccatnnttan gtcttgttgg gncacaattc 840
   tttgggcnaa gttttggttt tattnnaggg atttcaagnc ntttnttntt ntnaaaagnn 900
   ttctttcaat ttttttattg gtaaaccaac ctttnngccc aatgggggga acacnac    957
<210> 356
   <211> 891
   <212> DNA
   <213> Homo sapiens
<220>
   <221> misc_feature
   <222> (1)...(891)
   <223> n = a, t, c or g
<220>
   <223> nbla-12121-r1
<400> 356
   ggnnagttgg ggcctttttt tttttttttt ttgtagagat agggtctcac tatgttgcct 60
   aagctcgtct caaactcatg ggctcaagta atcctccttc ctcagcctcc caaactgcta 120
   ggatcacagg cgtgagctgc tgcacctagc cagaagatca attttgatac acactatggt 180
   atcaattgaa ttttttatat atttgactgt ggagaaaata atggactgaa aaccctagcg 240
   ttcttacttt gaaatccatc actgcatgtg caatgaggtc ataatcaagg gtggtgagga 300
   taccgagtca ggtctattct ggaaaatgca gtcctcctct gaaggagaag gtttgtctca 360
   agaagtcagc atcaaccttt taatctttct ataattgcat gctgtctaag ttttttccat 420
   ccaaccttcc ctctcttctt tgcaagtgta agactgtcat ccaactctga tggctctcct 480
   ggcctcctct aacttcccat ttttcctcac aggtatttcc cctcatacat aacttgcatg 540
   tctaatcctg tcttggagtc tgattctcat agacctcagc ctaacaaaaa tggcaccaga 600
   agtggtccaa gaaaacaaac agtataatag agatatagga acggctcata taccaccaag 660
   tgggcaaaaa gaacagtatc ctgactggng gatngtgaca cattgtccct tgaatcaana 720
   atggtaaaga actctagctc aatatttagg tagattcagc agtggaatta tctaaccaag 780
   nggncctata ctgggccttt acaaccaaaa tttaacagct tcatggtttg gcttttcttt 840
   aatactattn ccctcttttn tttgngaaan ccaattattt tttttttntt g          891
<210> 357
   <211> 978
   <212> DNA
   <213> Homo sapiens
<220>
   <221> misc_feature
   <222> (1)...(978)
   <223> n = a, t, c or g
<220>
   <223> nbla-12124-f
<400> 357
   gttgttcctc ggcactgttg gcctactggt gctgttgcta tggtgaaaac aaatcaatat 60
   cacatggcta ccagccagtc ccgcgctcag ggagtgccat agaaattcaa gatcaggtac 120
   tcaagattcc aagcaagaat tgggaaaaga ggatttagat gctgagctta agagaattcc 180
   ttatagtaga ataagtaggt tcaatctctc atgcattttc tcttcctttt ttcctgtact 240
   ttcctaccga taacaagaaa aagatgaaaa ggaaggtata ctgatttgtt tacatttcca 300
   aaatgacaaa acagattatc aacatagaaa taggttggca acattgcaag gggtgaagga 360
   ttctgattcg ggaaaaggtg gataactaca agacatgttg gttgttgaaa acatagtttt 420
   gggggccttt accatcttca ggtacagcta catgtcagag ctggctgggt tgtgccaagc 480
   agagaaacct ggcaactcta gggggcaggt gggcagtgga gataaagggg gttataaaaa 540
   ggtagggagg tgtgggcata aaaatcaaca agcaagaaag cccagagaga gctgcagggt 600
   caagagtcag gccaggcaag cacaggatcc ctgnggcatg gcagaacagg gagaggacag 660
   tttcgccctg ttcacgaaat ctgttnaaga cccaagctna agggctagcc anagaaaggc 720
   angaaaatag cttgcnnaga ggaagtnaag gtgtcatggg gcccaggcct tccttttccc 780
   ggaanggggt ncnncntnca accttggttg aaccattngg ttnccccccc tnggttgggg 840
   gangangcca aaanancttn ggnnattttt taaangcntt tggggtttcc nttttccaaa 900
   aaaaaattna anggccnggg aannnacttt ttanccgggc tttttgntna natttantnt 960
   taaccttccn ntttttnt 978
<210> 358
   <211> 916
   <212> DNA
   <213> Homo sapiens
<220>
   <221> misc_feature
   <222> (1)...(916)
   <223> n = a, t, c or g
<220>
   <223> nbla-12124-r1
<400> 358
   ggnnagttgg ggcctttttt tttttttttt ttgatttttt ttttgntttt ttttttgtaa 60
   ctctgctttt aaggaaaaag ggggaaagcg aaattctgca gagtcaggaa acaggtttcc 120
   ttgctagaga tagagacagt gagataaagg catggggctt cccccacacc ctgcagctca 180
   naagtgtggg acaggagtct aagacttcct gttgttggct ggtatttaca gctttttaga 240
   tgatatgtcc attttcccca ggcactttgg ggccaaaact cagtatgctg agactcagaa 300
   attgctctca gtagcttcat attttgattt ttttttgcac cattcttatc tatctattgc 360
   tcatctgaca tctttatatg ttatttaaac agtacttatc tggagaaata taacagaggt 420
   caagaaagta catacataca gatacctaaa actcaaaagt ccatctcttg ttctccccca 480
   atcactaaaa ctgccccaag cctaaaatta atctgatggt taacattgct aaattaatat 540
   tagccaataa agttattcaa gtctttgaaa cttacaattt tgcatcattt atttttgtca 600
   cacacacaaa ggcaggtatt aggttactga gttgggtttc tcaccaaata gcaaagccag 660
   ggaagcccan aggtgtttaa taaaccttgt tgactgattg atgggaatat actacctagt 720
   ttaattnaan caatgcattc ttttggnaaa aacactaagg gtaatttatt ttgggtcccn 780
   cttttttttt accccaagcc ggttttcttg cttgtnnttg ganggaaggg ccagggccna 840
   nnccccaaaa aaattnggtc ccntttaaag ccttnaagna gntttaaggg ttttacnaaa 900
   aaagaaaaaa ccnttn 916
<210> 359
   <211> 970
   <212> DNA
   <213> Homo sapiens
<220>
   <221> misc_feature
   <222> (1)...(970)
   <223> n = a, t, c or g
<220>
   <223> nbla-12137-f
<400> 359
   gttgntcctn ggcctgttgg cctactggat aacctgtttc ggaatagtct caagatgctg 60
   cttactggtg ggaaatcaag tcgtaaaaac aggtcaagtg gtggcatctc cacagaaagc 120
   gactgtgctt ttgagccaga ctacgctgtc ccgccacttc cagtgagtga aggtatgcag 180
   cacattcgga ttatggaggg catgtctcgc tctcttccat cctccccctt actcacacat 240
   cagtctatca gtgttcggct ccagcctgtg aagaagttaa ctggtgatgc tgaacaggag 300
   cttggccccc ctccatctgt agatgaggca gcaaacacac tcatgactcg tctgggtttc 360
   ctccttggag agaaagtcac agaagttcag ccaggtgacc aatacagcat ggaagtacag 420
   gatgaaaatc agacctcagc aatcacccag cggataagtc cttgttccac tctgactagc 480
   agcactgcct ctccaccagc cagtagcccc tgctctacac tcccacccat cagtacaaat 540
   gcaactgcca aggactgcag ctatggggct gttactagtc caacctctac ccttgaaagc 600
   agagatagtg gcatcattgc tacattaaca agctattctg aaaatgtgga acgcacaaaa 660
   tatgctgggg gaaagcagta aagaattang atctggagga aaacataaaa ccttgggcaa 720
   ttttcaaaaa atccancatg ggacctcctg ntttgntatt cggagtagat tgaaaaacat 780
   taacttgctt nccaccctat tnagtncttg gaataaagaa tccccacang aagaaanttt 840
   tgggaaaaca gtggnttaat tcttaaatca agtacanggc taatttcctt ttggnaatct 900
   taaatggccc ccggggccaa annttnagtt nngcaggcca ccnagttttt gccccacttt 960
   gggaaanant                                                    970
<210> 360
   <211> 950
   <212> DNA
   <213> Homo sapiens
<220>
   <221> misc_feature
   <222> (1)...(950)
   <223> n = a, t, c or g
<220>
   <223> nbla-12137-r1
<400> 360
   tgtggccttt tttttttttt tttggacttt ggaatatttg catatatata atgaaagatc 60
   ttggtgatag gacttgggtc taaacaagaa attcatatgt ttcatacaca ccttatacac 120
   atagcttgaa ggtaatttta ctcaacattt tgtgaaacaa agttttcact gcaacccatc 180
   acatgaagct aggtgtggaa ttttccactt gtggtgtcat gttggcactc agaaagtttt 240
   tgatttcgaa gcattttgca ttttggattt tcagattagg gatgctcaac ctgtacatta 300
   tgatcagcac agttcacctg caatatggct agggctgaat ctggtccgca gtcacttctg 360
   aaatccaaga tcttagaaag gttatttccc ccattaccac agccccttta catctgtttt 420
   ctttcagaat ttcgaggaac taatgcttcc aaatgttgat aattacacaa tgcctgtcta 480
   tgaggagtac aatgtatttg tgtttatctt tctctagact cagcagaaat gaactggtca 540
   catgctaaaa tggatcattt cacttcttag ggaggtttaa gtaagtatgg gaataggaga 600
   caaagcaact ttttggcttt ttgcccattt ccatctgttc ccgggcaccc tttanagccc 660
   ttctgtggtt ctaacctctt aaaatccctg ctgtgtgcga gcnccccccc atgctctgtc 720
   ttggcattcn ntagannaag ttcctcaaag ggtgttattg ttcttntggc tcattcccag 780
   ggttaagcan aggggncntt tncaaangtg gggccacgaa ccctcnggtg ggcctggcct 840
   tanctggaaa nttttgggcc ccgcgtgggc cnttgganga nttncnanng gggnanttnc 900
   gcccctggtc ccttggantt gggnaaaaaa aacnnccttg gtttttccca           950
<210> 361
   <211> 980
   <212> DNA
   <213> Homo sapiens
<220>
   <221> misc_feature
   <222> (1)...(980)
   <223> n = a, t, c or g
<220>
   <223> nbla-12156-f
<400> 361
   gntgattcct cngcctgttg gcctactggc attttgagtt gttctagtgg tatatgaagg 60
   aggctgggat aactagcttg aaagaaattc agtctagtta tagacatctt tggcattaat 120
   ctgatgttta ctagtgatat ctcatgctag gcagttatgc tttgcttcta ggggcttctc 180
   tttttaaaac aaaagaaagc tcttttcgtt ttctgtgtgc tgcatgctcc agtgtgtgtg 240
   tttacaccat cggttcttct ccctctagag attagcataa ctccctttgc tgttggattg 300
   ttattttgag caatatgttt tggaaaggtt ggttttcatc atgagtgcac gcaaatcatc 360
   agatgcatct gcttgctcct cttcagaaat atctgattcc tttgtgaaag agtttctagc 420
   caaagccaaa gaagactttt tgaaaaaatg ggagaatcca actcagaata atgccggact 480
   tgaagatttt gaaaggaaaa aaacccttgg aacaggttca tttggaagag tcatgttggt 540
   aaaacacaaa gccactgaac agtattatgc catgaagatc ttagataagc agaaggttgt 600
   taaactgaag caaatagagc atactttgaa tgagaaaaga atattacagg cagtgaattt 660
   ttcctttcct tgttcgactg gagtatgctt ttaagggata attctaaatt tataccatgg 720
   gttatggaat atgtcccctg ggggggtgaa aatgttttca catnctaaaa angaaattng 780
   gaaagggttc nngtngagcc ccccannccc cgggnttctt atgcnagcct ccanaataag 840
   gnggcntnnc nattttcggn gtacccttcc ctttcnactt aggacccctc attttttccg 900
   ggaggantct taaaaanccc tgngaaaaan tcttctttta aatttggacc cccttcaaag 960
   gggctttttt ttccccgccg                                       980
<210> 362
   <211> 942
   <212> DNA
   <213> Homo sapiens
<220>
   <221> misc_feature
   <222> (1)...(942)
   <223> n = a, t, c or g
<220>
   <223> nbla-12156-r1
<400> 362
   ngtggccttt tttttttttt tttggggttt ngaaccnnag nnttantngg naangggtna 60
   ntccatnnta cnttncnnta ntcnannncn tttnangggc aaaattnccn ntttnnngnn 120
   tttncntttn nnnnantaag gggggtanna nnnggggnta ntttnttttc cnnnnngggg 180
   nnnnnnantn gggnccttta nnttaaaaac caaaggtccc ccaantttcn ngnnttnttn 240
   ttaannaang gangggnaaa ccnaannntn ggnnnttant anggttttnn ggggnnnttt 300
   ttttangggn cccagnnttn aancngtttt natttnaaan nttnnncngn tnttnaantc 360
   caaantttna aggttggttt taaanngatt tttttttctt aaccggnnga ttttaaantt 420
   nacccantnn nnngggntng gcaanncggg gccatnnctt aaaatngann nccctttatt 480
   tttanntanc ccngtttttt tnannaangg taantttant tttnncngnt ncctngttna 540
   aaaatggnnt tnnttttttg attttaatta aaggtatttt tggtaannnt ncangacctt 600
   tttaatccng ccnnttaann gnttggaacc atttttttta ccatttccat ncttngtttt 660
   ggntttagna aaaaatnttt cttccnttgg ggattttaaa ggggattttg ggtnntcncc 720
   atcattntta aatntggacc nttntanccc tntttggnnn gtnttcccct ttttttnaaa 780
   aattttttnn aaaaaaanaa aanacnacct gncctttgng ctgggggtaa aanttttttc 840
   nnnanaaaaa gnttngggnt ttgggttccn ttttancaaa aagaggtttt ntttttccca 900
   naaaaccnaa accntttttt cntntaaaaa ntcngnggac cg                   942
<210> 363
   <211> 727
   <212> DNA
   <213> Homo sapiens
<220>
   <221> misc_feature
   <222> (1)...(727)
   <223> n = a, t, c or g
<220>
   <223> nbla-12204-f
<400> 363
   gnttgattcc tcggcactgt tggcctactg gcaatcaagt aattttatta gcttcttttg 60
   gaccctctaa atattgactt ctctcatgaa aaaataaatt gatgaaacta atgattacaa 120
   agatataatc attttttaaa aagtgattgc ccaatgtatt tctctaacaa ttgtcacaag 180
   agaaagcata acaataaaaa tacaaaaaca tacagattta gatgtaaaat ctatataagc 240
   tatattttta gggaggctaa gcagatagta ttactgtgga agaattatca agttttattc 300
   acctcaaatc ccactgggtt cttaaaactt gaaaattcaa attgtagaga attatgagac 360
   acaatgtgat gtttagttaa agtcatgcta tacctttctg ggccacatat tgctaactct 420
   gtggctaatt atgcaattaa ttctcaacgt atcaaagctt ttcactggca gtaaattctt 480
   tgccctcagg tgaagtggat tgaaaagaca tcaaggatca aggataatca ctttgaatct 540
   gttagttttt ccccctacat tccagacact ttaaatttgg atgctttcat tttttttaaa 600
   tcaaaccaca caaatatgca gatactttcc cagaatttcg cagttaaatg gctgatcctc 660
   ttgaaaacta accttaatgg aattctaaac atttcagttt agaatgactt tgaaaaattc 720
   cttagat                                                         727
<210> 364
   <211> 709
   <212> DNA
   <213> Homo sapiens
<220>
   <221> misc_feature
   <222> (1)...(709)
   <223> n = a, t, c or g
<220>
   <223> nbla-12204-r1
<400> 364
   ggnnngnttn gggccttttt tttttttttt tccttttttt tttttttttt tttttttttt 60
   tttttttttt tttttttttt tttttnggca gggggggtng gaananccaa tgtttttntt 120
   taanaaagtc ctgnttaaca gcntnttnta ncanaaaang nnggnttttt aaaacgggct 180
   tgaacttttn cncaatntgt tttgaacnct ggcntgcccn cctgngtnca tntntnctgn 240
   tnggngctnt ggttttaatt tttaatttca aaaaattttg ataaaactaa aangttaaat 300
   gncccctnta atgaaatcnc tgntttaact aacnccnncc ananggnttg ntttcnttgg 360
   aaaatttttt aaagcntttt gaattaaagt aanaactttg ttttttaaan aacnccnttt 420
   aanggncgat taanggnngc nntttgcccc aangnngggn ttccctaatt tgcctaangg 480
   taaaaancnc tggggngntt gttaanagtn ctgttttnca gggcctnccc cagngttttt 540
   gaatntaaat ttttannggg gaaaggggga atttgcctga acccaancaa ttnctgtant 600
   cagggaagtt tccaagctcc cctnntttac ggcaaanggg ttttgaaact ngcntttaaa 660
   aanaccatag gnggggngaa ttagtnnttt gggcngtttt atttttacn            709
<210> 365
   <211> 724
   <212> DNA
   <213> Homo sapiens
<220>
   <221> misc_feature
   <222> (1)...(724)
   <223> n = a, t, c or g
<220>
   <223> nbla-12217-f
<400> 365
   gnttgattcc tttgcactgt tggcctactg gattgtggga tgatcatggg aacgattctt 60
   ccttaagccg aagtcaaagg gaaatccacc ctcacgctgc atgtagtttt cggtcattta 120
   cacaaaccaa cagttttatg gagcgccttc tacacgctca gagataggga tacgtaagat 180
   aaatggccca gggtctggag aactagacga ataagcagtt tcagcacaat atgataaacc 240
   ccacctaaga ggcagggaaa cccctcaaaa ttagtttcct tattccccct atcttattcc 300
   tcctatctat atctttctta gggttgaaaa tcctaattaa aaagtagtaa ctcgcgggag 360
   atttcattgc taaacatttg tcaaccactt tcttgtttga ataagtgact tccccaaatt 420
   agcaaagacg actctgaaaa tagaatgctg cataggcaag attggatgag agagagaaga 480
   tcttggagac aggacaggta actggagatc gttcctgggc aagtgtgtat ggagaagtgt 540
   ccgtaccctc aatcagtaaa tcttagtgta atgtcatcag aatgagcatc aaagagagcc 600
   ttaagggacc cgagaataaa ctaggattct caaaccaaac atggcccaca gacagcttgg 660
   tggacaggca tgtttttctt ttgctgcact ctagcctggg cgacagtgcg aggctcttgt 720
   ctca                                                         724
<210> 366
   <211> 869
   <212> DNA
   <213> Homo sapiens
<220>
   <221> misc_feature
   <222> (1)...(869)
   <223> n = a, t, c or g
<220>
   <223> nbla-12217-r1
<400> 366
   gnattngtgg cctttttttt ttttcttttt tttttttttt tttttttttt tttntttttt 60
   nttttttttt tttttttnnt tntttntntt ncnnanggnn canttnnttt nntnanaagg 120
   ggggnttngn naaggagcnc tnactncnaa nncnnnaana cnngnggggn ggggnttttn 180
   cntnttnaan anggnngncc cncncnctnn nacctaaccc naaatggcct ngggggnccc 240
   nccccccttt nnngagntgn anggggnggn tgnnaacnnn nngggggaaa gacnggnacn 300
   gggcaggggg gntnnnttna ctgnanacan nagcannnga ctaagaatga cnnaagacnn 360
   aacnacnnnc caaggctnna aaggggtnng taananngaa aacnaggggc aagggggcat 420
   anggggtgag gaantnaanc tttaggancg gggggacnaa aaacanngga ngnntgaacn 480
   nncttgacat ngtgntacnc ttangaacga ngganacccc tgnaactcac tngncacttt 540
   atatncntct cccccngggg ggggaatcct naaaaaancg cnttttnttn accccgngaa 600
   atanaaacac ccccccntnt tngaangngg annnttcaca gggnggngnt ttnnncntat 660
   nggccccaan agtttccttt ncttannana anacaaaann cntttttcaa ggccccnnnn 720
   ttnnnncncc gnggggnctt tttaangncc canaaggcna agggncatnt gccntnctat 780
   cangggnggg ggggggcntt tnccataata ancgccaccc nagggggggg ggncanaanc 840
   caccncnagg ngnggggggg gcttcnaca                                  869

## Description

### Technical Field

This invention relates to nucleic acids whose expression is enhanced in human neuroblastoma with favorable prognosis based on comparison between human neuroblastoma with favorable prognosis and human neuroblastoma with unfavorable prognosis.

### Background Art

### (Tumorgenesis and Genes)

Individual tumors exhibit distinct characteristic natures, and their biological properties are not necessarily identical even though the basic principle of oncogenesis is the same. Rapid advances in the understanding of cancer from a molecular biological and molecular genetic perspective in recent years have opened the way to an explanation of oncogenesis and tumor cell biology on the genetic level.

### (Neuroblastomas)

Neuroblastoma is a pediatric cancer occurring in sympathetic gangliocytes and adrenal medullary cells which originate from cells of the peripheral sympathetic nervous system. Of these sympathetic nervous system cells, neural crest cells in the initial stage of development migrate to the abdomen, differentiating and maturing at sites where sympathetic ganglia are formed. Some of these cells migrate further to the adrenal bodies, penetrating through the adrenal cortex which is already in the process of formation, and reaching the medulla and forming medullary substance there. The neural crest cells also serve as a source of other peripheral nerve cells, differentiating into dorsal root ganglia (sensory nerves), skin pigment cells, thyroid C cells, some pulmonary cells, intestinal gangliocytes, and the like.

### (Prognosis for neuroblastoma)

Neuroblastoma is characterized by a varied clinical profile (Nakagawara, Shinkeigashu no Hassei to Sono Bunshi Kiko [Neuroblastoma Development and Molecular Mechanism], Shoni Naika 30, 143, 1998). For example, neuroblastomas occurring at less than one year of age have very favorable prognosis, with the majority undergoing differentiation and cell death, and spontaneous regression. Currently, most neuroblastomas discovered by a positive result in the commonly performed mass screening of 6-month-old infant urine are of the type which tend to undergo this spontaneous regression. On the other hand, neuroblastomas occurring at age 1 or higher are highly malignant and lead to death of the infant in the majority of cases. It is also hypothesized that a somatic mutation occurs in highly malignant neuroblastomas in infants older than one year of age, which are of monoclonal nature, whereas in naturally regressing neuroblastomas, the genetic mutation remains at only a germline mutation. See Knudson AG, et al.: Regression of neuroblastoma IV-S: A genetic hypothesis, N. Engl. J. Med. 302, 1254 (1980)).

### (Genes which allow the diagnosis of prognosis for neuroblastoma)

With recent advances in molecular biology research, it has become clear that expression of the high affinity nerve growth factor (NGF) receptor TrkA is closely connected with control of differentiation and cell death. See Nakagawara A., The NGF story and neuroblastoma, Med. Pediatr. Oncol., 31, 113 (1998). Trk is a membrane-spanning receptor, existing as the three main types, Trk-A, -B and -C. These Trk family receptors play an important role in specific nerve cell differentiation and survival in the central nervous and peripheral nervous systems. See Nakagawara, et al., Shinkeigasaiboushu ni Okeru Neurotrophin Juyoutai no Hatsugen to Yogo [Expression of Neurotrophin Receptors and Prognosis in Neuroblastoma], Shoni Geka (Pediatric Surgery), 29:425-432, 1997. The survival and differentiation of tumor cells is controlled by signals from Trk tyrosine kinase and Ret tyrosine kinase. In particular, the role of TrkA receptor is most significant, with TrkA expression being notably high in neuroblastomas with favorable prognosis, and its signals exerting a powerful control over survival and differentiation of tumor cells, and cell death (apoptosis). In neuroblastomas with unfavorable prognosis, on the other hand, TrkA expression is significantly suppressed, while tumor development is aided by a mechanism in which survival is promoted by signals from TrkB and Ret.

It has become clear that amplification of the neural oncogene N-myc has become clearly associated with the prognosis of neuroblastoma. See Nakagawara, Nou-shinkeishuyo no Tadankai Hatsugan [Multistage Oncogenesis of Cerebral and Neural Tumors], Molecular Medicine, 364, 366(1999). This gene, first cloned in neuroblastoma, is ordinarily only present in a single copy per haploid set in normal cells and neuroblastomas with favorable prognosis, whereas it has been found to be amplified several dozen times in neuroblastomas with unfavorable prognosis.

Up till the present time, however, no oncogene other than N-myc is known to be expressed in neuroblastomas, and absolutely no genetic information other than that of N-myc has been known in relation to favorable or unfavorable prognosis.

### Disclosure of the Invention

This invention has been accomplished in light of the circumstances described above, and its object is to identify the gene sequences which are related to favorable or unfavorable prognosis of neuroblastoma, and to allow the provision of their genetic information as well as the diagnosis for favorable or unfavorable prognosis.

As a result of conducting diligent research, the present inventors have examined the prognoses of neuroblastomas and have succeeded in constructing cDNA libraries from both clinical tissues with favorable prognosis and with unfavorable prognosis. Approximately 2400 clones were respectively obtained from these two types of cDNA libraries and were classified according to the prognosis of neuroblastoma (whether favorable or unfavorable).

Moreover, the present inventors found that the expression of a considerable number of the genes is enhanced only in clinical tissues of neuroblastoma with favorable prognosis among the classified genes.

Based on such knowledge, the present inventors have succeeded in providing base sequence information for the detection and cloning of the genes only expressed in clinical tissues with favorable prognosis.

Furthermore, based on the aforementioned base sequence information on the regions it has been made possible to carry out the method for detection of prognosis and to design tumor markers which can be used therefor, and this invention has thereupon been completed.

Specifically, this invention aims at providing a nucleic acid whose expression is enhanced in human neuroblastoma with favorable prognosis based on comparison between human neuroblastoma with favorable prognosis and human neuroblastoma with unfavorable prognosis, the nucleic acid comprising any one of base sequences set forth in claim 1.

The nucleic acids of this invention are those whose expression is enhanced in human neuroblastoma with favorable prognosis based on comparison between human neuroblastoma with favorable prognosis and human neuroblastoma with unfavorable prognosis and those nucleic acids are characterized by their capabilities of being used in the diagnosis for prognosis of human neuroblastoma.

The nucleic acid is a nucleic acid comprising any one of base sequences set forth in SEQ ID NO:121, SEQ ID NO:125, SEQ ID NO:132, ID NO:165, SEQ ID NO:171, SEQ ID NO:187, SEQ ID NO:188, SEQ ID 229, SEQ ID NO:232, SEQ ID NO:273 and SEQ ID NO:281 in the Sequence Listing.

This invention also provides a diagnostic agent for the detection of a neurological disease characterized by containing at least one nucleic acid comprising any one of the base sequence set forth claim 1. Specifically, such tumor detection diagnostic agents include DNA chips and microarrays both of which are produced using the above-mentioned nucleic acids, for example. Accordingly, this invention also providers a microarray composition characterized by containing plural numbers of nucleic acids comprising the whole of the base sequence set forth in SEQ ID NO:1 to SEQ ID NO:366 in the Sequence Listing. Preferably, such composition comprises all the nucleic acids, i.e., a total of 366 nucleic acids each of which comprises a portion or the whole of a base sequence set forth in SEQ ID NO:1 to SEQ ID NO:366 in the Sequence Listing. More preferably, the composition is that which contains a total of 13 nucleic acids each of which comprises a portion or the whole of a base sequence set forth in SEQ ID NO:121, SEQ ID NO:125, SEQ ID NO:132, SEQ ID NO:133, SEQ ID NO:165, SEQ ID NO:171, SEQ ID NO:187, SEQ ID NO:188, SEQ ID NO:210, SEQ ID NO:229, SEQ ID NO:232, SEQ ID NO:273 and SEQ ID NO:281 in the Sequence Listing.

Further there is provided an isolated nucleic acid which is DNA characterized by hybridizing to the nucleic acid mentioned above or to its complementary nuclei acid under stringent conditions.

In addition, this invention provides a method of diagnosing the prognosis of human neuroblastoma, the method comprising detecting the presence or absence of a nucleic acid comprising any one of base sequences set forth in claim 1 in a clinical tissue sample of the neuroblastoma.

### Brief Description of the Drawings

Fig. 1 is a figure corresponding to an electrophoregram showing an example of the results of determination of the gene expression levels in human neuroblastomas with favorable prognosis and with unfavorable prognosis by semi-quantitative PCR.
Fig. 2 is a figure corresponding to electron micrographs showing a primary culture containing SCG neurons of a new born mouse.

### Best Mode for Carrying Out the Invention

The term "nucleic acid(s)" as used in this invention refers to, for example, DNA or RNA, or polynucleotides derived therefrom which are active as DNA or RNA, and preferably they are DNA or RNA.

The term "hybridize under stringent conditions" means that two nucleic acid fragments hybridize to each other under the hybridization conditions described by Sambrook, J. et al. in "Expression of cloned genes in E. coli", Molecular Cloning: A Laboratory Manual (1989), Cold Spring Harbor Laboratory Press, New York, USA, 9.47-9.62 and 11.45-11.61.

More specifically, the "stringent conditions" refers to hybridization at approximately 45 °C, 6.0 x SSC, followed by washing, at 50 °C, 2.0 x SSC. The stringency may be selected by choosing a salt concentration in the washing step from approximately 2.0 x SSC, 50 °C as low stringency to approximately 0.2 x SSC, 50 °C as high stringency. Also, the temperature in the washing step may be increased from room temperature, or approximately 22 °C as low stringency conditions, to approximately 65 °C as high stringency conditions.

The term "isolated nucleic acid(s)" as used in the present specification refers to a nucleic acid or a polynucleotide containing substantially no cellular substances or culture medium, if prepared by recombinant DNA techniques, or containing substantially no precursor chemical substances or other chemical substances, if prepared by chemical synthesis.

The term "favorable prognosis" as used in the present specification refers to a condition of human neuroblastoma in which the tumor is localized or has become a regressing or benign sympathetic ganglion neoplasm, and is judged to have low malignancy based on N-myc or other tumor markers. According to a preferred embodiment of the invention, a favorable prognosis is a case of stage 1 or 2, with an onset age of less than one year and survival without recurrence for 5 or more years after surgery, and with no noted amplification of N-myc in the clinical tissue; however, there is no limitation to such specific cases. The term "unfavorable prognosis" as used in the present specification refers to a condition of human neuroblastoma in which progression of the tumor has been observed, and it is judged to have high malignancy based on N-myc or other tumor markers. According to a preferred embodiment of the invention, an unfavorable prognosis is a case of stage 4, with an onset age of greater than one year, death within 3 years after surgery and noted amplification of N-myc in the clinical tissue; however, there is no limitation to such specific cases.

The nucleic acids of this invention have been found in the clinical tissues of human neuroblastoma and such nucleic acids have the characteristics described below.

Neuroblastoma is a tumor consisting of actual nerve cells, of which only two types of tumor are known in humans, and analysis of the genes expressed therein is expected to provide very useful knowledge for understanding the biology of nerve cells. Specifically, it is extremely difficult, and practically impossible, to obtain site-specific homogeneous tissue from the brain or peripheral nerves. On the other hand, a neuroblastoma consists of an almost homogeneous nerve cell population (though tumorized) derived from peripheral sympathetic nerve cells, and thus offers a high possibility of obtaining homogeneous expression of neuro-related genes. Furthermore, since neuroblastoma is a type of cancer, it will characteristically have many important genes expressed in the immature stage of neurogenesis.

Clinically and biologically, neuroblastoma can be neatly classified into favorable prognosis and unfavorable prognosis types. Cancer cells from neuroblastoma with favorable prognosis are characterized by having a very slow rate of proliferation, with spontaneous regression beginning at some point. Findings to date have confirmed that nerve cell differentiation and apoptosis (nerve cell death) occur in the spontaneous regression, and that the differentiation which occurs in the maturation stages of normal nerve cells and programmed cell death are phenomena very closely resembling each other. Consequently, it is highly probable that the analysis of genes expressed in such tumors will lead to obtaining important information relating to nerve cell differentiation and apoptosis.

Neuroblastomas with unfavorable prognosis are tumors consisting of cancer cells which continue to exhibit definitely malignant proliferation. The probability is very high, therefore, that they have a large number of important genes connected with nerve cell proliferation or genes expressed in undifferentiated nerve cells. In other words, it is highly probable that these will allow the obtainment of genetic information completely different from the profile of genes expressed in neuroblastomas with favorable prognosis.

It is commonly reported that nerve cells contain more expressed gene types than cells derived from other organs. Neuroblastoma cell lines are derived from clinical tissues with unfavorable prognosis, and it is believed that the gene expression profile in the case of tumor development and progression is substantially altered from that of normal nerve cells.

Neuroblastoma is characteristically a pediatric tumor, and because of the very low possibility of effects by acquired factors, it is expected that analysis of the mechanism of cancerization will also yield embryological information with high probability. More surprisingly, the genes or the gene fragments according to this invention include genes whose expression is enhanced only in specific cell cycle phases, and this further suggests the very strong possibility of obtaining genetic information highly useful for the analysis of cancerization mechanisms and related to development and differentiation.

The nucleic acids, having the characteristics mentioned above and from which the aforementioned information can be obtained, are available from human neuroblastoma clinical tissues and have any of the base sequences set forth in SEQ ID NO:1 to NO:366 in the Sequence Listing, or a portion thereof.

As a result of comparing gene expression levels in clinical tissues from human neuroblastomas with favorable prognosis and with unfavorable prognosis, a highly significant difference was found in each of the nucleic acids having base sequences set forth in SEQ ID NO:1 to NO:366 in the Sequence Listing. That is, the expression of these nucleic acids was enhanced in human neuroblastomas with favorable prognosis. Thus, in addition to providing the useful genetic information described above, the base sequences set forth in SEQ ID NO:1 to NO:366 can also be utilized as data for tumor markers to diagnose favorable or unfavorable prognosis of neuroblastoma, by detecting DNA and/or RNA having any of these base sequences.

Specifically, this invention will make it possible to obtain various forms of diagnosis for prognosis on or relating to human neuroblastoma through the following means.

### (1) Probes for hybridization

The nucleic acid comprising the whole of a base sequence disclosed in claim 1 (which may be referred to as "nucleic acid(s) of the invention") may be at least used as a probe for hybridization in order to detect genes expressed in human neuroblastoma. The nucleic acids of the invention may also be used as probes for hybridization in order to determine gene expression in various tumors and normal tissues, to identify the distribution of the gene expression.

When the nucleic acid comprising the whole of a base sequence disclosed in this invention is used as a probe for hybridization, there are no particular imitations on the actual method of hybridization. As preferred methods there may be mentioned, for example, Northern hybridization, Southern hybridization, colony hybridization, dot hybridization, fluorescence in situ hybridization (FISH), in situ hybridization (ISH), DNA chip methods, and microarray methods.

As one application example of the hybridization, the nucleic acid of this invention may be used as a probe for Northern hybridization to measure the length of mRNA or to quantitatively detect gene expression in an assayed sample.

When the nucleic acid of the invention is used as a probe for Southern hybridization, it enables the detection of the presence or absence of the base sequence in genomic DNA of an assayed sample.

The nucleic acid comprising the whole of a base sequence disclosed in this invention can also be used as a probe for fluorescence in situ hybridization (FISH) to identify the location of the gene on a chromosome.

The nucleic acid of the invention can also be used as a probe for in situ hybridization (ISH) to identify the tissue distribution of gene expression.

When the nucleic acid of the invention is used as a probe for hybridization, a nucleic acid residue length of at least 40 is necessary; and among the gene sequences according to the invention, a nucleic acid having 40 or more contiguous residues is preferably used. More preferably, one having 60 or more nucleic acid residues is used.

Nucleic acid probe techniques are well known to one skilled in the art, and for example, conditions suitable for hybridization between a probe of specific length according to the invention and the target polynucleotide may be readily determined. In order to obtain hybridization conditions optimal to probes of various lengths, Sambrook et al. "Molecular Cloning: A Laboratory Manual, 2nd Edition, Cold Spring Harbor (1989) may be followed for such manipulations which are well known to one skilled in the art.

The probe is preferably labeled in an easily detectable fashion. The detectable label may be any type or portion which can be detected either visually or using devices. As commonly used detectable labels there may be mentioned radioactive isotopes such as ³²P, ¹⁴C, ¹²⁵I, ³H and ³⁵S_{.} Biotin-labeled nucleotides may be incorporated into DNA or RNA by nick translation, chemical or enzymatic means. The biotin-labeled probes are detected after hybridization using labeling means such as avidin/streptavidin, fluorescent labels, enzymes, gold colloidal complexes or the like. The nucleic acid may also be labeled by binding with a protein. Nucleic acid cross-linked to a radioactive or fluorescent histone single-stranded DNA binding protein may also be used.

### (2) Primers for use in PCR

For other possible methods of detecting genes, DNA that is the nucleic acid comprising a portion or the whole of a base sequence disclosed in this application can be used as a primer in a polymerase chain reaction (PCR). For example, RNA may be extracted from a sample to be assayed, and the gene expression can be semi-quantitatively measured by RT-PCR. This may be carried out by a method well known to one skilled in the art. For example, "Molecular Cloning: A Laboratory Manual," (T. Maniatis, Cold Spring Harbor Laboratory Press) or Idenshibyo Nyumon [Introduction to Genetic Diseases] (Takahisa, S.: Nankodo Publishing) may be followed.

When the DNA capable of hybridizing to the nucleic acid of the invention or a complementaly DNA thereof is used as a PCR primer, a base length of 10 to 60 is necessary; and among the gene sequences according to the invention, the nucleic acid having 10 to 60 contiguous bases is preferably used. More preferably, one having 15 to 30 bases is used. Generally, a primer sequence with a GC content of 40-60% is preferred. Also, there is preferably no difference in the Tm values of the two primers used for amplification. Preferably there is no annealing at the 3' ends of the primers and no secondary structure is formed in the primers.

### (3) Nucleic acid screening

The nucleic acid comprising the whole of a base sequence disclosed in this invention can also be used to detect the expression distribution of a gene which is expressed in various tissues or cells. This can be accomplished, for example, by using the nucleic acid of the invention as a probe for hybridization or as a primer for PCR.

Expression distribution of a gene can also be detected using a DNA chip, microarray or the like. That is, the nucleic acid of the invention may be directly attached to the chip or array. RNA extracted from cells of a tissue may be labeled there with a fluorescent substance or the like, hybridized, and an analysis can be made of the type of tissue cells with high expression of the gene. The DNA attached to the chip or array may be the reaction product of PCR using the nucleic acid of the invention. United State Patent No. 5605662 to Heller et al. describes an example of the method for attaching nucleic acids to a chip or array.

By utilizing the aforementioned technology, it is possible to use as a diagnostic agent, at leat one among the nucleic acids comprising the whole of base sequences disclosed in this invention. In recent years, it has becoming clear that the genetic information possessed by individuals governs their susceptibility to a certain disease as well as governs the effectiveness of a particular drug. The DNA chips or microarrays produced using the aforementioned nucleic acids are used to clarify the cause-effect relationship between the disease of a subject and the nucleic acid, which will enable not only the diagnosis of that disease but also the selection of a drug to be administered. Specifically, if the result detected using the DNA chip or microarray is employed as an indicator of selection of a drug to be administered, the expression level of one nucleic acid among the nucleic acids of the invention can be examined but also the expression levels of two or more nucleic acids can be comparatively examined to select the drug to be administered, which will then enable more accurate judgment. As used herein, the disease is not particularly limited insofar as it is that which can be diagnosed by the nucleic acids of this invention. Preferably, the disease is a neurological disease and more preferably, neuroblastoma.

### (9) DNA cloning

The nucleic acid comprising the whole of a base sequence disclosed in this invention can be at least used for cloning a gene which is expressed in human neuroblastoma. For example, by using the nucleic acid of the invention as a probe for Northern hybridization or Southern hybridization, or as a primer for PCR, cloning of a gene containing the nucleic acid comprising a portion or the whole of a base sequence disclosed in this invention is possible. As the genes capable of being subjected to cloning there may be mentioned genes with differing levels of expression particularly between neuroblastoma with favorable prognosis and neuroblastoma with unfavorable prognosis, genes whose forms of expression differ in other tissues or cancer cells, genes whose expression is cell cycle phase-dependent, genes induced upon neurodifferentiation and genes whose expression is regulated by oncogenes or tumor suppressor genes.

### (5) Methods of diagnosing tumor prognosis and tumor markers to be used therefor

The nucleic acid comprising the whole of a base sequence disclosed in this invention can be used as a probe for hybridization, or as a primer for PCR to determine the presence or absence of enhancement in the gene expression in sample cells, which enables the identification of prognosis. To determine the presence or absence of enhancement in the gene expression, any method that utilizes nucleic acids (probes) capable of hybridizing to the nucleic acid comprising any given sequence of the base sequence disclosed in this invention is provided for use. Prognosis can be diagnosed as favorable if the amount of nucleic acid hybridizing to the probe is increased in the sample cell. When the nucleic acid is used as a primer for PCR, RNA is extracted from the sample to be assayed and the gene expression can be semi-quantitatively measured by the RT-PCR method.

### (6) Antisense oligonucleotides

There are provided antisense oligonucleotides having the base sequences according to the invention. As will be considered in practicing this invention, the antisense oligonucleotides may readily be prepared such that they can bind to RNA corresponding to the base sequence of this invention and can thereby inhibit the synthesis of RNA.

### (7) Gene therapy

According to a further embodiment of this invention, there are provided therapeutic genes to be used in gene therapy. As will be considered in practicing this invention, the gene according to this invention may be transferred into a vector for use in gene transportation, whereby the transgene can be expressed by an arbitrary expression promoter and can be used for the gene therapy of cancers, for example.

### 1. Vectors

The transferable viral vectors may be prepared from DNA viruses or RNA viruses. They may be any viral vector of an MoMLV vector, a herpes virus vector, an Adenovirus vector, an AAV vector, a HIV vector, a Seidai virus vector and the like. One or more proteins among the constituent protein group of a viral vector are substituted by the constituent proteins of a different species of virus, or alternatively a part of the base sequence constituting genetic information is substituted by the base sequence of a different species of virus to form a viral vector of the pseudo-type which can also be used in this invention. For example, there is mentioned a pseudo-type viral vector wherein the Env protein (an envelop protein of HIV) is substituted by the VSV-G protein (an envelop protein of vesicular stomatitis virus or VSV) (Naldini L., et al., Science 272, 263-267, 1996). Further, virues having a host spectrum other than human is usable as the viral vector insofar as they are efficacious. As for the vectors other than those of viral origin, there may be used complexes of calcium phosphate and nucleic acid, ribosomes, cation-lipid complexes, Seidai virus liposomes, polymer carriers having polycation as the main chain and others. In addition, methods such as electroporation and gene guns may be used as a gene transfer system.

### 2. Expression promoters

As for the expression cassettes to be used for the therapeutic gene, any cassettes without any particular limitations may be used insofar as they can cause genes to express in the target cells. One skilled in the art can readily select such expression cassettes. Preferably, they are expression cassettes capable of gene expression in the cells derived from an animal, more preferably, expression cassettes capable of gene expression in the cells derived from a mammal, and most preferably expression cassettes capable of gene expression in the cells derived from a human. The gene promoters that can be used as expression cassettes include: for example, virus-derived promoters from an Adenovirus, a cytomegalovirus, a human immunodeficiency virus, a simian virus 40, a Rous sarcoma virus, a herpes simplex virus, a murine leukemia virus, a sinbis virus, a hepatitis type A virus, a hepatitis type B virus, a hepatitis type C virus, a papilloma virus, a human T cell leukemia virus, an influenza virus, a Japanese encephalitis virus, a JC virus, parbovirus B19, a poliovirus, and the like; mammal-derived promoters such as albumin, SRα, a heat shock protein, and an elongation factor; chimera type promoters such as a CAG promoter; and the promoters whose expression can be induced by tetracyclines, steroids and the like.

### EXAMPLES

This invention will now be explained in greater detail by way of the examples and production examples; however, the invention will not be restricted to those example.

### (Production Example 1) Construction of cDNA library from human neuroblastoma

### 1. Obtaining samples

Human neuroblastoma clinical tissue specimens were quasi-aseptically frozen immediately after surgical extraction and then preserved at -80°C.

### 2. Selecting samples with favorable prognosis

Prognosis of the samples obtained in 1. above was carried out based on the following criteria.

Favorable prognosis:
- Stage 1 or 2
- Age of onset less than one year
- Survival for longer than 5 years after surgery without recurrence
- No amplification of N-myc

Unfavorable prognosis:
- Stage 4
- Age of onset older than 4 years
- Death within 3 years after surgery
- Amplification of N-myc

Amplification of N-myc in the aforementioned sample types was confirmed in the following manner.

The clinical tissue sample obtained in 1. above was thinly sliced with a scalpel and then thoroughly homogenized after addition of 5 ml of TEN buffer (50 mM Tris-HCl (pH = 8.0)/1 mM EDTA/100 mM NaCl). Upon adding 750 µl of SDS (10%) and 125 µl of proteinase K (20 mg/ml) to the mixture, it was gently stirred and allowed to stand at 50 °C for 8 hours. This was followed by phenol/chloroform treatment and finally ethanol precipitation to obtain purified genomic DNA. A 5 µg portion of the obtained genomic DNA was completely digested with the restriction endonuclease EcoRI (NEB Inc.), and an N-myc probe was used to determine amplification of N-myc by Southern hybridization.

### 3. Preparation of mRNA from clinical tissue of human neuroblastoma with favorable prognosis

A 2-3 g portion of the clinical tissue sample of human neuroblastoma judged to have favorable prognosis in 2. above was treated using a Total RNA Extraction Kit (QIAGEN Inc.) and the total RNA was extracted. The extracted total RNA was purified using an oligo dT cellulose column (Collaborative Research, Inc.) to obtain a pool of mRNA with a polyA structure.

### 9. Dephosphorylation of mRNA

A 100-200 µg portion of the mRNA pool prepared in 3. above was dissolved in 67.3 µl of distilled sterile water containing 0.1% diethyl pyrocarbonate (DEPC), and then 20 µl of 5XBAP buffer (Tris-HCl (500 mM, pH = 7.0)/mercaptoethanol (50 mM)), 2.7 µl of RNasin (40 unit/µl: Promega Inc.) and 10 µl of BAP (0.25 unit/µl, bacteria-derived alkali phosphatase: Takara Shuzo Co. Ltd.) were added. The mixture was reacted at 37 °C for 1 hour to effect dephosphorylation of the 5' end of the mRNA. This was followed by phenol/chloroform treatment two times, and finally ethanol precipitation to obtain a purified dephosphorylated mRNA pool.

### 5. Decapping of dephosphorylated mRNA

The total amount of the dephosphorylated mRNA pool prepared in 4. above was dissolved in 75.3 µl of distilled sterile water containing 0.1% DEPC, and then 20 µl of 5XTAP buffer (sodium acetate (250 mM, pH = 5.5)/mercaptoethanol (50 mM), EDTA (5 mM, pH = 8.0)), 2.7 µl of RNasin (40 unit/µl) and 2 µl of TAP (tobacco acid pyrophosphatase: 20 unit/µl) were added. The mixture was reacted at 37 °C for 1 hour to effect decapping treatment of the 5' end of the dephosphorylated mRNA. The dephosphorylated mRNA of incomplete length with no capped structure remained without decapping, and with the 5' end dephosphorylated. This was followed by phenol/chloroform treatment and ethanol precipitation to obtain a purified decapped mRNA pool.

### 6. Preparation of oligo-capped mRNA

The total amount of the decapped mRNA pool prepared in 5. above was dissolved in 11 µl of distilled sterile water containing 0.1% DEPC, and then 4 µl of 5'-oligo RNA (5'-AGCAUCGAGUCGGCCUUGGCCUACUGG-3': 100 ng/µl), 10 µl of 1OX ligation buffer (Tris-HCl (500 mM, pH = 7.0)/mercaptoethanol (100 mM)), 10 µl of magnesium chloride (50 mM), 2.5 µl of ATP (24 mM), 2.5 µl of RNasin (40 unit/µl), 10 µl of T4 RNA ligase (25 unit/µl: Takara Shuzo Co. Ltd.) and 50 µl of polyethylene glycol (50% w/v, PEG8000: Sigma Corporation) were added. The mixture was reacted at 20 °C for 3 hours for ligation of the 5'-oligo RNA to the 5' end of the decapped mRNA. The dephosphorylated mRNA of incomplete length with no capped structure resulted in no ligation to the 5'-oligo RNA. This was followed by phenol/chloroform treatment and ethanol precipitation to obtain a purified oligo-capped mRNA pool.

### 7. Removal of DNA from oligo-capped mRNA

The oligo-capped mRNA pool prepared in 6. above was dissolved in 70.3 µl of distilled sterile water containing 0.1% DEPC, and then 4 µl of Tris-HCl (1 M, pH = 7.0), 5.0 µl of DTT (0. 1 M), 16 µl of magnesium chloride (50 mM), 2.7 µl of RNasin (40 unit/µl) and 2 µl of DNaseI (5 unit/µl: Takara Shuzo Co. Ltd.) were added. The mixture was reacted at 37 °C for 10 minutes to dissolve the excess DNA. This was followed by phenol/chloroform treatment and ethanol precipitation and column purification (S-400HR: Pharmacia Biotech Inc.), to obtain a purified DNA(-) oligo-capped mRNA pool.

### 8. Preparation of 1st strand cDNA

The DNA(-) oligo-capped mRNA pool prepared in 7. above was reverse transcribed using SuperScript II (kit by Life Tech Oriental, Inc.) to obtain a pool of 1st strand cDNA. The pool of DNA(-) oligo-capped mRNA was dissolved in 21 µl of sterile distilled water, and then 10 µl of 1OX First Strand buffer (kit accessory), 8 µl of dNTP mix (5 mM, kit accessory), 6 µl of DTT (0.1 M, kit accessory), 2.5 µl of oligo-dT adapter primer (5 pmol/µl, 5'-GCGGCTGAAGACGGCCTATGTGGCC TTTTTTTTTTTTTTTTT-3'), 2.0 µl of RNasin (40 unit/µl) and 2 µl of SuperScript II RTase (kit accessory) were added. The mixture was reacted at 42 °C for 3 hours to effect reverse transcription. This was followed by phenol/chloroform treatment, alkali treatment and neutralization treatment to dissolve all the RNA and purification was carried out by ethanol precipitation.

### 9. Preparation of 2nd strand cDNA

The 1st strand cDNA pool prepared in 8. above was subjected to PCR amplification using Gene Amp (kit by Perkin Elmer Inc.). The pool of 1st strand cDNA was dissolved in 52.4 µl of sterile distilled water, and then 30 µl of 3.3X Reaction buffer (kit accessory), 8 µl of dNTP mix (2.5 mM, kit accessory), 4.4 µl of magnesium acetate (25 mM, kit accessory), 1.6 µl of Primer F (10 pmol/µl, 5'-AGCATCGAGTCGGCCTTGTTG-3'), 1.6 µl of Primer R (10 pmol/µl, 5' -GCGCTGAAGACGGCCTATGT-3') and 2 µl of rTth (kit accessory) were added. A 100 µl portion of mineral oil was gently added to the mixture and overlayed thereon. After denaturing the reaction solution at 94 °C for 5 minutes, a cycle of 94 °C for 1 minute, 52 °C for 1 minute and 72 °C for 10 minutes was repeated 12 times, and then the solution was allowed to stand at 72 °C for 10 minutes to complete the PCR reaction. This was followed by phenol/chloroform treatment and ethanol precipitation to obtain a 2nd strand cDNA pool.

### 10. SfiI treatment of 2nd strand cDNA

The 2nd strand cDNA pool prepared in 9. above was dissolved in 87 µl of sterile distilled water, and then 10XNEB buffer (NEB Inc.), 100XBSA (bovine serum albumin available from NEB Inc.) and 2 µl of SfiI (restriction endonuclease, 20 unit/µl, NEB Inc.) were added. The mixture was reacted overnight at 50 °C to effect SfiI restriction endonuclease treatment. This was followed by phenol/chloroform treatment and ethanol precipitation to obtain a pool of cDNA which had been SfiI-treated at both ends.

### 11. Size fractionation of SfiI-treated cDNA

The Sfil-treated cDNA pool prepared in 10. above was electrophoresed on 1% agarose gel and a fraction with >2 kb was purified using Geneclean II (Bio101 Inc.). The purified cDNA pool was dissolved in 100 µl of sterile distilled water and allowed to stand at 37 °C for 6 hours. This was followed by phenol/chloroform treatment and ethanol precipitation to obtain a long-chain cDNA pool.

### 12. Preparation of cDNA library

The long-chain cDNA pool prepared in 11. above was ligated into the cloning vector pME18S-FL3 (provided by Prof. Sumio Kanno of the Institute of Medical Science, Tokyo University) using a DNA Ligation Kit ver.1 (kit by Takara Shuzo Co. Ltd.). The long-chain cDNA pool was dissolved in 8 µl of sterile distilled water, and then 1 µl of pME18S-FL3 pretreated with restriction endonuclease DraIII, 80 µl of Solution A (kit accessory) and 10 µl of Solution B (kit accessory) were added and reaction was conducted at 16 °C for 3 hours. This was followed by phenol/chloroform treatment and ethanol precipitation for purification to obtain a cDNA library.

### (Example 1) Transformation into E. coli

The cDNA library prepared in Production Example 1 above was used for transformation into *E. coli* (TOP-10: Invitrogen Corporation). The cDNA library was dissolved in 10 µl of sterile distilled water and mixed with TOP-10. The mixture was then incubated on ice for 30 minutes, at 40 °C for 1 minute and on ice for 5 minutes. After adding 500 µl of SOB medium, shake culturing was performed at 37 °C for 60 minutes. Appropriate amounts thereof were seeded onto ampicillin-containing agar media and culturing was continued at 37 °C for a day and a night to obtain *E. coli* clones.

The *E. coli* clones on agar media obtained were collected with toothpick and suspended in 120 µl of LB medium prepared in a 96-well plate. The 96-well plate was then allowed to stand overnight at 37 °C for culturing of the *E. coli.* A 72 µl portion of 60% glycerol solution was then added and preserved at - 20 °C (glycerol stock).

### (Example 2) Base sequence determination

### 1. Preparation of plasmid

The 10 µl of glycerol stock prepared in Example 1 was transferred to a 15 ml centrifugation tube, and then 3 ml of LB medium and 50 µg/ml of ampicillin were added and shaking was carried out overnight at 37 °C for culturing of the *E. coli.* A QIAprep Spin Miniprep Kit (QIAGEN Inc.) was then used to extract and purify a plasmid DNA from the E. *coli.*

### 2. Analysis of both end sequences

Both end sequences of the plasmid DNA prepared in 1. above were determined using a DNA Sequencing Kit (kit by ABI). There were combined 600 ng of plasmid DNA, 8 µl of premix (kit accessory) and 3.2 pmol of primers, and sterile distilled water was added to a total of 20 µl. After denaturing the mixture at 96 °C for 2 minutes, a cycle of 96 °C for 10 seconds, 50 °C for 5 seconds and 60 °C for 4 minutes was repeated 25 times for reaction. The product was then purified by ethanol precipitation. Sequencing was carried out by polyacrylamide gel electrophoresis under denaturing conditions, using ABI377 (ABI).

### (Example 3) Homology search using database

An Internet-mediated DNA sequence homology search was conducted for the samples on which the both end-sequences were analyzed in Example 2. The homology search was conducted using the BLAST of the NCBI (National Center of Biotechnology Information, USA).

### (Example 4) Determination of gene expression levels in human neuroblastomas with favorable prognosis and unfavorable prognosis by semi-quantitative PCR

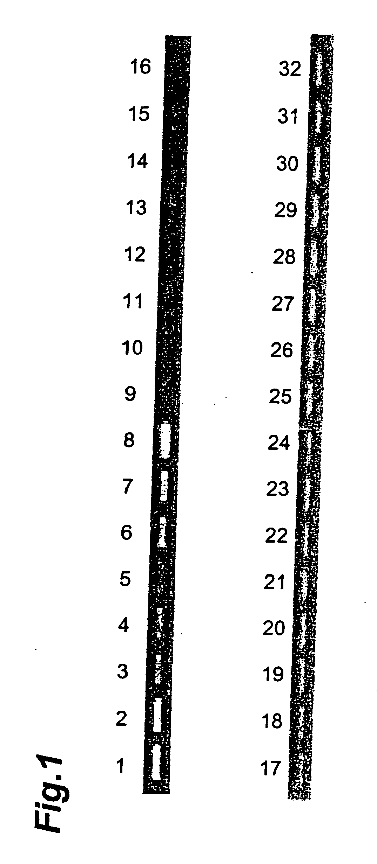

PCR primers were synthesized from portions of the genes obtained in Example 3, and the expression levels in the clinical tissues of human neuroblastomas with favorable prognosis and unfavorable prognosis were comparatively quantitated. mRNA was extracted from the human neuroblastoma clinical tissues by the method described in Examples 3, and rTaq (Takara Shuzo Co. Ltd.) was used for PCR reaction. Specifically, 5 µl of sterile distilled water, 2 µl of mRNA, 1 µl of 1OXrTaq buffer, 1 µl of 2 mM dNTPs, 0.5 µl each of the synthesized primer set and 0.5 µl of rTaq were combined. After denaturing the mixture at 95 °C for 2 minutes, a cycle of 95 °C for 15 seconds, 55 °C for 15 seconds and 72 °C for 20 seconds was repeated 35 times, and then the mixture was allowed to stand at 72 °C for 6 minutes for PCR reaction. The reaction solution was subjected to 1% agarose gel electrophoresis. One example of the results of determination of the gene expression levels in human neuroblastomas with favorable prognosis and unfavorable prognosis by semi-quantitative PCR is shown in Fig. 1. Here, the explanations of the respective lanes are as follows:
Lanes 1-8: expression of nbla-4183 (SEQ ID NO:154, SEQ ID NO:155 in the Sequence Listing) in a clinical sample of neuroblastoma with favorable prognosis;
Lanes 9-16: expression of nbla-4183 (SEQ ID NO:154, SEQ ID NO:155 in the Sequence Listing) in a clinical sample of neuroblastoma with unfavorable prognosis;
Lanes 17-24: expression of GAPDH in a clinical sample of neuroblastoma with favorable prognosis; and
Lanes 25-32: expression of GAPDH in a clinical sample of neuroblastoma with unfavorable prognosis.

Consequently, nucleic acids whose expression is enhanced only in human neuroblastoma with favorable prognosis have been identified in the base sequences set forth in SEQ ID NO:1 to SEQ ID NO:366.

### (Example 5) Gene group with varying gene expression levels by NGF regulated apoptosis in murine superior cervical gangliocytes.

It is known that NGF (nerve growth factor) exerts protective action against apoptosis in human neuroblastoma induced by an antimitotic agent (such as neocarzinostatin). (Cortazzo MH, J. Neurosci, 16, 3895-3899 (1996)). This action is believed to be due to the inhibition of apoptosis by the binding of NGF to p75. It has also been pointed out that the regression of neuroblastoma (causing favorable prognosis) at clinical stage is connected to the apoptosis of the tumor cells.

In order to identify the apoptosis-associated genes in neuroblastoma, the gene group with varying gene expression levels by NGF regulated apoptosis in murine superior cervical gangliocytes was examined.

Superior cervical gangliocytes (SCG neurons) were isolated from C57BL/6J mice (0 to 1 day of age) in 7 to 10 animals. Seven wells of cells were arranged to be grown on a 24-well cell culture plate at about 1 to about 2x10⁴ cells per well. RNA was further collected from about 1 to about 2x10⁴ cells (Day 1 as control).

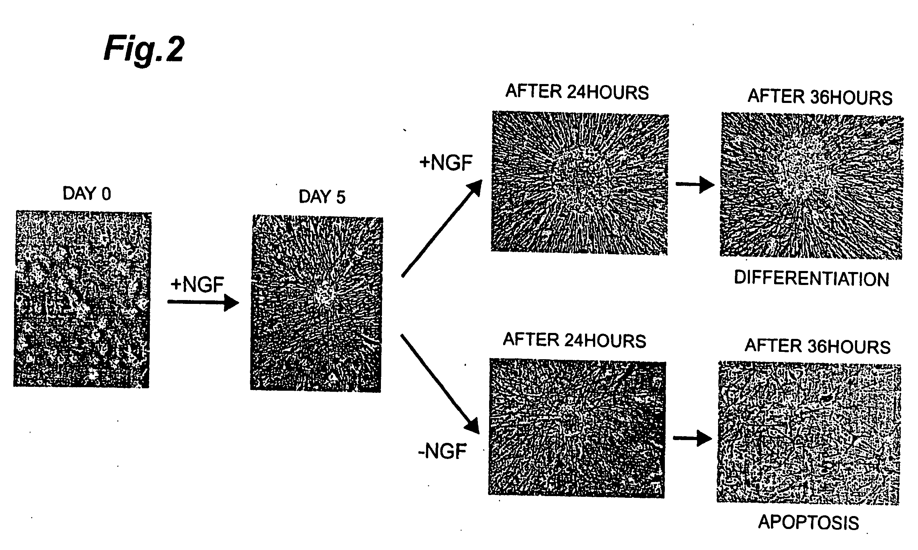

After the culture obtained by adding NGF (5 mg/ml) to the 7-well cells was incubated for. 5 days, RNA was collected from one-well cells (+NGF; after 5 days). Cultures in three wells each of the 6 wells were exchanged with cultures with addition of NGF (5 mg/ml) or anti-NGF antibodies (1%), and they were respectively incubated for 12 hours, 24 hours and 48 hours. Using portions of the samples, cells were examined under an electron microscope to identify the morphological differences between the case where NGF had been added and the case where anti-NGF antibodies had been added. The results are shown in Fig. 2. NGF induced differentiation was observed in the cells with NGF addition, whereas apoptosis was observed in the cells with anti-NGF antibody addition. As a result of NGF depletion by the anti-NGF antibodies, it was apparent that apoptosis was induced. RNA was collected from the respective well cells after 12 hr, 24 hr, and 48 hr culturing. These samples were denoted +NGF 12 hrs, +NGF 24 hrs, +NGF 48 hrs, -NGF 12 hrs, -NGF 24 hrs, and -NGF 48 hrs.

As described above, RNAs of the respectively treated cells from approximately 160 mice were concentrated by ethanol precipitation and cDNAs were synthesized from 1 µg RNAs using reverse transcriptase. These cDNAs were used as templates and gene expression levels were compared by PCR. The primers used were constructed from the homologous genes in mice based on the genes found in human neuroblastoma cells. The base sequences of the primers used for the comparison of expression are summarized in Table 1 together with the gene identification numbers (ID) for the targeted genes for comparison.

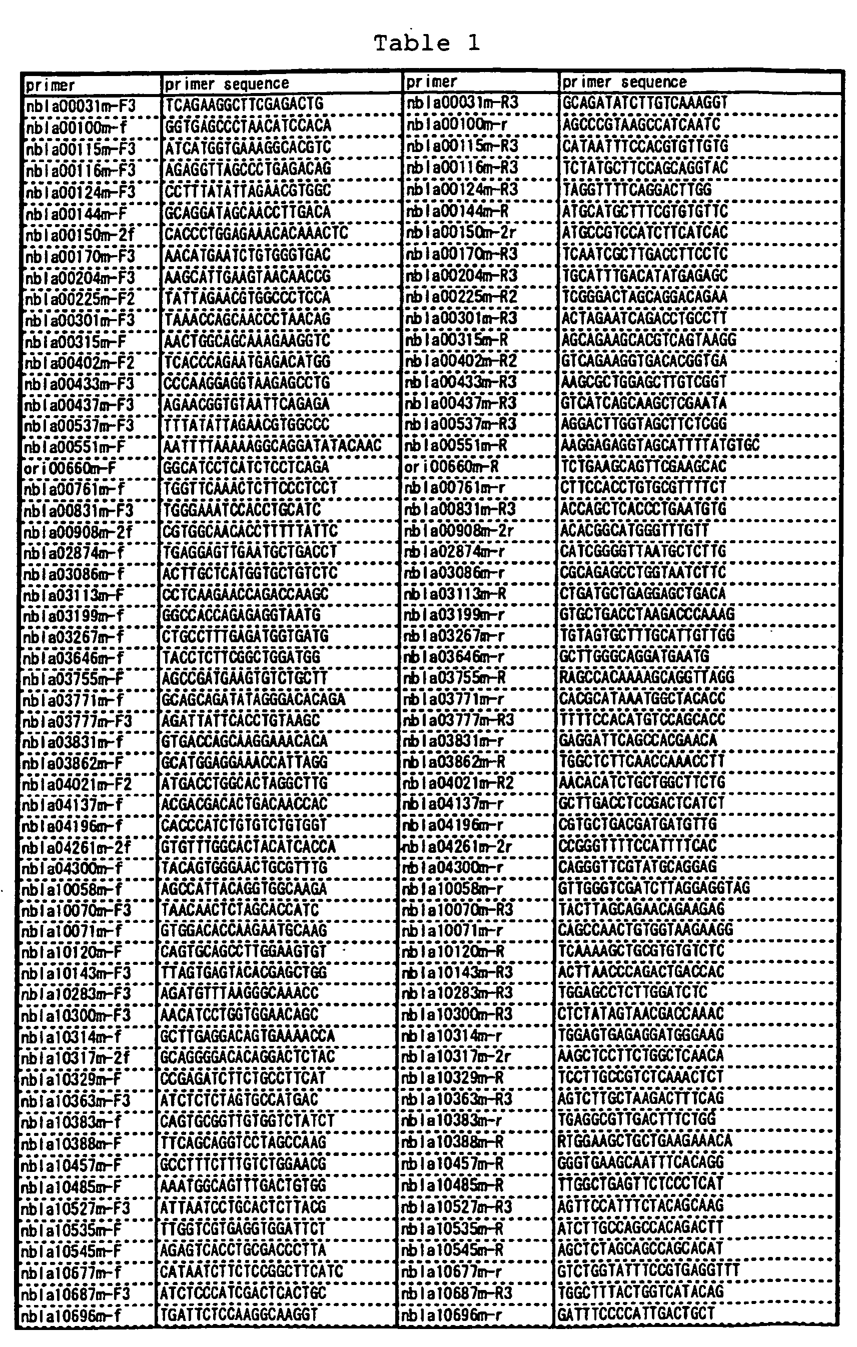

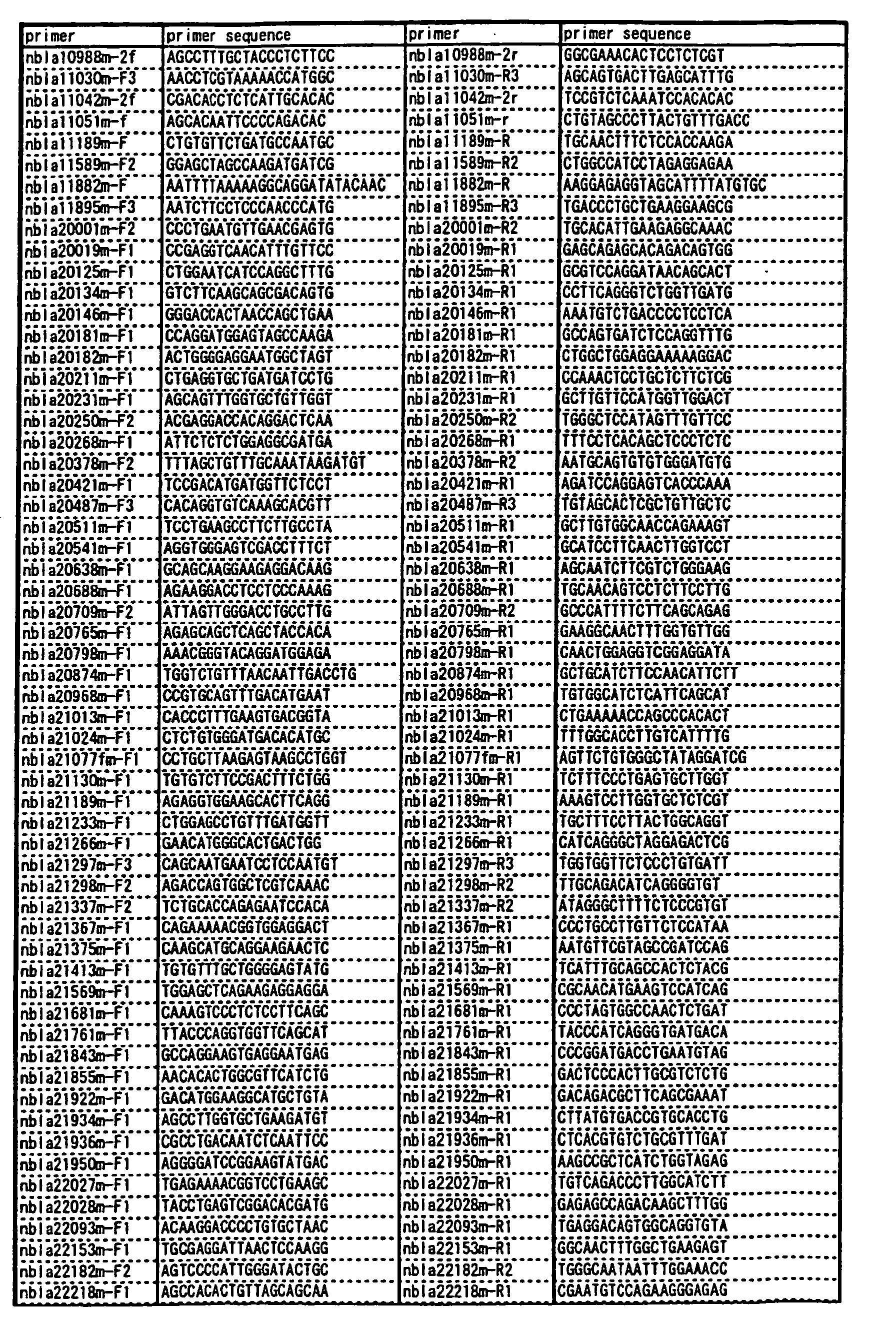

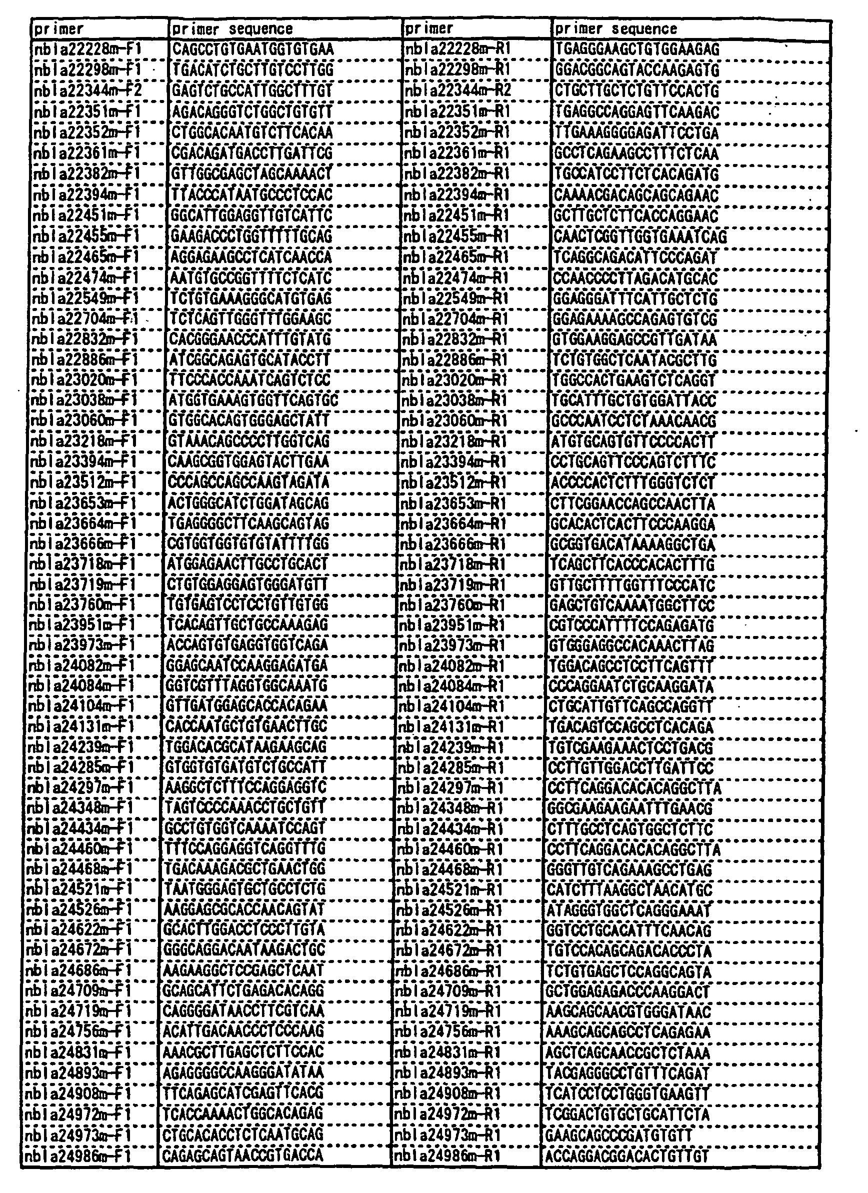

The results described below were obtained with the genes for which variations in the gene expression level were observed. The genes whose expression levels were decreased notably by NGF depletion (-NGF) were nbla-03646 (SEQ ID NO:121)(-NGF 12 hrs), nbla-03771 (SEQ ID NO:125)(-NGF 12 hrs), nbla-03831 (SEQ ID NO:132)(-NGF 24 hrs), nbla-04300 (SEQ ID NO:165)(-NGF 24 hrs), nbla-10120 (SEQ ID NO:171)(-NGF 48 hrs), nbla-10329 (SEQ ID NO:187)(-NGF 24 hrs), nbla-10363 (SEQ ID NO:188)(-NGF 12 hrs), nbla-10677 (SEQ ID NO:229)(-NGF 12 hrs), nbla-10696 (SEQ ID NO:232)(-NGF 12 hrs), nbla-11051 (SEQ ID NO:273) (-NGF 24 hrs), and nbla-11189 (SEQ ID NO:281)(-NGF 24 hrs). The genes whose expression levels were increased notably by NGF depletion (-NGF) was noted were nbla-03862 (SEQ ID NO:133)(-NGF 24 hrs) and nbla-10485 (SEQ ID NO:210)(-NGF 24 hrs). The gene whose expression level was increased notably by NGF addition was noted was nbla-10143 (SEQ ID NO:174)(+NGF).

The primer sets used to amplify those particular genes are shown in Table 2.

**Table 2**

| primer | primer sequence | primer | primer sequence |
|---|---|---|---|
| nbla03646m-f | TACCTCTTCGGCTGGATGG | nbla03646m-r | GCTTGGGCAGGATGAATG |
| nbla03771m-f | GCAGCAGATATAGGGACACAGA | nbla03771m-r | CACGCATAAATGGCTACACC |
| nbla03831m-f | GTGACCAGCAAGGAAACACA | nbla03831m-r | GAGGATTCAGCCACGAACA |
| nbla03862m-F | GCATGGAGGAAACCATTAGG | nbla03862m-R | TGGCTCTTCAACCAAACCTT |
| nbla04300m-f | TACAGTGGGAACTGCGTTTG | nbla04300m-f | CAGGGTTCGTATGCAGGAG |
| nbla10120m-F | CAGTGCAGCCTTGGAAGTGT | nbla10120m-R | TCAAAAGCTGCGTGTGTCTC |
| nbla10143m-F3 | CAGTGCAGCCTTGGAAGTGT | nbla10143m-R3 | TCAAAAGCTGCGTGTGTCTC |
| nbla10329m-F | CCGAGATCTTCTGCCTTCAT | nbla10329m-R | TCCTTGCCGTCTCAAACTCT |
| nbla10363m-F3 | ATCTCTCTAGTGCCATGAC | nbla10363m-R3 | AGTCTTGCTAAGACTTTCAG |
| nbla10485m-F | AAATGGCAGTTTGACTGTGG | nbla10485m-R | TTGGCTGAGTTCTCCCTCAT |
| nbla10677m-f | CATAATCTTCTCCGGCTTCATC | nbla10677m-r | GTCTGGTATTTCCGTGAGGTTT |
| nbla10696m-f | TGATTCTCCAAGGCAAGGT | nbla10696m-r | GATTTCCCCATTGACTGCT |
| nbla11051m-f | AGCACAATTCCCCAGACAC | nbla11051m-r | CTGTAGCCCTTACTGTTTGACC |
| nbla11189m-F | CTGTGTTCTGATGCCAATGC | nbla11189m-R | TGCAACTTTCTCCACCAAGA |

As described above, because the superior cervical gangliocytes are known to die (apoptosis) through NGF depletion, the genes that display variations in the expression level are closely connected to the mechanism of apoptosis.

### Industrial Applicability

As described above, this invention discloses gene sequences relating to favorable or unfavorable prognosis of neuroblastoma and will enable the provision of their genetic information and the diagnosis of favorable or unfavorable prognosis.

## Claims

1. A nucleic acid whose expression is enhanced in a human neuroblastoma with favorable prognosis based on comparison between a human neuroblastoma with a favorable prognosis and a human neuroblastoma with an unfavorable prognosis, the nucleic acid comprising any one of the sequences set forth in SEQ ID NO:121, SEQ ID NO:125, SEQ ID NO:132, SEQ ID NO:165, SEQ ID NO:171, SEQ ID NO:187, SEQ ID NO:188, SEQ ID 229, SEQ ID NO:232, SEQ ID NO:273 and SEQ ID NO:281 in the Sequence Listing.

2. A diagnostic agent for the detection of neuroblastomas having favorable prognosis containing at least one nucleic acid according to Claim 1.

3. An in vitro method for diagnosing the prognosis of a human neuroblastoma, the method comprising detecting the presence or absence of a nucleic acid according to claim 1 in a clinical tissue sample of the neuroblastoma.

4. An in vitro method for diagnosing the prognosis of a human neuroblastoma, the method comprising the steps of
detecting the presence or absence of enhancement in the expression of a nucleic acid according to claim 1 in a clinical tissue sample of the neuroblastoma;
comparing the expression level of the nucleic acid in the sample with the expression level of the nucleic acid in a neuroblastoma with unfavorable prognosis; and
diagnosing the prognosis of the human neuroblastoma as favorable if the expression of the nucleic acid is increased in the sample.

## Patentansprüche

1. Nukleinsäure, deren Expression in einem menschlichen Neuroblastom mit günstiger Progose erhöht ist, basierend auf einem Vergleich zwischen einem menschlichen Neuroblastom mit einer günstigen Prognose und einem menschlichen Neuroblastom mit einer ungünstigen Prognose, wobei die Nukleinsäure eine der Sequenzen umfasst, die in SEQ ID NR.:121, SEQ ID NR.:125, SEQ ID NR.:132, SEQ ID NR.:165, SEQ ID NR.:171, SEQ ID NR.:187, SEQ ID NR.:188, SEQ ID NR.:229, SEQ ID NR.:232, SEQ ID NR.:273 und SEQ ID NR.:281 des Sequenzprotokolls dargestellt sind.

2. Diagnostisches Agens für die Detektion von Neuroblastomen mit einer günstigen Prognose, das wenigstens eine Nukleinsäure gemäß Anspruch 1 enthält.

3. *In-vitro* Verfahren zum Diagnostizieren der Prognose von einem menschlichen Neuroblastom, wobei das Verfahren das Detektieren der Anwesenheit oder der Abwesenheit von einer Nukleinsäure gemäß Anspruch 1 in einer klinischen Gewebeprobe von dem Neuroblastom umfasst.

4. *In-vitro* Verfahren zum Diagnostizieren der Prognose von einem menschlichen Neuroblastom, wobei das Verfahren die Schritte umfasst:
Detektieren der Anwesenheit oder der Abwesenheit von einer Erhöhung in der Expression von einer Nukleinsäure gemäß Anspruch 1 in einer klinischen Gewebeprobe von dem Neuroblastom;
Vergleichen des Expressionslevels von der Nukleinsäure in der Probe mit dem Expressionslevel von der Nukleinsäure in einem Neuroblastom mit ungünstiger Prognose; und
Diagnostizieren der Prognose von dem menschlichen Neuroblastom als günstig, wenn die Expression von der Nukleinsäure in der Probe erhöht ist.

## Revendications

1. Acide nucléique dont l'expression est accrue dans un neuroblastome humain avec un pronostic favorable sur la base de la comparaison entre un neuroblastome humain avec un pronostic favorable et un neuroblastome humain avec un pronostic défavorable, l'acide nucléique comprenant au moins l'une quelconque des séquences définies dans SEQ ID NO: 121, SEQ ID NO: 125, SEQ ID NO: 132, SEQ ID NO: 165, SEQ ID NO: 171, SEQ ID NO: 187, SEQ ID NO: 188, SEQ ID NO: 229, SEQ ID NO: 232, SEQ ID NO: 273 et SEQ ID NO: 281 dans le listage de séquences.

2. Agent de diagnostic pour la détection de neuroblastomes ayant un pronostic favorable, contenant au moins un acide nucléique selon la revendication 1.

3. Méthode in vitro pour diagnostiquer le pronostic d'un neuroblastome humain, la méthode comprenant la détection de la présence ou de l'absence d'un acide nucléique selon la revendication 1 dans un échantillon clinique de tissu du neuroblastome.

4. Méthode in vitro pour diagnostiquer le pronostic d'un neuroblastome humain, la méthode comprenant les étapes de :
détection de la présence ou de l'absence de l'augmentation de l'expression d'un acide nucléique selon la revendication 1 dans un échantillon clinique de tissu du neuroblastome ;
comparaison du niveau d'expression de l'acide nucléique dans l'échantillon avec le niveau d'expression de l'acide nucléique dans un neuroblastome avec un pronostic défavorable ; et
diagnostic du pronostic du neuroblastome humain comme étant favorable si l'expression de l'acide nucléique est accrue dans l'échantillon.
